# EUROPEAN PATENT APPLICATION

(11) **EP 4 427 590 A2**
(43) Date of publication of application: **11.09.2024**
(21) Application number: 24176079.2
(22) Date of filing: 15.04.2022
(51) Int. Cl.: A01N 43/00

(54) **ANTIBODY DRUG CONJUGATES AND METHODS FOR MAKING THEREOF**

(30) Priority: 16.04.2021 US 202163176046 P; 08.10.2021 US 202163254031 P
(62) Divisional of application: 22726208.6
(71) Applicant: Novartis AG, 4056 Basel (CH)
(72) Inventor: FAN, Liqiong, Cambridge (US); HAINZL, Dominik, Cambridge (US); RAMOT, Roee, Cambridge (US); IYER, Shwetha, Cambridge (US); D'ALESSIO, Joseph Anthony, Cambridge (US); WUERSCH, Kuno, Basel (CH); PISTORIUS, Dominik, Basel (CH); MANCHADO ROBLES, Eusebio, Basel (CH); BUNTIN, Kathrin, Basel (CH); GABRIEL, Doris, Basel (CH); FESSLER, Boris, Basel (CH); MAUDENS, Pierre, Basel (CH); ROMANET, Vincent, Basel (CH); BLUEMMEL, Anne-Sophie, Basel (CH); HOFFMASTER, Keith, Cambridge (US); KHERA, Eshita, Cambridge (US); YERRAMILLI-RAO, Padmaja, Cambridge (US)
(74) Representative: Mathys & Squire

(57) **Abstract**

This application discloses microorganisms and methods of producing GNAQ/GNA11 inhibitors and methods of making antibody drug conjugates of anti-PMEL17 antibodies or antigen binding fragments conjugated to a GNAQ/GNA11 inhibitor. The disclosure also relates to formulations comprising antibody drug conjugates of anti-PMEL17 antibodies or antigen binding fragments conjugated to a GNAQ/GNA11 inhibitor and methods of treating or preventing cancer using the formulations.

## Description

### CROSS REFERENCE TO RELATED APPLICATIONS

This application claims the benefit of U.S. Provisional Application No. 63/176,046, filed April 16, 2021, and U.S. Provisional Application No. 63/254,031, filed October 8, 2021. The contents of the aforementioned applications are hereby incorporated by reference in their entirety.

### SEQUENCE LISTING

The instant application contains a Sequence Listing which has been submitted electronically in ASCII format and is hereby incorporated by reference in its entirety. Said ASCII copy, created on April 15, 2022, is named N2067-7179WO_SL.txt and is 239,071 bytes in size.

### BACKGROUND

Uveal melanoma (UM) is a rare malignancy that accounts for 3-5% of all melanomas with an incident rate of 6-10 cases per 1 million people and 2500-3000 new cases in the US per year. Despite excellent rates of local disease control (5 year-OSR 70%), up to 50% of patients will develop metastatic disease (liver 60%, lung 25%; median OS 13 months, 2 year-OSR 8%). No proven standard of care is available for patients who do develop metastatic disease. There is a high medical need for specifically approved treatments and dedicated disease management strategies in order to improve outcomes for patients with metastatic UM.

Oncogenic mutations of GNAQ or GNA11 are the hallmark mutations in uveal melanoma. Reduction of GNAQ/11 levels leads to inhibition of cell proliferation and apoptosis. The inhibitory effect can be achieved by targeted delivery of antibody drug conjugates that inhibit GNAQ/11 activities. Despite the recent progress, the need still exists for developing novel biological materials, manufacturing methods, and formulations for the antibody drug conjugates.

### SUMMARY

The disclosure provides, at least in part, genetically engineered microorganisms (*e.g.*, *Chromobacterium vaccinii*) that produce a depsipeptide, *e.g.,* compound (A1), at high titers and/or isolated yields. In some embodiments, the microorganism includes a non-native promoter operably linked to the biosynthetic gene cluster (BGC) of the depsipeptide. In some embodiments, the BGC of a natural product that interferes with the isolation of the depsipeptide is disrupted. Methods of using the microorganisms described herein to produce depsipeptides, *e.g.*, compound (A1), are also described. The depsipeptides, *e.g.,* compound (A1), produced by a method described herein can be used to produce antibody drug conjugates (*e.g.*, an antibody drug conjugate described herein), *e.g.,* in accordance with a process described herein.

The disclosure also provides, at least in part, processes for producing partially reoxidized antibodies or antigen binding fragments thereof. In some embodiments, the process comprises reducing one or more capped cysteine residues in an antibody or antigen binding fragment thereof to provide an antibody or antigen binding fragment thereof comprising one or more decapped cysteine residues. In some embodiments, the process comprises storing an antibody or antigen binding fragment thereof comprising one or more decapped cysteine residues under oxidation conditions sufficient to produce a partially reoxidized antibody or antigen binding fragment thereof. Also described herein are processes of making antibody drug conjugates (*e.g.*, an antibody drug conjugate described herein) that uses a process for producing partially reoxidized antibody or antigen binding fragment thereof described herein. The antibody drug conjugates made by a process described herein can be formulated to a formulation described herein.

The disclosure also provides, at least in part, formulations for antibody drug conjugates (e.g., an antibody drug conjugate described herein). In some embodiments, the formulation comprises an antibody drug conjugate described herein, a buffering agent, a stabilizing agent, and a surfactant, and has a pH of about 4.5 to about 6.5. In some embodiments, the formulation is a lyophilized formulation. The formulations described herein can be used to treat or prevent a disorder, *e.g.*, a cancer. In some embodiments, the cancer expresses PMEL17 and/or contains a mutation in the GNAQ or GNA11 gene. In some embodiments, the cancer is a carcinoma *(*e*.g.,* hepatocellular carcinoma), sarcoma, leukemia, lymphoma, eye cancer, eye neoplasm, melanoma (*e.g.,* uveal melanoma, non-uveal melanoma, malignant melanoma, ocular melanoma, subcutaneous melanoma, cutaneous melanoma, or mucosal melanoma), or a metastatic cancer thereof.

### Engineered Microorganisms and Methods of Producing Compounds

In an aspect, the disclosure features a microorganism, comprising a nucleic acid that comprises a nucleotide sequence encoding a polypeptide associated with the production of a compound having the structure of Formula (A1), wherein the nucleotide sequence is operably linked to a non-native promoter.

In some embodiments, the microorganism is a genetically engineered form of a microorganism that naturally produces the compound. In some embodiments, the microorganism is a bacterium. In some embodiments, the microorganism is a *Chromobacterium.* In some embodiments, the microorganism is *Chromobacterium vaccinii.* In some embodiments, the microorganism is *Chromobacterium vaccinii* DSM 25150 (= ATCC BAA-2314, = NRRL B-50840, = MWU205). In some embodiments, the microorganism is an isolated microorganism. In some embodiments, the microorganism is a synthetic microorganism.

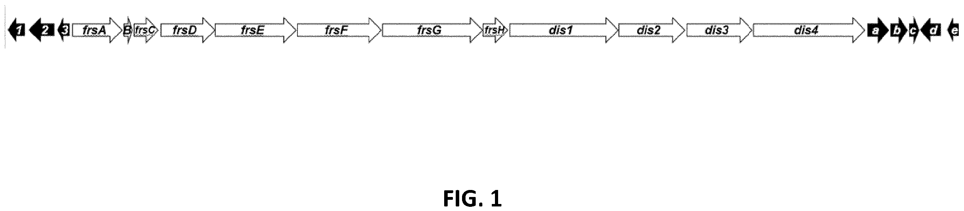

In some embodiments, the nucleic acid is on a chromosome of the microorganism, *e.g.*, naturally located on the chromosome or stably integrated to the chromosome. In some embodiments, the nucleotide sequence encoding a polypeptide associated with the production of the compound is located in a biosynthetic gene cluster (BGC). In some embodiments, the BGC is a compound (A1)-BGC. In some embodiments, the compound (A1)-BGC has the gene organization shown in **FIG. 1**.

In some embodiments, the BGC comprises one or more (e.g., two, three, four, five, six, seven, or all) *of frsA, frsB, frsC, frsD, frsE, frsF, frsG,* or *frsH,* or a homolog thereof. In some embodiments, the BGC comprises *frsA, frsB, frsC, frsD, frsE, frsF, frsG,* and *frsH,* or a homolog thereof.

In some embodiments, the nucleotide sequence encoding a polypeptide associated with the production of the compound is located in *frsA, frsB, frsC, frsD, frsE, frsF, frsG,* or *frsH,* or a homolog thereof. In some embodiments, the nucleotide sequence encoding a polypeptide associated with the production of the compound is located in a region comprising *frsA* or a homolog thereof. In some embodiments, the nucleotide sequence encoding a polypeptide associated with the production of the compound is located in a region comprising *frsA* and *frsB,* or a homolog thereof. In some embodiments, the nucleotide sequence encoding a polypeptide associated with the production of the compound is located in a region comprising *frsA, frsB, and frsC,* or a homolog thereof. In some embodiments, the nucleotide sequence encoding a polypeptide associated with the production of the compound is located in a region comprising *frsA, frsB, frsC, and frsD,* or a homolog thereof. In some embodiments, the nucleotide sequence encoding a polypeptide associated with the production of the compound is located in a region comprising *frsA, frsB, frsC, frsD, and frsE,* or a homolog thereof. In some embodiments, the nucleotide sequence encoding a polypeptide associated with the production of the compound is located in a region comprisingfrsa, *frsB, frsc, frsD, frsE, and frsF,* or a homolog thereof. In some embodiments, the nucleotide sequence encoding a polypeptide associated with the production of the compound is located in a region comprising *frsA*, *frsB, frsC, frsD, frsE, frsF,* and *frsG,* or a homolog thereof. In some embodiments, the nucleotide sequence encoding a polypeptide associated with the production of the compound is located in a region comprising *frsA, frsB, frsC, frsD, frsE, frsF, frsG,* and *FrsH,* or a homolog thereof.

In some embodiments, the nucleotide sequence encoding a polypeptide associated with the production of the compound comprises the coding sequence of *frsA, frsB, frsC, frsD, frsE, frsF, frsG,* or *frsH,* or a homolog thereof. In some embodiments, the nucleotide sequence encoding a polypeptide associated with the production of the compound comprises the coding sequence of *frsA* or a homolog thereof. In some embodiments, the nucleotide sequence encoding a polypeptide associated with the production of the compound comprises the coding sequence of *frsA* and *frsB,* or a homolog thereof. In some embodiments, the nucleotide sequence encoding a polypeptide associated with the production of the compound comprises the coding sequence of *frsA, frsB,* and *frsC,* or a homolog thereof. In some embodiments, the nucleotide sequence encoding a polypeptide associated with the production of the compound comprises the coding sequence of *frsA, frsB, frsC,* and *frsD,* or a homolog thereof. In some embodiments, the nucleotide sequence encoding a polypeptide associated with the production of the compound comprises the coding sequence of *frsA, frsB, frsC, frsD,* and *frsE,* or a homolog thereof. In some embodiments, the nucleotide sequence encoding a polypeptide associated with the production of the compound comprises the coding sequence *offrsa, frsb, frsc, frsd, frse,* and *frsF,* or a homolog thereof. In some embodiments, the nucleotide sequence encoding a polypeptide associated with the production of the compound comprises the coding sequence of*frsA, frsB, frsC, frsD, frsE, frsF,* and *frsG,* or a homolog thereof. In some embodiments, the nucleotide sequence encoding a polypeptide associated with the production of the compound comprises the coding sequence of *frsA, frsB, frsC, frsD, frsE, frsF, frsG,* and *FrsH,* or a homolog thereof.

In some embodiments, the nucleic acid comprises a nucleotide sequence resulted from a homologous recombination event (*e.g.,* for promoter exchange) using the nucleotide sequence of SEQ ID NO: 317, or a functional fragment thereof, or a nucleotide sequence having at least about 80%, about 85%, about 90%, about 95%, about 96%, about 97%, about 98%, or about 99% identity thereto, or differing by no more than about 100, about 90, about 80, about 70, about 60, about 50, about 40, about 30, about 20, about 15, about 10, about 5, or about 2 nucleotides therefrom.

In some embodiments, the nucleic acid comprises a nucleotide sequence associated GenBank accession number: BankIt2437961 BSeq#1 MW732719, or a functional fragment thereof, or a nucleotide sequence having at least about 75%, about 80%, about 85%, about 90%, about 95%, about 96%, about 97%, about 98%, or about 99% identity thereto.

In certain embodiments, the non-native promoter is from the same microorganism. In other embodiments, the non-native promoter is from a different microorganism.

In some embodiments, the non-native promoter comprises a *vioP* promoter, an *nptII* promoter, an *rbs* promoter, a J23119 promoter, a *pLpp* promoter, a *PS12burk* promoter, an *ErmE** promoter, or a *Pem* 7 promoter. In some embodiments, the non-native promoter comprises a *vioP* promoter, an *nptII* promoter, or an *rbs* promoter.

In some embodiments, the non-native promoter comprises the nucleotide sequence of any of SEQ ID NOs: 264-266, 268-271, or 316, or a functional fragment thereof, or a nucleotide sequence having at least about 80%, about 85%, about 90%, about 95%, about 96%, about 97%, about 98%, or about 99% identity thereto, or differing by no more than about 60, about 50, about 40, about 30, about 20, about 15, about 10, about 5, or about 2 nucleotides therefrom. In some embodiments, the non-native promoter comprises or consists of the nucleotide sequence of any of SEQ ID NOs: 264-266, 268-271, or 316 or a functional fragment thereof.

In some embodiments, the non-native promoter comprises a *vioP* promoter. In some embodiments, the non-native promoter comprises the nucleotide sequence of SEQ ID NO: 264, or a functional fragment thereof, or a nucleotide sequence having at least about 80%, about 85%, about 90%, about 95%, about 96%, about 97%, about 98%, or about 99% identity thereto, or differing by no more than about 60, about 50, about 40, about 30, about 20, about 15, about 10, about 5, or about 2 nucleotides therefrom. In some embodiments, the non-native promoter comprises or consists of the nucleotide sequence of SEQ ID NO: 264, or a functional fragment thereof.

In some embodiments, the non-native promoter comprises an *nptII* promoter. In some embodiments, the non-native promoter comprises the nucleotide sequence of SEQ ID NO: 265, or a functional fragment thereof, or a nucleotide sequence having at least 80%, 85%, 90%, 95%, 96%, 97%, 98%, or 99% identity thereto, or differing by no more than about 60, about 50, about 40, about 30, about 20, about 15, about 10, about 5, or about 2 nucleotides therefrom. In some embodiments, the non-native promoter comprises or consists of the nucleotide sequence of SEQ ID NO: 265, or a functional fragment thereof.

In some embodiments, the non-native promoter comprises an *rbs* promoter. In some embodiments, the non-native promoter comprises the nucleotide sequence of SEQ ID NO: 266, or a functional fragment thereof, or a nucleotide sequence having at least 80%, 85%, 90%, 95%, 96%, 97%, 98%, or 99% identity thereto, or differing by no more than 60, 50, 40, 30, 20, 15, 10, 5, or 2 nucleotides therefrom. In some embodiments, the non-native promoter comprises or consists of the nucleotide sequence of SEQ ID NO: 266, or a functional fragment thereof.

In some embodiments, the non-native promoter comprises a *J23119* promoter. In some embodiments, the non-native promoter comprises the nucleotide sequence of SEQ ID NO: 316, or a functional fragment thereof, or a nucleotide sequence having at least 80%, 85%, 90%, 95%, 96%, 97%, 98%, or 99% identity thereto, or differing by no more than 60, 50, 40, 30, 20, 15, 10, 5, or 2 nucleotides therefrom. In some embodiments, the non-native promoter comprises or consists of the nucleotide sequence of SEQ ID NO: 316, or a functional fragment thereof.

In some embodiments, the non-native promoter comprises a *pLpp* promoter. In some embodiments, the non-native promoter comprises the nucleotide sequence of SEQ ID NO: 268, or a functional fragment thereof, or a nucleotide sequence having at least 80%, 85%, 90%, 95%, 96%, 97%, 98%, or 99% identity thereto, or differing by no more than 60, 50, 40, 30, 20, 15, 10, 5, or 2 nucleotides therefrom. In some embodiments, the non-native promoter comprises or consists of the nucleotide sequence of SEQ ID NO: 268, or a functional fragment thereof.

In some embodiments, the non-native promoter comprises a *Pem* 7 promoter. In some embodiments, the non-native promoter comprises the nucleotide sequence of SEQ ID NO: 269, or a functional fragment thereof, or a nucleotide sequence having at least 80%, 85%, 90%, 95%, 96%, 97%, 98%, or 99% identity thereto, or differing by no more than 60, 50, 40, 30, 20, 15, 10, 5, or 2 nucleotides therefrom. In some embodiments, the non-native promoter comprises or consists of the nucleotide sequence of SEQ ID NO: 269, or a functional fragment thereof.

In some embodiments, the non-native promoter comprises a *PS12burk* promoter. In some embodiments, the non-native promoter comprises the nucleotide sequence of SEQ ID NO: 270, or a functional fragment thereof, or a nucleotide sequence having at least 80%, 85%, 90%, 95%, 96%, 97%, 98%, or 99% identity thereto, or differing by no more than 60, 50, 40, 30, 20, 15, 10, 5, or 2 nucleotides therefrom. In some embodiments, the non-native promoter comprises or consists of the nucleotide sequence of SEQ ID NO: 270, or a functional fragment thereof.

In some embodiments, the non-native promoter comprises an *ErmE** promoter. In some embodiments, the non-native promoter comprises the nucleotide sequence of SEQ ID NO: 271, or a functional fragment thereof, or a nucleotide sequence having at least 80%, 85%, 90%, 95%, 96%, 97%, 98%, or 99% identity thereto, or differing by no more than 60, 50, 40, 30, 20, 15, 10, 5, or 2 nucleotides therefrom. In some embodiments, the non-native promoter comprises or consists of the nucleotide sequence of SEQ ID NO: 271, or a functional fragment thereof.

In some embodiments, the non-native promoter controls the transcription of one or more (e.g., two, three, four, five, six, seven, or all) of *FrsA, FrsB, FrsC, FrsD, FrsE, FrsF, FrsG,* or *FrsH,* or a homolog thereof. In some embodiments, the non-native promoter controls the transcription *of FrsA, FrsB, FrsC, FrsD, FrsE, FrsF, FrsG,* and *FrsH,* or a homolog thereof. In some embodiments, the non-native promoter is inserted upstream of the coding region of *FrsA,* or a homolog thereof, *e.g.,* upstream of the coding region of all of *FrsA, FrsB, FrsC, FrsD, FrsE, FrsF, FrsG,* and *FrsH,* or a homolog thereof.

In some embodiments, the polypeptide associated with the production of the compound is a non-ribosomal peptide synthetase (NRPS) or a functional fragment thereof. In some embodiments, the NRPS is FrsA, FrsD, FrsE, FrsF, or FrsG, or a homolog thereof.

In some embodiments, the polypeptide associated with the production of the compound is a MbtH-like protein or a functional fragment thereof. In some embodiments, the MbtH-like protein is FrsB or a homolog thereof.

In some embodiments, the polypeptide associated with the production of the compound is a malate dehydrogenase. In some embodiments, the malate dehydrogenase is FrsC or a homolog thereof.

In some embodiments, the polypeptide associated with the production of the compound is a hydroxylase. In some embodiments, the hydroxylase is FrsH or a homolog thereof.

In some embodiments, the nucleic acid comprises a plurality of nucleotide sequences encoding a plurality of polypeptides associated with the production of the compound. In some embodiments, the plurality of polypeptides associated with the production of the compound comprises a plurality of NRPSs, or a functional fragment thereof. In some embodiments, the plurality of NRPSs comprise two or more (*e.g.,* three, four, or all) of FrsA, FrsD, FrsE, FrsF, or FrsG, or a homolog thereof. In some embodiments, the plurality of polypeptides associated with the production of the compound comprises an NRPS, a MbtH-like protein, a malate dehydrogenase, and a hydroxylase, or a functional fragment thereof.

In some embodiments, the plurality of polypeptides associated with the production of the compound comprises FrsA, FrsB, FrsC, FrsD, FrsE, FrsF, FrsG, and FrsH, or a homolog thereof.

In some embodiments, the microorganism has an increased titer of the compound. In some embodiments, the titer of the compound is increased by at least about 1, about 2, about 3, about 4, about 5, about 6, about 7, about 8, about 9, or about 10-fold, compared to a reference microorganism, *e.g.,* an otherwise identical microorganism that does not comprise the non-native promoter in the compound (A1)-BGC (e.g., a wild-type), when cultured under conditions that allow production of the compound. In some embodiments, the titer of the compound is increased by at least about 100 mg/L, about 200 mg/L, about 300 mg/L, about 400 mg/L, about 500 mg/L, about 600 mg/L, about 700 mg/L, about 800 mg/L, about 900 mg/L, or about 1000 mg/L, compared to a reference microorganism, *e.g.*, an otherwise identical microorganism that does not comprise the non-native promoter in the compound (A1)-BGC (e.g., a wild-type), when cultured under conditions that allow production of the compound.

In some embodiments, the BGC of a natural product that interferes with the isolation of the compound is altered, *e.g.*, disrupted. In some embodiments, the natural product comprises one or more (*e.g.,* two, three, or all) of: violacein, compound J, compound F5, compound F3, or compound D. In some embodiments, at least a portion of the BGC of the natural product interferes with the isolation of the compound is deleted. In some embodiments, the violacein-BGC is altered, *e.g.*, disrupted. In some embodiments, the compound J-BGC is altered, *e.g.*, disrupted. In some embodiments, the compound J-BGC is associated with the production of compound J, compound F5, compound F3, and compound D. In some embodiments, both the violacein-BGC and the compound J-BGC are altered, *e.g*., disrupted. In some embodiments, the production of the natural product is reduced, *e.g.*, by at least about 50%, about 60%, about 70%, about 80%, about 90%, about 95%, about 96%, about 97%, about 98%, about 99%, or about 100%.

In some embodiments, the microorganism has an increased isolation yield of the compound. In some embodiments, the isolation yield of the compound is increased by about 20%, about 30%, about 40%, about 50%, about 60%, about 70%, about 80%, about 90%, or more, compared to a reference microorganism, *e.g.*, an otherwise identical microorganism in which the BGC of the natural product that interferes with the isolation of the compound is not altered (*e.g.,* a wild-type).

In some embodiments, the BGC of a natural product that interferes with the isolation of the compound is not altered, *e.g.,* not disrupted.

In another aspect, the disclosure features a microorganism that produces a compound having the structure of Formula (A1), wherein the BGC of a natural product that interferes with the isolation of the compound is altered.

In some embodiments, the natural product comprises one or more (*e.g.,* two, three, or all) of: violacein, compound J, compound F5, compound F3, or compound D. In some embodiments, at least a portion of the BGC of the natural product interferes with the isolation of the compound is deleted. In some embodiments, the violacein-BGC is altered, *e.g.*, disrupted. In some embodiments, the compound J-BGC is altered, *e.g.*, disrupted. In some embodiments, the compound J-BGC is associated with the production of compound J, compound F5, compound F3, and compound D. In some embodiments, both the violacein-BGC and the compound J-BGC are altered, *e.g.*, disrupted. In some embodiments, the production of the natural product is reduced, *e.g.*, by at least about 50%, about 60%, about 70%, about 80%, about 90%, about 95%, about 96%, about 97%, about 98%, about 99%, or 100%.

In some embodiments, the microorganism has an increased isolation yield of the compound. In some embodiments, the isolation yield of the compound is increased by about 20%, about 30%, about 40%, about 50%, about 60%, about 70%, about 80%, about 90%, or more, compared to a reference microorganism, *e.g.*, an otherwise identical microorganism in which the BGC of the natural product that interferes with the isolation of the compound is not altered (*e.g.,* a wild-type).

In another aspect, the disclosure features a method of producing a compound having the structure of Formula (A1), comprising culturing (*e.g.,* fermenting) a microorganism described herein under conditions that allow the expression of the compound, thereby producing the compound.

In some embodiments, the microorganism is cultured (e.g., fermented) at about 50 L to about 500 L scale, *e.g.*, at about 50 L, about 100 L, about 150 L, about 200 L, about 250 L, about 300 L, about 350 L, about 400 L, about 450 L, or about 500 L scale. In some embodiments, the microorganism is cultured (*e.g.,* fermented) at about 1,000 L to about 20,000 L scale, *e.g.*, about 1,000 L to about 10,000 L or about 10,000 L to about 20,000 L, *e.g*., about 1,000 L, about 2,000 L, about 5,000 L, about 7,500 L, about 10,000 L, about 15,000 L, or about 20,000 L scale.

In some embodiments, the culturing (*e.g.,* fermentation) yields a titer of at least about 200 mL of the compound, *e.g.*, at least about 250 mg/mL, about 300 mg/L, about 400 mg/L, about 500 mg/L, about 600 mg/L, about 700 mg/L, about 800 mg/L, about 900 mg/L, about 1000 mg/L, about 1100 mg/L, about 1200 mg/L, about 1300 mg/L, about 1400 mg/L, about 1500 mg/L, about 1600 mg/L, about 1700 mg/L, about 1800 mg/L, about 1900 mg/L, or about 2000 mg/L of the compound.

In some embodiments, the method further comprises isolating the compound, *e.g*., to a purity of at least about 90%, *e.g.,* at least about 95%, about 96%, about 97%, about 98%, or about 99%, *e.g.,* as determined by a method described herein.

In another aspect, the disclosure features a nucleic acid that comprises a nucleotide sequence encoding a polypeptide associated with the production of a compound having the structure of Formula (A1), wherein the nucleotide sequence is operably linked to a non-native promoter.

In some embodiments, the nucleic acid is on a chromosome of the microorganism, *e.g*., naturally located on the chromosome or stably integrated to the chromosome. In some embodiments, the nucleotide sequence encoding a polypeptide associated with the production of the compound is located in a biosynthetic gene cluster (BGC). In some embodiments, the BGC is a compound (A1)-BGC. In some embodiments, the compound (A1)-BGC has the gene organization shown in **FIG. 1****.**

In some embodiments, the BGC comprises one or more (e.g., two, three, four, five, six, seven, or all) *offrsA, frsB, frsC, frsD, frsE, frsF, frsG,* or *frsH,* or a homolog thereof. In some embodiments, the BGC comprises *frsA, frsB, frsC, frsD, frsE, frsF, frsG,* and *frsH,* or a homolog thereof.

In some embodiments, the nucleotide sequence encoding a polypeptide associated with the production of the compound is located in *frsA, frsB, frsC, frsD, frsE, frsF, frsG, frsH,* or a homolog thereof. In some embodiments, the nucleotide sequence encoding a polypeptide associated with the production of the compound is located in a region comprising *frsA* or a homolog thereof. In some embodiments, the nucleotide sequence encoding a polypeptide associated with the production of the compound is located in a region comprising *frsA* and *frsB,* or a homolog thereof. In some embodiments, the nucleotide sequence encoding a polypeptide associated with the production of the compound is located in a region comprising *frsA, frsB, and frsC,* or a homolog thereof. In some embodiments, the nucleotide sequence encoding a polypeptide associated with the production of the compound is located in a region comprising *frsA, frsB, frsC, and frsD,* or a homolog thereof. In some embodiments, the nucleotide sequence encoding a polypeptide associated with the production of the compound is located in a region comprising *frsA, frsB, frsC, frsD, and frsE,* or a homolog thereof. In some embodiments, the nucleotide sequence encoding a polypeptide associated with the production of the compound is located in a region comprising*frsA*, *frsB, frsc, frsD, frsE,* and *frsF,* or a homolog thereof. In some embodiments, the nucleotide sequence encoding a polypeptide associated with the production of the compound is located in a region comprising*frsA*, *frsB, frsC, frsD, frsE, frsF,* and *frsG,* or a homolog thereof. In some embodiments, the nucleotide sequence encoding a polypeptide associated with the production of the compound is located in a region comprising *frsA*, *frsB, frsC, frsD, frsE, frsF, frsG,* and *FrsH,* or a homolog thereof.

In some embodiments, the nucleotide sequence encoding a polypeptide associated with the production of the compound comprises the coding sequence of *frsA, frsB, frsC, frsD, frsE, frsF, frsG, frsH,* or a homolog thereof. In some embodiments, the nucleotide sequence encoding a polypeptide associated with the production of the compound comprises the coding sequence of *frsA* or a homolog thereof. In some embodiments, the nucleotide sequence encoding a polypeptide associated with the production of the compound comprises the coding sequence of *frsA* and *frsB,* or a homolog thereof. In some embodiments, the nucleotide sequence encoding a polypeptide associated with the production of the compound comprises the coding sequence of *frsA, frsB, and frsC,* or a homolog thereof. In some embodiments, the nucleotide sequence encoding a polypeptide associated with the production of the compound comprises the coding sequence of *frsA, frsB, frsC, and frsD,* or a homolog thereof. In some embodiments, the nucleotide sequence encoding a polypeptide associated with the production of the compound comprises the coding sequence of *frsA, frsB, frsC, frsD,* and *frsE,* or a homolog thereof. In some embodiments, the nucleotide sequence encoding a polypeptide associated with the production of the compound comprises the coding sequence of *frsA, frsB, frsc, frsD, frsE,* and*frsF,* or a homolog thereof. In some embodiments, the nucleotide sequence encoding a polypeptide associated with the production of the compound comprises the coding sequence *of frsA, frsB, frsC', frsD, frsE, frsF,* and *frsG,* or a homolog thereof. In some embodiments, the nucleotide sequence encoding a polypeptide associated with the production of the compound comprises the coding sequence of *frsA, frsB, frsC, frsD, frsE, frsF, frsG,* and *FrsH,* or a homolog thereof.

In some embodiments, the nucleic acid comprises the nucleotide sequence of SEQ ID NO: 317, or a functional fragment thereof, or a nucleotide sequence having at least 80%, about 85%, about 90%, about 95%, about 96%, about 97%, about 98%, or about 99% identity thereto, or differing by no more than about 100, about 90, about 80, about 70, about 60, about 50, about 40, about 30, about 20, about 15, about 10, about 5, or about 2 nucleotides therefrom.

In some embodiments, the nucleic acid comprises a nucleotide sequence associated GenBank accession number: BankIt2437961 BSeq#1 MW732719, or a functional fragment thereof, or a nucleotide sequence having at least about 75%, about 80%, about 85%, about 90%, about 95%, about 96%, about 97%, about 98%, or about 99% identity thereto.

In certain embodiments, the non-native promoter is from the same microorganism. In other embodiments, the non-native promoter is from a different microorganism.

In some embodiments, the non-native promoter comprises a *vioP* promoter, an *nptII* promoter, an *rbs* promoter, a *J23119* promoter, a *pLpp* promoter, a *PS12burk* promoter, an *ErmE** promoter, or a *Pem* 7 promoter. In some embodiments, the non-native promoter comprises a *vioP* promoter, an *nptII* promoter, or an *rbs* promoter.

In some embodiments, the non-native promoter comprises the nucleotide sequence of any of SEQ ID NOs: 264-266, 268-271, or 316, or a functional fragment thereof, or a nucleotide sequence having at least about 80%, about 85%, about 90%, about 95%, about 96%, about 97%, about 98%, or about 99% identity thereto, or differing by no more than about 60, about 50, about 40, about 30, about 20, about 15, about 10, about 5, or about 2 nucleotides therefrom. In some embodiments, the non-native promoter comprises or consists of the nucleotide sequence of any of SEQ ID NOs: 264-266, 268-271, or 316, or a functional fragment thereof.

In some embodiments, the non-native promoter comprises a *vioP* promoter. In some embodiments, the non-native promoter comprises the nucleotide sequence of SEQ ID NO: 264, or a functional fragment thereof, or a nucleotide sequence having at least 80%, about 85%, about 90%, about 95%, about 96%, about 97%, about 98%, or about 99% identity thereto, or differing by no more than about 60, about 50, about 40, about 30, about 20, about 15, about 10, about 5, or about 2 nucleotides therefrom. In some embodiments, the non-native promoter comprises or consists of the nucleotide sequence of SEQ ID NO: 264, or a functional fragment thereof.

In some embodiments, the non-native promoter comprises an *nptII* promoter. In some embodiments, the non-native promoter comprises the nucleotide sequence of SEQ ID NO: 265, or a functional fragment thereof, or a nucleotide sequence having at least 80%, 85%, 90%, 95%, 96%, 97%, 98%, or 99% identity thereto, or differing by no more than about 60, about 50, about 40, about 30, about 20, about 15, about 10, about 5, or about 2 nucleotides therefrom. In some embodiments, the non-native promoter comprises or consists of the nucleotide sequence of SEQ ID NO: 265, or a functional fragment thereof.

In some embodiments, the non-native promoter comprises an *rbs* promoter. In some embodiments, the non-native promoter comprises the nucleotide sequence of SEQ ID NO: 266, or a functional fragment thereof, or a nucleotide sequence having at least 80%, about 85%, about 90%, about 95%, about 96%, about 97%, about 98%, or about 99% identity thereto, or differing by no more than about 60, about 50, about 40, about 30, about 20, about 15, about 10, about 5, or about 2 nucleotides therefrom. In some embodiments, the non-native promoter comprises or consists of the nucleotide sequence of SEQ ID NO: 266, or a functional fragment thereof.

In some embodiments, the non-native promoter comprises a *J23119* promoter. In some embodiments, the non-native promoter comprises the nucleotide sequence of SEQ ID NO: 316, or a functional fragment thereof, or a nucleotide sequence having at least 80%, about 85%, about 90%, about 95%, about 96%, about 97%, about 98%, or about 99% identity thereto, or differing by no more than about 60, about 50, about 40, about 30, about 20, about 15, about 10, about 5, or about 2 nucleotides therefrom. In some embodiments, the non-native promoter comprises or consists of the nucleotide sequence of SEQ ID NO: 316, or a functional fragment thereof.

In some embodiments, the non-native promoter comprises a *pLpp* promoter. In some embodiments, the non-native promoter comprises the nucleotide sequence of SEQ ID NO: 268, or a functional fragment thereof, or a nucleotide sequence having at least 80%, about 85%, about 90%, about 95%, about 96%, about 97%, about 98%, or about 99% identity thereto, or differing by no more than about 60, about 50, about 40, about 30, about 20, about 15, about 10, about 5, or about 2 nucleotides therefrom. In some embodiments, the non-native promoter comprises or consists of the nucleotide sequence of SEQ ID NO: 268, or a functional fragment thereof.

In some embodiments, the non-native promoter comprises a *Pem* 7 promoter. In some embodiments, the non-native promoter comprises the nucleotide sequence of SEQ ID NO: 269, or a functional fragment thereof, or a nucleotide sequence having at least 80%, about 85%, about 90%, about 95%, about 96%, about 97%, about 98%, or about 99% identity thereto, or differing by no more than about 60, about 50, about 40, about 30, about 20, about 15, about 10, about 5, or about 2 nucleotides therefrom. In some embodiments, the non-native promoter comprises or consists of the nucleotide sequence of SEQ ID NO: 269, or a functional fragment thereof.

In some embodiments, the non-native promoter comprises a *PS12burk* promoter. In some embodiments, the non-native promoter comprises the nucleotide sequence of SEQ ID NO: 270, or a functional fragment thereof, or a nucleotide sequence having at least 80%, about 85%, about 90%, about 95%, about 96%, about 97%, about 98%, or about 99% identity thereto, or differing by no more than about 60, about 50, about 40, about 30, about 20, about 15, about 10, about 5, or about 2 nucleotides therefrom. In some embodiments, the non-native promoter comprises or consists of the nucleotide sequence of SEQ ID NO: 270, or a functional fragment thereof.

In some embodiments, the non-native promoter comprises an *ErmE** promoter. In some embodiments, the non-native promoter comprises the nucleotide sequence of SEQ ID NO: 271, or a functional fragment thereof, or a nucleotide sequence having at least 80%, about 85%, about 90%, about 95%, about 96%, about 97%, about 98%, or about 99% identity thereto, or differing by no more than about 60, about 50, about 40, about 30, about 20, about 15, about 10, about 5, or about 2 nucleotides therefrom. In some embodiments, the non-native promoter comprises or consists of the nucleotide sequence of SEQ ID NO: 271, or a functional fragment thereof.

In some embodiments, the non-native promoter controls the transcription of one or more (e.g., two, three, four, five, six, seven, or all) of *FrsA, FrsB, FrsC, FrsD, FrsE, FrsF, FrsG,* or *FrsH,* or a homolog thereof. In some embodiments, the non-native promoter controls the transcription *of FrsA, FrsB, FrsC, FrsD, FrsE, FrsF, FrsG,* and *FrsH,* or a homolog thereof. In some embodiments, the non-native promoter is inserted upstream of the coding region of *FrsA,* or a homolog thereof, *e.g.,* upstream of the coding region of all of *FrsA, FrsB, FrsC, FrsD, FrsE, FrsF, FrsG,* and *FrsH,* or a homolog thereof.

In some embodiments, the polypeptide associated with the production of the compound is a non-ribosomal peptide synthetase (NRPS) or a functional fragment thereof. In some embodiments, the NRPS is FrsA, FrsD, FrsE, FrsF, or FrsG, or a homolog thereof.

In some embodiments, the polypeptide associated with the production of the compound is a MbtH-like protein or a functional fragment thereof. In some embodiments, the MbtH-like protein is FrsB or a homolog thereof.

In some embodiments, the polypeptide associated with the production of the compound is a malate dehydrogenase. In some embodiments, the malate dehydrogenase is FrsC or a homolog thereof.

In some embodiments, the polypeptide associated with the production of the compound is a hydroxylase. In some embodiments, hydroxylase is FrsH or a homolog thereof.

In some embodiments, the nucleic acid comprises a plurality of nucleotide sequences encoding a plurality of polypeptides associated with the production of the compound. In some embodiments, the plurality of polypeptides associated with the production of the compound comprises a plurality of NRPSs, or a functional fragment thereof. In some embodiments, the plurality of NRPSs comprise two or more (*e.g.,* three, four, or all) of FrsA, FrsD, FrsE, FrsF, or FrsG, or a homolog thereof. In some embodiments, the plurality of polypeptides associated with the production of the compound comprises an NRPS, a MbtH-like protein, a malate dehydrogenase, and a hydroxylase, or a functional fragment thereof.

In some embodiments, the plurality of polypeptides associated with the production of the compound comprises FrsA, FrsB, FrsC, FrsD, FrsE, FrsF, FrsG, and FrsH, or a homolog thereof.

In another aspect, the disclosure features a nucleic acid comprising a nucleotide sequence encoding a polypeptide associated with the production of one or more of: compound J, compound F5, compound F3, or compound D.

In some embodiments, the nucleic acid is an isolated nucleic acid. In some embodiments, the nucleic acid is a non-naturally occurring nucleic acid. In some embodiments, the nucleic acid is a synthetic nucleic acid. In some embodiments, the nucleic acid comprises a mutation, *e.g.*, a deletion.

In some embodiments, the nucleic acid comprises a plurality of nucleotide sequences encoding a plurality of polypeptides associated with the production of one or more of: compound J, compound F5, compound F3, or compound D.

In some embodiments, the nucleotide sequence is from a BGC. In some embodiments, the BGC is a compound J-BGC. In some embodiments, the compound J-BGC has the gene organization shown in **FIG. 1****.**

In some embodiments, the nucleotide sequence comprises one or more (*e.g.,* two, three, or all) of *dis1, dis2, dis3,* or *dis4,* or a homolog thereof. In some embodiments, the BGC comprises *dis1, dis2, dis3,* and *dis4,* or a homolog thereof.

In some embodiments, the nucleic acid comprises a nucleotide sequence associated GenBank accession number: BankIt2437961 BSeq#1 MW732719, or a functional fragment thereof, or a nucleotide sequence having at least about 75%, *e.g.,* at least about 80%, about 85%, about 90%, about 95%, about 96%, about 97%, about 98%, or about 99% identity thereto.

In another aspect, the disclosure features a vector comprising a nucleic acid described herein. In another aspect, the disclosure features a cell comprising a nucleic acid described herein or a vector described herein.

In another aspect, the disclosure features a method of engineering a cell, comprising altering a nucleotide sequence encoding a polypeptide associated with the production of one or more of: compound J, compound F5, compound F3, or compound D, thereby engineering the cell.

In some embodiments, the cell is a microorganism that naturally produces a compound having the structure of Formula (A1). In some embodiments, the microorganism is a bacterium, *e.g.*, *Chromobacterium.* In some embodiments, the microorganism is *Chromobacterium vaccinii, e.g., Chromobacterium vaccinii* DSM 25150 (= ATCC BAA-2314, = NRRL B-50840, = MWU205).

In some embodiments, the nucleotide sequence is disrupted, *e.g*., at least a portion of the nucleotide sequence is deleted.

In some embodiments, the nucleic acid comprises a plurality of nucleotide sequences encoding a plurality of polypeptides associated with the production of one or more of: compound J, compound F5, compound F3, or compound D. In some embodiments, the production of one or more (e.g., two, three, or all) of: compound J, compound F5, compound F3, or compound D is reduced, *e.g.,* by at least about 50%, *e.g.,* at least about 60%, about 70%, about 80%, about 90%, about 95%, about 96%, about 97%, about 98%, about 99%, or about 100%.

In some embodiments, the nucleotide sequence is from a BGC. In some embodiments, the BGC is a compound J-BGC. In some embodiments, the compound J-BGC has the gene organization shown in **FIG. 1**. In some embodiments, the nucleotide sequence comprises one or more (*e.g.,* two, three, or all) of *dis1, dis2, dis3,* or *dis4,* or a homolog thereof. In some embodiments, the BGC comprises *dis1, dis2, dis3,* and *dis4,* or a homolog thereof. In some embodiments, the nucleic acid comprises a nucleotide sequence associated GenBank accession number: BankIt2437961 BSeq#1 MW732719, or a functional fragment thereof, or a nucleotide sequence having at least about 75%, about 80%, about 85%, about 90%, about 95%, about 96%, about 97%, about 98%, or about 99% identity thereto.

In some embodiments, the alteration increases the isolation yield of the compound. In some embodiments, the isolation yield of the compound is increased by about 20%, about 30%, about 40%, about 50%, about 60%, about 70%, about 80%, about 90%, or more, compared to a reference microorganism, *e.g.*, an otherwise identical microorganism in which the BGC of the natural product that interferes with the isolation of the compound is not altered (*e.g.,* a wild-type).

### Processes for Partial Reoxidation and Making Antibody Drug Conjugates

In another aspect, the disclosure features a process for producing a partially reoxidized antibody or antigen binding fragment thereof, comprising: providing an antibody or antigen binding fragment thereof comprising one or more decapped cysteine residues; and storing the antibody or antigen binding fragment thereof comprising one or more decapped cysteine residues under oxidation conditions, thereby producing the partially reoxidized antibody or antigen binding fragment thereof.

In some embodiments, the process further comprises providing an antibody or antigen binding fragment thereof comprising one or more capped cysteine residues. In some embodiments, the one or more capped cysteine residues are located in the constant domain a heavy chain of the antibody or antigen binding fragment thereof. In some embodiments, the antibody or antigen binding fragment thereof comprises four capped cysteine residues. In some embodiments, the antibody or antigen binding fragment thereof comprises two capped cysteine residues. In some embodiments, the antibody or antigen binding fragment thereof comprises a capped cysteine residue located in a CH1 region of the antibody or antigen binding fragment thereof. In some embodiments, the antibody or antigen binding fragment thereof comprises a capped cysteine residue located in each CH1 region of the antibody or antigen binding fragment thereof. In some embodiments, the capped cysteine residue is an engineered surface-exposed cysteine residue. In some embodiments, the antibody or antigen binding fragment thereof comprises a capped cysteine residue located at residue 152 (EU numbering) in the CH1 region of the antibody or antigen binding fragment thereof. In some embodiments, the one or more capped cysteine residues are capped with a cysteine or a glutathione.

In some embodiments, the process further comprises reducing one or more capped cysteine residues to provide an antibody or antigen binding fragment thereof comprising one or more decapped cysteine residues. In some embodiments, the one or more decapped cysteine residues are located in the constant domain a heavy chain of the antibody or antigen binding fragment thereof. In some embodiments, the antibody or antigen binding fragment thereof comprises four decapped cysteine residues. In some embodiments, the antibody or antigen binding fragment thereof comprises two decapped cysteine residues. In some embodiments, the antibody or antigen binding fragment thereof comprises a decapped cysteine residue located in a CH1 region of the antibody or antigen binding fragment thereof. In some embodiments, the antibody or antigen binding fragment thereof comprises a decapped cysteine residue located in each CH1 region of the antibody or antigen binding fragment thereof. In some embodiments, the decapped cysteine residue is an engineered surface-exposed cysteine residue. In some embodiments, the antibody or antigen binding fragment thereof comprises a decapped cysteine residue located at residue 152 (EU numbering) in the CH1 region of the antibody or antigen binding fragment thereof.

In some embodiments, the process comprises reducing the one or more capped cysteine residues by contacting the antibody or antigen binding fragment thereof comprising one or more capped cysteine residues with a reduction wash buffer, thereby providing an antibody or antigen binding fragment comprising one or more decapped cysteine residue.

In some embodiments, the reduction wash buffer comprises about 5 mM to about 50 mM cysteine, *e.g.*, about 10 mM to about 40 mM, about 20 mM to about 30 mM, about 5 mM to about 15 mM, about 5 mM to about 25 mM, about 5 mM to about 35 mM, about 5 mM to about 45 mM, about 40 mM to about 50 mM, about 30 mM to about 50 mM, about 20 mM to about 50 mM, about 10 mM to about 50 mM, about 10 mM to about 20 mM, about 15 mM to about 25 mM, about 25 mM to about 35 mM, about 30 mM to about 40 mM, or about 35 mM to about 45 mM, *e.g.,* about 10 mM, about 12 mM, about 14 mM, about 16 mM, about 20 mM, about 25 mM, or about 30 mM. In some embodiments, the reduction wash buffer comprises about 5 mM to about 15 mM cysteine, *e.g.*, about 10 mM cysteine.

In some embodiments, the reduction wash buffer has a pH of about 6.0 to about 7.5, *e.g.,* about 6.5 to about 7.2 or about 6.8 to about 7.0. In some embodiments, the reduction wash buffer has a pH of about 6.8 to about 7.0, *e.g.*, about 6.9.

In some embodiments, contacting the antibody or antigen binding fragment thereof comprising one or more capped cysteine residues with the reduction wash buffer is performed on a column (*e.g.,* a cation exchange chromatography column).

In some embodiments, the process further comprises collecting the antibody or antigen binding fragment thereof comprising one or more decapped cysteine residues in an eluate after contacting with the reduction wash buffer. In some embodiments, the eluate has a pH of about 5.5 to about 6.0, *e.g.,* about 5.8. In some embodiments, the concentration of the antibody or antigen binding fragment thereof comprising one or more decapped cysteine residues in the eluate is about 5 g/L to 25 g/L, *e.g.*, about 8 g/L to about 20 g/L, about 10 g/L to about 18 g/L, about 12 g/L to about 15 g/L, about 5 g/L to about 20 g/L, about 5 g/L to about 15 g/L, about 5 g/L to about 10 g/L, about 20 g/L to about 25 g/L, about 15 g/L to about 25 g/L, about 10 g/L to about 25 g/L, about 10 g/L to about 20 g/L, about 13 g/L to about 14 g/L, about 12 g/L to about 15 g/L, *e.g.*, about 5 g/L, about 6 g/L, about 7 g/L, about 8 g/L, about 9 g/L, about 10 g/L, about 11 g/L, about 12 g/L, about 13 g/L, about 13.5 g/L, about 14 g/L, about 15 g/L, about 16 g/L, about 17 g/L, about 18 g/L, about 19 g/L, about 20 g/L, about 21 g/L, about 22 g/L, about 23 g/L, about 24 g/L, or about 25 g/L.

In some embodiments, the antibody or antigen binding fragment thereof comprising one or more decapped cysteine residues is stored in the eluate with stirring. In some embodiments, the antibody or antigen binding fragment thereof comprising one or more decapped cysteine residues is stored in the eluate without stirring.

In some embodiments, the antibody or antigen binding fragment thereof comprising one or more decapped cysteine residues is stored in a container filled to a maximum of about 50% to about 70% volume, *e.g.,* for about 12 hours to about 96 hours, *e.g.,* about 12 hours to about 84 hours, about 24 hours to about 84 hours, about 36 hours to about 72 hours, about 48 hours to about 60 hours, about 24 hours to about 72 hours, about 24 hours to about 60 hours, about 24 hours to about 48 hours, about 24 hours to about 36 hours, about 72 hours to about 84 hours, about 60 hours to about 84 hours, about 48 hours to about 84 hours, about 36 hours to about 84 hours, about 12 hours to about 36 hours, about 24 hours to about 48 hours, about 36 hours to about 60 hours, about 48 hours to about 72 hours, or about 72 hours to about 96 hours, *e.g.*, about 12 hours, about 24 hours, about 36 hours, about 48 hours, about 60 hours, about 72 hours, about 84 hours, or about 96 hours. In some embodiments, the antibody or antigen binding fragment thereof comprising one or more decapped cysteine residues is stored in the container filled to a maximum of about 60%. In some embodiments, the antibody or antigen binding fragment thereof comprising one or more decapped cysteine residues is stored for about 48 hours to about 72 hours, *e.g.*, without stirring. In some embodiments, the antibody or antigen binding fragment thereof comprising one or more decapped cysteine residues is stored for about 12 hours to about 36 hours, *e.g.,* about 24 hours, *e.g.,* with stirring.

In some embodiments, the antibody or antigen binding fragment thereof comprising one or more decapped cysteine residues is stored at room temperature. In some embodiments, the antibody or antigen binding fragment thereof comprising one or more decapped cysteine residues is stored at about 2 °C to about 8 °C (*e.g.* about 4 °C), *e.g.,* for at least 48 hours (*e.g.,* at least about 48 hours to about 96 hours).

In some embodiments, the antibody or antigen binding fragment thereof comprising one or more decapped cysteine residues is stored with air overlay.

In some embodiments, the process further comprises purifying the partially reoxidized antibody or antigen binding fragment thereof.

In some embodiments, the process further comprises conjugating a linker-drug moiety *(e.g.,* a linker-drug moiety described herein) to the partially reoxidized antibody or antigen binding fragment thereof to produce an antibody drug conjugate (*e.g.,* an antibody drug conjugate described herein).

In some embodiments, the process further comprises pre-forming a linker-drug moiety of the following Formula (B):

R⁸-L_{B}-(D)ₙ (B)

wherein:
D is a GNAQ inhibitor, a GNA11 inhibitor or an inhibitor of GNAQ and GNA11;
R⁸ is a reactive group;
L_{B} is a cleavable or non-cleavable linker, and
n is 1, 2, 3 or 4;

In some embodiments, D is a GNAQ inhibitor described herein. In some embodiments, D is a GNA11 inhibitor described herein. In some embodiments, D is an inhibitor of GNAQ and GNA11 as described herein. In some embodiments, R is a reactive group described herein. In some embodiments, L_{B} is a cleavable linker described herein. In some embodiments, L_{B} is a non-cleavable linker described herein.

In some embodiments, the antibody or antigen binding fragment thereof binds to PMEL17. In some embodiments, the antibody or antigen binding fragment thereof is an antibody or antigen binding fragment thereof described herein.

In some embodiments, the process further comprises purifying the antibody drug conjugate.

In some embodiments, the process results in at least about 75%, *e.g.,* at least about 80%, about 85%, about 90%, or about 95%, on-site coupling, *e.g.*, one linker-drug moiety per heavy chain ("HC1"). In some embodiments, the process results in less than about 25%, *e.g.,* at least about 20%, about 15%, about 10%, or about 5%, off-site coupling, *e.g.*, one linker-drug moiety per light chain ("LC1") and/or two linker-drug moieties per heavy chain ("HC2"). In some embodiments, the process results in at least about 70%, *e.g.,* at least about 75%, about 80%, about 85%, about 90%, or about 95%, purity, *e.g.*, as determined by non-reduced CE-SDS.

In another aspect, the disclosure features a process for producing an anti-PMEL17 antibody drug conjugate, comprising:
providing an antibody or antigen binding fragment thereof comprising one or more capped cysteine residues with a reduction wash buffer, thereby providing an antibody or antigen binding fragment comprising one or more decapped cysteine residues;
storing the antibody or fragment thereof comprising one or more decapped cysteine residues under oxidation conditions, thereby producing a partially reoxidized antibody or antigen binding fragment thereof; and
conjugating a linker-drug moiety to the partially reoxidized antibody or antigen binding fragment thereof, thereby producing the anti-PMEL17 antibody drug conjugate,
wherein the antibody or antigen binding fragment thereof binds to PMEL17; and
wherein the linker-drug moiety of the following Formula (B):

   R⁸-L_{B}-(D)ₙ (B)

   wherein:
   D is a GNAQ inhibitor, a GNA11 inhibitor or an inhibitor of GNAQ and GNA11;
   R⁸ is a reactive group;
   L_{B} is a cleavable or non-cleavable linker, and
   n is 1, 2, 3 or 4.

### Formulations of Antibody Drug Conjugates

In another aspect, the disclosure features a formulation comprising an antibody drug conjugate, a buffering agent, a stabilizing agent, and a surfactant, wherein the formulation has a pH of 4.5 to 6.5, wherein the antibody drug conjugate comprises the formula (C)

Ab-(L_{A}-(D)ₙ)_{y} (C)

wherein:
D is a GNAQ inhibitor, a GNA11 inhibitor or an inhibitor of GNAQ and GNA11;
Ab is an antibody or antigen binding fragment thereof that binds to human PMEL17 protein;
L_{A} is a linker;
n is 1, 2, 3 or 4, and
y is 1, 2, 3 or 4.

In an embodiment, the D is a GNAQ inhibitor described herein, *e.g.,* a GNA11 inhibitor described herein or an inhibitor of GNAQ and GNA11 described herein.

In an embodiment, the antibody drug conjugate is present at a concentration of about 5 mg/mL to about 30 mg/mL, *e.g.*, about 10 mg to about 30 mg, about 15 mg/mL to about 25 mg/mL, about 18 mg/mL to about 22 mg/mL, about 10 mg/mL to 25 mg/mL, about 10 mg/mL to about 20 mg/mL, about 10 mg/mL to about 15 mg/mL, about 25 mg to about 30 mg/mL, about 20 mg/mL to about 30 mg/mL, about 5 mg/mL to about 15 mg/mL, about 15 mg/mL to about 25 mg/mL, or about 18 mg/mL to about 22 mg/mL, *e.g.*, about 5 mg/mL, about 6 mg/mL, about 7 mg/mL, about 8 mg/mL, about 9 mg/mL, about 10 mg/mL, about 11 mg/mL, about 12 mg/mL, about 13 mg/mL, about 14 mg/mL, about 15 mg/mL, about 16 mg/mL, about 17 mg/mL, about 18 mg/mL, about 19 mg/mL, about 20 mg/mL, about 21 mg/mL, about 22 mg/mL, about 23 mg/mL, about 24 mg/mL, or about 25 mg/mL.

In an embodiment, the antibody drug conjugate is present at a concentration of about 15 mg/mL to about 25 mg/mL, *e.g.*, about 20 mg/mL.

In an embodiment, the buffering agent comprises histidine. In an embodiment, the buffering agent comprises succinate. In an embodiment, the buffering agent is present at a concentration of about 5 mM to about 50 mM, *e.g.*, about 10 mM to about 40 nM, about 15 mM to about 35 mM, about 20 mM to about 30 mM, about 5 mM to about 40 mM, about 5 mM to about 30 mM, about 5 mM to about 20 mM, about 5 mM to about 15 mM, about 5 mM to about 10 mM, about 40 mM to about 50 mM, about 35 mM to about 50 mM, about 30 mM to about 50 mM, about 25 mM to about 50 mM, about 20 mM to about 50 mM, about 15 mM to about 50 mM, about 10 mM to about 50 mM, about 10 mM to about 20 mM, about 15 mM to about 25 mM, about 25 mM to about 35 mM, about 30 mM to about 40 mM, or about 35 mM to about 45 mM, *e.g.,* about 5 mM, about 10 mM, about 15 mM, about 20 mM, about 25 mM, about 30 mM, about 35 mM, about 40 mM, about 45 mM, or about 50 mM. In an embodiment, the buffering agent is present at a concentration of about 15 mM to about 25 mM, *e.g.*, about 20 mM.

In an embodiment, the stabilizing agent comprises sucrose. In an embodiment, the stabilizing agent comprises trehalose. In an embodiment, the stabilizing agent is present at a concentration of about 100 mM to about 500 mM, *e*.*g.*, about 150 mM to about 450 mM, about 200 mM to about 400 mM, about 250 mM to about 350 mM, about 100 mM to about 450 mM, about 100 mM to about 400 mM, about 100 mM to about 350 mM, about 100 mM to about 300 mM, about 100 mM to about 250 mM, about 100 mM to about 200 mM, about 100 mM to about 150 mM, about 450 mM to about 500 mM, about 400 mM to about 500 mM, about 350 mM to about 500 mM, about 300 mM to about 500 mM, about 250 mM to about 500 mM, about 200 mM to about 500 mM, about 150 mM to about 500 mM, about 150 mM to about 250 mM, about 200 mM to about 250 mM, about 200 mM to about 300 mM, about 300 mM to about 400 mM, or about 350 mM to about 450 mM, *e.g.*, about 100 mM, about 150 mM, about 200 mM, about 240 mM, about 250 mM, about 300 mM, about 350 mM, about 400 mM, about 450 mM, or about 500 mM. In an embodiment, the stabilizing agent and is present at a concentration of about 200 mM to about 300 mM, *e.g.*, about 240 mM.

In an embodiment, the surfactant comprises polysorbate 20. In an embodiment, the surfactant comprises polysorbate 80. In an embodiment, the surfactant is present at a concentration of about 0.01% to about 0.06%, *e.g.,* about 0.02% to about 0.05%, about 0.03% to about 0.04%, about 0.01% to about 0.05%, about 0.01% to about 0.04%, about 0.01% to about 0.03%, about 0.01% to about 0.02%, about 0.05% to about 0.06%, about 0.04% to about 0.06%, about 0.03% to about 0.06%, about 0.02% to about 0.06%, about 0.02% to about 0.04%, about 0.03% to about 0.05%, *e.g.,* about 0.01%, about 0.02%, about 0.03%, about 0.04%, about 0.05%, or about 0.06%. In an embodiment, the surfactant is present at a concentration of about 0.01% to about 0.03%, *e.g.,* about 0.02%.

In an embodiment, the formulation has a pH of about 4.7 to about 5.3, about 5.0 to about 6.0, about 5.2 to about 5.8, about 5.4 to about 5.6, about 5.2 to about 6.0, about 5.4 to about 6.0, about 5.6 to about 6.0, about 5.8 to about 6.0, about 5 to about 5.8, about 5 to about 5.6.0, about 5 to about 5.4, about 5 to about 5.2, about 4.8 to about 5.2, about 5.1 to about 5.3, about 5.2 to about 5.4, about 5.3 to about 5.5, about 5.5 to about 5.7, about 5.6 to about 5.8, about 5.7 to about 5.9, about 4.9 to about 5.5, about 5.5 to about 6.1, or about 5.7 to about 6.3, *e.g.,* about 4.5, about 4.6, about 4.7, about 4.8, about 4.9, about 5, about 5.1, about 5.2, about 5.3, about 5.4, about 5.5, about 5.6, about 5.7, about 5.8, about 5.9, about 6.0, about 6.1, about 6.2, about 6.3, about 6.4, or about 6.5. In an embodiment, the formulation has a pH of about 5.0 to about 5.6, *e.g.,* about 5.3.

In an embodiment, the formulation comprises about 15 mg/mL to about 25 mg/mL of the antibody drug conjugate, about 15 mM to about 25 mM of a buffering agent comprising histidine or succinate, about 200 mM to about 300 mM of a stabilizing agent comprising sucrose or trehalose, and about 0.01% to about 0.03% of a surfactant comprising polysorbate 20 or polysorbate 80, wherein the formulation has a pH of about 5.0 to about 5.6.

In an embodiment, the formulation comprises about 15 mg/mL to about 25 mg/mL of the antibody drug conjugate, about 15 mM to about 25 mM of a buffering agent comprising histidine, about 200 mM to about 300 mM of a stabilizing agent comprising sucrose, and about 0.01% to about 0.03% of a surfactant comprising polysorbate 20, wherein the formulation has a pH of about 5.0 to about 5.6.

In an embodiment, the formulation comprises about 20 mg/mL of the antibody drug conjugate, about 20 mM of a buffering agent comprising histidine or succinate, about 240 mM of a stabilizing agent comprising sucrose or trehalose, and about 0.02% of a surfactant comprising polysorbate 20 or polysorbate 80, wherein the formulation has a pH of about 5.3.

In an embodiment, the formulation comprises about 20 mg/mL of the antibody drug conjugate, about 20 mM of a buffering agent comprising histidine, about 240 mM of a stabilizing agent comprising sucrose, and about 0.02% of a surfactant comprising polysorbate 20, wherein the formulation has a pH of about 5.3.

In an embodiment, the formulation comprises about 15 mg/mL to about 25 mg/mL of the antibody drug conjugate, about 15 mM to about 25 mM of a buffering agent comprising histidine or succinate, about 200 mM to about 300 mM of a stabilizing agent comprising sucrose or trehalose, and about 0.01% to about 0.03% of a surfactant comprising polysorbate 20 or polysorbate 80, wherein the formulation has a pH of about 4.7 to about 5.3.

In an embodiment, the formulation comprises about 15 mg/mL to about 25 mg/mL of the antibody drug conjugate, about 15 mM to about 25 mM of a buffering agent comprising histidine, about 200 mM to about 300 mM of a stabilizing agent comprising sucrose, and about 0.01% to about 0.03% of a surfactant comprising polysorbate 20, wherein the formulation has a pH of about 4.7 to about 5.3.

In an embodiment, the formulation comprises about 20 mg/mL of the antibody drug conjugate, about 20 mM of a buffering agent comprising histidine or succinate, about 240 mM of a stabilizing agent comprising sucrose or trehalose, and about 0.02% of a surfactant comprising polysorbate 20 or polysorbate 80, wherein the formulation has a pH of about 5.0.

In an embodiment, the formulation comprises about 20 mg/mL of the antibody drug conjugate, about 20 mM of a buffering agent comprising histidine, about 240 mM of a stabilizing agent comprising sucrose, and about 0.02% of a surfactant comprising polysorbate 20, wherein the formulation has a pH of about 5.0.

In an embodiment, the formulation comprises about 15 mg/mL to about 25 mg/mL of the antibody drug conjugate, about 15 mM to about 25 mM of a buffering agent comprising histidine or succinate, about 200 mM to about 300 mM of a stabilizing agent comprising sucrose or trehalose, and about 0.01% to about 0.03% of a surfactant comprising polysorbate 20 or polysorbate 80, wherein the formulation has a pH of about 5.2 to about 5.8.

In an embodiment, the formulation comprises about 15 mg/mL to about 25 mg/mL of the antibody drug conjugate, about 15 mM to about 25 mM of a buffering agent comprising histidine, about 200 mM to about 300 mM of a stabilizing agent comprising sucrose, and about 0.01% to about 0.03% of a surfactant comprising polysorbate 20, wherein the formulation has a pH of about 5.2 to about 5.8.

In an embodiment, the formulation comprises about 20 mg/mL of the antibody drug conjugate, about 20 mM of a buffering agent comprising histidine or succinate, about 240 mM of a stabilizing agent comprising sucrose or trehalose, and about 0.02% of a surfactant comprising polysorbate 20 or polysorbate 80, wherein the formulation has a pH of about 5.5.

In an embodiment, the formulation comprises about 20 mg/mL of the antibody drug conjugate, about 20 mM of a buffering agent comprising histidine, about 240 mM of a stabilizing agent comprising sucrose, and about 0.02% of a surfactant comprising polysorbate 20, wherein the formulation has a pH of about 5.5.

In an embodiment, the formulation comprises about 15 mg/mL to about 25 mg/mL of the antibody drug conjugate, about 15 mM to about 25 mM of a buffering agent comprising histidine or succinate, about 200 mM to about 300 mM of a stabilizing agent comprising sucrose or trehalose, and about 0.03% to about 0.05% of a surfactant comprising polysorbate 20 or polysorbate 80, wherein the formulation has a pH of about 5.2 to about 5.8.

In an embodiment, the formulation comprises about 15 mg/mL to about 25 mg/mL of the antibody drug conjugate, about 15 mM to about 25 mM of a buffering agent comprising histidine, about 200 mM to about 300 mM of a stabilizing agent comprising sucrose, and about 0.03% to about 0.05% of a surfactant comprising polysorbate 20, wherein the formulation has a pH of about 5.2 to about 5.8.

In an embodiment, the formulation comprises about 20 mg/mL of the antibody drug conjugate, about 20 mM of a buffering agent comprising histidine or succinate, about 240 mM of a stabilizing agent comprising sucrose or trehalose, and about 0.04% of a surfactant comprising polysorbate 20 or polysorbate 80, wherein the formulation has a pH of about 5.5.

In an embodiment, the formulation comprises about 20 mg/mL of the antibody drug conjugate, about 20 mM of a buffering agent comprising histidine, about 240 mM of a stabilizing agent comprising sucrose, and about 0.04% of a surfactant comprising polysorbate 20, wherein the formulation has a pH of about 5.5.

In an embodiment, the formulation comprises about 5 mg/mL to about 15 mg/mL of the antibody drug conjugate, about 15 mM to about 25 mM of a buffering agent comprising histidine or succinate, about 200 mM to about 300 mM of a stabilizing agent comprising sucrose or trehalose, and about 0.03% to about 0.05% of a surfactant comprising polysorbate 20 or polysorbate 80, wherein the formulation has a pH of about 5.2 to about 5.8.

In an embodiment, the formulation comprises about 5 mg/mL to about 15 mg/mL of the antibody drug conjugate, about 15 mM to about 25 mM of a buffering agent comprising histidine, about 200 mM to about 300 mM of a stabilizing agent comprising sucrose, and about 0.03% to about 0.05% of a surfactant comprising polysorbate 20, wherein the formulation has a pH of about 5.2 to about 5.8.

In an embodiment, the formulation comprises about 10 mg/mL of the antibody drug conjugate, about 20 mM of a buffering agent comprising histidine or succinate, about 240 mM of a stabilizing agent comprising sucrose or trehalose, and about 0.02% of a surfactant comprising polysorbate 20 or polysorbate 80, wherein the formulation has a pH of about 5.5.

In an embodiment, the formulation comprises about 10 mg/mL of the antibody drug conjugate, about 20 mM of a buffering agent comprising histidine, about 240 mM of a stabilizing agent comprising sucrose, and about 0.02% of a surfactant comprising polysorbate 20, wherein the formulation has a pH of about 5.5.

In an embodiment, the formulation comprises about 15 mg/mL to about 25 mg/mL of the antibody drug conjugate, about 15 mM to about 25 mM of a buffering agent comprising histidine or succinate, about 200 mM to about 300 mM of a stabilizing agent comprising sucrose or trehalose, and about 0.01% to about 0.03% of a surfactant comprising polysorbate 20 or polysorbate 80, wherein the formulation has a pH of about 5.7 to about 6.3.

In an embodiment, the formulation comprises about 15 mg/mL to about 25 mg/mL of the antibody drug conjugate, about 15 mM to about 25 mM of a buffering agent comprising histidine, about 200 mM to about 300 mM of a stabilizing agent comprising sucrose, and about 0.01% to about 0.03% of a surfactant comprising polysorbate 20, wherein the formulation has a pH of about 5.7 to about 6.3.

In an embodiment, the formulation comprises about 20 mg/mL of the antibody drug conjugate, about 20 mM of a buffering agent comprising histidine or succinate, about 240 mM of a stabilizing agent comprising sucrose or trehalose, and about 0.02% of a surfactant comprising polysorbate 20 or polysorbate 80, wherein the formulation has a pH of about 6.0.

In an embodiment, the formulation comprises about 20 mg/mL of the antibody drug conjugate, about 20 mM of a buffering agent comprising histidine, about 240 mM of a stabilizing agent comprising sucrose, and about 0.02% of a surfactant comprising polysorbate 20, wherein the formulation has a pH of about 6.0.

In an embodiment, the formulation comprises about 15 mg/mL to about 25 mg/mL of the antibody drug conjugate, about 15 mM to about 25 mM of a buffering agent comprising histidine or succinate, about 200 mM to about 300 mM of a stabilizing agent comprising sucrose or trehalose, and about 0.01% to about 0.03% of a surfactant comprising polysorbate 20 or polysorbate, wherein the formulation has a pH of about 4.9 to about 5.5.

In an embodiment, the formulation comprises about 15 mg/mL to about 25 mg/mL of the antibody drug conjugate, about 15 mM to about 25 mM of a buffering agent comprising histidine, about 200 mM to about 300 mM of a stabilizing agent comprising sucrose, and about 0.01% to about 0.03% of a surfactant comprising polysorbate 20, wherein the formulation has a pH of about 4.9 to about 5.5.

In an embodiment, the formulation comprises about 20 mg/mL of the antibody drug conjugate, about 20 mM of a buffering agent comprising histidine or succinate, about 240 mM of a stabilizing agent comprising sucrose or trehalose, and about 0.02% of a surfactant comprising polysorbate 20 or polysorbate 80, wherein the formulation has a pH of about 5.2.

In an embodiment, the formulation comprises about 20 mg/mL of the antibody drug conjugate, about 20 mM of a buffering agent comprising histidine, about 240 mM of a stabilizing agent comprising sucrose, and about 0.02% of a surfactant comprising polysorbate 20, wherein the formulation has a pH of about 5.2.

In an embodiment, the formulation comprises about 15 mg/mL to about 25 mg/mL of the antibody drug conjugate, about 15 mM to about 25 mM of a buffering agent comprising histidine or succinate, about 200 mM to about 300 mM of a stabilizing agent comprising sucrose or trehalose, and about 0.01% to about 0.03% of a surfactant comprising polysorbate 20 or polysorbate 80, wherein the formulation has a pH of about 5.5 to about 6.1.

In an embodiment, the formulation comprises about 15 mg/mL to about 25 mg/mL of the antibody drug conjugate, about 15 mM to about 25 mM of a buffering agent comprising histidine, about 200 mM to about 300 mM of a stabilizing agent comprising sucrose, and about 0.01% to about 0.03% of a surfactant comprising polysorbate 20, wherein the formulation has a pH of about 5.5 to about 6.1.

In an embodiment, the formulation comprises about 20 mg/mL of the antibody drug conjugate, about 20 mM of a buffering agent comprising histidine or succinate, about 240 mM of a stabilizing agent comprising sucrose or trehalose, and about 0.02% of a surfactant comprising polysorbate 20 or polysorbate 80, wherein the formulation has a pH of about 5.8.

In an embodiment, the formulation comprises about 20 mg/mL of the antibody drug conjugate, about 20 mM of a buffering agent comprising histidine, about 240 mM of a stabilizing agent comprising sucrose, and about 0.02% of a surfactant comprising polysorbate 20, wherein the formulation has a pH of about 5.8.

In another aspect, the disclosure features a lyophilized formulation, which is lyophilized from a formulation described herein.

In an embodiment, about 5 mL to about 5.5 mL of the formulation is lyophilized.

In an embodiment, the D in the lyophilized formulation is at least 1, 2, 3, 4, 5, 6, 7, 8, 9, or 10-fold more stable, compared to an otherwise identical formulation that is not lyophilized, after storage for about 0, about 4, or about 8 weeks, at about 5°C, 25°C, or 40°C, *e.g.,* as determined by CZE. In an embodiment, the percentage of D that has a ring-opening conformation in the lyophilized formulation is at least 1, 2, 3, 4, 5, 6, 7, 8, 9, or 10-fold lower, compared to an otherwise identical formulation that is not lyophilized, after storage for about 0, about 4, or about 8 weeks, at about 5°C, 25°C, or 40°C, *e.g.,* as determined by CZE.

In an embodiment, the lyophilized formulation comprises about 80 mg to about 120 mg (e.g., about 107 mg) of the antibody drug conjugate.

In an embodiment, the level of monomers in the lyophilized formulation is at least about 95%, *e.g.,* at least about 96%, 97%, 98%, or 99%, after storage for about 0, about 4, or about 8 weeks, at about 5°C, 25°C, or 40°C, *e.g.,* as determined by SEC.

In an embodiment, the level of fragments in the lyophilized formulation is less than about 3%, *e.g.,* less than about 2.5%, 2%, 1.5%, 1%, or 0.5%, after storage for about 0, about 4, or about 8 weeks, at about 5°C, 25°C, or 40°C, *e.g.,* as determined by SEC.

In an embodiment, the level of aggregates in the lyophilized formulation is less than about 3%, *e.g.,* less than about 2.5%, 2%, 1.5%, 1%, or 0.5%, after storage for about 0, about 4, or about 8 weeks, at about 5°C, 25°C, or 40°C, *e.g.,* as determined by SEC.

In an embodiment, the level of degradation in the lyophilized formulation is less than about 2%, *e.g.,* less than about 1.5%, 1%, or 0.5%, after storage for about 4 weeks or about 8 weeks, at about 5°C, 25°C, or 40°C, *e.g.,* as determined by SEC.

In an embodiment, the level of particles greater than or equal to 10 µm in the lyophilized formulation is less than about 300 particles/ml, *e.g.*, less than about 280 particles/mL, less than about 260 particles/mL, less than about 240 particles/mL, less than about 220 particles/mL, less than about 200 particles/mL, less than about 180 particles/mL, less than about 160 particles/mL, less than about 140 particles/mL, 120 particles/mL, less than about 100 particles/mL, 80 particles/mL, 60 particles/mL, 40 particles/mL, 20 particles/mL, or 10 particles/mL, after storage for about 0, about 4, or about 8 weeks, at about 5°C, 25°C, or 40°C, *e.g.,* as determined by light obscuration.

In an embodiment, the level of particles greater than 10 µm in the lyophilized formulation is increased by no more than about 3-fold, *e.g.,* no more than about 2.5, 2, 1.5, 1, or 0.5-fold, after storage for about 4 or about 8 weeks, at about 5°C, 25°C, or 40°C, *e.g.,* as determined by light obscuration.

In an embodiment, the level of particles greater than 25 µm in the lyophilized formulation is increased by no more than about 3-fold, *e.g.,* no more than about 2.5, 2, 1.5, 1, or 0.5-fold, after storage for about 4 or about 8 weeks, at about 5°C, 25°C, or 40°C, *e.g.,* as determined by light obscuration.

In an embodiment, the level of particles greater than 25 µm in the lyophilized formulation is less than about 20 particles/mL, *e.g.*, less than about 15 particles/mL, 10 particles/mL, 5 particles/mL, or 2 particles/mL, after storage for about 0, about 4, or about 8 weeks, at about 5°C, 25°C, or 40°C, *e.g.,* as determined by light obscuration.

In an embodiment, the level of impurity in the lyophilized formulation is less than about 15%, *e.g.,* less than about 14%, 13%, 12%, 11%, 10%, 9%, 8%, 7%, 6%, 5%, 4%, 3%, 2%, or 1%, after storage for about 0, about 4, or about 8 weeks, at about 5°C, 25°C, or 40°C, *e.g.,* as determined by capillary electrophoresis.

In an embodiment, the level of impurity in the lyophilized formulation is increased by no more than 20%, *e.g.,* no more than about 15%, 10%, 5%, 2%, or 1%, after storage for about 4 weeks or about 8 weeks, at about 5°C, 25°C, or 40°C, *e.g.,* as determined by capillary electrophoresis.

In an embodiment, the level of neutral variants in the lyophilized formulation is greater than about 50%, *e.g.,* greater than about 55%, 60%, 65%, 70%, 75%, 80%, 85%, or 90%, after storage for about 0, about 4, or about 8 weeks, at about 5°C, 25°C, or 40°C, *e.g.,* as determined by capillary zone electrophoresis (CZE).

In an embodiment, the level of neutral variants in the lyophilized formulation is decreased by no more than about 20%, *e.g.,* no more than about 15%, 10%, 5%, or 2%, after storage for about 4 weeks or about 8 weeks, at about 5°C, 25°C, or 40°C, *e.g.,* as determined by CZE.

In an embodiment, the level of acidic variants in the lyophilized formulation is less than about 30%, *e.g.,* less than about 25%, 20%, 15%, 5%, or 2%, after storage for about 0, about 4, or about 8 weeks, at about 5°C, 25°C, or 40°C, *e.g.,* as determined by CZE.

In an embodiment, the level of acid variants in the lyophilized formulation is increased by no more than about 50%, *e.g.,* no more than about 40%, 30%, 20%, 10%, or 5%, after storage for about 4 weeks or about 8 weeks, at about 5°C, 25°C, or 40°C, *e.g.,* as determined by CZE.

In an embodiment, the level of basic variants in the lyophilized formulation is less than about 30%, *e.g.,* less than about 25%, 20%, 15%, 5%, or 2%, after storage for about 0, about 4, or about 8 weeks, at about 5°C, 25°C, or 40°C, *e.g.,* as determined by CZE.

In an embodiment, the level of basic variants in the lyophilized formulation is increased by no more than about 50%, *e.g.,* no more than about 40%, 30%, 20%, 10%, or 5%, after storage for about 4 weeks or about 8 weeks, at about 5°C, 25°C, or 40°C, *e.g.,* as determined by CZE.

In an embodiment, the potency of the lyophilized formulation is decreased by no more than about 25%, *e.g.,* no more than about 20%, 15%, 10%, 5%, or 2%, after storage for about 8 weeks, at about 5°C, 25°C, or 40°C, *e.g.,* as determined by a bioactivity assay.

In an embodiment, the osmolality of the lyophilized formulation is about 200 mOsm/L to 400 mOsm/L, *e.g.*, 200 mOsm/L to 300 mOsm/L, 250 mOsm/L to 350 mOsm/L, 270 mOsm/L to 330 mOsm/L, 290 mOsm/L to 310 mOsm/L, 270 mOsm/L to 350 mOsm/L, 290 mOsm/L to 350 mOsm/L, 310 mOsm/L to 350 mOsm/L, 330 mOsm/L to 350 mOsm/L, 250 mOsm/L to 330 mOsm/L, 250 mOsm/L to 310 mOsm/L, 250 mOsm/L to 290 mOsm/L, 250 mOsm/L to 270 mOsm/L, 260 mOsm/L to 280 mOsm/L, 270 mOsm/L to 290 mOsm/L, 280 mOsm/L to 300 mOsm/L, 300 mOsm/L to 320 mOsm/L, 310 mOsm/L to 330 mOsm/L, or 320 mOsm/L to 340 mOsm/L, *e.g.*, 200 mOsm/L, 250 mOsm/L, 260 mOsm/L, 270 mOsm/L, 280 mOsm/L, 290 mOsm/L, 300 mOsm/L, 310 mOsm/L, 320 mOsm/L, 330 mOsm/L, 340 mOsm/L, 350 mOsm/L, or 400 mOsm/L.

In another aspect, the disclosure features a liquid formulation, which is reconstituted from a lyophilized formulation described herein.

In another aspect, the disclosure features a container comprising a lyophilized formulation described herein.

In an embodiment, the container is a vial, *e.g.,* a 25 R glass vial. In an embodiment, the container further comprises a stopper and a cap (e.g., a flip-off aluminum cap).

### Methods of Treatment

In another aspect, the disclosure features a method of treating a cancer, comprising administering to a subject in need thereof an antibody drug conjugate (ADC) described herein, or a formulation described herein, at a dose of 1 mg/kg to 16 mg/kg, thereby treating the cancer.

In an embodiment, the ADC is administered at a dose of 2 mg/kg to 15 mg/kg. In an embodiment, the ADC is administered at a dose of 1 mg/kg, 2 mg/kg, 4 mg/kg, 8 mg/kg, 12 mg/kg, or 16 mg/kg. In an embodiment, the ADC is administered once a week, once every two weeks, or once every four weeks. In an embodiment, the ADC is administered once every two weeks. In an embodiment, the ADC is administered intravenously.

In an embodiment, the subject has been treated with tebentafusp prior to the administration of the ADC. In an embodiment, the subject has not been treated with tebentafusp prior to the administration of the ADC.

In an embodiment, the cancer expresses PMEL17, contains a mutation of the GNAQ or GNA11 gene, or the melanoma expresses PMEL17 and contains a mutation of GNAQ, GNA11, or both. In an embodiment, the cancer is a melanoma. In an embodiment, the cancer is a malignant melanoma, uveal melanoma, ocular melanoma, an eye cancer, an eye neoplasm, a cutaneous melanoma, or mucosal melanoma.

In an embodiment, the ADC has the following structure: 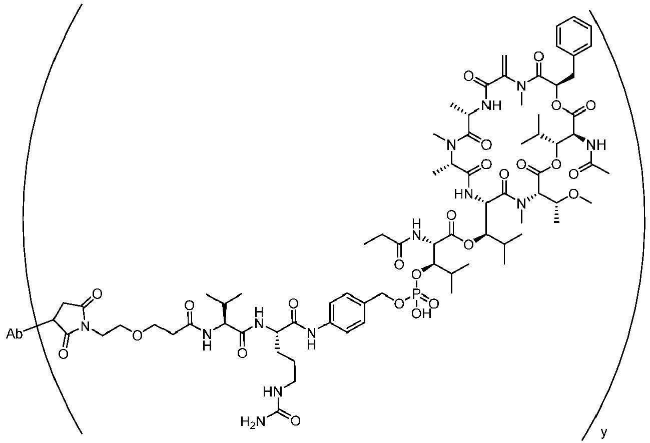 wherein:
Ab is an antibody or antigen binding fragment thereof that binds to human PMEL17 protein and y is 1, 2, 3, or 4. In an embodiment, y is 2.

In an embodiment, the Ab comprises:
(a) a heavy chain CDR1 of SEQ ID NO: 1, a heavy chain CDR2 of SEQ ID NO: 2, a heavy chain CDR3 of SEQ ID NO: 3, a light chain CDR1 of SEQ ID NO: 14, a light chain CDR2 of SEQ ID NO: 15, and a light chain CDR3 of SEQ ID NO: 16;
(b) a heavy chain CDR1 of SEQ ID NO: 4, a heavy chain CDR2 of SEQ ID NO: 2, a heavy chain CDR3 of SEQ ID NO: 3, a light chain CDR1 of SEQ ID NO: 14, a light chain CDR2 of SEQ ID NO:15, and a light chain CDR3 of SEQ ID NO: 16;
(c) a heavy chain CDR1 of SEQ ID NO: 5, a heavy chain CDR2 of SEQ ID NO: 6, a heavy chain CDR3 of SEQ ID NO: 3, a light chain CDR1 of SEQ ID NO: 17, a light chain CDR2 of SEQ ID NO: 18, and a light chain CDR3 of SEQ ID NO: 19; or
(d) a heavy chain CDR1 of SEQ ID NO: 7, a heavy chain CDR2 of SEQ ID NO: 8, a heavy chain CDR3 of SEQ ID NO: 9, a light chain CDR1 of SEQ ID NO: 20, a light chain CDR2 of SEQ ID NO: 18, and a light chain CDR3 of SEQ ID NO: 16.

In an embodiment, the Ab comprises a heavy chain variable region (VH) comprising the amino acid sequence of SEQ ID NO: 10 and a light chain variable region (VL) comprising the amino acid sequence of SEQ ID NO: 25. In an embodiment, the Ab comprises a heavy chain comprising the amino acid sequence of SEQ ID NO: 283 and a light chain comprising the amino acid sequence of SEQ ID NO: 27.

In an embodiment, the Ab comprises a heavy chain amino acid sequence comprising an N-glycosylation site. In an embodiment, the N-glycosylation site is located at Asn306.

In an embodiment, the method further comprises determining the expression of one or more biomarkers. In an embodiment, the one or more biomarkers is selected from premelanosome protein 17 (PMEL17), phosphorylated ERK (pERK), cluster of differentiation 8 (CD8), programmed death-ligand 1 (PD-L1), Dual specificity phosphatase 6 (DUSP6), Ras guanyl-releasing protein 3 (RASGRP3), or any combination thereof.

In an embodiment, the expression of the one or more biomarkers is determined in a sample from the subject. In an embodiment, the sample is obtained from the subject before, during, and/or after administration of the ADC. In an embodiment, the sample is a tumor sample. For example, the tumor sample can be an archived tissue block or obtained by a needle biopsy. In an embodiment, the mRNA expression of the one or more biomarkers is determined. In an embodiment, the protein expression of the one or more biomarkers is determined. In an embodiment, the method further comprises detecting one or more cancer-related genes by next-generation DNA sequencing, performing a whole transcriptome analysis, determining the expression of one or more immune and cancer-related genes, or a combination thereof.

In an embodiment, the method further comprises evaluating cell-free DNA (cfDNA) in a sample from the subject. In an embodiment, the sample is obtained from the subject before, during, and/or after administration of the ADC. In an embodiment, the sample is a blood sample. In an embodiment, evaluating the cell-free DNA determines one or more of: driver mutation status, predictive pharmacodynamics, tumor mutational burden (TMB), or resistance markers.

In an aspect, the disclosure features a method of evaluating a treatment (*e.g.,* a treatment described herein) of a disease (*e.g.,* a disease described herein) in a subject (*e.g.,* a subject described herein), comprising determining the expression of one or more biomarkers and/or evaluating cell-free DNA (cfDNA) in a sample (*e.g.,* a sample described herein) from the subject, in accordance with a method described herein.

In an aspect, the disclosure features a method of evaluating progression of a disease (*e.g.,* a disease described herein) in a subject (*e.g.,* a subject described herein), comprising determining the expression of one or more biomarkers and/or evaluating cell-free DNA (cfDNA) in a sample (*e.g.,* a sample described herein) from the subject, in accordance with a method described herein.

In an aspect, the disclosure features a method of selecting a treatment (*e.g.,* a treatment described herein) for a disease (*e.g.,* a disease described herein) in a subject (*e.g.,* a subject described herein), comprising determining the expression of one or more biomarkers and/or evaluating cell-free DNA (cfDNA) in a sample (*e.g.,* a sample described herein) from the subject, in accordance with a method described herein.

In an aspect, the disclosure features a method of selecting a subject (*e.g.,* a subject described herein) for a treatment (e*.g.,* a treatment described herein) for a disease (*e.g.,* a disease described herein), comprising determining the expression of one or more biomarkers and/or evaluating cell-free DNA (cfDNA) in a sample (*e.g.,* a sample described herein) from the subject, in accordance with a method described herein.

### Other Embodiments

The aspects and embodiments described herein may include any of the embodiments below.

In some embodiments, the antibody or antigen binding fragment thereof that binds PMEL17 comprises:
(a) a heavy chain variable region that comprises a heavy chain CDR1 (Complementarity Determining Region 1) of SEQ ID NO:1, 4, 5 or 7, a heavy chain CDR2 (Complementarity Determining Region 2) of SEQ ID NO:2, 6 or 8, and a heavy chain CDR3 (Complementarity Determining Region 3) of SEQ ID NO:3 or 9; and a light chain variable region that comprises a light chain CDR1 (Complementarity Determining Region 1) of SEQ ID NO:14, 17 or 20, a light chain CDR2 (Complementarity Determining Region 2) of SEQ ID NO:15 or 18, and a light chain CDR3 (Complementarity Determining Region 3) of SEQ ID NO:16 or 19;
(b) a heavy chain variable region that comprises a heavy chain CDR1 of SEQ ID NO:33, 36, 37 or 39, a heavy chain CDR2 of SEQ ID NO:34, 38 or 40; a heavy chain CDR3 of SEQ ID NO:35 or 41; a light chain CDR1 of SEQ ID NO:46, 49 or 52; a light chain CDR2 of SEQ ID NO:47 or 50; and a light chain CDR3 of SEQ ID NO:48 or 51;
(c) a heavy chain variable region that comprises a heavy chain CDR1 of SEQ ID NO:5, 7, 57 or 60, a heavy chain CDR2 of SEQ ID NO:58, 61 or 62; a heavy chain CDR3 of SEQ ID NO:59 or 63; a light chain CDR1 of SEQ ID NO:68, 71 or 74; a light chain CDR2 of SEQ ID NO:69 or 72; and a light chain CDR3 of SEQ ID NO:70 or 73;
(d) a heavy chain variable region that comprises a heavy chain CDR1 of SEQ ID NO:79, 82, 83 or 85, a heavy chain CDR2 of SEQ ID NO:80, 84 or 86; a heavy chain CDR3 of SEQ ID NO:81 or 87; a light chain CDR1 of SEQ ID NO:92, 95 or 98; a light chain CDR2 of SEQ ID NO:93 or 96; and a light chain CDR3 of SEQ ID NO:94 or 97;
(e) a heavy chain variable region that comprises a heavy chain CDR1 of SEQ ID NO:103, 106, 107 or 109, a heavy chain CDR2 of SEQ ID NO:104, 108 or 110; a heavy chain CDR3 of SEQ ID NO:105 or 111; a light chain CDR1 of SEQ ID NO:49, 52 or 116; a light chain CDR2 of SEQ ID NO:47 or 50; and a light chain CDR3 of SEQ ID NO:117 or 118;
(f) a heavy chain variable region that comprises a heavy chain CDR1 of SEQ ID NO: 123, 126, 127 or 129, a heavy chain CDR2 of SEQ ID NO:124, 128 or 130; a heavy chain CDR3 of SEQ ID NO:125 or 131; a light chain CDR1 of SEQ ID NO:136, 139 or 142; a light chain CDR2 of SEQ ID NO:137 or 140; and a light chain CDR3 of SEQ ID NO:138 or 141;
(g) a heavy chain variable region that comprises a heavy chain CDR1 of SEQ ID NO:123, 126, 127 or 129, a heavy chain CDR2 of SEQ ID NO:124, 128 or 130; a heavy chain CDR3 of SEQ ID NO:147 or 148; a light chain CDR1 of SEQ ID NO:153, 156 or 158; a light chain CDR2 of SEQ ID NO:50 or 154; and a light chain CDR3 of SEQ ID NO:155 or 157;
(h) a heavy chain variable region that comprises a heavy chain CDR1 of SEQ ID NO:103, 106, 107 or 109, a heavy chain CDR2 of SEQ ID NO:104, 108 or 110; a heavy chain CDR3 of SEQ ID NO:163 or 164; a light chain CDR1 of SEQ ID NO:49, 52 or 116; a light chain CDR2 of SEQ ID NO:47 or 50; and a light chain CDR3 of SEQ ID NO:169 or 170;
(i) a heavy chain variable region that comprises a heavy chain CDR1 of SEQ ID NO:175, 178, 179 or 181, a heavy chain CDR2 of SEQ ID NO:176, 180 or 182; a heavy chain CDR3 of SEQ ID NO:177 or 183; a light chain CDR1 of SEQ ID NO:49, 52 or 116; a light chain CDR2 of SEQ ID NO:47 or 50; and a light chain CDR3 of SEQ ID NO:188 or 189;
(j) a heavy chain variable region that comprises a heavy chain CDR1 of SEQ ID NO: 103, 106, 107 or 109, a heavy chain CDR2 of SEQ ID NO: 104, 108 or 110; a heavy chain CDR3 of SEQ ID NO:194 or 195; a light chain CDR1 of SEQ ID NO: 49, 52 or 116; a light chain CDR2 of SEQ ID NO: 47 or 50; and a light chain CDR3 of SEQ ID NO:200 or 201;
(k) a heavy chain variable region that comprises a heavy chain CDR1 of SEQ ID NO:206, 209, 210 or 212, a heavy chain CDR2 of SEQ ID NO:207, 211 or 213; a heavy chain CDR3 of SEQ ID NO:208 or 214; a light chain CDR1 of SEQ ID NO:153, 156 or 158; a light chain CDR2 of SEQ ID NO:50 or 154; and a light chain CDR3 of SEQ ID NO:219 or 220;
(l) a heavy chain variable region that comprises a heavy chain CDR1 of SEQ ID NO: 206, 209, 210 or 212, a heavy chain CDR2 of SEQ ID NO: 207, 211 or 213; a heavy chain CDR3 of SEQ ID NO:225 or 226; a light chain CDR1 of SEQ ID NO:136, 139 or 142; a light chain CDR2 of SEQ ID NO:137 or 140; and a light chain CDR3 of SEQ ID NO:231 or 232;
(m) a heavy chain variable region that comprises a heavy chain variable region that comprises an HCDR1 of SEQ ID NO: 206, 209, 210 or 212, an HCDR2 of SEQ ID NO: 207, 211 or 213, and an HCDR3 of SEQ ID NO:237 or 238; and a light chain variable region that comprises an LCDR1 of SEQ ID NO:243, 245 or 247, an LCDR2 of SEQ ID NO:47 or 50, and an LCDR3 of SEQ ID NO:244 or 246;
(n) a heavy chain variable region that comprises a heavy chain variable region that comprises an HCDR1 of SEQ ID NO: 206, 209, 210 or 212, an HCDR2 of SEQ ID NO: 207, 211 or 213, and an HCDR3 of SEQ ID NO:252 or 253; and a light chain variable region that comprises an LCDR1 of SEQ ID NO:153, 156 or 158, an LCDR2 of SEQ ID NO:50 or 154, and an LCDR3 of SEQ ID NO:258 or 259;
(o) a heavy chain CDR1 of SEQ ID NO:1, a heavy chain CDR2 of SEQ ID NO:2, a heavy chain CDR3 of SEQ ID NO:3, a light chain CDR1 of SEQ ID NO:14, a light chain CDR2 of SEQ ID NO:15, and a light chain CDR3 of SEQ ID NO:16;
(p) a heavy chain CDR1 of SEQ ID NO: 4, a heavy chain CDR2 of SEQ ID NO:2, a heavy chain CDR3 of SEQ ID NO:3, a light chain CDR1 of SEQ ID NO:14, a light chain CDR2 of SEQ ID NO:15, and a light chain CDR3 of SEQ ID NO:16;
(q) a heavy chain CDR1 of SEQ ID NO:5, a heavy chain CDR2 of SEQ ID NO:6, a heavy chain CDR3 of SEQ ID NO:3, a light chain CDR1 of SEQ ID NO:17, a light chain CDR2 of SEQ ID NO: 18, and a light chain CDR3 of SEQ ID NO: 19;
(r) a heavy chain CDR1 of SEQ ID NO:7, a heavy chain CDR2 of SEQ ID NO:8, a heavy chain CDR3 of SEQ ID NO:9, a light chain CDR1 of SEQ ID NO:20, a light chain CDR2 of SEQ ID NO:18, and a light chain CDR3 of SEQ ID NO:16;
(s) a heavy chain CDR1 of SEQ ID NO:33, a heavy chain CDR2 of SEQ ID NO:34, a heavy chain CDR3 of SEQ ID NO:35, a light chain CDR1 of SEQ ID NO:46, a light chain CDR2 of SEQ ID NO:47, and a light chain CDR3 of SEQ ID NO:48;
(t) a heavy chain CDR1 of SEQ ID NO:36, a heavy chain CDR2 of SEQ ID NO:34, a heavy chain CDR3 of SEQ ID NO:35, a light chain CDR1 of SEQ ID NO:46, a light chain CDR2 of SEQ ID NO:47, and a light chain CDR3 of SEQ ID NO:48;
(u) a heavy chain CDR1 of SEQ ID NO:37, a heavy chain CDR2 of SEQ ID NO:38, a heavy chain CDR3 of SEQ ID NO:35, a light chain CDR1 of SEQ ID NO:49, a light chain CDR2 of SEQ ID NO:50, and a light chain CDR3 of SEQ ID NO:51;
(v) a heavy chain CDR1 of SEQ ID NO: 39, a heavy chain CDR2 of SEQ ID NO:40, a heavy chain CDR3 of SEQ ID NO:41, a light chain CDR1 of SEQ ID NO:52, a light chain CDR2 of SEQ ID NO:50, and a light chain CDR3 of SEQ ID NO:48;
(w) a heavy chain CDR1 of SEQ ID NO:57, a heavy chain CDR2 of SEQ ID NO:58, a heavy chain CDR3 of SEQ ID NO:59, a light chain CDR1 of SEQ ID NO:68, a light chain CDR2 of SEQ ID NO:69, and a light chain CDR3 of SEQ ID NO:70;
(x) a heavy chain CDR1 of SEQ ID NO:60, a heavy chain CDR2 of SEQ ID NO:58, a heavy chain CDR3 of SEQ ID NO:59, a light chain CDR1 of SEQ ID NO:68, a light chain CDR2 of SEQ ID NO:69, and a light chain CDR3 of SEQ ID NO:70;
(y) a heavy chain CDR1 of SEQ ID NO:5, a heavy chain CDR2 of SEQ ID NO:61, a heavy chain CDR3 of SEQ ID NO:59, a light chain CDR1 of SEQ ID NO:71, a light chain CDR2 of SEQ ID NO:72, and a light chain CDR3 of SEQ ID NO:73;
(z) a heavy chain CDR1 of SEQ ID NO:7, a heavy chain CDR2 of SEQ ID NO:62, a heavy chain CDR3 of SEQ ID NO:63, a light chain CDR1 of SEQ ID NO:74, a light chain CDR2 of SEQ ID NO:72, and a light chain CDR3 of SEQ ID NO:70;
(aa) a heavy chain CDR1 of SEQ ID NO:79, , a heavy chain CDR2 of SEQ ID NO:80, a heavy chain CDR3 of SEQ ID NO:81, a light chain CDR1 of SEQ ID NO:92, a light chain CDR2 of SEQ ID NO:93, and a light chain CDR3 of SEQ ID NO:94;
(bb) a heavy chain CDR1 of SEQ ID NO:82, a heavy chain CDR2 of SEQ ID NO:80, a heavy chain CDR3 of SEQ ID NO:81, a light chain CDR1 of SEQ ID NO:92, a light chain CDR2 of SEQ ID NO:93, and a light chain CDR3 of SEQ ID NO:94;
(cc) a heavy chain CDR1 of SEQ ID NO:83, a heavy chain CDR2 of SEQ ID NO:84, a heavy chain CDR3 of SEQ ID NO:81, a light chain CDR1 of SEQ ID NO:95, a light chain CDR2 of SEQ ID NO:96, and a light chain CDR3 of SEQ ID NO: 97;
(dd) a heavy chain CDR1 of SEQ ID NO: 85, a heavy chain CDR2 of SEQ ID NO:86, a heavy chain CDR3 of SEQ ID NO:87, a light chain CDR1 of SEQ ID NO:98, a light chain CDR2 of SEQ ID NO:96, and a light chain CDR3 of SEQ ID NO:94;
(ee) a heavy chain CDR1 of SEQ ID NO:103, a heavy chain CDR2 of SEQ ID NO:104, a heavy chain CDR3 of SEQ ID NO:105, a light chain CDR1 of SEQ ID NO: 116; a light chain CDR2 of SEQ ID NO:47; and a light chain CDR3 of SEQ ID NO:117;
(ff) a heavy chain CDR1 of SEQ ID NO:106, a heavy chain CDR2 of SEQ ID NO:104, a heavy chain CDR3 of SEQ ID NO:105, a light chain CDR1 of SEQ ID NO: 116, a light chain CDR2 of SEQ ID NO:47, and a light chain CDR3 of SEQ ID NO:117;
(gg) a heavy chain CDR1 of SEQ ID NO:107, a heavy chain CDR2 of SEQ ID NO:108, a heavy chain CDR3 of SEQ ID NO:105, a light chain CDR1 of SEQ ID NO:49, a light chain CDR2 of SEQ ID NO:50, and a light chain CDR3 of SEQ ID NO:118;
(hh) a heavy chain CDR1 of SEQ ID NO:109, a heavy chain CDR2 of SEQ ID NO:110, a heavy chain CDR3 of SEQ ID NO:111, a light chain CDR1 of SEQ ID NO:52 a light chain CDR2 of SEQ ID NO:50, and a light chain CDR3 of SEQ ID NO:117;
(ii) a heavy chain CDR1 of SEQ ID NO:123, a heavy chain CDR2 of SEQ ID NO:124, a heavy chain CDR3 of SEQ ID NO:125, a light chain CDR1 of SEQ ID NO:136, a light chain CDR2 of SEQ ID NO:137,and a light chain CDR3 of SEQ ID NO:138;
(jj) a heavy chain CDR1 of SEQ ID NO:126, a heavy chain CDR2 of SEQ ID NO:124, a heavy chain CDR3 of SEQ ID NO:125, a light chain CDR1 of SEQ ID NO:136, a light chain CDR2 of SEQ ID NO:137, and a light chain CDR3 of SEQ ID NO:138;
(kk) a heavy chain CDR1 of SEQ ID NO:127, a heavy chain CDR2 of SEQ ID NO:128, a heavy chain CDR3 of SEQ ID NO:125, a light chain CDR1 of SEQ ID NO:139, a light chain CDR2 of SEQ ID NO:140, and a light chain CDR3 of SEQ ID NO: 141;
(ll) a heavy chain CDR1 of SEQ ID NO: 129, a heavy chain CDR2 of SEQ ID NO:130, a heavy chain CDR3 of SEQ ID NO:131, a light chain CDR1 of SEQ ID NO:142, a light chain CDR2 of SEQ ID NO:140, and a light chain CDR3 of SEQ ID NO:138;
(mm) a heavy chain CDR1 of SEQ ID NO:123, a heavy chain CDR2 of SEQ ID NO:124, a heavy chain CDR3 of SEQ ID NO:147, a light chain CDR1 of SEQ ID NO:153, a light chain CDR2 of SEQ ID NO:154, and a light chain CDR3 of SEQ ID NO:155;
(nn) a heavy chain CDR1 of SEQ ID NO:126, a heavy chain CDR2 of SEQ ID NO:124, a heavy chain CDR3 of SEQ ID NO:147, a light chain CDR1 of SEQ ID NO:153, a light chain CDR2 of SEQ ID NO: 154, and a light chain CDR3 of SEQ ID NO:155;
(oo) a heavy chain CDR1 of SEQ ID NO:127, a heavy chain CDR2 of SEQ ID NO:128, a heavy chain CDR3 of SEQ ID NO:147, a light chain CDR1 of SEQ ID NO:156, a light chain CDR2 of SEQ ID NO:50, and a light chain CDR3 of SEQ ID NO:157;
(pp) a heavy chain CDR1 of SEQ ID NO: 129, a heavy chain CDR2 of SEQ ID NO:130, a heavy chain CDR3 of SEQ ID NO:148, a light chain CDR1 of SEQ ID NO:158, a light chain CDR2 of SEQ ID NO:50, and a light chain CDR3 of SEQ ID NO:155;
(qq) a heavy chain CDR1 of SEQ ID NO:103, a heavy chain CDR2 of SEQ ID NO:104, a heavy chain CDR3 of SEQ ID NO:163, a light chain CDR1 of SEQ ID NO: 116, a light chain CDR2 of SEQ ID NO:47, and a light chain CDR3 of SEQ ID NO:169;
(rr) a heavy chain CDR1 of SEQ ID NO:106, a heavy chain CDR2 of SEQ ID NO:104, a heavy chain CDR3 of SEQ ID NO:163, a light chain CDR1 of SEQ ID NO:116, a light chain CDR2 of SEQ ID NO:47, and a light chain CDR3 of SEQ ID NO:169;
(ss) a heavy chain CDR1 of SEQ ID NO:107, a heavy chain CDR2 of SEQ ID NO:108, a heavy chain CDR3 of SEQ ID NO:163, a light chain CDR1 of SEQ ID NO:49, a light chain CDR2 of SEQ ID NO:50, and a light chain CDR3 of SEQ ID NO:170;
(tt) a heavy chain CDR1 of SEQ ID NO: 109, a heavy chain CDR2 of SEQ ID NO:110, a heavy chain CDR3 of SEQ ID NO:164, a light chain CDR1 of SEQ ID NO:52, a light chain CDR2 of SEQ ID NO:50, and a light chain CDR3 of SEQ ID NO:169;
(uu) a heavy chain CDR1 of SEQ ID NO:175, a heavy chain CDR2 of SEQ ID NO:176, a heavy chain CDR3 of SEQ ID NO:177, a light chain CDR1 of SEQ ID NO:116, a light chain CDR2 of SEQ ID NO:47, and a light chain CDR3 of SEQ ID NO:188;
(vv) a heavy chain CDR1 of SEQ ID NO:178, a heavy chain CDR2 of SEQ ID NO:176, a heavy chain CDR3 of SEQ ID NO:177, a light chain CDR1 of SEQ ID NO:116, a light chain CDR2 of SEQ ID NO:47, and a light chain CDR3 of SEQ ID NO:188;
(ww) a heavy chain CDR1 of SEQ ID NO:179, a heavy chain CDR2 of SEQ ID NO:180, a heavy chain CDR3 of SEQ ID NO:177, a light chain CDR1 of SEQ ID NO:49, a light chain CDR2 of SEQ ID NO:50, and a light chain CDR3 of SEQ ID NO:189;
(xx) a heavy chain CDR1 of SEQ ID NO: 181, a heavy chain CDR2 of SEQ ID NO:182; a heavy chain CDR3 of SEQ ID NO:183, a light chain CDR1 of SEQ ID NO:52, a light chain CDR2 of SEQ ID NO:50, and a light chain CDR3 of SEQ ID NO:188;
(yy) a heavy chain CDR1 of SEQ ID NO: 103, a heavy chain CDR2 of SEQ ID NO: 104, a heavy chain CDR3 of SEQ ID NO:194, a light chain CDR1 of SEQ ID NO: 116, a light chain CDR2 of SEQ ID NO: 47, and a light chain CDR3 of SEQ ID NO:200;
(zz) a heavy chain CDR1 of SEQ ID NO: 106, a heavy chain CDR2 of SEQ ID NO: 104, a heavy chain CDR3 of SEQ ID NO:194, a light chain CDR1 of SEQ ID NO: 116, a light chain CDR2 of SEQ ID NO: 47, and a light chain CDR3 of SEQ ID NO:200;
(aaa) a heavy chain CDR1 of SEQ ID NO: 107, a heavy chain CDR2 of SEQ ID NO: 108, a heavy chain CDR3 of SEQ ID NO: 194, a light chain CDR1 of SEQ ID NO: 49, a light chain CDR2 of SEQ ID NO: 50, and a light chain CDR3 of SEQ ID NO: 201;
(bbb) a heavy chain CDR1 of SEQ ID NO: 109, a heavy chain CDR2 of SEQ ID NO: 110, a heavy chain CDR3 of SEQ ID NO: 195, a light chain CDR1 of SEQ ID NO: 52, a light chain CDR2 of SEQ ID NO: 50, and a light chain CDR3 of SEQ ID NO:200;
(ccc) a heavy chain CDR1 of SEQ ID NO:206, a heavy chain CDR2 of SEQ ID NO:207, a heavy chain CDR3 of SEQ ID NO:208, a light chain CDR1 of SEQ ID NO:153, a light chain CDR2 of SEQ ID NO:154, and a light chain CDR3 of SEQ ID NO:219;
(ddd) a heavy chain CDR1 of SEQ ID NO:209, a heavy chain CDR2 of SEQ ID NO:207, a heavy chain CDR3 of SEQ ID NO:208, a light chain CDR1 of SEQ ID NO:153, a light chain CDR2 of SEQ ID NO: 154, and a light chain CDR3 of SEQ ID NO:219;
(eee) a heavy chain CDR1 of SEQ ID NO:210, a heavy chain CDR2 of SEQ ID NO:211, a heavy chain CDR3 of SEQ ID NO:208, a light chain CDR1 of SEQ ID NO:156, a light chain CDR2 of SEQ ID NO:50, and a light chain CDR3 of SEQ ID NO:220;
(fff) a heavy chain CDR1 of SEQ ID NO: 212, a heavy chain CDR2 of SEQ ID NO:213, a heavy chain CDR3 of SEQ ID NO:214, a light chain CDR1 of SEQ ID NO:158, a light chain CDR2 of SEQ ID NO:50, and a light chain CDR3 of SEQ ID NO:219;
(ggg) a heavy chain CDR1 of SEQ ID NO: 206, a heavy chain CDR2 of SEQ ID NO: 207, a heavy chain CDR3 of SEQ ID NO:225, a light chain CDR1 of SEQ ID NO:136, a light chain CDR2 of SEQ ID NO:137,and a light chain CDR3 of SEQ ID NO:231;
(hhh) a heavy chain CDR1 of SEQ ID NO: 209, a heavy chain CDR2 of SEQ ID NO: 207, a heavy chain CDR3 of SEQ ID NO:225, a light chain CDR1 of SEQ ID NO:136, a light chain CDR2 of SEQ ID NO:137, and a light chain CDR3 of SEQ ID NO:231;
(iii) a heavy chain CDR1 of SEQ ID NO: 210, a heavy chain CDR2 of SEQ ID NO: 211, a heavy chain CDR3 of SEQ ID NO:225, a light chain CDR1 of SEQ ID NO:139, a light chain CDR2 of SEQ ID NO:140, and a light chain CDR3 of SEQ ID NO: 232;
(jjj) a heavy chain CDR1 of SEQ ID NO: 212, a heavy chain CDR2 of SEQ ID NO: 213, a heavy chain CDR3 of SEQ ID NO: 226, a light chain CDR1 of SEQ ID NO:142; a light chain CDR2 of SEQ ID NO: 140; and a light chain CDR3 of SEQ ID NO:231;
(kkk) a heavy chain variable region that comprises an HCDR1 of SEQ ID NO: 206, an HCDR2 of SEQ ID NO: 207, and an HCDR3 of SEQ ID NO:237,and a light chain variable region that comprises an LCDR1 of SEQ ID NO:243, an LCDR2 of SEQ ID NO:47, and an LCDR3 of SEQ ID NO:244;
(lll) a heavy chain variable region that comprises an HCDR1 of SEQ ID NO: 209, an HCDR2 of SEQ ID NO: 207, and an HCDR3 of SEQ ID NO:237, and a light chain variable region that comprises an LCDR1 of SEQ ID NO:243, an LCDR2 of SEQ ID NO:47, and an LCDR3 of SEQ ID NO:244;
(mmm) a heavy chain variable region that comprises an HCDR1 of SEQ ID NO: 210, an HCDR2 of SEQ ID NO: 211, and an HCDR3 of SEQ ID NO:237, and a light chain variable region that comprises an LCDR1 of SEQ ID NO:245, an LCDR2 of SEQ ID NO:50, and an LCDR3 of SEQ ID NO:246;
(nnn) a heavy chain variable region that comprises an HCDR1 of SEQ ID NO: 212, an HCDR2 of SEQ ID NO: 213, and an HCDR3 of SEQ ID NO:238; and a light chain variable region that comprises an LCDR1 of SEQ ID NO:247, an LCDR2 of SEQ ID NO: 50, and an LCDR3 of SEQ ID NO:244;
(ooo) a heavy chain variable region that comprises an HCDR1 of SEQ ID NO: 206, an HCDR2 of SEQ ID NO: 207, and an HCDR3 of SEQ ID NO:252,and a light chain variable region that comprises an LCDR1 of SEQ ID NO:153, an LCDR2 of SEQ ID NO: 154, and an LCDR3 of SEQ ID NO:258;
(ppp) a heavy chain variable region that comprises an HCDR1 of SEQ ID NO: 209, an HCDR2 of SEQ ID NO: 207, and an HCDR3 of SEQ ID NO:252, and a light chain variable region that comprises an LCDR1 of SEQ ID NO:153, an LCDR2 of SEQ ID NO: 154, and an LCDR3 of SEQ ID NO:258;
(qqq) a heavy chain variable region that comprises an HCDR1 of SEQ ID NO: 210, an HCDR2 of SEQ ID NO: 211, and an HCDR3 of SEQ ID NO:252, and a light chain variable region that comprises an LCDR1 of SEQ ID NO:156, an LCDR2 of SEQ ID NO:50, and an LCDR3 of SEQ ID NO:259; or
(rrr) a heavy chain variable region that comprises an HCDR1 of SEQ ID NO: 212, an HCDR2 of SEQ ID NO: 213, and an HCDR3 of SEQ ID NO: 253; and a light chain variable region that comprises an LCDR1 of SEQ ID NO:158, an LCDR2 of SEQ ID NO:50, and an LCDR3 of SEQ ID NO:258.

In some embodiments, the antibody or antigen binding fragment thereof that binds PMEL17 comprises:
(a) a heavy chain variable region (VH) comprising the amino acid sequence of SEQ ID NO:10, and a light chain variable region (VL) comprising the amino acid sequence of SEQ ID NO:21;
(b) a heavy chain variable region (VH) comprising the amino acid sequence of SEQ ID NO:10, and a light chain variable region (VL) comprising the amino acid sequence of SEQ ID NO:25;
(c) a heavy chain variable region (VH) comprising the amino acid sequence of SEQ ID NO:10, and a light chain variable region (VL) comprising the amino acid sequence of SEQ ID NO:29;
(d) a heavy chain variable region (VH) comprising the amino acid sequence of SEQ ID NO:42, and a light chain variable region (VL) comprising the amino acid sequence of SEQ ID NO:53;
(e) a heavy chain variable region (VH) comprising the amino acid sequence of SEQ ID NO:64, and a light chain variable region (VL) comprising the amino acid sequence of SEQ ID NO:75;
(f) a heavy chain variable region (VH) comprising the amino acid sequence of SEQ ID NO:88, and a light chain variable region (VL) comprising the amino acid sequence of SEQ ID NO:99;
(g) heavy chain variable region (VH) comprising the amino acid sequence of SEQ ID NO:112, and a light chain variable region (VL) comprising the amino acid sequence of SEQ ID NO:119;
(h) heavy chain variable region (VH) comprising the amino acid sequence of SEQ ID NO:132, and a light chain variable region (VL) comprising the amino acid sequence of SEQ ID NO:143;
(i) heavy chain variable region (VH) comprising the amino acid sequence of SEQ ID NO:149, and a light chain variable region (VL) comprising the amino acid sequence of SEQ ID NO: 159;
(j) heavy chain variable region (VH) comprising the amino acid sequence of SEQ ID NO:165, and a light chain variable region (VL) comprising the amino acid sequence of SEQ ID NO: 171;
(k) heavy chain variable region (VH) comprising the amino acid sequence of SEQ ID NO:184, and a light chain variable region (VL) comprising the amino acid sequence of SEQ ID NO:190;
(l) heavy chain variable region (VH) comprising the amino acid sequence of SEQ ID NO:196, and a light chain variable region (VL) comprising the amino acid sequence of SEQ ID NO:202;
(m) heavy chain variable region (VH) comprising the amino acid sequence of SEQ ID NO:215, and a light chain variable region (VL) comprising the amino acid sequence of SEQ ID NO:221;
(n) heavy chain variable region (VH) comprising the amino acid sequence of SEQ ID NO:227, and a light chain variable region (VL) comprising the amino acid sequence of SEQ ID NO:233;
(o) heavy chain variable region (VH) comprising the amino acid sequence of SEQ ID NO:239, and a light chain variable region (VL) comprising the amino acid sequence of SEQ ID NO:248; or
(p) heavy chain variable region (VH) comprising the amino acid sequence of SEQ ID NO:254, and a light chain variable region (VL) comprising the amino acid sequence of SEQ ID NO:260.

In some embodiments, the antibody or antigen binding fragment thereof that binds PMEL17 comprises:
(a) a heavy chain comprising the amino acid sequence of SEQ ID NO:12, and a light chain comprising the amino acid sequence of SEQ ID NO:23;
(b) a heavy chain comprising the amino acid sequence of SEQ ID NO:12, and a light chain comprising the amino acid sequence of SEQ ID NO:27;
(c) a heavy chain comprising the amino acid sequence of SEQ ID NO:12, and a light chain comprising the amino acid sequence of SEQ ID NO:31;
(d) a heavy chain comprising the amino acid sequence of SEQ ID NO:44, and a light chain comprising the amino acid sequence of SEQ ID NO:55;
(e) a heavy chain comprising the amino acid sequence of SEQ ID NO:66, and a light chain comprising the amino acid sequence of SEQ ID NO:77;
(f) a heavy chain comprising the amino acid sequence of SEQ ID NO:90, and a light chain comprising the amino acid sequence of SEQ ID NO:101;
(g) a heavy chain comprising the amino acid sequence of SEQ ID NO:114, and a light chain comprising the amino acid sequence of SEQ ID NO:121.;
(h) a heavy chain comprising the amino acid sequence of SEQ ID NO:134, and a light chain comprising the amino acid sequence of SEQ ID NO:145;
(i) a heavy chain comprising the amino acid sequence of SEQ ID NO:151, and a light chain comprising the amino acid sequence of SEQ ID NO:161;
(j) a heavy chain comprising the amino acid sequence of SEQ ID NO:167, and a light chain comprising the amino acid sequence of SEQ ID NO:173;
(k) a heavy chain comprising the amino acid sequence of SEQ ID NO:186, and a light chain comprising the amino acid sequence of SEQ ID NO:192;
(l) a heavy chain comprising the amino acid sequence of SEQ ID NO:198, and a light chain comprising the amino acid sequence of SEQ ID NO:204;
(m) a heavy chain comprising the amino acid sequence of SEQ ID NO:217, and a light chain comprising the amino acid sequence of SEQ ID NO:223;
(n) a heavy chain comprising the amino acid sequence of SEQ ID NO:229, and a light chain comprising the amino acid sequence of SEQ ID NO:235;
(o) a heavy chain comprising the amino acid sequence of SEQ ID NO:241, and a light chain comprising the amino acid sequence of SEQ ID NO:250;
(p) heavy chain comprising the amino acid sequence of SEQ ID NO:256, and a light chain comprising the amino acid sequence of SEQ ID NO:262;
(q) a heavy chain comprising the amino acid sequence of SEQ ID NO:283, and a light chain comprising the amino acid sequence of SEQ ID NO:27;
(r) a heavy chain comprising the amino acid sequence of SEQ ID NO:283, and a light chain comprising the amino acid sequence of SEQ ID NO:31;
(s) a heavy chain comprising the amino acid sequence of SEQ ID NO:315, and a light chain comprising the amino acid sequence of SEQ ID NO:101.

In some embodiments, the antibody or antigen binding fragment thereof comprises one or more cysteine substitutions. In some embodiments, the antibody or antigen binding fragment thereof comprises one or more cysteine substitutions selected from E152C, S375C, or both E152C and S375C of the heavy chain of the antibody or antigen binding fragment thereof, wherein the position is numbered according to the EU system. In some embodiments, the antibody or antigen binding fragment thereof comprises an E152C substitution of the heavy chain of the antibody or antigen binding fragment thereof, wherein the position is numbered according to the EU system.

In some embodiments, the antibody molecule is a monoclonal antibody. In some embodiments, the antibody molecule is an isolated antibody. In some embodiments, the antibody molecule is a synthetic antibody. In some embodiments, the antibody molecule is an engineered antibody.

In some embodiments, the antibody or antigen binding fragment is encoded by a nucleic acid described herein. In some embodiments, the nucleic acid comprises the nucleotide sequence of SEQ ID NOs: 13, 24, 28, 32, 45, 56, 67, 78, 91, 102, 115, 122, 135, 146, 152, 162, 168, 174, 187, 193, 199, 205, 218, 224, 230, 236, 242, 251, 257, 263, 319, or 320. In some embodiments, the nucleic acid is present in a vector, *e.g.,* a vector described herein. In some embodiments, the nucleic acid or vector is present in a host cell, *e.g.,* a host cell described herein.

In some embodiments, the antibody or antigen binding fragment is produced by a method comprising cultivating the host cell and recovering the antibody or antigen binding fragment from cell culture. In some embodiments, recovering the antibody from cell culture comprises the steps of: removing cells and filtering the culture; purifying the culture by affinity chromatography; inactivating any viruses in the culture by adjusting the pH to 3.4-3.6, then readjusting the pH to 5.8-6.2 and filtering the culture; purifying the culture by cation exchange chromatography and performing on-column reduction of the culture; performing anion exchange chromatography on the culture; removing viruses by nanofiltration; filtering the culture containing the antibody; and obtaining purified antibody.

In some embodiments, the antibody drug conjugate is an antibody drug conjugate made by a process described herein.

In some embodiments, the antibody drug conjugate comprises the formula (C)

Ab-(L_{A}-(D)ₙ)_{y} (C)

wherein:
D is a GNAQ inhibitor, a GNA11 inhibitor or an inhibitor of GNAQ and GNA11;
Ab is an antibody or antigen binding fragment thereof that binds to human PMEL17 protein;
L_{A} is a linker;
n is 1, 2, 3 or 4, and
y is 1, 2, 3 or 4.

In some embodiments, said n is 1. In some embodiments, said y is about 1 to about 4. In some embodiments, said linker is a cleavable linker or a non-cleavable linker. In some embodiments, the linker comprises a ValCit peptide linker. In some

In some embodiments, said drug moiety is an inhibitor of GNAQ and GNA11.

In some embodiments, the D is

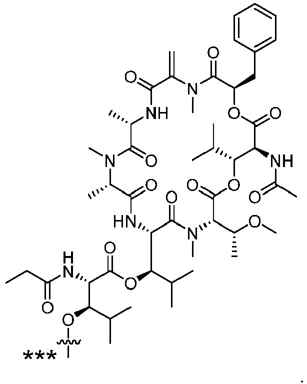

In some embodiments, the antibody drug conjugate has the following structure,

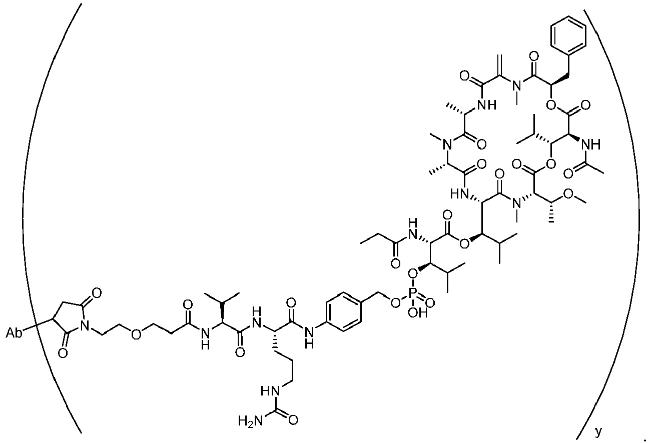

In some embodiments, the antibody drug conjugate has the following Formula (C-2): 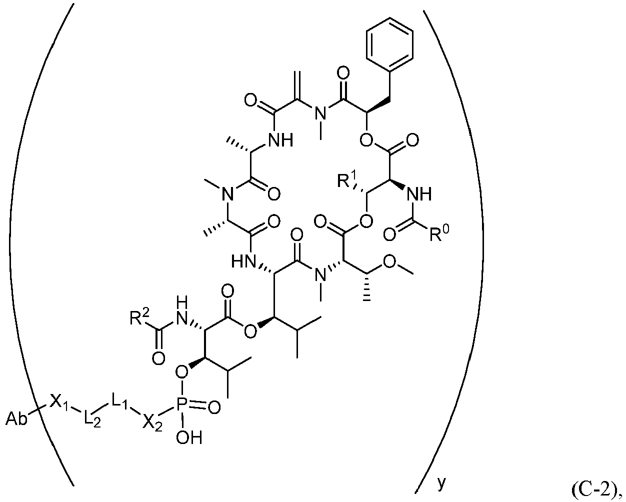 wherein:
R⁰ is methyl or ethyl;
R¹ is methyl or isopropyl;
R² is methyl or ethyl;
Ab is an antibody or antigen binding fragment thereof that binds to human PMEL17 protein;
X₁ is a bivalent coupling group;
X₂ is a self-immolative spacer;
L₁ is a bivalent peptide linker;
L₂ is a bond or a linker, and
y is 1, 2, 3 or 4.

In some embodiments, the antibody drug conjugate is present in a pharmaceutical composition comprising the antibody drug conjugate and a pharmaceutically acceptable carrier. In some embodiments, the antibody drug conjugate is present in a formulation described herein.

In some embodiments, the antibody drug conjugate or formulation is used as a medicament.

In some embodiments, the antibody drug conjugate or formulation is used in a method of treating or preventing cancer in a patient in need thereof, comprising administering to said patient the antibody drug conjugate or formulation, wherein the cancer expresses PMEL17, contains a mutation of the GNAQ or GNA11 gene, or the cancer expresses PMEL17 and contains a mutation of GNAQ, GNA11, or both. In some embodiments, the cancer is a carcinoma (*e.g.,* hepatocellular carcinoma), sarcoma, leukemia, lymphoma, eye cancer, eye neoplasm, melanoma (*e.g.,* uveal melanoma, non-uveal melanoma, malignant melanoma, ocular melanoma, subcutaneous melanoma, cutaneous melanoma, or mucosal melanoma), or a metastatic cancer thereof.

In some embodiments, the antibody drug conjugate or formulation is administered to the patient in combination with one or more additional therapeutic compounds.

In some embodiments, the one or more additional therapeutic compounds is selected from a standard of care chemotherapeutic, an MDM2 inhibitor, an MRC2 inhibitor, a PKC inhibitor, a MAPK inhibitor, a costimulatory molecule, or a checkpoint inhibitor.

In some embodiments, the costimulatory molecule is selected from an agonist of OX40, CD2, CD27, CDS, ICAM-1, LFA-1 (CD11a/CD18), ICOS (CD278), 4-1BB (CD137), GITR, CD30, CD40, BAFFR, HVEM, CD7, LIGHT, NKG2C, SLAMF7, NKp80, CD160, B7-H3, STING, or CD83 ligand.

In some embodiments, the checkpoint inhibitor is selected from an inhibitor of PD-1, PD-L1, PD-L2, CTLA4, TIM3, LAG3, VISTA, BTLA, TIGIT, LAIR1, CD160, 2B4 and/or TGFR beta.

### BRIEF DESCRIPTION OF THE DRAWINGS

**FIG. 1** depicts the gene organization and transposable element containing BGCs of compound (A1) (*frsA-H*) and compound J (*dis1-4*) in *C. vaccinii* (76974 bp).
**FIG. 2** depicts the scheme of the insertion strategy used for promoter exchanges.
**FIG. 3** depicts the production of compound J in disruption mutants compared to wild type.
**FIG. 4** depicts the MS/MS spectrum of compound J.
**FIG. 5** depicts the MS/MS spectrum of compound F5.
**FIG. 6** depicts the MS/MS spectrum of compound F3.
**FIG. 7** depicts the MS/MS spectrum of compound D.
**FIG. 8** depicts the schematic view of compound J biosynthesis. Figure discloses "Ser Val His Val Leu Val" as SEQ ID NO: 324.
**FIG. 9** depicts the exemplary on-site and off-side conjugations.
**FIG. 10A** depicts the organization of the compound (A1)-BGC (white: NRPS genes, black: genes encoding modifying enzymes) and the representation of exchanged promoters.
**FIG. 10B** depicts a bar chart of the titers of compound (A1) from different *C. vaccinii* promoter exchange mutants including fold changes compared to wild type.
**FIG. 11A** depicts the HPLC-MS extracted ion chromatograms (EIC) 1002.535 - 1002.545 Da of culture extracts of *C. vaccinii* wild type and *C. vaccinii vioP* mutant.
**FIG. 11B** depicts the HPLC-MS EIC 1020.49 - 1020.50 Da of culture extract of *C. vaccinii vioP* mutant fed with _{L}-Met revealing three novel analogs of compound (A1).
**FIG. 12A** depicts the HPLC-MS extracted ion chromatograms (EIC) of compound (A1) at *m*/*z* 1002.52 - 1002.57 following incubation in human plasma at 5 µg/mL and 37 °C for 8 h (bottom) and the same sample spiked with additional 100 ng of compound 5 (final conc. 6 µg/mL).
**FIG. 12B** depicts the absolute quantification and fractions of compounds (A1) and 5 following incubation of compound (A1) in human plasma at 5 µg/mL and 37 °C for 24 h.
**FIG. 13A** depicts the effects of compounds (A1) and 2-8 on proliferation of Gq or G11 mutant uveal melanoma (UM) cell lines and Gq or G11 wild type skin melanoma cell lines. Growth of UM cell lines driven by constitutively active Gq or G11 mutants was inhibited by compound (A1) with sub nM GI50s for the 92.1 and MP41 cell lines, whereas the growth of skin melanoma cell lines, harboring homozygous BRAF V600E mutations, A375, and heterozygous NRAS Q61K mutations, SK-MEL-30 was unaffected by compound (A1).
**FIG. 13B** depicts the effects of compounds 9-15 on proliferation of Gq or G11 mutant UM cell lines and Gq or G11 wild type skin melanoma cell lines. Growth of UM cell lines driven by constitutively active Gq or G11 mutants was inhibited by compound (A1) with sub nM GI50s for the 92.1 and MP41 cell lines, whereas the growth of skin melanoma cell lines, harboring homozygous BRAF V600E mutations, A375, and heterozygous NRAS Q61K mutations, SK-MEL-30 was unaffected by compound (A1).
**FIG. 14** depicts SEC data of exemplary ADC formulations.
**FIG. 15** depicts light obscuration data of exemplary ADC formulations.
**FIG. 16** depicts capillary electrophoresis analysis of exemplary ADC formulations.
**FIG. 17** depicts capillary zone electrophoresis (CZE) analysis of exemplary ADC formulations.
**FIG. 18** depicts analysis of exemplary lyophilisate ADC formulations.
**FIG. 19** depicts data for pH and ring-opening of the payload using (CZE).
**FIG. 20** depicts potency decay data for exemplary ADC formulations after a 2-month storage period.
**FIG. 21** depicts the mean exposure profiles of total antibody (tmAb), active ADC (tADCa), and inactive ADC (tADCi) after a single i.v. dose of 3 mg/kg of compound (E) in the cynomolgus monkey.

### DETAILED DESCRIPTION

This disclosure is based, at least in part, on the discovery that certain microorganisms (*e.g.,* Chromobacterium) can be genetically engineered to increase the production of certain depsipeptides (*e.g.,* compound (A1)). Compound (A1) can be isolated from the leaves of *A. crenata,* but the very low quantities and the complex matrix may prevent access to sufficient amounts of compound (A1) to allow drug development efforts and commercial manufacturing. The genetically engineered microorganisms (*e.g., Chromobacterium*)*,* nucleic acids, and methods described herein provide developable production routes for making compound (A1) with elevated titers and/or increased isolated yields to grant access to sufficient amounts of the drug substance precursor.

This disclosure is also based, at least in part, on the discovery that by targeting a higher overreduction and designing a robust, partial reoxidation step efficient decapping of engineered cysteine residues and desired drug-to-antibody ratios can be achieved without comprising on antibody purity. Overreduction and opening of intrachain disulfide bridges can lead to undesired off-site coupling. In some embodiments, an inherent LC-HC instability can result in susceptibility to overreduction. Without wishing to be bound by theory, it is believed that in some embodiments efficient, partial reoxidation of opened intrachain disulfide bridges is important for minimizing off-site conjugation to achieve high efficacy *in vivo* and to reduce linker payload release. Factors that can be assessed for the partial reoxidation step include, but are not limited to, one or more of: different vessel geometry, defined headspace (e.g., 60%) or not, mixing (*e.g.,* 100 rpm) or not, or room temperature or cold room. In some embodiments, by adding a partial reoxidation step described herein into the GMP manufacturing process the purity of final drug substance can be increased to about 95%, *e.g.,* as determined by non-reduced CE SDS. In certain embodiments, a drug-to-antibody ratio of about 2 is achieved. In other embodiments, a drug-to-antibody ratio of about 4 is achieved.

### Definitions

Unless stated otherwise, the following terms and phrases as used herein are intended to have the following meanings.

As used herein, the singular form "a" or "an" includes plural references unless indicated otherwise.

The term "or" is used herein to mean, and is used interchangeably with, the term "and/or" unless context clearly indicates otherwise.

"About" and "approximately" shall generally mean an acceptable degree of error for the quantity measured given the nature or precision of the measurements. Exemplary degrees of error are within 20 percent (%), typically, within 10%, and more typically, within 5% of a given value or range of values.

The term "biosynthetic gene cluster" or "BGC" interchangeably refers to a locally clustered group of two or more genes that together encode a biosynthetic pathway for the production of a metabolite. In some embodiments, the metabolite is a secondary or specialized metabolite, which is not directly involved in the normal growth, development, or reproduction of the organism that produce the metabolite.

The term "alkyl" refers to a monovalent saturated hydrocarbon chain having the specified number of carbon atoms. For example, C₁-C₆alkyl refers to an alkyl group having from 1 to 6 carbon atoms. Alkyl groups may be straight or branched. Representative branched alkyl groups have one, two, or three branches. Examples of alkyl groups include, but are not limited to, methyl, ethyl, propyl (n-propyl and isopropyl), butyl (n-butyl, isobutyl, sec-butyl, and t-butyl), pentyl (n-pentyl, isopentyl, and neopentyl), and hexyl.

"Cleavable" as used herein refers to a linking group or linker component that connects two moieties by covalent connections, but breaks down to sever the covalent connection between the moieties under physiologically relevant conditions, typically a cleavable linking group is severed in vivo more rapidly in an intracellular environment than when outside a cell, causing release of the payload to preferentially occur inside a targeted cell. Cleavage may be enzymatic or non-enzymatic, but generally releases a payload from an antibody without degrading the antibody. Cleavage may leave some portion of a linking group or linker component attached to the payload, or it may release the payload without any residue of the linking group.

"Non-cleavable" as used herein refers to a linking group or linker component that is not especially susceptible to breaking down under physiological conditions, *e.g*., it is at least as stable as the antibody or antigen binding fragment portion of the conjugate. Such linking groups are sometimes referred to as 'stable', meaning they are sufficiently resistant to degradation to keep the payload connected to antibody or antigen binding fragment until the antibody or antigen binding fragment is itself at least partially degraded, *i.e.,* the degradation of the antibody or antigen binding fragment precedes cleavage of the linking group *in vivo.* Degradation of the antibody portion of an ADC having a stable or non-cleavable linking group may leave some or all of the linking group, *e.g*., one or more amino acid groups from an antibody, attached to the payload or drug moiety that is delivered in vivo.

The term "antibody" as used herein refers to a polypeptide of the immunoglobulin family that is capable of binding a corresponding antigen non-covalently, reversibly, and in a specific manner. For example, a naturally occurring IgG antibody is a tetramer comprising at least two heavy (H) chains and two light (L) chains inter-connected by disulfide bonds. Each heavy chain is comprised of a heavy chain variable region (abbreviated herein as VH) and a heavy chain constant region. The heavy chain constant region is comprised of three domains, CH1, CH2 and CH3. Each light chain is comprised of a light chain variable region (abbreviated herein as VL) and a light chain constant region. The light chain constant region is comprised of one domain, CL. The VH and VL regions can be further subdivided into regions of hypervariability, termed complementarity determining regions (CDR), interspersed with regions that are more conserved, termed framework regions (FR). Each VH and VL is composed of three CDRs and four FRs arranged from amino-terminus to carboxy-terminus in the following order: FR1, CDR1, FR2, CDR2, FR3, CDR3, and FR4. The variable regions of the heavy and light chains contain a binding domain that interacts with an antigen. The constant regions of the antibodies may mediate the binding of the immunoglobulin to host tissues or factors, including various cells of the immune system (*e.g.,* effector cells) and the first component (C1q) of the classical complement system.

The term "antibody" includes, but is not limited to, monoclonal antibodies, human antibodies, humanized antibodies, chimeric antibodies, and anti-idiotypic (anti-Id) antibodies (including, *e.g*., anti-Id antibodies to antibodies of the disclosure). The antibodies can be of any isotype/class (*e.g.*, IgG, IgE, IgM, IgD, IgA and IgY), or subclass (*e.g.,* IgG1, IgG2, IgG3, IgG4, IgA1 and IgA2).

"Complementarity-determining domains" or "complementary-determining regions ("CDRs") interchangeably refer to the hypervariable regions of VL and VH. The CDRs are the target protein-binding site of the antibody chains that harbors specificity for such target protein. There are three CDRs (CDR1-3, numbered sequentially from the N-terminus) in each human VL or VH, constituting about 15-20% of the variable domains. The CDRs are structurally complementary to the epitope of the target protein and are thus directly responsible for the binding specificity. The remaining stretches of the VL or VH, the so-called framework regions, exhibit less variation in amino acid sequence (Kuby, Immunology, 4th ed., Chapter 4. W.H. Freeman & Co., New York, 2000).

The positions of the CDRs and framework regions can be determined using various well known definitions in the art, *e.g.*, Kabat, Chothia, international ImMunoGeneTics database (IMGT) (on the worldwide web at www.imgt.org/), and AbM (see, *e.g.,* Johnson et al., Nucleic Acids Res., 29:205-206 (2001); Chothia and Lesk, J. Mol. Biol., 196:901-917 (1987); Chothia et al., Nature, 342:877-883 (1989); Chothia et al., J. Mol. Biol., 227:799-817 (1992); Al-Lazikani et al., J. Mol. Biol., 273:927-748 (1997)). Definitions of antigen combining sites are also described in the following: Ruiz et al., Nucleic Acids Res., 28:219-221 (2000); and Lefranc, M.P., Nucleic Acids Res., 29:207-209 (2001); MacCallum et al., J. Mol. Biol., 262:732-745 (1996); and Martin et al., Proc. Natl. Acad. Sci. USA, 86:9268-9272 (1989); Martin et al., Methods Enzymol., 203:121-153 (1991); and Rees et al., In Sternberg M.J.E. (ed.), Protein Structure Prediction, Oxford University Press, Oxford, 141-172 (1996).

Both the light and heavy chains are divided into regions of structural and functional homology. The terms "constant" and "variable" are used functionally. In this regard, it will be appreciated that the variable domains of both the light (VL) and heavy (VH) chain portions determine antigen recognition and specificity. Conversely, the constant domains of the light chain (CL) and the heavy chain (CH1, CH2 or CH3) confer important biological properties such as secretion, transplacental mobility, Fc receptor binding, complement binding, and the like. By convention, the numbering of the constant region domains increases as they become more distal from the antigen binding site or amino-terminus of the antibody. The N-terminus is a variable region and at the C-terminus is a constant region; the CH3 and CL domains actually comprise the carboxy-terminal domains of the heavy and light chain, respectively.

The term "antigen binding fragment", as used herein, refers to one or more portions of an antibody that retain the ability to specifically interact with (*e.g.,* by binding, steric hindrance, stabilizing/destabilizing, spatial distribution) an epitope of an antigen. Examples of binding fragments include, but are not limited to, single-chain Fvs (scFv), camelid antibodies, disulfide-linked Fvs (sdFv), Fab fragments, F(ab') fragments, a monovalent fragment consisting of the VL, VH, CL and CH1 domains; a F(ab)2 fragment, a bivalent fragment comprising two Fab fragments linked by a disulfide bridge at the hinge region; a Fd fragment consisting of the VH and CH1 domains; a Fv fragment consisting of the VL and VH domains of a single arm of an antibody; a dAb fragment (Ward et al., Nature 341:544-546, 1989), which consists of a VH domain; and an isolated complementarity determining region (CDR), or other epitope-binding fragments of an antibody.

Furthermore, although the two domains of the Fv fragment, VL and VH, are coded for by separate genes, they can be joined, using recombinant methods, by a synthetic linker that enables them to be made as a single protein chain in which the VL and VH regions pair to form monovalent molecules (known as single chain Fv ("scFv"); *see, e.g.,* Bird et al., Science 242:423-426, 1988; and Huston et al., Proc. Natl. Acad. Sci. 85:5879-5883, 1988). Such single chain antibodies are also intended to be encompassed within the term "antigen binding fragment." These antigen binding fragments are obtained using conventional techniques known to those of skill in the art, and the fragments are screened for utility in the same manner as are intact antibodies.

Antigen binding fragments can also be incorporated into single domain antibodies, maxibodies, minibodies, single domain antibodies, intrabodies, diabodies, triabodies, tetrabodies, v-NAR and bis-scFv (*see, e.g.,* Hollinger and Hudson, Nature Biotechnology 23:1126-1136, 2005). Antigen binding fragments can be grafted into scaffolds based on polypeptides such as fibronectin type III (Fn3) (*see* U.S. Pat. No. 6,703,199, which describes fibronectin polypeptide monobodies).

Antigen binding fragments can be incorporated into single chain molecules comprising a pair of tandem Fv segments (VH-CH1-VH-CH1) which, together with complementary light chain polypeptides, form a pair of antigen binding regions (Zapata et al., Protein Eng. 8:1057-1062, 1995; and U.S. Pat. No. 5,641,870).

The term "monoclonal antibody" or "monoclonal antibody composition" as used herein refers to polypeptides, including antibodies and antigen binding fragments that have substantially identical amino acid sequence or are derived from the same genetic source. This term also includes preparations of antibody molecules of single molecular composition. A monoclonal antibody composition displays a single binding specificity and affinity for a particular epitope.

The term "human antibody", as used herein, includes antibodies having variable regions in which both the framework and CDR regions are derived from sequences of human origin. Furthermore, if the antibody contains a constant region, the constant region also is derived from such human sequences, *e.g.*, human germline sequences, or mutated versions of human germline sequences or antibody containing consensus framework sequences derived from human framework sequences analysis, for example, as described in Knappik et al., J. Mol. Biol. 296:57-86, 2000). Also included are antibodies derived from human sequences wherein one or more CDRs has been mutated for affinity maturation or for manufacturing/payload conjugation purposes. *See* Kilpatrick et al., "Rapid development of affinity matured monoclonal antibodies using RIMMS," Hybridoma. 1997 Aug;16(4):381-9.

The human antibodies of the disclosure may include amino acid residues not encoded by human sequences (*e.g.,* mutations introduced by random or site-specific mutagenesis *in vitro* or by somatic mutation *in vivo,* or a conservative substitution to promote stability or manufacturing).

The term "recognize" as used herein refers to an antibody or antigen binding fragment thereof that finds and interacts (*e.g.*, binds) with its epitope, whether that epitope is linear or conformational. The term "epitope" refers to a site on an antigen to which an antibody or antigen binding fragment of the disclosure specifically binds. Epitopes can be formed both from contiguous amino acids or noncontiguous amino acids juxtaposed by tertiary folding of a protein. Epitopes formed from contiguous amino acids are typically retained on exposure to denaturing solvents, whereas epitopes formed by tertiary folding are typically lost on treatment with denaturing solvents. An epitope typically includes at least 3, 4, 5, 6, 7, 8, 9, 10, 11, 12, 13, 14 or 15 amino acids in a unique spatial conformation. Methods of determining spatial conformation of epitopes include techniques in the art, for example, x-ray crystallography and 2-dimensional nuclear magnetic resonance (*see, e.g.,* Epitope Mapping Protocols in Methods in Molecular Biology, Vol. 66, G. E. Morris, Ed. (1996)).

The term "affinity" as used herein refers to the strength of interaction between antibody and antigen at single antigenic sites. Within each antigenic site, the variable region of the antibody "arm" interacts through weak non-covalent forces with antigen at numerous sites; the more interactions, the stronger the affinity.

The term "isolated antibody" refers to an antibody that is substantially free of other antibodies having different antigenic specificities. An isolated antibody that specifically binds to one antigen may, however, have cross-reactivity to other antigens. Moreover, an isolated antibody may be substantially free of other cellular material and/or chemicals.

The term "corresponding human germline sequence" refers to the nucleic acid sequence encoding a human variable region amino acid sequence or subsequence that shares the highest determined amino acid sequence identity with a reference variable region amino acid sequence or subsequence in comparison to all other all other known variable region amino acid sequences encoded by human germline immunoglobulin variable region sequences. The corresponding human germline sequence can also refer to the human variable region amino acid sequence or subsequence with the highest amino acid sequence identity with a reference variable region amino acid sequence or subsequence in comparison to all other evaluated variable region amino acid sequences. The corresponding human germline sequence can be framework regions only, complementarity determining regions only, framework and complementarity determining regions, a variable segment (as defined above), or other combinations of sequences or subsequences that comprise a variable region. Sequence identity can be determined using the methods described herein, for example, aligning two sequences using BLAST, ALIGN, or another alignment algorithm known in the art. The corresponding human germline nucleic acid or amino acid sequence can have at least about 90%, 91, 92%, 93%, 94%, 95%, 96%, 97%, 98%, 99%, or 100% sequence identity with the reference variable region nucleic acid or amino acid sequence. Corresponding human germline sequences can be determined, for example, through the publicly available international ImMunoGeneTics database (IMGT) (on the worldwide web at www.imgt.org/) and V-base (on the worldwide web at vbase.mrc-cpe.cam.ac.uk).

The phrase "specifically binds" or "selectively binds," when used in the context of describing the interaction between an antigen (*e.g.,* a protein) and an antibody, antibody fragment, or antibody-derived binding agent, refers to a binding reaction that is determinative of the presence of the antigen in a heterogeneous population of proteins and other biologics, *e.g.,* in a biological sample, *e.g.,* a blood, serum, plasma or tissue sample. Thus, under certain designated immunoassay conditions, the antibodies or binding agents with a particular binding specificity bind to a particular antigen at least two times the background and do not substantially bind in a significant amount to other antigens present in the sample. In one embodiment, under designated immunoassay conditions, the antibody or binding agent with a particular binding specificity binds to a particular antigen at least ten (10) times the background and does not substantially bind in a significant amount to other antigens present in the sample. Specific binding to an antibody or binding agent under such conditions may require the antibody or agent to have been selected for its specificity for a particular protein. As desired or appropriate, this selection may be achieved by subtracting out antibodies that cross-react with molecules from other species (*e.g.*, mouse or rat) or other subtypes. Alternatively, in some embodiments, antibodies or antibody fragments are selected that cross-react with certain desired molecules.

A variety of immunoassay formats may be used to select antibodies specifically immunoreactive with a particular protein. For example, solid-phase ELISA immunoassays are routinely used to select antibodies specifically immunoreactive with a protein *(see, e.g.,* Harlow & Lane, Using Antibodies, A Laboratory Manual (1998), for a description of immunoassay formats and conditions that can be used to determine specific immunoreactivity). Typically a specific or selective binding reaction will produce a signal at least twice over the background signal and more typically at least 10 to 100 times over the background.

The term "equilibrium dissociation constant (KD, M)" refers to the dissociation rate constant (kd, time-1) divided by the association rate constant (ka, time-1, M-1). Equilibrium dissociation constants can be measured using any known method in the art. The antibodies of the present disclosure generally will have an equilibrium dissociation constant of less than about 10⁻⁷ or 10⁻⁸ M, for example, less than about 10⁻⁹ M or 10⁻¹⁰ M, in some embodiments, less than about 10⁻¹¹ M, 10⁻¹² M or 10⁻¹³ M.

The term "bioavailability" refers to the systemic availability (*i.e.,* blood/plasma levels) of a given amount of drug administered to a patient. Bioavailability is an absolute term that indicates measurement of both the time (rate) and total amount (extent) of drug that reaches the general circulation from an administered dosage form.

As used herein, the phrase "consisting essentially of" refers to the genera or species of active pharmaceutical agents included in a method or composition, as well as any excipients inactive for the intended purpose of the methods or compositions. In some embodiments, the phrase "consisting essentially of" expressly excludes the inclusion of one or more additional active agents other than an antibody drug conjugate of the disclosure. In some embodiments, the phrase "consisting essentially of" expressly excludes the inclusion of one or more additional active agents other than an antibody drug conjugate of the disclosure and a second co-administered agent.

The term "amino acid" refers to naturally occurring, synthetic, and unnatural amino acids, as well as amino acid analogs and amino acid mimetics that function in a manner similar to the naturally occurring amino acids. Naturally occurring amino acids are those encoded by the genetic code, as well as those amino acids that are later modified, *e.g.,* hydroxyproline, γ-carboxyglutamate, and O-phosphoserine. Amino acid analogs refer to compounds that have the same basic chemical structure as a naturally occurring amino acid, *i.e.,* an α-carbon that is bound to a hydrogen, a carboxyl group, an amino group, and an R group, *e.g*., homoserine, norleucine, methionine sulfoxide, methionine methyl sulfonium. Such analogs have modified R groups (*e.g.*, norleucine) or modified peptide backbones, but retain the same basic chemical structure as a naturally occurring amino acid. Amino acid mimetics refers to chemical compounds that have a structure that is different from the general chemical structure of an amino acid, but that functions in a manner similar to a naturally occurring amino acid.

The term "conservatively modified variant" applies to both amino acid and nucleic acid sequences. With respect to particular nucleic acid sequences, conservatively modified variants refer to those nucleic acids which encode identical or essentially identical amino acid sequences, or where the nucleic acid does not encode an amino acid sequence, to essentially identical sequences. Because of the degeneracy of the genetic code, a large number of functionally identical nucleic acids encode any given protein. For instance, the codons GCA, GCC, GCG and GCU all encode the amino acid alanine. Thus, at every position where an alanine is specified by a codon, the codon can be altered to any of the corresponding codons described without altering the encoded polypeptide. Such nucleic acid variations are "silent variations," which are one species of conservatively modified variations. Every nucleic acid sequence herein which encodes a polypeptide also describes every possible silent variation of the nucleic acid. One of skill will recognize that each codon in a nucleic acid (except AUG, which is ordinarily the only codon for methionine, and TGG, which is ordinarily the only codon for tryptophan) can be modified to yield a functionally identical molecule. Accordingly, each silent variation of a nucleic acid that encodes a polypeptide is implicit in each described sequence.

For polypeptide sequences, "conservatively modified variants" include individual substitutions, deletions or additions to a polypeptide sequence which result in the substitution of an amino acid with a chemically similar amino acid. Conservative substitution tables providing functionally similar amino acids are well known in the art. Such conservatively modified variants are in addition to and do not exclude polymorphic variants, interspecies homologs, and alleles of the disclosure. The following eight groups contain amino acids that are conservative substitutions for one another: 1) Alanine (A), Glycine (G); 2) Aspartic acid (D), Glutamic acid (E); 3) Asparagine (N), Glutamine (Q); 4) Arginine (R), Lysine (K); 5) Isoleucine (I), Leucine (L), Methionine (M), Valine (V); 6) Phenylalanine (F), Tyrosine (Y), Tryptophan (W); 7) Serine (S), Threonine (T); and 8) Cysteine (C), Methionine (M) (*see, e.g.,* Creighton, Proteins (1984)). In some embodiments, the term "conservative sequence modifications" are used to refer to amino acid modifications that do not significantly affect or alter the binding characteristics of the antibody containing the amino acid sequence.

The term "optimized" as used herein refers to a nucleotide sequence that has been altered to encode an amino acid sequence using codons that are preferred in the production cell or organism, generally a eukaryotic cell, for example, a yeast cell, a Pichia cell, a fungal cell, a Trichoderma cell, a Chinese Hamster Ovary cell (CHO) or a human cell. The optimized nucleotide sequence is engineered to retain completely or as much as possible the amino acid sequence originally encoded by the starting nucleotide sequence, which is also known as the "parental" sequence.

The terms "percent identical" or "percent identity," in the context of two or more nucleic acids or polypeptide sequences, refers to the extent to which two or more sequences or subsequences that are the same. Two sequences are "identical" if they have the same sequence of amino acids or nucleotides over the region being compared. Two sequences are "substantially identical" if two sequences have a specified percentage of amino acid residues or nucleotides that are the same (*i.e.,* 60% identity, optionally 65%, 70%, 75%, 80%, 85%, 90%, 95%, or 99% identity over a specified region, or, when not specified, over the entire sequence), when compared and aligned for maximum correspondence over a comparison window, or designated region as measured using one of the following sequence comparison algorithms or by manual alignment and visual inspection. Optionally, the identity exists over a region that is at least about 30 nucleotides (or 10 amino acids) in length, or more preferably over a region that is 100 to 500 or 1000 or more nucleotides (or 20, 50, 200 or more amino acids) in length.

For sequence comparison, typically one sequence acts as a reference sequence, to which test sequences are compared. When using a sequence comparison algorithm, test and reference sequences are entered into a computer, subsequence coordinates are designated, if necessary, and sequence algorithm program parameters are designated. Default program parameters can be used, or alternative parameters can be designated. The sequence comparison algorithm then calculates the percent sequence identities for the test sequences relative to the reference sequence, based on the program parameters.

A "comparison window", as used herein, includes reference to a segment of any one of the number of contiguous positions selected from the group consisting of from 20 to 600, usually about 50 to about 200, more usually about 100 to about 150 in which a sequence may be compared to a reference sequence of the same number of contiguous positions after the two sequences are optimally aligned. Methods of alignment of sequences for comparison are well known in the art. Optimal alignment of sequences for comparison can be conducted, *e.g*., by the local homology algorithm of Smith and Waterman, Adv. Appl. Math. 2:482c (1970), by the homology alignment algorithm of Needleman and Wunsch, J. Mol. Biol. 48:443 (1970), by the search for similarity method of Pearson and Lipman, Proc. Natl. Acad. Sci. USA 85:2444 (1988), by computerized implementations of these algorithms (GAP, BESTFIT, FASTA, and TFASTA in the Wisconsin Genetics Software Package, Genetics Computer Group, 575 Science Dr., Madison, WI), or by manual alignment and visual inspection (*see*, *e.g.*, Brent et al., Current Protocols in Molecular Biology, 2003).

Two examples of algorithms that are suitable for determining percent sequence identity and sequence similarity are the BLAST and BLAST 2.0 algorithms, which are described in Altschul et al., Nuc. Acids Res. 25:3389-3402, 1977; and Altschul et al., J. Mol. Biol. 215:403-410, 1990, respectively. Software for performing BLAST analyses is publicly available through the National Center for Biotechnology Information. This algorithm involves first identifying high scoring sequence pairs (HSPs) by identifying short words of length W in the query sequence, which either match or satisfy some positive-valued threshold score T when aligned with a word of the same length in a database sequence. T is referred to as the neighborhood word score threshold (Altschul *et al.*, *supra*). These initial neighborhood word hits act as seeds for initiating searches to find longer HSPs containing them. The word hits are extended in both directions along each sequence for as far as the cumulative alignment score can be increased. Cumulative scores are calculated using, for nucleotide sequences, the parameters M (reward score for a pair of matching residues; always > 0) and N (penalty score for mismatching residues; always < 0). For amino acid sequences, a scoring matrix is used to calculate the cumulative score. Extension of the word hits in each direction are halted when: the cumulative alignment score falls off by the quantity X from its maximum achieved value; the cumulative score goes to zero or below, due to the accumulation of one or more negative-scoring residue alignments; or the end of either sequence is reached. The BLAST algorithm parameters W, T, and X determine the sensitivity and speed of the alignment. The BLASTN program (for nucleotide sequences) uses as defaults a word length (W) of 11, an expectation (E) or 10, M=5, N=-4 and a comparison of both strands. For amino acid sequences, the BLASTP program uses as defaults a word length of 3, and expectation (E) of 10, and the BLOSUM62 scoring matrix (*see* Henikoff and Henikoff, (1989) Proc. Natl. Acad. Sci. USA 89:10915) alignments (B) of 50, expectation (E) of 10, M=5, N=-4, and a comparison of both strands.

The BLAST algorithm also performs a statistical analysis of the similarity between two sequences (*see, e.g.*, Karlin and Altschul, Proc. Natl. Acad. Sci. USA 90:5873-5787, 1993). One measure of similarity provided by the BLAST algorithm is the smallest sum probability (P(N)), which provides an indication of the probability by which a match between two nucleotide or amino acid sequences would occur by chance. For example, a nucleic acid is considered similar to a reference sequence if the smallest sum probability in a comparison of the test nucleic acid to the reference nucleic acid is less than about 0.2, more preferably less than about 0.01, and most preferably less than about 0.001.

The percent identity between two amino acid sequences can also be determined using the algorithm of E. Meyers and W. Miller, Comput. Appl. Biosci. 4:11-17 (1988) which has been incorporated into the ALIGN program (version 2.0), using a PAM120 weight residue table, a gap length penalty of 12 and a gap penalty of 4. In addition, the percent identity between two amino acid sequences can be determined using the Needleman and Wunsch, J. Mol. Biol. 48:444-453 (1970) algorithm which has been incorporated into the GAP program in the GCG software package (available at www.gcg.com), using either a BLOSUM62 matrix or a PAM250 matrix, and a gap weight of 16, 14, 12, 10, 8, 6, or 4 and a length weight of 1, 2, 3, 4, 5, or 6.

Other than percentage of sequence identity noted above, another indication that two nucleic acid sequences or polypeptides are substantially identical is that the polypeptide encoded by the first nucleic acid is immunologically cross reactive with the antibodies raised against the polypeptide encoded by the second nucleic acid, as described below. Thus, a polypeptide is typically substantially identical to a second polypeptide, for example, where the two peptides differ only by conservative substitutions. Another indication that two nucleic acid sequences are substantially identical is that the two molecules or their complements hybridize to each other under stringent conditions, as described below. Yet another indication that two nucleic acid sequences are substantially identical is that the same primers can be used to amplify the sequence.

The term "nucleic acid" is used herein interchangeably with the term "polynucleotide" and refers to deoxyribonucleotides or ribonucleotides and polymers thereof in either single- or doublestranded form. The term encompasses nucleic acids containing known nucleotide analogs or modified backbone residues or linkages, which are synthetic, naturally occurring, and non-naturally occurring, which have similar binding properties as the reference nucleic acid, and which are metabolized in a manner similar to the reference nucleotides. Examples of such analogs include, without limitation, phosphorothioates, phosphoramidates, methyl phosphonates, chiral-methyl phosphonates, 2-O-methyl ribonucleotides, peptide-nucleic acids (PNAs).

Unless otherwise indicated, a particular nucleic acid sequence also implicitly encompasses conservatively modified variants thereof (e.g., degenerate codon substitutions) and complementary sequences, as well as the sequence explicitly indicated. Specifically, as detailed below, degenerate codon substitutions may be achieved by generating sequences in which the third position of one or more selected (or all) codons is substituted with mixed-base and/or deoxyinosine residues (Batzer et al., (1991) Nucleic Acid Res. 19:5081; Ohtsuka et al., (1985) J. Biol. Chem. 260:2605-2608; and Rossolini et al., (1994) Mol. Cell. Probes 8:91-98).

The term "operably linked" in the context of nucleic acids refers to a functional relationship between two or more polynucleotide (*e.g*., DNA) segments. Typically, it refers to the functional relationship of a transcriptional regulatory sequence to a transcribed sequence. For example, a promoter or enhancer sequence is operably linked to a coding sequence if it stimulates or modulates the transcription of the coding sequence in an appropriate host cell or other expression system. Generally, promoter transcriptional regulatory sequences that are operably linked to a transcribed sequence are physically contiguous to the transcribed sequence, *i.e.*, they are cis-acting. However, some transcriptional regulatory sequences, such as enhancers, need not be physically contiguous or located in close proximity to the coding sequences whose transcription they enhance.

The terms "polypeptide" and "protein" are used interchangeably herein to refer to a polymer of amino acid residues. The terms apply to amino acid polymers in which one or more amino acid residue is an artificial chemical mimetic of a corresponding naturally occurring amino acid, as well as to naturally occurring amino acid polymers and non-naturally occurring amino acid polymer. Unless otherwise indicated, a particular polypeptide sequence also implicitly encompasses conservatively modified variants thereof.

The term "antibody drug conjugate" or "immunoconjugate" as used herein refers to the linkage of an antibody or an antigen binding fragment thereof with another agent, such as a chemotherapeutic agent, a toxin, an immunotherapeutic agent, an imaging probe, and the like. The linkage can be covalent bonds, or non-covalent interactions such as through electrostatic forces. Various linkers, known in the art, can be employed in order to form the antibody drug conjugate. Additionally, the antibody drug conjugate can be provided in the form of a fusion protein that may be expressed from a polynucleotide encoding the immunoconjugate. As used herein, "fusion protein" refers to proteins created through the joining of two or more genes or gene fragments which originally coded for separate proteins (including peptides and polypeptides). Translation of the fusion gene results in a single protein with functional properties derived from each of the original proteins.

The term "subject" includes human and non-human animals. Non-human animals include all vertebrates, *e.g*., mammals and non-mammals, such as non-human primates, sheep, dog, cow, chickens, amphibians, and reptiles. Except when noted, the terms "patient" or "subject" are used herein interchangeably.

The term "cytotoxin", or "cytotoxic agent" as used herein, refers to any agent that is detrimental to the growth and proliferation of cells and may act to reduce, inhibit, or destroy a cell or malignancy.

The term "anti-cancer agent" as used herein refers to any agent that can be used to treat or prevent a cell proliferative disorder such as cancer, including but not limited to, cytotoxic agents, chemotherapeutic agents, radiotherapy and radiotherapeutic agents, targeted anti-cancer agents, and immunotherapeutic agents.

The term "drug moiety" or "payload" as used herein refers to a chemical moiety that is conjugated to an antibody or antigen binding fragment of the disclosure, and can include any therapeutic or diagnostic agent, for example, an anti-cancer, anti-inflammatory, anti-infective (*e.g*., anti-fungal, antibacterial, anti-parasitic, anti-viral), or an anesthetic agent. For example, the drug moiety can be an anti-cancer agent, such as a cytotoxin. In certain embodiments, a drug moiety is a target inhibitor compound. In addition, a payload can be a biophysical probe, a fluorophore, a spin label, an infrared probe, an affinity probe, a chelator, a spectroscopic probe, a radioactive probe, a lipid molecule, a polyethylene glycol, a polymer, a spin label, DNA, RNA, a protein, a peptide, a surface, an antibody, an antibody fragment, a nanoparticle, a quantum dot, a liposome, a PLGA particle, a saccharide or a polysaccharide.

In some embodiments, the drug moiety or payload is a GNAQ inhibitor, a GNA11 inhibitor or an inhibitor of GNAQ and GNA11 (GNAQ/GNA11 inhibitor). In some embodiments, a GNAQ/11 inhibitor is a molecule that inhibits GNAQ/11-mediated production of IP3 and/or exhibits a dose-response antiproliferative effect in cells dependent on GNAQ/11 signaling (*i.e*., GNAQ/11 mutant uveal melanoma cells). In some embodiments, a GNAQ/11 inhibitor is a compound that stabilizes GNAQ/11 in the inactive GDP-bound state and prevents GDP release, or binds to the active GTP-bound state and prevents GNAQ/11 interaction with downstream effectors. In some embodiments, a GNAQ/11 inhibitor functions by inhibiting a mutant GNAQ and/or GNA11, such as one comprising a Q209L/P mutation. Methods for attaching such drug moieties to a linker compatible with the targeting moiety are given in the present disclosure, along with the methods known in the art. See, *e.g.*, Singh et al., (2009) Therapeutic Antibodies: Methods and Protocols, vol. 525, 445-457.

GNAQ (Guanine nucleotide-binding protein G(q) subunit alpha, also known as CMC1, G-ALPHA-q, GAQ, SWS, and G protein subunit alpha q) and GNA11 (Guanine nucleotide-binding protein subunit alpha-11, also known as FBH, FBH2, FHH2, GNA-11, HHC2, HYPOC2, and G protein subunit alpha 11) are closely related GTPases that constitute α subunits of heterotrimeric G proteins acting downstream of G protein-coupled receptors (GPCRs). The α subunits act as a switch for activation of G proteins by exchanging the guanosine diphosphate (GDP) for guanosine triphosphate (GTP), leading to the activation of distinct downstream effectors. The activation is terminated by the intrinsic GTPase activity, as GTP is hydrolyzed to GDP (Van Raamsdonk et al., 2010, N Engl J Med.; 363(23):2191-9). The classical activation of Gq protein cascade occurs via phospholipase C-β (PLC-β), which hydrolyses phospholipid phosphatidylinositol 4,5-biphosphate to release two potent second messengers: D-myo-inositol 1,4,5-triphosphate (IP3) and diacylglycerol (DAG). Following the transient increase of intracellular Ca²⁺, IP3 is rapidly transformed into IP2, IP1, and myo-inositol. On the other hand, DAG activates protein kinase C (PKC), leading to a cascade of phosphorylation of RAF, MEK, and ERK, which translocate to the nucleus to regulate cell proliferation and survival (Krantz et al., 2017, Clin Ophthalmol.; 11:279-289).

Exemplary amino acid and nucleotide sequences of human GNAQ have been published in GenBank with Accession Nos. NP_002063 and NM_002072, respectively. Exemplary amino acid and nucleotide sequences of human GNAQ are also described in International Application Publication No. WO 2020/128612 as SEQ ID NOs: 268 and 269, respectively.

Exemplary amino acid and nucleotide sequences of human GNA11 have been published in GenBank with Accession Nos. NP_002058 and NM_002067, respectively. Exemplary amino acid and nucleotide sequences of human GNA11 are also described in International Application Publication No. WO 2020/128612 as SEQ ID NOs: 270 and 271, respectively.

"Tumor" refers to neoplastic cell growth and proliferation, whether malignant or benign, and all pre-cancerous and cancerous cells and tissues.

The term "anti-tumor activity" means a reduction in the rate of tumor cell proliferation, viability, or metastatic activity. For example, anti-tumor activity can be shown by a decline in growth rate of abnormal cells that arises during therapy or tumor size stability or reduction, or longer survival due to therapy as compared to control without therapy. Such activity can be assessed using accepted in vitro or in vivo tumor models, including but not limited to xenograft models, allograft models, MMTV models, and other known models known in the art to investigate anti-tumor activity.

The term "malignancy" refers to a non-benign tumor or a cancer. As used herein, the term "cancer" includes a malignancy characterized by deregulated or uncontrolled cell growth. Exemplary cancers include, but are not limited to, carcinomas (*e.g*., hepatocellular carcinoma), sarcomas, leukemias, lymphomas, eye cancers, eye neoplasms, and melanomas (*e.g*., uveal melanoma, non-uveal melanoma, malignant melanoma, ocular melanoma, subcutaneous melanoma, cutaneous melanoma, or mucosal melanoma).

The term "cancer" includes primary malignant tumors (*e.g*., those whose cells have not migrated to sites in the subject's body other than the site of the original tumor) and secondary malignant tumors (*e.g*., those arising from metastasis, the migration of tumor cells to secondary sites that are different from the site of the original tumor).

The term "PMEL17" (also referred to as premelanosome protein (PMEL), D12S53E, ME20, ME20-M, ME20M, P1, P100, gp100, SI, SIL, and silver locus protein homolog (SILV)) refers to a single-pass Type I transmembrane protein produced by melanocytes and involved in melanin synthesis. Exemplary amino acid and nucleotide sequences of human PMEL17 have been published in GenBank with the following Accession Nos.: NP_008859, NP_001307050, NP_001307051, NP_001186982, NP_001186983 (amino acid sequences), and NM_006928, NM_001200053, NM_001200054, NM_001320121, NM_001320122 (nucleotide sequences). Exemplary amino acid and nucleotide sequences of human PMEL17 are also described in International Application Publication No. WO 2020/128612 as SEQ ID NOs: 272-276 (amino acid sequences) and SEQ ID NOs: 277-281 (nucleotide sequences). As used herein, the term "PMEL17" is used to refer collectively to all naturally occurring isoforms of PMEL17 protein, or a variant thereof.

The term "variant" refers to a polypeptide that has a substantially identical amino acid sequence to a reference polypeptide, or is encoded by a substantially identical nucleotide sequence, and is capable of having one or more activities of the reference polypeptide. For example, a variant can have about 85%, 90%, 91%, 92%, 93%, 94%, 95%, 96%, 97%, 98%, 99% or higher sequence identity to a reference polypeptide, while retain one or more activities of the reference polypeptide.

As used herein, the terms "treat," "treating," or "treatment" of any disease or disorder refers in one embodiment, to ameliorating the disease or disorder (*i.e.*, slowing or arresting or reducing the development of the disease or at least one of the clinical symptoms thereof). In another embodiment, "treat," "treating," or "treatment" refers to alleviating or ameliorating at least one physical parameter including those which may not be discernible by the patient. In yet another embodiment, "treat," "treating," or "treatment" refers to modulating the disease or disorder, either physically, (*e.g*., stabilization of a discernible symptom), physiologically, (*e.g*., stabilization of a physical parameter), or both.

As used herein, the term "prevent", "preventing" or "prevention" of any disease or disorder refers to the prophylactic treatment of the disease or disorder; or delaying the onset or progression of the disease or disorder

The term "therapeutically acceptable amount" or "therapeutically effective dose" interchangeably refers to an amount sufficient to effect the desired result (*i.e.*, a reduction in tumor size, inhibition of tumor growth, prevention of metastasis, inhibition or prevention of viral, bacterial, fungal or parasitic infection). In some embodiments, a therapeutically acceptable amount does not induce or cause undesirable side effects. In some embodiments, a therapeutically acceptable amount induces or causes side effects but only those that are acceptable by the healthcare providers in view of a patient's condition. A therapeutically acceptable amount can be determined by first administering a low dose, and then incrementally increasing that dose until the desired effect is achieved. A "prophylactically effective dosage," and a "therapeutically effective dosage," of the molecules of the disclosure can prevent the onset of, or result in a decrease in severity of, respectively, disease symptoms, including symptoms associated with cancer.

The term "co-administer" refers to the presence of two active agents in the blood of an individual. Active agents that are co-administered can be concurrently or sequentially delivered.

The term "inhibition," "inhibitor," "binding antagonist" or "antagonist" includes a reduction in a certain parameter, *e.g*., an activity, of a given molecule. For example, inhibition of an activity of a given molecule, of at least 5%, 10%, 20%, 30%, 40% or more is included by this term. Thus, inhibition need not be 100%.

The term "activation," "activator," or "agonist" includes an increase in a certain parameter, *e.g*., an activity, of a given molecule. For example, increase of an activity of a given molecule of at least 5%, 10%, 25%, 50%, 75% or more is included by this term.

### Chromobacterium

*Chromobacterium* is a genus of Gram-negative rod-shaped bacteria. Currently, eleven species within the genus are known, which are *C. alkanivorans*, *C. amazonense*, *C. aquaticum*, *C. haemolyticum, C. phragmitis*, *C. piscinae*, *C. pseudoviolaceum*, *C. rhizoryzae*, *C. subtsugae*, *C. vaccinii,* and *C. violaceum.*

In some embodiments, the *Chromobacterium* is *C. vaccinii. C. vaccinii* was isolated from soil of a cranberry tree (Soby et al. (2013) Int J Syst Evol Microbiol.;63(5):1840-6) and deposited at both the DSMZ and the ATCC strain collections (*Chromobacterium vaccinii* DSM 25150 = ATCC BAA-2314, = NRRL B-50840, = MWU205). The GenBank accession number for the 16S rRNA gene sequence of the strain is JN120869. A draft genome of *C. vaccinii* DSM 25150 was published by Vöing et al. (2015) Genome Announc 3, e00477-15 (GenBank accession number: NZ_JZJL00000000).

*C. vaccinii* grows readily on nutrient agar, producing distinctive smooth low convex colonies with a dark violet metallic sheen, due to production of violacein, upon incubation at 28 °C for 2 days. *C. vaccinii* is unlikely to cause any disease in healthy adult humans and is of minimal potential hazard to laboratory personnel and the environment.

The microorganisms described herein include, for example, a mutant form of *C. vaccinii* that naturally produces a depsipeptide, *e.g.,* compound (A1). In some embodiments, the *C. vaccinii* mutant comprise a mutation that increases the titer of compound (A1). In some embodiments, the *C. vaccinii* mutant comprises a mutation that increases the isolated yield of compound (A1). In some embodiments, the *C. vaccinii* mutant comprise a mutation that increases the titer of compound (A1) and a mutation that increases the isolated yield of compound (A1). In some embodiments, the *C. vaccinii* mutant comprises a promoter insertion mutation (*e.g*., a promoter insertion mutation described herein). In some embodiments, the *C. vaccinii* mutant comprises a disruption mutation (*e.g.*, a disruption mutation described herein). In some embodiments, the *C. vaccinii* mutant comprises a plurality of disruption mutations (*e.g*., a plurality of disruption mutations described herein). In some embodiments, the *C. vaccinii* mutant comprises a promoter insertion mutation (*e.g*., a promoter insertion mutation described herein) and a disruption mutation (*e.g*., a disruption mutation described herein). In some embodiments, the *C. vaccinii* mutant comprises a promoter insertion mutation (*e.g*., a promoter insertion mutation described herein) and a plurality of disruption mutations (*e.g*., a plurality of disruption mutations described herein).

### Promoter insertion mutants

In some embodiments, the *C. vaccinii* mutant has an increased titer of compound (A1) compared to a reference *C. vaccinii, e.g.,* a wild type *C. vaccinii,* when cultured under identical or similar conditions. For example, the native promoter upstream of the compound (A1)-BGC can be altered, *e.g.,* replaced with a non-native promoter, *e.g.,* a stronger, non-native promoter. Without wishing to be bound by theory, it is believed that the compound (A1)-BGC contains a single operon and its transcription is likely driven by a single promoter.

The non-native promoter may be any promoter sequence which is not naturally operably linked to the compound (A1)-BGC and which, when so operably linked, increases the transcriptional activity of the compound (A1)-BGC.

In some embodiments, the non-native promoter is from *C. vaccinii, e.g.,* a promoter that controls the expression of a BGC other than the compound (A1)-BGC, *e.g.,* a *vioP* promoter. In other embodiments, the non-native promoter is not from *C. vaccinii.* In some embodiments, the non-native promoter is from *E. coli.*

In some embodiments, the non-native promoter is a constitutive promoter. In some embodiments, the non-native promoter is an inducible promoter. In some embodiments, the promoter, *e.g.,* a vioP promoter, is induced by the production of homoserine lactones. In some embodiments, the non-native promoter is a violacein promoter. In some embodiments, the non-native promoter is a ribosomal promoter. In some embodiments, the non-native promoter is located in a transposon.

In certain embodiments, the non-native promoter is naturally occurring promoter. In other embodiments, the non-native promoter is a synthetic promoter. In other embodiments, the non-native promoter is a consensus promoter, *e.g*., comprising a consensus sequence of a group of related naturally occurring promoters.

In some embodiments, the non-native promoter is a *vioP* promoter. In some embodiments, the non-native promoter is a *nptII* promoter. In some embodiments, the non-native promoter is an *rbs* promoter.

Other non-native promoters include, but are not limited to, a J23119 promoter (*e.g*., as described at parts.igem.org), a pLpp promoter (*e.g*., as described at parts.igem.org), a PS12burk promoter (*e.g*., as described in Choi et al. (2008) App Environ Microbiol 74(4):1064-75), an Erme* promoter (*e.g*., as described in Bibb et al. (1994) Mol Microbiol 14(3):533-45), or a Pem7 promoter (*e.g.*, as described in Zobel et al. (2015) ACS Synthetic Biology 4:1341-51).

In some embodiments, the non-native promoter is a promoter described in **Table 1.** In some embodiments, the heterologous promoter comprises a nucleotide sequence described in **Table 1,** or a functional fragment thereof. In some embodiments, the heterologous promoter comprises the nucleotide sequence of SEQ ID NO: 264, or a sequence having at least about 50%, about 55%, about 60%, about 65%, about 70%, about 75%, about 80%, about 85%, about 90%, about 95%, about 96%, about 97%, about 98%, or about 99% identity thereto, or a functional fragment thereof. In some embodiments, the heterologous promoter comprises the nucleotide sequence of SEQ ID NO: 265, or a sequence having at least about 50%, about 55%, about 60%, about 65%, about 70%, about 75%, about 80%, about 85%, about 90%, about 95%, about 96%, about 97%, about 98%, or about 99% identity thereto, or a functional fragment thereof. In some embodiments, the heterologous promoter comprises the nucleotide sequence of SEQ ID NO: 266, or a sequence having at least about 50%, about 55%, about 60%, about 65%, about 70%, about 75%, about 80%, about 85%, about 90%, about 95%, about 96%, about 97%, about 98%, or about 99% identity thereto, or a functional fragment thereof. In some embodiments, the heterologous promoter comprises the nucleotide sequence of SEQ ID NO: 316, or a sequence having at least about 50%, about 55%, about 60%, about 65%, about 70%, about 75%, about 80%, about 85%, about 90%, about 95%, about 96%, about 97%, about 98%, or about 99% identity thereto, or a functional fragment thereof. In some embodiments, the heterologous promoter comprises the nucleotide sequence of SEQ ID NO: 268, or a sequence having at least about 50%, about 55%, about 60%, about 65%, about 70%, about 75%, about 80%, about 85%, about 90%, about 95%, about 96%, about 97%, about 98%, or about 99% identity thereto, or a functional fragment thereof. In some embodiments, the heterologous promoter comprises the nucleotide sequence of SEQ ID NO: 269, or a sequence having at least about 50%, about 55%, about 60%, about 65%, about 70%, about 75%, about 80%, about 85%, about 90%, about 95%, about 96%, about 97%, about 98%, or about 99% identity thereto, or a functional fragment thereof. In some embodiments, the heterologous promoter comprises the nucleotide sequence of SEQ ID NO: 270, or a sequence having at least about 50%, about 55%, about 60%, about 65%, about 70%, about 75%, about 80%, about 85%, about 90%, about 95%, about 96%, about 97%, about 98%, or about 99% identity thereto, or a functional fragment thereof. In some embodiments, the heterologous promoter comprises the nucleotide sequence of SEQ ID NO: 271, or a sequence having at least about 50%, about 55%, about 60%, about 65%, about 70%, about 75%, about 80%, about 85%, about 90%, about 95%, about 96%, about 97%, about 98%, or about 99% identity thereto, or a functional fragment thereof.

**Table 1. Nucleotide sequences of exemplary promoters**

| **Promoter Name** | **Nucleotide Sequence** | **SEQ ID NO.** |
|---|---|---|
| *vioP* | CGTCGTTGATCCCAGGCAGCCCTTTGTCTCCGTTTCTCCACGTCATGCCC TGACCCTTGGAACAGGATGGGCCGTCCTGTCAGACAATATGCTTGATGAA TTAGTTCGGTTGAACTAACGTGGCTGAAAATTACAAAGGCATTTAATTTT AAAAATATAAATGCCTTATAAATTTCATGCCGGGAAACCGGTCATGACGA GGATACAAGAGGCTGCATCCCGATTTCGAGGCGAGAGTGCCAAGCATTTA CGTCGTCCATGCCCGTTCGTTGTTGCCGCGCGGCGGGCGTGAATTGAACA GTCAAAGGGACATTCGCG | 264 |
| *nptII* | TGGACAGCAAGCGAACCGGAATTGCCAGCTGGGGCGCCCTCTGGTAAGGT TGGGAAGCCCTGCAAAGTAAACTGGATGGCTTTCTTGCCGCCAAGGATCT GATGGCGCAGGGGATCAAGATCTGATCAAGAGACAGGATAAGGAGGTACA GATTCAT | 265 |
| *rbs* | CACCGGCCTGACGGCCAAGTGTTGAAAAAACGCTGCTCCATCAACGGTTA ACGTTGGCGGGGCGGCGTTTTTTATTTGACGCTGGGCCGTTGACTAAAAT ATAATCCTCCGTCCTTTCTGGAGGCGTGTTGTCTTCGGGAAGAATCAACT AGGAACTGATAGTA | 266 |
| *J23119* | TTGACAGCTAGCTCAGTCCTAGGTATAATGCTAGC | 316 |
| *pLpp* | ATCAAAAAAATATTCTCAACATAAAAAACTTTGTGTAATACTTGTAACG | 268 |
| *Pem7* | GTTGACAATTAATCATCGGCATAGTATATCGGCATAGTATAATACGACAA GGTGAGGAACTAAACCATG | 269 |
| *PS12burk* | GAGCTGTTGACTCGCTTGGGATTTTCGGAATATCATGCCGGGTGGCCCG GGAAAGCCACGTTGTGTCTCAAAATCTCTGATGTTACATTGCACAAGATA AAAATATATCATCATGAACAATAAAACTGTCTGCTTACATAAACAGTAAT ACAAGGGGTGTT | 270 |
| *ErmE** | GGATCCGACGTCCATGCGAGTGTCCGTTCGAGTGGCGGCTTGCGCCCGAT GCTAGTCGCGGTTGATCGGCGATCGCAGGTGCACGCGGTCCATCTTGACG GCTGGCGAGAGGTGCGGGGAGGATCTGACCGACGCGGTCCACACGTGGCA CCGCGATGCTGTTGACAGCCAATCGTGCCGGTTGGTAGAATACAGAACCA CTCCACA | 271 |

For example, the strain *Chromobacterium vaccinii vioP-frsA* is a mutant generated from strain *Chromobacterium vaccinii* DSM 25150 by insertion of the promoter *vioP* in front of the compound (A1)-BGC, creating a translational fusion. Promoter *vioP* was extracted from the strain's inherent violacein BGC. The nucleotide sequence of promoter *vioP* is shown in **Table 1.** The generation of this mutant is described in Example 1.

The violacein promoter from *C. vaccinii* and the one from *C. violaceum* same essentially the same function and about 82% sequence identify. The vioP promoter from *C. violaceum* is described, *e.g.,* in Morohoshi et al. (2010) Bioscie. Biotechnol. Biochem.;74 (10),2116-2119 and Zhang et al. (2011) Appl Microbiol Biotechnol;90:1963-1971.

As another example, the strain *Chromobacterium vaccinii nptII-frsA* is a mutant generated from strain *Chromobacterium vaccinii* DSM 25150 by insertion of the promoter *nptII* in front of the compound (A1)-BGC, creating a translational fusion. Promoter *nptII* is a constitutive promoter. The nucleotide sequence of promoter *nptII* is shown in **Table 1.** The generation of this mutant IS described in Example 1.

As yet another example, the strain *Chromobacterium vaccinii rbs-frsA* is a mutant generated from strain *Chromobacterium vaccinii* DSM 25150 by insertion of the promoter *rbs* in front of the Compound (A1)-BGC, creating a translational fusion. The ribosomal promoter *rbs* is a constitutive promoter. The nucleotide sequence of promoter *rbs* is shown in **Table 1.** The generation of this mutant is described in Example 1.

As still another example, promoter *vioP, nptII* or *rbs* can be replaced with promoter *J23119, P_{S12}burk, Erme*,* or *pLpp.* Promoter *J23119* is a synthetic promoter. The nucleotide sequence of promoter *J23119* is shown in **Table 1.** Promoter *P_{S12}burk* is a ribosomal promoter from *Burkholderia thailandensis.* The nucleotide sequence of promoter *P_{S12}burk* is shown in **Table 1.** Promoter *P_{S12}burk* is also described in Choi et al. (2008) App Environ Microbiol 74(4):1064-75). *P_{S12}burk* may also be known as *S12burk.* Promoter *ErmE** is a 23S rRNA methylase promoter from *Saccharopolyspora erythraea.* The nucleotide sequence of promoter *ErmE** is shown in **Table 1.** Promoter *ErmE** is also described in described in Bibb et al. (1994) Mol Microbiol 14(3):533-45. Promoter *pLpp* is a synthetic promoter. The nucleotide sequence of promoter *pLpp* is shown in **Table 1.** Promoter *pLpp* is also described in parts.igem.org. *pLpp* may also be known as *PLpp.*

In some embodiments, the titer of compound (A1) of the *C. vaccinii* promoter insertion mutant is increased by at least about 1, 2, 3, 4, 5, 6, 7, 8, 9, or 10-fold, compared to the titer of a reference *C. vaccinii, e.g.,* a *C. vaccinii* that does not have the promoter insertion mutation in the compound (A1)-BGC or a wild type *C. vaccinii,* when cultured under identical conditions. In some embodiments, the titer of compound (A1) of the *C. vaccinii* promoter insertion mutant is increased at least about 3-fold, *e.g.*, at least about 3.5-fold, compared to the titer of compound (A1) of a wild type *C. vaccinii.*

In some embodiments, the titer of compound (A1) of the *C. vaccinii* promoter insertion mutant is increased by at least about 100 mg/L, about 200 mg/L, about 300 mg/L, about 400 mg/L, about 500 mg/L, about 600 mg/L, about 700 mg/L, about 800 mg/L, about 900 mg/L, or about 1000 mg/L, compared to the titer of a reference *C. vaccinii, e.g.*, a *C. vaccinii* that does not have the promoter insertion mutation in the compound (A1)-BGC or a wild type *C. vaccinii,* when cultured under identical conditions. In some embodiments, the titer of compound (A1) of the *C. vaccinii* promoter insertion mutant is increased, *e.g*., from no more than about 300 mg/L to at least about 600 mg/L or at least about 800 mg/L, compared to the titer of compound (A1) of a wild type *C. vaccinii.* In some embodiments, the titer of compound (A1) of the *C. vaccinii* promoter insertion mutant is at least about 200 mg/L, about 250 mg/L, about 300 mg/L, about 350 mg/L, about 400 mg/L, about 450 mg/L, about 500 mg/L, about 550 mg/L, about 600 mg/L, about 650 mg/L, about 700 mg/L, about 750 mg/L, about 800 mg/L, about 850 mg/L, about 900 mg/L, about 950 mg/L, about 1000 mg/L, about 1100 mg/L, about 1200 mg/L, about 1300 mg/L, about 1400 mg/L, about 1500 mg/L, about 1600 mg/L, about 1700 mg/L, about 1800 mg/L, about 1900 mg/L, or about 2000 mg/L. In some embodiments, the titer of compound (A1) of the *C. vaccinii* mutant is at least about 800 mg/L, *e.g.*, about 800 mg/L to about 850 mg/L. In some embodiment, the titer of compound (A1) of the *C. vaccinii* promoter insertion mutant is at least about 600 mg/L, *e.g.*, about 600 mg/L to about 650 mg/L, *e.g*., when cultured in an animal component-free medium.

The *C. vaccinii* promoter insertion mutants described herein can be used to produce compound (A1) in a small scale (*e.g.*, a research scale) or in a large scale (*e.g.*, a production scale). In some embodiments, compound (A1) is produced in a fermenter or a bioreactor. In some embodiments, compound (A1) is produced in a fermentation volume of about 50 L to about 250 L, *e.g*., about 50 L to about 100 L, about 100 L to about 150 L, or about 150 L to about 250 L, *e.g*., about 50 L, about 100 L, about 150 L, about 200 L, or about 250 L. In some embodiments, compound (A1) is produced in a fermentation volume of about 1,000 L to about 20,000 L, *e.g*., about 1,000 L to about 10,000 L or about 10,000 L to about 20,000 L, *e.g*., about 1,000 L, about 2,000 L, about 5,000 L, about 7,500 L, about 10,000 L, about 15,000 L, or about 20,000 L.

### Disruption mutants

Alternatively, or in combination, in some embodiments, the *C. vaccinii* mutant has an increased isolate yield of compound (A1) compared to the wild type *C. vaccinii* when cultured under identical conditions. For example, the level or activity of another natural product produced by *C. vaccinii* can be reduced, *e.g.*, the BGC of such natural product can be disrupted. Without wishing to be bound by theory, it is believed that in addition to compound (A1) and its analogs, *C. vaccinii* produces at least two additional natural products classes, which interfere to a certain extent with the purification of compound (A1). These belong to the violacein-class and a class of cyclic lipodepsipeptides, with compound J being the most abundant representative. In some embodiments, the isolate yield of compound (A1) can be increased when the complexity of the byproducts that interferes with the purification of compound (A1) is decreased.

In some embodiments, the BGC of violacein is altered, *e.g*., disrupted, or one or more genes in the violacein-BGC knocked out. In some embodiments, the level or activity of violacein is reduced, *e.g*., eliminated.

Violacein is a purple pigment of bis-indole structure that is common to most *Chromobacteria* and even accountable for the naming of this genus. Violacein has a chemical structure of:

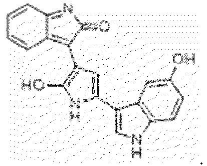

Its biosynthesis has been elucidated and the respective BGC is described, *e.g.*, in August et al. (2000) J Mol Microbiol Biotechnol.; 2(4):513-519. The high amounts of violacein, present in the fermentation broth, are troublesome during the extraction process due to precipitation at every evaporation step and the time and effort required for subsequent solubilization. Therefore, a size-exclusion chromatography can be introduced as first purification step, to remove violacein and avoid further precipitation.

In some embodiments, the BGC of compound J is altered, *e.g*., disrupted, or one or more genes in the compound J-BGC knocked out. For example, the production one or more (*e.g*., two, three, four, or more) compounds from the compound J-BGC (*e.g*., any one, two, three, or all of compound J, compound F5, compound F3, or compound D) is reduced, *e.g*., eliminated. In some embodiments, the production of compound J, compound F5, compound F3, and compound D is reduced, *e.g*., eliminated.

In some embodiments, the level or activity of Compound J is reduced, *e.g*., eliminated. In some embodiments, the BGC of Compound J is altered, *e.g*., disrupted, or one or more genes in the Compound J-BGC knocked out.

Compound J has a chemical structure of:

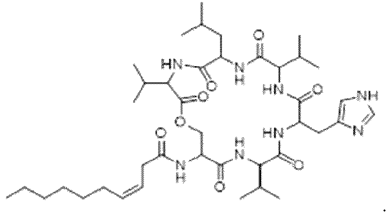

A retrobiosynthetic analysis of Compound J (cyclic hexadepsipeptide of biosynthetic sequence: N-Acyl-Ser-Val-His-Val-Leu-Val (SEQ ID NO: 318)) allowed for identification of its BGC from the sequenced genome of *C*. *vaccinii.* Upon size-exclusion chromatography members of the Compound J class co-elute in the Compound (A1)-rich fractions and cause upon solubilization for further purification by preparative HPLC a very high viscosity (almost gel-like) hampering the injection onto the column. Consequently, the fractions typically need further dilution and the compound amounts to be processed per run are limited.

In some embodiments, the level or activity of Compound F5 is reduced, *e.g.*, eliminated.

Compound F5 has a chemical structure of:

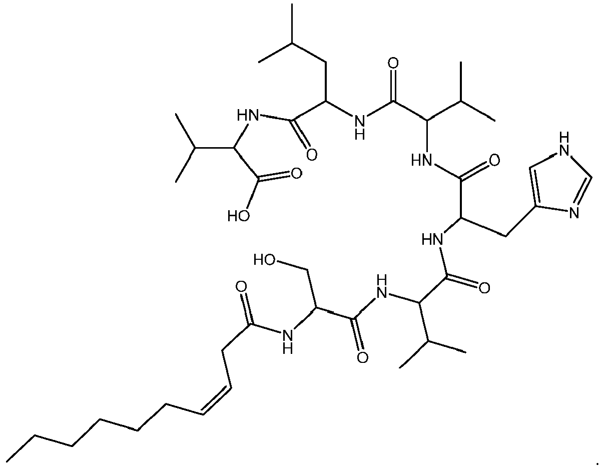

Compound F5 is a linear lipopeptide of the sequence *N*-(Z)-dec-3-enoyl-¹Ser-²Val-³His-⁴Val-⁵Leu-⁶Val (SEQ ID NO: 321). In comparison to compound J, the lactone between the ¹Ser hydroxyl group and the C-terminal carboxyl group of ⁶Val has been hydrolyzed.

In some embodiments, the level or activity of Compound F3 is reduced, *e.g*., eliminated.

Compound F3 has a chemical structure of:

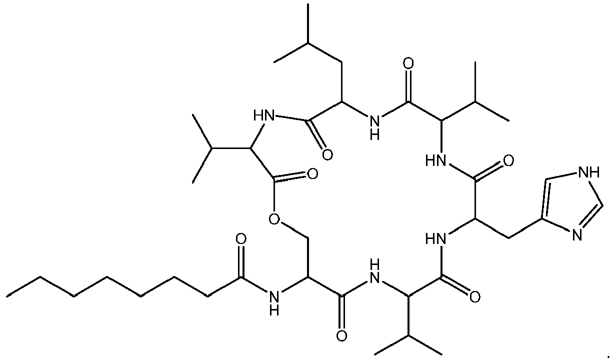

Compound F3 is a cyclic lipodepsipeptide of the sequence *N*-octanoyl-¹Ser-²Val-³His-⁴Val-⁵Leu-⁶Val (SEQ ID NO: 322). The hexapeptide lactone ring is formed between the ¹Ser hydroxyl group and the C-terminal carboxyl group of ⁶Val.

In some embodiments, the level or activity of Compound D is reduced, *e.g*., eliminated.

Compound D has a chemical structure of:

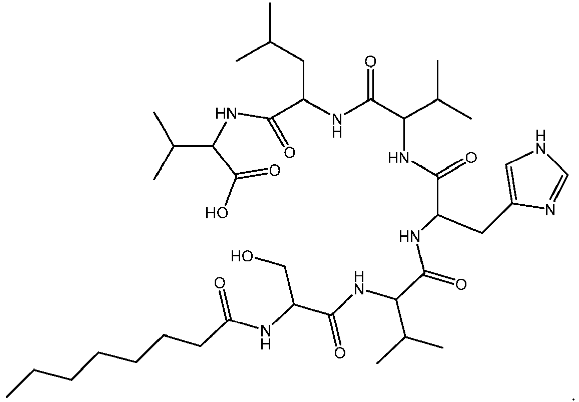

Compound D is a linear lipopeptide of the sequence N-octanyl-' Ser-²Val-³His-⁴Val-⁵Leu-⁶Val (SEQ ID NO: 323). In comparison to compound F3 the lactone between the ¹Ser hydroxyl group and the C-terminal carboxyl group of ⁶Val has been hydrolyzed.

In some embodiments, the *C. vaccinii* disruption mutant has two or more (*e.g*., three or four) disruption mutations. For example, both violacein-BGC and compound J-BGC can be altered, *e.g.*, disrupted.

In some embodiments, the isolated yield of compound (A1) of the *C. vaccinii* disruption mutant is increased by at least about 10%, about 20%, about 30%, about 40%, about 50%, about 60%, about 70%, about 80%, or about 90%, or at least about 1, about 2, about 3, about 4, or about 5-fold, compared to the isolated yield of a reference *C. vaccinii, e.g.*, a *C. vaccinii* that does not have the disruption mutation or a wild type *C. vaccinii,* when cultured under identical conditions. In some embodiments, the isolated yield of compound (A1) of the *C. vaccinii* disruption mutant is at least about 60%, *e.g.*, about 65% to about 70%. In some embodiments, the isolated yield of Compound (A1) of the wild type *C. vaccinii* is about 35% to about 45%, *e.g.*, about 40%.

In some embodiments, the titer of compound (A1) of the *C. vaccinii* disruption mutant differs by no more than about 50%, about 40%, about 35%, about 30%, about 25%, about 20%, about 15%, or about 10%, compared to the titer of a reference *C. vaccinii, e.g.*, a *C. vaccinii* that does not have the disruption mutation or a wild type *C. vaccinii,* when cultured under identical conditions. In some embodiments, the titer of compound (A1) of the *C. vaccinii* disruption mutant is at least about 200 mg/L, about 250 mg/L, about 300 mg/L, about 350 mg/L, about 400 mg/L, about 450 mg/L, about 500 mg/L, about 550 mg/L, about 600 mg/L, about 650 mg/L, about 700 mg/L, about 750 mg/L, about 800 mg/L, about 850 mg/L, about 900 mg/L, about 950 mg/L, about 1000 mg/L, about 1100 mg/L, about 1200 mg/L, about 1300 mg/L, about 1400 mg/L, about 1500 mg/L, about 1600 mg/L, about 1700 mg/L, about 1800 mg/L, about 1900 mg/L, or about 2000 mg/L. In some embodiments, the titer of compound (A1) of the *C. vaccinii* disruption mutant is at least about 800 mg/L, *e.g.*, about 800 mg/L to about 850 mg/L. In some embodiment, the titer of compound (A1) of the *C. vaccinii* disruption mutant is at least about 600 mg/L, *e.g.*, about 600 mg/L to about 650 mg/L, *e.g.*, when cultured in an animal component-free medium.

The *C. vaccinii* disruption mutants described herein can be used to produce compound (A1) in a small scale (*e.g.*, a research scale) or in a large scale (*e.g.*, a production scale). In some embodiments, compound (A1) is produced in a fermenter or a bioreactor. In some embodiments, compound (A1) is produced in a fermentation volume of about 50 L to about 250 L, *e.g*., about 50 L to about 100 L, about 100 L to about 150 L, or about 150 L to about 250 L, *e.g*., about 50 L, about 100 L, about 150 L, about 200 L, or about 250 L. In some embodiments, compound (A1) is produced in a fermentation volume of about 1,000 L to about 20,000 L, *e.g*., about 1,000 L to about 10,000 L or about 10,000 L to about 20,000 L, *e.g*., about 1,000 L, about 2,000 L, about 5,000 L, about 7,500 L, about 10,000 L, about 15,000 L, or about 20,000 L.

### Biosynthesis of Compound (A1)

The depsipeptide, compound (A1), is described in Fujioka et al. (1988) J. Org. Chem.; 53 (12) 2820-2825. It was isolated from a methanol extract of the whole plant of *Ardisia crenata.* Almost two decades later, it was discovered that compound (A1) is in fact produced by a strictly obligate bacterial endosymbiont, *Candidates Burkholderia crenata,* of the plant *Ardisia crenata* (Carlier et al. (2016) Environ Microbiol.;18(8):2507-22; GenBank accession number LGTG01000643.1 for the DNA sequence of the biosynthetic gene cluster (BGC) encoding the biosynthetic genes responsible for biosynthesis of compound (A1)). Compound (A1) biosynthesis by heterologous expression in *E. coli.* is described, *e.g.*, in Crüsemann et al. (2018) Angew. Chem. Int. Ed.; 57, 836 -840.

As described in Example 1 herein, translated amino acid sequences of the compound (A1)-BGC from *B. crenata* were used as query sequence in a genome mining effort on bacterial genomes from sequence databases and bacterial genomes from sequencing efforts performed with PacBio sequencing technology. A cluster with very high homology in translated amino acid sequence and identical prediction of protein functions was discovered in the sequenced genome of *C. vaccinii.* With the knowledge that *C. vaccinii* harbors the compound (A1)-BGC it was possible to retrieve meaningful BLAST hits from its published draft genome (Vöing *et al.* (2015) Genome Announc 3, e00477-15) for *frsC* and *frsH,* but not for the full length genes *frsA, frsD, frsE, frsF, frsG.* A similar approach led to the identification of *C. vaccinii* as producer of A1 by Hermes *et al* (2021). However, the cluster was spread over six contigs and extensive PCR-based gap closure had to be performed to obtain the sequence of the entire compound (A1)-BGC (GenBank accession number: MT876545). The sequence generated in Example 1 herein demonstrates the advantage of PacBio sequencing concerning highly identical repetitive sequence stretches. The complete compound (A1)-BGC could be identified including the up- and downstream regions encoding an additional large NRPS and several transposable elements (GenBank accession number: BankIt2437961 BSeq#1 MW732719). The gene organization on transposable element containing BGCs of compound (A1) (*frsA-H*) and compound J (*dis1-4*) in *C. vaccinii* (76974 bp) is shown in **FIG. 1****.** The organization of genome region containing compound (A1)-BGC and NRPS A is further shown in **Table 2-1.**

The biosynthesis of Compound (A1), including, but not limited to, the complete compound (A1)-BGC, is also disclosed, *e.g.*, in Pistorius et al. Genetic Engineering of *Chromobacterium Vaccinii* DSM 25150 for Improved Production of FR900359. ChemRxiv. Cambridge: Cambridge Open Engage; 2021, which is incorporated by reference in its entirety.

### Cell Culture and Fermentation

The microorganisms described herein can be cultured under conditions that allow for the production of compound (A1). In general, conditions that may be optimized include the type and amount of carbon source, the type and amount of nitrogen source, the carbon-to-nitrogen ratio, the oxygen level, growth temperature, pH, length of the biomass production phase, length of target product accumulation phase, and time of cell harvest.

Culture media generally contain a suitable carbon source. Carbon sources may include, but are not limited to, monosaccharides (*e.g*., glucose, fructose, xylose), disaccharides (*e.g.*, lactose, sucrose), oligosaccharides, polysaccharides (*e.g*., starch, cellulose, hemicellulose, other lignocellulosic materials or mixtures thereof), sugar alcohols (*e.g*., glycerol), and renewable feedstocks (*e.g*., cheese whey permeate, corn steep liquor, sugar beet molasses, barley malt). Carbon sources also can be selected from one or more of the following non-limiting examples: linear or branched alkanes (*e.g*., hexane), linear or branched alcohols (*e.g*., hexanol), fatty acids (*e.g*., about 10 carbons to about 22 carbons), esters of fatty acids, monoglycerides, diglycerides, triglycerides, phospholipids and various commercial sources of fatty acids including vegetable oils (*e.g*., soybean oil) and animal fats. A carbon source may include one-carbon sources (*e.g*., carbon dioxide, methanol, formaldehyde, formate and carbon-containing amines) from which metabolic conversion into key biochemical intermediates can occur. It is expected that the source of carbon utilized may encompass a wide variety of carbon-containing sources and will only be limited by the choice of the engineered microorganism(s). Non-limiting examples of acceptable growth media include Luria broth (LB) agar, King's medium B (KMB) agar, tryptic soy medium, yeast extract mannitol medium (YEM), glycerol yeast extract (GYEA), yeast extract-peptone-glycerol (YPG), peptone yeast extract glucose (PYG) MacConkey agar, or malt extract agar, or in flasks containing suitable liquid media such as, tryptic soy broth, LB broth, YEM broth, KMB broth, GYEA broth. YPG broth, PYG broth, etc.

Nitrogen may be supplied from an inorganic (*e.g*., (NH₄)₂SO₄) or organic source (*e.g*., urea or glutamate). In addition to appropriate carbon and nitrogen sources, culture media also can contain suitable minerals, salts, cofactors, buffers, vitamins, metal ions (*e.g*., Mn²⁺, Co²⁺, Zn²⁺, Mg²⁺) and other components suitable for culture of microorganisms. Other chemicals may be added to the culture as necessary. Non-limiting example of such chemicals include, tryptone peptone, calcium carbonate, propionic acid, sodium propionate, L-isoleucine, L-valine, L-phenylalanine, L-phenylbutyric acid, L-phenyllactic acid, sodium chloride, glycerol, Bacto soytone, Bacto peptone, Bacto soytone peptone, Bacto yeast extract, Soy peptone F, Soy peptone papaic digest, veggie peptone, veggie yeast extract, broadbean peptone, casein peptone, HEPES, ammonium sulfate, magnesium sulfate, soyamine, Amicase^{®}, Tubermine^{®}, soy protein, yeast extract without salt, Pharmamedia^{®}, potato starch, L-phenyllactate, and L-phenylpyruvate.

Microorganisms sometimes are cultured in complex media (*e.g*., yeast extract-peptone-dextrose broth (YPD)). Culture media in some embodiments are common commercially prepared media, *e.g*., LB media. Other defined or synthetic growth media are known in the arts and may also be used.

Microorganisms may be cultured on or in solid, semi-solid or liquid media. In some embodiments, media is drained from cells adhering to a plate. In certain embodiments, a liquid-cell mixture is centrifuged at a speed sufficient to pellet the cells but not disrupt the cells and allow extraction of the media, as known in the art. The cells may then be resuspended in fresh media. The product of interest may be purified from culture media according to methods known in the art. In certain embodiments, target product is extracted from the cultured microorganisms. The microorganism cells may be concentrated through centrifugation at speed sufficient to shear the cell membranes. In some embodiments, the cells may be physically disrupted (*e.g*., shear force, sonication) or chemically disrupted (*e.g*., contacted with detergent or other lysing agent). The phases may be separated by centrifugation or other method known in the art and target product may be isolated according to known methods.

Fermentation conditions can include any culture conditions suitable for maintaining a microorganism (*e.g*., in a static or proliferative state). For example, fermentation conditions can include several parameters, including without limitation, temperature, oxygen content, nutrient content (*e.g*., glucose content), pH, agitation level (*e.g*., rotations per minute), gas flow rate (*e.g.*, air, oxygen, nitrogen gas), redox potential, cell density (*e.g*., optical density), cell viability and the like. A change in fermentation conditions (*e.g*., switching fermentation conditions) is an alteration, modification or shift of one or more fermentation parameters. For example, one can change fermentation conditions by increasing or decreasing temperature, increasing or decreasing pH (*e.g*., adding or removing an acid, a base or carbon dioxide), increasing or decreasing oxygen content (*e.g.*, introducing air, oxygen, carbon dioxide, nitrogen), increasing or decreasing air pressure (*e.g*., by introducing air, oxygen, carbon dioxide, nitrogen), increasing or decreasing agitation, and/or adding or removing a nutrient (*e.g*., one or more sugars or sources of sugar, biomass, vitamin and the like), increasing or decreasing the ratio of culture and flask volume, or combinations of the foregoing. Examples of fermentation conditions are described herein. Aerobic conditions often comprise greater than about 50% dissolved oxygen (*e.g*., about 52%, about 54%, about 56%, about 58%, about 60%, about 62%, about 64%, about 66%, about 68%, about 70%, about 72%, about 74%, about 76%, about 78%, about 80%, about 82%, about 84%, about 86%, about 88%, about 90%, about 91%, about 92%, about 93%, about 94%, about 95%, about 96%, about 97%, about 98% or about 99%, or greater than any one of the foregoing). Anaerobic conditions often comprise less than about 50% dissolved oxygen (*e.g*., about 1%, about 2%, about 4%, about 6%, about 8%, about 10%, about 12%, about 14%, about 16%, about 18%, about 20%, about 22%, about 24%, about 26%, about 28%, about 30%, about 32%, about 34%, about 36%, about 38%, about 40%, about 42%, about 44%, about 46%, about 48%, or less than any one of the foregoing).

A variety of fermentation processes may be applied for the production of a product of interest. In some embodiments, the production of a target product from a recombinant microbial host is conducted using a batch, fed-batch or continuous fermentation process, for example.

A batch fermentation process often is a closed system where the media composition is fixed at the beginning of the process and not subject to further additions beyond those required for maintenance of pH and oxygen level during the process. At the beginning of the culturing process the media is inoculated with the desired organism and growth or metabolic activity is permitted to occur without adding additional sources (*e.g*., carbon and nitrogen sources) to the medium. In batch processes the metabolite and biomass compositions of the system change constantly up to the time the culture is terminated. In a typical batch process, cells proceed through a static lag phase to a high-growth log phase and finally to a stationary phase, wherein the growth rate is diminished or halted. Left untreated, cells in the stationary phase will eventually die.

A variation of the standard batch process is the fed-batch process, where the carbon source is continually added to the fermenter over the course of the fermentation process. Fed-batch processes are useful when catabolite repression is apt to inhibit the metabolism of the cells or where it is desirable to have limited amounts of carbon source in the media at any one time. Measurement of the carbon source concentration in fed-batch systems may be estimated on the basis of the changes of measurable factors such as pH, dissolved oxygen and the partial pressure of waste gases (*e.g*., CO.sub.2). Batch and fed-batch culturing methods are known in the art. Examples of such methods may be found in Thomas D. Brock in Biotechnology: A Textbook of Industrial Microbiology, 2.sup.nd ed., (1989) Sinauer Associates Sunderland, Mass. and Deshpande, Mukund V., Appl. Biochem. Biotechnol., 36:227 (1992).

In continuous fermentation process a defined media often is continuously added to a bioreactor while an equal amount of culture volume is removed simultaneously for product recovery. Continuous cultures generally maintain cells in the log phase of growth at a constant cell density. Continuous or semi-continuous culture methods permit the modulation of one factor or any number of factors that affect cell growth or end product concentration. For example, an approach may limit the carbon source and allow all other parameters to moderate metabolism. In some systems, a number of factors affecting growth may be altered continuously while the cell concentration, measured by media turbidity, is kept constant. Continuous systems often maintain steady state growth and thus the cell growth rate often is balanced against cell loss due to media being drawn off the culture. Methods of modulating nutrients and growth factors for continuous culture processes, as well as techniques for maximizing the rate of product formation, are known and a variety of methods are detailed by Brock, supra.

The product of interest may be provided within cultured microbes containing target product, and cultured microbes may be supplied fresh or frozen in a liquid media or dried. Fresh or frozen microbes may be contained in appropriate moisture-proof containers that may also be temperature controlled as necessary. Product of interest sometimes is provided in culture medium that is substantially cell-free. In some embodiments, the product of interest is provided in substantially pure form (*e.g*., 50% pure or greater, 60% pure or greater, 70% pure or greater, 80% pure or greater, 90% pure or greater, 95% pure or greater, 99% pure or greater or 99.5% pure or greater). In some embodiments, the product of interest may be modified into any one of a number of downstream products.

### Drug Moiety (D)

In some embodiments, the drug moiety (D) of the antibody drug conjugate of the disclosure is a GNAQ inhibitor, a GNA11 inhibitor, or an inhibitor of GNAQ and GNA11 (GNAQ/GNA11 inhibitor). In some embodiments, the drug moiety (D) of the antibody drug conjugate of the disclosure is a GNAQ inhibitor. In some embodiments, the drug moiety (D) of the antibody drug conjugate of the disclosure is a GNA11 inhibitor. In some embodiments, the drug moiety (D) of the antibody drug conjugate of the disclosure is an inhibitor of GNAQ and GNA11 (GNAQ/GNA11 inhibitor).

In some embodiments, the drug moiety (D) of the antibody drug conjugate of the disclosure is a compound having the structure of Formula (A) 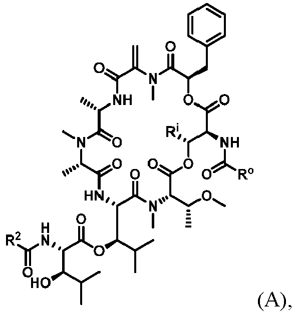 wherein Ro is methyl or ethyl, Ri is methyl or i-propyl, and R2 is methyl or ethyl.

In some embodiments, the drug moiety (D) of the antibody drug conjugate of the disclosure is a compound (A1) having the following structure,

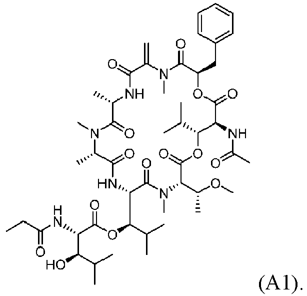

Synthesis of Compound (A1) is also described in Example 3 of International Application Publication No. WO 2020/128612, which is incorporated by reference in its entirety.

The metabolic stability and PK properties of Compound (A1) are also described in Examples 8 and 9, respectively, of International Application Publication No. WO 2020/128612, which is incorporated by reference in its entirety.

In some embodiments, the drug moiety (D) of the antibody drug conjugate of the disclosure is a compound having the structure of Formula (Ab) 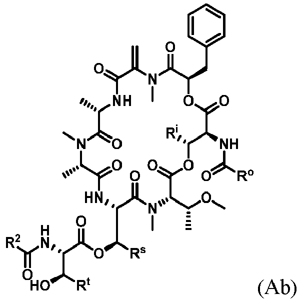 wherein R° is methyl or ethyl, Rⁱ, R^{s}, and R^{t} are each independently methyl, i-propyl, or methylthiomethyl, and R² is methyl or ethyl.

In some embodiments, the drug moiety (D) of the antibody drug conjugate of the disclosure is a compound (Ab1) having the following structure,

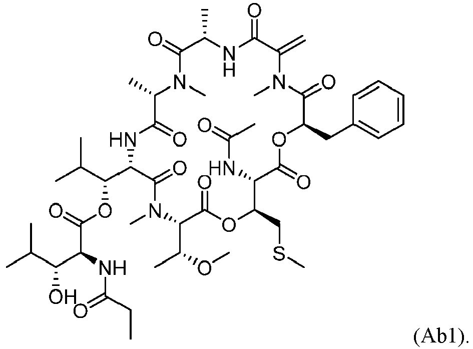

In some embodiments, the drug moiety (D) of the antibody drug conjugate of the disclosure is a compound (Ab2) having the following structure,

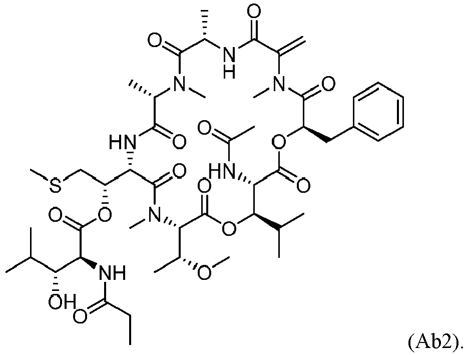

In some embodiments, the drug moiety (D) of the antibody drug conjugate of the disclosure is a compound (Ab3) having the following structure,

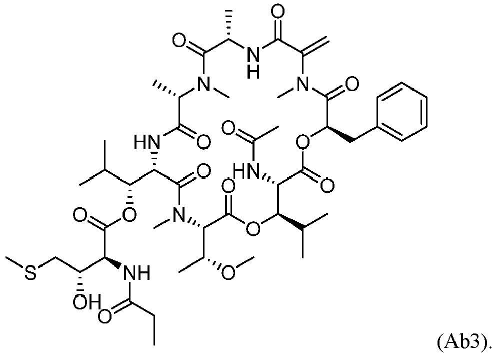

In some embodiments, the drug moiety (D) of the antibody drug conjugate of the disclosure is a compound having the structure of Formula (Ac) 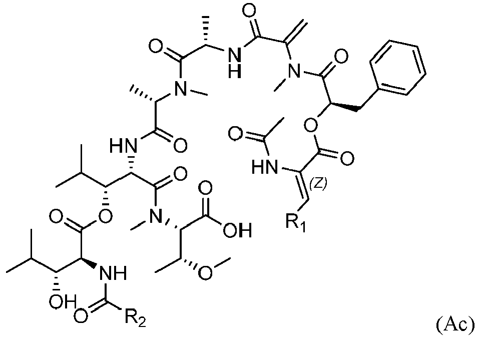 wherein R¹ is methyl or isopropyl, and R² is methyl or ethyl.

In some embodiments, the drug moiety (D) of the antibody drug conjugate of the disclosure is a compound (Ac1) having the following structure,

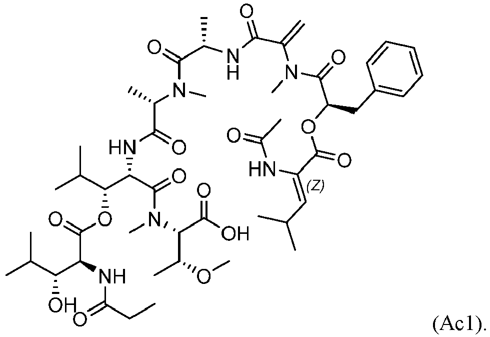

In some embodiments, the drug moiety (D) of the antibody drug conjugate of the disclosure is a compound (Ac2) having the following structure,

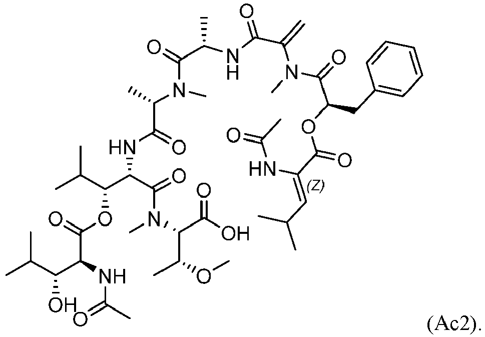

In some embodiments, the drug moiety (D) of the antibody drug conjugate of the disclosure is a compound (Ac3) having the following structure,

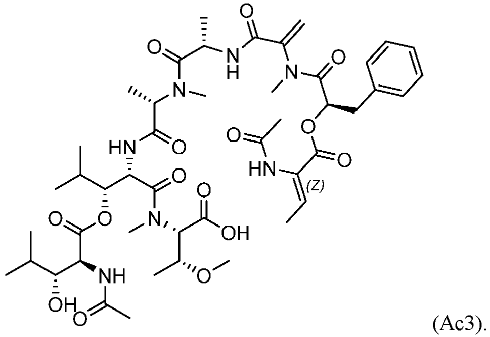

In some embodiments, the drug moiety (D) of the antibody drug conjugate of the disclosure is a compound (Ac4) having the following structure, 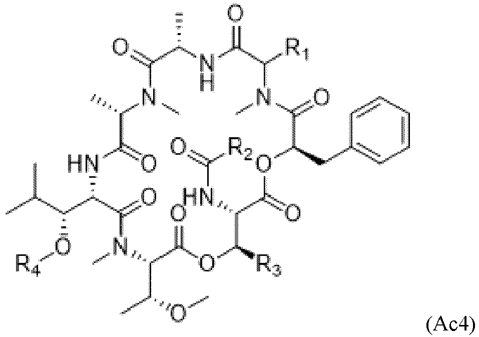 wherein R₁ is a methyl or a =CH2, R₂ is a methyl or an ethyl, R₃ is methyl, ethyl, or isopropyl, and R₄ is a H, *N*-Ac-Hle, or*N*-Pr-Hle.

In some embodiments, the drug moiety (D) of the antibody drug conjugate of the disclosure is any of compounds (A1) and 2-15, *e.g.*, as disclosed in Example 3.

### Linker-Drug Moiety (L_{A}-(D)ₙ)

In some embodiments, the Linker-Drug moiety, ((L_{A}-(D)ₙ))), of the Antibody Drug Conjugate of the disclosure comprises one or more Drug moieties covalently attached to a linker (L_{A}), wherein the one or more Drug moieties are each independently selected from a GNAQ inhibitor, a GNA11 inhibitor or an inhibitor of GNAQ and GNA11 (GNAQ/GNA11 inhibitor).

In some embodiments, the Linker-Drug moiety, ((L_{A}-(D)ₙ))), of the Antibody Drug Conjugate of the disclosure comprises one or more Drug moieties covalently attached to a linker (L_{A}), wherein the one or more Drug moieties are each independently selected from a GNAQ inhibitor.

In some embodiments, the Linker-Drug moiety, ((L_{A}-(D)ₙ))), of the Antibody Drug Conjugate of the disclosure comprises one or more Drug moieties attached to a linker (L_{A}), wherein the one or more Drug moieties are each independently selected from a GNA11 inhibitor.

In some embodiments, the Linker-Drug moiety, ((L_{A}-(D)ₙ))), of the Antibody Drug Conjugate of the disclosure comprises one or more Drug moieties covalently attached to a linker (L_{A}), wherein the one or more Drug moieties are each independently selected from an inhibitor of GNAQ and GNA11 (GNAQ/GNA11 inhibitor).

In some embodiments, the Linker-Drug moiety, ((L_{A}-(D)ₙ))), of the Antibody Drug Conjugate of the disclosure comprises one or more Drug moieties covalently attached to a linker (L_{A}), wherein the linker (L_{A}) is a cleavable linker and the one or more Drug moieties are each independently selected from a GNAQ inhibitor, a GNA11 inhibitor or an inhibitor of GNAQ and GNA11 (GNAQ/GNA11 inhibitor).

In some embodiments, the Linker-Drug moiety, ((L_{A}-(D)ₙ))), of the Antibody Drug Conjugate of the disclosure comprises one or more Drug moieties covalently attached to a linker (L_{A}), wherein the linker (L_{A}) is a cleavable linker and the one or more Drug moieties are each independently selected from a GNAQ inhibitor.

In some embodiments, the Linker-Drug moiety, ((L_{A}-(D)ₙ))), of the Antibody Drug Conjugate of the disclosure comprises one or more Drug moieties covalently attached to a linker (L_{A}), wherein the linker (L_{A}) is a cleavable linker and the one or more Drug moieties are each independently selected from a GNA11 inhibitor.

In some embodiments, the Linker-Drug moiety, ((L_{A}-(D)ₙ))), of the Antibody Drug Conjugate of the disclosure comprises one or more Drug moieties covalently attached to a linker (L_{A}), wherein the linker (L_{A}) is a cleavable linker and the one or more Drug moieties are each independently selected from an inhibitor of GNAQ and GNA11 (GNAQ/GNA11 inhibitor).

In some embodiments, the Linker-Drug moiety, ((L_{A}-(D)ₙ))), of the Antibody Drug Conjugate of the disclosure comprises one or more Drug moieties covalently attached to a linker (L_{A}), wherein the linker (L_{A}) is a non-cleavable linker and the one or more Drug moieties are each independently selected from a GNAQ inhibitor, a GNA11 inhibitor or an inhibitor of GNAQ and GNA11 (GNAQ/GNA11 inhibitor).

In some embodiments, the Linker-Drug moiety, ((L_{A}-(D)ₙ))), of the Antibody Drug Conjugate of the disclosure comprises one or more Drug moieties covalently attached to a linker (L_{A}), wherein the linker (L_{A}) is a non-cleavable linker and the one or more Drug moieties are each independently selected from a GNAQ inhibitor.

In some embodiments, the Linker-Drug moiety, ((L_{A}-(D)ₙ))), of the Antibody Drug Conjugate of the disclosure comprises one or more Drug moieties covalently attached to a linker (L_{A}), wherein the linker (L_{A}) is a non-cleavable linker and the one or more Drug moieties are each independently selected from a GNA11 inhibitor.

In some embodiments, the Linker-Drug moiety, ((L_{A}-(D)ₙ))), of the Antibody Drug Conjugate of the disclosure comprises one or more Drug moieties covalently attached to a linker (L_{A}), wherein the linker (L_{A}) is a non-cleavable linker and the one or more Drug moieties are each independently selected from an inhibitor of GNAQ and GNA11 (GNAQ/GNA11 inhibitor).

In some embodiments, the linker (L_{A}) of the Linker-Drug moiety, ((L_{A}-(D)ₙ))), of the Antibody Drug Conjugate of the disclosure has the following formula: 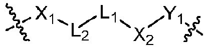 wherein:
X₁ is a bivalent coupling group;
X₂ is a self-immolative spacer;
Y₁ is 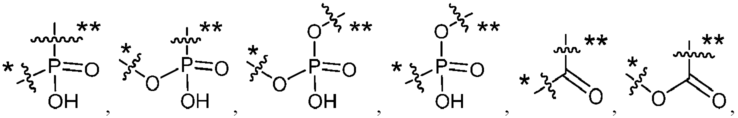 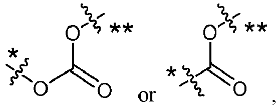 where the * of Y₁ indicates the point of attachment to X₂ and the ** of Y₁ indicates the other point of attachment;
L₁ is a bivalent peptide linker, and
L₂ is a bond or a linker.

In some embodiments, the Linker-Drug moiety, ((L_{A}-(D)ₙ))), of the Antibody Drug Conjugate of the disclosure has the following formula: 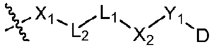 wherein:
D is a GNAQ inhibitor, a GNA11 inhibitor or an inhibitor of GNAQ and GNA11;
X₁ is a bivalent coupling group;
X₂ is a self-immolative spacer;
Y₁ is 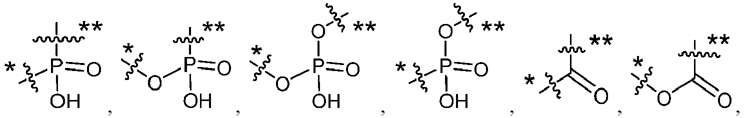 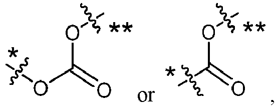 where the * of Y₁ indicates the point of attachment to X₂ and the ** of Y₁ indicates the point of attachment to D;
L₁ is a bivalent peptide linker, and
L₂ is a bond or a linker.

### Linker-Drug Compounds (L_{B}-(D)ₙ)

In some embodiments, the Linker-Drug of the disclosure is a compound having the structure of Formula (B), or stereoisomers or pharmaceutically acceptable salts thereof,

R⁸-L_{B}-(D)ₙ (B)

wherein:
D is a GNAQ inhibitor, a GNA11 inhibitor or an inhibitor of GNAQ and GNA11;
R⁸ is a reactive group;
L_{B} is a cleavable linker or non-cleavable linker, and
n is 1, 2, 3 or 4.

In some embodiments, the Linker-Drug of the disclosure having the structure of Formula (B), or stereoisomers or pharmaceutically acceptable salts thereof, wherein
D is a GNAQ inhibitor, a GNA11 inhibitor or an inhibitor of GNAQ and GNA11;
R⁸ is a reactive group;
L_{B} is a cleavable linker comprising one or more linker components selected from a self-immolative spacer, a phosphate group, a carbonate group and a bivalent peptide linker, and
n is 1, 2, 3 or 4.

In some embodiments, the Linker-Drug of the disclosure is a compound having the structure of Formula (B-1), or stereoisomers or pharmaceutically acceptable salts thereof, 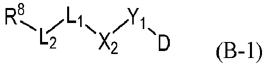 wherein:
D is a GNAQ inhibitor, a GNA11 inhibitor or an inhibitor of GNAQ and GNA11;
R⁸ is a reactive group;
X₂ is a self-immolative spacer;
Y₁ is 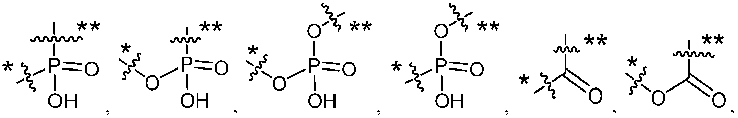 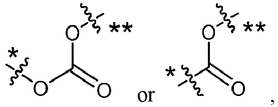 where the * of Y₁ indicates the point of attachment to X₂ and the ** of Y₁ indicates the point of attachment to D;
L₁ is a bivalent peptide linker, and
L₂ is a bond or a linker.

Certain embodiments and examples of the Linker-Drug compounds of the disclosure are provided in the following listing of additional, enumerated embodiments. It will be recognized that features specified in each embodiment may be combined with other specified features to provide further embodiments of the present disclosure.

Embodiment 1. The compound of Formula (B) or Formula (B-1), or stereoisomers or a pharmaceutically acceptable salt thereof, wherein D is a GNAQ inhibitor.

Embodiment 2. The compound of Formula (B) or Formula (B-1), or stereoisomers or a pharmaceutically acceptable salt thereof, wherein D is a GNA11 inhibitor.

Embodiment 3. The compound of Formula (B) or Formula (B-1), or stereoisomers or a pharmaceutically acceptable salt thereof, wherein D is an inhibitor of GNAQ and GNA11.

Embodiment 4. The compound of Formula (B) or Formula (B-1), or stereoisomers or a pharmaceutically acceptable salt thereof, wherein D is 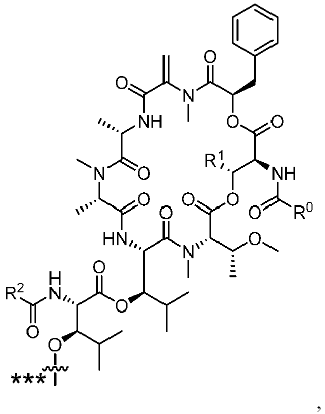 wherein R⁰ is methyl or ethyl, R¹ is methyl or isopropyl, R² is methyl or ethyl, and the *** indicates the point of attachment to L_{B} or Y₁.

Embodiment 4a. The compound of Formula (Bb) or Formula (Bb-1), or stereoisomers or a pharmaceutically acceptable salt thereof, wherein D is 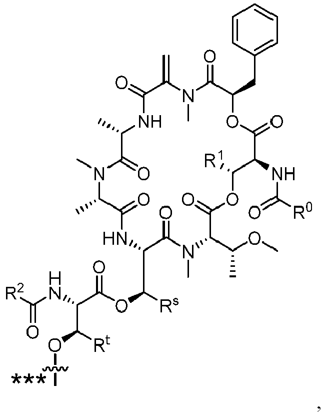 wherein R⁰ is methyl or ethyl, R', R^{s}, R^{t} are each independently methyl, isopropyl, or methylthiomethyl, R² is methyl or ethyl, and the *** indicates the point of attachment to L_{B} or Y₁.

Embodiment 4b. The compound of Formula (Bc) or Formula (Bc-1), or stereoisomers or a pharmaceutically acceptable salt thereof, wherein D is 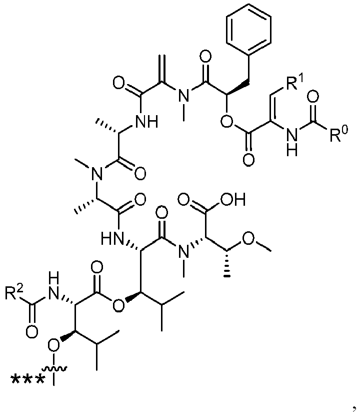 wherein R⁰ is methyl or ethyl, R¹ is methyl or isopropyl, R² is methyl or ethyl, and the *** indicates the point of attachment to L_{B} or Y₁.

Embodiment 5. The compound of Formula (B) or Formula (B-1), or stereoisomers or a pharmaceutically acceptable salt thereof, wherein D is 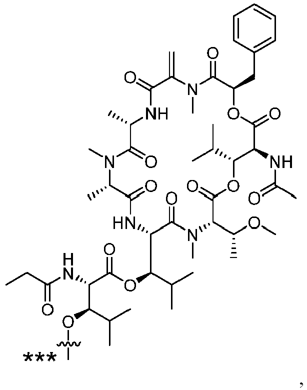 where the *** indicates the point of attachment to L_{B} or Y₁.

Embodiment 6. The compound of Formula (B) or Formula (B-1), or stereoisomers or a pharmaceutically acceptable salt thereof, having the structure of Formula (B-2), or a pharmaceutically acceptable salt thereof: 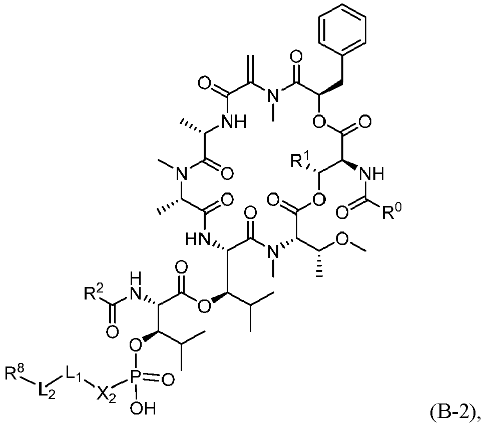 wherein:
R⁰ is methyl or ethyl,;
R¹ is methyl or isopropyl;
R² is methyl or ethyl, and
X₂, Li, L₂ and R⁸ are as define in compounds of Formula (B-1) above.

Embodiment 6a. The compound of Formula (Bb) or Formula (Bb-1), or stereoisomers or a pharmaceutically acceptable salt thereof, having the structure of Formula (Bb-2), or a pharmaceutically acceptable salt thereof: 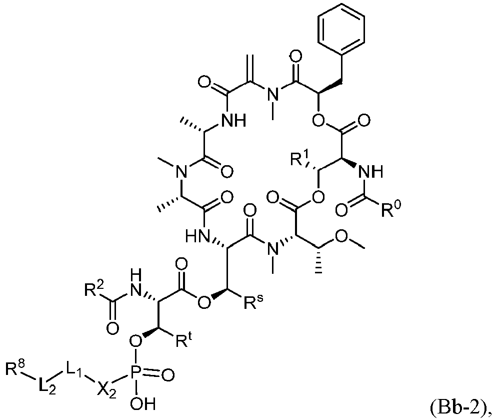 wherein:
R⁰ is methyl or ethyl;
R¹, R^{s}, and R^{t} are each independently methyl, methylthiomethyl or isopropyl;
R² is methyl or ethyl, and
X₂, L₁, L₂ and R⁸ are as define in compounds of Formula (Bb-1) above.

Embodiment 6b. The compound of Formula (Bc) or Formula (Bc-1), or stereoisomers or a pharmaceutically acceptable salt thereof, having the structure of Formula (Bc-2), or a pharmaceutically acceptable salt thereof: 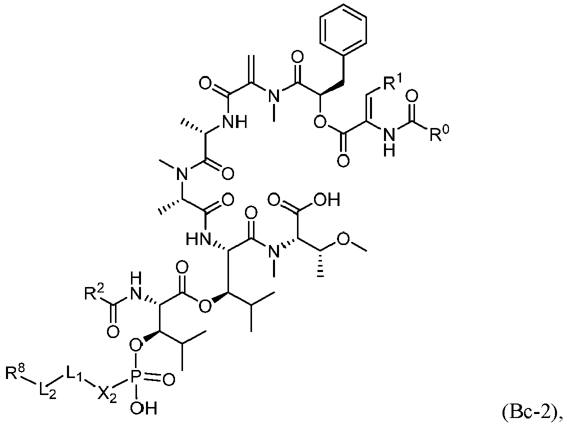 wherein:
R⁰ is methyl or ethyl,;
R¹ is methyl or isopropyl;
R² is methyl or ethyl, and
X₂, L₁, L₂ and R⁸ are as define in compounds of Formula (Bc-1) above.

Embodiment 7. The compound of Formula (B), Formula (B-1) or Formula (B-2), or stereoisomers or a pharmaceutically acceptable salt thereof, having the structure of Formula (B-2a), or a pharmaceutically acceptable salt thereof: 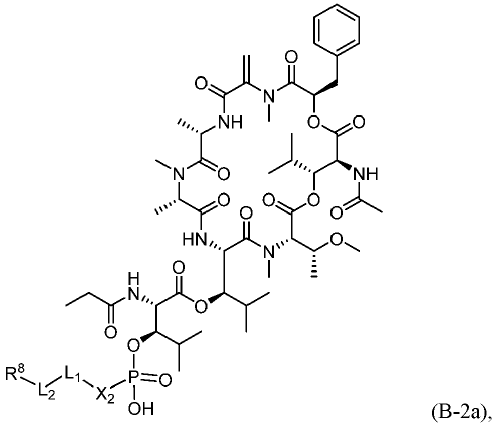 wherein:
X₂, L₁, L₂ and R⁸ are as define in compounds of Formula (B-1) above.

Embodiment 8. The compound of Formula (B) or Formula (B-1), or stereoisomers or a pharmaceutically acceptable salt thereof, having the structure of Formula (B-3), or a pharmaceutically acceptable salt thereof: 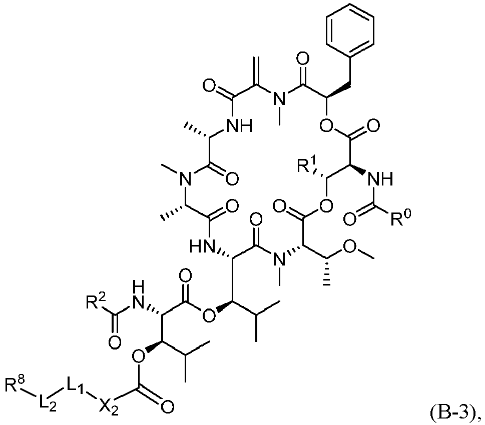 wherein:
R⁰ is methyl or ethyl;
R¹ is methyl or isopropyl;
R² is methyl or ethyl, and
X₂, L₁, L₂ and R⁸ are as define in compounds of Formula (B-1) above.

Embodiment 8a. The compound of Formula (Bb) or Formula (Bb-1), or stereoisomers or a pharmaceutically acceptable salt thereof, having the structure of Formula (Bb-3), or a pharmaceutically acceptable salt thereof: 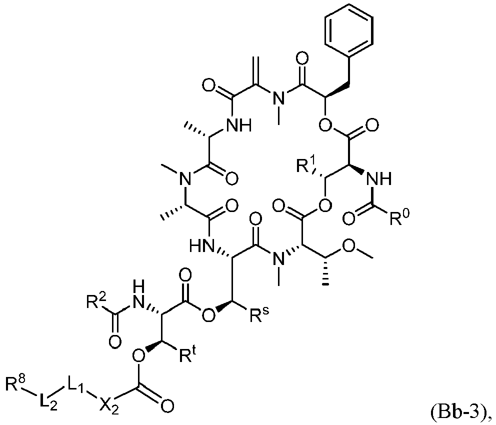 wherein:
R⁰ is methyl or ethyl;
R', R^{s}, and R^{t} are each independently methyl, methylthiomethyl, or isopropyl;
R² is methyl or ethyl, and
X₂, L₁, L₂ and R⁸ are as define in compounds of Formula (Bb-1) above.

Embodiment 8b. The compound of Formula (Bc) or Formula (Bc-1), or stereoisomers or a pharmaceutically acceptable salt thereof, having the structure of Formula (Bc-3), or a pharmaceutically acceptable salt thereof: 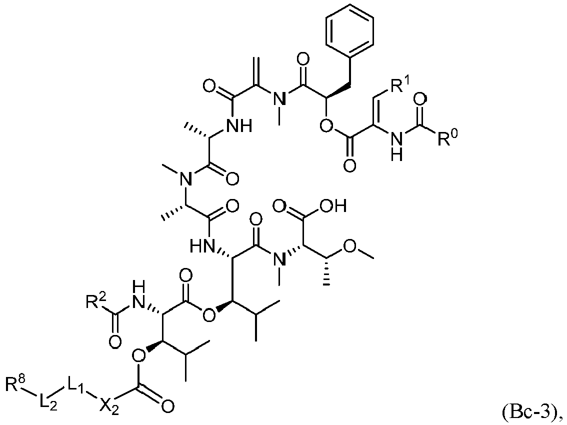 wherein:
R⁰ is methyl or ethyl;
R¹ is methyl or isopropyl;
R² is methyl or ethyl, and
X₂, L₁, L₂ and R⁸ are as define in compounds of Formula (Bc-1) above.

Embodiment 9. The compound of Formula (B), Formula (B-1) or Formula (B-3), or stereoisomers or a pharmaceutically acceptable salt thereof, having the structure of Formula (B-3a), or a pharmaceutically acceptable salt thereof: 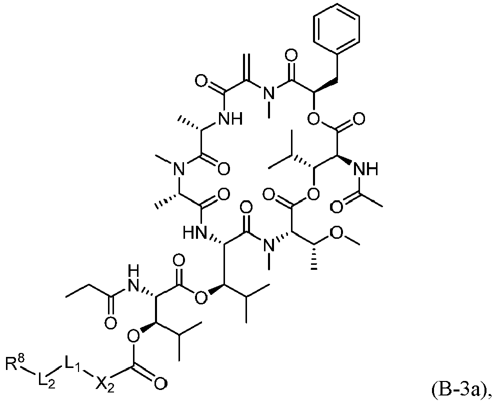 wherein:
X₂, L₁, L₂ and R⁸ are as define in compounds of Formula (B-1) above.

Embodiment 10. The compound of Formula (B-2) of Embodiment 6, Formula (B-3) of Embodiment 8, or a pharmaceutically acceptable salt thereof:
wherein:
R⁰ is methyl or ethyl;
R¹ is methyl or isopropyl;
R² is methyl or ethyl;
X₂ is a self-immolative spacer selected from 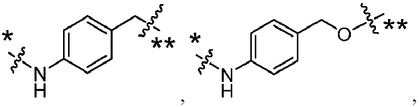 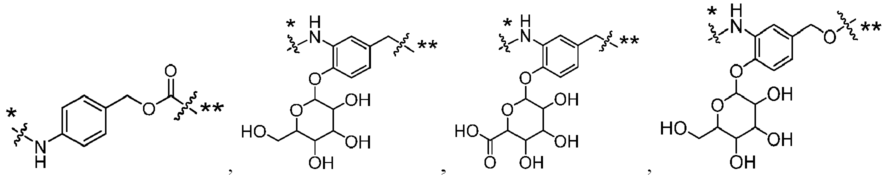 and 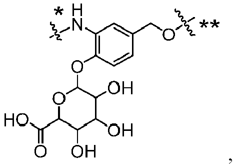 where the * of X₂ indicates the point of attachment to L₁ and the ** of X₂ indicates the point of attachment to the 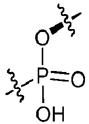 group or the point of attachment to 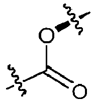 group;
L₁ is a bivalent peptide linker comprising 2 to 4 amino acid residues;
L₂ is a linker,
R⁸ is selected from 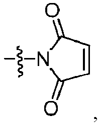 -N₃, -ONH₂, -NR⁴C(=O)CH=CH₂, SH, -SSR¹³, - S(=O)₂(CH=CH₂), -NR⁴S(=O)₂(CH=CH₂), -NR⁴C(=O)CH₂Br, -NR⁴C(=O)CH₂I, -NHC(=O)CH₂Br, - NHC(=O)CH₂I, -C(=O)NHNH₂, 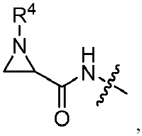 -CO₂H, -NH₂, 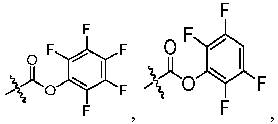 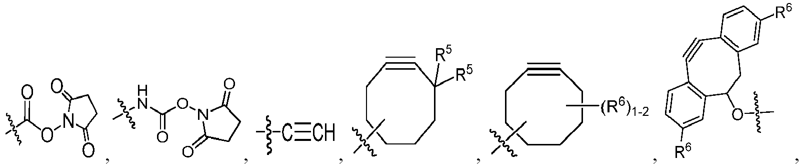 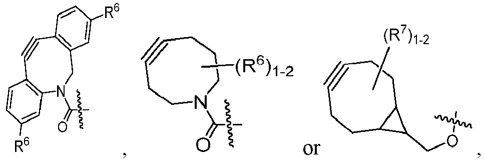 wherein:
each R₄ is independently selected from H and C₁-C₆alkyl;
each R⁵ is independently selected from H, C₁-C₆alkyl, F, Cl, and -OH;
each R⁶ is independently selected from H, C₁-C₆alkyl, F, Cl, -NH₂, -OCH₃, -OCH₂CH₃, - N(CH₃)₂, -CN, -NOz and -0H,
   and
each R⁷ is independently selected from H, C₁₋₆alkyl, fluoro, benzyloxy substituted with - C(=O)OH, benzyl substituted with -C(=O)OH, C₁₋₄alkoxy substituted with -C(=O)OH and C₁₋₄alkyl substituted with -C(=O)OH.

Embodiment 11. The compound of any one of Embodiments 1 to 10, wherein X₂ is a self-immolative spacer selected from 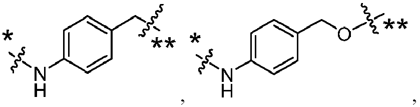 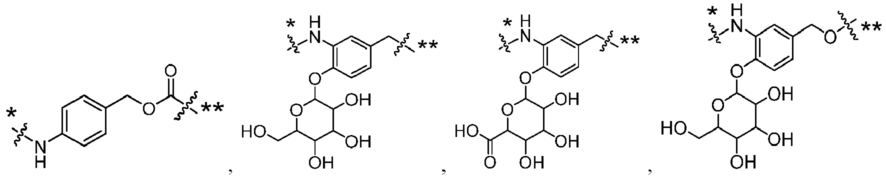 and 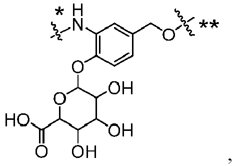 where the * of X₂ indicates the point of attachment to L₁ and the ** of X₂ indicates the point of attachment to Y₁, the point of attachment to the 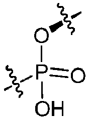 group or the point of attachment to 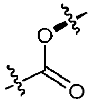 group.

Embodiment 12. The compound of any one of Embodiments 1 to 11, wherein X₂ is 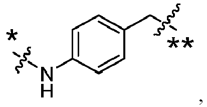 where the * of X₂ indicates the point of attachment to L₁ and the ** of X₂ indicates the point of attachment to Y₁, the point of attachment to the 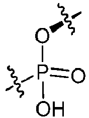 group or the point of attachment to 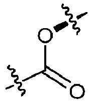 group.

Embodiment 13. The compound of any one of Embodiments 1 to 12, wherein L₁ is a bivalent peptide linker comprising 2 to 4 amino acid residues.

Embodiment 14. The compound of any one of Embodiments 1 to 12, wherein L₁ is a is a bivalent peptide linker comprising an amino acid residue selected from valine, citrulline, lysine, isoleucine, phenylalanine, methionine, asparagine, proline, alanine, leucine, tryptophan, and tyrosine.

Embodiment 15. The compound of any one of Embodiments 1 to 12, wherein L₁ is a bivalent peptide linker comprising at least one valine (Val) or citrulline (Cit) residue.

Embodiment 16. The compound of any one of Embodiments 1 to 12, wherein L₁ is a bivalent dipeptide linker selected from ValCit, PheLys, ValAla and ValLys.

Embodiment 17. The compound of any one of Embodiments 1 to 12, wherein L₁ is a bivalent dipeptide linker selected from 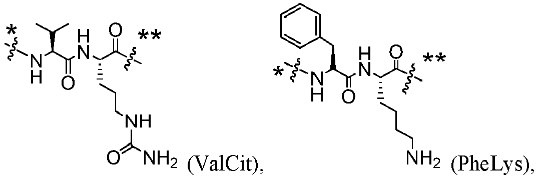 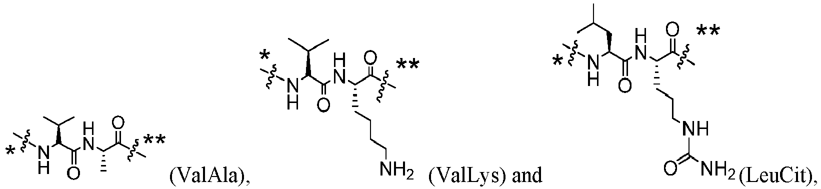 where the * of L₁ indicates the attachment point to L₂ and the ** of L₁ indicates the attachment point to X₂.

Embodiment 18. The compound of any one of Embodiments 1 to 12, wherein L₁ is ValCit.

Embodiment 19. The compound of any one of Embodiments 1 to 12, wherein
L₁ is 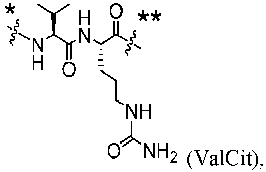 where the * of L₁ indicates the attachment point to L₂ and the ** of L₁ indicates the attachment point to X₂.

Embodiment 20. The compound of any one of Embodiments 1 to 19, wherein L₂ is a linker.

Embodiment 21. The compound of any one of Embodiments 1 to 19, wherein L₂ is a linker selected from:
-*C(=O)((CH₂)ₘO)ₚ(CH₂)ₘ**-, -*C(=O)(CH₂)ₘ**-, -*C(=O)(CH₂)ₙNHC(=O)(CH₂)ₘ**-, - *C(=O)(CH₂)ₘNHC(=O)((CH₂)ₘO)ₚ(CH₂)ₘ**-, -* ((CH₂)ₘO)ₚ(CH₂)ₘ**-, -* ((CH₂)ₘO)ₚ(CH₂)ₘ**-, - (CH₂)ₘ-, -*(CH₂)ₘNHC(=O)(CH₂)ₘ**-, * (CH₂)ₘNHC(=O)(CH₂)ₘC(=O)NH(CH₂)ₘ**-, * ((CH₂)ₘO)ₚ(CH₂)ₘNHC(=O)(CH₂)ₘ**-, * *((CH₂)ₘO)ₚCH₂)ₘC(=O)NH(CH₂)ₘ**-, -* (CH₂)ₘC(R₃)₂**-, and -* (CH₂)ₘC(R₃)₂SS(CH₂)ₘNHC(=O)(CH₂)ₘ**-, where the * of L₂ indicates the attachment point to L₁ and the ** of L₂ indicates the point of attachment to R₈;
and wherein:
   each R₃ is independently selected from H and C₁-C₆alkyl;
   each m is independently selected from 1, 2, 3, 4, 5, 6, 7, 8, 9 and 10, and
   each p is independently selected from 1, 2, 3, 4, 5, 6, 7, 8, 9, 10, 11, 12, 13 and 14.

Embodiment 22. The compound of any one of Embodiments 1 to 19, wherein L₂ is - *C(=O)((CH₂)ₘO)ₚ(CH₂)ₘ**- or -*C(=O)(CH₂)ₘ**-, where the * of L₂ indicates the point of attachment to L₁ and the ** of L₂ indicates the point of attachment to R₈,
and wherein each m is independently selected from 1, 2, 3, 4, 5, 6, 7, 8, 9 or 10 and p is 1, 2, 3, 4, 5, 6, 7, 8, 9, 10, 11, 12, 13 or 14.

Embodiment 23. The compound of any one of Embodiments 1 to 19, wherein L₂ is 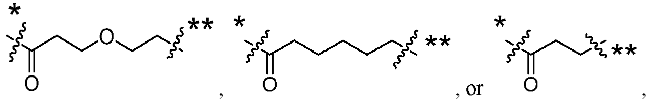 where the * of L₂ indicates the point of attachment to L₁ and the ** of L₂ indicates the point of attachment to R₈.

Embodiment 24. The compound of any one of Embodiments 1 to 23, wherein R⁸ is 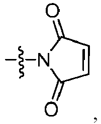 -N₃, -ONH₂, -NR⁴C(=O)CH=CH₂, SH, -SSR¹³, -S(=O)₂(CH=CH₂), -NR⁴S(=O)₂(CH=CH₂), - NR⁴C(=O)CH₂Br, -NR⁴C(=O)CH₂I, -NHC(=O)CH₂Br, -NHC(=O)CH₂I, -C(=O)NHNH₂, 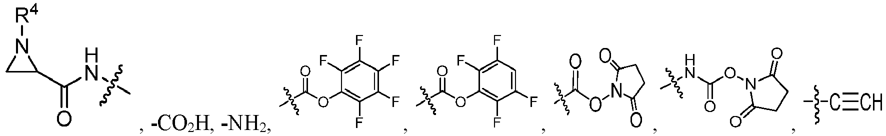 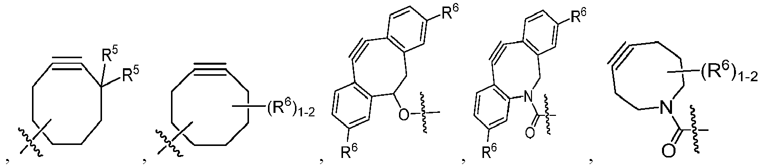 or 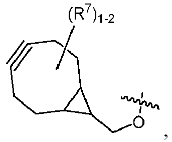 wherein:
each R⁴ is independently selected from H and C₁-C₆alkyl;
each R⁵ is independently selected from H, C₁-C₆alkyl, F, Cl, and -OH;
each R⁶ is independently selected from H, C₁-C₆alkyl, F, Cl, -NH₂, -OCH₃, -OCH₂CH₃, - N(CH₃)₂, -CN, -NOz and -0H,
   and
each R⁷ is independently selected from H, C₁₋₆alkyl, fluoro, benzyloxy substituted with - C(=O)OH, benzyl substituted with -C(=O)OH, C₁₋₄alkoxy substituted with -C(=O)OH and C₁₋₄alkyl substituted with -C(=O)OH.

Embodiment 25. The compound of any one of Embodiments 1 to 23, wherein R⁸ is 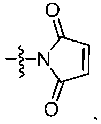 -ONH₂,

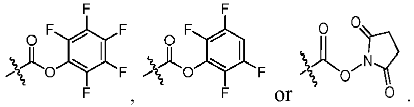

Embodiment 26. The compound of any one of Embodiments 1 to 23, wherein R⁸ is

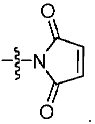

Embodiment 27. The compound of Formula (B-2) of Embodiment 6, or a pharmaceutically acceptable salt thereof:
wherein:
R⁰ is methyl or ethyl;
R¹ is methyl or isopropyl;
R² is methyl or ethyl;
X₂ is 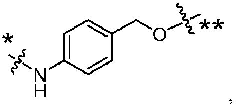 where the * of X₂ indicates the point of attachment to L₁ and the ** of X₂ indicates the point of attachment to the 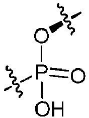 group;
L₁ is 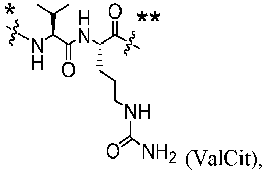 where the * of L₁ indicates the attachment point to L₂ and the ** of L₁ indicates the attachment point to X₂;
L₂ is 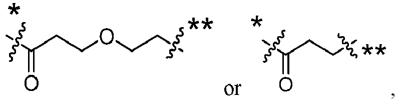 where the * of L₂ indicates the point of attachment to L₁ and the ** of L₂ indicates the point of attachment to R₈, and
R⁸ is

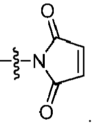

Embodiment 28. The compound of Formula (B-3) of Embodiment 8, or a pharmaceutically acceptable salt thereof: wherein:
R⁰ is methyl or ethyl;
R¹ is methyl or isopropyl;
R² is methyl or ethyl;
X₂ is 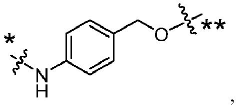 where the * of X₂ indicates the point of attachment to L₁ and the ** of X₂ indicates the point of attachment to the 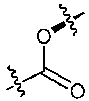 group;
L₁ is 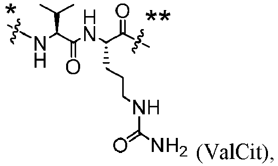 where the * of L₁ indicates the attachment point to L₂ and the ** of L₁ indicates the attachment point to X₂;
L₂ is 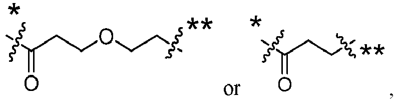 where the * of L₂ indicates the point of attachment to L₁ and the ** of L₂ indicates the point of attachment to R₈,
   and
R⁸ is

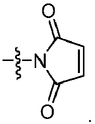

Embodiment 29. The compound of Formula (B), Formula (B-1) or Formula (B-2), wherein the compound is

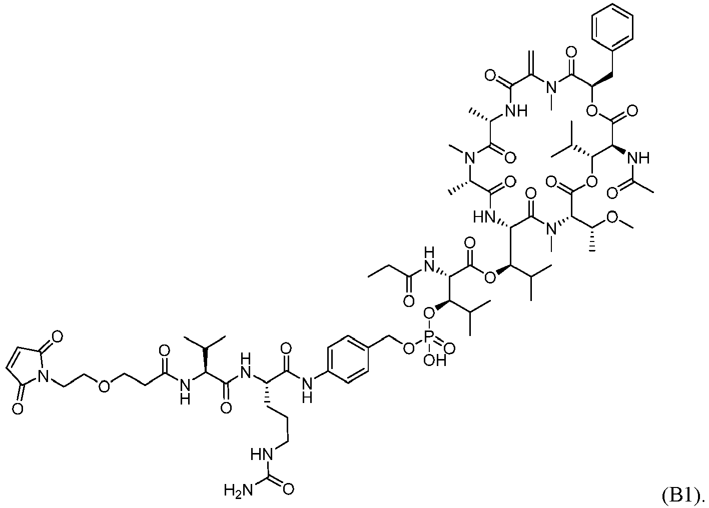

Embodiment 30. The compound of Formula (B), Formula (B-1) or Formula (B-2), wherein the compound is

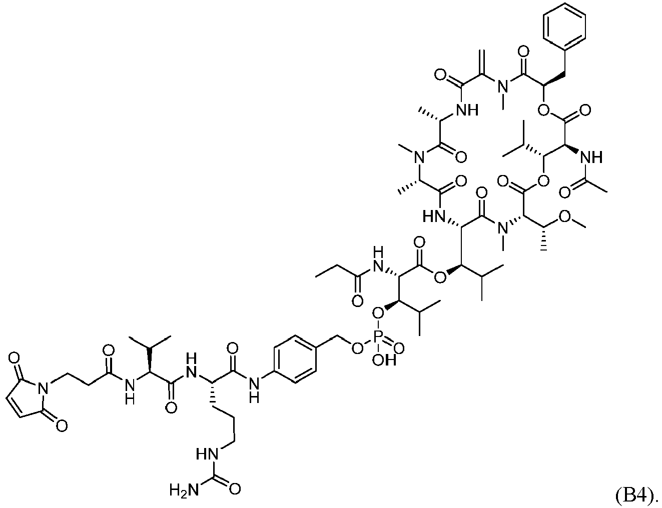

Embodiment 31. The compound of Formula (B), Formula (B-1) or Formula (B-3), wherein the compound is

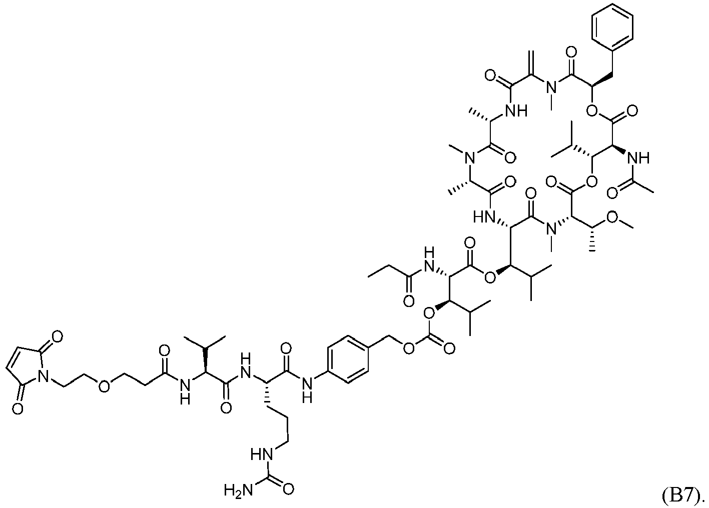

Embodiment 32. The compound of Formula (B), Formula (B-1) or Formula (B-3), wherein the compound is

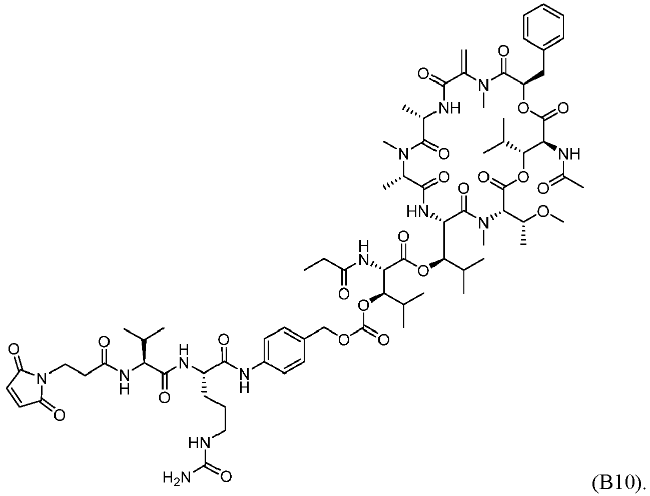

Synthesis of exemplary Linker-Drug Compounds are also described in Example 1 of International Application Publication No. WO 2020/128612, which is incorporated by reference in its entirety.

### Antibody Drug Conjugates

In some embodiments, the antibody drug conjugate of the disclosure is a conjugate of Formula (C):

Ab-(L_{A}-(D)ₙ)_{y} (C)

wherein:
D is a drug moiety;
Ab is an antibody or antigen binding fragment thereof that binds to human PMEL17 protein;
L_{A} is a linker;
n is 1, 2, 3 or 4, and
y is 1, 2, 3 or 4,
where the Linker-Drug moiety (L_{A}-(D)ₙ) is covalently attached to the antibody or antigen binding fragment thereof.

In some embodiments, the Antibody Drug Conjugate of the disclosure having the structure of Formula (C), wherein:
D is a GNAQ inhibitor, a GNA11 inhibitor or an inhibitor of GNAQ and GNA11;
Ab is an antibody or antigen binding fragment thereof that binds to human PMEL17 protein;
L_{A} is a cleavable linker comprising one or more linker components selected from a self-immolative spacer, a phosphate group, a carbonate group and a bivalent peptide linker;
n is 1, 2, 3 or 4, and
y is 1, 2, 3 or 4,
where the Linker-Drug moiety (L_{A}-(D)ₙ) is covalently attached to the antibody or antigen binding fragment thereof.

In some embodiments, the Antibody Drug Conjugate of Formula (C) is a conjugate of Formula (C-1): 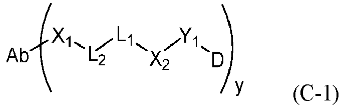 wherein:
D is a GNAQ inhibitor, a GNA11 inhibitor or an inhibitor of GNAQ and GNA11;
Ab is an antibody or antigen binding fragment thereof that binds to human PMEL17 protein;
X₁ is a bivalent coupling group;
X₂ is a self-immolative spacer;
Y₁ is 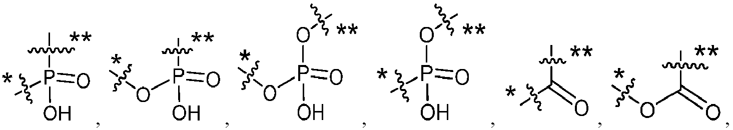 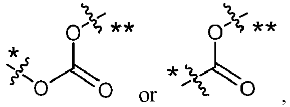 where the * of Y₁ indicates the point of attachment to X₂ and the ** of Y₁ indicates the point of attachment to D;
L₁ is a bivalent peptide linker;
L₂ is a bond or a linker, and
y is 1, 2, 3 or 4.

In the conjugates of Formula (C), one or more Linker-Drug moiety (L_{B}-(D)ₙ) can be covalently attached to the antibody or antigen binding fragment thereof, Ab, thereby covalently attaching one or more drug moieties, D, to the antibody or antigen binding fragment thereof, Ab, through linker, L_{A}. L_{A} is any chemical moiety that is capable of linking the antibody or antigen binding fragment thereof, Ab, to one or more drug moieties, D. The conjugates of Formula (C), wherein one or more drug moieties, D, are covalently linked to an antibody or antigen binding fragment thereof, Ab, can be formed using a bifunctional or multifunctional linker reagent having one or more reactive functional groups that are the same or different. One of the reactive functional groups of the bifunctional or multifunctional linker reagent is used to react with a group on the antibody or antigen binding fragment thereof, Ab, by way of example, a thiol or an amine (*e.g.* a cysteine, an N-terminus or amino acid side chain such as lysine) to form a covalent linkage with one end of the linker L_{A}. Such reactive functional groups of the bifunctional or multifunctional linker reagent include, but are not limited to, a maleimide, a thiol and an NHS ester. The other reactive functional group or groups of the bifunctional or multifunctional linker reagent are used to covalently attached one or more drug moieties, D, to linker L_{A}.

In some embodiments, L_{A} is a cleavable linker. In some embodiments, L_{A} is a non-cleavable linker. In some embodiments, L_{A} is an acid-labile linker, photo-labile linker, peptidase cleavable linker, esterase cleavable linker, glycosidase cleavable linker, phosphodiesterase cleavable linker, a disulfide bond reducible linker, a hydrophilic linker, or a dicarboxylic acid-based linker.

In some embodiments, L_{A} is a cleavable linker comprising one or more linker components selected from a self-immolative spacer, a phosphate group, a carbonate group and a bivalent peptide linker.

In some embodiments, L_{A} is a cleavable linker comprising one or more linker components selected from a self-immolative spacer, a phosphate group, a carbonate group, a bivalent peptide linker and a bivalent coupling group.

In some embodiments, L_{A} is a cleavable linker comprising one or more linker components selected from a self-immolative spacer, a phosphate group and a bivalent peptide linker.

In some embodiments, L_{A} is a cleavable linker comprising one or more linker components selected from a self-immolative spacer, a phosphate group, a bivalent peptide linker and a bivalent coupling group.

In some embodiments, the linker (L_{A}) has the following formula: 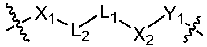 wherein:
X₁ is a bivalent coupling group;
X₂ is a self-immolative spacer;
Y₁ is 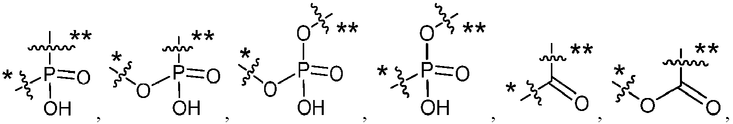 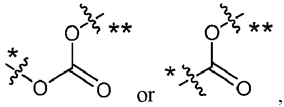 where the * of Y₁ indicates the point of attachment to X₂, and the ** of Y₁ indicates the other point of attachment;
L₁ is a bivalent peptide linker, and
L₂ is a bond or a linker.

In some embodiments, the linker (L_{A}) has the following formula: 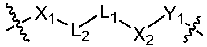 wherein:
X₁ is a bivalent coupling group;
X₂ is a self-immolative spacer;
Y₁ is 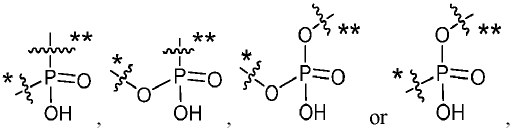 where the * of Y₁ indicates the point of attachment to X₂;
L₁ is a bivalent peptide linker, and
L₂ is a bond or a linker.

In some embodiments, the linker (L_{A}) has the following formula: 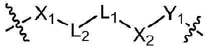 wherein:
X₁ is a bivalent coupling group;
X₂ is a self-immolative spacer;
Y₁ is 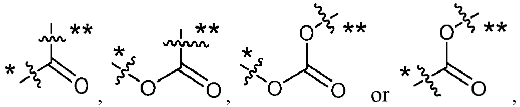 where the * of Y₁ indicates the point of attachment to X₂;
L₁ is a bivalent peptide linker, and
L₂ is a bond or a linker.

While the drug to antibody ratio has an exact integer value for a specific conjugate molecule (*e.g*., the product of n and y in Formula (C), it is understood that the value will often be an average value when used to describe a sample containing many molecules, due to some degree of heterogeneity, typically associated with the conjugation step. The average loading for a sample of a conjugate is referred to herein as the drug to antibody ratio, or "DAR." In some embodiments, the DAR is between about 1 and about 5, and typically is about 1, 2, 3, or 4. In some embodiments, at least 50% of a sample by weight is compound having the average DAR plus or minus 2, and preferably at least 50% of the sample is a conjugate that contains the average DAR plus or minus 1. Other embodiments include conjugates wherein the DAR is about 2. In some embodiments, a DAR of `about y' means the measured value for DAR is within 20% of the product of n and y in Formula (I). In some embodiments, a DAR of `about n' means the measured value for DAR is within 20% of n in Formula (II).

In some embodiments, the average molar ratio of the drug to the antibody in the conjugates of Formula (C) (*i.e.*, average value of the product of n and y, also known as drug to antibody ratio (DAR)) is about 1 to about 10, about 1 to about 6 (*e.g*., 0.9, 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, 5.0, 5.1, 5.2, 5.3, 5.4, 5.5, 5.6, 5.7, 5.8, 5.9, or 6.0), about 1 to about 5, about 1.5 to about 4.5, or about 2 to about 4.

In some embodiments provided by the disclosure, the conjugate has substantially high purity and has one or more of the following features: (a) greater than about 90% (*e.g*., greater than or equal to about 91%, about 92%, about 93%, about 94%, about 95%, about 96%, about 97%, about 98%, about 99%, or about 100%), preferably greater than about 95%, of conjugate species are monomeric, (b) unconjugated linker level in the conjugate preparation is less than about 10% (*e.g*., less than or equal to about 9%, about 8%, about 7%, about 6%, about 5%, about 4%, about 3%, about 2%, about 1%, or about 0%) (relative to total linker), (c) less than 10% of conjugate species are crosslinked (*e.g*., less than or equal to about 9%, about 8%, about 7%, about 6%, about 5%, about 4%, about 3%, about 2%, about 1%, or about 0%), (d) free drug (ADP-induced platelet aggregation inhibitor, *e.g*., a GNAQ inhibitor, a GNA11 inhibitor, or a GNAQ and a GNA11 inhibitor) level in the conjugate preparation is less than about 2% (*e.g*., less than or equal to about 1.5%, about 1.4%, about 1.3%, about 1.2%, about 1.1%, about 1.0%, about 0.9%, about 0.8%, about 0.7%, about 0.6%, about 0.5%, about 0.4%, about 0.3%, about 0.2%, about 0.1%, or about 0%) (mol/mol relative to total drug).

Certain embodiments and examples of the Antibody Drug Conjugates of the disclosure are provided in the following listing of additional, enumerated embodiments. It will be recognized that features specified in each embodiment may be combined with other specified features to provide further embodiments of the present disclosure.

Embodiment 33. The conjugate of Formula (C) or Formula (C-1), wherein D is a GNAQ inhibitor.

Embodiment 34. The conjugate of Formula (C) or Formula (C-1), wherein D is a GNA11 inhibitor.

Embodiment 35. The conjugate of Formula (C) or Formula (C-1), wherein D is an inhibitor of GNAQ and GNA11.

Embodiment 36. The conjugate of Formula (C) or Formula (C-1), wherein D is 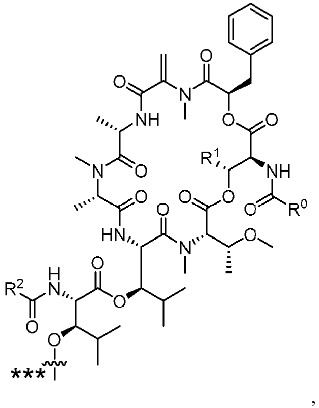 wherein R⁰ is methyl or ethyl, R₁ is methyl or isopropyl, R₂ is methyl or ethyl, and the *** indicates the point of attachment to L_{A} or Y₁.

Embodiment 36a. The conjugate of Formula (Cb) or Formula (Cb-1), wherein D is 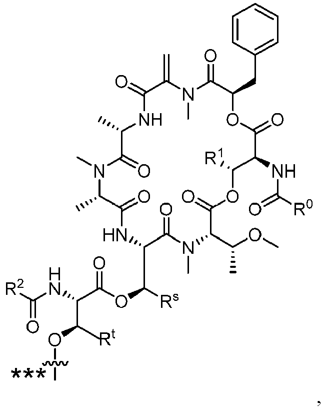 wherein R⁰ is methyl or ethyl, R', R^{s}, and R^{t} are each independently methyl, methylthiomethyl, or isopropyl, R₂ is methyl or ethyl, and the *** indicates the point of attachment to L_{A} or Y₁.

Embodiment 36b. The conjugate of Formula (Cc) or Formula (Cc-1), wherein D is 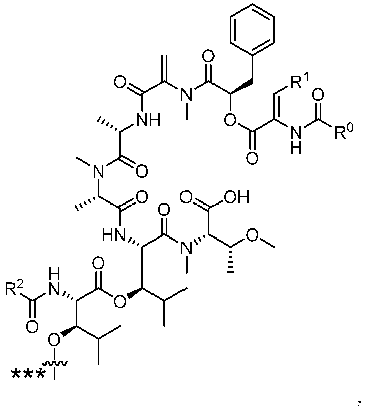 wherein R⁰ is methyl or ethyl, R₁ is methyl or isopropyl, R₂ is methyl or ethyl, and the *** indicates the point of attachment to L_{A} or Y₁.

Embodiment 37. The conjugate of Formula (C) or Formula (C-1), wherein D is 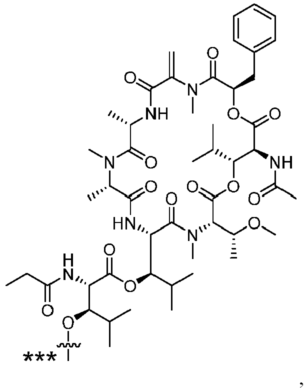 where the *** indicates the point of attachment to L_{A} or Y₁.

Embodiment 38. The conjugate of Formula (C) or Formula (C-1), having the structure of Formula (C-2): 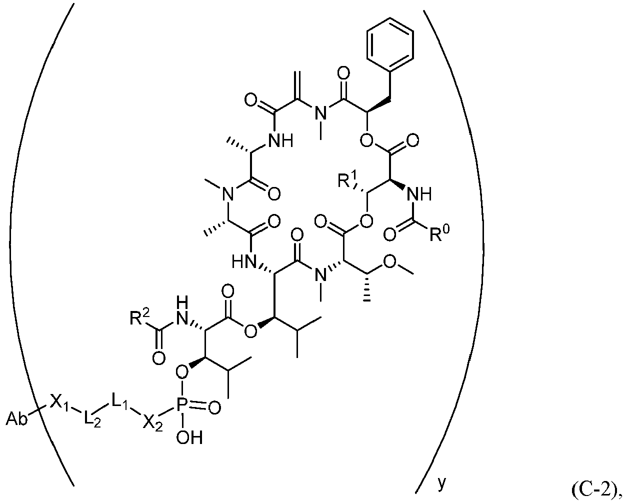 wherein:
R⁰ is methyl or ethyl;
R¹ is methyl or isopropyl;
R² is methyl or ethyl;
Ab is an antibody or antigen binding fragment thereof that binds to human PMEL17 protein;
X₁ is a bivalent coupling group;
X₂ is a self-immolative spacer;
L₁ is a bivalent peptide linker;
L₂ is a bond or a linker, and
y is 1, 2, 3 or 4.

Embodiment 38a. The conjugate of Formula (Cb) or Formula (Cb-1), having the structure of Formula (Cb-2): 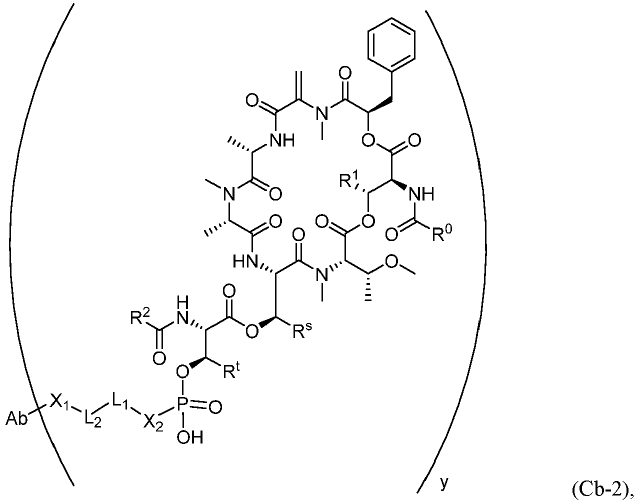 wherein:
R⁰ is methyl or ethyl;
R', R^{s}, and R^{t} are each independently methyl, methylthiomethyl, or isopropyl;
R² is methyl or ethyl;
Ab is an antibody or antigen binding fragment thereof that binds to human PMEL17 protein;
X₁ is a bivalent coupling group;
X₂ is a self-immolative spacer;
L₁ is a bivalent peptide linker;
L₂ is a bond or a linker, and
y is 1, 2, 3 or 4.

Embodiment 38b. The conjugate of Formula (Cc) or Formula (Cc-1), having the structure of Formula (Cc-2): 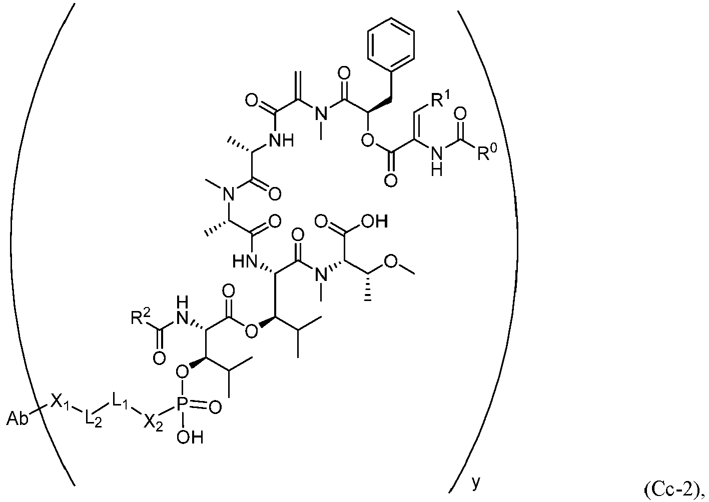 wherein:
R⁰ is methyl or ethyl;
R¹ is methyl or isopropyl;
R² is methyl or ethyl;
Ab is an antibody or antigen binding fragment thereof that binds to human PMEL17 protein;
X₁ is a bivalent coupling group;
X₂ is a self-immolative spacer;
L₁ is a bivalent peptide linker;
L₂ is a bond or a linker, and
y is 1, 2, 3 or 4.

Embodiment 39. The conjugate of Formula (C) or Formula (C-1), having the structure of Formula (C-2a): 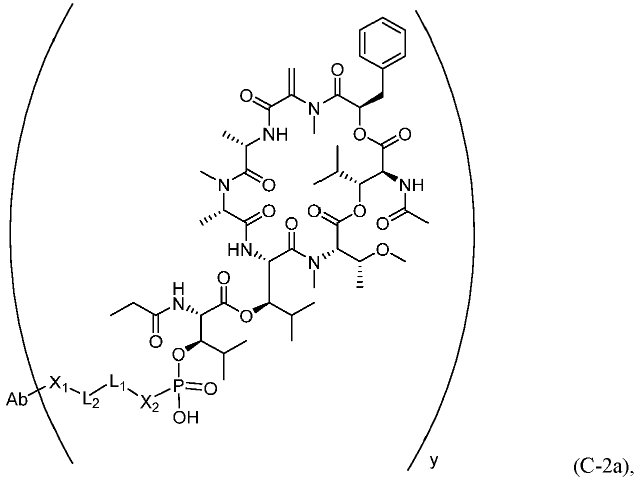 wherein:
Ab is an antibody or antigen binding fragment thereof that binds to human PMEL17 protein;
X₁ is a bivalent coupling group;
X₂ is a self-immolative spacer;
L₁ is a bivalent peptide linker;
L₂ is a bond or a linker, and
y is 1, 2, 3 or 4.

Embodiment 40. The conjugate of Formula (C) or Formula (C-1), having the structure of Formula (C-3): 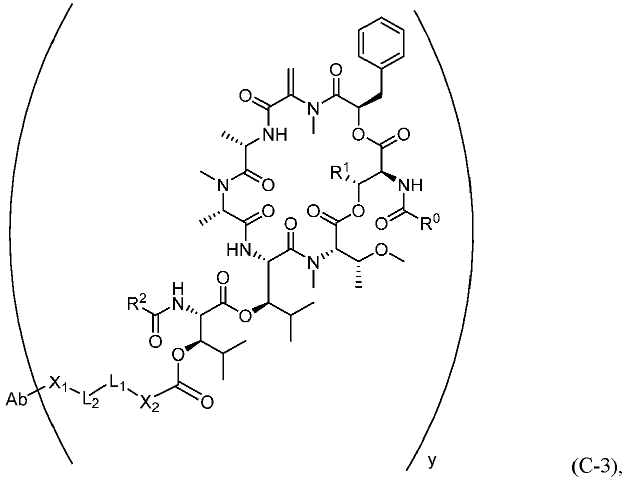 wherein:
R⁰ is methyl or ethyl;
R¹ is methyl or isopropyl;
R² is methyl or ethyl;
Ab is an antibody or antigen binding fragment thereof that binds to human PMEL17 protein;
X₁ is a bivalent coupling group;
X₂ is a self-immolative spacer;
L₁ is a bivalent peptide linker;
L₂ is a bond or a linker, and
y is 1, 2, 3 or 4.

Embodiment 40a. The conjugate of Formula (Cb) or Formula (Cb-1), having the structure of Formula (Cb-3): 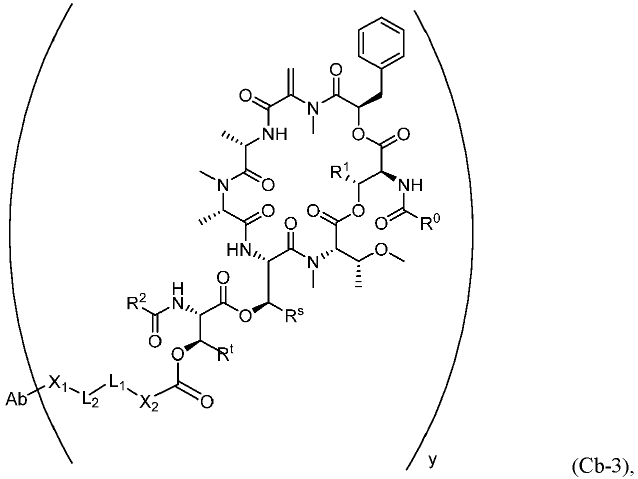 wherein:
R⁰ is methyl or ethyl;
R¹, R^{s}, and R^{t} are each independently methyl, methylthiomethyl, or isopropyl;
R² is methyl or ethyl;
Ab is an antibody or antigen binding fragment thereof that binds to human PMEL17 protein;
X₁ is a bivalent coupling group;
X₂ is a self-immolative spacer;
L₁ is a bivalent peptide linker;
L₂ is a bond or a linker, and
y is 1, 2, 3 or 4.

Embodiment 40b. The conjugate of Formula (Cc) or Formula (Cc-1), having the structure of Formula (Cc-3): 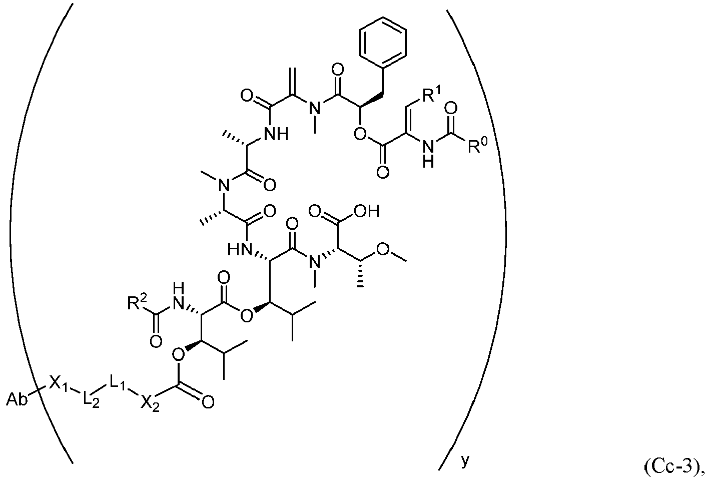 wherein:
R⁰ is methyl or ethyl;
R¹ is methyl or isopropyl;
R² is methyl or ethyl;
Ab is an antibody or antigen binding fragment thereof that binds to human PMEL17 protein;
X₁ is a bivalent coupling group;
X₂ is a self-immolative spacer;
L₁ is a bivalent peptide linker;
L₂ is a bond or a linker, and
y is 1, 2, 3 or 4.

Embodiment 41. The conjugate of Formula (C) or Formula (C-1), having the structure of Formula (C-3a): 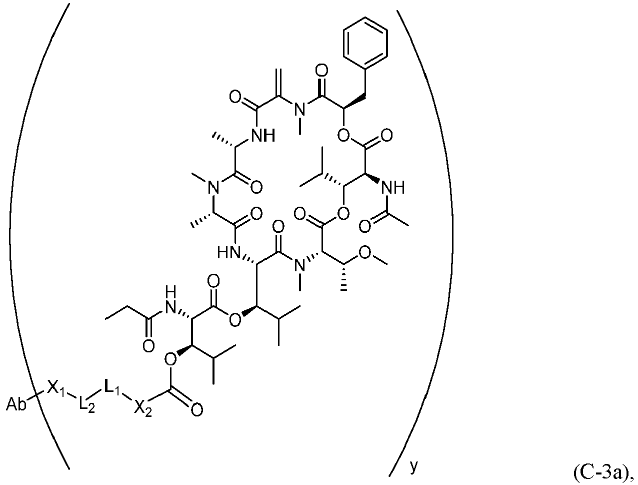 wherein:
Ab is an antibody or antigen binding fragment thereof that binds to human PMEL17 protein;
X₁ is a bivalent coupling group;
X₂ is a self-immolative spacer;
L₁ is a bivalent peptide linker;
L₂ is a bond or a linker, and
y is 1, 2, 3 or 4.

Embodiment 42. The conjugate of Formula (C-2) of Embodiment 54 or the conjugate of Formula (C-3) of Embodiment 58:
wherein:
R⁰ is methyl or ethyl;
R¹ is methyl or isopropyl;
R² is methyl or ethyl;
Ab is an antibody or antigen binding fragment thereof that binds to human PMEL17 protein;
X₂ is a self-immolative spacer selected from 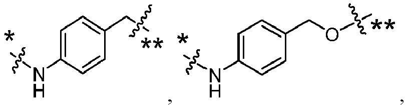 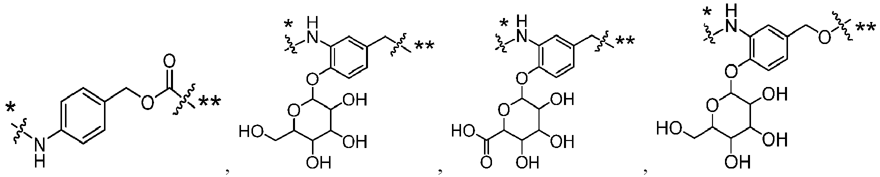 and 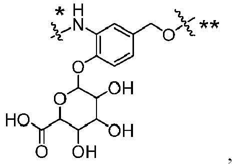 where the * of X₂ indicates the point of attachment to L₁ and the ** of X₂ indicates the point of attachment to the 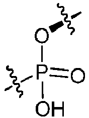 group or the point of attachment to 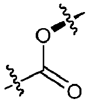 group;
L₁ is a bivalent peptide linker comprising 2 to 4 amino acid residues;
L₂ is a linker;
X₁ is a bivalent coupling group selected from 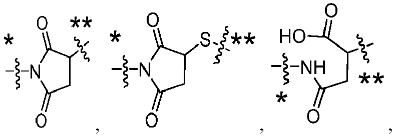 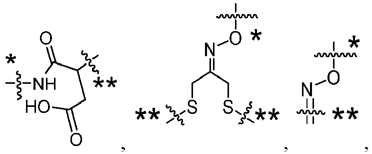 -*NR⁴C(=O)CH₂**-, -*NHC(=O)CH₂**-, - *S(=O)₂CH₂CH₂**-, -* (CH₂)₂S(=O)₂CH₂CH₂**-, -*NR⁴S(=O)₂CH₂CH₂**-, - *NR⁴C(=O)CH₂CH₂**-, -NH-, -C(=O)-, -*NHC(=O) **-, -*CH₂NHCH₂CH₂**-, - *NHCH₂CH₂**-, -S-, 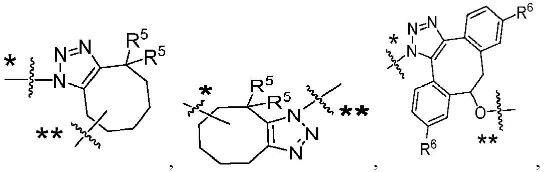 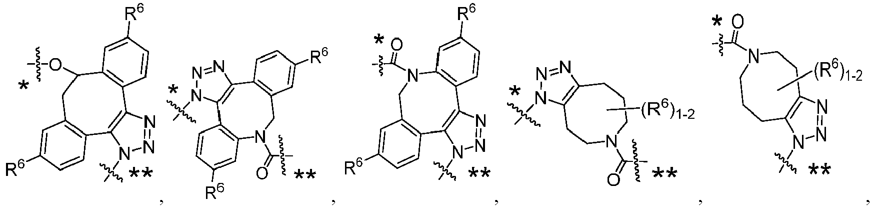 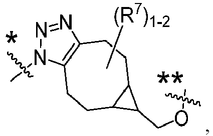 **-, where the * of X₁ indicates the point of attachment to L₂ and the ** of X₁ indicates the point of attachment to Ab; and wherein:
   each R⁴ is independently selected from H and C₁-C₆alkyl;
   each R⁵ is independently selected from H, C₁-C₆alkyl, F, Cl, and -OH;
   each R⁶ is independently selected from H, C₁-C₆alkyl, F, Cl, -NH₂, -OCH₃, -OCH₂CH₃, - N(CH₃)₂, -CN, -NO₂ and -OH, and
   each R⁷ is independently selected from H, C₁₋₆alkyl, fluoro, benzyloxy substituted with - C(=O)OH, benzyl substituted with -C(=O)OH, C₁₋₄alkoxy substituted with -C(=O)OH and C₁₋₄alkyl substituted with -C(=O)OH, and
   y is 1, 2, 3 or 4.

Embodiment 43. The conjugate of any one of Embodiments 38 to 42, wherein X₂ is a self-immolative spacer selected from 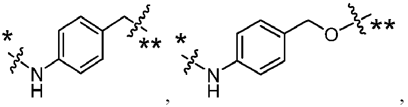 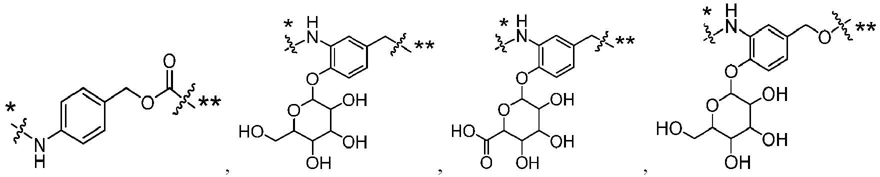 and 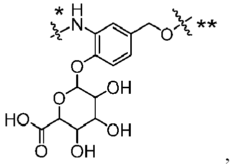 where the * of X₂ indicates the point of attachment to L₁ and the ** of X₂ indicates the point of attachment to Y₁ or the point of attachment to the 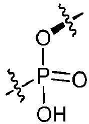 group or the point of attachment to 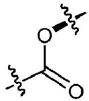 group.

Embodiment 44. The conjugate of any one of Embodiments 38 to 43, wherein X₂ is 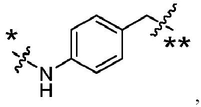 where the * of X₂ indicates the point of attachment to L₁ and the ** of X₂ indicates the point of attachment to Y₁ or the point of attachment to the 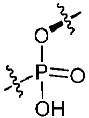 group or the point of attachment to 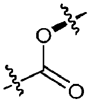 group.

Embodiment 45. The conjugate of any one of Embodiments 38 to 44, wherein L₁ is a bivalent peptide linker comprising 2 to 4 amino acid residues.

Embodiment 46. The conjugate of any one of Embodiments 38 to 45, wherein L₁ is abivalent peptide linker comprising an amino acid residue selected from valine, citrulline, lysine, isoleucine, phenylalanine, methionine, asparagine, proline, alanine, leucine, tryptophan, and tyrosine.

Embodiment 47. The conjugate of any one of Embodiments 38-44, wherein L₁ is a bivalent peptide linker comprising at least one valine (Val) or citrulline (Cit) residue.

Embodiment 48. The conjugate of any one of Embodiments 38 to 44, wherein L₁ is a bivalent dipeptide linker selected from ValCit, PheLys, ValAla and ValLys.

Embodiment 49. The conjugate of any one of Embodiments 38 to 44, wherein L₁ is a bivalent dipeptide linker selected from 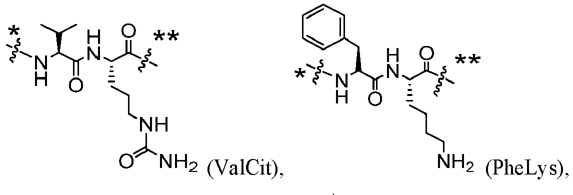 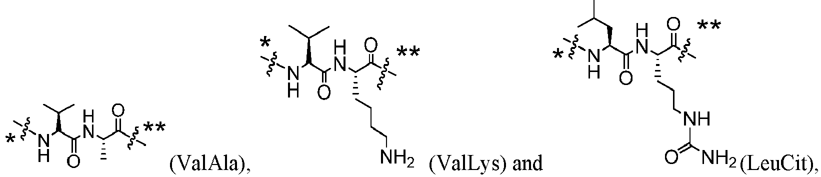 where the * of L₁ indicates the attachment point to L₂ and the ** of L₁ indicates the attachment point to X₂.

Embodiment 50. The conjugate of any one of Embodiments 38 to 44, wherein L₁ is ValCit.

Embodiment 51. The conjugate of any one of Embodiments 38 to 44, wherein
L₁ is 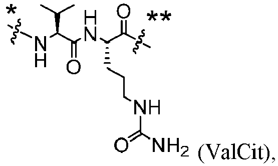 where the * of L₁ indicates the attachment point to L₂ and the ** of L₁ indicates the attachment point to X₂.

Embodiment 52. The conjugate of any one of Embodiments 38 to 51, wherein L₂ is a linker.

Embodiment 53. The conjugate of any one of Embodiments 38 to 51, wherein L₂ is a linker selected from:
-*C(=O)((CH₂)ₘO)ₚ(CH₂)ₘ**-, -*C(=O)(CH₂)ₘ**-, -*C(=O)(CH₂)ₙNHC(=O)(CH₂)ₘ**-, - *C(=O)(CH₂)ₘNHC(=O)((CH₂)ₘO)ₚ(CH₂)ₘ**-, -* ((CH₂)ₘO)ₚ(CH₂)ₘ**-, -* ((CH₂)ₘO)ₚ(CH₂)ₘ**-, - (CH₂)ₘ-, -*(CH₂)ₘNHC(=O)(CH₂)ₘ**-, -* (CH₂)ₘNHC(=O)(CH₂)ₘC(=O)NH(CH₂)ₘ**-, * ((CH₂)ₘO)ₚ(CH₂)ₘNHC(=O)(CH₂)ₘ**-, * *((CH₂)ₘO)ₚCH₂)ₘC(=O)NH(CH₂)ₘ**-, -* (CH₂)ₘC(R₃)₂**-, and -* (CH₂)ₘC(R₃)₂SS(CH₂)ₘNHC(=O)(CH₂)ₘ**-, where the * of L₂ indicates the attachment point to L₁ and the ** of L₂ indicates the point of attachment to X₁;
and wherein:
   each R₃ is independently selected from H and C₁-C₆alkyl;
   each m is independently selected from 1, 2, 3, 4, 5, 6, 7, 8, 9 and 10, and
   each p is independently selected from 1, 2, 3, 4, 5, 6, 7, 8, 9, 10, 11, 12, 13 and 14.
Embodiment 54. The conjugate of any one of Embodiments 38 to 51, wherein L₂ is - *C(=O)((CH₂)ₘO)ₚ(CH₂)ₘ**- or -*C(=O)(CH₂)ₘ**-, where the * of L₂ indicates the point of attachment to L₁ and the ** of L₂ indicates the point of attachment to X₁,
   and wherein each m is independently selected from 1, 2, 3, 4, 5, 6, 7, 8, 9 or 10 and p is 1, 2, 3, 4, 5, 6, 7, 8, 9, 10, 11, 12, 13 or 14.

Embodiment 55. The conjugate of any one of Embodiments 38 to 51, wherein L₂ is 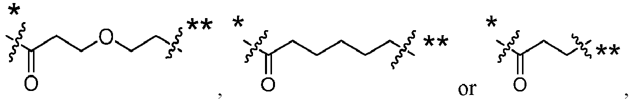 where the * of L₂ indicates the point of attachment to L₁ and the ** of L₂ indicates the point of attachment to X₁.

Embodiment 56. The conjugate of any one of Embodiments 38 to 55, wherein X₁ is a bivalent coupling group selected from 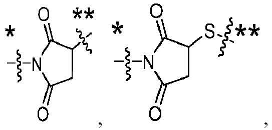 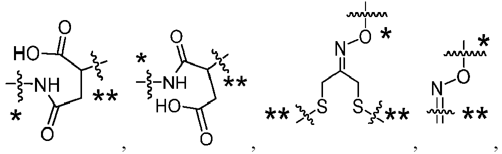 -*NR⁴C(=O)CH₂**-, - *NHC(=O)CH₂**-, -*S(=O)₂CH₂CH₂**-, -* (CH₂)₂S(=O)₂CH₂CH₂**-, - *NR⁴S(=O)₂CH₂CH₂**-, -*NR⁴C(=O)CH₂CH₂**-, -NH-, -C(=O)-, -*NHC(=O) **-, - *CH₂NHCH₂CH₂**-, -*NHCH₂CH₂**-, -S-, 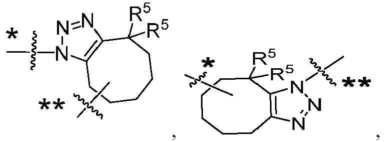 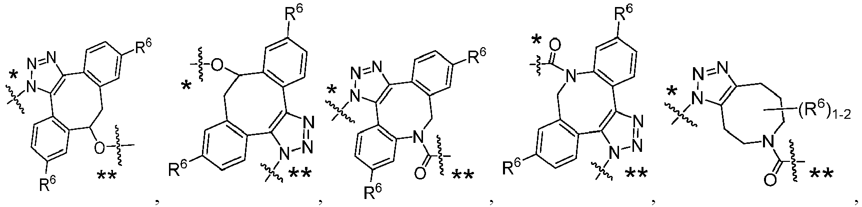 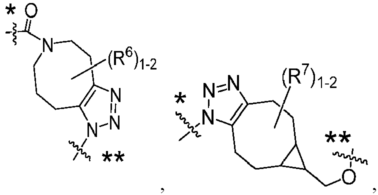 **-, where the * of X₁ indicates the point of attachment to L₂ and the ** of X₁ indicates the point of attachment to Ab;
and wherein:
each R⁴ is independently selected from H and C₁-C₆alkyl;
each R⁵ is independently selected from H, C₁-C₆alkyl, F, Cl, and -OH;
each R⁶ is independently selected from H, C₁-C₆alkyl, F, Cl, -NH₂, -OCH₃, -OCH₂CH₃, - N(CH₃)₂, -CN, -NO₂ and -OH,
   and
each R⁷ is independently selected from H, C₁₋₆alkyl, fluoro, benzyloxy substituted with - C(=O)OH, benzyl substituted with -C(=O)OH, C₁₋₄alkoxy substituted with -C(=O)OH and C₁₋₄alkyl substituted with -C(=O)OH.

Embodiment 57. The conjugate of any one of Embodiments 38 to 55,
wherein X₁ is a bivalent coupling group selected from 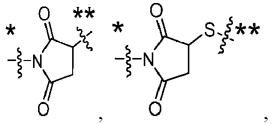 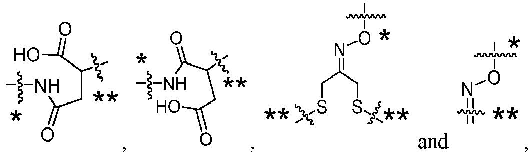 where the * of X₁ indicates the point of attachment to L₂ and the ** of X₁ indicates the point of attachment to Ab.

Embodiment 58. The conjugate of any one of Embodiments 38 to 55, wherein X₁ is a bivalent coupling group selected from 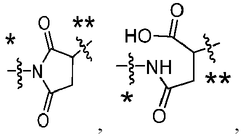 and 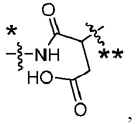 where the * of X₁ indicates the point of attachment to L₂ and the ** of X₁ indicates the point of attachment to Ab.

Embodiment 59. The conjugate of Formula (C-2) of Embodiment 38 wherein:
Ab is an antibody or antigen binding fragment thereof that binds to human PMEL17 protein;
R⁰ is methyl or ethyl;
R¹ is methyl or isopropyl;
R² is methyl or ethyl;
X₁ is a bivalent coupling group selected from 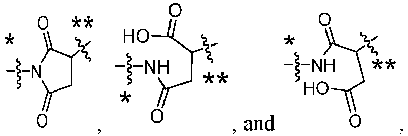 where the * of X₁ indicates the point of attachment to L₂ and the ** of X₁ indicates the point of attachment to Ab
X₂ is 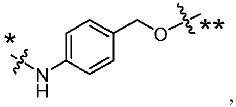 where the * of X₂ indicates the point of attachment to L₁ and the ** of X₂ indicates the point of attachment to the 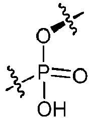 group;
L₁ is 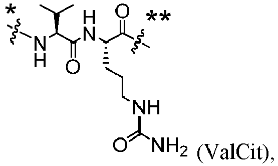 where the * of L₁ indicates the attachment point to L₂ and the ** of L₁ indicates the attachment point to X₂;
L₂ is 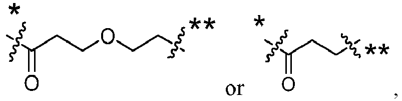 where the * of L₂ indicates the point of attachment to L₁ and the ** of L₂ indicates the point of attachment to R₈,
   and
y is 1, 2, 3 or 4.

Embodiment 60. The conjugate of Formula (C-3) of Embodiment 40 wherein:
Ab is an antibody or antigen binding fragment thereof that binds to human PMEL17 protein;
R⁰ is methyl or ethyl;
R¹ is methyl or isopropyl;
R² is methyl or ethyl;
X₁ is a bivalent coupling group selected from 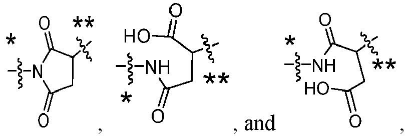 where the * of X₁ indicates the point of attachment to L₂ and the ** of X₁ indicates the point of attachment to Ab
X₂ is 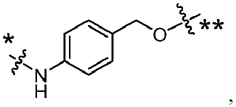 where the * of X₂ indicates the point of attachment to L₁ and the ** of X₂ indicates the point of attachment to the 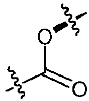 group;
L₁ is 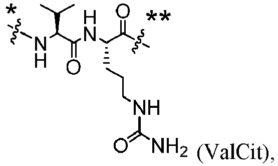 where the * of L₁ indicates the attachment point to L₂ and the ** of L₁ indicates the attachment point to X₂;
L₂ is 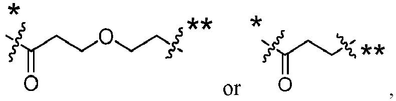 where the * of L₂ indicates the point of attachment to L₁ and the ** of L₂ indicates the point of attachment to R₈,
   and
y is 1, 2, 3 or 4.

Embodiment 61. The conjugate of Formula (C), Formula (C-1) or Formula (C-2) having the structure: 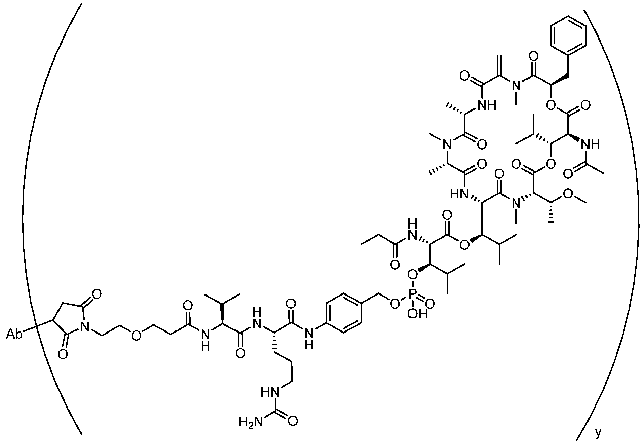 wherein:
y is 2 or 4 and
Ab is an antibody or antigen binding fragment thereof that binds to human PMEL17 protein.

Embodiment 62. The conjugate of Formula (C), Formula (C-1) or Formula (C-2) having the structure: 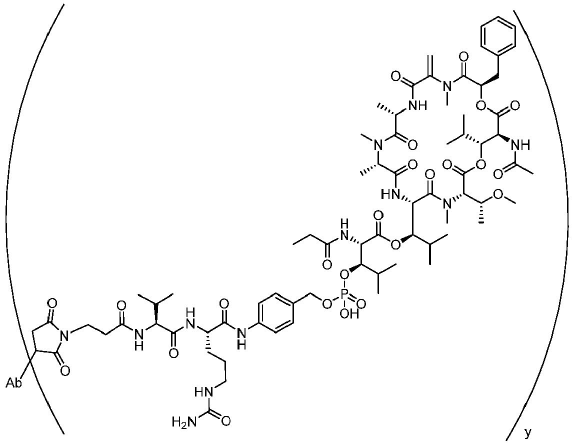 wherein:
y is 2 or 4 and
Ab is an antibody or antigen binding fragment thereof that binds to human PMEL17 protein.

Embodiment 63. The conjugate of Formula (C), Formula (C-1) or Formula (C-3) having the structure: 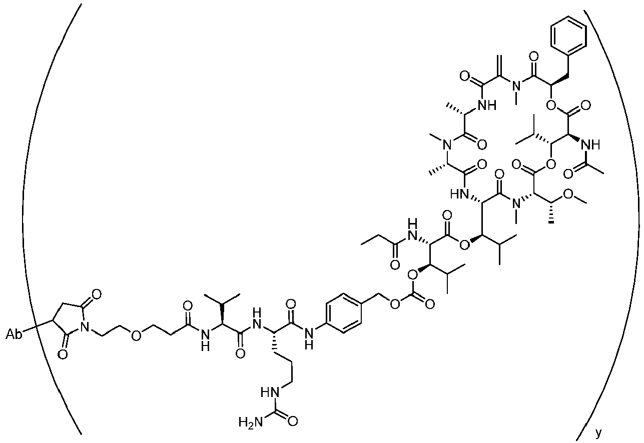 wherein:
y is 2 or 4 and
Ab is an antibody or antigen binding fragment thereof that binds to human PMEL17 protein.

Embodiment 64. The conjugate of Formula (C), Formula (C-1) or Formula (C-3) having the structure: 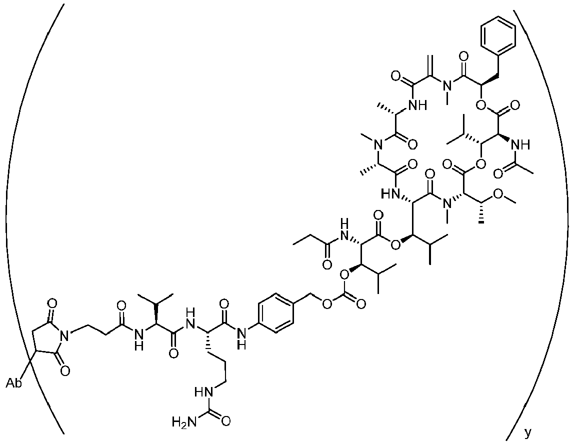 wherein:
y is 2 or 4 and
Ab is an antibody or antigen binding fragment thereof that binds to human PMEL17 protein.

Embodiment 65. The conjugate of compound (E) having the structure: 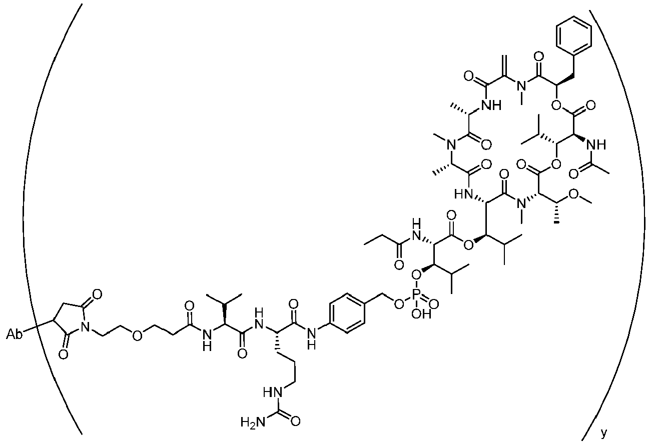 wherein:
y is 2; and
Ab is an antibody or antigen binding fragment thereof that binds to human PMEL17 protein.

Further, the antibodies, antibody fragments (*e*.*g*., antigen binding fragments) or functional equivalents of the present disclosure may be conjugated to a drug moiety that modifies a given biological response. Drug moieties are not to be construed as limited to classical chemical therapeutic agents. For example, the drug moiety may be a protein, peptide, or polypeptide possessing a desired biological activity. Such proteins may include, for example, a toxin such as abrin, ricin A, pseudomonas exotoxin, cholera toxin, or diphtheria toxin, a protein such as tumor necrosis factor, α-interferon, β-interferon, nerve growth factor, platelet derived growth factor, tissue plasminogen activator, a cytokine, an apoptotic agent, an anti-angiogenic agent, or, a biological response modifier such as, for example, a lymphokine.

In one embodiment, the antibodies, antibody fragments (*e*.*g*., antigen binding fragments) or functional equivalents of the present disclosure are conjugated to a drug moiety, such as a cytotoxin, a drug (*e*.*g*., an immunosuppressant) or a radiotoxin. Examples of cytotoxins include but are not limited to, taxanes (*see, e.g.,* International (PCT) Patent Application Nos. WO 01/38318 and PCT/US03/02675), DNA-alkylating agents (*e*.*g*., CC-1065 analogs), anthracyclines, tubulysin analogs, duocarmycin analogs, auristatin E, auristatin F, maytansinoids, pyrrolobenzodiazipines (PBDs), and cytotoxic agents comprising a reactive polyethylene glycol moiety (*see, e.g.,* Sasse et al., J. Antibiot. (Tokyo), 53, 879-85 (2000), Suzawa et al., Bioorg. Med. Chem., 8, 2175-84 (2000), Ichimura et al., J. Antibiot. (Tokyo), 44, 1045-53 (1991), Francisco et al., Blood (2003) (electronic publication prior to print publication), U.S. Pat. Nos. 5,475,092, 6,340,701, 6,372,738, and 6,436,931, U.S. Patent Application Publication No. 2001/0036923 A1, Pending U.S. patent application Ser. Nos. 10/024,290 and 10/116,053, and International (PCT) Patent Application No. WO 01/49698), taxon, cytochalasin B, gramicidin D, ethidium bromide, emetine, mitomycin, etoposide, tenoposide, vincristine, vinblastine, t. colchicin, doxorubicin, daunorubicin, dihydroxy anthracin dione, mitoxantrone, mithramycin, actinomycin D, 1-dehydrotestosterone, glucocorticoids, procaine, tetracaine, lidocaine, propranolol, and puromycin and analogs or homologs thereof. Therapeutic agents also include, for example, anti-metabolites (*e*.*g*., methotrexate, 6-mercaptopurine, 6-thioguanine, cytarabine, 5-fluorouracil decarbazine), ablating agents (*e*.*g*., mechlorethamine, thiotepa chlorambucil, meiphalan, carmustine (BSNU) and lomustine (CCNU), cyclophosphamide, busulfan, dibromomannitol, streptozotocin, mitomycin C, and cis-dichlorodiamine platinum (II) (DDP) cisplatin, anthracyclines (*e*.*g*., daunorubicin (formerly daunomycin) and doxorubicin), antibiotics (*e.g*., dactinomycin (formerly actinomycin), bleomycin, mithramycin, and anthramycin (AMC)), and anti-mitotic agents (*e*.*g*., vincristine and vinblastine). (See *e.g.,* Seattle Genetics US20090304721).

Other examples of cytotoxins that can be conjugated to the antibodies, antibody fragments (antigen binding fragments) or functional equivalents of the disclosure include duocarmycins, calicheamicins, maytansines and auristatins, and derivatives thereof.

Various types of cytotoxins, linkers and methods for conjugating therapeutic agents to antibodies are known in the art, *see, e.g.,* Saito et al., (2003) Adv. Drug Deliv. Rev. 55:199-215; Trail et al., (2003) Cancer Immunol. Immunother. 52:328-337; Payne, (2003) Cancer Cell 3:207-212; Allen, (2002) Nat. Rev. Cancer 2:750-763; Pastan and Kreitman, (2002) Curr. Opin. Investig. Drugs 3:1089-1091; Senter and Springer, (2001) Adv. Drug Deliv. Rev. 53:247-264.

The antibodies, antibody fragments (*e*.*g*., antigen binding fragments) or functional equivalents of the present disclosure can also be conjugated to a radioactive isotope to generate cytotoxic radiopharmaceuticals, referred to as radioimmunoconjugates. Examples of radioactive isotopes that can be conjugated to antibodies for use diagnostically or therapeutically include, but are not limited to, iodine-131, indium-111, yttrium-90, and lutetium-177. Methods for preparing radioimmunoconjugates are established in the art. Examples of radioimmunoconjugates are commercially available, including Zevalin^{™} (DEC Pharmaceuticals) and Bexxar^{™} (Corixa Pharmaceuticals), and similar methods can be used to prepare radioimmunoconjugates using the antibodies of the disclosure. In certain embodiments, the macrocyclic chelator is 1,4,7,10-tetraazacyclododecane-N,N',N",N‴-tetraacetic acid (DOTA) which can be attached to the antibody via a linker molecule. Such linker molecules are commonly known in the art and described in Denardo et al., (1998) Clin Cancer Res. 4(10):2483-90; Peterson et al., (1999) Bioconjug. Chem. 10(4):553-7; and Zimmerman et al., (1999) Nucl. Med. Biol. 26(8):943-50, each incorporated by reference in their entireties.

The antibodies, antibody fragments (*e*.*g*., antigen binding fragments) or functional equivalents of the present disclosure can also conjugated to a heterologous protein or polypeptide (or fragment thereof, preferably to a polypeptide of at least about 10, at least about 20, at least about 30, at least about 40, at least about 50, at least about 60, at least about 70, at least about 80, at least about 90 or at least about 100 amino acids) to generate fusion proteins. In particular, the disclosure provides fusion proteins comprising an antibody fragment (*e*.*g*., antigen binding fragment) described herein (*e*.*g*., a Fab fragment, Fd fragment, Fv fragment, F(ab)₂ fragment, a VH domain, a VH CDR, a VL domain or a VL CDR) and a heterologous protein, polypeptide, or peptide.

Additional fusion proteins may be generated through the techniques of gene-shuffling, motif-shuffling, exon-shuffling, and/or codon-shuffling (collectively referred to as "DNA shuffling"). DNA shuffling may be employed to alter the activities of antibodies of the disclosure or fragments thereof (*e*.*g*., antibodies or fragments thereof with higher affinities and lower dissociation rates). *See, generally,* U.S. Patent Nos. 5,605,793, 5,811,238, 5,830,721, 5,834,252, and 5,837,458; Patten et al., (1997) Curr. Opinion Biotechnol. 8:724-33; Harayama, (1998) Trends Biotechnol. 16(2):76-82; Hansson et al., (1999) J. Mol. Biol. 287:265-76; and Lorenzo and Blasco, (1998) Biotechniques 24(2):308- 313 (each of these patents and publications are hereby incorporated by reference in its entirety). Antibodies or fragments thereof, or the encoded antibodies or fragments thereof, may be altered by being subjected to random mutagenesis by error-prone PCR, random nucleotide insertion or other methods prior to recombination. A polynucleotide encoding an antibody or fragment thereof that specifically binds to an antigen may be recombined with one or more components, motifs, sections, parts, domains, fragments, etc. of one or more heterologous molecules.

Moreover, the antibodies, antibody fragments (*e*.*g*., antigen binding fragments) or functional equivalents of the present disclosure can be conjugated to marker sequences, such as a peptide, to facilitate purification. In preferred embodiments, the marker amino acid sequence is a hexa-histidine peptide (SEQ ID NO: 267), such as the tag provided in a pQE vector (QIAGEN, Inc., 9259 Eton Avenue, Chatsworth, CA, 91311), among others, many of which are commercially available. As described in Gentz et al., (1989) Proc. Natl. Acad. Sci. USA 86:821-824, for instance, hexa-histidine (SEQ ID NO: 267) provides for convenient purification of the fusion protein. Other peptide tags useful for purification include, but are not limited to, the hemagglutinin ("HA") tag, which corresponds to an epitope derived from the influenza hemagglutinin protein (Wilson et al., (1984) Cell 37:767), and the "FLAG" tag (A. Einhauer et al., J. Biochem. Biophys. Methods 49: 455-465, 2001). According to the present disclosure, antibodies or antigen binding fragments can also be conjugated to tumor-penetrating peptides in order to enhance their efficacy.

In other embodiments, the antibodies, antibody fragments (e.g., antigen binding fragments) or functional equivalents of the present disclosure are conjugated to a diagnostic or detectable agent. Such immunoconjugates can be useful for monitoring or prognosing the onset, development, progression and/or severity of a disease or disorder as part of a clinical testing procedure, such as determining the efficacy of a particular therapy. Such diagnosis and detection can be accomplished by coupling the antibody to detectable substances including, but not limited to, various enzymes, such as, but not limited to, horseradish peroxidase, alkaline phosphatase, beta-galactosidase, or acetylcholinesterase; prosthetic groups, such as, but not limited to, streptavidin/biotin and avidin/biotin; fluorescent materials, such as, but not limited to, Alexa Fluor 350, Alexa Fluor 405, Alexa Fluor 430, Alexa Fluor 488, Alexa Fluor 500, Alexa Fluor 514, Alexa Fluor 532, Alexa Fluor 546, Alexa Fluor 555, Alexa Fluor 568, Alexa Fluor 594, Alexa Fluor 610, Alexa Fluor 633, Alexa Fluor 647, Alexa Fluor 660, Alexa Fluor 680, Alexa Fluor 700, Alexa Fluor 750, umbelliferone, fluorescein, fluorescein isothiocyanate, rhodamine, dichlorotriazinylamine fluorescein, dansyl chloride or phycoerythrin; luminescent materials, such as, but not limited to, luminol; bioluminescent materials, such as but not limited to, luciferase, luciferin, and aequorin; radioactive materials, such as, but not limited to, iodine (¹³¹I, ¹²⁵I, ¹²³I, and ¹²¹I,), carbon (¹⁴C), sulfur (³⁵S), tritium (³H), indium (¹¹⁵In, ¹¹³In, ¹¹²In, and ¹¹¹In,), technetium (⁹⁹Tc), thallium (²⁰¹Ti), gallium (⁶⁸Ga, ⁶⁷Ga), palladium (¹⁰³Pd), molybdenum (⁹⁹Mo), xenon (¹³³Xe), fluorine (¹⁸F), ¹⁵³Sm, ¹⁷⁷Lu, ¹⁵⁹Gd, ¹⁴⁹Pm, ¹⁴⁰La, ¹⁷⁵Yb, ¹⁶⁶Ho, ⁹⁰Y, ⁴⁷Sc, ¹⁸⁶Re, ¹⁸⁸Re, ¹⁴²Pr, ¹⁰⁵Rh, ⁹⁷Ru, ⁶⁸Ge, ⁵⁷Co, ⁶⁵Zn, ⁸⁵Sr, ³²P, ¹⁵³Gd, ¹⁶⁹Yb, ⁵¹Cr, ⁵⁴Mn, ⁷⁵Se, ⁶⁴Cu, ¹¹³Sn, and ¹¹⁷Sn; and positron emitting metals using various positron emission tomographies, and nonradioactive paramagnetic metal ions.

The antibodies, antibody fragments (*e*.*g*., antigen binding fragments) or functional equivalents of the disclosure may also be attached to solid supports, which are particularly useful for immunoassays or purification of the target antigen. Such solid supports include, but are not limited to, glass, cellulose, polyacrylamide, nylon, polystyrene, polyvinyl chloride or polypropylene.

### Conjugation and Preparation of ADCs

### Processes for Making Antibody Drug conjugates of Formula (C), Formula (C-1) and Formula (C-2)

A general reaction scheme for the formation of conjugates of Formula (C) is shown in Scheme 1 below: 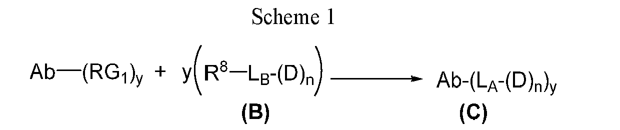 where: RG₁ is a reactive group on the antibody or antigen binding fragment thereof, Ab, by way of example only a thiol, amine, or ketone, which reacts with a compatible reactive group, R⁸, attached to the linker-drug compound thereby covalently linking antibody or antigen binding fragment thereof, Ab, to one or more linker-drug moieties. Non-limiting examples of such reactions of RG₁ and R⁸ groups are a maleimide (R⁸) reacting with a thiol (RG₁) to give a succinimide ring, or a hydroxylamine (R⁸) reacting with a ketone (RG₁) to give an oxime.

In one embodiment, D is a GNAQ inhibitor, a GNA11 inhibitor, or an inhibitor of GNAQ and GNA11 (GNAQ/GNA11 inhibitor), Lₐ is a linker further comprising a bivalent coupling group formed when RG₁ and R⁸ react, n is 1, 2, 3 or 4, and y is 1, 2, 3 or 4.

A general reaction scheme for the formation of conjugates of Formula (C-1) is shown in Scheme 2 below: 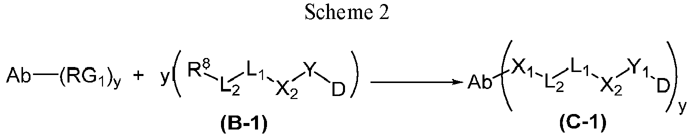 where: RG₁ is a reactive group on the antibody or antigen binding fragment thereof, Ab, by way of example only a thiol, amine, or ketone, which reacts with a compatible reactive group, R⁸, attached to the linker-drug moiety thereby covalently linking antibody or antigen binding fragment thereof, Ab, to one or more linker-drug moieties. Non-limiting examples of such reactions of RG₁ and R⁸ groups are a maleimide (R⁸) reacting with a thiol (RG₁) to give a succinimide ring, or a hydroxylamine (R⁸) reacting with a ketone (RG₁) to give an oxime.

In one embodiment, D is a GNAQ inhibitor, a GNA11 inhibitor, or an inhibitor of GNAQ and GNA11 (GNAQ/GNA11 inhibitor), X₁ is a bivalent coupling group formed when RG₁ and R⁸ react (*e*.*g*., a succinimide ring or an oxime), Y₁ is a phosphate group, X₂ is a self-immolative spacer, L₁ is a bivalent peptide linker, L₂ is a bond or a linker, and y is 1, 2, 3 or 4.

A general reaction scheme for the formation of conjugates of Formula (C-2) is shown in Scheme 3 below: 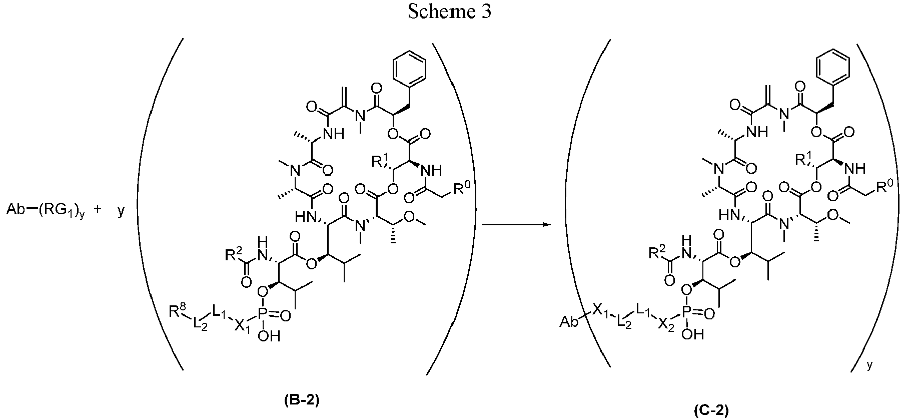 where: RG₁ is a reactive group on the antibody or antigen binding fragment thereof, Ab, by way of example only a thiol, amine, or ketone, which reacts with a compatible reactive group, R⁸, attached to the linker-drug moiety thereby covalently linking antibody or antigen binding fragment thereof, Ab, to one or more linker-drug moieties. Non-limiting examples of such reactions of RG₁ and R⁸ groups are a maleimide (R⁸) reacting with a thiol (RG₁) to give a succinimide ring, or a hydroxylamine (R⁸) reacting with a ketone (RG₁) to give an oxime.

Here R⁰ is methyl, R¹ is isopropyl, R² is ethyl, X₁ is a bivalent coupling group formed when RG₁ and R⁸ react (*e.g.* a succinimide ring or an oxime), X₂ is a self-immolative spacer, L₁ is a bivalent peptide linker, L₂ is a bond or a linker, and y is 1, 2, 3 or 4.

A general reaction scheme for the formation of conjugates of Formula (C-2) is shown in Scheme 4 below: 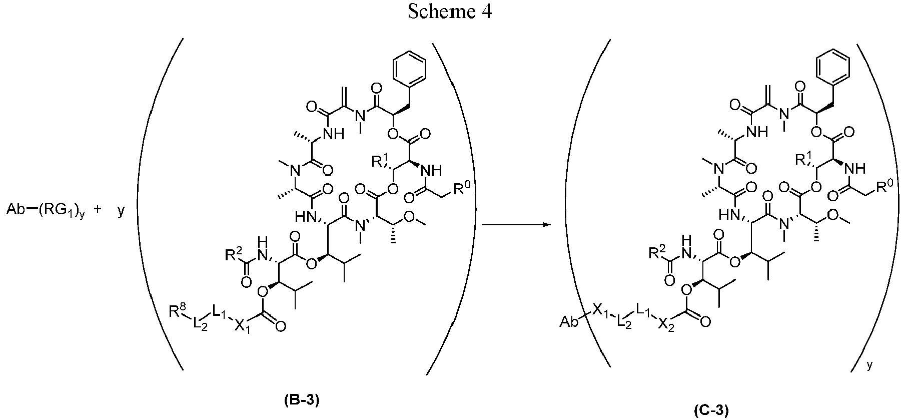 where: RG₁ is a reactive group on the antibody or antigen binding fragment thereof, Ab, by way of example only a thiol, amine or ketone, which reacts with a compatible reactive group, R⁸, attached to the linker-drug moiety thereby covalently linking antibody or antigen binding fragment thereof, Ab, to one or more linker-drug moieties. Non-limiting examples of such reactions of RG₁ and R⁸ groups are a maleimide (R⁸) reacting with a thiol (RG₁) to give a succinimide ring, or a hydroxylamine (R⁸) reacting with a ketone (RG₁) to give an oxime.

Here R⁰ is methyl, R¹ is isopropyl, R² is ethyl, X₁ is a bivalent coupling group formed when RG₁ and R⁸ react (*e*.*g*., a succinimide ring or an oxime), X₂ is a self-immolative spacer, L₁ is a bivalent peptide linker, L₂ is a bond or a linker, and y is 1, 2, 3 or 4.

### Process for Conjugation to Engineered Cysteine Antibody Residues

Conjugates of the disclosure can be prepared using cysteine residues engineered into an antibody by, for example, site-directed mutagenesis. Such site-specific conjugates are homogenous and have improved properties (Junutula JR, Raab H, Clark S, Bhakta S, Leipold DD, Weir S, Chen Y, Simpson M, Tsai SP, Dennis MS, Lu Y, Meng YG, Ng C, Yang J, Lee CC, Duenas E, Gorrell J, Katta V, Kim A, McDorman K, Flagella K, Venook R, Ross S, Spencer SD, Lee Wong W, Lowman HB, Vandlen R, Sliwkowski MX, Scheller RH, Polakis P, Mallet W. (2008) Nature Biotechnology 26:925-932.)

Because engineered cysteines in antibodies expressed in mammalian cells are modified by adducts (disulfides) such as glutathione (GSH) and/or cysteine during their biosynthesis (Chen *et al.* 2009), the engineered cysteine residues in the product as initially expressed are unreactive to thiol reactive reagents such as maleimido or bromo-or iodo-acetamide groups. To conjugate payload to an engineered cysteine after expression, glutathione or cysteine adducts need to be removed by reducing these disulfide adducts, which generally entails also reducing native disulfides in the expressed protein. Deprotection of adducted engineered cysteines can be accomplished by first exposing antibody to a reducing agent, *e*.*g*., dithiothreitol (DTT), TCEP, or reduced cysteine, followed by a procedure that allows for re-oxidation of all native disulfide bonds of an antibody to restore and/or stabilize the functional antibody structure.

Several methods can be employed to reduce and re-oxidize antibodies with engineered cysteine residues for preparation of antibody drug conjugates. Attempts to follow re-oxidation protocols previously described in the literature using high concentration of CuSO₄ resulted in protein precipitation (Junutula JR, Raab H, Clark S, Bhakta S, Leipold DD, Weir S, Chen Y, Simpson M, Tsai SP, Dennis MS, Lu Y, Meng YG, Ng C, Yang J, Lee CC, Duenas E, Gorrell J, Katta V, Kim A, McDorman K, Flagella K, Venook R, Ross S, Spencer SD, Lee Wong W, Lowman HB, Vandlen R, Sliwkowski MX, Scheller RH, Polakis P, Mallet W. (2008) Nature Biotechnology 26:925). We have successfully prepared and obtained antibody drug conjugates with several different methods for reduction and antibody re-oxidation.

The following is a method to reduce and re-oxidize antibodies with engineered cysteine residues for preparation of antibody drug conjugates: Freshly prepared DTT is added to purified Cys mutant antibodies to a final concentration of 10 mM. After incubation with DTT at room temperature for 1 hour, mixture is dialyzed at 4°C against PBS for three days with daily buffer exchange to remove DTT and re-oxidize native disulfide bonds of the antibody. An alternative method is to remove reducing reagents through a desalting column such as Sephadex G-25, equilibrated with PBS. Once protein is fully reduced, 1 mM oxidized ascorbate (dehydro-ascorbic acid) is optionally added to desalted samples and re-oxidation incubations are carried out for 20-24 hours.

In another exemplary method, deprotection of engineered Cys residues is accomplished by adding fully reduced cysteine at 20 mM concentration to antibodies bound to protein A-Sepharose resin. Reduction of the Cys adducts is achieved by incubation for approximately 30-60 minutes at room temperature, then reductant is rapidly removed by washing resin with 50 beds of PBS. Re-oxidation of the reduced antibody is achieved by incubating washed slurry at room temperature with or without addition of 50-2000 nM CuCl₂ as an accelerant. With the exception of use of copper sulfate, examples herein use each of the protocols described herein with similar results. Reoxidation restores intra-chain disulfides, while dialysis, desalting or protein A chromatography removes reducing agent as well as cysteines and glutathiones initially connected to engineered cysteine(s) of the antibody. HPLC reverse phase chromatography is typically used to monitor the reoxidation process: Antibodies are loaded onto a PLRP-S column (4000 Å, 50 mm x 2.1 mm, Agilent) heated to 80° C and eluted using a linear gradient of 30-45% CH3CN in water containing 0.1% TFA at 1.5 mL/min. and peak detection at 215, 254, and 280 nm.

After re-oxidation, the antibody is conjugated to a linker-drug compound of, by way of example, compounds of Formula (B), Formula (B-1), Formula (B-2) or Formula (B-3) (see schemes 1-4). By way of example, a compound of Formula (B), Formula (B-1), Formula (B-2) or Formula (B-3) is added to re-oxidized Cys mutant antibody at 5-10 molar equivalents relative to antibody in PBS buffer (pH 7.2). Incubations are carried out for 1-2 hours. The conjugation process is monitored by reverse-phase HPLC, which is able to separate conjugated antibodies from non-conjugated ones. Conjugation reaction mixtures are analyzed on a PRLP-S column (4000 Å, 50 mm x 2.1 mm, Agilent) heated to 80°C and elution of the column are carried out by a linear gradient of 30-60% acetonitrile in water containing 0.1% TFA at a flow rate of 1.5 ml/min. Elution of proteins from the column is monitored at 280 nm, 254 nm and 215 nm.

Alternatively, for antibodies bound to a Protein A resin, once the antibody is re-oxidized, the resin is washed with 10 column volumes PBS and the resin is then resuspended in equal volume PBS and an 8x excess of a compound of Formula (B), Formula (B-1), Formula (B-2) or Formula (B-3) (in DMSO) is added and incubated at room temperature for 2 hours. The resin is then washed with 50 column volumes of PBS and the resulting antibody drug conjugate is eluted from the Protein A resin, neutralized with 1/10 volume 1 M Tris pH 9.0 and buffer exchanged into appropriate buffer to perform preparative size exclusion chromatography (if needed).

Immunoconjugates are also characterized in terms of average loading of a drug moiety to antibody binding moiety, generally referred to as drug-to-antibody ratio (DAR). The DAR value is extrapolated, for example, from LC-MS data for reduced and deglycosylated samples. LC/MS allows quantitation of the average number of molecules of payload (drug moiety) attached to an antibody in an ADC. HPLC separates an antibody into light and heavy chains, and also separates heavy chain (HC) and light chain (LC) according to the number of Linker-Payload groups per chain. Mass spectral data enables identification of the component species in the mixture, *e*.*g*., LC, LC+1, LC+2, HC, HC+1, HC+2, etc. From average loading of LC and HC chains, the average DAR can be calculated for an ADC. The DAR for a given immunoconjugate sample represents the average number of drug (payload) molecules attached to a tetrameric antibody containing two light chains and two heavy chains.

Throughout the text of this application, should there be a discrepancy between the text of the specification and the sequence listing, the text of the specification shall prevail.

**Table 2. Examples of Anti-PMEL17 Antibodies**

| **G1 E152C_S375C_wt LC** | | |
|---|---|---|
| SEQ ID NO: 1 | HCDR1 (Combined) | GGTFSDYAIT |
| SEQ ID NO: 2 | HCDR2 (Combined) | GIIPIFGTANYAQKFQG |
| SEQ ID NO: 3 | HCDR3 (Combined) | EGGLLTDISYSRYWFAY |
| SEQ ID NO: 4 | HCDR1 (Kabat) | DYAIT |
| SEQ ID NO: 2 | HCDR2 (Kabat) | GIIPIFGTANYAQKFQG |
| SEQ ID NO: 3 | HCDR3 (Kabat) | EGGLLTDISYSRYWFAY |
| SEQ ID NO: 5 | HCDR1 (Chothia) | GGTFSDY |
| SEQ ID NO: 6 | HCDR2 (Chothia) | IPIFGT |
| SEQ ID NO: 3 | HCDR3 (Chothia) | EGGLLTDISYSRYWFAY |
| SEQ ID NO: 7 | HCDR1 (IMGT) | GGTFSDYA |
| SEQ ID NO: 8 | HCDR2 (IMGT) | IIPIFGTA |
| SEQ ID NO: 9 | HCDR3 (IMGT) | AREGGLLTDISYSRYWFAY |
| SEQ ID NO: 10 | VH | QVQLVQSGAEVKKPGSSVKVSCKASGGTFSDYAITWVR QAPGQGLEWMGGIIPIFGTANYAQKFQGRVTITADEST STAYMELSSLRSEDTAVYYCAREGGLLTDISYSRYWFA YWGQGTLVTVSS |
| SEQ ID NO: 11 | DNA VH | CAGGTGCAATTGGTGCAGAGCGGTGCCGAAGTGAAAAA ACCGGGCAGCAGCGTGAAAGTTAGCTGCAAAGCATCCG GAGGGACGTTTTCTGACTACGCTATCACTTGGGTGCGC CAGGCCCCGGGCCAGGGCCTCGAGTGGATGGGCGGTAT CATCCCGATCTTCGGCACTGCGAACTACGCCCAGAAAT TTCAGGGCCGGGTGACCATTACCGCCGATGAAAGCACC AGCACCGCCTATATGGAACTGAGCAGCCTGCGCAGCGA AGATACGGCCGTGTATTATTGCGCGCGTGAAGGTGGTC TGCTGACTGACATCTCTTACTCTCGTTACTGGTTCGCT TACTGGGGCCAAGGCACCCTGGTGACTGTTAGCTCA |
| SEQ ID NO: 12 | Heavy Chain | QVQLVQSGAEVKKPGSSVKVSCKASGGTFSDYAITWVR QAPGQGLEWMGGIIPIFGTANYAQKFQGRVTITADEST STAYMELSSLRSEDTAVYYCAREGGLLTDISYSRYWFA YWGQGTLVTVSSASTKGPSVFPLAPSSKSTSGGTAALG CLVKDYFPCPVTVSWNSGALTSGVHTFPAVLQSSGLYS LSSVVTVPSSSLGTQTYICNVNHKPSNTKVDKRVEPKS CDKTHTCPPCPAPELLGGPSVFLFPPKPKDTLMISRTP EVTCVVVDVSHEDPEVKFNWYVDGVEVHNAKTKPREEQ YNSTYRVVSVLTVLHQDWLNGKEYKCKVSNKALPAPIE KTISKAKGQPREPQVYTLPPSREEMTKNQVSLTCLVKG FYPCDIAVEWESNGQPENNYKTTPPVLDSDGSFFLYSK LTVDKSRWQQGNVFSCSVMHEALHNHYTQKSLSLSPGK |
| SEQ ID NO: 13 | DNA Heavy Chain | CAGGTGCAATTGGTGCAGAGCGGTGCCGAAGTGAAAAA ACCGGGCAGCAGCGTGAAAGTTAGCTGCAAAGCATCCG GAGGGACGTTTTCTGACTACGCTATCACTTGGGTGCGC CAGGCCCCGGGCCAGGGCCTCGAGTGGATGGGCGGTAT CATCCCGATCTTCGGCACTGCGAACTACGCCCAGAAAT TTCAGGGCCGGGTGACCATTACCGCCGATGAAAGCACC AGCACCGCCTATATGGAACTGAGCAGCCTGCGCAGCGA AGATACGGCCGTGTATTATTGCGCGCGTGAAGGTGGTC TGCTGACTGACATCTCTTACTCTCGTTACTGGTTCGCT TACTGGGGCCAAGGCACCCTGGTGACTGTTAGCTCAGC TAGCACCAAGGGCCCAAGTGTGTTTCCCCTGGCCCCCA GCAGCAAGTCTACTTCCGGCGGAACTGCTGCCCTGGGT TGCCTGGTGAAGGACTACTTCCCCTGTCCCGTGACAGT GTCCTGGAACTCTGGGGCTCTGACTTCCGGCGTGCACA CCTTCCCCGCCGTGCTGCAGAGCAGCGGCCTGTACAGC CTGAGCAGCGTGGTGACAGTGCCCTCCAGCTCTCTGGG AACCCAGACCTATATCTGCAACGTGAACCACAAGCCCA GCAACACCAAGGTGGACAAGAGAGTGGAGCCCAAGAGC TGCGACAAGACCCACACCTGCCCCCCCTGCCCAGCTCC AGAACTGCTGGGAGGGCCTTCCGTGTTCCTGTTCCCCC CCAAGCCCAAGGACACCCTGATGATCAGCAGGACCCCC GAGGTGACCTGCGTGGTGGTGGACGTGTCCCACGAGGA |
| | | CCCAGAGGTGAAGTTCAACTGGTACGTGGACGGCGTGG AGGTGCACAACGCCAAGACCAAGCCCAGAGAGGAGCAG TACAACAGCACCTACAGGGTGGTGTCCGTGCTGACCGT GCTGCACCAGGACTGGCTGAACGGCAAAGAATACAAGT GCAAAGTCTCCAACAAGGCCCTGCCAGCCCCAATCGAA AAGACAATCAGCAAGGCCAAGGGCCAGCCACGGGAGCC CCAGGTGTACACCCTGCCCCCCAGCCGGGAGGAGATGA CCAAGAACCAGGTGTCCCTGACCTGTCTGGTGAAGGGC TTCTACCCCTGTGATATCGCCGTGGAGTGGGAGAGCAA CGGCCAGCCCGAGAACAACTACAAGACCACCCCCCCAG TGCTGGACAGCGACGGCAGCTTCTTCCTGTACAGCAAG CTGACCGTGGACAAGTCCAGGTGGCAGCAGGGCAACGT GTTCAGCTGCAGCGTGATGCACGAGGCCCTGCACAACC ACTACACCCAGAAGTCCCTGAGCCTGAGCCCCGGCAAG |
| SEQ ID NO: 14 | LCDR1 (Combined) | SGDALRDKFVY |
| SEQ ID NO: 15 | LCDR2 (Combined) | DDNNRPS |
| SEQ ID NO: 16 | LCDR3 (Combined) | QSWDHSYSLW |
| SEQ ID NO: 14 | LCDR1 (Kabat) | SGDALRDKFVY |
| SEQ ID NO: 15 | LCDR2 (Kabat) | DDNNRPS |
| SEQ ID NO: 16 | LCDR3 (Kabat) | QSWDHSYSLW |
| SEQ ID NO: 17 | LCDR1 (Chothia) | DALRDKF |
| SEQ ID NO: 18 | LCDR2 (Chothia) | DDN |
| SEQ ID NO: 19 | LCDR3 (Chothia) | WDHSYSLV |
| SEQ ID NO: 20 | LCDR1 (IMGT) | ALRDKF |
| SEQ ID NO: 18 | LCDR2 (IMGT) | DDN |
| SEQ ID NO: 16 | LCDR3 (IMGT) | QSWDHSYSLW |
| SEQ ID NO: 21 | VL | DIELTQPPSVSVSPGQTASITCSGDALRDKFVYWYQQK PGQAPVLVIYDDNNRPSGIPERFSGSNSGNTATLTISG TQAEDEADYYCQSWDHSYSLVVFGGGTKLTVL |
| SEQ ID NO: 22 | DNA VL | GATATCGAACTGACCCAGCCGCCGAGCGTGAGCGTGAG CCCGGGCCAGACCGCGAGCATTACCTGTAGCGGCGATG CTCTGCGTGACAAATTCGTTTACTGGTACCAGCAGAAA CCGGGCCAGGCGCCGGTGCTGGTGATCTACGACGACAA CAACCGTCCGAGCGGCATCCCGGAACGTTTTAGCGGAT CCAACAGCGGCAACACCGCGACCCTGACCATTAGCGGC ACCCAGGCGGAAGACGAAGCGGATTATTACTGCCAGTC TTGGGACCATTCTTACTCTCTGGTTGTGTTTGGCGGCG GCACGAAGTTAACTGTCCTG |
| SEQ ID NO: 23 | Light Chain | DIELTQPPSVSVSPGQTASITCSGDALRDKFVYWYQQK PGQAPVLVIYDDNNRPSGIPERFSGSNSGNTATLTISG TQAEDEADYYCQSWDHSYSLVVFGGGTKLTVLGQPKAA PSVTLFPPSSEELQANKATLVCLISDFYPGAVTVAWKA DSSPVKAGVETTTPSKQSNNKYAASSYLSLTPEQWKSH RSYSCQVTHEGSTVEKTVAPTECS |
| SEQ ID NO: 24 | DNA Light Chain | GATATCGAACTGACCCAGCCGCCGAGCGTGAGCGTGAG CCCGGGCCAGACCGCGAGCATTACCTGTAGCGGCGATG CTCTGCGTGACAAATTCGTTTACTGGTACCAGCAGAAA CCGGGCCAGGCGCCGGTGCTGGTGATCTACGACGACAA CAACCGTCCGAGCGGCATCCCGGAACGTTTTAGCGGAT CCAACAGCGGCAACACCGCGACCCTGACCATTAGCGGC ACCCAGGCGGAAGACGAAGCGGATTATTACTGCCAGTC TTGGGACCATTCTTACTCTCTGGTTGTGTTTGGCGGCG |
| | | GCACGAAGTTAACTGTCCTGGGACAACCTAAGGCCGCT CCCTCCGTGACCCTGTTCCCCCCCAGCTCCGAGGAACT GCAGGCCAACAAGGCCACCCTGGTGTGCCTGATCAGCG ACTTCTACCCTGGCGCCGTGACCGTGGCCTGGAAGGCC GACAGCAGCCCCGTGAAGGCCGGCGTGGAGACAACCAC CCCCAGCAAGCAGAGCAACAACAAGTACGCCGCCAGCA GCTACCTGAGCCTGACCCCCGAGCAGTGGAAGAGCCAC AGAAGCTACAGCTGCCAGGTCACCCACGAGGGCAGCAC CGTGGAGAAAACCGTGGCCCCCACCGAGTGCAGC |

| **G1 E152C_S375C_3J LC** | | |
|---|---|---|
| SEQ ID NO: 1 | HCDR1 (Combined) | GGTFSDYAIT |
| SEQ ID NO: 2 | HCDR2 (Combined) | GIIPIFGTANYAQKFQG |
| SEQ ID NO: 3 | HCDR3 (Combined) | EGGLLTDISYSRYWFAY |
| SEQ ID NO: 4 | HCDR1 (Kabat) | DYAIT |
| SEQ ID NO: 2 | HCDR2 (Kabat) | GIIPIFGTANYAQKFQG |
| SEQ ID NO: 3 | HCDR3 (Kabat) | EGGLLTDISYSRYWFAY |
| SEQ ID NO: 5 | HCDR1 (Chothia) | GGTFSDY |
| SEQ ID NO: 6 | HCDR2 (Chothia) | IPIFGT |
| SEQ ID NO: 3 | HCDR3 (Chothia) | EGGLLTDISYSRYWFAY |
| SEQ ID NO: 7 | HCDR1 (IMGT) | GGTFSDYA |
| SEQ ID NO: 8 | HCDR2 (IMGT) | IIPIFGTA |
| SEQ ID NO: 9 | HCDR3 (IMGT) | AREGGLLTDISYSRYWFAY |
| SEQ ID NO: 10 | VH | QVQLVQSGAEVKKPGSSVKVSCKASGGTFSDYAITWVR QAPGQGLEWMGGIIPIFGTANYAQKFQGRVTITADEST STAYMELSSLRSEDTAVYYCAREGGLLTDISYSRYWFA YWGQGTLVTVSS |
| SEQ ID NO: 11 | DNA VH | CAGGTGCAATTGGTGCAGAGCGGTGCCGAAGTGAAAAA ACCGGGCAGCAGCGTGAAAGTTAGCTGCAAAGCATCCG GAGGGACGTTTTCTGACTACGCTATCACTTGGGTGCGC CAGGCCCCGGGCCAGGGCCTCGAGTGGATGGGCGGTAT CATCCCGATCTTCGGCACTGCGAACTACGCCCAGAAAT TTCAGGGCCGGGTGACCATTACCGCCGATGAAAGCACC AGCACCGCCTATATGGAACTGAGCAGCCTGCGCAGCGA AGATACGGCCGTGTATTATTGCGCGCGTGAAGGTGGTC TGCTGACTGACATCTCTTACTCTCGTTACTGGTTCGCT TACTGGGGCCAAGGCACCCTGGTGACTGTTAGCTCA |
| SEQ ID NO: 12 | Heavy Chain | QVQLVQSGAEVKKPGSSVKVSCKASGGTFSDYAITWVR QAPGQGLEWMGGIIPIFGTANYAQKFQGRVTITADEST STAYMELSSLRSEDTAVYYCAREGGLLTDISYSRYWFA YWGQGTLVTVSSASTKGPSVFPLAPSSKSTSGGTAALG CLVKDYFPCPVTVSWNSGALTSGVHTFPAVLQSSGLYS LSSVVTVPSSSLGTQTYICNVNHKPSNTKVDKRVEPKS CDKTHTCPPCPAPELLGGPSVFLFPPKPKDTLMISRTP EVTCVVVDVSHEDPEVKFNWYVDGVEVHNAKTKPREEQ YNSTYRVVSVLTVLHQDWLNGKEYKCKVSNKALPAPIE KTISKAKGQPREPQVYTLPPSREEMTKNQVSLTCLVKG FYPCDIAVEWESNGQPENNYKTTPPVLDSDGSFFLYSK LTVDKSRWQQGNVFSCSVMHEALHNHYTQKSLSLSPGK |
| SEQ ID NO: 13 | DNA Heavy Chain | CAGGTGCAATTGGTGCAGAGCGGTGCCGAAGTGAAAAA ACCGGGCAGCAGCGTGAAAGTTAGCTGCAAAGCATCCG GAGGGACGTTTTCTGACTACGCTATCACTTGGGTGCGC CAGGCCCCGGGCCAGGGCCTCGAGTGGATGGGCGGTAT |
| | | CATCCCGATCTTCGGCACTGCGAACTACGCCCAGAAAT TTCAGGGCCGGGTGACCATTACCGCCGATGAAAGCACC AGCACCGCCTATATGGAACTGAGCAGCCTGCGCAGCGA AGATACGGCCGTGTATTATTGCGCGCGTGAAGGTGGTC TGCTGACTGACATCTCTTACTCTCGTTACTGGTTCGCT TACTGGGGCCAAGGCACCCTGGTGACTGTTAGCTCAGC TAGCACCAAGGGCCCAAGTGTGTTTCCCCTGGCCCCCA GCAGCAAGTCTACTTCCGGCGGAACTGCTGCCCTGGGT TGCCTGGTGAAGGACTACTTCCCCTGTCCCGTGACAGT GTCCTGGAACTCTGGGGCTCTGACTTCCGGCGTGCACA CCTTCCCCGCCGTGCTGCAGAGCAGCGGCCTGTACAGC CTGAGCAGCGTGGTGACAGTGCCCTCCAGCTCTCTGGG AACCCAGACCTATATCTGCAACGTGAACCACAAGCCCA GCAACACCAAGGTGGACAAGAGAGTGGAGCCCAAGAGC TGCGACAAGACCCACACCTGCCCCCCCTGCCCAGCTCC AGAACTGCTGGGAGGGCCTTCCGTGTTCCTGTTCCCCC CCAAGCCCAAGGACACCCTGATGATCAGCAGGACCCCC GAGGTGACCTGCGTGGTGGTGGACGTGTCCCACGAGGA CCCAGAGGTGAAGTTCAACTGGTACGTGGACGGCGTGG AGGTGCACAACGCCAAGACCAAGCCCAGAGAGGAGCAG TACAACAGCACCTACAGGGTGGTGTCCGTGCTGACCGT GCTGCACCAGGACTGGCTGAACGGCAAAGAATACAAGT GCAAAGTCTCCAACAAGGCCCTGCCAGCCCCAATCGAA AAGACAATCAGCAAGGCCAAGGGCCAGCCACGGGAGCC CCAGGTGTACACCCTGCCCCCCAGCCGGGAGGAGATGA CCAAGAACCAGGTGTCCCTGACCTGTCTGGTGAAGGGC TTCTACCCCTGTGATATCGCCGTGGAGTGGGAGAGCAA CGGCCAGCCCGAGAACAACTACAAGACCACCCCCCCAG TGCTGGACAGCGACGGCAGCTTCTTCCTGTACAGCAAG CTGACCGTGGACAAGTCCAGGTGGCAGCAGGGCAACGT GTTCAGCTGCAGCGTGATGCACGAGGCCCTGCACAACC ACTACACCCAGAAGTCCCTGAGCCTGAGCCCCGGCAAG |
| SEQ ID NO: 14 | LCDR1 (Combined) | SGDALRDKFVY |
| SEQ ID NO: 15 | LCDR2 (Combined) | DDNNRPS |
| SEQ ID NO: 16 | LCDR3 (Combined) | QSWDHSYSLW |
| SEQ ID NO: 14 | LCDR1 (Kabat) | SGDALRDKFVY |
| SEQ ID NO: 15 | LCDR2 (Kabat) | DDNNRPS |
| SEQ ID NO: 16 | LCDR3 (Kabat) | QSWDHSYSLW |
| SEQ ID NO: 17 | LCDR1 (Chothia) | DALRDKF |
| SEQ ID NO: 18 | LCDR2 (Chothia) | DDN |
| SEQ ID NO: 19 | LCDR3 (Chothia) | WDHSYSLV |
| SEQ ID NO: 20 | LCDR1 (IMGT) | ALRDKF |
| SEQ ID NO: 18 | LCDR2 (IMGT) | DDN |
| SEQ ID NO: 16 | LCDR3 (IMGT) | QSWDHSYSLVV |
| SEQ ID NO: 25 | VL | SYELTQPLSVSVALGQTARITCSGDALRDKFVYWYQQK PGQAPVLVIYDDNNRPSGIPERFSGSNSGNTATLTISR AQAGDEADYYCQSWDHSYSLVVFGGGTKLTVL |
| SEQ ID NO: 26 | DNA VL | SYELTQPLSVSVALGQTARITCSGDALRDKFVYWYQQK PGQAPVLVIYDDNNRPSGIPERFSGSNSGNTATLTISR AQAGDEADYYCQSWDHSYSLVVFGGGTKLTVL AGCTACGAGCTGACCCAGCCGCTGTCGGTGTCAGTCGC CCTGGGACAAACTGCCAGGATCACTTGTTCCGGGGACG CATTGCGGGACAAGTTCGTGTACTGGTACCAGCAGAAG CCGGGTCAAGCCCCAGTGCTCGTGATCTACGACGACAA CAACCGGCCTTCCGGTATCCCCGAACGCTTCTCCGGAT |
| | | CCAATAGCGGAAACACCGCCACCCTGACCATTTCGAGA GCTCAGGCCGGGGATGAAGCGGACTACTACTGCCAGTC ATGGGATCACTCGTACTCCCTCGTCGTGTTTGGAGGCG GCACGAAGCTTACTGTGCTG |
| SEQ ID NO: 27 | Light Chain | SYELTQPLSVSVALGQTARITCSGDALRDKFVYWYQQK PGQAPVLVIYDDNNRPSGIPERFSGSNSGNTATLTISR AQAGDEADYYCQSWDHSYSLVVFGGGTKLTVLGQPKAA PSVTLFPPSSEELQANKATLVCLISDFYPGAVTVAWKA DSSPVKAGVETTTPSKQSNNKYAASSYLSLTPEQWKSH RSYSCQVTHEGSTVEKTVAPTECS |
| SEQ ID NO: 28 | DNA Light Chain | AGCTACGAGCTGACCCAGCCGCTGTCGGTGTCAGTCGC CCTGGGACAAACTGCCAGGATCACTTGTTCCGGGGACG CATTGCGGGACAAGTTCGTGTACTGGTACCAGCAGAAG CCGGGTCAAGCCCCAGTGCTCGTGATCTACGACGACAA CAACCGGCCTTCCGGTATCCCCGAACGCTTCTCCGGAT CCAATAGCGGAAACACCGCCACCCTGACCATTTCGAGA GCTCAGGCCGGGGATGAAGCGGACTACTACTGCCAGTC ATGGGATCACTCGTACTCCCTCGTCGTGTTTGGAGGCG GCACGAAGCTTACTGTGCTGGGCCAGCCTAAGGCCGCT CCCTCCGTGACCCTGTTCCCCCCCAGCTCCGAGGAACT GCAGGCCAACAAGGCCACCCTGGTGTGCCTGATCAGCG ACTTCTACCCTGGCGCCGTGACCGTGGCCTGGAAGGCC GACAGCAGCCCCGTGAAGGCCGGCGTGGAGACAACCAC CCCCAGCAAGCAGAGCAACAACAAGTACGCCGCCAGCA GCTACCTGAGCCTGACCCCCGAGCAGTGGAAGAGCCAC AGAAGCTACAGCTGCCAGGTCACCCACGAGGGCAGCAC CGTGGAGAAAACCGTGGCCCCCACCGAGTGCAGC |

| **G1 E152C_S375C_3R LC** | | |
|---|---|---|
| SEQ ID NO: 1 | HCDR1 (Combined) | GGTFSDYAIT |
| SEQ ID NO: 2 | HCDR2 (Combined) | GIIPIFGTANYAQKFQG |
| SEQ ID NO: 3 | HCDR3 (Combined) | EGGLLTDISYSRYWFAY |
| SEQ ID NO: 4 | HCDR1 (Kabat) | DYAIT |
| SEQ ID NO: 2 | HCDR2 (Kabat) | GIIPIFGTANYAQKFQG |
| SEQ ID NO: 3 | HCDR3 (Kabat) | EGGLLTDISYSRYWFAY |
| SEQ ID NO: 5 | HCDR1 (Chothia) | GGTFSDY |
| SEQ ID NO: 6 | HCDR2 (Chothia) | IPIFGT |
| SEQ ID NO: 3 | HCDR3 (Chothia) | EGGLLTDISYSRYWFAY |
| SEQ ID NO: 7 | HCDR1 (IMGT) | GGTFSDYA |
| SEQ ID NO: 8 | HCDR2 (IMGT) | IIPIFGTA |
| SEQ ID NO: 9 | HCDR3 (IMGT) | AREGGLLTDISYSRYWFAY |
| SEQ ID NO: 10 | VH | QVQLVQSGAEVKKPGSSVKVSCKASGGTFSDYAITWVR QAPGQGLEWMGGIIPIFGTANYAQKFQGRVTITADEST STAYMELSSLRSEDTAVYYCAREGGLLTDISYSRYWFA YWGQGTLVTVSS |
| SEQ ID NO: 11 | DNA VH | CAGGTGCAATTGGTGCAGAGCGGTGCCGAAGTGAAAAA ACCGGGCAGCAGCGTGAAAGTTAGCTGCAAAGCATCCG GAGGGACGTTTTCTGACTACGCTATCACTTGGGTGCGC CAGGCCCCGGGCCAGGGCCTCGAGTGGATGGGCGGTAT CATCCCGATCTTCGGCACTGCGAACTACGCCCAGAAAT TTCAGGGCCGGGTGACCATTACCGCCGATGAAAGCACC AGCACCGCCTATATGGAACTGAGCAGCCTGCGCAGCGA AGATACGGCCGTGTATTATTGCGCGCGTGAAGGTGGTC |
| | | TGCTGACTGACATCTCTTACTCTCGTTACTGGTTCGCT TACTGGGGCCAAGGCACCCTGGTGACTGTTAGCTCA |
| SEQ ID NO: 12 | Heavy Chain | QVQLVQSGAEVKKPGSSVKVSCKASGGTFSDYAITWVR QAPGQGLEWMGGIIPIFGTANYAQKFQGRVTITADEST STAYMELSSLRSEDTAVYYCAREGGLLTDISYSRYWFA YWGQGTLVTVSSASTKGPSVFPLAPSSKSTSGGTAALG CLVKDYFPCPVTVSWNSGALTSGVHTFPAVLQSSGLYS LSSVVTVPSSSLGTQTYICNVNHKPSNTKVDKRVEPKS CDKTHTCPPCPAPELLGGPSVFLFPPKPKDTLMISRTP EVTCVVVDVSHEDPEVKFNWYVDGVEVHNAKTKPREEQ YNSTYRVVSVLTVLHQDWLNGKEYKCKVSNKALPAPIE KTISKAKGQPREPQVYTLPPSREEMTKNQVSLTCLVKG FYPCDIAVEWESNGQPENNYKTTPPVLDSDGSFFLYSK LTVDKSRWQQGNVFSCSVMHEALHNHYTQKSLSLSPGK |
| SEQ ID NO: 13 | DNA Heavy Chain | CAGGTGCAATTGGTGCAGAGCGGTGCCGAAGTGAAAAA ACCGGGCAGCAGCGTGAAAGTTAGCTGCAAAGCATCCG GAGGGACGTTTTCTGACTACGCTATCACTTGGGTGCGC CAGGCCCCGGGCCAGGGCCTCGAGTGGATGGGCGGTAT CATCCCGATCTTCGGCACTGCGAACTACGCCCAGAAAT TTCAGGGCCGGGTGACCATTACCGCCGATGAAAGCACC AGCACCGCCTATATGGAACTGAGCAGCCTGCGCAGCGA AGATACGGCCGTGTATTATTGCGCGCGTGAAGGTGGTC TGCTGACTGACATCTCTTACTCTCGTTACTGGTTCGCT TACTGGGGCCAAGGCACCCTGGTGACTGTTAGCTCAGC TAGCACCAAGGGCCCAAGTGTGTTTCCCCTGGCCCCCA GCAGCAAGTCTACTTCCGGCGGAACTGCTGCCCTGGGT TGCCTGGTGAAGGACTACTTCCCCTGTCCCGTGACAGT GTCCTGGAACTCTGGGGCTCTGACTTCCGGCGTGCACA CCTTCCCCGCCGTGCTGCAGAGCAGCGGCCTGTACAGC CTGAGCAGCGTGGTGACAGTGCCCTCCAGCTCTCTGGG AACCCAGACCTATATCTGCAACGTGAACCACAAGCCCA GCAACACCAAGGTGGACAAGAGAGTGGAGCCCAAGAGC TGCGACAAGACCCACACCTGCCCCCCCTGCCCAGCTCC AGAACTGCTGGGAGGGCCTTCCGTGTTCCTGTTCCCCC CCAAGCCCAAGGACACCCTGATGATCAGCAGGACCCCC GAGGTGACCTGCGTGGTGGTGGACGTGTCCCACGAGGA CCCAGAGGTGAAGTTCAACTGGTACGTGGACGGCGTGG AGGTGCACAACGCCAAGACCAAGCCCAGAGAGGAGCAG TACAACAGCACCTACAGGGTGGTGTCCGTGCTGACCGT GCTGCACCAGGACTGGCTGAACGGCAAAGAATACAAGT GCAAAGTCTCCAACAAGGCCCTGCCAGCCCCAATCGAA AAGACAATCAGCAAGGCCAAGGGCCAGCCACGGGAGCC CCAGGTGTACACCCTGCCCCCCAGCCGGGAGGAGATGA CCAAGAACCAGGTGTCCCTGACCTGTCTGGTGAAGGGC TTCTACCCCTGTGATATCGCCGTGGAGTGGGAGAGCAA CGGCCAGCCCGAGAACAACTACAAGACCACCCCCCCAG TGCTGGACAGCGACGGCAGCTTCTTCCTGTACAGCAAG CTGACCGTGGACAAGTCCAGGTGGCAGCAGGGCAACGT GTTCAGCTGCAGCGTGATGCACGAGGCCCTGCACAACC ACTACACCCAGAAGTCCCTGAGCCTGAGCCCCGGCAAG |
| SEQ ID NO: 14 | LCDR1 (Combined) | SGDALRDKFVY |
| SEQ ID NO: 15 | LCDR2 (Combined) | DDNNRPS |
| SEQ ID NO: 16 | LCDR3 (Combined) | QSWDHSYSLW |
| SEQ ID NO: 14 | LCDR1 (Kabat) | SGDALRDKFVY |
| SEQ ID NO: 15 | LCDR2 (Kabat) | DDNNRPS |
| SEQ ID NO: 16 | LCDR3 (Kabat) | QSWDHSYSLW |
| SEQ ID NO: 17 | LCDR1 (Chothia) | DALRDKF |
| SEQ ID NO: 18 | LCDR2 (Chothia) | DDN |
| SEQ ID NO: 19 | LCDR3 (Chothia) | WDHSYSLV |
| SEQ ID NO: 20 | LCDR1 (IMGT) | ALRDKF |
| SEQ ID NO: 18 | LCDR2 (IMGT) | DDN |
| SEQ ID NO: 16 | LCDR3 (IMGT) | QSWDHSYSLW |
| SEQ ID NO: 29 | VL | SYELTQPPSVSVSPGQTASITCSGDALRDKFVYWYQQK PGQSPVLVIYDDNNRPSGIPERFSGSNSGNTATLTISG TQAMDEADYYCQSWDHSYSLVVFGGGTKLTVL |
| SEQ ID NO: 30 | DNA VL | TCGTACGAGCTCACTCAACCGCCTTCTGTGTCCGTGTC ACCCGGGCAGACTGCCTCCATTACCTGTTCGGGAGATG CCCTGCGCGACAAGTTTGTGTACTGGTACCAGCAGAAG CCCGGACAGTCGCCAGTGCTCGTCATCTATGACGACAA CAACAGACCTTCCGGTATCCCGGAACGGTTCAGCGGAA GCAATTCCGGCAACACCGCTACCCTGACCATTAGCGGC ACTCAGGCCATGGACGAAGCGGATTACTACTGCCAATC CTGGGACCACTCATACTCCCTTGTGGTGTTCGGTGGCG GAACGAAGCTGACCGTCCTG |
| SEQ ID NO: 31 | Light Chain | SYELTQPPSVSVSPGQTASITCSGDALRDKFVYWYQQK PGQSPVLVIYDDNNRPSGIPERFSGSNSGNTATLTISG TQAMDEADYYCQSWDHSYSLVVFGGGTKLTVLGQPKAA PSVTLFPPSSEELQANKATLVCLISDFYPGAVTVAWKA DSSPVKAGVETTTPSKQSNNKYAASSYLSLTPEQWKSH RSYSCQVTHEGSTVEKTVAPTECS |
| SEQ ID NO: 32 | DNA Light Chain | TCGTACGAGCTCACTCAACCGCCTTCTGTGTCCGTGTC ACCCGGGCAGACTGCCTCCATTACCTGTTCGGGAGATG CCCTGCGCGACAAGTTTGTGTACTGGTACCAGCAGAAG CCCGGACAGTCGCCAGTGCTCGTCATCTATGACGACAA CAACAGACCTTCCGGTATCCCGGAACGGTTCAGCGGAA GCAATTCCGGCAACACCGCTACCCTGACCATTAGCGGC ACTCAGGCCATGGACGAAGCGGATTACTACTGCCAATC CTGGGACCACTCATACTCCCTTGTGGTGTTCGGTGGCG GAACGAAGCTGACCGTCCTGGGCCAGCCTAAGGCCGCT CCCTCCGTGACCCTGTTCCCCCCCAGCTCCGAGGAACT GCAGGCCAACAAGGCCACCCTGGTGTGCCTGATCAGCG ACTTCTACCCTGGCGCCGTGACCGTGGCCTGGAAGGCC GACAGCAGCCCCGTGAAGGCCGGCGTGGAGACAACCAC CCCCAGCAAGCAGAGCAACAACAAGTACGCCGCCAGCA GCTACCTGAGCCTGACCCCCGAGCAGTGGAAGAGCCAC AGAAGCTACAGCTGCCAGGTCACCCACGAGGGCAGCAC CGTGGAGAAAACCGTGGCCCCCACCGAGTGCAGC |

| **G4 E152_S375C** | | |
|---|---|---|
| SEQ ID NO: 33 | HCDR1 (Combined) | GGTFSTYAIS |
| SEQ ID NO: 34 | HCDR2 (Combined) | RIIPILGIANYAQKFQG |
| SEQ ID NO: 35 | HCDR3 (Combined) | EVRMIFDY |
| SEQ ID NO: 36 | HCDR1 (Kabat) | TYAIS |
| SEQ ID NO: 34 | HCDR2 (Kabat) | RIIPILGIANYAQKFQG |
| SEQ ID NO: 35 | HCDR3 (Kabat) | EVRMIFDY |
| SEQ ID NO: 37 | HCDR1 (Chothia) | GGTFSTY |
| SEQ ID NO: 38 | HCDR2 (Chothia) | IPILGI |
| SEQ ID NO: 35 | HCDR3(Chothia) | EVRMIFDY |
| SEQ ID NO: 39 | HCDR1 (IMGT) | GGTFSTYA |
| SEQ ID NO: 40 | HCDR2 (IMGT) | IIPILGIA |
| SEQ ID NO: 41 | HCDR3 (IMGT) | AREVRMIFDY |
| SEQ ID NO: 42 | VH | QVQLVQSGAEVKKPGSSVKVSCKASGGTFSTYAISWVR QAPGQGLEWMGRIIPILGIANYAQKFQGRVTITADEST STAYMELSSLRSEDTAVYYCAREVRMIFDYWGQGTLVT VSS |
| SEQ ID NO: 43 | DNA VH | CAGGTGCAATTGGTGCAGAGCGGTGCCGAAGTGAAAAA ACCGGGCAGCAGCGTGAAAGTTAGCTGCAAAGCATCCG GAGGGACGTTTTCTACTTACGCTATCTCTTGGGTGCGC CAGGCCCCGGGCCAGGGCCTCGAGTGGATGGGCCGTAT CATCCCGATCCTGGGCATCGCGAACTACGCCCAGAAAT TTCAGGGCCGGGTGACCATTACCGCCGATGAAAGCACC AGCACCGCCTATATGGAACTGAGCAGCCTGCGCAGCGA AGATACGGCCGTGTATTATTGCGCGCGTGAAGTTCGTA TGATCTTCGATTACTGGGGCCAAGGCACCCTGGTGACT GTTAGCTCA |
| SEQ ID NO: 44 | Heavy Chain | QVQLVQSGAEVKKPGSSVKVSCKASGGTFSTYAISWVR QAPGQGLEWMGRIIPILGIANYAQKFQGRVTITADEST STAYMELSSLRSEDTAVYYCAREVRMIFDYWGQGTLVT VSSASTKGPSVFPLAPSSKSTSGGTAALGCLVKDYFPC PVTVSWNSGALTSGVHTFPAVLQSSGLYSLSSVVTVPS SSLGTQTYICNVNHKPSNTKVDKRVEPKSCDKTHTCPP CPAPELLGGPSVFLFPPKPKDTLMISRTPEVTCVVVDV SHEDPEVKFNWYVDGVEVHNAKTKPREEQYNSTYRVVS VLTVLHQDWLNGKEYKCKVSNKALPAPIEKTISKAKGQ PREPQVYTLPPSREEMTKNQVSLTCLVKGFYPCDIAVE WESNGQPENNYKTTPPVLDSDGSFFLYSKLTVDKSRWQ QGNVFSCSVMHEALHNHYTQKSLSLSPGK |
| SEQ ID NO: 45 | DNA Heavy Chain | CAGGTGCAATTGGTGCAGAGCGGTGCCGAAGTGAAAAA ACCGGGCAGCAGCGTGAAAGTTAGCTGCAAAGCATCCG GAGGGACGTTTTCTACTTACGCTATCTCTTGGGTGCGC CAGGCCCCGGGCCAGGGCCTCGAGTGGATGGGCCGTAT CATCCCGATCCTGGGCATCGCGAACTACGCCCAGAAAT TTCAGGGCCGGGTGACCATTACCGCCGATGAAAGCACC AGCACCGCCTATATGGAACTGAGCAGCCTGCGCAGCGA AGATACGGCCGTGTATTATTGCGCGCGTGAAGTTCGTA TGATCTTCGATTACTGGGGCCAAGGCACCCTGGTGACT GTTAGCTCAGCCTCTACGAAAGGCCCAAGCGTATTTCC CCTGGCTCCTTCTAGTAAATCAACCTCAGGTGGTACAG CAGCCCTTGGCTGCCTGGTCAAAGACTATTTCCCCTGT CCGGTGACCGTCTCATGGAACTCAGGTGCTTTGACATC TGGTGTGCATACATTCCCAGCTGTGCTGCAAAGTAGTG GACTGTACAGCCTTTCCAGCGTGGTCACGGTGCCAAGT AGCTCCTTGGGTACTCAGACTTATATCTGCAATGTGAA CCACAAGCCCTCTAACACGAAGGTGGACAAGCGCGTGG AGCCCAAATCTTGCGATAAGACGCATACTTGTCCCCCA TGCCCTGCTCCTGAGCTGTTGGGAGGCCCGTCAGTGTT CTTGTTCCCTCCGAAGCCTAAGGACACTTTGATGATAA GTAGGACACCAGAGGTGACTTGCGTGGTGGTTGATGTG TCCCATGAAGATCCCGAGGTCAAATTTAATTGGTACGT AGATGGTGTCGAAGTTCACAATGCTAAGACTAAGCCAA |
| | | GGGAAGAGCAGTACAACAGTACATATAGGGTAGTCTCC GTGCTGACAGTCCTCCACCAGGACTGGTTGAACGGCAA GGAATACAAATGTAAGGTGTCAAACAAAGCTCTGCCTG CTCCCATTGAGAAAACAATCTCTAAAGCCAAAGGCCAG CCGAGAGAGCCCCAAGTCTACACTTTGCCCCCGAGCAG GGAGGAAATGACCAAGAATCAGGTGAGTCTGACGTGCC TCGTCAAAGGATTTTATCCATGCGATATTGCAGTTGAA TGGGAGAGCAATGGCCAGCCAGAGAACAACTATAAAAC CACACCACCCGTGCTCGACTCTGATGGCAGCTTCTTCC TCTATAGCAAGCTGACAGTCGATAAATCTCGCTGGCAG CAAGGCAATGTGTTCTCCTGCTCCGTCATGCACGAGGC TTTGCATAACCATTATACTCAAAAATCTCTGTCCCTGT CACCTGGTAAA |
| SEQ ID NO: 46 | LCDR1 (Combined) | RASQSISSYLA |
| SEQ ID NO: 47 | LCDR2 (Combined) | AASSLQS |
| SEQ ID NO: 48 | LCDR3 (Combined) | QQSYDYYT |
| SEQ ID NO: 46 | LCDR1 (Kabat) | RASQSISSYLA |
| SEQ ID NO: 47 | LCDR2 (Kabat) | AASSLQS |
| SEQ ID NO: 48 | LCDR3 (Kabat) | QQSYDYYT |
| SEQ ID NO: 49 | LCDR1 (Chothia) | SQSISSY |
| SEQ ID NO: 50 | LCDR2 (Chothia) | AAS |
| SEQ ID NO: 51 | LCDR3 (Chothia) | SYDYY |
| SEQ ID NO: 52 | LCDR1 (IMGT) | QSISSY |
| SEQ ID NO: 50 | LCDR2 (IMGT) | AAS |
| SEQ ID NO: 48 | LCDR3 (IMGT) | QQSYDYYT |
| SEQ ID NO: 53 | VL | DIQMTQSPSSLSASVGDRVTITCRASQSISSYLAWYQQ KPGKAPKLLIYAASSLQSGVPSRFSGSGSGTDFTLTIS SLOPEDFATYYCOOSYDYYTFGOGTKVEIK |
| SEQ ID NO: 54 | DNA VL | GATATCCAGATGACCCAGAGCCCGAGCAGCCTGAGCGC CAGCGTGGGCGATCGCGTGACCATTACCTGCAGAGCCA GCCAGTCTATTTCTTCTTACCTGGCTTGGTACCAGCAG AAACCGGGCAAAGCGCCGAAACTATTAATCTACGCTGC TTCTTCTCTGCAAAGCGGCGTGCCGAGCCGCTTTAGCG GCAGCGGATCCGGCACCGATTTCACCCTGACCATTAGC TCTCTGCAACCGGAAGACTTTGCGACCTATTATTGCCA GCAGTCTTACGACTACTACACCTTTGGCCAGGGCACGA AAGTTGAAATTAAA |
| SEQ ID NO: 55 | Light Chain | DIQMTQSPSSLSASVGDRVTITCRASQSISSYLAWYQQ KPGKAPKLLIYAASSLQSGVPSRFSGSGSGTDFTLTIS SLQPEDFATYYCQQSYDYYTFGQGTKVEIKRTVAAPSV FIFPPSDEQLKSGTASVVCLLNNFYPREAKVQWKVDNA LQSGNSQESVTEQDSKDSTYSLSSTLTLSKADYEKHKV YACEVTHQGLSSPVTKSFNRGEC |
| SEQ ID NO: 56 | DNA Light Chain | GATATCCAGATGACCCAGAGCCCGAGCAGCCTGAGCGC CAGCGTGGGCGATCGCGTGACCATTACCTGCAGAGCCA GCCAGTCTATTTCTTCTTACCTGGCTTGGTACCAGCAG AAACCGGGCAAAGCGCCGAAACTATTAATCTACGCTGC TTCTTCTCTGCAAAGCGGCGTGCCGAGCCGCTTTAGCG GCAGCGGATCCGGCACCGATTTCACCCTGACCATTAGC TCTCTGCAACCGGAAGACTTTGCGACCTATTATTGCCA GCAGTCTTACGACTACTACACCTTTGGCCAGGGCACGA AAGTTGAAATTAAACGTACGGTGGCAGCTCCGTCTGTT |
| | | TTCATCTTTCCACCTAGCGACGAGCAACTCAAAAGTGG TACAGCATCCGTGGTTTGTCTGCTGAACAATTTTTACC CCAGGGAGGCTAAGGTCCAGTGGAAAGTCGATAACGCT CTTCAGTCTGGCAACAGTCAGGAGAGCGTCACAGAGCA GGACTCTAAGGATAGCACTTATAGTCTGTCCTCCACGC TGACACTGTCTAAAGCGGATTATGAGAAGCACAAGGTT TACGCCTGTGAGGTAACGCACCAAGGACTCTCCTCCCC AGTTACCAAATCTTTCAACAGAGGAGAATGT |

| **M1 E152C_S375C** | | |
|---|---|---|
| SEQ ID NO: 57 | HCDR1 (Combined) | GGTFSDYAIS |
| SEQ ID NO: 58 | HCDR2 (Combined) | GIIPIFGDANYAQKFQG |
| SEQ ID NO: 59 | HCDR3 (Combined) | EGSSYFYMAY |
| SEQ ID NO: 60 | HCDR1 (Kabat) | DYAIS |
| SEQ ID NO: 58 | HCDR2 (Kabat) | GIIPIFGDANYAQKFQG |
| SEQ ID NO: 59 | HCDR3 (Kabat) | EGSSYFYMAY |
| SEQ ID NO: 5 | HCDR1 (Chothia) | GGTFSDY |
| SEQ ID NO: 61 | HCDR2 (Chothia) | IPIFGD |
| SEQ ID NO: 59 | HCDR3 (Chothia) | EGSSYFYMAY |
| SEQ ID NO: 7 | HCDR1 (IMGT) | GGTFSDYA |
| SEQ ID NO: 62 | HCDR2 (IMGT) | IIPIFGDA |
| SEQ ID NO: 63 | HCDR3 (IMGT) | AREGSSYFYMAY |
| SEQ ID NO: 64 | VH | QVQLVQSGAEVKKPGSSVKVSCKASGGTFSDYAISWVR QAPGQGLEWMGGIIPIFGDANYAQKFQGRVTITADEST STAYMELSSLRSEDTAVYYCAREGSSYFYMAYWGQGTL VTVSS |
| SEQ ID NO: 65 | DNA VH | CAGGTTCAGCTGGTGCAGTCTGGCGCCGAAGTGAAGAA ACCTGGCAGCAGCGTGAAGGTGTCCTGCAAAGCAAGCG GCGGCACCTTCAGCGATTACGCCATCTCTTGGGTCCGA CAGGCCCCTGGACAAGGCTTGGAATGGATGGGCGGCAT CATCCCCATCTTCGGCGACGCCAATTACGCCCAGAAAT TCCAGGGCAGAGTGACCATCACCGCCGACGAGTCTACA AGCACCGCCTACATGGAACTGAGCAGCCTGAGAAGCGA GGACACCGCCGTGTACTACTGTGCCAGAGAGGGCAGCA GCTACTTCTACATGGCCTATTGGGGCCAGGGCACCCTG GTCACAGTTAGCTCT |
| SEQ ID NO: 66 | Heavy Chain | QVQLVQSGAEVKKPGSSVKVSCKASGGTFSDYAISWVR QAPGQGLEWMGGIIPIFGDANYAQKFQGRVTITADEST STAYMELSSLRSEDTAVYYCAREGSSYFYMAYWGQGTL VTVSSASTKGPSVFPLAPSSKSTSGGTAALGCLVKDYF PCPVTVSWNSGALTSGVHTFPAVLQSSGLYSLSSVVTV PSSSLGTQTYICNVNHKPSNTKVDKRVEPKSCDKTHTC PPCPAPELLGGPSVFLFPPKPKDTLMISRTPEVTCVVV DVSHEDPEVKFNWYVDGVEVHNAKTKPREEQYNSTYRV VSVLTVLHQDWLNGKEYKCKVSNKALPAPIEKTISKAK GQPREPQVYTLPPSREEMTKNQVSLTCLVKGFYPCDIA VEWESNGQPENNYKTTPPVLDSDGSFFLYSKLTVDKSR WQQGNVFSCSVMHEALHNHYTQKSLSLSPGK |
| SEQ ID NO: 67 | DNA Heavy Chain | CAGGTTCAGCTGGTGCAGTCTGGCGCCGAAGTGAAGAA ACCTGGCAGCAGCGTGAAGGTGTCCTGCAAAGCAAGCG GCGGCACCTTCAGCGATTACGCCATCTCTTGGGTCCGA CAGGCCCCTGGACAAGGCTTGGAATGGATGGGCGGCAT CATCCCCATCTTCGGCGACGCCAATTACGCCCAGAAAT |
| | | TCCAGGGCAGAGTGACCATCACCGCCGACGAGTCTACA AGCACCGCCTACATGGAACTGAGCAGCCTGAGAAGCGA GGACACCGCCGTGTACTACTGTGCCAGAGAGGGCAGCA GCTACTTCTACATGGCCTATTGGGGCCAGGGCACCCTG GTCACAGTTAGCTCTGCTAGCACCAAGGGCCCAAGTGT GTTTCCCCTGGCCCCCAGCAGCAAGTCTACTTCCGGCG GAACTGCTGCCCTGGGTTGCCTGGTGAAGGACTACTTC CCCTGTCCCGTGACAGTGTCCTGGAACTCTGGGGCTCT GACTTCCGGCGTGCACACCTTCCCCGCCGTGCTGCAGA GCAGCGGCCTGTACAGCCTGAGCAGCGTGGTGACAGTG CCCTCCAGCTCTCTGGGAACCCAGACCTATATCTGCAA CGTGAACCACAAGCCCAGCAACACCAAGGTGGACAAGA GAGTGGAGCCCAAGAGCTGCGACAAGACCCACACCTGC CCCCCCTGCCCAGCTCCAGAACTGCTGGGAGGGCCTTC CGTGTTCCTGTTCCCCCCCAAGCCCAAGGACACCCTGA TGATCAGCAGGACCCCCGAGGTGACCTGCGTGGTGGTG GACGTGTCCCACGAGGACCCAGAGGTGAAGTTCAACTG GTACGTGGACGGCGTGGAGGTGCACAACGCCAAGACCA AGCCCAGAGAGGAGCAGTACAACAGCACCTACAGGGTG GTGTCCGTGCTGACCGTGCTGCACCAGGACTGGCTGAA CGGCAAAGAATACAAGTGCAAAGTCTCCAACAAGGCCC TGCCAGCCCCAATCGAAAAGACAATCAGCAAGGCCAAG GGCCAGCCACGGGAGCCCCAGGTGTACACCCTGCCCCC CAGCCGGGAGGAGATGACCAAGAACCAGGTGTCCCTGA CCTGTCTGGTGAAGGGCTTCTACCCCTGTGATATCGCC GTGGAGTGGGAGAGCAACGGCCAGCCCGAGAACAACTA CAAGACCACCCCCCCAGTGCTGGACAGCGACGGCAGCT TCTTCCTGTACAGCAAGCTGACCGTGGACAAGTCCAGG TGGCAGCAGGGCAACGTGTTCAGCTGCAGCGTGATGCA CGAGGCCCTGCACAACCACTACACCCAGAAGTCCCTGA GCCTGAGCCCCGGCAAG |
| SEQ ID NO: 68 | LCDR1 (Combined) | SGDNIGSKLAS |
| SEQ ID NO: 69 | LCDR2 (Combined) | DDSNRPS |
| SEQ ID NO: 70 | LCDR3 (Combined) | AATAGDRWAYV |
| SEQ ID NO: 68 | LCDR1 (Kabat) | SGDNIGSKLAS |
| SEQ ID NO: 69 | LCDR2 (Kabat) | DDSNRPS |
| SEQ ID NO: 70 | LCDR3 (Kabat) | AATAGDRWAYV |
| SEQ ID NO: 71 | LCDR1 (Chothia) | DNIGSKL |
| SEQ ID NO: 72 | LCDR2 (Chothia) | DDS |
| SEQ ID NO: 73 | LCDR3 (Chothia) | TAGDRWAY |
| SEQ ID NO: 74 | LCDR1 (IMGT) | NIGSKL |
| SEQ ID NO: 72 | LCDR2 (IMGT) | DDS |
| SEQ ID NO: 70 | LCDR3 (IMGT) | AATAGDRWAYV |
| SEQ ID NO: 75 | VL | SYELTQPLSVSVALGQTARITCSGDNIGSKLASWYQQK PGQAPVLVIYDDSNRPSGIPERFSGSNSGNTATLTISR AQAGDEADYYCAATAGDRWAYVFGGGTKLTVL |
| SEQ ID NO: 76 | DNA VL | AGCTATGAGCTGACACAGCCTCTGTCCGTGTCTGTGGC TCTGGGACAGACCGCCAGAATCACCTGTAGCGGCGACA ACATCGGCAGCAAGCTGGCCTCTTGGTATCAGCAGAAG CCTGGACAGGCCCCTGTGCTGGTCATCTACGACGACAG CAATAGACCCAGCGGCATCCCCGAGAGATTCAGCGGCA GCAATAGCGGCAATACCGCCACACTGACCATCAGCAGA |
| | | GCACAGGCTGGCGACGAGGCCGATTACTATTGTGCTGC CACAGCCGGCGACAGATGGGCCTATGTTTTTGGCGGCG GAACAAAGCTGACCGTGCTG |
| SEQ ID NO: 77 | Light Chain | SYELTQPLSVSVALGQTARITCSGDNIGSKLASWYQQK PGQAPVLVIYDDSNRPSGIPERFSGSNSGNTATLTISR AQAGDEADYYCAATAGDRWAYVFGGGTKLTVLGQPKAA PSVTLFPPSSEELQANKATLVCLISDFYPGAVTVAWKA DSSPVKAGVETTTPSKQSNNKYAASSYLSLTPEQWKSH RSYSCQVTHEGSTVEKTVAPTECS |
| SEQ ID NO: 78 | DNA Light Chain | AGCTATGAGCTGACACAGCCTCTGTCCGTGTCTGTGGC TCTGGGACAGACCGCCAGAATCACCTGTAGCGGCGACA ACATCGGCAGCAAGCTGGCCTCTTGGTATCAGCAGAAG CCTGGACAGGCCCCTGTGCTGGTCATCTACGACGACAG CAATAGACCCAGCGGCATCCCCGAGAGATTCAGCGGCA GCAATAGCGGCAATACCGCCACACTGACCATCAGCAGA GCACAGGCTGGCGACGAGGCCGATTACTATTGTGCTGC CACAGCCGGCGACAGATGGGCCTATGTTTTTGGCGGCG GAACAAAGCTGACCGTGCTGGGACAGCCTAAGGCCGCT CCCTCCGTGACCCTGTTCCCCCCCAGCTCCGAGGAACT GCAGGCCAACAAGGCCACCCTGGTGTGCCTGATCAGCG ACTTCTACCCTGGCGCCGTGACCGTGGCCTGGAAGGCC GACAGCAGCCCCGTGAAGGCCGGCGTGGAGACAACCAC CCCCAGCAAGCAGAGCAACAACAAGTACGCCGCCAGCA GCTACCTGAGCCTGACCCCCGAGCAGTGGAAGAGCCAC AGAAGCTACAGCTGCCAGGTCACCCACGAGGGCAGCAC CGTGGAGAAAACCGTGGCCCCCACCGAGTGCAGC |

| **M2 E152C_S375C** | | |
|---|---|---|
| SEQ ID NO: 79 | HCDR1 (Combined) | GFTFSSFGMS |
| SEQ ID NO: 80 | HCDR2 (Combined) | AISYSGSDTYYADSVKG |
| SEQ ID NO: 81 | HCDR3 (Combined) | DVGVMDY |
| SEQ ID NO: 82 | HCDR1 (Kabat) | SFGMS |
| SEQ ID NO: 80 | HCDR2 (Kabat) | AISYSGSDTYYADSVKG |
| SEQ ID NO: 81 | HCDR3 (Kabat) | DVGVMDY |
| SEQ ID NO: 83 | HCDR1 (Chothia) | GFTFSSF |
| SEQ ID NO: 84 | HCDR2 (Chothia) | SYSGSD |
| SEQ ID NO: 81 | HCDR3 (Chothia) | DVGVMDY |
| SEQ ID NO: 85 | HCDR1 (IMGT) | GFTFSSFG |
| SEQ ID NO: 86 | HCDR2 (IMGT) | ISYSGSDT |
| SEQ ID NO: 87 | HCDR3 (IMGT) | ARDVGVMDY |
| SEQ ID NO: 88 | VH | QVQLLESGGGLVQPGGSLRLSCAASGFTFSSFGMSWVR QAPGKGLEWVSAISYSGSDTYYADSVKGRFTISRDNSK NTLYLQMNSLRAEDTAVYYCARDVGVMDYWGQGTLVTV SS |
| SEQ ID NO: 89 | DNA VH | CAGGTTCAGCTGCTGGAATCTGGCGGAGGACTGGTTCA ACCTGGCGGCTCTCTGAGACTGTCTTGTGCCGCCAGCG GCTTCACCTTCAGCAGCTTTGGCATGAGCTGGGTCCGA CAGGCCCCTGGCAAAGGACTTGAATGGGTGTCCGCCAT CAGCTACAGCGGCAGCGATACCTACTACGCCGACAGCG TGAAGGGCAGATTCACCATCTCCAGAGACAACAGCAAG AACACCCTGTACCTGCAGATGAACAGCCTGAGAGCCGA GGACACCGCCGTGTACTACTGTGCCAGAGATGTGGGCG |
| | | TGATGGACTATTGGGGCCAGGGCACACTGGTCACCGTT AGCTCT |
| SEQ ID NO: 90 | Heavy Chain | QVQLLESGGGLVQPGGSLRLSCAASGFTFSSFGMSWVR QAPGKGLEWVSAISYSGSDTYYADSVKGRFTISRDNSK NTLYLQMNSLRAEDTAVYYCARDVGVMDYWGQGTLVTV SSASTKGPSVFPLAPSSKSTSGGTAALGCLVKDYFPCP VTVSWNSGALTSGVHTFPAVLQSSGLYSLSSVVTVPSS SLGTQTYICNVNHKPSNTKVDKRVEPKSCDKTHTCPPC PAPELLGGPSVFLFPPKPKDTLMISRTPEVTCVVVDVS HEDPEVKFNWYVDGVEVHNAKTKPREEQYNSTYRVVSV LTVLHQDWLNGKEYKCKVSNKALPAPIEKTISKAKGQP REPQVYTLPPSREEMTKNQVSLTCLVKGFYPCDIAVEW ESNGQPENNYKTTPPVLDSDGSFFLYSKLTVDKSRWQQ GNVFSCSVMHEALHNHYTQKSLSLSPGK |
| SEQ ID NO: 91 | DNA Heavy Chain | CAGGTTCAGCTGCTGGAATCTGGCGGAGGACTGGTTCA ACCTGGCGGCTCTCTGAGACTGTCTTGTGCCGCCAGCG GCTTCACCTTCAGCAGCTTTGGCATGAGCTGGGTCCGA CAGGCCCCTGGCAAAGGACTTGAATGGGTGTCCGCCAT CAGCTACAGCGGCAGCGATACCTACTACGCCGACAGCG TGAAGGGCAGATTCACCATCTCCAGAGACAACAGCAAG AACACCCTGTACCTGCAGATGAACAGCCTGAGAGCCGA GGACACCGCCGTGTACTACTGTGCCAGAGATGTGGGCG TGATGGACTATTGGGGCCAGGGCACACTGGTCACCGTT AGCTCTGCTAGCACCAAGGGCCCAAGTGTGTTTCCCCT GGCCCCCAGCAGCAAGTCTACTTCCGGCGGAACTGCTG CCCTGGGTTGCCTGGTGAAGGACTACTTCCCCTGTCCC GTGACAGTGTCCTGGAACTCTGGGGCTCTGACTTCCGG CGTGCACACCTTCCCCGCCGTGCTGCAGAGCAGCGGCC TGTACAGCCTGAGCAGCGTGGTGACAGTGCCCTCCAGC TCTCTGGGAACCCAGACCTATATCTGCAACGTGAACCA CAAGCCCAGCAACACCAAGGTGGACAAGAGAGTGGAGC CCAAGAGCTGCGACAAGACCCACACCTGCCCCCCCTGC CCAGCTCCAGAACTGCTGGGAGGGCCTTCCGTGTTCCT GTTCCCCCCCAAGCCCAAGGACACCCTGATGATCAGCA GGACCCCCGAGGTGACCTGCGTGGTGGTGGACGTGTCC CACGAGGACCCAGAGGTGAAGTTCAACTGGTACGTGGA CGGCGTGGAGGTGCACAACGCCAAGACCAAGCCCAGAG AGGAGCAGTACAACAGCACCTACAGGGTGGTGTCCGTG CTGACCGTGCTGCACCAGGACTGGCTGAACGGCAAAGA ATACAAGTGCAAAGTCTCCAACAAGGCCCTGCCAGCCC CAATCGAAAAGACAATCAGCAAGGCCAAGGGCCAGCCA CGGGAGCCCCAGGTGTACACCCTGCCCCCCAGCCGGGA GGAGATGACCAAGAACCAGGTGTCCCTGACCTGTCTGG TGAAGGGCTTCTACCCCTGTGATATCGCCGTGGAGTGG GAGAGCAACGGCCAGCCCGAGAACAACTACAAGACCAC CCCCCCAGTGCTGGACAGCGACGGCAGCTTCTTCCTGT ACAGCAAGCTGACCGTGGACAAGTCCAGGTGGCAGCAG GGCAACGTGTTCAGCTGCAGCGTGATGCACGAGGCCCT GCACAACCACTACACCCAGAAGTCCCTGAGCCTGAGCC CCGGCAAG |
| SEQ ID NO: 92 | LCDR1 (Combined) | SGDNLGTYYAH |
| SEQ ID NO: 93 | LCDR2 (Combined) | SQSHRPS |
| SEQ ID NO: 94 | LCDR3 (Combined) | GAWDAPSPELV |
| SEQ ID NO: 92 | LCDR1 (Kabat) | SGDNLGTYYAH |
| SEQ ID NO: 93 | LCDR2 (Kabat) | SQSHRPS |
| SEQ ID NO: 94 | LCDR3 (Kabat) | GAWDAPSPELV |
| SEQ ID NO: 95 | LCDR1 (Chothia) | DNLGTYY |
| SEQ ID NO: 96 | LCDR2 (Chothia) | SQS |
| SEQ ID NO: 97 | LCDR3 (Chothia) | WDAPSPEL |
| SEQ ID NO: 98 | LCDR1 (IMGT) | NLGTYY |
| SEQ ID NO: 96 | LCDR2 (IMGT) | SQS |
| SEQ ID NO: 94 | LCDR3 (IMGT) | GAWDAPSPELV |
| SEQ ID NO: 99 | VL | SYELTQPLSVSVALGQTARITCSGDNLGTYYAHWYQQK PGQAPVLVIYSQSHRPSGIPERFSGSNSGNTATLTISR AQAGDEADYYCGAWDAPSPELVFGGGTKLTVL |
| SEQ ID NO: 100 | DNA VL | AGCTATGAGCTGACACAGCCTCTGTCCGTGTCTGTGGC TCTGGGACAGACCGCCAGAATCACCTGTAGCGGCGATA ACCTGGGCACCTACTACGCCCACTGGTATCAGCAGAAG CCTGGACAGGCTCCCGTGCTGGTCATCTACAGCCAGTC TCACAGACCCAGCGGCATCCCCGAGAGATTCAGCGGCA GCAATAGCGGCAATACCGCCACACTGACCATCAGCAGA GCACAGGCTGGCGACGAGGCCGATTACTATTGTGGCGC TTGGGACGCCCCATCTCCTGAGCTTGTTTTTGGCGGAG GCACCAAGCTGACAGTGCTG |
| SEQ ID NO: 101 | Light Chain | SYELTQPLSVSVALGQTARITCSGDNLGTYYAHWYQQK PGQAPVLVIYSQSHRPSGIPERFSGSNSGNTATLTISR AQAGDEADYYCGAWDAPSPELVFGGGTKLTVLGQPKAA PSVTLFPPSSEELQANKATLVCLISDFYPGAVTVAWKA DSSPVKAGVETTTPSKQSNNKYAASSYLSLTPEQWKSH RSYSCQVTHEGSTVEKTVAPTECS |
| SEQ ID NO: 102 | DNA Light Chain | AGCTATGAGCTGACACAGCCTCTGTCCGTGTCTGTGGC TCTGGGACAGACCGCCAGAATCACCTGTAGCGGCGATA ACCTGGGCACCTACTACGCCCACTGGTATCAGCAGAAG CCTGGACAGGCTCCCGTGCTGGTCATCTACAGCCAGTC TCACAGACCCAGCGGCATCCCCGAGAGATTCAGCGGCA GCAATAGCGGCAATACCGCCACACTGACCATCAGCAGA GCACAGGCTGGCGACGAGGCCGATTACTATTGTGGCGC TTGGGACGCCCCATCTCCTGAGCTTGTTTTTGGCGGAG GCACCAAGCTGACAGTGCTGGGACAGCCTAAGGCCGCT CCCTCCGTGACCCTGTTCCCCCCCAGCTCCGAGGAACT GCAGGCCAACAAGGCCACCCTGGTGTGCCTGATCAGCG ACTTCTACCCTGGCGCCGTGACCGTGGCCTGGAAGGCC GACAGCAGCCCCGTGAAGGCCGGCGTGGAGACAACCAC CCCCAGCAAGCAGAGCAACAACAAGTACGCCGCCAGCA GCTACCTGAGCCTGACCCCCGAGCAGTGGAAGAGCCAC AGAAGCTACAGCTGCCAGGTCACCCACGAGGGCAGCAC CGTGGAGAAAACCGTGGCCCCCACCGAGTGCAGC |

| **Y1 E152C_S375C** | | |
|---|---|---|
| SEQ ID NO: 103 | HCDR1 (Combined) | GFTFSSYAMS |
| SEQ ID NO: 104 | HCDR2 (Combined) | AISGSGGSTYYADSVKG |
| SEQ ID NO: 105 | HCDR3 (Combined) | AFRLYWLDV |
| SEQ ID NO: 106 | HCDR1 (Kabat) | SYAMS |
| SEQ ID NO: 104 | HCDR2 (Kabat) | AISGSGGSTYYADSVKG |
| SEQ ID NO: 105 | HCDR3 (Kabat) | AFRLYWLDV |
| SEQ ID NO: 107 | HCDR1 (Chothia) | GFTFSSY |
| SEQ ID NO: 108 | HCDR2 (Chothia) | SGSGGS |
| SEQ ID NO: 105 | HCDR3(Chothia) | AFRLYWLDV |
| SEQ ID NO: 109 | HCDR1 (IMGT) | GFTFSSYA |
| SEQ ID NO: 110 | HCDR2 (IMGT) | ISGSGGST |
| SEQ ID NO: 111 | HCDR3 (IMGT) | ARAFRLYWLDV |
| SEQ ID NO: 112 | VH | EVQLLESGGGLVQPGGSLRLSCAASGFTFSSYAMSWVR QAPGKGLEWVSAISGSGGSTYYADSVKGRFTISRDNSK NTLYLQMNSLRAEDTAVYYCARAFRLYWLDVWGQGTLV TVSS |
| SEQ ID NO: 113 | DNA VH | GAAGTTCAGCTGCTGGAATCTGGCGGAGGACTGGTTCA ACCTGGCGGCTCTCTGAGACTGTCTTGTGCCGCCAGCG GCTTCACCTTTAGCAGCTACGCCATGAGCTGGGTCCGA CAGGCTCCTGGCAAAGGCCTTGAATGGGTGTCCGCCAT CTCTGGCTCTGGCGGCAGCACATATTACGCCGACTCTG TGAAGGGCAGATTCACCATCAGCCGGGACAACAGCAAG AACACCCTGTACCTGCAGATGAACAGCCTGAGAGCCGA GGACACCGCCGTGTACTATTGTGCCAGAGCCTTCCGGC TGTACTGGCTGGATGTTTGGGGACAGGGCACCCTGGTC ACAGTGTCATCT |
| SEQ ID NO: 114 | Heavy Chain | EVQLLESGGGLVQPGGSLRLSCAASGFTFSSYAMSWVR QAPGKGLEWVSAISGSGGSTYYADSVKGRFTISRDNSK NTLYLQMNSLRAEDTAVYYCARAFRLYWLDVWGQGTLV TVSSASTKGPSVFPLAPSSKSTSGGTAALGCLVKDYFP CPVTVSWNSGALTSGVHTFPAVLQSSGLYSLSSVVTVP SSSLGTQTYICNVNHKPSNTKVDKRVEPKSCDKTHTCP PCPAPELLGGPSVFLFPPKPKDTLMISRTPEVTCVVVD VSHEDPEVKFNWYVDGVEVHNAKTKPREEQYNSTYRVV SVLTVLHQDWLNGKEYKCKVSNKALPAPIEKTISKAKG QPREPQVYTLPPSREEMTKNQVSLTCLVKGFYPCDIAV EWESNGQPENNYKTTPPVLDSDGSFFLYSKLTVDKSRW QQGNVFSCSVMHEALHNHYTQKSLSLSPGK |
| SEQ ID NO: 115 | DNA Heavy Chain | GAAGTTCAGCTGCTGGAATCTGGCGGAGGACTGGTTCA ACCTGGCGGCTCTCTGAGACTGTCTTGTGCCGCCAGCG GCTTCACCTTTAGCAGCTACGCCATGAGCTGGGTCCGA CAGGCTCCTGGCAAAGGCCTTGAATGGGTGTCCGCCAT CTCTGGCTCTGGCGGCAGCACATATTACGCCGACTCTG TGAAGGGCAGATTCACCATCAGCCGGGACAACAGCAAG AACACCCTGTACCTGCAGATGAACAGCCTGAGAGCCGA GGACACCGCCGTGTACTATTGTGCCAGAGCCTTCCGGC TGTACTGGCTGGATGTTTGGGGACAGGGCACCCTGGTC ACAGTGTCATCTGCTAGCACCAAGGGCCCAAGTGTGTT TCCCCTGGCCCCCAGCAGCAAGTCTACTTCCGGCGGAA CTGCTGCCCTGGGTTGCCTGGTGAAGGACTACTTCCCC TGTCCCGTGACAGTGTCCTGGAACTCTGGGGCTCTGAC TTCCGGCGTGCACACCTTCCCCGCCGTGCTGCAGAGCA GCGGCCTGTACAGCCTGAGCAGCGTGGTGACAGTGCCC TCCAGCTCTCTGGGAACCCAGACCTATATCTGCAACGT GAACCACAAGCCCAGCAACACCAAGGTGGACAAGAGAG TGGAGCCCAAGAGCTGCGACAAGACCCACACCTGCCCC CCCTGCCCAGCTCCAGAACTGCTGGGAGGGCCTTCCGT GTTCCTGTTCCCCCCCAAGCCCAAGGACACCCTGATGA TCAGCAGGACCCCCGAGGTGACCTGCGTGGTGGTGGAC GTGTCCCACGAGGACCCAGAGGTGAAGTTCAACTGGTA CGTGGACGGCGTGGAGGTGCACAACGCCAAGACCAAGC |
| | | CCAGAGAGGAGCAGTACAACAGCACCTACAGGGTGGTG TCCGTGCTGACCGTGCTGCACCAGGACTGGCTGAACGG CAAAGAATACAAGTGCAAAGTCTCCAACAAGGCCCTGC CAGCCCCAATCGAAAAGACAATCAGCAAGGCCAAGGGC CAGCCACGGGAGCCCCAGGTGTACACCCTGCCCCCCAG CCGGGAGGAGATGACCAAGAACCAGGTGTCCCTGACCT GTCTGGTGAAGGGCTTCTACCCCTGTGATATCGCCGTG GAGTGGGAGAGCAACGGCCAGCCCGAGAACAACTACAA GACCACCCCCCCAGTGCTGGACAGCGACGGCAGCTTCT TCCTGTACAGCAAGCTGACCGTGGACAAGTCCAGGTGG CAGCAGGGCAACGTGTTCAGCTGCAGCGTGATGCACGA GGCCCTGCACAACCACTACACCCAGAAGTCCCTGAGCC TGAGCCCCGGCAAG |
| SEQ ID NO: 116 | LCDR1 (Combined) | RASQSISSYLN |
| SEQ ID NO: 47 | LCDR2 (Combined) | AASSLQS |
| SEQ ID NO: 117 | LCDR3 (Combined) | QQVYSAPVT |
| SEQ ID NO: 116 | LCDR1 (Kabat) | RASQSISSYLN |
| SEQ ID NO: 47 | LCDR2 (Kabat) | AASSLQS |
| SEQ ID NO: 117 | LCDR3 (Kabat) | QQVYSAPVT |
| SEQ ID NO: 49 | LCDR1 (Chothia) | SQSISSY |
| SEQ ID NO: 50 | LCDR2 (Chothia) | AAS |
| SEQ ID NO: 118 | LCDR3 (Chothia) | VYSAPV |
| SEQ ID NO: 52 | LCDR1 (IMGT) | QSISSY |
| SEQ ID NO: 50 | LCDR2 (IMGT) | AAS |
| SEQ ID NO: 117 | LCDR3 (IMGT) | QQVYSAPVT |
| SEQ ID NO: 119 | VL | DIQMTQSPSSLSASVGDRVTITCRASQSISSYLNWYQQ KPGKAPKLLIYAASSLQSGVPSRFSGSGSGTDFTLTIS SLOPEDFATYYCOOVYSAPVTFGOGTKVEIK |
| SEQ ID NO: 120 | DNA VL | GACATCCAGATGACACAGAGCCCTAGCAGCCTGTCTGC CAGCGTGGGAGACAGAGTGACCATCACCTGTAGAGCCA GCCAGAGCATCAGCAGCTACCTGAACTGGTATCAGCAG AAGCCCGGCAAGGCCCCTAAACTGCTGATCTATGCCGC CAGCTCTCTGCAGTCTGGCGTGCCAAGCAGATTTTCTG GCAGCGGCTCTGGCACCGACTTCACCCTGACCATATCT AGCCTGCAGCCAGAGGACTTCGCCACCTACTACTGCCA GCAGGTCTACAGCGCCCCTGTGACATTTGGCCAGGGCA CCAAGGTGGAAATCAAG |
| SEQ ID NO: 121 | Light Chain | DIQMTQSPSSLSASVGDRVTITCRASQSISSYLNWYQQ KPGKAPKLLIYAASSLQSGVPSRFSGSGSGTDFTLTIS SLQPEDFATYYCQQVYSAPVTFGQGTKVEIKRTVAAPS VFIFPPSDEQLKSGTASVVCLLNNFYPREAKVQWKVDN ALQSGNSQESVTEQDSKDSTYSLSSTLTLSKADYEKHK VYACEVTHOGLSSPVTKSFNRGEC |
| SEQ ID NO: 122 | DNA Light Chain | GACATCCAGATGACACAGAGCCCTAGCAGCCTGTCTGC CAGCGTGGGAGACAGAGTGACCATCACCTGTAGAGCCA GCCAGAGCATCAGCAGCTACCTGAACTGGTATCAGCAG AAGCCCGGCAAGGCCCCTAAACTGCTGATCTATGCCGC CAGCTCTCTGCAGTCTGGCGTGCCAAGCAGATTTTCTG GCAGCGGCTCTGGCACCGACTTCACCCTGACCATATCT AGCCTGCAGCCAGAGGACTTCGCCACCTACTACTGCCA GCAGGTCTACAGCGCCCCTGTGACATTTGGCCAGGGCA CCAAGGTGGAAATCAAGCGTACGGTGGCCGCTCCCAGC |
| | | GTGTTCATCTTCCCCCCAGCGACGAGCAGCTGAAGAG TGGCACCGCCAGCGTGGTGTGCCTGCTGAACAACTTCT ACCCCCGGGAGGCCAAGGTGCAGTGGAAGGTGGACAAC GCCCTGCAGAGCGGCAACAGCCAGGAGAGCGTCACCGA GCAGGACAGCAAGGACTCCACCTACAGCCTGAGCAGCA CCCTGACCCTGAGCAAGGCCGACTACGAGAAGCATAAG GTGTACGCCTGCGAGGTGACCCACCAGGGCCTGTCCAG CCCCGTGACCAAGAGCTTCAACAGGGGCGAGTGC |

| **Y2 E152C_S375C** | | |
|---|---|---|
| SEQ ID NO: 123 | HCDR1 (Combined) | GFTFSNYWIS |
| SEQ ID NO: 124 | HCDR2 (Combined) | RIKSKTYGGTTDYAEPVKG |
| SEQ ID NO: 125 | HCDR3 (Combined) | TSRRSYAFDY |
| SEQ ID NO: 126 | HCDR1 (Kabat) | NYWIS |
| SEQ ID NO: 124 | HCDR2 (Kabat) | RIKSKTYGGTTDYAEPVKG |
| SEQ ID NO: 125 | HCDR3 (Kabat) | TSRRSYAFDY |
| SEQ ID NO: 127 | HCDR1 (Chothia) | GFTFSNY |
| SEQ ID NO: 128 | HCDR2 (Chothia) | KSKTYGGT |
| SEQ ID NO: 125 | HCDR3 (Chothia) | TSRRSYAFDY |
| SEQ ID NO: 129 | HCDR1 (IMGT) | GFTFSNYW |
| SEQ ID NO: 130 | HCDR2 (IMGT) | IKSKTYGGTT |
| SEQ ID NO: 131 | HCDR3 (IMGT) | ARTSRRSYAFDY |
| SEQ ID NO: 132 | VH | EVQLVESGGGLVKPGGSLRLSCAASGFTFSNYWISWVR QAPGKGLEWVGRIKSKTYGGTTDYAEPVKGRFTISRDD SKNTLYLQMNSLKTEDTAVYYCARTSRRSYAFDYWGQG TLVTVSS |
| SEQ ID NO: 133 | DNA VH | GAAGTGCAGCTGGTGGAATCTGGCGGCGGACTTGTGAA ACCTGGCGGCTCTCTGAGACTGAGCTGTGCCGCTTCCG GCTTCACCTTCAGCAACTACTGGATCAGCTGGGTCCGA CAGGCCCCTGGCAAAGGACTTGAGTGGGTCGGACGGAT CAAGAGCAAGACCTACGGCGGCACCACCGATTATGCCG AGCCTGTGAAGGGCAGATTCACCATCAGCCGGGACGAC AGCAAGAACACCCTGTACCTGCAGATGAACAGCCTGAA AACCGAGGACACCGCCGTGTACTACTGCGCCAGAACCA GCAGAAGAAGCTACGCCTTCGACTACTGGGGCCAGGGC ACACTGGTTACCGTTAGCTCT |
| SEQ ID NO: 134 | Heavy Chain | EVQLVESGGGLVKPGGSLRLSCAASGFTFSNYWISWVR QAPGKGLEWVGRIKSKTYGGTTDYAEPVKGRFTISRDD SKNTLYLQMNSLKTEDTAVYYCARTSRRSYAFDYWGQG TLVTVSSASTKGPSVFPLAPSSKSTSGGTAALGCLVKD YFPCPVTVSWNSGALTSGVHTFPAVLQSSGLYSLSSVV TVPSSSLGTQTYICNVNHKPSNTKVDKRVEPKSCDKTH TCPPCPAPELLGGPSVFLFPPKPKDTLMISRTPEVTCV VVDVSHEDPEVKFNWYVDGVEVHNAKTKPREEQYNSTY RVVSVLTVLHQDWLNGKEYKCKVSNKALPAPIEKTISK AKGQPREPQVYTLPPSREEMTKNQVSLTCLVKGFYPCD IAVEWESNGQPENNYKTTPPVLDSDGSFFLYSKLTVDK SRWOOGNVFSCSVMHEALHNHYTOKSLSLSPGK |
| SEQ ID NO: 135 | DNA Heavy Chain | GAAGTGCAGCTGGTGGAATCTGGCGGCGGACTTGTGAA ACCTGGCGGCTCTCTGAGACTGAGCTGTGCCGCTTCCG GCTTCACCTTCAGCAACTACTGGATCAGCTGGGTCCGA CAGGCCCCTGGCAAAGGACTTGAGTGGGTCGGACGGAT CAAGAGCAAGACCTACGGCGGCACCACCGATTATGCCG |
| | | AGCCTGTGAAGGGCAGATTCACCATCAGCCGGGACGAC AGCAAGAACACCCTGTACCTGCAGATGAACAGCCTGAA AACCGAGGACACCGCCGTGTACTACTGCGCCAGAACCA GCAGAAGAAGCTACGCCTTCGACTACTGGGGCCAGGGC ACACTGGTTACCGTTAGCTCTGCTAGCACCAAGGGCCC AAGTGTGTTTCCCCTGGCCCCCAGCAGCAAGTCTACTT CCGGCGGAACTGCTGCCCTGGGTTGCCTGGTGAAGGAC TACTTCCCCTGTCCCGTGACAGTGTCCTGGAACTCTGG GGCTCTGACTTCCGGCGTGCACACCTTCCCCGCCGTGC TGCAGAGCAGCGGCCTGTACAGCCTGAGCAGCGTGGTG ACAGTGCCCTCCAGCTCTCTGGGAACCCAGACCTATAT CTGCAACGTGAACCACAAGCCCAGCAACACCAAGGTGG ACAAGAGAGTGGAGCCCAAGAGCTGCGACAAGACCCAC ACCTGCCCCCCCTGCCCAGCTCCAGAACTGCTGGGAGG GCCTTCCGTGTTCCTGTTCCCCCCCAAGCCCAAGGACA CCCTGATGATCAGCAGGACCCCCGAGGTGACCTGCGTG GTGGTGGACGTGTCCCACGAGGACCCAGAGGTGAAGTT CAACTGGTACGTGGACGGCGTGGAGGTGCACAACGCCA AGACCAAGCCCAGAGAGGAGCAGTACAACAGCACCTAC AGGGTGGTGTCCGTGCTGACCGTGCTGCACCAGGACTG GCTGAACGGCAAAGAATACAAGTGCAAAGTCTCCAACA AGGCCCTGCCAGCCCCAATCGAAAAGACAATCAGCAAG GCCAAGGGCCAGCCACGGGAGCCCCAGGTGTACACCCT GCCCCCCAGCCGGGAGGAGATGACCAAGAACCAGGTGT CCCTGACCTGTCTGGTGAAGGGCTTCTACCCCTGTGAT ATCGCCGTGGAGTGGGAGAGCAACGGCCAGCCCGAGAA CAACTACAAGACCACCCCCCCAGTGCTGGACAGCGACG GCAGCTTCTTCCTGTACAGCAAGCTGACCGTGGACAAG TCCAGGTGGCAGCAGGGCAACGTGTTCAGCTGCAGCGT GATGCACGAGGCCCTGCACAACCACTACACCCAGAAGT CCCTGAGCCTGAGCCCCGGCAAG |
| SEQ ID NO: 136 | LCDR1 (Combined) | RASQSISSWLA |
| SEQ ID NO: 137 | LCDR2 (Combined) | DASSLES |
| SEQ ID NO: 138 | LCDR3 (Combined) | QQITRYPVT |
| SEQ ID NO: 136 | LCDR1 (Kabat) | RASQSISSWLA |
| SEQ ID NO: 137 | LCDR2 (Kabat) | DASSLES |
| SEQ ID NO: 138 | LCDR3 (Kabat) | QQITRYPVT |
| SEQ ID NO: 139 | LCDR1 (Chothia) | SQSISSW |
| SEQ ID NO: 140 | LCDR2 (Chothia) | DAS |
| SEQ ID NO: 141 | LCDR3 (Chothia) | ITRYPV |
| SEQ ID NO: 142 | LCDR1 (IMGT) | QSISSW |
| SEQ ID NO: 140 | LCDR2 (IMGT) | DAS |
| SEQ ID NO: 138 | LCDR3 (IMGT) | QQITRYPVT |
| SEQ ID NO: 143 | VL | DIQMTQSPSTLSASVGDRVTITCRASQSISSWLAWYQQ KPGKAPKLLIYDASSLESGVPSRFSGSGSGTEFTLTIS SLOPEDFATYYCOOITRYPVTFGOGTKVEIK |
| SEQ ID NO: 144 | DNA VL | GACATCCAGATGACACAGAGCCCCAGCACACTGTCTGC CAGCGTGGGAGACAGAGTGACCATCACCTGTAGAGCCA GCCAGAGCATCTCCTCTTGGCTGGCCTGGTATCAGCAG AAGCCTGGCAAGGCCCCTAAGCTGCTGATCTACGATGC CAGCAGCCTGGAAAGCGGCGTGCCAAGCAGATTTTCTG GCAGCGGCTCTGGCACCGAGTTCACCCTGACCATATCT |
| | | AGCCTGCAGCCAGAGGACTTCGCCACCTACTACTGCCA GCAGATCACAAGATACCCCGTGACCTTTGGCCAGGGCA CCAAGGTGGAAATCAAG |
| SEQ ID NO: 145 | Light Chain | 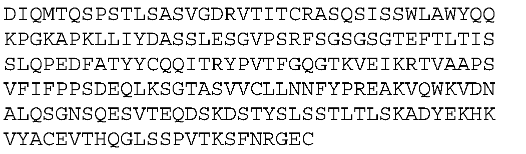 |
| SEQ ID NO: 146 | DNA Light Chain | GACATCCAGATGACACAGAGCCCCAGCACACTGTCTGC CAGCGTGGGAGACAGAGTGACCATCACCTGTAGAGCCA GCCAGAGCATCTCCTCTTGGCTGGCCTGGTATCAGCAG AAGCCTGGCAAGGCCCCTAAGCTGCTGATCTACGATGC CAGCAGCCTGGAAAGCGGCGTGCCAAGCAGATTTTCTG GCAGCGGCTCTGGCACCGAGTTCACCCTGACCATATCT AGCCTGCAGCCAGAGGACTTCGCCACCTACTACTGCCA GCAGATCACAAGATACCCCGTGACCTTTGGCCAGGGCA CCAAGGTGGAAATCAAGCGTACGGTGGCCGCTCCCAGC GTGTTCATCTTCCCCCCCAGCGACGAGCAGCTGAAGAG TGGCACCGCCAGCGTGGTGTGCCTGCTGAACAACTTCT ACCCCCGGGAGGCCAAGGTGCAGTGGAAGGTGGACAAC GCCCTGCAGAGCGGCAACAGCCAGGAGAGCGTCACCGA GCAGGACAGCAAGGACTCCACCTACAGCCTGAGCAGCA CCCTGACCCTGAGCAAGGCCGACTACGAGAAGCATAAG GTGTACGCCTGCGAGGTGACCCACCAGGGCCTGTCCAG CCCCGTGACCAAGAGCTTCAACAGGGGCGAGTGC |

| **Y3 E152C_S375C** | | |
|---|---|---|
| SEQ ID NO: 123 | HCDR1 (Combined) | GFTFSNYWIS |
| SEQ ID NO: 124 | HCDR2 (Combined) | RIKS KTYGGTTDYAE PVKG |
| SEQ ID NO: 147 | HCDR3 (Combined) | VSGYYSHSGGFDV |
| SEQ ID NO: 126 | HCDR1 (Kabat) | NYWIS |
| SEQ ID NO: 124 | HCDR2 (Kabat) | RIKSKTYGGTTDYAEPVKG |
| SEQ ID NO: 147 | HCDR3 (Kabat) | VSGYYSHSGGFDV |
| SEQ ID NO: 127 | HCDR1 (Chothia) | GFTFSNY |
| SEQ ID NO: 128 | HCDR2 (Chothia) | KSKTYGGT |
| SEQ ID NO: 147 | HCDR3 (Chothia) | VSGYYSHSGGFDV |
| SEQ ID NO: 129 | HCDR1 (IMGT) | GFTFSNYW |
| SEQ ID NO: 130 | HCDR2 (IMGT) | IKSKTYGGTT |
| SEQ ID NO: 148 | HCDR3 (IMGT) | ARVSGYYSHSGGFDV |
| SEQ ID NO: 149 | VH | EVQLVESGGGLVKPGGSLRLSCAASGFTFSNYWISWVR QAPGKGLEWVGRIKSKTYGGTTDYAEPVKGRFTISRDD SKNTLYLQMNSLKTEDTAVYYCARVSGYYSHSGGFDVW GQGTLVTVSS |
| SEQ ID NO: 150 | DNA VH | GAAGTGCAGCTGGTGGAATCTGGCGGCGGACTTGTGAA ACCTGGCGGCTCTCTGAGACTGAGCTGTGCCGCTTCCG GCTTCACCTTCAGCAACTACTGGATCAGCTGGGTCCGA CAGGCCCCTGGCAAAGGACTTGAGTGGGTCGGACGGAT CAAGAGCAAGACCTACGGCGGCACCACCGATTATGCCG AGCCTGTGAAGGGCAGATTCACCATCAGCCGGGACGAC AGCAAGAACACCCTGTACCTGCAGATGAACAGCCTGAA AACCGAGGACACCGCCGTGTACTACTGCGCCAGAGTGT |
| | | CTGGCTACTACTCTCACAGCGGCGGCTTTGATGTGTGG GGCCAGGGAACACTGGTCACCGTTAGTTCT |
| SEQ ID NO: 151 | Heavy Chain | EVQLVESGGGLVKPGGSLRLSCAASGFTFSNYWISWVR QAPGKGLEWVGRIKSKTYGGTTDYAEPVKGRFTISRDD SKNTLYLQMNSLKTEDTAVYYCARVSGYYSHSGGFDVW GQGTLVTVSSASTKGPSVFPLAPSSKSTSGGTAALGCL VKDYFPCPVTVSWNSGALTSGVHTFPAVLQSSGLYSLS SVVTVPSSSLGTQTYICNVNHKPSNTKVDKRVEPKSCD KTHTCPPCPAPELLGGPSVFLFPPKPKDTLMISRTPEV TCVVVDVSHEDPEVKFNWYVDGVEVHNAKTKPREEQYN STYRVVSVLTVLHQDWLNGKEYKCKVSNKALPAPIEKT ISKAKGQPREPQVYTLPPSREEMTKNQVSLTCLVKGFY PCDIAVEWESNGQPENNYKTTPPVLDSDGSFFLYSKLT VDKSRWQQGNVFSCSVMHEALHNHYTQKSLSLSPGK |
| SEQ ID NO: 152 | DNA Heavy Chain | GAAGTGCAGCTGGTGGAATCTGGCGGCGGACTTGTGAA ACCTGGCGGCTCTCTGAGACTGAGCTGTGCCGCTTCCG GCTTCACCTTCAGCAACTACTGGATCAGCTGGGTCCGA CAGGCCCCTGGCAAAGGACTTGAGTGGGTCGGACGGAT CAAGAGCAAGACCTACGGCGGCACCACCGATTATGCCG AGCCTGTGAAGGGCAGATTCACCATCAGCCGGGACGAC AGCAAGAACACCCTGTACCTGCAGATGAACAGCCTGAA AACCGAGGACACCGCCGTGTACTACTGCGCCAGAGTGT CTGGCTACTACTCTCACAGCGGCGGCTTTGATGTGTGG GGCCAGGGAACACTGGTCACCGTTAGTTCTGCTAGCAC CAAGGGCCCAAGTGTGTTTCCCCTGGCCCCCAGCAGCA AGTCTACTTCCGGCGGAACTGCTGCCCTGGGTTGCCTG GTGAAGGACTACTTCCCCTGTCCCGTGACAGTGTCCTG GAACTCTGGGGCTCTGACTTCCGGCGTGCACACCTTCC CCGCCGTGCTGCAGAGCAGCGGCCTGTACAGCCTGAGC AGCGTGGTGACAGTGCCCTCCAGCTCTCTGGGAACCCA GACCTATATCTGCAACGTGAACCACAAGCCCAGCAACA CCAAGGTGGACAAGAGAGTGGAGCCCAAGAGCTGCGAC AAGACCCACACCTGCCCCCCCTGCCCAGCTCCAGAACT GCTGGGAGGGCCTTCCGTGTTCCTGTTCCCCCCCAAGC CCAAGGACACCCTGATGATCAGCAGGACCCCCGAGGTG ACCTGCGTGGTGGTGGACGTGTCCCACGAGGACCCAGA GGTGAAGTTCAACTGGTACGTGGACGGCGTGGAGGTGC ACAACGCCAAGACCAAGCCCAGAGAGGAGCAGTACAAC AGCACCTACAGGGTGGTGTCCGTGCTGACCGTGCTGCA CCAGGACTGGCTGAACGGCAAAGAATACAAGTGCAAAG TCTCCAACAAGGCCCTGCCAGCCCCAATCGAAAAGACA ATCAGCAAGGCCAAGGGCCAGCCACGGGAGCCCCAGGT GTACACCCTGCCCCCCAGCCGGGAGGAGATGACCAAGA ACCAGGTGTCCCTGACCTGTCTGGTGAAGGGCTTCTAC CCCTGTGATATCGCCGTGGAGTGGGAGAGCAACGGCCA GCCCGAGAACAACTACAAGACCACCCCCCCAGTGCTGG ACAGCGACGGCAGCTTCTTCCTGTACAGCAAGCTGACC GTGGACAAGTCCAGGTGGCAGCAGGGCAACGTGTTCAG CTGCAGCGTGATGCACGAGGCCCTGCACAACCACTACA CCCAGAAGTCCCTGAGCCTGAGCCCCGGCAAG |
| SEQ ID NO: 153 | LCDR1 (Combined) | RASQGISNYLA |
| SEQ ID NO: 154 | LCDR2 (Combined) | AASTLQS |
| SEQ ID NO: 155 | LCDR3 (Combined) | QKTWRTPGT |
| SEQ ID NO: 153 | LCDR1 (Kabat) | RASQGISNYLA |
| SEQ ID NO: 154 | LCDR2 (Kabat) | AASTLQS |
| SEQ ID NO: 155 | LCDR3 (Kabat) | QKTWRTPGT |
| SEQ ID NO: 156 | LCDR1 (Chothia) | SQGISNY |
| SEQ ID NO: 50 | LCDR2 (Chothia) | AAS |
| SEQ ID NO: 157 | LCDR3 (Chothia) | TWRTPG |
| SEQ ID NO: 158 | LCDR1 (IMGT) | QGISNY |
| SEQ ID NO: 50 | LCDR2 (IMGT) | AAS |
| SEQ ID NO: 155 | LCDR3 (IMGT) | QKTWRTPGT |
| SEQ ID NO: 159 | VL | DIQMTQSPSSLSASVGDRVTITCRASQGISNYLAWYQQ KPGKVPKLLIYAASTLQSGVPSRFSGSGSGTDFTLTIS SLQPEDVATYYCQKTWRTPGTFGQGTKVEIK |
| SEQ ID NO: 160 | DNA VL | GACATCCAGATGACACAGAGCCCTAGCAGCCTGTCTGC CAGCGTGGGAGACAGAGTGACCATCACCTGTAGAGCCA GCCAGGGCATCAGCAACTACCTGGCCTGGTATCAGCAG AAACCCGGCAAGGTGCCCAAGCTGCTGATCTACGCTGC CAGCACACTGCAGAGCGGAGTGCCTAGCAGATTTTCTG GCAGCGGCTCCGGCACCGATTTCACCCTGACCATATCT AGCCTGCAGCCAGAGGACGTGGCCACCTACTACTGTCA GAAAACCTGGCGGACCCCTGGCACATTTGGCCAGGGAA CAAAGGTGGAAATCAAG |
| SEQ ID NO: 161 | Light Chain | DIQMTQSPSSLSASVGDRVTITCRASQGISNYLAWYQQ KPGKVPKLLIYAASTLQSGVPSRFSGSGSGTDFTLTIS SLQPEDVATYYCQKTWRTPGTFGQGTKVEIKRTVAAPS VFIFPPSDEQLKSGTASVVCLLNNFYPREAKVQWKVDN ALQSGNSQESVTEQDSKDSTYSLSSTLTLSKADYEKHK VYACEVTHQGLSSPVTKSFNRGEC |
| SEQ ID NO: 162 | DNA Light Chain | GACATCCAGATGACACAGAGCCCTAGCAGCCTGTCTGC CAGCGTGGGAGACAGAGTGACCATCACCTGTAGAGCCA GCCAGGGCATCAGCAACTACCTGGCCTGGTATCAGCAG AAACCCGGCAAGGTGCCCAAGCTGCTGATCTACGCTGC CAGCACACTGCAGAGCGGAGTGCCTAGCAGATTTTCTG GCAGCGGCTCCGGCACCGATTTCACCCTGACCATATCT AGCCTGCAGCCAGAGGACGTGGCCACCTACTACTGTCA GAAAACCTGGCGGACCCCTGGCACATTTGGCCAGGGAA CAAAGGTGGAAATCAAGCGTACGGTGGCCGCTCCCAGC GTGTTCATCTTCCCCCCCAGCGACGAGCAGCTGAAGAG TGGCACCGCCAGCGTGGTGTGCCTGCTGAACAACTTCT ACCCCCGGGAGGCCAAGGTGCAGTGGAAGGTGGACAAC GCCCTGCAGAGCGGCAACAGCCAGGAGAGCGTCACCGA GCAGGACAGCAAGGACTCCACCTACAGCCTGAGCAGCA CCCTGACCCTGAGCAAGGCCGACTACGAGAAGCATAAG GTGTACGCCTGCGAGGTGACCCACCAGGGCCTGTCCAG CCCCGTGACCAAGAGCTTCAACAGGGGCGAGTGC |

| **Y4 E152C_S375C** | | |
|---|---|---|
| SEQ ID NO: 103 | HCDR1 (Combined) | GFTFSSYAMS |
| SEQ ID NO: 104 | HCDR2 (Combined) | AISGSGGSTYYADSVKG |
| SEQ ID NO: 163 | HCDR3 (Combined) | SRLIAPWLDY |
| SEQ ID NO: 106 | HCDR1 (Kabat) | SYAMS |
| SEQ ID NO: 104 | HCDR2 (Kabat) | AISGSGGSTYYADSVKG |
| SEQ ID NO: 163 | HCDR3 (Kabat) | SRLIAPWLDY |
| SEQ ID NO: 107 | HCDR1 (Chothia) | GFTFSSY |
| SEQ ID NO: 108 | HCDR2 (Chothia) | SGSGGS |
| SEQ ID NO: 163 | HCDR3(Chothia) | SRLIAPWLDY |
| SEQ ID NO: 109 | HCDR1 (IMGT) | GFTFSSYA |
| SEQ ID NO: 110 | HCDR2 (IMGT) | ISGSGGST |
| SEQ ID NO: 164 | HCDR3 (IMGT) | ARSRLIAPWLDY |
| SEQ ID NO: 165 | VH | EVQLLESGGGLVQPGGSLRLSCAASGFTFSSYAMSWVR QAPGKGLEWVSAISGSGGSTYYADSVKGRFTISRDNSK NTLYLQMNSLRAEDTAVYYCARSRLIAPWLDYWGQGTL VTVSS |
| SEQ ID NO: 166 | DNA VH | GAAGTTCAGCTGCTGGAATCTGGCGGAGGACTGGTTCA ACCTGGCGGCTCTCTGAGACTGTCTTGTGCCGCCAGCG GCTTCACCTTTAGCAGCTACGCCATGAGCTGGGTCCGA CAGGCTCCTGGCAAAGGCCTTGAATGGGTGTCCGCCAT CTCTGGCTCTGGCGGCAGCACATATTACGCCGACTCTG TGAAGGGCAGATTCACCATCAGCCGGGACAACAGCAAG AACACCCTGTACCTGCAGATGAACAGCCTGAGAGCCGA GGACACCGCCGTGTACTACTGTGCCAGAAGCAGACTGA TCGCCCCTTGGCTGGATTATTGGGGCCAGGGCACACTG GTCACCGTGTCATCT |
| SEQ ID NO: 167 | Heavy Chain | EVQLLESGGGLVQPGGSLRLSCAASGFTFSSYAMSWVR QAPGKGLEWVSAISGSGGSTYYADSVKGRFTISRDNSK NTLYLQMNSLRAEDTAVYYCARSRLIAPWLDYWGQGTL VTVSSASTKGPSVFPLAPSSKSTSGGTAALGCLVKDYF PCPVTVSWNSGALTSGVHTFPAVLQSSGLYSLSSVVTV PSSSLGTQTYICNVNHKPSNTKVDKRVEPKSCDKTHTC PPCPAPELLGGPSVFLFPPKPKDTLMISRTPEVTCVVV DVSHEDPEVKFNWYVDGVEVHNAKTKPREEQYNSTYRV VSVLTVLHQDWLNGKEYKCKVSNKALPAPIEKTISKAK GQPREPQVYTLPPSREEMTKNQVSLTCLVKGFYPCDIA VEWESNGQPENNYKTTPPVLDSDGSFFLYSKLTVDKSR WQQGNVFSCSVMHEALHNHYTQKSLSLSPGK |
| SEQ ID NO: 168 | DNA Heavy Chain | GAAGTTCAGCTGCTGGAATCTGGCGGAGGACTGGTTCA ACCTGGCGGCTCTCTGAGACTGTCTTGTGCCGCCAGCG GCTTCACCTTTAGCAGCTACGCCATGAGCTGGGTCCGA CAGGCTCCTGGCAAAGGCCTTGAATGGGTGTCCGCCAT CTCTGGCTCTGGCGGCAGCACATATTACGCCGACTCTG TGAAGGGCAGATTCACCATCAGCCGGGACAACAGCAAG AACACCCTGTACCTGCAGATGAACAGCCTGAGAGCCGA GGACACCGCCGTGTACTACTGTGCCAGAAGCAGACTGA TCGCCCCTTGGCTGGATTATTGGGGCCAGGGCACACTG GTCACCGTGTCATCTGCTAGCACCAAGGGCCCAAGTGT GTTTCCCCTGGCCCCCAGCAGCAAGTCTACTTCCGGCG GAACTGCTGCCCTGGGTTGCCTGGTGAAGGACTACTTC CCCTGTCCCGTGACAGTGTCCTGGAACTCTGGGGCTCT GACTTCCGGCGTGCACACCTTCCCCGCCGTGCTGCAGA GCAGCGGCCTGTACAGCCTGAGCAGCGTGGTGACAGTG CCCTCCAGCTCTCTGGGAACCCAGACCTATATCTGCAA CGTGAACCACAAGCCCAGCAACACCAAGGTGGACAAGA GAGTGGAGCCCAAGAGCTGCGACAAGACCCACACCTGC CCCCCCTGCCCAGCTCCAGAACTGCTGGGAGGGCCTTC CGTGTTCCTGTTCCCCCCCAAGCCCAAGGACACCCTGA TGATCAGCAGGACCCCCGAGGTGACCTGCGTGGTGGTG GACGTGTCCCACGAGGACCCAGAGGTGAAGTTCAACTG GTACGTGGACGGCGTGGAGGTGCACAACGCCAAGACCA |
| | | AGCCCAGAGAGGAGCAGTACAACAGCACCTACAGGGTG GTGTCCGTGCTGACCGTGCTGCACCAGGACTGGCTGAA CGGCAAAGAATACAAGTGCAAAGTCTCCAACAAGGCCC TGCCAGCCCCAATCGAAAAGACAATCAGCAAGGCCAAG GGCCAGCCACGGGAGCCCCAGGTGTACACCCTGCCCCC CAGCCGGGAGGAGATGACCAAGAACCAGGTGTCCCTGA CCTGTCTGGTGAAGGGCTTCTACCCCTGTGATATCGCC GTGGAGTGGGAGAGCAACGGCCAGCCCGAGAACAACTA CAAGACCACCCCCCCAGTGCTGGACAGCGACGGCAGCT TCTTCCTGTACAGCAAGCTGACCGTGGACAAGTCCAGG TGGCAGCAGGGCAACGTGTTCAGCTGCAGCGTGATGCA CGAGGCCCTGCACAACCACTACACCCAGAAGTCCCTGA GCCTGAGCCCCGGCAAG |
| SEQ ID NO: 116 | LCDR1 (Combined) | RASQSISSYLN |
| SEQ ID NO: 47 | LCDR2 (Combined) | AASSLQS |
| SEQ ID NO: 169 | LCDR3 (Combined) | QQVYGSPPT |
| SEQ ID NO: 116 | LCDR1 (Kabat) | RASQSISSYLN |
| SEQ ID NO: 47 | LCDR2 (Kabat) | AASSLQS |
| SEQ ID NO: 169 | LCDR3 (Kabat) | QQVYGSPPT |
| SEQ ID NO: 49 | LCDR1 (Chothia) | SQSISSY |
| SEQ ID NO: 50 | LCDR2 (Chothia) | AAS |
| SEQ ID NO: 170 | LCDR3 (Chothia) | VYGSPP |
| SEQ ID NO: 52 | LCDR1 (IMGT) | QSISSY |
| SEQ ID NO: 50 | LCDR2 (IMGT) | AAS |
| SEQ ID NO: 169 | LCDR3 (IMGT) | QQVYGSPPT |
| SEQ ID NO: 171 | VL | DIQMTQSPSSLSASVGDRVTITCRASQSISSYLNWYQQ KPGKAPKLLIYAASSLQSGVPSRFSGSGSGTDFTLTIS SLQPEDFATYYCQQVYGSPPTFGQGTKVEIK |
| SEQ ID NO: 172 | DNA VL | GACATCCAGATGACACAGAGCCCTAGCAGCCTGTCTGC CAGCGTGGGAGACAGAGTGACCATCACCTGTAGAGCCA GCCAGAGCATCAGCAGCTACCTGAACTGGTATCAGCAG AAGCCCGGCAAGGCCCCTAAACTGCTGATCTATGCCGC CAGCTCTCTGCAGTCTGGCGTGCCAAGCAGATTTTCTG GCAGCGGCTCTGGCACCGACTTCACCCTGACCATATCT AGCCTGCAGCCAGAGGACTTCGCCACCTACTACTGCCA GCAGGTCTACGGCAGCCCTCCTACATTTGGCCAGGGCA CCAAGGTGGAAATCAAG |
| SEQ ID NO: 173 | Light Chain | DIQMTQSPSSLSASVGDRVTITCRASQSISSYLNWYQQ KPGKAPKLLIYAASSLQSGVPSRFSGSGSGTDFTLTIS SLQPEDFATYYCQQVYGSPPTFGQGTKVEIKRTVAAPS VFIFPPSDEQLKSGTASVVCLLNNFYPREAKVQWKVDN ALQSGNSQESVTEQDSKDSTYSLSSTLTLSKADYEKHK VYACEVTHQGLSSPVTKSFNRGEC |
| SEQ ID NO: 174 | DNA Light Chain | GACATCCAGATGACACAGAGCCCTAGCAGCCTGTCTGC CAGCGTGGGAGACAGAGTGACCATCACCTGTAGAGCCA GCCAGAGCATCAGCAGCTACCTGAACTGGTATCAGCAG AAGCCCGGCAAGGCCCCTAAACTGCTGATCTATGCCGC CAGCTCTCTGCAGTCTGGCGTGCCAAGCAGATTTTCTG GCAGCGGCTCTGGCACCGACTTCACCCTGACCATATCT AGCCTGCAGCCAGAGGACTTCGCCACCTACTACTGCCA GCAGGTCTACGGCAGCCCTCCTACATTTGGCCAGGGCA CCAAGGTGGAAATCAAGCGTACGGTGGCCGCTCCCAGC |
| | | GTGTTCATCTTCCCCCCCAGCGACGAGCAGCTGAAGAG TGGCACCGCCAGCGTGGTGTGCCTGCTGAACAACTTCT ACCCCCGGGAGGCCAAGGTGCAGTGGAAGGTGGACAAC GCCCTGCAGAGCGGCAACAGCCAGGAGAGCGTCACCGA GCAGGACAGCAAGGACTCCACCTACAGCCTGAGCAGCA CCCTGACCCTGAGCAAGGCCGACTACGAGAAGCATAAG GTGTACGCCTGCGAGGTGACCCACCAGGGCCTGTCCAG CCCCGTGACCAAGAGCTTCAACAGGGGCGAGTGC |

| **Y5E152C_S375C** | | |
|---|---|---|
| SEQ ID NO: 175 | HCDR1 (Combined) | GYSFTSYWIG |
| SEQ ID NO: 176 | HCDR2 (Combined) | IIYPGDSDTRYSPSFQG |
| SEQ ID NO: 177 | HCDR3 (Combined) | GSSAASGLSGDL |
| SEQ ID NO: 178 | HCDR1 (Kabat) | SYWIG |
| SEQ ID NO: 176 | HCDR2 (Kabat) | IIYPGDSDTRYSPSFQG |
| SEQ ID NO: 177 | HCDR3 (Kabat) | GSSAASGLSGDL |
| SEQ ID NO: 179 | HCDR1 (Chothia) | GYSFTSY |
| SEQ ID NO: 180 | HCDR2 (Chothia) | YPGDSD |
| SEQ ID NO: 177 | HCDR3 (Chothia) | GSSAASGLSGDL |
| SEQ ID NO: 181 | HCDR1 (IMGT) | GYSFTSYW |
| SEQ ID NO: 182 | HCDR2 (IMGT) | IYPGDSDT |
| SEQ ID NO: 183 | HCDR3 (IMGT) | ARGSSAASGLSGDL |
| SEQ ID NO: 184 | VH | EVQLVQSGAEVKKPGESLKISCKGSGYSFTSYWIGWVR QMPGKGLEWMGIIYPGDSDTRYSPSFQGQVTISADKSI STAYLQWSSLKASDTAMYYCARGSSAASGLSGDLWGQG TLVTVSS |
| SEQ ID NO: 185 | DNA VH | GAAGTTCAGCTGGTGCAGTCTGGCGCCGAAGTGAAGAA GCCTGGCGAGAGCCTGAAGATCTCCTGCAAAGGCAGCG GCTACAGCTTCACCAGCTACTGGATCGGCTGGGTCCGA CAGATGCCTGGCAAAGGCCTTGAGTGGATGGGCATCAT CTACCCCGGCGACAGCGACACCAGATACAGCCCTAGCT TTCAGGGCCAAGTGACCATCAGCGCCGACAAGAGCATC AGCACAGCCTACCTGCAGTGGTCCAGCCTGAAGGCCTC TGACACCGCCATGTACTACTGTGCCAGAGGAAGCTCTG CCGCCTCTGGACTGTCTGGCGATCTTTGGGGACAGGGC ACACTGGTCACAGTGTCTAGT |
| SEQ ID NO: 186 | Heavy Chain | EVQLVQSGAEVKKPGESLKISCKGSGYSFTSYWIGWVR QMPGKGLEWMGIIYPGDSDTRYSPSFQGQVTISADKSI STAYLQWSSLKASDTAMYYCARGSSAASGLSGDLWGQG TLVTVSSASTKGPSVFPLAPSSKSTSGGTAALGCLVKD YFPCPVTVSWNSGALTSGVHTFPAVLQSSGLYSLSSVV TVPSSSLGTQTYICNVNHKPSNTKVDKRVEPKSCDKTH TCPPCPAPELLGGPSVFLFPPKPKDTLMISRTPEVTCV VVDVSHEDPEVKFNWYVDGVEVHNAKTKPREEQYNSTY RVVSVLTVLHQDWLNGKEYKCKVSNKALPAPIEKTISK AKGQPREPQVYTLPPSREEMTKNQVSLTCLVKGFYPCD IAVEWESNGQPENNYKTTPPVLDSDGSFFLYSKLTVDK SRWOOGNVFSCSVMHEALHNHYTOKSLSLSPGK |
| SEQ ID NO: 187 | DNA Heavy Chain | GAAGTTCAGCTGGTGCAGTCTGGCGCCGAAGTGAAGAA GCCTGGCGAGAGCCTGAAGATCTCCTGCAAAGGCAGCG GCTACAGCTTCACCAGCTACTGGATCGGCTGGGTCCGA CAGATGCCTGGCAAAGGCCTTGAGTGGATGGGCATCAT CTACCCCGGCGACAGCGACACCAGATACAGCCCTAGCT |
| | | TTCAGGGCCAAGTGACCATCAGCGCCGACAAGAGCATC AGCACAGCCTACCTGCAGTGGTCCAGCCTGAAGGCCTC TGACACCGCCATGTACTACTGTGCCAGAGGAAGCTCTG CCGCCTCTGGACTGTCTGGCGATCTTTGGGGACAGGGC ACACTGGTCACAGTGTCTAGTGCTAGCACCAAGGGCCC AAGTGTGTTTCCCCTGGCCCCCAGCAGCAAGTCTACTT CCGGCGGAACTGCTGCCCTGGGTTGCCTGGTGAAGGAC TACTTCCCCTGTCCCGTGACAGTGTCCTGGAACTCTGG GGCTCTGACTTCCGGCGTGCACACCTTCCCCGCCGTGC TGCAGAGCAGCGGCCTGTACAGCCTGAGCAGCGTGGTG ACAGTGCCCTCCAGCTCTCTGGGAACCCAGACCTATAT CTGCAACGTGAACCACAAGCCCAGCAACACCAAGGTGG ACAAGAGAGTGGAGCCCAAGAGCTGCGACAAGACCCAC ACCTGCCCCCCCTGCCCAGCTCCAGAACTGCTGGGAGG GCCTTCCGTGTTCCTGTTCCCCCCCAAGCCCAAGGACA CCCTGATGATCAGCAGGACCCCCGAGGTGACCTGCGTG GTGGTGGACGTGTCCCACGAGGACCCAGAGGTGAAGTT CAACTGGTACGTGGACGGCGTGGAGGTGCACAACGCCA AGACCAAGCCCAGAGAGGAGCAGTACAACAGCACCTAC AGGGTGGTGTCCGTGCTGACCGTGCTGCACCAGGACTG GCTGAACGGCAAAGAATACAAGTGCAAAGTCTCCAACA AGGCCCTGCCAGCCCCAATCGAAAAGACAATCAGCAAG GCCAAGGGCCAGCCACGGGAGCCCCAGGTGTACACCCT GCCCCCCAGCCGGGAGGAGATGACCAAGAACCAGGTGT CCCTGACCTGTCTGGTGAAGGGCTTCTACCCCTGTGAT ATCGCCGTGGAGTGGGAGAGCAACGGCCAGCCCGAGAA CAACTACAAGACCACCCCCCCAGTGCTGGACAGCGACG GCAGCTTCTTCCTGTACAGCAAGCTGACCGTGGACAAG TCCAGGTGGCAGCAGGGCAACGTGTTCAGCTGCAGCGT GATGCACGAGGCCCTGCACAACCACTACACCCAGAAGT CCCTGAGCCTGAGCCCCGGCAAG |
| SEQ ID NO: 116 | LCDR1 (Combined) | RASQSISSYLN |
| SEQ ID NO: 47 | LCDR2 (Combined) | AASSLQS |
| SEQ ID NO: 188 | LCDR3 (Combined) | QQDYYSPFT |
| SEQ ID NO: 116 | LCDR1 (Kabat) | RASQSISSYLN |
| SEQ ID NO: 47 | LCDR2 (Kabat) | AASSLQS |
| SEQ ID NO: 188 | LCDR3 (Kabat) | QQDYYSPFT |
| SEQ ID NO: 49 | LCDR1 (Chothia) | SQSISSY |
| SEQ ID NO: 50 | LCDR2 (Chothia) | AAS |
| SEQ ID NO: 189 | LCDR3 (Chothia) | DYYSPF |
| SEQ ID NO: 52 | LCDR1 (IMGT) | QSISSY |
| SEQ ID NO: 50 | LCDR2 (IMGT) | AAS |
| SEQ ID NO: 188 | LCDR3 (IMGT) | QQDYYSPFT |
| SEQ ID NO: 190 | VL | DIQMTQSPSSLSASVGDRVTITCRASQSISSYLNWYQQ KPGKAPKLLIYAASSLQSGVPSRFSGSGSGTDFTLTIS SLQPEDFATYYCQQDYYSPFTFGQGTKVEIK |
| SEQ ID NO: 191 | DNA VL | GACATCCAGATGACACAGAGCCCTAGCAGCCTGTCTGC CAGCGTGGGAGACAGAGTGACCATCACCTGTAGAGCCA GCCAGAGCATCAGCAGCTACCTGAACTGGTATCAGCAG AAGCCCGGCAAGGCCCCTAAACTGCTGATCTATGCCGC CAGCTCTCTGCAGTCTGGCGTGCCAAGCAGATTTTCTG GCAGCGGCTCTGGCACCGACTTCACCCTGACCATATCT |
| | | AGCCTGCAGCCAGAGGACTTCGCCACCTACTACTGCCA GCAGGACTACTACAGCCCCTTCACCTTTGGCCAGGGCA CCAAGGTGGAAATCAAG |
| SEQ ID NO: 192 | Light Chain | DIQMTQSPSSLSASVGDRVTITCRASQSISSYLNWYQQ KPGKAPKLLIYAASSLQSGVPSRFSGSGSGTDFTLTIS SLQPEDFATYYCQQDYYSPFTFGQGTKVEIKRTVAAPS VFIFPPSDEQLKSGTASVVCLLNNFYPREAKVQWKVDN ALQSGNSQESVTEQDSKDSTYSLSSTLTLSKADYEKHK VYACEVTHQGLSSPVTKSFNRGEC |
| SEQ ID NO: 193 | DNA Light Chain | GACATCCAGATGACACAGAGCCCTAGCAGCCTGTCTGC CAGCGTGGGAGACAGAGTGACCATCACCTGTAGAGCCA GCCAGAGCATCAGCAGCTACCTGAACTGGTATCAGCAG AAGCCCGGCAAGGCCCCTAAACTGCTGATCTATGCCGC CAGCTCTCTGCAGTCTGGCGTGCCAAGCAGATTTTCTG GCAGCGGCTCTGGCACCGACTTCACCCTGACCATATCT AGCCTGCAGCCAGAGGACTTCGCCACCTACTACTGCCA GCAGGACTACTACAGCCCCTTCACCTTTGGCCAGGGCA CCAAGGTGGAAATCAAGCGTACGGTGGCCGCTCCCAGC GTGTTCATCTTCCCCCCCAGCGACGAGCAGCTGAAGAG TGGCACCGCCAGCGTGGTGTGCCTGCTGAACAACTTCT ACCCCCGGGAGGCCAAGGTGCAGTGGAAGGTGGACAAC GCCCTGCAGAGCGGCAACAGCCAGGAGAGCGTCACCGA GCAGGACAGCAAGGACTCCACCTACAGCCTGAGCAGCA CCCTGACCCTGAGCAAGGCCGACTACGAGAAGCATAAG GTGTACGCCTGCGAGGTGACCCACCAGGGCCTGTCCAG CCCCGTGACCAAGAGCTTCAACAGGGGCGAGTGC |

| **Y6 E152C_S375C** | | |
|---|---|---|
| SEQ ID NO: 103 | HCDR1 (Combined) | GFTFSSYAMS |
| SEQ ID NO: 104 | HCDR2 (Combined) | AISGSGGSTYYADSVKG |
| SEQ ID NO: 194 | HCDR3 (Combined) | AYKLSWLDL |
| SEQ ID NO: 106 | HCDR1 (Kabat) | SYAMS |
| SEQ ID NO: 104 | HCDR2 (Kabat) | AISGSGGSTYYADSVKG |
| SEQ ID NO: 194 | HCDR3 (Kabat) | AYKLSWLDL |
| SEQ ID NO: 107 | HCDR1 (Chothia) | GFTFSSY |
| SEQ ID NO: 108 | HCDR2 (Chothia) | SGSGGS |
| SEQ ID NO: 194 | HCDR3 (Chothia) | AYKLSWLDL |
| SEQ ID NO: 109 | HCDR1 (IMGT) | GFTFSSYA |
| SEQ ID NO: 110 | HCDR2 (IMGT) | ISGSGGST |
| SEQ ID NO: 195 | HCDR3 (IMGT) | ARAYKLSWLDL |
| SEQ ID NO: 196 | VH | EVQLLESGGGLVQPGGSLRLSCAASGFTFSSYAMSWVR QAPGKGLEWVSAISGSGGSTYYADSVKGRFTISRDNSK NTLYLQMNSLRAEDTAVYYCARAYKLSWLDLWGQGTLV TVSS |
| SEQ ID NO: 197 | DNA VH | GAAGTTCAGCTGCTGGAATCTGGCGGAGGACTGGTTCA ACCTGGCGGCTCTCTGAGACTGTCTTGTGCCGCCAGCG GCTTCACCTTTAGCAGCTACGCCATGAGCTGGGTCCGA CAGGCTCCTGGCAAAGGCCTTGAATGGGTGTCCGCCAT CTCTGGCTCTGGCGGCAGCACATATTACGCCGACTCTG TGAAGGGCAGATTCACCATCAGCCGGGACAACAGCAAG AACACCCTGTACCTGCAGATGAACAGCCTGAGAGCCGA GGACACCGCCGTGTACTATTGTGCCAGAGCCTACAAGC |
| | | TGAGCTGGCTGGATCTTTGGGGCCAGGGCACACTGGTC ACAGTGTCATCT |
| SEQ ID NO: 198 | Heavy Chain | EVQLLESGGGLVQPGGSLRLSCAASGFTFSSYAMSWVR QAPGKGLEWVSAISGSGGSTYYADSVKGRFTISRDNSK NTLYLQMNSLRAEDTAVYYCARAYKLSWLDLWGQGTLV TVSSASTKGPSVFPLAPSSKSTSGGTAALGCLVKDYFP CPVTVSWNSGALTSGVHTFPAVLQSSGLYSLSSVVTVP SSSLGTQTYICNVNHKPSNTKVDKRVEPKSCDKTHTCP PCPAPELLGGPSVFLFPPKPKDTLMISRTPEVTCVVVD VSHEDPEVKFNWYVDGVEVHNAKTKPREEQYNSTYRVV SVLTVLHQDWLNGKEYKCKVSNKALPAPIEKTISKAKG QPREPQVYTLPPSREEMTKNQVSLTCLVKGFYPCDIAV EWESNGQPENNYKTTPPVLDSDGSFFLYSKLTVDKSRW QQGNVFSCSVMHEALHNHYTQKSLSLSPGK |
| SEQ ID NO: 199 | DNA Heavy Chain | GAAGTTCAGCTGCTGGAATCTGGCGGAGGACTGGTTCA ACCTGGCGGCTCTCTGAGACTGTCTTGTGCCGCCAGCG GCTTCACCTTTAGCAGCTACGCCATGAGCTGGGTCCGA CAGGCTCCTGGCAAAGGCCTTGAATGGGTGTCCGCCAT CTCTGGCTCTGGCGGCAGCACATATTACGCCGACTCTG TGAAGGGCAGATTCACCATCAGCCGGGACAACAGCAAG AACACCCTGTACCTGCAGATGAACAGCCTGAGAGCCGA GGACACCGCCGTGTACTATTGTGCCAGAGCCTACAAGC TGAGCTGGCTGGATCTTTGGGGCCAGGGCACACTGGTC ACAGTGTCATCTGCTAGCACCAAGGGCCCAAGTGTGTT TCCCCTGGCCCCCAGCAGCAAGTCTACTTCCGGCGGAA CTGCTGCCCTGGGTTGCCTGGTGAAGGACTACTTCCCC TGTCCCGTGACAGTGTCCTGGAACTCTGGGGCTCTGAC TTCCGGCGTGCACACCTTCCCCGCCGTGCTGCAGAGCA GCGGCCTGTACAGCCTGAGCAGCGTGGTGACAGTGCCC TCCAGCTCTCTGGGAACCCAGACCTATATCTGCAACGT GAACCACAAGCCCAGCAACACCAAGGTGGACAAGAGAG TGGAGCCCAAGAGCTGCGACAAGACCCACACCTGCCCC CCCTGCCCAGCTCCAGAACTGCTGGGAGGGCCTTCCGT GTTCCTGTTCCCCCCCAAGCCCAAGGACACCCTGATGA TCAGCAGGACCCCCGAGGTGACCTGCGTGGTGGTGGAC GTGTCCCACGAGGACCCAGAGGTGAAGTTCAACTGGTA CGTGGACGGCGTGGAGGTGCACAACGCCAAGACCAAGC CCAGAGAGGAGCAGTACAACAGCACCTACAGGGTGGTG TCCGTGCTGACCGTGCTGCACCAGGACTGGCTGAACGG CAAAGAATACAAGTGCAAAGTCTCCAACAAGGCCCTGC CAGCCCCAATCGAAAAGACAATCAGCAAGGCCAAGGGC CAGCCACGGGAGCCCCAGGTGTACACCCTGCCCCCCAG CCGGGAGGAGATGACCAAGAACCAGGTGTCCCTGACCT GTCTGGTGAAGGGCTTCTACCCCTGTGATATCGCCGTG GAGTGGGAGAGCAACGGCCAGCCCGAGAACAACTACAA GACCACCCCCCCAGTGCTGGACAGCGACGGCAGCTTCT TCCTGTACAGCAAGCTGACCGTGGACAAGTCCAGGTGG CAGCAGGGCAACGTGTTCAGCTGCAGCGTGATGCACGA GGCCCTGCACAACCACTACACCCAGAAGTCCCTGAGCC TGAGCCCCGGCAAG |
| SEQ ID NO: 116 | LCDR1 (Combined) | RASQSISSYLN |
| SEQ ID NO: 47 | LCDR2 (Combined) | AASSLQS |
| SEQ ID NO: 200 | LCDR3 (Combined) | QQVWYAPVT |
| SEQ ID NO: 116 | LCDR1 (Kabat) | RASQSISSYLN |
| SEQ ID NO: 47 | LCDR2 (Kabat) | AASSLQS |
| SEQ ID NO: 200 | LCDR3 (Kabat) | QQVWYAPVT |
| SEQ ID NO: 49 | LCDR1 (Chothia) | SQSISSY |
| SEQ ID NO: 50 | LCDR2 (Chothia) | AAS |
| SEQ ID NO: 201 | LCDR3 (Chothia) | VWYAPV |
| SEQ ID NO: 52 | LCDR1 (IMGT) | QSISSY |
| SEQ ID NO: 50 | LCDR2 (IMGT) | AAS |
| SEQ ID NO: 200 | LCDR3 (IMGT) | QQVWYAPVT |
| SEQ ID NO: 202 | VL | DIQMTQSPSSLSASVGDRVTITCRASQSISSYLNWYQQ KPGKAPKLLIYAASSLQSGVPSRFSGSGSGTDFTLTIS SLQPEDFATYYCQQVWYAPVTFGQGTKVEIK |
| SEQ ID NO: 203 | DNA VL | GACATCCAGATGACACAGAGCCCTAGCAGCCTGTCTGC CAGCGTGGGAGACAGAGTGACCATCACCTGTAGAGCCA GCCAGAGCATCAGCAGCTACCTGAACTGGTATCAGCAG AAGCCCGGCAAGGCCCCTAAACTGCTGATCTATGCCGC CAGCTCTCTGCAGTCTGGCGTGCCAAGCAGATTTTCTG GCAGCGGCTCTGGCACCGACTTCACCCTGACCATATCT AGCCTGCAGCCAGAGGACTTCGCCACCTACTACTGCCA GCAAGTTTGGTACGCCCCTGTGACCTTTGGCCAGGGCA CCAAGGTGGAAATCAAG |
| SEQ ID NO: 204 | Light Chain | DIQMTQSPSSLSASVGDRVTITCRASQSISSYLNWYQQ KPGKAPKLLIYAASSLQSGVPSRFSGSGSGTDFTLTIS SLQPEDFATYYCQQVWYAPVTFGQGTKVEIKRTVAAPS VFIFPPSDEQLKSGTASVVCLLNNFYPREAKVQWKVDN ALQSGNSQESVTEQDSKDSTYSLSSTLTLSKADYEKHK VYACEVTHQGLSSPVTKSFNRGEC |
| SEQ ID NO: 205 | DNA Light Chain | 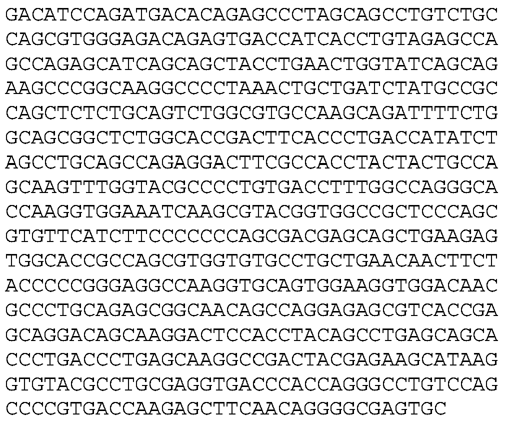 |

| **Y7 E152C_S375C** | | |
|---|---|---|
| SEQ ID NO: 206 | HCDR1 (Combined) | GFTFSNAWMS |
| SEQ ID NO: 207 | HCDR2 (Combined) | RIKSKTDAGTTDYAAPVKG |
| SEQ ID NO: 208 | HCDR3 (Combined) | TIYPSAPSSSLDY |
| SEQ ID NO: 209 | HCDR1 (Kabat) | NAWMS |
| SEQ ID NO: 207 | HCDR2 (Kabat) | RIKSKTDAGTTDYAAPVKG |
| SEQ ID NO: 208 | HCDR3 (Kabat) | TIYPSAPSSSLDY |
| SEQ ID NO: 210 | HCDR1 (Chothia) | GFTFSNA |
| SEQ ID NO: 211 | HCDR2 (Chothia) | KSKTDAGT |
| SEQ ID NO: 208 | HCDR3(Chothia) | TIYPSAPSSSLDY |
| SEQ ID NO: 212 | HCDR1 (IMGT) | GFTFSNAW |
| SEQ ID NO: 213 | HCDR2 (IMGT) | IKSKTDAGTT |
| SEQ ID NO: 214 | HCDR3 (IMGT) | ARTIYPSAPSSSLDY |
| SEQ ID NO: 215 | VH | EVQLVESGGGLVKPGGSLRLSCAASGFTFSNAWMSWVR QAPGKGLEWVGRIKSKTDAGTTDYAAPVKGRFTISRDD SKNTLYLQMNSLKTEDTAVYYCARTIYPSAPSSSLDYW GQGTLVTVSS |
| SEQ ID NO: 216 | DNA VH | GAAGTGCAGCTGGTGGAATCTGGCGGCGGACTTGTGAA ACCTGGCGGCTCTCTGAGACTGAGCTGTGCCGCTTCCG GCTTCACCTTCAGCAATGCCTGGATGAGCTGGGTCCGA CAGGCCCCTGGAAAAGGCCTTGAGTGGGTCGGACGGAT CAAGAGCAAGACCGATGCCGGCACCACCGATTATGCTG CCCCTGTGAAGGGCAGATTCACCATCAGCAGGGACGAC AGCAAGAACACCCTGTACCTGCAGATGAACAGCCTGAA AACCGAGGACACCGCCGTGTACTACTGCGCCAGAACAA TCTACCCCAGCGCTCCTAGCAGCAGCCTGGATTATTGG GGCCAGGGCACACTGGTCACCGTGTCATCT |
| SEQ ID NO: 217 | Heavy Chain | EVQLVESGGGLVKPGGSLRLSCAASGFTFSNAWMSWVR QAPGKGLEWVGRIKSKTDAGTTDYAAPVKGRFTISRDD SKNTLYLQMNSLKTEDTAVYYCARTIYPSAPSSSLDYW GQGTLVTVSSASTKGPSVFPLAPSSKSTSGGTAALGCL VKDYFPCPVTVSWNSGALTSGVHTFPAVLQSSGLYSLS SVVTVPSSSLGTQTYICNVNHKPSNTKVDKRVEPKSCD KTHTCPPCPAPELLGGPSVFLFPPKPKDTLMISRTPEV TCVVVDVSHEDPEVKFNWYVDGVEVHNAKTKPREEQYN STYRVVSVLTVLHQDWLNGKEYKCKVSNKALPAPIEKT ISKAKGQPREPQVYTLPPSREEMTKNQVSLTCLVKGFY PCDIAVEWESNGQPENNYKTTPPVLDSDGSFFLYSKLT VDKSRWQQGNVFSCSVMHEALHNHYTQKSLSLSPGK |
| SEQ ID NO: 218 | DNA Heavy Chain | GAAGTGCAGCTGGTGGAATCTGGCGGCGGACTTGTGAA ACCTGGCGGCTCTCTGAGACTGAGCTGTGCCGCTTCCG GCTTCACCTTCAGCAATGCCTGGATGAGCTGGGTCCGA CAGGCCCCTGGAAAAGGCCTTGAGTGGGTCGGACGGAT CAAGAGCAAGACCGATGCCGGCACCACCGATTATGCTG CCCCTGTGAAGGGCAGATTCACCATCAGCAGGGACGAC AGCAAGAACACCCTGTACCTGCAGATGAACAGCCTGAA AACCGAGGACACCGCCGTGTACTACTGCGCCAGAACAA TCTACCCCAGCGCTCCTAGCAGCAGCCTGGATTATTGG GGCCAGGGCACACTGGTCACCGTGTCATCTGCTAGCAC CAAGGGCCCAAGTGTGTTTCCCCTGGCCCCCAGCAGCA AGTCTACTTCCGGCGGAACTGCTGCCCTGGGTTGCCTG GTGAAGGACTACTTCCCCTGTCCCGTGACAGTGTCCTG GAACTCTGGGGCTCTGACTTCCGGCGTGCACACCTTCC CCGCCGTGCTGCAGAGCAGCGGCCTGTACAGCCTGAGC AGCGTGGTGACAGTGCCCTCCAGCTCTCTGGGAACCCA GACCTATATCTGCAACGTGAACCACAAGCCCAGCAACA CCAAGGTGGACAAGAGAGTGGAGCCCAAGAGCTGCGAC AAGACCCACACCTGCCCCCCCTGCCCAGCTCCAGAACT GCTGGGAGGGCCTTCCGTGTTCCTGTTCCCCCCCAAGC CCAAGGACACCCTGATGATCAGCAGGACCCCCGAGGTG ACCTGCGTGGTGGTGGACGTGTCCCACGAGGACCCAGA GGTGAAGTTCAACTGGTACGTGGACGGCGTGGAGGTGC |
| | | ACAACGCCAAGACCAAGCCCAGAGAGGAGCAGTACAAC AGCACCTACAGGGTGGTGTCCGTGCTGACCGTGCTGCA CCAGGACTGGCTGAACGGCAAAGAATACAAGTGCAAAG TCTCCAACAAGGCCCTGCCAGCCCCAATCGAAAAGACA ATCAGCAAGGCCAAGGGCCAGCCACGGGAGCCCCAGGT GTACACCCTGCCCCCCAGCCGGGAGGAGATGACCAAGA ACCAGGTGTCCCTGACCTGTCTGGTGAAGGGCTTCTAC CCCTGTGATATCGCCGTGGAGTGGGAGAGCAACGGCCA GCCCGAGAACAACTACAAGACCACCCCCCCAGTGCTGG ACAGCGACGGCAGCTTCTTCCTGTACAGCAAGCTGACC GTGGACAAGTCCAGGTGGCAGCAGGGCAACGTGTTCAG CTGCAGCGTGATGCACGAGGCCCTGCACAACCACTACA CCCAGAAGTCCCTGAGCCTGAGCCCCGGCAAG |
| SEQ ID NO: 153 | LCDR1 (Combined) | RASQGISNYLA |
| SEQ ID NO: 154 | LCDR2 (Combined) | AASTLQS |
| SEQ ID NO: 219 | LCDR3 (Combined) | QQLIFFPLT |
| SEQ ID NO: 153 | LCDR1 (Kabat) | RASQGISNYLA |
| SEQ ID NO: 154 | LCDR2 (Kabat) | AASTLQS |
| SEQ ID NO: 219 | LCDR3 (Kabat) | QQLIFFPLT |
| SEQ ID NO: 156 | LCDR1 (Chothia) | SQGISNY |
| SEQ ID NO: 50 | LCDR2 (Chothia) | AAS |
| SEQ ID NO: 220 | LCDR3 (Chothia) | LIFFPL |
| SEQ ID NO: 158 | LCDR1 (IMGT) | QGISNY |
| SEQ ID NO: 50 | LCDR2 (IMGT) | AAS |
| SEQ ID NO: 219 | LCDR3 (IMGT) | QQLIFFPLT |
| SEQ ID NO: 221 | VL | DIQMTQSPSSLSASVGDRVTITCRASQGISNYLAWYQQ KPGKVPKLLIYAASTLQSGVPSRFSGSGSGTDFTLTIS SLQPEDVATYYCQQLIFFPLTFGQGTKVEIK |
| SEQ ID NO: 222 | DNA VL | 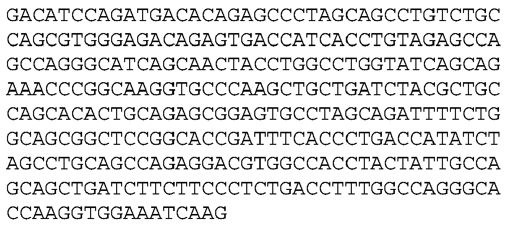 |
| SEQ ID NO: 223 | Light Chain | 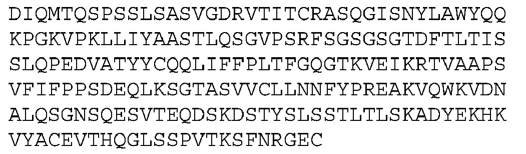 |
| SEQ ID NO: 224 | DNA Light Chain | GACATCCAGATGACACAGAGCCCTAGCAGCCTGTCTGC CAGCGTGGGAGACAGAGTGACCATCACCTGTAGAGCCA GCCAGGGCATCAGCAACTACCTGGCCTGGTATCAGCAG AAACCCGGCAAGGTGCCCAAGCTGCTGATCTACGCTGC CAGCACACTGCAGAGCGGAGTGCCTAGCAGATTTTCTG GCAGCGGCTCCGGCACCGATTTCACCCTGACCATATCT AGCCTGCAGCCAGAGGACGTGGCCACCTACTATTGCCA GCAGCTGATCTTCTTCCCTCTGACCTTTGGCCAGGGCA CCAAGGTGGAAATCAAGCGTACGGTGGCCGCTCCCAGC |
| | | GTGTTCATCTTCCCCCCAGCGACGAGCAGCTGAAGAG TGGCACCGCCAGCGTGGTGTGCCTGCTGAACAACTTCT ACCCCCGGGAGGCCAAGGTGCAGTGGAAGGTGGACAAC GCCCTGCAGAGCGGCAACAGCCAGGAGAGCGTCACCGA GCAGGACAGCAAGGACTCCACCTACAGCCTGAGCAGCA CCCTGACCCTGAGCAAGGCCGACTACGAGAAGCATAAG GTGTACGCCTGCGAGGTGACCCACCAGGGCCTGTCCAG CCCCGTGACCAAGAGCTTCAACAGGGGCGAGTGC |

| **Y8 E152C_S375C** | | |
|---|---|---|
| SEQ ID NO: 206 | HCDR1 (Combined) | GFTFSNAWMS |
| SEQ ID NO: 207 | HCDR2 (Combined) | RIKSKTDAGTTDYAAPVKG |
| SEQ ID NO: 225 | HCDR3 (Combined) | ASHRLHSLFDV |
| SEQ ID NO: 209 | HCDR1 (Kabat) | NAWMS |
| SEQ ID NO: 207 | HCDR2 (Kabat) | RIKSKTDAGTTDYAAPVKG |
| SEQ ID NO: 225 | HCDR3 (Kabat) | ASHRLHSLFDV |
| SEQ ID NO: 210 | HCDR1 (Chothia) | GFTFSNA |
| SEQ ID NO: 211 | HCDR2 (Chothia) | KSKTDAGT |
| SEQ ID NO: 225 | HCDR3 (Chothia) | ASHRLHSLFDV |
| SEQ ID NO: 212 | HCDR1 (IMGT) | GFTFSNAW |
| SEQ ID NO: 213 | HCDR2 (IMGT) | IKSKTDAGTT |
| SEQ ID NO: 226 | HCDR3 (IMGT) | ARASHRLHSLFDV |
| SEQ ID NO: 227 | VH | EVQLVESGGGLVKPGGSLRLSCAASGFTFSNAWMSWVR QAPGKGLEWVGRIKSKTDAGTTDYAAPVKGRFTISRDD SKNTLYLQMNSLKTEDTAVYYCARASHRLHSLFDVWGQ GTLVTVSS |
| SEQ ID NO: 228 | DNA VH | GAAGTGCAGCTGGTGGAATCTGGCGGCGGACTTGTGAA ACCTGGCGGCTCTCTGAGACTGAGCTGTGCCGCTTCCG GCTTCACCTTCAGCAATGCCTGGATGAGCTGGGTCCGA CAGGCCCCTGGAAAAGGCCTTGAGTGGGTCGGACGGAT CAAGAGCAAGACCGATGCCGGCACCACCGATTATGCTG CCCCTGTGAAGGGCAGATTCACCATCAGCAGGGACGAC AGCAAGAACACCCTGTACCTGCAGATGAACAGCCTGAA AACCGAGGACACCGCCGTGTACTACTGTGCCAGAGCCT CTCACAGACTGCACAGCCTGTTTGACGTGTGGGGCCAG GGAACACTGGTCACCGTTAGTTCT |
| SEQ ID NO: 229 | Heavy Chain | EVQLVESGGGLVKPGGSLRLSCAASGFTFSNAWMSWVR QAPGKGLEWVGRIKSKTDAGTTDYAAPVKGRFTISRDD SKNTLYLQMNSLKTEDTAVYYCARASHRLHSLFDVWGQ GTLVTVSSASTKGPSVFPLAPSSKSTSGGTAALGCLVK DYFPCPVTVSWNSGALTSGVHTFPAVLQSSGLYSLSSV VTVPSSSLGTQTYICNVNHKPSNTKVDKRVEPKSCDKT HTCPPCPAPELLGGPSVFLFPPKPKDTLMISRTPEVTC VVVDVSHEDPEVKFNWYVDGVEVHNAKTKPREEQYNST YRVVSVLTVLHQDWLNGKEYKCKVSNKALPAPIEKTIS KAKGQPREPQVYTLPPSREEMTKNQVSLTCLVKGFYPC DIAVEWESNGQPENNYKTTPPVLDSDGSFFLYSKLTVD KSRWQQGNVFSCSVMHEALHNHYTQKSLSLSPGK |
| SEQ ID NO: 230 | DNA Heavy Chain | GAAGTGCAGCTGGTGGAATCTGGCGGCGGACTTGTGAA ACCTGGCGGCTCTCTGAGACTGAGCTGTGCCGCTTCCG GCTTCACCTTCAGCAATGCCTGGATGAGCTGGGTCCGA CAGGCCCCTGGAAAAGGCCTTGAGTGGGTCGGACGGAT CAAGAGCAAGACCGATGCCGGCACCACCGATTATGCTG |
| | | CCCCTGTGAAGGGCAGATTCACCATCAGCAGGGACGAC AGCAAGAACACCCTGTACCTGCAGATGAACAGCCTGAA AACCGAGGACACCGCCGTGTACTACTGTGCCAGAGCCT CTCACAGACTGCACAGCCTGTTTGACGTGTGGGGCCAG GGAACACTGGTCACCGTTAGTTCTGCTAGCACCAAGGG CCCAAGTGTGTTTCCCCTGGCCCCCAGCAGCAAGTCTA CTTCCGGCGGAACTGCTGCCCTGGGTTGCCTGGTGAAG GACTACTTCCCCTGTCCCGTGACAGTGTCCTGGAACTC TGGGGCTCTGACTTCCGGCGTGCACACCTTCCCCGCCG TGCTGCAGAGCAGCGGCCTGTACAGCCTGAGCAGCGTG GTGACAGTGCCCTCCAGCTCTCTGGGAACCCAGACCTA TATCTGCAACGTGAACCACAAGCCCAGCAACACCAAGG TGGACAAGAGAGTGGAGCCCAAGAGCTGCGACAAGACC CACACCTGCCCCCCCTGCCCAGCTCCAGAACTGCTGGG AGGGCCTTCCGTGTTCCTGTTCCCCCCCAAGCCCAAGG ACACCCTGATGATCAGCAGGACCCCCGAGGTGACCTGC GTGGTGGTGGACGTGTCCCACGAGGACCCAGAGGTGAA GTTCAACTGGTACGTGGACGGCGTGGAGGTGCACAACG CCAAGACCAAGCCCAGAGAGGAGCAGTACAACAGCACC TACAGGGTGGTGTCCGTGCTGACCGTGCTGCACCAGGA CTGGCTGAACGGCAAAGAATACAAGTGCAAAGTCTCCA ACAAGGCCCTGCCAGCCCCAATCGAAAAGACAATCAGC AAGGCCAAGGGCCAGCCACGGGAGCCCCAGGTGTACAC CCTGCCCCCCAGCCGGGAGGAGATGACCAAGAACCAGG TGTCCCTGACCTGTCTGGTGAAGGGCTTCTACCCCTGT GATATCGCCGTGGAGTGGGAGAGCAACGGCCAGCCCGA GAACAACTACAAGACCACCCCCCCAGTGCTGGACAGCG ACGGCAGCTTCTTCCTGTACAGCAAGCTGACCGTGGAC AAGTCCAGGTGGCAGCAGGGCAACGTGTTCAGCTGCAG CGTGATGCACGAGGCCCTGCACAACCACTACACCCAGA AGTCCCTGAGCCTGAGCCCCGGCAAG |
| SEQ ID NO: 136 | LCDR1 (Combined) | RASQSISSWLA |
| SEQ ID NO: 137 | LCDR2 (Combined) | DASSLES |
| SEQ ID NO: 231 | LCDR3 (Combined) | QQGLFYPHT |
| SEQ ID NO: 136 | LCDR1 (Kabat) | RASQSISSWLA |
| SEQ ID NO: 137 | LCDR2 (Kabat) | DASSLES |
| SEQ ID NO: 231 | LCDR3 (Kabat) | QQGLFYPHT |
| SEQ ID NO: 139 | LCDR1 (Chothia) | SQSISSW |
| SEQ ID NO: 140 | LCDR2 (Chothia) | DAS |
| SEQ ID NO: 232 | LCDR3 (Chothia) | GLFYPH |
| SEQ ID NO: 142 | LCDR1 (IMGT) | QSISSW |
| SEQ ID NO: 140 | LCDR2 (IMGT) | DAS |
| SEQ ID NO: 231 | LCDR3 (IMGT) | QQGLFYPHT |
| SEQ ID NO: 233 | VL | 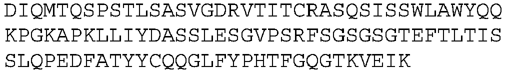 |
| SEQ ID NO: 234 | DNA VL | 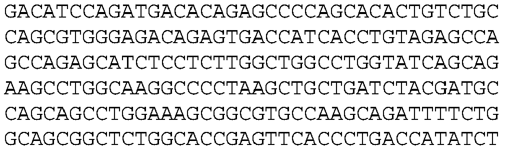 |
| | | 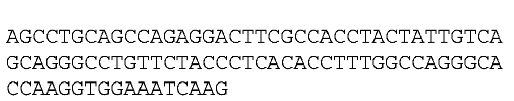 |
| SEQ ID NO: 235 | Light Chain | DIQMTQSPSTLSASVGDRVTITCRASQSISSWLAWYQQ KPGKAPKLLIYDASSLESGVPSRFSGSGSGTEFTLTIS SLQPEDFATYYCQQGLFYPHTFGQGTKVEIKRTVAAPS VFIFPPSDEQLKSGTASVVCLLNNFYPREAKVQWKVDN ALQSGNSQESVTEQDSKDSTYSLSSTLTLSKADYEKHK VYACEVTHQGLSSPVTKSFNRGEC |
| SEQ ID NO: 236 | DNA Light Chain | GACATCCAGATGACACAGAGCCCCAGCACACTGTCTGC CAGCGTGGGAGACAGAGTGACCATCACCTGTAGAGCCA GCCAGAGCATCTCCTCTTGGCTGGCCTGGTATCAGCAG AAGCCTGGCAAGGCCCCTAAGCTGCTGATCTACGATGC CAGCAGCCTGGAAAGCGGCGTGCCAAGCAGATTTTCTG GCAGCGGCTCTGGCACCGAGTTCACCCTGACCATATCT AGCCTGCAGCCAGAGGACTTCGCCACCTACTATTGTCA GCAGGGCCTGTTCTACCCTCACACCTTTGGCCAGGGCA CCAAGGTGGAAATCAAGCGTACGGTGGCCGCTCCCAGC GTGTTCATCTTCCCCCCCAGCGACGAGCAGCTGAAGAG TGGCACCGCCAGCGTGGTGTGCCTGCTGAACAACTTCT ACCCCCGGGAGGCCAAGGTGCAGTGGAAGGTGGACAAC GCCCTGCAGAGCGGCAACAGCCAGGAGAGCGTCACCGA GCAGGACAGCAAGGACTCCACCTACAGCCTGAGCAGCA CCCTGACCCTGAGCAAGGCCGACTACGAGAAGCATAAG GTGTACGCCTGCGAGGTGACCCACCAGGGCCTGTCCAG CCCCGTGACCAAGAGCTTCAACAGGGGCGAGTGC |

| **Y9 E152C_S375C** | | |
|---|---|---|
| SEQ ID NO: 206 | HCDR1 (Combined) | GFTFSNAWMS |
| SEQ ID NO: 207 | HCDR2 (Combined) | RIKSKTDAGTTDYAAPVKG |
| SEQ ID NO: 237 | HCDR3 (Combined) | DEYPWGWFDV |
| SEQ ID NO: 209 | HCDR1 (Kabat) | NAWMS |
| SEQ ID NO: 207 | HCDR2 (Kabat) | RIKSKTDAGTTDYAAPVKG |
| SEQ ID NO: 237 | HCDR3 (Kabat) | DEYPWGWFDV |
| SEQ ID NO: 210 | HCDR1 (Chothia) | GFTFSNA |
| SEQ ID NO: 211 | HCDR2 (Chothia) | KSKTDAGT |
| SEQ ID NO: 237 | HCDR3 (Chothia) | DEYPWGWFDV |
| SEQ ID NO: 212 | HCDR1 (IMGT) | GFTFSNAW |
| SEQ ID NO: 213 | HCDR2 (IMGT) | IKSKTDAGTT |
| SEQ ID NO: 238 | HCDR3 (IMGT) | ARDEYPWGWFDV |
| SEQ ID NO: 239 | VH | EVQLVESGGGLVKPGGSLRLSCAASGFTFSNAWMSWVR QAPGKGLEWVGRIKSKTDAGTTDYAAPVKGRFTISRDD SKNTLYLQMNSLKTEDTAVYYCARDEYPWGWFDVWGQG TLVTVSS |
| SEQ ID NO: 240 | DNA VH | GAAGTGCAGCTGGTGGAATCTGGCGGCGGACTTGTGAA ACCTGGCGGCTCTCTGAGACTGAGCTGTGCCGCTTCCG GCTTCACCTTCAGCAATGCCTGGATGAGCTGGGTCCGA CAGGCCCCTGGAAAAGGCCTTGAGTGGGTCGGACGGAT CAAGAGCAAGACCGATGCCGGCACCACCGATTATGCTG CCCCTGTGAAGGGCAGATTCACCATCAGCAGGGACGAC AGCAAGAACACCCTGTACCTGCAGATGAACAGCCTGAA AACCGAGGACACCGCCGTGTACTACTGCGCCAGAGATG |
| | | AGTACCCCTGGGGATGGTTCGATGTGTGGGGACAGGGA ACCCTGGTCACCGTTAGTTCT |
| SEQ ID NO: 241 | Heavy Chain | EVQLVESGGGLVKPGGSLRLSCAASGFTFSNAWMSWVR QAPGKGLEWVGRIKSKTDAGTTDYAAPVKGRFTISRDD SKNTLYLQMNSLKTEDTAVYYCARDEYPWGWFDVWGQG TLVTVSSASTKGPSVFPLAPSSKSTSGGTAALGCLVKD YFPCPVTVSWNSGALTSGVHTFPAVLQSSGLYSLSSVV TVPSSSLGTQTYICNVNHKPSNTKVDKRVEPKSCDKTH TCPPCPAPELLGGPSVFLFPPKPKDTLMISRTPEVTCV VVDVSHEDPEVKFNWYVDGVEVHNAKTKPREEQYNSTY RVVSVLTVLHQDWLNGKEYKCKVSNKALPAPIEKTISK AKGQPREPQVYTLPPSREEMTKNQVSLTCLVKGFYPCD IAVEWESNGQPENNYKTTPPVLDSDGSFFLYSKLTVDK SRWQQGNVFSCSVMHEALHNHYTQKSLSLSPGK |
| SEQ ID NO: 242 | DNA Heavy Chain | GAAGTGCAGCTGGTGGAATCTGGCGGCGGACTTGTGAA ACCTGGCGGCTCTCTGAGACTGAGCTGTGCCGCTTCCG GCTTCACCTTCAGCAATGCCTGGATGAGCTGGGTCCGA CAGGCCCCTGGAAAAGGCCTTGAGTGGGTCGGACGGAT CAAGAGCAAGACCGATGCCGGCACCACCGATTATGCTG CCCCTGTGAAGGGCAGATTCACCATCAGCAGGGACGAC AGCAAGAACACCCTGTACCTGCAGATGAACAGCCTGAA AACCGAGGACACCGCCGTGTACTACTGCGCCAGAGATG AGTACCCCTGGGATGGTTCGATGTGTGGGGACAGGGA ACCCTGGTCACCGTTAGTTCTGCTAGCACCAAGGGCCC AAGTGTGTTTCCCCTGGCCCCCAGCAGCAAGTCTACTT CCGGCGGAACTGCTGCCCTGGGTTGCCTGGTGAAGGAC TACTTCCCCTGTCCCGTGACAGTGTCCTGGAACTCTGG GGCTCTGACTTCCGGCGTGCACACCTTCCCCGCCGTGC TGCAGAGCAGCGGCCTGTACAGCCTGAGCAGCGTGGTG ACAGTGCCCTCCAGCTCTCTGGGAACCCAGACCTATAT CTGCAACGTGAACCACAAGCCCAGCAACACCAAGGTGG ACAAGAGAGTGGAGCCCAAGAGCTGCGACAAGACCCAC ACCTGCCCCCCCTGCCCAGCTCCAGAACTGCTGGGAGG GCCTTCCGTGTTCCTGTTCCCCCCCAAGCCCAAGGACA CCCTGATGATCAGCAGGACCCCCGAGGTGACCTGCGTG GTGGTGGACGTGTCCCACGAGGACCCAGAGGTGAAGTT CAACTGGTACGTGGACGGCGTGGAGGTGCACAACGCCA AGACCAAGCCCAGAGAGGAGCAGTACAACAGCACCTAC AGGGTGGTGTCCGTGCTGACCGTGCTGCACCAGGACTG GCTGAACGGCAAAGAATACAAGTGCAAAGTCTCCAACA AGGCCCTGCCAGCCCCAATCGAAAAGACAATCAGCAAG GCCAAGGGCCAGCCACGGGAGCCCCAGGTGTACACCCT GCCCCCCAGCCGGGAGGAGATGACCAAGAACCAGGTGT CCCTGACCTGTCTGGTGAAGGGCTTCTACCCCTGTGAT ATCGCCGTGGAGTGGGAGAGCAACGGCCAGCCCGAGAA CAACTACAAGACCACCCCCCCAGTGCTGGACAGCGACG GCAGCTTCTTCCTGTACAGCAAGCTGACCGTGGACAAG TCCAGGTGGCAGCAGGGCAACGTGTTCAGCTGCAGCGT GATGCACGAGGCCCTGCACAACCACTACACCCAGAAGT CCCTGAGCCTGAGCCCCGGCAAG |
| SEQ ID NO: 243 | LCDR1 (Combined) | RASQGISSWLA |
| SEQ ID NO: 47 | LCDR2 (Combined) | AASSLQS |
| SEQ ID NO: 244 | LCDR3 (Combined) | QQYIFYPLT |
| SEQ ID NO: 243 | LCDR1 (Kabat) | RASQGISSWLA |
| SEQ ID NO: 47 | LCDR2 (Kabat) | AASSLQS |
| SEQ ID NO: 244 | LCDR3 (Kabat) | QQYIFYPLT |
| SEQ ID NO: 245 | LCDR1 (Chothia) | SQGISSW |
| SEQ ID NO: 50 | LCDR2 (Chothia) | AAS |
| SEQ ID NO: 246 | LCDR3 (Chothia) | YIFYPL |
| SEQ ID NO: 247 | LCDR1 (IMGT) | QGISSW |
| SEQ ID NO: 50 | LCDR2 (IMGT) | AAS |
| SEQ ID NO: 244 | LCDR3 (IMGT) | QQYIFYPLT |
| SEQ ID NO: 248 | VL | DIQMTQSPSSVSASVGDRVTITCRASQGISSWLAWYQQ KPGKAPKLLIYAASSLQSGVPSRFSGSGSGTDFTLTIS SLQPEDFATYYCQQYIFYPLTFGQGTKVEIK |
| SEQ ID NO: 249 | DNA VL | GACATCCAGATGACACAGAGCCCTAGCTCCGTGTCTGC CAGCGTGGGAGACAGAGTGACCATCACCTGTAGAGCCA GCCAGGGCATCTCTTCTTGGCTGGCCTGGTATCAGCAG AAGCCTGGCAAGGCCCCTAAGCTGCTGATCTATGCCGC TTCCAGTCTGCAGAGCGGCGTGCCAAGCAGATTTTCTG GCAGCGGCTCTGGCACCGACTTCACCCTGACCATATCT AGCCTGCAGCCAGAGGACTTCGCCACCTACTACTGCCA GCAGTACATCTTCTACCCTCTGACCTTCGGCCAGGGCA CCAAGGTGGAAATCAAG |
| SEQ ID NO: 250 | Light Chain | DIQMTQSPSSVSASVGDRVTITCRASQGISSWLAWYQQ KPGKAPKLLIYAASSLQSGVPSRFSGSGSGTDFTLTIS SLQPEDFATYYCQQYIFYPLTFGQGTKVEIKRTVAAPS VFIFPPSDEQLKSGTASVVCLLNNFYPREAKVQWKVDN ALQSGNSQESVTEQDSKDSTYSLSSTLTLSKADYEKHK VYACEVTHQGLSSPVTKSFNRGEC |
| SEQ ID NO: 251 | DNA Light Chain | GACATCCAGATGACACAGAGCCCTAGCTCCGTGTCTGC CAGCGTGGGAGACAGAGTGACCATCACCTGTAGAGCCA GCCAGGGCATCTCTTCTTGGCTGGCCTGGTATCAGCAG AAGCCTGGCAAGGCCCCTAAGCTGCTGATCTATGCCGC TTCCAGTCTGCAGAGCGGCGTGCCAAGCAGATTTTCTG GCAGCGGCTCTGGCACCGACTTCACCCTGACCATATCT AGCCTGCAGCCAGAGGACTTCGCCACCTACTACTGCCA GCAGTACATCTTCTACCCTCTGACCTTCGGCCAGGGCA CCAAGGTGGAAATCAAGCGTACGGTGGCCGCTCCCAGC GTGTTCATCTTCCCCCCCAGCGACGAGCAGCTGAAGAG TGGCACCGCCAGCGTGGTGTGCCTGCTGAACAACTTCT ACCCCCGGGAGGCCAAGGTGCAGTGGAAGGTGGACAAC GCCCTGCAGAGCGGCAACAGCCAGGAGAGCGTCACCGA GCAGGACAGCAAGGACTCCACCTACAGCCTGAGCAGCA CCCTGACCCTGAGCAAGGCCGACTACGAGAAGCATAAG GTGTACGCCTGCGAGGTGACCCACCAGGGCCTGTCCAG CCCCGTGACCAAGAGCTTCAACAGGGGCGAGTGC |

| **Y10 E152C_S375C** | | |
|---|---|---|
| SEQ ID NO: 206 | HCDR1 (Combined) | GFTFSNAWMS |
| SEQ ID NO: 207 | HCDR2 (Combined) | RIKSKTDAGTTDYAAPVKG |
| SEQ ID NO: 252 | HCDR3 (Combined) | VASPSAPGGFDY |
| SEQ ID NO: 209 | HCDR1 (Kabat) | NAWMS |
| SEQ ID NO: 207 | HCDR2 (Kabat) | RIKSKTDAGTTDYAAPVKG |
| SEQ ID NO: 252 | HCDR3 (Kabat) | VASPSAPGGFDY |
| SEQ ID NO: 210 | HCDR1 (Chothia) | GFTFSNA |
| SEQ ID NO: 211 | HCDR2 (Chothia) | KSKTDAGT |
| SEQ ID NO: 252 | HCDR3(Chothia) | VASPSAPGGFDY |
| SEQ ID NO: 212 | HCDR1 (IMGT) | GFTFSNAW |
| SEQ ID NO: 213 | HCDR2 (IMGT) | IKSKTDAGTT |
| SEQ ID NO: 253 | HCDR3 (IMGT) | ARVASPSAPGGFDY |
| SEQ ID NO: 254 | VH | EVQLVESGGGLVKPGGSLRLSCAASGFTFSNAWMSWVR QAPGKGLEWVGRIKSKTDAGTTDYAAPVKGRFTISRDD SKNTLYLQMNSLKTEDTAVYYCARVASPSAPGGFDYWG QGTLVTVSS |
| SEQ ID NO: 255 | DNA VH | GAAGTGCAGCTGGTGGAATCTGGCGGCGGACTTGTGAA ACCTGGCGGCTCTCTGAGACTGAGCTGTGCCGCTTCCG GCTTCACCTTCAGCAATGCCTGGATGAGCTGGGTCCGA CAGGCCCCTGGAAAAGGCCTTGAGTGGGTCGGACGGAT CAAGAGCAAGACCGATGCCGGCACCACCGATTATGCTG CCCCTGTGAAGGGCAGATTCACCATCAGCAGGGACGAC AGCAAGAACACCCTGTACCTGCAGATGAACAGCCTGAA AACCGAGGACACCGCCGTGTACTACTGCGCCAGAGTGG CTTCTCCTTCTGCTCCCGGCGGATTCGATTATTGGGGC CAGGGAACACTGGTCACCGTGTCTAGT |
| SEQ ID NO: 256 | Heavy Chain | EVQLVESGGGLVKPGGSLRLSCAASGFTFSNAWMSWVR QAPGKGLEWVGRIKSKTDAGTTDYAAPVKGRFTISRDD SKNTLYLQMNSLKTEDTAVYYCARVASPSAPGGFDYWG QGTLVTVSSASTKGPSVFPLAPSSKSTSGGTAALGCLV KDYFPCPVTVSWNSGALTSGVHTFPAVLQSSGLYSLSS VVTVPSSSLGTQTYICNVNHKPSNTKVDKRVEPKSCDK THTCPPCPAPELLGGPSVFLFPPKPKDTLMISRTPEVT CVVVDVSHEDPEVKFNWYVDGVEVHNAKTKPREEQYNS TYRVVSVLTVLHQDWLNGKEYKCKVSNKALPAPIEKTI SKAKGQPREPQVYTLPPSREEMTKNQVSLTCLVKGFYP CDIAVEWESNGQPENNYKTTPPVLDSDGSFFLYSKLTV DKSRWQQGNVFSCSVMHEALHNHYTQKSLSLSPGK |
| SEQ ID NO: 257 | DNA Heavy Chain | GAAGTGCAGCTGGTGGAATCTGGCGGCGGACTTGTGAA ACCTGGCGGCTCTCTGAGACTGAGCTGTGCCGCTTCCG GCTTCACCTTCAGCAATGCCTGGATGAGCTGGGTCCGA CAGGCCCCTGGAAAAGGCCTTGAGTGGGTCGGACGGAT CAAGAGCAAGACCGATGCCGGCACCACCGATTATGCTG CCCCTGTGAAGGGCAGATTCACCATCAGCAGGGACGAC AGCAAGAACACCCTGTACCTGCAGATGAACAGCCTGAA AACCGAGGACACCGCCGTGTACTACTGCGCCAGAGTGG CTTCTCCTTCTGCTCCCGGCGGATTCGATTATTGGGGC CAGGGAACACTGGTCACCGTGTCTAGTGCTAGCACCAA GGGCCCAAGTGTGTTTCCCCTGGCCCCCAGCAGCAAGT CTACTTCCGGCGGAACTGCTGCCCTGGGTTGCCTGGTG AAGGACTACTTCCCCTGTCCCGTGACAGTGTCCTGGAA CTCTGGGGCTCTGACTTCCGGCGTGCACACCTTCCCCG CCGTGCTGCAGAGCAGCGGCCTGTACAGCCTGAGCAGC GTGGTGACAGTGCCCTCCAGCTCTCTGGGAACCCAGAC CTATATCTGCAACGTGAACCACAAGCCCAGCAACACCA AGGTGGACAAGAGAGTGGAGCCCAAGAGCTGCGACAAG ACCCACACCTGCCCCCCCTGCCCAGCTCCAGAACTGCT GGGAGGGCCTTCCGTGTTCCTGTTCCCCCCCAAGCCCA AGGACACCCTGATGATCAGCAGGACCCCCGAGGTGACC TGCGTGGTGGTGGACGTGTCCCACGAGGACCCAGAGGT GAAGTTCAACTGGTACGTGGACGGCGTGGAGGTGCACA |
| | | ACGCCAAGACCAAGCCCAGAGAGGAGCAGTACAACAGC ACCTACAGGGTGGTGTCCGTGCTGACCGTGCTGCACCA GGACTGGCTGAACGGCAAAGAATACAAGTGCAAAGTCT CCAACAAGGCCCTGCCAGCCCCAATCGAAAAGACAATC AGCAAGGCCAAGGGCCAGCCACGGGAGCCCCAGGTGTA CACCCTGCCCCCCAGCCGGGAGGAGATGACCAAGAACC AGGTGTCCCTGACCTGTCTGGTGAAGGGCTTCTACCCC TGTGATATCGCCGTGGAGTGGGAGAGCAACGGCCAGCC CGAGAACAACTACAAGACCACCCCCCCAGTGCTGGACA GCGACGGCAGCTTCTTCCTGTACAGCAAGCTGACCGTG GACAAGTCCAGGTGGCAGCAGGGCAACGTGTTCAGCTG CAGCGTGATGCACGAGGCCCTGCACAACCACTACACCC AGAAGTCCCTGAGCCTGAGCCCCGGCAAG |
| SEQ ID NO: 153 | LCDR1 (Combined) | RASQGISNYLA |
| SEQ ID NO: 154 | LCDR2 (Combined) | AASTLQS |
| SEQ ID NO: 258 | LCDR3 (Combined) | QQSLFAPFT |
| SEQ ID NO: 153 | LCDR1 (Kabat) | RASQGISNYLA |
| SEQ ID NO: 154 | LCDR2 (Kabat) | AASTLQS |
| SEQ ID NO: 258 | LCDR3 (Kabat) | QQSLFAPFT |
| SEQ ID NO: 156 | LCDR1 (Chothia) | SQGISNY |
| SEQ ID NO: 50 | LCDR2 (Chothia) | AAS |
| SEQ ID NO: 259 | LCDR3 (Chothia) | SLFAPF |
| SEQ ID NO: 158 | LCDR1 (IMGT) | QGISNY |
| SEQ ID NO: 50 | LCDR2 (IMGT) | AAS |
| SEQ ID NO: 258 | LCDR3 (IMGT) | QQSLFAPFT |
| SEQ ID NO: 260 | VL | DIQMTQSPSSLSASVGDRVTITCRASQGISNYLAWYQQ KPGKVPKLLIYAASTLQSGVPSRFSGSGSGTDFTLTIS SLOPEDVATYYCOOSLFAPFTFGOGTKVEIK |
| SEQ ID NO: 261 | DNA VL | GACATCCAGATGACACAGAGCCCTAGCAGCCTGTCTGC CAGCGTGGGAGACAGAGTGACCATCACCTGTAGAGCCA GCCAGGGCATCAGCAACTACCTGGCCTGGTATCAGCAG AAACCCGGCAAGGTGCCCAAGCTGCTGATCTACGCTGC CAGCACACTGCAGAGCGGAGTGCCTAGCAGATTTTCTG GCAGCGGCTCCGGCACCGATTTCACCCTGACCATATCT AGCCTGCAGCCAGAGGACGTGGCCACCTACTACTGTCA GCAGAGCCTGTTCGCCCCTTTCACCTTTGGCCAGGGCA CCAAGGTGGAAATCAAG |
| SEQ ID NO: 262 | Light Chain | DIQMTQSPSSLSASVGDRVTITCRASQGISNYLAWYQQ KPGKVPKLLIYAASTLQSGVPSRFSGSGSGTDFTLTIS SLQPEDVATYYCQQSLFAPFTFGQGTKVEIKRTVAAPS VFIFPPSDEQLKSGTASVVCLLNNFYPREAKVQWKVDN ALQSGNSQESVTEQDSKDSTYSLSSTLTLSKADYEKHK |
| SEQ ID NO: 263 | DNA Light Chain | VYACEVTHQGLSSPVTKSFNRGEC GACATCCAGATGACACAGAGCCCTAGCAGCCTGTCTGC CAGCGTGGGAGACAGAGTGACCATCACCTGTAGAGCCA GCCAGGGCATCAGCAACTACCTGGCCTGGTATCAGCAG AAACCCGGCAAGGTGCCCAAGCTGCTGATCTACGCTGC CAGCACACTGCAGAGCGGAGTGCCTAGCAGATTTTCTG GCAGCGGCTCCGGCACCGATTTCACCCTGACCATATCT AGCCTGCAGCCAGAGGACGTGGCCACCTACTACTGTCA GCAGAGCCTGTTCGCCCCTTTCACCTTTGGCCAGGGCA CCAAGGTGGAAATCAAGCGTACGGTGGCCGCTCCCAGC |
| | | GTGTTCATCTTCCCCCCCAGCGACGAGCAGCTGAAGAG TGGCACCGCCAGCGTGGTGTGCCTGCTGAACAACTTCT ACCCCCGGGAGGCCAAGGTGCAGTGGAAGGTGGACAAC GCCCTGCAGAGCGGCAACAGCCAGGAGAGCGTCACCGA GCAGGACAGCAAGGACTCCACCTACAGCCTGAGCAGCA CCCTGACCCTGAGCAAGGCCGACTACGAGAAGCATAAG GTGTACGCCTGCGAGGTGACCCACCAGGGCCTGTCCAG CCCCGTGACCAAGAGCTTCAACAGGGGCGAGTGC |

| **G1 E152C_3J LC** | | |
|---|---|---|
| SEQ ID NO: 1 | HCDR1 (Combined) | GGTFSDYAIT |
| SEQ ID NO: 2 | HCDR2 (Combined) | GIIPIFGTANYAQKFQG |
| SEQ ID NO: 3 | HCDR3 (Combined) | EGGLLTDISYSRYWFAY |
| SEQ ID NO: 4 | HCDR1 (Kabat) | DYAIT |
| SEQ ID NO: 2 | HCDR2 (Kabat) | GIIPIFGTANYAQKFQG |
| SEQ ID NO: 3 | HCDR3 (Kabat) | EGGLLTDISYSRYWFAY |
| SEQ ID NO: 5 | HCDR1 (Chothia) | GGTFSDY |
| SEQ ID NO: 6 | HCDR2 (Chothia) | IPIFGT |
| SEQ ID NO: 3 | HCDR3 (Chothia) | EGGLLTDISYSRYWFAY |
| SEQ ID NO: 7 | HCDR1 (IMGT) | GGTFSDYA |
| SEQ ID NO: 8 | HCDR2 (IMGT) | IIPIFGTA |
| SEQ ID NO: 9 | HCDR3 (IMGT) | AREGGLLTDISYSRYWFAY |
| SEQ ID NO: 10 | VH | QVQLVQSGAEVKKPGSSVKVSCKASGGTFSDYAITWVR QAPGQGLEWMGGIIPIFGTANYAQKFQGRVTITADEST STAYMELSSLRSEDTAVYYCAREGGLLTDISYSRYWFA YWGQGTLVTVSS |
| SEQ ID NO: 11 | DNA VH | CAGGTGCAATTGGTGCAGAGCGGTGCCGAAGTGAAAAA ACCGGGCAGCAGCGTGAAAGTTAGCTGCAAAGCATCCG GAGGGACGTTTTCTGACTACGCTATCACTTGGGTGCGC CAGGCCCCGGGCCAGGGCCTCGAGTGGATGGGCGGTAT CATCCCGATCTTCGGCACTGCGAACTACGCCCAGAAAT TTCAGGGCCGGGTGACCATTACCGCCGATGAAAGCACC AGCACCGCCTATATGGAACTGAGCAGCCTGCGCAGCGA AGATACGGCCGTGTATTATTGCGCGCGTGAAGGTGGTC TGCTGACTGACATCTCTTACTCTCGTTACTGGTTCGCT TACTGGGGCCAAGGCACCCTGGTGACTGTTAGCTCA |
| SEQ ID NO: 283 | Heavy Chain. Glycosylation site is bolded and italicized. | QVQLVQSGAEVKKPGSSVKVSCKASGGTFSDYAITWVR QAPGQGLEWMGGIIPIFGTANYAQKFQGRVTITADEST STAYMELSSLRSEDTAVYYCAREGGLLTDISYSRYWFA YWGQGTLVTVSSASTKGPSVFPLAPSSKSTSGGTAALG CLVKDYFPCPVTVSWNSGALTSGVHTFPAVLQSSGLYS LSSVVTVPSSSLGTQTYICNVNHKPSNTKVDKRVEPKS CDKTHTCPPCPAPELLGGPSVFLFPPKPKDTLMISRTP EVTCVVVDVSHEDPEVKFNWYVDGVEVHNAKTKPREEQ YNSTYRVVSVLTVLHQDWLNGKEYKCKVSNKALPAPIE KTISKAKGQPREPQVYTLPPSREEMTKNQVSLTCLVKG FYPSDIAVEWESNGQPENNYKTTPPVLDSDGSFFLYSK LTVDKSRWQQGNVFSCSVMHEALHNHYTQKSLSLSPGK |
| SEQ ID NO: 319 | DNA Heavy Chain | CAGGTGCAGCTGGTGCAGTCCGGCGCCGAGGTGAAAAA ACCCGGCTCCTCCGTGAAAGTGTCCTGCAAAGCCTCCG GCGGCACATTCTCCGACTACGCCATCACATGGGTGAGG CAGGCCCCCGGCCAGGGCCTGGAGTGGATGGGCGGCAT CATCCCCATCTTCGGCACAGCCAACTACGCCCAGAAAT |
| | | TCCAGGGCAGGGTGACAATCACAGCCGACGAGTCCACA TCCACAGCCTACATGGAGCTGTCCTCCCTGAGGTCCGA GGACACAGCCGTGTACTACTGCGCCAGGGAGGGCGGCC TGCTGACAGACATCTCCTACTCCAGGTACTGGTTCGCC TACTGGGGCCAGGGCACACTGGTGACAGTGTCCTCCGC CTCCACAAAGGGCCCCTCCGTGTTCCCCCTGGCCCCCT CCTCCAAATCCACATCCGGCGGCACAGCCGCCCTGGGC TGCCTGGTGAAAGACTACTTCCCCTGCCCCGTGACAGT GTCCTGGAACTCCGGCGCCCTGACATCCGGCGTGCACA CATTCCCCGCCGTGCTGCAGTCCTCCGGCCTGTACTCC CTGTCCTCCGTGGTGACAGTGCCCTCCTCCTCCCTGGG CACACAGACATACATCTGCAACGTGAACCACAAACCCT CCAACACAAAAGTGGACAAAAGGGTGGAGCCCAAATCC TGCGACAAAACACACACATGCCCCCCCTGCCCCGCCCC CGAGCTGCTGGGCGGCCCCTCCGTGTTCCTGTTCCCCC CCAAACCCAAAGACACACTGATGATCTCCAGGACACCC GAGGTGACATGCGTGGTGGTGGACGTGTCCCACGAGGA CCCCGAGGTGAAATTCAACTGGTACGTGGACGGCGTGG AGGTGCACAACGCCAAAACAAAACCCAGGGAGGAGCAG TACAACTCCACATACAGGGTGGTGTCCGTGCTGACAGT GCTGCACCAGGACTGGCTGAACGGCAAAGAGTACAAAT GCAAAGTGTCCAACAAAGCCCTGCCCGCCCCCATCGAG AAAACAATCTCCAAAGCCAAAGGCCAGCCCAGGGAGCC CCAGGTGTACACACTGCCCCCCTCCAGGGAGGAGATGA CAAAAAACCAGGTGTCCCTGACATGCCTGGTGAAAGGC TTCTACCCCTCCGACATCGCCGTGGAGTGGGAGTCCAA CGGCCAGCCCGAGAACAACTACAAAACAACACCCCCCG TGCTGGACTCCGACGGCTCCTTCTTCCTGTACTCCAAA CTGACAGTGGACAAATCCAGGTGGCAGCAGGGCAACGT GTTCTCCTGCTCCGTGATGCACGAGGCCCTGCACAACC ACTACACACAGAAATCCCTGTCCCTGTCCCCCGGCAAA |
| SEQ ID NO: 14 | LCDR1 (Combined) | SGDALRDKFVY |
| SEQ ID NO: 15 | LCDR2 (Combined) | DDNNRPS |
| SEQ ID NO: 16 | LCDR3 (Combined) | QSWDHSYSLW |
| SEQ ID NO: 14 | LCDR1 (Kabat) | SGDALRDKFVY |
| SEQ ID NO: 15 | LCDR2 (Kabat) | DDNNRPS |
| SEQ ID NO: 16 | LCDR3 (Kabat) | QSWDHSYSLW |
| SEQ ID NO: 17 | LCDR1 (Chothia) | DALRDKF |
| SEQ ID NO: 18 | LCDR2 (Chothia) | DDN |
| SEQ ID NO: 19 | LCDR3 (Chothia) | WDHSYSLV |
| SEQ ID NO: 20 | LCDR1 (IMGT) | ALRDKF |
| SEQ ID NO: 18 | LCDR2 (IMGT) | DDN |
| SEQ ID NO: 16 | LCDR3 (IMGT) | QSWDHSYSLVV |
| SEQ ID NO: 25 | VL | SYELTQPLSVSVALGQTARITCSGDALRDKFVYWYQQK PGQAPVLVIYDDNNRPSGIPERFSGSNSGNTATLTISR AQAGDEADYYCQSWDHSYSLVVFGGGTKLTVL |
| SEQ ID NO: 26 | DNA VL | AGCTACGAGCTGACCCAGCCGCTGTCGGTGTCAGTCGC CCTGGGACAAACTGCCAGGATCACTTGTTCCGGGGACG CATTGCGGGACAAGTTCGTGTACTGGTACCAGCAGAAG CCGGGTCAAGCCCCAGTGCTCGTGATCTACGACGACAA CAACCGGCCTTCCGGTATCCCCGAACGCTTCTCCGGAT CCAATAGCGGAAACACCGCCACCCTGACCATTTCGAGA |
| | | GCTCAGGCCGGGGATGAAGCGGACTACTACTGCCAGTC ATGGGATCACTCGTACTCCCTCGTCGTGTTTGGAGGCG GCACGAAGCTTACTGTGCTG |
| SEQ ID NO: 27 | Light Chain | SYELTQPLSVSVALGQTARITCSGDALRDKFVYWYQQK PGQAPVLVIYDDNNRPSGIPERFSGSNSGNTATLTISR AQAGDEADYYCQSWDHSYSLVVFGGGTKLTVLGQPKAA PSVTLFPPSSEELQANKATLVCLISDFYPGAVTVAWKA DSSPVKAGVETTTPSKQSNNKYAASSYLSLTPEQWKSH RSYSCQVTHEGSTVEKTVAPTECS |
| SEQ ID NO: 28 | DNA Light Chain | AGCTACGAGCTGACCCAGCCGCTGTCGGTGTCAGTCGC CCTGGGACAAACTGCCAGGATCACTTGTTCCGGGGACG CATTGCGGGACAAGTTCGTGTACTGGTACCAGCAGAAG CCGGGTCAAGCCCCAGTGCTCGTGATCTACGACGACAA CAACCGGCCTTCCGGTATCCCCGAACGCTTCTCCGGAT CCAATAGCGGAAACACCGCCACCCTGACCATTTCGAGA GCTCAGGCCGGGGATGAAGCGGACTACTACTGCCAGTC ATGGGATCACTCGTACTCCCTCGTCGTGTTTGGAGGCG GCACGAAGCTTACTGTGCTGGGCCAGCCTAAGGCCGCT CCCTCCGTGACCCTGTTCCCCCCCAGCTCCGAGGAACT GCAGGCCAACAAGGCCACCCTGGTGTGCCTGATCAGCG ACTTCTACCCTGGCGCCGTGACCGTGGCCTGGAAGGCC GACAGCAGCCCCGTGAAGGCCGGCGTGGAGACAACCAC CCCCAGCAAGCAGAGCAACAACAAGTACGCCGCCAGCA GCTACCTGAGCCTGACCCCCGAGCAGTGGAAGAGCCAC AGAAGCTACAGCTGCCAGGTCACCCACGAGGGCAGCAC CGTGGAGAAAACCGTGGCCCCCACCGAGTGCAGC |

| **G1 E152C_3R LC** | | |
|---|---|---|
| SEQ ID NO: 1 | HCDR1 (Combined) | GGTFSDYAIT |
| SEQ ID NO: 2 | HCDR2 (Combined) | GIIPIFGTANYAQKFQG |
| SEQ ID NO: 3 | HCDR3 (Combined) | EGGLLTDISYSRYWFAY |
| SEQ ID NO: 4 | HCDR1 (Kabat) | DYAIT |
| SEQ ID NO: 2 | HCDR2 (Kabat) | GIIPIFGTANYAQKFQG |
| SEQ ID NO: 3 | HCDR3 (Kabat) | EGGLLTDISYSRYWFAY |
| SEQ ID NO: 5 | HCDR1 (Chothia) | GGTFSDY |
| SEQ ID NO: 6 | HCDR2 (Chothia) | IPIFGT |
| SEQ ID NO: 3 | HCDR3 (Chothia) | EGGLLTDISYSRYWFAY |
| SEQ ID NO: 7 | HCDR1 (IMGT) | GGTFSDYA |
| SEQ ID NO: 8 | HCDR2 (IMGT) | IIPIFGTA |
| SEQ ID NO: 9 | HCDR3 (IMGT) | AREGGLLTDISYSRYWFAY |
| SEQ ID NO: 10 | VH | QVQLVQSGAEVKKPGSSVKVSCKASGGTFSDYAITWVR QAPGQGLEWMGGIIPIFGTANYAQKFQGRVTITADEST STAYMELSSLRSEDTAVYYCAREGGLLTDISYSRYWFA YWGQGTLVTVSS |
| SEQ ID NO: 11 | DNA VH | CAGGTGCAATTGGTGCAGAGCGGTGCCGAAGTGAAAAA ACCGGGCAGCAGCGTGAAAGTTAGCTGCAAAGCATCCG GAGGGACGTTTTCTGACTACGCTATCACTTGGGTGCGC CAGGCCCCGGGCCAGGGCCTCGAGTGGATGGGCGGTAT CATCCCGATCTTCGGCACTGCGAACTACGCCCAGAAAT TTCAGGGCCGGGTGACCATTACCGCCGATGAAAGCACC AGCACCGCCTATATGGAACTGAGCAGCCTGCGCAGCGA AGATACGGCCGTGTATTATTGCGCGCGTGAAGGTGGTC |
| | | TGCTGACTGACATCTCTTACTCTCGTTACTGGTTCGCT TACTGGGGCCAAGGCACCCTGGTGACTGTTAGCTCA |
| SEQ ID NO: 283 | Heavy Chain. Glycosylation site is bolded and italicized. | QVQLVQSGAEVKKPGSSVKVSCKASGGTFSDYAITWVR QAPGQGLEWMGGIIPIFGTANYAQKFQGRVTITADEST STAYMELSSLRSEDTAVYYCAREGGLLTDISYSRYWFA YWGQGTLVTVSSASTKGPSVFPLAPSSKSTSGGTAALG CLVKDYFPCPVTVSWNSGALTSGVHTFPAVLQSSGLYS LSSVVTVPSSSLGTQTYICNVNHKPSNTKVDKRVEPKS CDKTHTCPPCPAPELLGGPSVFLFPPKPKDTLMISRTP EVTCVVVDVSHEDPEVKFNWYVDGVEVHNAKTKPREEQ YNSTYRVVSVLTVLHQDWLNGKEYKCKVSNKALPAPIE KTISKAKGQPREPQVYTLPPSREEMTKNQVSLTCLVKG FYPSDIAVEWESNGQPENNYKTTPPVLDSDGSFFLYSK LTVDKSRWQQGNVFSCSVMHEALHNHYTQKSLSLSPGK |
| SEQ ID NO: 319 | DNA Heavy Chain | CAGGTGCAGCTGGTGCAGTCCGGCGCCGAGGTGAAAAA ACCCGGCTCCTCCGTGAAAGTGTCCTGCAAAGCCTCCG GCGGCACATTCTCCGACTACGCCATCACATGGGTGAGG CAGGCCCCCGGCCAGGGCCTGGAGTGGATGGGCGGCAT CATCCCCATCTTCGGCACAGCCAACTACGCCCAGAAAT TCCAGGGCAGGGTGACAATCACAGCCGACGAGTCCACA TCCACAGCCTACATGGAGCTGTCCTCCCTGAGGTCCGA GGACACAGCCGTGTACTACTGCGCCAGGGAGGGCGGCC TGCTGACAGACATCTCCTACTCCAGGTACTGGTTCGCC TACTGGGGCCAGGGCACACTGGTGACAGTGTCCTCCGC CTCCACAAAGGGCCCCTCCGTGTTCCCCCTGGCCCCCT CCTCCAAATCCACATCCGGCGGCACAGCCGCCCTGGGC TGCCTGGTGAAAGACTACTTCCCCTGCCCCGTGACAGT GTCCTGGAACTCCGGCGCCCTGACATCCGGCGTGCACA CATTCCCCGCCGTGCTGCAGTCCTCCGGCCTGTACTCC CTGTCCTCCGTGGTGACAGTGCCCTCCTCCTCCCTGGG CACACAGACATACATCTGCAACGTGAACCACAAACCCT CCAACACAAAAGTGGACAAAAGGGTGGAGCCCAAATCC TGCGACAAAACACACACATGCCCCCCCTGCCCCGCCCC CGAGCTGCTGGGCGGCCCCTCCGTGTTCCTGTTCCCCC CCAAACCCAAAGACACACTGATGATCTCCAGGACACCC GAGGTGACATGCGTGGTGGTGGACGTGTCCCACGAGGA CCCCGAGGTGAAATTCAACTGGTACGTGGACGGCGTGG AGGTGCACAACGCCAAAACAAAACCCAGGGAGGAGCAG TACAACTCCACATACAGGGTGGTGTCCGTGCTGACAGT GCTGCACCAGGACTGGCTGAACGGCAAAGAGTACAAAT GCAAAGTGTCCAACAAAGCCCTGCCCGCCCCCATCGAG AAAACAATCTCCAAAGCCAAAGGCCAGCCCAGGGAGCC CCAGGTGTACACACTGCCCCCCTCCAGGGAGGAGATGA CAAAAAACCAGGTGTCCCTGACATGCCTGGTGAAAGGC TTCTACCCCTCCGACATCGCCGTGGAGTGGGAGTCCAA CGGCCAGCCCGAGAACAACTACAAAACAACACCCCCCG TGCTGGACTCCGACGGCTCCTTCTTCCTGTACTCCAAA CTGACAGTGGACAAATCCAGGTGGCAGCAGGGCAACGT GTTCTCCTGCTCCGTGATGCACGAGGCCCTGCACAACC ACTACACACAGAAATCCCTGTCCCTGTCCCCCGGCAAA |
| SEQ ID NO: 14 | LCDR1 (Combined) | SGDALRDKFVY |
| SEQ ID NO: 15 | LCDR2 (Combined) | DDNNRPS |
| SEQ ID NO: 16 | LCDR3 (Combined) | QSWDHSYSLW |
| SEQ ID NO: 14 | LCDR1 (Kabat) | SGDALRDKFVY |
| SEQ ID NO: 15 | LCDR2 (Kabat) | DDNNRPS |
| SEQ ID NO: 16 | LCDR3 (Kabat) | QSWDHSYSLW |
| SEQ ID NO: 17 | LCDR1 (Chothia) | DALRDKF |
| SEQ ID NO: 18 | LCDR2 (Chothia) | DDN |
| SEQ ID NO: 19 | LCDR3 (Chothia) | WDHSYSLV |
| SEQ ID NO: 20 | LCDR1 (IMGT) | ALRDKF |
| SEQ ID NO: 18 | LCDR2 (IMGT) | DDN |
| SEQ ID NO: 16 | LCDR3 (IMGT) | QSWDHSYSLW |
| SEQ ID NO: 29 | VL | SYELTQPPSVSVSPGQTASITCSGDALRDKFVYWYQQK PGQSPVLVIYDDNNRPSGIPERFSGSNSGNTATLTISG TQAMDEADYYCQSWDHSYSLVVFGGGTKLTVL |
| SEQ ID NO: 30 | DNA VL | TCGTACGAGCTCACTCAACCGCCTTCTGTGTCCGTGTC ACCCGGGCAGACTGCCTCCATTACCTGTTCGGGAGATG CCCTGCGCGACAAGTTTGTGTACTGGTACCAGCAGAAG CCCGGACAGTCGCCAGTGCTCGTCATCTATGACGACAA CAACAGACCTTCCGGTATCCCGGAACGGTTCAGCGGAA GCAATTCCGGCAACACCGCTACCCTGACCATTAGCGGC ACTCAGGCCATGGACGAAGCGGATTACTACTGCCAATC CTGGGACCACTCATACTCCCTTGTGGTGTTCGGTGGCG GAACGAAGCTGACCGTCCTG |
| SEQ ID NO: 31 | Light Chain | SYELTQPPSVSVSPGQTASITCSGDALRDKFVYWYQQK PGQSPVLVIYDDNNRPSGIPERFSGSNSGNTATLTISG TQAMDEADYYCQSWDHSYSLVVFGGGTKLTVLGQPKAA PSVTLFPPSSEELQANKATLVCLISDFYPGAVTVAWKA DSSPVKAGVETTTPSKQSNNKYAASSYLSLTPEQWKSH RSYSCQVTHEGSTVEKTVAPTECS |
| SEQ ID NO: 32 | DNA Light Chain | TCGTACGAGCTCACTCAACCGCCTTCTGTGTCCGTGTC ACCCGGGCAGACTGCCTCCATTACCTGTTCGGAGATG CCCTGCGCGACAAGTTTGTGTACTGGTACCAGCAGAAG CCCGGACAGTCGCCAGTGCTCGTCATCTATGACGACAA CAACAGACCTTCCGGTATCCCGGAACGGTTCAGCGGAA GCAATTCCGGCAACACCGCTACCCTGACCATTAGCGGC ACTCAGGCCATGGACGAAGCGGATTACTACTGCCAATC CTGGGACCACTCATACTCCCTTGTGGTGTTCGGTGGCG GAACGAAGCTGACCGTCCTGGGCCAGCCTAAGGCCGCT CCCTCCGTGACCCTGTTCCCCCCCAGCTCCGAGGAACT GCAGGCCAACAAGGCCACCCTGGTGTGCCTGATCAGCG ACTTCTACCCTGGCGCCGTGACCGTGGCCTGGAAGGCC GACAGCAGCCCCGTGAAGGCCGGCGTGGAGACAACCAC CCCCAGCAAGCAGAGCAACAACAAGTACGCCGCCAGCA GCTACCTGAGCCTGACCCCCGAGCAGTGGAAGAGCCAC AGAAGCTACAGCTGCCAGGTCACCCACGAGGGCAGCAC CGTGGAGAAAACCGTGGCCCCCACCGAGTGCAGC |

| **M2 E152C** | | |
|---|---|---|
| SEQ ID NO: 79 | HCDR1 (Combined) | GFTFSSFGMS |
| SEQ ID NO: 80 | HCDR2 (Combined) | AISYSGSDTYYADSVKG |
| SEQ ID NO: 81 | HCDR3 (Combined) | DVGVMDY |
| SEQ ID NO: 82 | HCDR1 (Kabat) | SFGMS |
| SEQ ID NO: 80 | HCDR2 (Kabat) | AISYSGSDTYYADSVKG |
| SEQ ID NO: 81 | HCDR3 (Kabat) | DVGVMDY |
| SEQ ID NO: 83 | HCDR1 (Chothia) | GFTFSSF |
| SEQ ID NO: 84 | HCDR2 (Chothia) | SYSGSD |
| SEQ ID NO: 81 | HCDR3(Chothia) | DVGVMDY |
| SEQ ID NO: 85 | HCDR1 (IMGT) | GFTFSSFG |
| SEQ ID NO: 86 | HCDR2 (IMGT) | ISYSGSDT |
| SEQ ID NO: 87 | HCDR3 (IMGT) | ARDVGVMDY |
| SEQ ID NO: 88 | VH | QVQLLESGGGLVQPGGSLRLSCAASGFTFSSFGMSWVR QAPGKGLEWVSAISYSGSDTYYADSVKGRFTISRDNSK NTLYLQMNSLRAEDTAVYYCARDVGVMDYWGQGTLVTV SS |
| SEQ ID NO: 89 | DNA VH | CAGGTTCAGCTGCTGGAATCTGGCGGAGGACTGGTTCA ACCTGGCGGCTCTCTGAGACTGTCTTGTGCCGCCAGCG GCTTCACCTTCAGCAGCTTTGGCATGAGCTGGGTCCGA CAGGCCCCTGGCAAAGGACTTGAATGGGTGTCCGCCAT CAGCTACAGCGGCAGCGATACCTACTACGCCGACAGCG TGAAGGGCAGATTCACCATCTCCAGAGACAACAGCAAG AACACCCTGTACCTGCAGATGAACAGCCTGAGAGCCGA GGACACCGCCGTGTACTACTGTGCCAGAGATGTGGGCG TGATGGACTATTGGGGCCAGGGCACACTGGTCACCGTT AGCTCT |
| SEQ ID NO: 315 | Heavy Chain | QVQLLESGGGLVQPGGSLRLSCAASGFTFSSFGMSWVR QAPGKGLEWVSAISYSGSDTYYADSVKGRFTISRDNSK NTLYLQMNSLRAEDTAVYYCARDVGVMDYWGQGTLVTV SSASTKGPSVFPLAPSSKSTSGGTAALGCLVKDYFPCP VTVSWNSGALTSGVHTFPAVLQSSGLYSLSSVVTVPSS SLGTQTYICNVNHKPSNTKVDKRVEPKSCDKTHTCPPC PAPELLGGPSVFLFPPKPKDTLMISRTPEVTCVVVDVS HEDPEVKFNWYVDGVEVHNAKTKPREEQYNSTYRVVSV LTVLHQDWLNGKEYKCKVSNKALPAPIEKTISKAKGQP REPQVYTLPPSREEMTKNQVSLTCLVKGFYPSDIAVEW ESNGQPENNYKTTPPVLDSDGSFFLYSKLTVDKSRWQQ GNVFSCSVMHEALHNHYTQKSLSLSPGK |
| SEQ ID NO: 320 | DNA Heavy Chain | CAGGTTCAGCTGCTGGAATCTGGCGGAGGACTGGTTCA ACCTGGCGGCTCTCTGAGACTGTCTTGTGCCGCCAGCG GCTTCACCTTCAGCAGCTTTGGCATGAGCTGGGTCCGA CAGGCCCCTGGCAAAGGACTTGAATGGGTGTCCGCCAT CAGCTACAGCGGCAGCGATACCTACTACGCCGACAGCG TGAAGGGCAGATTCACCATCTCCAGAGACAACAGCAAG AACACCCTGTACCTGCAGATGAACAGCCTGAGAGCCGA GGACACCGCCGTGTACTACTGTGCCAGAGATGTGGGCG TGATGGACTATTGGGGCCAGGGCACACTGGTCACCGTT AGCTCTGCTAGCACCAAGGGCCCAAGTGTGTTTCCCCT GGCCCCCAGCAGCAAGTCTACTTCCGGCGGAACTGCTG CCCTGGGTTGCCTGGTGAAGGACTACTTCCCCTGTCCC GTGACAGTGTCCTGGAACTCTGGGGCTCTGACTTCCGG CGTGCACACCTTCCCCGCCGTGCTGCAGAGCAGCGGCC TGTACAGCCTGAGCAGCGTGGTGACAGTGCCCTCCAGC TCTCTGGGAACCCAGACCTATATCTGCAACGTGAACCA CAAGCCCAGCAACACCAAGGTGGACAAGAGAGTGGAGC CCAAGAGCTGCGACAAGACCCACACCTGCCCCCCCTGC CCAGCTCCAGAACTGCTGGGAGGGCCTTCCGTGTTCCT GTTCCCCCCCAAGCCCAAGGACACCCTGATGATCAGCA GGACCCCCGAGGTGACCTGCGTGGTGGTGGACGTGTCC CACGAGGACCCAGAGGTGAAGTTCAACTGGTACGTGGA CGGCGTGGAGGTGCACAACGCCAAGACCAAGCCCAGAG |
| | | AGGAGCAGTACAACAGCACCTACAGGGTGGTGTCCGTG CTGACCGTGCTGCACCAGGACTGGCTGAACGGCAAAGA ATACAAGTGCAAAGTCTCCAACAAGGCCCTGCCAGCCC CAATCGAAAAGACAATCAGCAAGGCCAAGGGCCAGCCA CGGGAGCCCCAGGTGTACACCCTGCCCCCCAGCCGGGA GGAGATGACCAAGAACCAGGTGTCCCTGACCTGTCTGG TGAAGGGCTTCTACCCCAGTGATATCGCCGTGGAGTGG GAGAGCAACGGCCAGCCCGAGAACAACTACAAGACCAC CCCCCCAGTGCTGGACAGCGACGGCAGCTTCTTCCTGT ACAGCAAGCTGACCGTGGACAAGTCCAGGTGGCAGCAG GGCAACGTGTTCAGCTGCAGCGTGATGCACGAGGCCCT GCACAACCACTACACCCAGAAGTCCCTGAGCCTGAGCC CCGGCAAG |
| SEQ ID NO: 92 | LCDR1 (Combined) | SGDNLGTYYAH |
| SEQ ID NO: 93 | LCDR2 (Combined) | SQSHRPS |
| SEQ ID NO: 94 | LCDR3 (Combined) | GAWDAPSPELV |
| SEQ ID NO: 92 | LCDR1 (Kabat) | SGDNLGTYYAH |
| SEQ ID NO: 93 | LCDR2 (Kabat) | SQSHRPS |
| SEQ ID NO: 94 | LCDR3 (Kabat) | GAWDAPSPELV |
| SEQ ID NO: 95 | LCDR1 (Chothia) | DNLGTYY |
| SEQ ID NO: 96 | LCDR2 (Chothia) | SQS |
| SEQ ID NO: 97 | LCDR3 (Chothia) | WDAPSPEL |
| SEQ ID NO: 98 | LCDR1 (IMGT) | NLGTYY |
| SEQ ID NO: 96 | LCDR2 (IMGT) | SQS |
| SEQ ID NO: 94 | LCDR3 (IMGT) | GAWDAPSPELV |
| SEQ ID NO: 99 | VL | SYELTQPLSVSVALGQTARITCSGDNLGTYYAHWYQQK PGQAPVLVIYSQSHRPSGIPERFSGSNSGNTATLTISR AQAGDEADYYCGAWDAPSPELVFGGGTKLTVL |
| SEQ ID NO: 100 | DNA VL | AGCTATGAGCTGACACAGCCTCTGTCCGTGTCTGTGGC TCTGGGACAGACCGCCAGAATCACCTGTAGCGGCGATA ACCTGGGCACCTACTACGCCCACTGGTATCAGCAGAAG CCTGGACAGGCTCCCGTGCTGGTCATCTACAGCCAGTC TCACAGACCCAGCGGCATCCCCGAGAGATTCAGCGGCA GCAATAGCGGCAATACCGCCACACTGACCATCAGCAGA GCACAGGCTGGCGACGAGGCCGATTACTATTGTGGCGC TTGGGACGCCCCATCTCCTGAGCTTGTTTTTGGCGGAG GCACCAAGCTGACAGTGCTG |
| SEQ ID NO: 101 | Light Chain | SYELTQPLSVSVALGQTARITCSGDNLGTYYAHWYQQK PGQAPVLVIYSQSHRPSGIPERFSGSNSGNTATLTISR AQAGDEADYYCGAWDAPSPELVFGGGTKLTVLGQPKAA PSVTLFPPSSEELQANKATLVCLISDFYPGAVTVAWKA DSSPVKAGVETTTPSKQSNNKYAASSYLSLTPEQWKSH RSYSCQVTHEGSTVEKTVAPTECS |
| SEQ ID NO: 102 | DNA Light Chain | AGCTATGAGCTGACACAGCCTCTGTCCGTGTCTGTGGC TCTGGGACAGACCGCCAGAATCACCTGTAGCGGCGATA ACCTGGGCACCTACTACGCCCACTGGTATCAGCAGAAG CCTGGACAGGCTCCCGTGCTGGTCATCTACAGCCAGTC TCACAGACCCAGCGGCATCCCCGAGAGATTCAGCGGCA GCAATAGCGGCAATACCGCCACACTGACCATCAGCAGA GCACAGGCTGGCGACGAGGCCGATTACTATTGTGGCGC TTGGGACGCCCCATCTCCTGAGCTTGTTTTTGGCGGAG GCACCAAGCTGACAGTGCTGGGACAGCCTAAGGCCGCT |
| | | CCCTCCGTGACCCTGTTCCCCCCCAGCTCCGAGGAACT GCAGGCCAACAAGGCCACCCTGGTGTGCCTGATCAGCG ACTTCTACCCTGGCGCCGTGACCGTGGCCTGGAAGGCC GACAGCAGCCCCGTGAAGGCCGGCGTGGAGACAACCAC CCCCAGCAAGCAGAGCAACAACAAGTACGCCGCCAGCA GCTACCTGAGCCTGACCCCCGAGCAGTGGAAGAGCCAC AGAAGCTACAGCTGCCAGGTCACCCACGAGGGCAGCAC CGTGGAGAAAACCGTGGCCCCCACCGAGTGCAGC |

Generation of anti-PMEL17 antibodies are also described in Example 2 of International Application Publication No. WO 2020/128612, which is incorporated by reference in its entirety.

Other antibodies of the disclosure include those where the amino acids or nucleic acids encoding the amino acids have been mutated, yet have at least about 60, about 70, about 80, about 90 or about 95 percent identity to the sequences described in **Table 2.** In some embodiments, about 1, about 2, about 3, about 4 or about 5 amino acids have been mutated in the variable regions when compared with the variable regions depicted in the sequence described in **Table 2,** while retaining substantially the same therapeutic activity as the antibodies listed in **Table 2.**

Since each of these antibodies can bind to PMEL17, the VH, VL, full length light chain, and full-length heavy chain sequences (amino acid sequences and the nucleotide sequences encoding the amino acid sequences) can be "mixed and matched" to create other PMEL17-binding antibodies of the disclosure. Such "mixed and matched" PMEL17-binding antibodies can be tested using the binding assays known in the art (*e.g*., ELISAs, and other assays described in the Example section). When these chains are mixed and matched, a VH sequence from a particular VH/VL pairing should be replaced with a structurally similar VH sequence. Likewise, a full length heavy chain sequence from a particular full length heavy chain / full length light chain pairing should be replaced with a structurally similar full length heavy chain sequence. Likewise, a VL sequence from a particular VH/VL pairing should be replaced with a structurally similar VL sequence. Likewise a full length light chain sequence from a particular full length heavy chain / full length light chain pairing should be replaced with a structurally similar full length light chain sequence. Accordingly, in some embodiments, the disclosure provides an isolated monoclonal antibody or antigen binding region thereof having: a heavy chain variable region comprising an amino acid sequence selected from the group consisting of SEQ ID NOs: 10, 42, 64, 88, 112, 132, 149, 165, 184, 196, 215, 227, 239 or 254; and a light chain variable region comprising an amino acid sequence selected from the group consisting of SEQ ID NOs: 21, 25, 29, 53, 75, 99, 119, 143, 159, 171, 190, 202, 221, 233, 248 or 260; wherein the antibody specifically binds to PMEL17.

In some embodiments, the disclosure provides (i) an isolated monoclonal antibody having: a full length heavy chain comprising an amino acid sequence that has been optimized for expression in the cell of a mammalian expression system selected from the group consisting of SEQ ID NOs: 12, 44, 66, 90, 114, 134, 151, 167, 186, 198, 217, 229, 241,256, or 315; and a full length light chain comprising an amino acid sequence that has been optimized for expression in the cell of a mammalian selected from the group consisting of SEQ ID NOs: 23, 27, 31, 55, 77, 101, 121, 145, 161, 173, 192, 204, 223, 235, 250 or 262; or (ii) a functional protein comprising an antigen binding portion thereof.

In some embodiments, the disclosure provides PMEL17-binding antibodies that comprise the heavy chain and light chain CDR1s, CDR2s and CDR3s as described in **Table 2,** or combinations thereof. The amino acid sequences of the VH CDR1s of the antibodies are shown, for example, in SEQ ID NOs: 1, 4, 5, 7, 33, 36, 37, 39, 57, 60, 79, 82, 83, 85, 103, 106, 107, 109, 123, 126, 127, 129, 175, 178, 179, 181, 206, 209, 210, and 212. The amino acid sequences of the VH CDR2s of the antibodies and are shown, for example, in SEQ ID NOs: 2, 6, 8, 34, 38, 40, 58, 61, 62, 80, 84, 86, 104, 108, 110, 124, 128, 130, 176, 180, 182, 207, 211, and 213. The amino acid sequences of the VH CDR3s of the antibodies are shown, for example, in SEQ ID NOs: 3, 9, 35, 41, 59, 63, 81, 87, 105, 111, 125, 131, 147, 148, 163, 164, 177, 183, 194, 195, 208, 214, 225, 226, 237, 238, 252, and 253. The amino acid sequences of the VL CDR1s of the antibodies are shown, for example, in SEQ ID NOs: 14, 17, 20, 46, 49, 52, 68, 71, 74, 92, 95, 98, 116, 136, 139, 142, 153, 156, 158, 243, 245, and 247. The amino acid sequences of the VL CDR2s of the antibodies are shown, for example, in SEQ ID Nos: 15, 18, 47, 50, 69, 72, 93, 96, 137, 140, and 154. The amino acid sequences of the VL CDR3s of the antibodies are shown, for example, in SEQ ID NOs: 16, 19, 48, 51, 70, 73, 94, 97, 117, 118, 138, 141, 155, 157, 169, 170188, 189, 200, 201, 219, 220, 231, 232, 244, 246, 258, and 259.

Given that each of these antibodies can bind to PMEL17 and that antigen-binding specificity is provided primarily by the CDR1, 2 and 3 regions, the VH CDR1, CDR2 and CDR3 sequences and VL CDR1, CDR2 and CDR3 sequences can be "mixed and matched" (*i.e.*, CDRs from different antibodies can be mixed and matched. Such "mixed and matched" PMEL17-binding antibodies can be tested using the binding assays known in the art and those described in the Examples (*e.g*., ELISAs). When VH CDR sequences are mixed and matched, the CDR1, CDR2 and/or CDR3 sequence from a particular VH sequence should be replaced with a structurally similar CDR sequence(s). Likewise, when VL CDR sequences are mixed and matched, the CDR1, CDR2 and/or CDR3 sequence from a particular VL sequence should be replaced with a structurally similar CDR sequence(s). It will be readily apparent to the ordinarily skilled artisan that novel VH and VL sequences can be created by substituting one or more VH and/or VL CDR region sequences with structurally similar sequences from the CDR sequences shown herein for monoclonal antibodies of the present disclosure.

Accordingly, in some embodiments, the present disclosure provides an isolated monoclonal antibody or antigen binding region thereof comprising a heavy chain CDR1 comprising an amino acid sequence selected from the group consisting of SEQ ID NOs: 1, 4, 5, 7, 33, 36, 37, 39, 57, 60, 79, 82, 83, 85, 103, 106, 107, 109, 123, 126, 127, 129, 175, 178, 179, 181, 206, 209, 210, and 212; a heavy chain CDR2 comprising an amino acid sequence selected from the group consisting of SEQ ID NOs: 2, 6, 8, 34, 38, 40, 58, 61, 62, 80, 84, 86, 104, 108, 110, 124, 128, 130, 176, 180, 182, 207, 211, and 213; a heavy chain CDR3 comprising an amino acid sequence selected from the group consisting of SEQ ID NOs: 3, 9, 35, 41, 59, 63, 81, 87, 105, 111, 125, 131, 147, 148, 163, 164, 177, 183, 194, 195, 208, 214, 225, 226, 237, 238, 252, and 253; a light chain CDR1 comprising an amino acid sequence selected from the group consisting of SEQ ID NOs: 14, 17, 20, 46, 49, 52, 68, 71, 74, 92, 95, 98, 116, 136, 139, 142, 153, 156, 158, 243, 245, and 247; a light chain CDR2 comprising an amino acid sequence selected from the group consisting of SEQ ID NOs: 15, 18, 47, 50, 69, 72, 93, 96, 137, 140, and 154; and a light chain CDR3 comprising an amino acid sequence selected from the group consisting of SEQ ID NOs: 16, 19, 48, 51, 70, 73, 94, 97, 117, 118, 138, 141, 155, 157, 169, 170, 188, 189, 200, 201, 219, 220, 231, 232, 244, 246, 258, and 259; wherein the antibody specifically binds PMEL17.

In a specific embodiment, an antibody or antibody fragment (*e.g*., antigen binding fragments) that specifically binds to PMEL17 comprises a heavy chain CDR1 of SEQ ID NO:1, 4, 5 or 7, a heavy chain CDR2 of SEQ ID NO:2, 6 or 8; a heavy chain CDR3 of SEQ ID NO:3 or 9; a light chain CDR1 of SEQ ID NO:14, 17 or 20; a light chain CDR2 of SEQ ID NO:15 or 18; and a light chain CDR3 of SEQ ID NO:16 or 19.

In a specific embodiment, an antibody or antibody fragment (*e.g*., antigen binding fragments) that specifically binds to PMEL17 comprises a heavy chain CDR1 of SEQ ID NO:33, 36, 37 or 39, a heavy chain CDR2 of SEQ ID NO:34, 38 or 40; a heavy chain CDR3 of SEQ ID NO:35 or 41; a light chain CDR1 of SEQ ID NO:46, 49 or 52; a light chain CDR2 of SEQ ID NO:47 or 50; and a light chain CDR3 of SEQ ID NO:48 or 51.

In a specific embodiment, an antibody or antibody fragment (*e.g*., antigen binding fragments) that specifically binds to PMEL17 comprises a heavy chain CDR1 of SEQ ID NO:5, 7, 57 or 60, a heavy chain CDR2 of SEQ ID NO:58, 61 or 62; a heavy chain CDR3 of SEQ ID NO:59 or 63; a light chain CDR1 of SEQ ID NO:68, 71 or 74; a light chain CDR2 of SEQ ID NO:69 or 72; and a light chain CDR3 of SEQ ID NO:70 or 73.

In a specific embodiment, an antibody or antibody fragment (*e.g*., antigen binding fragments) that specifically binds to PMEL17 comprises a heavy chain CDR1 of SEQ ID NO:79, 82, 83 or 85, a heavy chain CDR2 of SEQ ID NO:80, 84 or 86; a heavy chain CDR3 of SEQ ID NO:81 or 87; a light chain CDR1 of SEQ ID NO:92, 95 or 98; a light chain CDR2 of SEQ ID NO:93 or 96; and a light chain CDR3 of SEQ ID NO:94 or 97.

In a specific embodiment, an antibody or antibody fragment (*e.g*., antigen binding fragments) that specifically binds to PMEL17 comprises a heavy chain CDR1 of SEQ ID NO:103, 106, 107 or 109, a heavy chain CDR2 of SEQ ID NO:104, 108 or 110; a heavy chain CDR3 of SEQ ID NO:105 or 111; a light chain CDR1 of SEQ ID NO:49, 52 or 116; a light chain CDR2 of SEQ ID NO:47 or 50; and a light chain CDR3 of SEQ ID NO:117 or 118.

In a specific embodiment, an antibody or antibody fragment (*e.g*., antigen binding fragments) that specifically binds to PMEL17 comprises a heavy chain CDR1 of SEQ ID NO:123, 126, 127 or 129, a heavy chain CDR2 of SEQ ID NO:124, 128 or 130; a heavy chain CDR3 of SEQ ID NO:125 or 131; a light chain CDR1 of SEQ ID NO:136, 139 or 142; a light chain CDR2 of SEQ ID NO:137 or 140; and a light chain CDR3 of SEQ ID NO:138 or 141.

In a specific embodiment, an antibody or antibody fragment (*e.g*., antigen binding fragments) that specifically binds to PMEL17 comprises a heavy chain CDR1 of SEQ ID NO:123, 126, 127 or 129, a heavy chain CDR2 of SEQ ID NO:124, 128 or 130; a heavy chain CDR3 of SEQ ID NO:147 or 148; a light chain CDR1 of SEQ ID NO:153, 156 or 158; a light chain CDR2 of SEQ ID NO:50 or 154; and a light chain CDR3 of SEQ ID NO:155 or 157.

In a specific embodiment, an antibody or antibody fragment (*e.g*., antigen binding fragments) that specifically binds to PMEL17 comprises a heavy chain CDR1 of SEQ ID NO:103, 106, 107 or 109, a heavy chain CDR2 of SEQ ID NO:104, 108 or 110; a heavy chain CDR3 of SEQ ID NO:163 or 164; a light chain CDR1 of SEQ ID NO:49, 52 or 116; a light chain CDR2 of SEQ ID NO:47 or 50; and a light chain CDR3 of SEQ ID NO:169 or 170.

In a specific embodiment, an antibody or antibody fragment (*e.g*., antigen binding fragments) that specifically binds to PMEL17 comprises a heavy chain CDR1 of SEQ ID NO:175, 178, 179 or 181, a heavy chain CDR2 of SEQ ID NO:176, 180 or 182; a heavy chain CDR3 of SEQ ID NO:177 or 183; a light chain CDR1 of SEQ ID NO:49, 52 or 116; a light chain CDR2 of SEQ ID NO:47 or 50; and a light chain CDR3 of SEQ ID NO:188 or 189.

In a specific embodiment, an antibody or antibody fragment (*e.g*., antigen binding fragments) that specifically binds to PMEL17 comprises a heavy chain CDR1 of SEQ ID NO: 103, 106, 107 or 109, a heavy chain CDR2 of SEQ ID NO: 104, 108 or 110; a heavy chain CDR3 of SEQ ID NO:194 or 195; a light chain CDR1 of SEQ ID NO: 49, 52 or 116; a light chain CDR2 of SEQ ID NO: 47 or 50; and a light chain CDR3 of SEQ ID NO:200 or 201.

In a specific embodiment, an antibody or antibody fragment (*e.g*., antigen binding fragments) that specifically binds to PMEL17 comprises a heavy chain CDR1 of SEQ ID NO:206, 209, 210 or 212, a heavy chain CDR2 of SEQ ID NO:207, 211 or 213; a heavy chain CDR3 of SEQ ID NO:208 or 214; a light chain CDR1 of SEQ ID NO:153, 156 or 158; a light chain CDR2 of SEQ ID NO:50 or 154; and a light chain CDR3 of SEQ ID NO:219 or 220.

In a specific embodiment, an antibody or antibody fragment (*e.g*., antigen binding fragments) that specifically binds to PMEL17 comprises a heavy chain CDR1 of SEQ ID NO: 206, 209, 210 or 212, a heavy chain CDR2 of SEQ ID NO: 207, 211 or 213; a heavy chain CDR3 of SEQ ID NO:225 or 226; a light chain CDR1 of SEQ ID NO:136, 139 or 142; a light chain CDR2 of SEQ ID NO:137 or 140; and a light chain CDR3 of SEQ ID NO:231 or 232.

In a specific embodiment, an antibody or antibody fragment (*e.g*., antigen binding fragments) that specifically binds to PMEL17 comprises a heavy chain variable region that comprises an HCDR1 of SEQ ID NO: 206, 209, 210 or 212, an HCDR2 of SEQ ID NO: 207, 211 or 213, and an HCDR3 of SEQ ID NO:237 or 238; and a light chain variable region that comprises an LCDR1 of SEQ ID NO:243, 245 or 247, an LCDR2 of SEQ ID NO:47 or 50, and an LCDR3 of SEQ ID NO:244 or 246.

In a specific embodiment, an antibody or antibody fragment (*e.g*., antigen binding fragments) that specifically binds to PMEL17 comprises a heavy chain variable region that comprises an HCDR1 of SEQ ID NO: 206, 209, 210 or 212, an HCDR2 of SEQ ID NO: 207, 211 or 213, and an HCDR3 of SEQ ID NO:252 or 253; and a light chain variable region that comprises an LCDR1 of SEQ ID NO:153, 156 or 158, an LCDR2 of SEQ ID NO:50 or 154, and an LCDR3 of SEQ ID NO:258 or 259.

In a specific embodiment, an antibody or antibody fragment (*e.g*., antigen binding fragments) that specifically binds to PMEL17 comprises CDR sequences selected from:
a heavy chain CDR1 of SEQ ID NO:1, , a heavy chain CDR2 of SEQ ID NO:2, a heavy chain CDR3 of SEQ ID NO:3, a light chain CDR1 of SEQ ID NO:14, a light chain CDR2 of SEQ ID NO:15, and a light chain CDR3 of SEQ ID NO:16;
a heavy chain CDR1 of SEQ ID NO: 4, a heavy chain CDR2 of SEQ ID NO:2, a heavy chain CDR3 of SEQ ID NO:3, a light chain CDR1 of SEQ ID NO:14, a light chain CDR2 of SEQ ID NO:15, and a light chain CDR3 of SEQ ID NO:16;
a heavy chain CDR1 of SEQ ID NO:5, a heavy chain CDR2 of SEQ ID NO:6, a heavy chain CDR3 of SEQ ID NO:3, a light chain CDR1 of SEQ ID NO:17, a light chain CDR2 of SEQ ID NO: 18, and a light chain CDR3 of SEQ ID NO: 19; or
a heavy chain CDR1 of SEQ ID NO:7, a heavy chain CDR2 of SEQ ID NO:8, a heavy chain CDR3 of SEQ ID NO:9, a light chain CDR1 of SEQ ID NO:20, a light chain CDR2 of SEQ ID NO:18, and a light chain CDR3 of SEQ ID NO:16.

In a specific embodiment, an antibody or antibody fragment (*e.g*., antigen binding fragments) that specifically binds to PMEL17 comprises CDR sequences selected from:
a heavy chain CDR1 of SEQ ID NO:33, a heavy chain CDR2 of SEQ ID NO:34, a heavy chain CDR3 of SEQ ID NO:35, a light chain CDR1 of SEQ ID NO:46, a light chain CDR2 of SEQ ID NO:47, and a light chain CDR3 of SEQ ID NO:48;
a heavy chain CDR1 of SEQ ID NO:36, a heavy chain CDR2 of SEQ ID NO:34, a heavy chain CDR3 of SEQ ID NO:35, a light chain CDR1 of SEQ ID NO:46, a light chain CDR2 of SEQ ID NO:47, and a light chain CDR3 of SEQ ID NO:48;
a heavy chain CDR1 of SEQ ID NO:37, a heavy chain CDR2 of SEQ ID NO:38, a heavy chain CDR3 of SEQ ID NO:35, a light chain CDR1 of SEQ ID NO:49, a light chain CDR2 of SEQ ID NO:50, and a light chain CDR3 of SEQ ID NO:51; or
a heavy chain CDR1 of SEQ ID NO: 39, a heavy chain CDR2 of SEQ ID NO:40, a heavy chain CDR3 of SEQ ID NO:41, a light chain CDR1 of SEQ ID NO:52, a light chain CDR2 of SEQ ID NO:50, and a light chain CDR3 of SEQ ID NO:48.

In a specific embodiment, an antibody or antibody fragment (*e.g*., antigen binding fragments) that specifically binds to PMEL17 comprises CDR sequences selected from:
a) a heavy chain CDR1 of SEQ ID NO:57, a heavy chain CDR2 of SEQ ID NO:58, a heavy chain CDR3 of SEQ ID NO:59, a light chain CDR1 of SEQ ID NO:68, a light chain CDR2 of SEQ ID NO:69, and a light chain CDR3 of SEQ ID NO:70;
b) a heavy chain CDR1 of SEQ ID NO:60, a heavy chain CDR2 of SEQ ID NO:58, a heavy chain CDR3 of SEQ ID NO:59, a light chain CDR1 of SEQ ID NO:68, a light chain CDR2 of SEQ ID NO:69, and a light chain CDR3 of SEQ ID NO:70;
c) a heavy chain CDR1 of SEQ ID NO:5, a heavy chain CDR2 of SEQ ID NO:61, a heavy chain CDR3 of SEQ ID NO:59, a light chain CDR1 of SEQ ID NO:71, a light chain CDR2 of SEQ ID NO:72, and a light chain CDR3 of SEQ ID NO:73; or
d) a heavy chain CDR1 of SEQ ID NO:7, a heavy chain CDR2 of SEQ ID NO:62, a heavy chain CDR3 of SEQ ID NO:63, a light chain CDR1 of SEQ ID NO:74, a light chain CDR2 of SEQ ID NO:72, and a light chain CDR3 of SEQ ID NO:70.

In a specific embodiment, an antibody or antibody fragment (*e.g*., antigen binding fragments) that specifically binds to PMEL17 comprises CDR sequences selected from:
a heavy chain CDR1 of SEQ ID NO:79, , a heavy chain CDR2 of SEQ ID NO:80, a heavy chain CDR3 of SEQ ID NO:81, a light chain CDR1 of SEQ ID NO:92, a light chain CDR2 of SEQ ID NO:93, and a light chain CDR3 of SEQ ID NO:94;
a heavy chain CDR1 of SEQ ID NO:82, a heavy chain CDR2 of SEQ ID NO:80, a heavy chain CDR3 of SEQ ID NO:81, a light chain CDR1 of SEQ ID NO:92, a light chain CDR2 of SEQ ID NO:93, and a light chain CDR3 of SEQ ID NO:94;
a heavy chain CDR1 of SEQ ID NO:83, a heavy chain CDR2 of SEQ ID NO:84, a heavy chain CDR3 of SEQ ID NO:81, a light chain CDR1 of SEQ ID NO:95, a light chain CDR2 of SEQ ID NO:96, and a light chain CDR3 of SEQ ID NO: 97; or
a heavy chain CDR1 of SEQ ID NO: 85, a heavy chain CDR2 of SEQ ID NO:86, a heavy chain CDR3 of SEQ ID NO:87, a light chain CDR1 of SEQ ID NO:98, a light chain CDR2 of SEQ ID NO:96, and a light chain CDR3 of SEQ ID NO:94.

In a specific embodiment, an antibody or antibody fragment (*e.g*., antigen binding fragments) that specifically binds to PMEL17 comprises CDR sequences selected from:
a heavy chain CDR1 of SEQ ID NO:103, a heavy chain CDR2 of SEQ ID NO:104, a heavy chain CDR3 of SEQ ID NO:105, a light chain CDR1 of SEQ ID NO: 116; a light chain CDR2 of SEQ ID NO:47; and a light chain CDR3 of SEQ ID NO:117;
a heavy chain CDR1 of SEQ ID NO:106, a heavy chain CDR2 of SEQ ID NO:104, a heavy chain CDR3 of SEQ ID NO:105, a light chain CDR1 of SEQ ID NO: 116, a light chain CDR2 of SEQ ID NO:47, and a light chain CDR3 of SEQ ID NO:117;
a heavy chain CDR1 of SEQ ID NO:107, a heavy chain CDR2 of SEQ ID NO:108, a heavy chain CDR3 of SEQ ID NO:105, a light chain CDR1 of SEQ ID NO:49, a light chain CDR2 of SEQ ID NO:50, and a light chain CDR3 of SEQ ID NO:118; or
a heavy chain CDR1 of SEQ ID NO:109, a heavy chain CDR2 of SEQ ID NO:110, a heavy chain CDR3 of SEQ ID NO:111, a light chain CDR1 of SEQ ID NO:52 a light chain CDR2 of SEQ ID NO:50, and a light chain CDR3 of SEQ ID NO:117.

In a specific embodiment, an antibody or antibody fragment (*e.g*., antigen binding fragments) that specifically binds to PMEL17 comprises CDR sequences selected from:
a heavy chain CDR1 of SEQ ID NO:123, a heavy chain CDR2 of SEQ ID NO:124, a heavy chain CDR3 of SEQ ID NO:125, a light chain CDR1 of SEQ ID NO:136, a light chain CDR2 of SEQ ID NO:137,and a light chain CDR3 of SEQ ID NO:138;
a heavy chain CDR1 of SEQ ID NO:126, a heavy chain CDR2 of SEQ ID NO:124, a heavy chain CDR3 of SEQ ID NO:125, a light chain CDR1 of SEQ ID NO:136, a light chain CDR2 of SEQ ID NO:137, and a light chain CDR3 of SEQ ID NO:138;
a heavy chain CDR1 of SEQ ID NO:127, a heavy chain CDR2 of SEQ ID NO:128, a heavy chain CDR3 of SEQ ID NO:125, a light chain CDR1 of SEQ ID NO:139, a light chain CDR2 of SEQ ID NO:140, and a light chain CDR3 of SEQ ID NO: 141; or
a heavy chain CDR1 of SEQ ID NO: 129, a heavy chain CDR2 of SEQ ID NO:130, a heavy chain CDR3 of SEQ ID NO:131, a light chain CDR1 of SEQ ID NO:142, a light chain CDR2 of SEQ ID NO:140, and a light chain CDR3 of SEQ ID NO:138.

In a specific embodiment, an antibody or antibody fragment (*e.g*., antigen binding fragments) that specifically binds to PMEL17 comprises CDR sequences selected from:
a heavy chain CDR1 of SEQ ID NO:123, a heavy chain CDR2 of SEQ ID NO:124, a heavy chain CDR3 of SEQ ID NO:147, a light chain CDR1 of SEQ ID NO:153, a light chain CDR2 of SEQ ID NO:154, and a light chain CDR3 of SEQ ID NO:155;
a heavy chain CDR1 of SEQ ID NO:126, a heavy chain CDR2 of SEQ ID NO:124, a heavy chain CDR3 of SEQ ID NO:147, a light chain CDR1 of SEQ ID NO:153, a light chain CDR2 of SEQ ID NO: 154, and a light chain CDR3 of SEQ ID NO:155;
a heavy chain CDR1 of SEQ ID NO:127, a heavy chain CDR2 of SEQ ID NO:128, a heavy chain CDR3 of SEQ ID NO:147, a light chain CDR1 of SEQ ID NO:156, a light chain CDR2 of SEQ ID NO:50, and a light chain CDR3 of SEQ ID NO:157; or
a heavy chain CDR1 of SEQ ID NO: 129, a heavy chain CDR2 of SEQ ID NO:130, a heavy chain CDR3 of SEQ ID NO:148, a light chain CDR1 of SEQ ID NO:158, a light chain CDR2 of SEQ ID NO:50, and a light chain CDR3 of SEQ ID NO:155.

In a specific embodiment, an antibody or antibody fragment (*e.g*., antigen binding fragments) that specifically binds to PMEL17 comprises CDR sequences selected from:
a heavy chain CDR1 of SEQ ID NO:103, a heavy chain CDR2 of SEQ ID NO:104, a heavy chain CDR3 of SEQ ID NO:163, a light chain CDR1 of SEQ ID NO: 116, a light chain CDR2 of SEQ ID NO:47, and a light chain CDR3 of SEQ ID NO:169;
a heavy chain CDR1 of SEQ ID NO:106, a heavy chain CDR2 of SEQ ID NO:104, a heavy chain CDR3 of SEQ ID NO:163, a light chain CDR1 of SEQ ID NO:116, a light chain CDR2 of SEQ ID NO:47, and a light chain CDR3 of SEQ ID NO:169;
a heavy chain CDR1 of SEQ ID NO:107, a heavy chain CDR2 of SEQ ID NO:108, a heavy chain CDR3 of SEQ ID NO:163, a light chain CDR1 of SEQ ID NO:49, a light chain CDR2 of SEQ ID NO:50, and a light chain CDR3 of SEQ ID NO:170; or
a heavy chain CDR1 of SEQ ID NO: 109, a heavy chain CDR2 of SEQ ID NO:110, a heavy chain CDR3 of SEQ ID NO:164, a light chain CDR1 of SEQ ID NO:52, a light chain CDR2 of SEQ ID NO:50, and a light chain CDR3 of SEQ ID NO:169.

In a specific embodiment, an antibody or antibody fragment (*e.g*., antigen binding fragments) that specifically binds to PMEL17 comprises CDR sequences selection from:
a heavy chain CDR1 of SEQ ID NO:175, a heavy chain CDR2 of SEQ ID NO:176, a heavy chain CDR3 of SEQ ID NO:177, a light chain CDR1 of SEQ ID NO:116, a light chain CDR2 of SEQ ID NO:47, and a light chain CDR3 of SEQ ID NO:188;
a heavy chain CDR1 of SEQ ID NO:178, a heavy chain CDR2 of SEQ ID NO:176, a heavy chain CDR3 of SEQ ID NO:177, a light chain CDR1 of SEQ ID NO:116, a light chain CDR2 of SEQ ID NO:47, and a light chain CDR3 of SEQ ID NO:188;
a heavy chain CDR1 of SEQ ID NO:179, a heavy chain CDR2 of SEQ ID NO:180, a heavy chain CDR3 of SEQ ID NO:177, a light chain CDR1 of SEQ ID NO:49, a light chain CDR2 of SEQ ID NO:50, and a light chain CDR3 of SEQ ID NO:189; or
a heavy chain CDR1 of SEQ ID NO: 181, a heavy chain CDR2 of SEQ ID NO:182; a heavy chain CDR3 of SEQ ID NO:183, a light chain CDR1 of SEQ ID NO:52, a light chain CDR2 of SEQ ID NO:50, and a light chain CDR3 of SEQ ID NO:188.

In a specific embodiment, an antibody or antibody fragment (*e.g*., antigen binding fragments) that specifically binds to PMEL17 comprises CDR sequences selected from:
a heavy chain CDR1 of SEQ ID NO: 103, a heavy chain CDR2 of SEQ ID NO: 104, a heavy chain CDR3 of SEQ ID NO:194, a light chain CDR1 of SEQ ID NO: 116, a light chain CDR2 of SEQ ID NO: 47, and a light chain CDR3 of SEQ ID NO:200;
a heavy chain CDR1 of SEQ ID NO: 106, a heavy chain CDR2 of SEQ ID NO: 104, a heavy chain CDR3 of SEQ ID NO:194, a light chain CDR1 of SEQ ID NO: 116, a light chain CDR2 of SEQ ID NO: 47, and a light chain CDR3 of SEQ ID NO:200;
a heavy chain CDR1 of SEQ ID NO: 107, a heavy chain CDR2 of SEQ ID NO: 108, a heavy chain CDR3 of SEQ ID NO:194, a light chain CDR1 of SEQ ID NO: 49, a light chain CDR2 of SEQ ID NO: 50, and a light chain CDR3 of SEQ ID NO: 201; or
a heavy chain CDR1 of SEQ ID NO: 109, a heavy chain CDR2 of SEQ ID NO: 110, a heavy chain CDR3 of SEQ ID NO:195, a light chain CDR1 of SEQ ID NO: 52, a light chain CDR2 of SEQ ID NO: 50, and a light chain CDR3 of SEQ ID NO:200.

In a specific embodiment, an antibody or antibody fragment (*e.g*., antigen binding fragments) that specifically binds to PMEL17 comprises CDR sequences selected from:
a heavy chain CDR1 of SEQ ID NO:206, a heavy chain CDR2 of SEQ ID NO:207, a heavy chain CDR3 of SEQ ID NO:208, a light chain CDR1 of SEQ ID NO:153, a light chain CDR2 of SEQ ID NO:154, and a light chain CDR3 of SEQ ID NO:219;
a heavy chain CDR1 of SEQ ID NO:209, a heavy chain CDR2 of SEQ ID NO:207, a heavy chain CDR3 of SEQ ID NO:208, a light chain CDR1 of SEQ ID NO:153, a light chain CDR2 of SEQ ID NO: 154, and a light chain CDR3 of SEQ ID NO:219;
a heavy chain CDR1 of SEQ ID NO:210, a heavy chain CDR2 of SEQ ID NO:211, a heavy chain CDR3 of SEQ ID NO:208, a light chain CDR1 of SEQ ID NO:156, a light chain CDR2 of SEQ ID NO:50, and a light chain CDR3 of SEQ ID NO:220; or
a heavy chain CDR1 of SEQ ID NO: 212, a heavy chain CDR2 of SEQ ID NO:213, a heavy chain CDR3 of SEQ ID NO:214, a light chain CDR1 of SEQ ID NO:158, a light chain CDR2 of SEQ ID NO:50, and a light chain CDR3 of SEQ ID NO:219.

In a specific embodiment, an antibody or antibody fragment (*e.g*., antigen binding fragments) that specifically binds to PMEL17 comprises CDR sequences selected from:
a heavy chain CDR1 of SEQ ID NO: 206, a heavy chain CDR2 of SEQ ID NO: 207, a heavy chain CDR3 of SEQ ID NO:225, a light chain CDR1 of SEQ ID NO:136, a light chain CDR2 of SEQ ID NO:137,and a light chain CDR3 of SEQ ID NO:231;
a heavy chain CDR1 of SEQ ID NO: 209, a heavy chain CDR2 of SEQ ID NO: 207, a heavy chain CDR3 of SEQ ID NO:225, a light chain CDR1 of SEQ ID NO:136, a light chain CDR2 of SEQ ID NO:137, and a light chain CDR3 of SEQ ID NO:231;
a heavy chain CDR1 of SEQ ID NO: 210, a heavy chain CDR2 of SEQ ID NO: 211, a heavy chain CDR3 of SEQ ID NO:225, a light chain CDR1 of SEQ ID NO:139, a light chain CDR2 of SEQ ID NO:140, and a light chain CDR3 of SEQ ID NO: 232; or
a heavy chain CDR1 of SEQ ID NO: 212, a heavy chain CDR2 of SEQ ID NO: 213, a heavy chain CDR3 of SEQ ID NO: 226, a light chain CDR1 of SEQ ID NO:142; a light chain CDR2 of SEQ ID NO: 140; and a light chain CDR3 of SEQ ID NO:231.

In a specific embodiment, an antibody or antibody fragment (*e.g*., antigen binding fragments) that specifically binds to PMEL17 comprises CDR sequences selected from:
a heavy chain variable region that comprises an HCDR1 of SEQ ID NO: 206, an HCDR2 of SEQ ID NO: 207, and an HCDR3 of SEQ ID NO:237, and a light chain variable region that comprises an LCDR1 of SEQ ID NO:243, an LCDR2 of SEQ ID NO:47, and an LCDR3 of SEQ ID NO:244;
a heavy chain variable region that comprises an HCDR1 of SEQ ID NO: 209, an HCDR2 of SEQ ID NO: 207, and an HCDR3 of SEQ ID NO:237, and a light chain variable region that comprises an LCDR1 of SEQ ID NO:243, an LCDR2 of SEQ ID NO:47, and an LCDR3 of SEQ ID NO:244;
a heavy chain variable region that comprises an HCDR1 of SEQ ID NO: 210, an HCDR2 of SEQ ID NO: 211, and an HCDR3 of SEQ ID NO:237, and a light chain variable region that comprises an LCDR1 of SEQ ID NO:245, an LCDR2 of SEQ ID NO:50, and an LCDR3 of SEQ ID NO:246; or
a heavy chain variable region that comprises an HCDR1 of SEQ ID NO: 212, an HCDR2 of SEQ ID NO: 213, and an HCDR3 of SEQ ID NO:238; and a light chain variable region that comprises an LCDR1 of SEQ ID NO:247, an LCDR2 of SEQ ID NO: 50, and an LCDR3 of SEQ ID NO:244.

In a specific embodiment, an antibody or antibody fragment (*e.g*., antigen binding fragments) that specifically binds to PMEL17 comprises CDR sequences selected from:
a heavy chain variable region that comprises an HCDR1 of SEQ ID NO: 206, an HCDR2 of SEQ ID NO: 207, and an HCDR3 of SEQ ID NO:252, and a light chain variable region that comprises an LCDR1 of SEQ ID NO:153, an LCDR2 of SEQ ID NO: 154, and an LCDR3 of SEQ ID NO:258;
a heavy chain variable region that comprises an HCDR1 of SEQ ID NO: 209, an HCDR2 of SEQ ID NO: 207, and an HCDR3 of SEQ ID NO:252, and a light chain variable region that comprises an LCDR1 of SEQ ID NO:153, an LCDR2 of SEQ ID NO:154, and an LCDR3 of SEQ ID NO:258;
a heavy chain variable region that comprises an HCDR1 of SEQ ID NO: 210, an HCDR2 of SEQ ID NO: 211, and an HCDR3 of SEQ ID NO:252, and a light chain variable region that comprises an LCDR1 of SEQ ID NO:156, an LCDR2 of SEQ ID NO:50, and an LCDR3 of SEQ ID NO:259; or
a heavy chain variable region that comprises an HCDR1 of SEQ ID NO: 212, an HCDR2 of SEQ ID NO: 213, and an HCDR3 of SEQ ID NO: 253; and a light chain variable region that comprises an LCDR1 of SEQ ID NO:158, an LCDR2 of SEQ ID NO:50, and an LCDR3 of SEQ ID NO:258.

In a specific embodiment, an antibody or antibody fragment (*e.g*., antigen binding fragments) that specifically binds to PMEL17 comprises a heavy chain variable region (VH) comprising the amino acid sequence of SEQ ID NO:10, and a light chain variable region (VL) comprising the amino acid sequence of SEQ ID NO:21.

In a specific embodiment, an antibody or antibody fragment (*e.g*., antigen binding fragments) that specifically binds to PMEL17 comprises a heavy chain variable region (VH) comprising the amino acid sequence of SEQ ID NO:10, and a light chain variable region (VL) comprising the amino acid sequence of SEQ ID NO:25.

In a specific embodiment, an antibody or antibody fragment (*e.g*., antigen binding fragments) that specifically binds to PMEL17 comprises a heavy chain variable region (VH) comprising the amino acid sequence of SEQ ID NO:10, and a light chain variable region (VL) comprising the amino acid sequence of SEQ ID NO:29.

In a specific embodiment, an antibody or antibody fragment (*e.g*., antigen binding fragments) that specifically binds to PMEL17 comprises a heavy chain variable region (VH) comprising the amino acid sequence of SEQ ID NO:42, and a light chain variable region (VL) comprising the amino acid sequence of SEQ ID NO:53.

In a specific embodiment, an antibody or antibody fragment (*e.g*., antigen binding fragments) that specifically binds to PMEL17 comprises a heavy chain variable region (VH) comprising the amino acid sequence of SEQ ID NO:64, and a light chain variable region (VL) comprising the amino acid sequence of SEQ ID NO:75.

In a specific embodiment, an antibody or antibody fragment (*e.g*., antigen binding fragments) that specifically binds to PMEL17 comprises a heavy chain variable region (VH) comprising the amino acid sequence of SEQ ID NO:88, and a light chain variable region (VL) comprising the amino acid sequence of SEQ ID NO:99.

In a specific embodiment, an antibody or antibody fragment (*e.g*., antigen binding fragments) that specifically binds to PMEL17 comprises a heavy chain variable region (VH) comprising the amino acid sequence of SEQ ID NO:112, and a light chain variable region (VL) comprising the amino acid sequence of SEQ ID NO:119.

In a specific embodiment, an antibody or antibody fragment (*e.g*., antigen binding fragments) that specifically binds to PMEL17 comprises a heavy chain variable region (VH) comprising the amino acid sequence of SEQ ID NO:132, and a light chain variable region (VL) comprising the amino acid sequence of SEQ ID NO:143.

In a specific embodiment, an antibody or antibody fragment (*e.g*., antigen binding fragments) that specifically binds to PMEL17 comprises a heavy chain variable region (VH) comprising the amino acid sequence of SEQ ID NO:149, and a light chain variable region (VL) comprising the amino acid sequence of SEQ ID NO:159.

In a specific embodiment, an antibody or antibody fragment (*e.g*., antigen binding fragments) that specifically binds to PMEL17 comprises a heavy chain variable region (VH) comprising the amino acid sequence of SEQ ID NO:165, and a light chain variable region (VL) comprising the amino acid sequence of SEQ ID NO:171.

In a specific embodiment, an antibody or antibody fragment (*e.g*., antigen binding fragments) that specifically binds to PMEL17 comprises a heavy chain variable region (VH) comprising the amino acid sequence of SEQ ID NO:184, and a light chain variable region (VL) comprising the amino acid sequence of SEQ ID NO:190.

In a specific embodiment, an antibody or antibody fragment (*e.g*., antigen binding fragments) that specifically binds to PMEL17 comprises a heavy chain variable region (VH) comprising the amino acid sequence of SEQ ID NO:196, and a light chain variable region (VL) comprising the amino acid sequence of SEQ ID NO:202.

In a specific embodiment, an antibody or antibody fragment (*e.g*., antigen binding fragments) that specifically binds to PMEL17 comprises a heavy chain variable region (VH) comprising the amino acid sequence of SEQ ID NO:215, and a light chain variable region (VL) comprising the amino acid sequence of SEQ ID NO:221.

In a specific embodiment, an antibody or antibody fragment (*e.g*., antigen binding fragments) that specifically binds to PMEL17 comprises a heavy chain variable region (VH) comprising the amino acid sequence of SEQ ID NO:227, and a light chain variable region (VL) comprising the amino acid sequence of SEQ ID NO:233.

In a specific embodiment, an antibody or antibody fragment (*e.g*., antigen binding fragments) that specifically binds to PMEL17 comprises a heavy chain variable region (VH) comprising the amino acid sequence of SEQ ID NO:239, and a light chain variable region (VL) comprising the amino acid sequence of SEQ ID NO:248.

In a specific embodiment, an antibody or antibody fragment (*e.g*., antigen binding fragments) that specifically binds to PMEL17 comprises a heavy chain variable region (VH) comprising the amino acid sequence of SEQ ID NO:254, and a light chain variable region (VL) comprising the amino acid sequence of SEQ ID NO:260.

In a specific embodiment, an antibody or antibody fragment (*e.g*., antigen binding fragments) that specifically binds to PMEL17 comprises a heavy chain comprising the amino acid sequence of SEQ ID NO:12, and a light chain comprising the amino acid sequence of SEQ ID NO:23.

In a specific embodiment, an antibody or antibody fragment (*e.g*., antigen binding fragments) that specifically binds to PMEL17 comprises a heavy chain comprising the amino acid sequence of SEQ ID NO:12, and a light chain comprising the amino acid sequence of SEQ ID NO:27.

In a specific embodiment, an antibody or antibody fragment (*e.g*., antigen binding fragments) that specifically binds to PMEL17 comprises a heavy chain comprising the amino acid sequence of SEQ ID NO:12, and a light chain comprising the amino acid sequence of SEQ ID NO:31.

In a specific embodiment, an antibody or antibody fragment (*e.g*., antigen binding fragments) that specifically binds to PMEL17 comprises a heavy chain comprising the amino acid sequence of SEQ ID NO:44, and a light chain comprising the amino acid sequence of SEQ ID NO:55.

In a specific embodiment, an antibody or antibody fragment (*e.g*., antigen binding fragments) that specifically binds to PMEL17 comprises a heavy chain comprising the amino acid sequence of SEQ ID NO:66, and a light chain comprising the amino acid sequence of SEQ ID NO:77.

In a specific embodiment, an antibody or antibody fragment (*e.g*., antigen binding fragments) that specifically binds to PMEL17 comprises a heavy chain comprising the amino acid sequence of SEQ ID NO:90, and a light chain comprising the amino acid sequence of SEQ ID NO:101.

In a specific embodiment, an antibody or antibody fragment (*e.g*., antigen binding fragments) that specifically binds to PMEL17 comprises a heavy chain comprising the amino acid sequence of SEQ ID NO:114, and a light chain comprising the amino acid sequence of SEQ ID NO:121.

In a specific embodiment, an antibody or antibody fragment (*e.g*., antigen binding fragments) that specifically binds to PMEL17 comprises a heavy chain comprising the amino acid sequence of SEQ ID NO:134, and a light chain comprising the amino acid sequence of SEQ ID NO:145.

In a specific embodiment, an antibody or antibody fragment (*e.g*., antigen binding fragments) that specifically binds to PMEL17 comprises a heavy chain comprising the amino acid sequence of SEQ ID NO:151, and a light chain comprising the amino acid sequence of SEQ ID NO:161.

In a specific embodiment, an antibody or antibody fragment (*e.g*., antigen binding fragments) that specifically binds to PMEL17 comprises a heavy chain comprising the amino acid sequence of SEQ ID NO:167, and a light chain comprising the amino acid sequence of SEQ ID NO:173.

In a specific embodiment, an antibody or antibody fragment (*e.g*., antigen binding fragments) that specifically binds to PMEL17 comprises a heavy chain comprising the amino acid sequence of SEQ ID NO:186, and a light chain comprising the amino acid sequence of SEQ ID NO:192.

In a specific embodiment, an antibody or antibody fragment (*e.g*., antigen binding fragments) that specifically binds to PMEL17 comprises a heavy chain comprising the amino acid sequence of SEQ ID NO:198, and a light chain comprising the amino acid sequence of SEQ ID NO:204.

In a specific embodiment, an antibody or antibody fragment (*e.g*., antigen binding fragments) that specifically binds to PMEL17 comprises a heavy chain comprising the amino acid sequence of SEQ ID NO:217, and a light chain comprising the amino acid sequence of SEQ ID NO:223.

In a specific embodiment, an antibody or antibody fragment (*e.g*., antigen binding fragments) that specifically binds to PMEL17 comprises a heavy chain comprising the amino acid sequence of SEQ ID NO:229, and a light chain comprising the amino acid sequence of SEQ ID NO:235.

In a specific embodiment, an antibody or antibody fragment (*e.g*., antigen binding fragments) that specifically binds to PMEL17 comprises a heavy chain comprising the amino acid sequence of SEQ ID NO:241, and a light chain comprising the amino acid sequence of SEQ ID NO:250.

In a specific embodiment, an antibody or antibody fragment (*e.g*., antigen binding fragments) that specifically binds to PMEL17 comprises a heavy chain comprising the amino acid sequence of SEQ ID NO:256, and a light chain comprising the amino acid sequence of SEQ ID NO:262.

In a specific embodiment, an antibody or antibody fragment (*e.g*., antigen binding fragments) that specifically binds to PMEL17 comprises a heavy chain comprising the amino acid sequence of SEQ ID NO:283, and a light chain comprising the amino acid sequence of SEQ ID NO:27.

In a specific embodiment, an antibody or antibody fragment (*e.g*., antigen binding fragments) that specifically binds to PMEL17 comprises a heavy chain comprising the amino acid sequence of SEQ ID NO:283, and a light chain comprising the amino acid sequence of SEQ ID NO:31.

In a specific embodiment, an antibody or antibody fragment (*e.g*., antigen binding fragments) that specifically binds to PMEL17 comprises a heavy chain comprising the amino acid sequence of SEQ ID NO:315, and a light chain comprising the amino acid sequence of SEQ ID NO:101.

In certain embodiments, an antibody that specifically binds to PMEL17 is an antibody or antibody fragment (*e.g*., antigen binding fragment) that is described in **Table 2.**

In some embodiments, an antibody or antibody fragment (*e.g*., antigen binding fragments) that specifically binds to PMEL17 belongs to the IgG1/ λ isotype subclass. The N-glycosylation site is located at Asn306 on the heavy chain of the antibody or fragment. In some embodiments, an antibody or antibody fragment (*e.g.*, antigen binding fragments) that specifically binds to PMEL17 comprises an N-glycosylation site located at Asn306 of the heavy chain. Both heavy chains of the antibody or antibody fragment (*e.g*., antigen binding fragments) comprise oligosaccharide chains linked to the protein backbone at Asn306.

### 1. Identification of Epitopes and Antibodies That Bind to the Same Epitope

The disclosure also provides antibodies and antibody fragments (*e.g*., antigen binding fragments) that specifically bind to the same epitope as the anti-PMEL17 antibodies described in **Table 2,** or cross compete with the antibodies described in **Table 2.** Additional antibodies and antibody fragments (e.g., antigen binding fragments) can therefore be identified based on their ability to cross-compete (*e.g*., to competitively inhibit the binding of, in a statistically significant manner) with other antibodies of the disclosure in PMEL17 binding assays, for example, via BIACORE or assays known to persons skilled in the art for measuring binding. The ability of a test antibody to inhibit the binding of antibodies and antibody fragments (*e.g*., antigen binding fragments) of the present disclosure to a PMEL17 (*e.g*., human PMEL17) demonstrates that the test antibody can compete with that antibody or antibody fragment (*e.g*., antigen binding fragments) for binding to PMEL17; such an antibody may, according to non-limiting theory, bind to the same or a related (*e.g.*, a structurally similar or spatially proximal or overlapping) epitope on the PMEL17 protein as the antibody or antibody fragment (*e.g*., antigen binding fragments) with which it competes. In certain embodiments, the antibodies that bind to the same epitope on PMEL17 as the antibodies or antibody fragments (*e.g*., antigen binding fragments) described in **Table 2** are human or humanized monoclonal antibodies. Such human or humanized monoclonal antibodies can be prepared and isolated as described herein.

### 2. Further Alteration of the Framework of Fc Region

The immunoconjugates of the disclosure may comprise modified antibodies or antigen binding fragments thereof that further comprise modifications to framework residues within VH and/or VL, *e.g.*, to improve the properties of the antibody. In some embodiments, the framework modifications are made to decrease the immunogenicity of the antibody. For example, one approach is to "back-mutate" one or more framework residues to the corresponding germline sequence. More specifically, an antibody that has undergone somatic mutation may contain framework residues that differ from the germline sequence from which the antibody is derived. Such residues can be identified by comparing the antibody framework sequences to the germline sequences from which the antibody is derived. To return the framework region sequences to their germline configuration, the somatic mutations can be "back-mutated" to the germline sequence by, for example, site-directed mutagenesis. Such "back-mutated" antibodies are also intended to be encompassed by the disclosure.

Another type of framework modification involves mutating one or more residues within the framework region, or even within one or more CDR regions, to remove T-cell epitopes to thereby reduce the potential immunogenicity of the antibody. This approach is also referred to as "deimmunization" and is described in further detail in U.S. Application Publication No. 2003/0153043.

In addition, or in the alternative to modifications made within the framework or CDR regions, antibodies of the disclosure may be engineered to include modifications within the Fc region, typically to alter one or more functional properties of the antibody, such as serum half-life, complement fixation, Fc receptor binding, and/or antigen-dependent cellular cytotoxicity (ADCC). Furthermore, an antibody of the disclosure may be chemically modified (*e.g*., one or more chemical moieties can be attached to the antibody) or be modified to alter its glycosylation, again to alter one or more functional properties of the antibody. Each of these embodiments is described in further detail below.

In some embodiments, the hinge region of CH1 is modified such that the number of cysteine residues in the hinge region is altered, *e.g*., increased or decreased. This approach is described further in U.S. Patent No. 5,677,425. The number of cysteine residues in the hinge region of CH1 is altered to, for example, facilitate assembly of the light and heavy chains or to increase or decrease the stability of the antibody.

In some embodiments, the antibody or antibody fragment disclosed herein include modified or engineered amino acid residues, *e.g*., one or more cysteine residues, as sites for conjugation to a drug moiety (Junutula JR, et al., Nat Biotechnol 2008, 26:925-932). In one embodiment, the disclosure provides a modified antibody or antibody fragment comprising a substitution of one or more amino acids with cysteine at the positions described herein. Sites for cysteine substitution are in the constant regions of the antibody or antibody fragment and are thus applicable to a variety of antibody or antibody fragment, and the sites are selected to provide stable and homogeneous conjugates. A modified antibody or fragment can have one, two or more cysteine substitutions, and these substitutions can be used in combination with other modification and conjugation methods as described herein. Methods for inserting cysteine at specific locations of an antibody are known in the art, *see, e.g.*, Lyons et al., (1990) Protein Eng., 3:703-708, WO 2011/005481, WO2014/124316, WO 2015/138615. In certain embodiments, a modified antibody comprises a substitution of one or more amino acids with cysteine on its constant region selected from positions 117, 119, 121, 124, 139, 152, 153, 155, 157, 164, 169, 171, 174, 189, 191, 195, 197, 205, 207, 246, 258, 269, 274, 286, 288, 290, 292, 293, 320, 322, 326, 333, 334, 335, 337, 344, 355, 360, 375, 382, 390, 392, 398, 400 and 422 of a heavy chain of the antibody, and wherein the positions are numbered according to the EU system. In some embodiments a modified antibody or antibody fragment comprises a substitution of one or more amino acids with cysteine on its constant region selected from positions 107, 108, 109, 114, 129, 142, 143, 145, 152, 154, 156, 159, 161, 165, 168, 169, 170, 182, 183, 197, 199, and 203 of a light chain of the antibody or antibody fragment, wherein the positions are numbered according to the EU system, and wherein the light chain is a human kappa light chain. In certain embodiments a modified antibody or antibody fragment thereof comprises a combination of substitution of two or more amino acids with cysteine on its constant regions wherein the combinations comprise substitutions at positions 375 of an antibody heavy chain, position 152 of an antibody heavy chain, position 360 of an antibody heavy chain, or position 107 of an antibody light chain and wherein the positions are numbered according to the EU system. In certain embodiments a modified antibody or antibody fragment thereof comprises a substitution of one amino acid with cysteine on its constant regions wherein the substitution is position 375 of an antibody heavy chain, position 152 of an antibody heavy chain, position 360 of an antibody heavy chain, position 107 of an antibody light chain, position 165 of an antibody light chain or position 159 of an antibody light chain and wherein the positions are numbered according to the EU system, and wherein the light chain is a kappa chain. In particular embodiments, a modified antibody or antibody fragment thereof comprises a combination of substitution of two amino acids with cysteine on its constant regions wherein the combinations comprise substitutions at positions 375 of an antibody heavy chain and position 152 of an antibody heavy chain, wherein the positions are numbered according to the EU system. In particular embodiments a modified antibody or antibody fragment thereof comprises a substitution of one amino acid with cysteine at position 360 of an antibody heavy chain, wherein the positions are numbered according to the EU system. In other particular embodiments a modified antibody or antibody fragment thereof comprises a substitution of one amino acid with cysteine at position 107 of an antibody light chain and wherein the positions are numbered according to the EU system, and wherein the light chain is a kappa chain.

In additional embodiments antibodies or antibody fragments (*e.g*., antigen binding fragment) useful in immunoconjugates of the disclosure include modified or engineered antibodies, such as an antibody modified to introduce one or more other reactive amino acid (other than cysteine), including Pcl, pyrrolysine, peptide tags (such as S6, A1 and ybbR tags), and non-natural amino acids, in place of at least one amino acid of the native sequence, thus providing a reactive site on the antibody or antigen binding fragment for conjugation to a drug moiety or a linker-drug moiety with complementary reactivity. For example, the antibodies or antibody fragments can be modified to incorporate Pcl or pyrrolysine (W. Ou, et al., (2011) PNAS 108 (26), 10437-10442; WO 2014/124258) or unnatural amino acids (J.Y. Axup, et al., Proc Natl Acad Sci U S A, 109 (2012), pp. 16101-16106; for review, see C.C. Liu and P.G. Schultz (2010) Annu Rev Biochem 79, 413-444; C.H. Kim, et al., (2013) Curr Opin Chem Biol. 17, 412-419) as sites for conjugation to a drug. Similarly, peptide tags for enzymatic conjugation methods can be introduced into an antibody (Strop P., et al., Chem Biol. 2013, 20(2):161-7; Rabuka D., Curr Opin Chem Biol. 2010 Dec;14(6):790-6; Rabuka D, et al., Nat Protoc. 2012, 7(6):1052-67). One other example is the use of 4'-phosphopantetheinyl transferases (PPTase) for the conjugation of Co-enzyme A analogs (WO 2013/184514), and (Grünewald et al., (2015) Bioconjugate Chem. 26 (12), 2554-62). Methods for conjugating such modified or engineered antibodies with payloads or linker-payload combinations are known in the art.

In another embodiment, the Fc hinge region of an antibody is mutated to decrease the biological half-life of the antibody. More specifically, one or more amino acid mutations are introduced into the CH2-CH3 domain interface region of the Fc-hinge fragment such that the antibody has impaired Staphylococcyl protein A (SpA) binding relative to native Fc-hinge domain SpA binding. This approach is described in further detail in U.S. Patent No. 6,165,745.

In yet other embodiments, the Fc region is altered by replacing at least one amino acid residue with a different amino acid residue to alter the effector functions of the antibody. For example, one or more amino acids can be replaced with a different amino acid residue such that the antibody has an altered affinity for an effector ligand but retains the antigen-binding ability of the parent antibody. The effector ligand to which affinity is altered can be, for example, an Fc receptor or the C1 component of complement. This approach is described in, *e.g.*, U.S. Patent Nos. 5,624,821 and 5,648,260.

In another embodiment, one or more amino acids selected from amino acid residues can be replaced with a different amino acid residue such that the antibody has altered C1q binding and/or reduced or abolished complement dependent cytotoxicity (CDC). This approach is described in, *e.g.*, U.S. PatentNos. 6,194,551.

In another embodiment, one or more amino acid residues are altered to thereby alter the ability of the antibody to fix complement. This approach is described in, *e.g.*, the PCT Publication WO 94/29351. Allotypic amino acid residues include, but are not limited to, constant region of a heavy chain of the IgG1, IgG2, and IgG3 subclasses as well as constant region of a light chain of the kappa isotype as described by Jefferis et al., MAbs. 1:332-338 (2009).

Antibody fusion protein complexes containing such mutations mediate reduced or no antibody-dependent cellular cytotoxicity (ADCC) or complement-dependent cytotoxicity (CDC). In some embodiments, amino acid residues L234 and L235 of the IgG1 constant region are substituted to A234 and A235. In some embodiments, amino acid residue N267 of the IgG1 constant region is substituted to A267. In some embodiments, amino acid residues D265 and P329 of the IgG1 constant region are substituted to A265 and A329. Other antibody Fc silencing mutations may also be used.

In another embodiment, one or more amino acid residues are altered to thereby alter the ability of the antibody to fix complement. This approach is described in, *e.g.*, the PCT Publication WO 94/29351 by Bodmer et al. In a specific embodiment, one or more amino acids of an antibody or antigen binding fragment thereof of the disclosure are replaced by one or more allotypic amino acid residues. Allotypic amino acid residues also include, but are not limited to, the constant region of the heavy chain of the IgG1, IgG2, and IgG3 subclasses as well as the constant region of the light chain of the kappa isotype as described by Jefferis et al., MAbs. 1:332-338 (2009).

In still another embodiment, the glycosylation of an antibody is modified. For example, an aglycosylated antibody can be made (*i.e.*, the antibody lacks glycosylation). Glycosylation can be altered to, for example, increase the affinity of the antibody for "antigen." Such carbohydrate modifications can be accomplished by, for example, altering one or more sites of glycosylation within the antibody sequence. For example, one or more amino acid substitutions can be made that result in elimination of one or more variable region framework glycosylation sites to thereby eliminate glycosylation at that site. Such aglycosylation may increase the affinity of the antibody for antigen. Such an approach is described in, *e.g.*, U.S. Patent Nos. 5,714,350 and 6,350,861.

In some embodiments, the antibody is modified to increase its biological half-life. Various approaches are possible. For example, one or more of the following mutations can be introduced: T252L, T254S, T256F, as described in U.S. Patent No. 6,277,375. Alternatively, to increase the biological half-life, the antibody can be altered within the CH1 or CL region to contain a salvage receptor binding epitope taken from two loops of a CH2 domain of an Fc region of an IgG, as described in U.S. Patent Nos. 5,869,046 and 6,121,022.

### 3. Production of the anti-PMEL17 Antibodies

Anti-PMEL17 antibodies and antibody fragments (*e.g*., antigen binding fragments) thereof can be produced by any means known in the art, including but not limited to, recombinant expression, chemical synthesis, and enzymatic digestion of antibody tetramers, whereas full-length monoclonal antibodies can be obtained by, *e.g*., hybridoma or recombinant production. Recombinant expression can be from any appropriate host cells known in the art, for example, mammalian host cells, bacterial host cells, yeast host cells, insect host cells, etc.

The disclosure further provides polynucleotides encoding the antibodies described herein, *e.g*., polynucleotides encoding heavy or light chain variable regions or segments comprising the complementarity determining regions as described herein. In some embodiments, the polynucleotide encoding the heavy chain variable regions has at least 85%, 89%, 90%, 91%, 92%, 93%, 94%, 95%, 96%, 97%, 98%, 99%, or 100% nucleic acid sequence identity with a polynucleotide selected from the group consisting of SEQ ID NOs: 11, 43, 65, 89, 113, 133, 150, 166, 185, 197, 216, 228, 240, and 255. In some embodiments, the polynucleotide encoding the light chain variable regions has at least 85%, 89%, 90%, 91%, 92%, 93%, 94%, 95%, 96%, 97%, 98%, 99%, or 100% nucleic acid sequence identity with a polynucleotide selected from the group consisting of SEQ ID NOs: 22, 26, 30, 54, 76, 100, 120, 144, 160, 172, 191, 203, 222, 234, 249, and 261.

In some embodiments, the polynucleotide encoding the heavy chain has at least 85%, 89%, 90%, 91%, 92%, 93%, 94%, 95%, 96%, 97%, 98%, 99%, or 100% nucleic acid sequence identity with a polynucleotide of SEQ ID NO: 13, 45, 67, 91, 115, 135, 152, 168, 187, 199, 218, 230, 242, 257, 319, and 320. In some embodiments, the polynucleotide encoding the light chain has at least 85%, 89%, 90%, 91%, 92%, 93%, 94%, 95%, 96%, 97%, 98%, 99%, or 100% nucleic acid sequence identity with a polynucleotide of SEQ ID NO: 24, 28, 32, 56, 78, 102, 122, 146, 162, 174, 193, 205, 224, 236, 251, and 263.

The polynucleotides of the disclosure can encode only the variable region sequence of an anti-PMEL17 antibody. They can also encode both a variable region and a constant region of the antibody. Some of the polynucleotide sequences encode a polypeptide that comprises variable regions of both the heavy chain and the light chain of one of the exemplified mouse anti-PMEL17 antibody. Some other polynucleotides encode two polypeptide segments that respectively are substantially identical to the variable regions of the heavy chain and the light chain of one of the mouse antibodies.

The polynucleotide sequences can be produced by *de novo* solid-phase DNA synthesis or by PCR mutagenesis of an existing sequence (*e.g*., sequences as described in the Examples below) encoding an anti-PMEL17 antibody or its binding fragment. Direct chemical synthesis of nucleic acids can be accomplished by methods known in the art, such as the phosphotriester method of Narang et al., Meth. Enzymol. 68:90, 1979; the phosphodiester method of Brown et al., Meth. Enzymol. 68:109, 1979; the diethylphosphoramidite method of Beaucage et al., Tetra. Lett., 22:1859, 1981; and the solid support method of U.S. Patent No. 4,458,066. Introducing mutations to a polynucleotide sequence by PCR can be performed as described in, *e.g.,* PCR Technology: Principles and Applications for DNA Amplification, H.A. Erlich (Ed.), Freeman Press, NY, NY, 1992; PCR Protocols: A Guide to Methods and Applications, Innis et al. (Ed.), Academic Press, San Diego, CA, 1990; Mattila et al., Nucleic Acids Res. 19:967, 1991; and Eckert et al., PCR Methods and Applications 1:17, 1991.

Also provided in the disclosure are expression vectors and host cells for producing the anti-PMEL17 antibodies described above. Various expression vectors can be employed to express the polynucleotides encoding the anti-PMEL17 antibody chains or binding fragments. Both viral-based and nonviral expression vectors can be used to produce the antibodies in a mammalian host cell. Nonviral vectors and systems include plasmids, episomal vectors, typically with an expression cassette for expressing a protein or RNA, and human artificial chromosomes (*see, e.g.*, Harrington et al., Nat Genet 15:345, 1997). For example, nonviral vectors useful for expression of the anti-PMEL17 polynucleotides and polypeptides in mammalian (*e.g*., human) cells include pThioHis A, B & C, pcDNA^{™}3.1/His, pEBVHis A, B, & C (Invitrogen, San Diego, CA), MPSV vectors, and numerous other vectors known in the art for expressing other proteins. Useful viral vectors include vectors based on retroviruses, adenoviruses, adeno-associated viruses, herpes viruses, vectors based on SV40, papilloma virus, HBP Epstein Barr virus, vaccinia virus vectors and Semliki Forest virus (SFV). *See* Brent *et al.*, *supra,* Smith, Annu. Rev. Microbiol. 49:807, 1995; and Rosenfeld et al., Cell 68:143, 1992.

The choice of expression vector depends on the intended host cells in which the vector is to be expressed. Typically, the expression vectors contain a promoter and other regulatory sequences (*e.g*., enhancers) that are operably linked to the polynucleotides encoding an anti-PMEL17 antibody chain or fragment. In some embodiments, an inducible promoter is employed to prevent expression of inserted sequences except under inducing conditions. Inducible promoters include, *e.g*., arabinose, lacZ, metallothionein promoter or a heat shock promoter. Cultures of transformed organisms can be expanded under noninducing conditions without biasing the population for coding sequences whose expression products are better tolerated by the host cells. In addition to promoters, other regulatory elements may also be required or desired for efficient expression of an anti-PMEL17 antibody chain or fragment. These elements typically include an ATG initiation codon and adjacent ribosome binding site or other sequences. In addition, the efficiency of expression may be enhanced by the inclusion of enhancers appropriate to the cell system in use (*see, e.g.*, Scharf et al., Results Probl. Cell Differ. 20:125, 1994; and Bittner et al., Meth. Enzymol., 153:516, 1987). For example, the SV40 enhancer or CMV enhancer may be used to increase expression in mammalian host cells.

The expression vectors may also provide a secretion signal sequence position to form a fusion protein with polypeptides encoded by inserted anti-PMEL17 antibody sequences. More often, the inserted anti-PMEL17 antibody sequences are linked to a signal sequences before inclusion in the vector. Vectors to be used to receive sequences encoding anti-PMEL17 antibody light and heavy chain variable domains sometimes also encode constant regions or parts thereof. Such vectors allow expression of the variable regions as fusion proteins with the constant regions thereby leading to production of intact antibodies or fragments thereof. Typically, such constant regions are human.

The host cells for harboring and expressing the anti-PMEL17 antibody chains can be either prokaryotic or eukaryotic. *E. coli* is one prokaryotic host useful for cloning and expressing the polynucleotides of the disclosure. Other microbial hosts suitable for use include bacilli, such as Bacillus subtilis, and other enterobacteriaceae, such as Salmonella, Serratia, and various Pseudomonas species. In these prokaryotic hosts, one can also make expression vectors, which typically contain expression control sequences compatible with the host cell (*e.g*., an origin of replication). In addition, any number of a variety of well-known promoters will be present, such as the lactose promoter system, a tryptophan (trp) promoter system, a beta-lactamase promoter system, or a promoter system from phage lambda. The promoters typically control expression, optionally with an operator sequence, and have ribosome binding site sequences and the like, for initiating and completing transcription and translation. Other microbes, such as yeast, can also be employed to express anti-PMEL17 polypeptides of the disclosure. Insect cells in combination with baculovirus vectors can also be used.

In some embodiments, mammalian host cells are used to express and produce the anti-PMEL17 polypeptides of the disclosed. For example, they can be either a hybridoma cell line expressing endogenous immunoglobulin genes (*e.g*., the myeloma hybridoma clones as described in the Examples) or a mammalian cell line harboring an exogenous expression vector (*e.g*., the SP2/0 myeloma cells exemplified below). These include any normal mortal or normal or abnormal immortal animal or human cell. For example, a number of suitable host cell lines capable of secreting intact immunoglobulins have been developed, including the CHO cell lines, various Cos cell lines, HeLa cells, myeloma cell lines, transformed B-cells and hybridomas. The use of mammalian tissue cell culture to express polypeptides is discussed generally in, *e.g*., Winnacker, From Genes to Clones, VCH Publishers, N.Y., N.Y., 1987. Expression vectors for mammalian host cells can include expression control sequences, such as an origin of replication, a promoter, and an enhancer (*see, e.g.*, Queen et al., Immunol. Rev. 89:49-68, 1986), and necessary processing information sites, such as ribosome binding sites, RNA splice sites, polyadenylation sites, and transcriptional terminator sequences. These expression vectors usually contain promoters derived from mammalian genes or from mammalian viruses. Suitable promoters may be constitutive, cell type-specific, stage-specific, and/or modulatable or regulatable. Useful promoters include, but are not limited to, the metallothionein promoter, the constitutive adenovirus major late promoter, the dexamethasone-inducible MMTV promoter, the SV40 promoter, the MRP polIII promoter, the constitutive MPSV promoter, the tetracycline-inducible CMV promoter (such as the human immediate-early CMV promoter), the constitutive CMV promoter, and promoter-enhancer combinations known in the art.

Methods for introducing expression vectors containing the polynucleotide sequences of interest vary depending on the type of cellular host. For example, calcium chloride transfection is commonly utilized for prokaryotic cells, whereas calcium phosphate treatment or electroporation may be used for other cellular hosts (*see generally* Sambrook et al., Molecular Cloning: A Laboratory Manual, 4th ed.). Other methods include, *e.g*., electroporation, calcium phosphate treatment, liposome-mediated transformation, injection and microinjection, ballistic methods, virosomes, immunoliposomes, polycation:nucleic acid conjugates, naked DNA, artificial virions, fusion to the herpes virus structural protein VP22 (Elliot and O'Hare, Cell 88:223, 1997), agent-enhanced uptake of DNA, and *ex vivo* transduction. For long-term, high-yield production of recombinant proteins, stable expression will often be desired. For example, cell lines which stably express anti-PMEL17 antibody chains or binding fragments can be prepared using expression vectors of the disclosure which contain viral origins of replication or endogenous expression elements and a selectable marker gene. Following introduction of the vector, cells may be allowed to grow for 1-2 days in an enriched media before they are switched to selective media. The purpose of the selectable marker is to confer resistance to selection, and its presence allows growth of cells which successfully express the introduced sequences in selective media. Resistant, stably transfected cells can be proliferated using tissue culture techniques appropriate to the cell type.

Process for the production of anti-PMEL antibody drug conjugates are also described in Example 4 of International Application Publication No. WO 2020/128612, which is incorporated by reference in its entirety. The PK properties of exemplary ADCs are also described in Example 18 of International Application Publication No. WO 2020/128612, which is incorporated by reference in its entirety. The *in vitro* stability of anti-PMEL17-GNAQ/11i ADCs in buffer, mouse, rat, and human plasma and *in vivo* stability of anti-PMEL17-GNAQ/11i ADCs in mouse are also described in Example 19 of International Application Publication No. WO 2020/128612, which is incorporated by reference in its entirety.

### Therapeutic Uses

The antibody drug conjugates or formulations of the disclosure are useful in a variety of applications including, but not limited to, treatment or prevention of cancer, such as solid cancers or heme malignancies. In certain embodiments, the antibody drug conjugates or formulations of the disclosure are useful for inhibiting tumor growth, inducing differentiation, reducing tumor volume, and/or reducing the tumorigenicity of a tumor. The methods of use can be *in vitro*, *ex vivo*, or *in vivo* methods.

In some embodiments, the disclosure provides a method of treating or preventing a disease comprising administering the antibody drug conjugates or formulations of the disclosure to a patient. The disclosure also provides use of the antibody drug conjugates or formulations of the disclosure to treat or prevent disease in a patient. In some embodiments, the disclosure provides antibody drug conjugates or formulations of the disclosure for use in the treatment or prevention of disease in a patient. In further embodiments, the disclosure provides use of the antibody drug conjugates or formulations of the disclosure in the manufacture of a medicament for treatment or prevention of disease in a patient.

In certain embodiments, the disease treated with the antibody drug conjugates or formulations of the disclosure is a cancer. In certain embodiments, the cancer is characterized by PMEL17 expressing cells to which the antibody drug conjugates of the disclosure binds. In certain embodiments, the cancer is characterized by an increase in expression of PMEL17 relative to a healthy patient. In some embodiments, the expression of PMEL17 may be measured by an increase in PMEL17 RNA. In other embodiments, the cancer is characterized by an increase in DNA copy number of PMEL17. Other methods of measuring or determining levels of PMEL17 expression are known to persons skilled in the art. In certain embodiments, the cancer is characterized by a mutation, *e.g*., an activating mutation affecting Q209 or R183, in the GNAQ and/or the GNA11 gene. Examples of diseases which can be treated and/or prevented include, but are not limited to, carcinomas (*e.g*., hepatocellular carcinoma), sarcomas, leukemias, lymphomas, eye cancers, eye neoplasms, melanomas (*e.g*., uveal melanoma, non-uveal melanoma, malignant melanoma, ocular melanoma, subcutaneous melanoma, cutaneous melanoma, or mucosal melanoma), or metastatic cancer thereof. In some embodiments, the disease is a melanoma. In some embodiments, the melanoma is a non-uveal melanoma that contains a mutation of GNAQ, GNA11, or both. Without wishing to be bound by theory, it is believed that in some embodiments, non-uveal melanomas harboring GNAQ/11 mutations may be biologically more related to uveal melanoma than melanomas harboring other mutations (*e.g*., BRAF, NRAS). In some embodiments, the melanoma does not harbor a BRAF mutation, an NRAS mutation, or both. In some embodiments, the melanoma is mucosal melanoma that contains a mutation of GNAQ, GNA11, or both.

The disclosure provides for methods of treating or preventing a cancer comprising administering a therapeutically effective amount of the antibody drug conjugates or formulations of the disclosure. In certain embodiments, the cancer is a solid cancer such as carcinoma, hepatocellular carcinoma, sarcoma, lymphoma, eye cancer, eye neoplasm, melanoma, uveal melanoma, non-uveal melanoma, malignant melanoma, ocular melanoma, subcutaneous melanoma, cutaneous melanoma, mucosal melanoma, or a metastatic cancer thereof. In certain embodiments, the subject is a human. In certain embodiments, the cancer is a resistant cancer and/or relapsed cancer.

In certain embodiments, the disclosure provides for methods of inhibiting tumor growth comprising administering to a subject a therapeutically effective amount of the antibody drug conjugates or formulations of the disclosure. In certain embodiments, the tumor is of a solid cancer such as carcinoma, hepatocellular carcinoma, sarcoma, lymphoma, eye cancer, eye neoplasm, melanoma, uveal melanoma, non-uveal melanoma, malignant melanoma, ocular melanoma, subcutaneous melanoma, cutaneous melanoma, mucosal melanoma, or a metastatic cancer thereof. In certain embodiments, the subject is a human. In certain embodiments, the subject has a tumor or has had a tumor removed.

In certain embodiments, the tumor expresses the PMEL17 to which the antibody drug conjugate binds. In certain embodiments, the tumor overexpresses the human PMEL17. In certain embodiments, the tumor has an increase copy number of the PMEL17 gene. In certain embodiments, the tumor is characterized by a mutation, *e.g*., an activating mutation affecting Q209 or R183, in the GNAQ and/or the GNA11 gene.

The current disclosure also provides for methods of selecting patients for treatment with antibody drug conjugates or formulation of the disclosure comprising administering a therapeutically effective amount of said antibody drug conjugates or formulations. In some embodiments of the disclosure the methods comprise selecting a patient by measuring for expression of PMEL17. In some embodiments of the disclosure the methods comprise selecting a patient by identifying a mutation, *e.g*., an activating mutation affecting Q209 or R183, in the GNAQ or the GNA11 gene. In certain embodiments, the methods comprise measuring the level of PMEL17 expression in the patient as well as detecting for the GNAQ and/or GNA11 gene.

In certain embodiments, the subject or patient of any of the methods described herein has been treated with a bispecific gp100 peptide-HLA-directed CD3 T cell engager, *e.g*., tebentafusp, prior to administration of the antibody drug conjugate or formulation of the disclosure. In other embodiments, the subject or patient of any of the methods described herein has not been treated with a bispecific gp100 peptide-HLA-directed CD3 T cell engager, *e.g*., tebentafusp, prior to administration of the antibody drug conjugate or formulation of the disclosure.

In some embodiments, the subject or patient of any of the methods described herein has a uveal melanoma (*e.g*., a metastatic uveal melanoma) and has been treated with a bispecific gp100 peptide-HLA-directed CD3 T cell engager, *e.g*., tebentafusp, prior to administration of the antibody drug conjugate or formulation of the disclosure. In some embodiments, the subject or patient of any of the methods described herein has a uveal melanoma (*e.g*., a metastatic uveal melanoma) and has not been treated with a bispecific gp100 peptide-HLA-directed CD3 T cell engager, *e.g*., tebentafusp, prior to administration of the antibody drug conjugate or formulation of the disclosure. Without wishing to be bound by theory, it is believed that in some embodiments, certain tebentafusp-treated patients may have up to a 50% decrease in PMEL17 expression.

For the treatment or prevention of the disease, the appropriate dosage of the antibody drug conjugates or formulations of the disclosure depends on various factors, such as the type of disease to be treated, the severity and course of the disease, the responsiveness of the disease, previous therapy, patient's clinical history, and so on. The antibody-drug conjugate or formulation can be administered one time or over a series of treatments lasting from several days to several months, or until a cure is effected or a diminution of the disease state is achieved (*e.g*., reduction in tumor size). Optimal dosing schedules can be calculated from measurements of drug accumulation in the body of the patient and will vary depending on the relative potency of antibody drug conjugates or formulations. The treating physician can estimate repetition rates for dosing based on measured residence times and concentrations of the drug in bodily fluids or tissues.

The methods described herein can further comprise determining the expression of one or more biomarkers in a sample from the subject or patient. Exemplary biomarkers that can be used in such methods include, but are not limited to, premelanosome protein 17 (PMEL17), phosphorylated ERK (pERK), cluster of differentiation 8 (CD8), programmed death-ligand 1 (PD-L1), Dual specificity phosphatase 6 (DUSP6), Ras guanyl-releasing protein 3 (RASGRP3), or any combination thereof.

In some embodiments, the sample is a tumor sample. In some embodiments, the sample is a sample from tissue adjacent to the tumor. In some embodiments, the sample is a body fluid sample, *e.g.,* blood, spinal fluid, etc. In some embodiments, the sample comprises cell-free DNA (cfDNA). In some embodiments, the sample is a non-diseased tissue to be used as a reference.

In some embodiments, the biomarkers are measured by methods known in the art. For example, expression of biomarkers can be measured and/or monitored by quantifying nucleic acid samples from the tissues or fluid samples from the subject or patient. Typical methods of quantifying include polymerase chain reaction (PCR), real-time PCR, Southern blotting, Northern blotting, in situ hybridization, DNA sequencing, and/or whole transcriptome analysis. In another example, expression of biomarkers can be measured and/or monitored by measuring protein levels of one or more biomarkers by techniques such as Western blotting, enzyme-linked immunosorbent assay (ELISA), and/or mass spectrometry.

The *in vitro* and *in vivo* anti-cancer activities of the GNAQ/11 inhibitors described herein and the antibody drug conjugates described herein are also described in Examples of International Application Publication No. WO 2020/128612, which is incorporated by reference in its entirety.

### Methods of Treating or Diagnosing Cancer

In certain instances, an antibody drug conjugate or formulation of the present disclosure is used in a method of treating a cancer.

In one aspect, the disclosure relates to treatment of a subject *in vivo* using an ADC described herein, or a formulation comprising an ADC described herein, such that growth of cancerous tumors is inhibited or reduced.

In certain embodiments, the ADC or formulation is administered or used in accordance with a dosage regimen disclosed herein. In certain embodiments, the the ADC or formulation is administered in an amount effective to treat a cancer or a symptom thereof.

The ADC or formulation described herein can be used alone to inhibit the growth of cancerous tumors. Alternatively, the ADC or formulation described herein can be used in combination with a second therapeutic agent or modality as described herein. The ADC or formulation, and the second therapeutic agent or modality, can be administered in either order or simultaneously.

Accordingly, in one aspect, the disclosure provides a method of inhibiting growth of tumor cells in a subject, comprising administering to the subject a therapeutically effective amount of an ADC or formulation described herein, *e.g*., in accordance with a dosage regimen described herein. In an embodiment, the ADC is administered in the form of a formulation described herein.

In another aspect, a method of treating a subject, *e.g*., reducing or ameliorating, a hyperproliferative condition or disorder (*e.g*., a cancer or a metastatic lesion thereof) in a subject is provided. The method includes administering to the subject an ADC described herein, or a formulation comprising an ADC described herein, in accordance with a dosage regimen disclosed herein.

In certain embodiments, the cancer expresses PMEL 17, contains a mutation of the GNAQ or GNA11 gene, or the melanoma expresses PMEL17 and contains a mutation of GNAQ, GNA11, or both. Exemplary cancers include, but are not limited to, solid tumors, hematological cancers, soft tissue tumors, and metastatic lesions. In an embodiment, the cancer is a melanoma. In certain embodiments, the melanoma is a uveal melanoma, non-uveal melanoma, malignant melanoma, ocular melanoma, subcutaneous melanoma, cutaneous melanoma, or mucosal melanoma. In certain embodiments, the cancer is a carcinoma, a sarcoma, a leukemia, or a lymphoma. In an embodiment, the carcinoma is a hepatocellular carcinoma. In certain embodiments, the cancer is an eye cancer or neoplasm. In some embodiments, the cancer is an MSI-high cancer. In some embodiments, the cancer is a metastatic cancer. In other embodiments, the cancer is an advanced cancer. In other embodiments, the cancer is a relapsed or refractory cancer.

Methods, ADCs, compositions, and formulations disclosed herein are useful for treating metastatic lesions associated with the aforementioned cancers.

In some embodiments, the ADC is administered at a dose of 1 mg/kg to 20 mg/kg (*e.g*., 1 mg/kg to 16 mg/kg or 2 mg/kg to 15 mg/kg, *e.g*., once a week, once every two weeks, or once every four weeks, intravenously. In some embodiments, the ADC is administered at a dose of 1 mg/kg, 2 mg/kg, 4 mg/kg, 8 mg/kg, 12 mg/kg, or 16 mg/kg), *e.g*., once a week, once every two weeks, or once every four weeks, intravenously.

In an embodiment, the subject has been treated with tebentafusp prior to the administration of the ADC. In an embodiment, the subject has not been treated with tebentafusp prior to the administration of the ADC.

In some embodiments, the method further comprises determining the expression of one or more biomarkers in a sample from the subject. Such methods can be used to evaluate (*e.g*., monitor) a cancer treatment comprising an ADC or formulation described herein, to evaluate (*e.g*., monitor) a disease described herein (*e.g*., the progression of a disease described herein), to select a treatment comprising an ADC or formulation described herein for a subject having a cancer, and/or to select a subject for a cancer treatment comprising an ADC or formulation described herein. In some embodiments, based on the determination of the expression of the one or more biomarkers, the treatment can be initiated, continued, or discontinued.

Exemplary biomarkers include, but are not limited to, PMEL17, pERK, CD8, PD-L1, DUSP6, RASGRP3, or any combination thereof. Exemplary samples include, but are not limited to, tumor samples, tumor-adjacent tissues, bodily fluids (*e.g*., blood, serum, spinal fluid, or urine). In some embodiments, the sample comprises cell-free DNA (cfDNA). In some embodiments, the sample is a non-diseased tissue to be used as a reference. The biomarkers can be measured by methods known in the art, *e.g*., by measuring the nucleic acid and/or protein levels in a sample.

### Combination Therapy

In certain instances, an antibody drug conjugate or formulation of the present disclosure is combined with other therapeutic treatments, such as surgery and radiation therapy, therapeutic agents, such as other anti-cancer agents, anti-allergic agents, anti-nausea agents (or anti-emetics), pain relievers, cytoprotective agents, and combinations thereof.

In some embodiments, an antibody drug conjugate or formulation of the present disclosure is combined in a pharmaceutical combination formulation, or dosing regimen as combination therapy, with a second compound having anti-cancer properties. The second compound of the pharmaceutical combination formulation or dosing regimen can have complementary activities to the antibody or immunoconjugate of the combination such that they do not adversely affect each other. For example, an antibody drug conjugate or formulation of the present disclosure can be administered in combination with, but not limited to, a chemotherapeutic agent, immunomodulatory agents, a tyrosine kinase inhibitor, a GNAQ/GNA11 downstream signaling pathway inhibitor, IAP inhibitors, Bcl2 inhibitors, Mcl1 inhibitors, and other GNAQ/GNA11 inhibitors.

The term "pharmaceutical combination" as used herein refers to either a fixed combination in one dosage unit form, or non-fixed combination or a kit of parts for the combined administration where two or more therapeutic agents may be administered independently at the same time or separately within time intervals, especially where these time intervals allow that the combination partners show a cooperative, *e.g*., synergistic effect.

The term "combination therapy" refers to the administration of two or more therapeutic agents to treat or prevent a therapeutic condition or disorder described in the present disclosure. Such administration encompasses co-administration of these therapeutic agents in a substantially simultaneous manner, such as in a single capsule having a fixed ratio of active ingredients. Alternatively, such administration encompasses co-administration in multiple, or in separate containers (*e.g*., capsules, powders, and liquids) for each active ingredient. Powders and/or liquids may be reconstituted or diluted to a desired dose prior to administration. In addition, such administration also encompasses use of each type of therapeutic agent in a sequential manner, either at approximately the same time or at different times. In either case, the treatment regimen will provide beneficial effects of the drug combination in treating or preventing the conditions or disorders described herein.

The combination therapy can provide "synergy" and prove "synergistic", *i.e.*, the effect achieved when the active ingredients used together is greater than the sum of the effects that results from using the compounds separately. A synergistic effect can be attained when the active ingredients are: (1) co-formulated and administered or delivered simultaneously in a combined, unit dosage formulation; (2) delivered by alternation or in parallel as separate formulations; or (3) by some other regimen. When delivered in alternation therapy, a synergistic effect can be attained when the compounds are administered or delivered sequentially, *e.g*., by different injections in separate syringes. In general, during alternation therapy, an effective dosage of each active ingredient is administered sequentially, *i.e.*, serially, whereas in combination therapy, effective dosages of two or more active ingredients are administered together.

General Chemotherapeutic agents considered for use in combination therapies include anastrozole (Arimidex^{®}), bicalutamide (Casodex^{®}), bleomycin sulfate (Blenoxane^{®}), busulfan (Myleran^{®}), busulfan injection (Busulfex^{®}), capecitabine (Xeloda^{®}), N4-pentoxycarbonyl-5-deoxy-5-fluorocyridine, carboplatin (Paraplatin^{®}), carmustine (BiCNU^{®}), chlorambucil (Leukeran^{®}), cisplatin (Platinol^{®}), cladribine (Leustatin^{®}), cyclophosphamide (Cytoxan^{®} or Neosar^{®}), cytarabine, cytosine arabinoside (Cytosar-U^{®}), cytarabine liposome injection (DepoCyt^{®}), dacarbazine (DTIC-Dome^{®}), dactinomycin (Actinomycin D, Cosmegan), daunorubicin hydrochloride (Cerubidine^{®}), daunorubicin citrate liposome injection (DaunoXome^{®}), dexamethasone, docetaxel (Taxotere^{®}), doxorubicin hydrochloride (Adriamycin^{®}, Rubex^{®}), etoposide (Vepesid^{®}), fludarabine phosphate (Fludara^{®}), 5-fluorouracil (Adrucil^{®}, Efudex^{®}), flutamide (Eulexin^{®}), tezaciribine, Gemcitabine (difluorodeoxycitidine), hydroxyurea (Hydrea^{®}), Idarubicin (Idamycin^{®}), ifosfamide (IFEX^{®}), irinotecan (Camptosar^{®}), L-asparaginase (ELSPAR^{®}), leucovorin calcium, melphalan (Alkeran^{®}), 6-mercaptopurine (Purinethol^{®}), methotrexate (Folex^{®}), mitoxantrone (Novantrone^{®}), mylotarg, paclitaxel (Taxol^{®}), phoenix (Yttrium90/MX-DTPA), pentostatin, polifeprosan 20 with carmustine implant (Gliadel^{®}), tamoxifen citrate (Nolvadex^{®}), teniposide (Vumon^{®}), 6-thioguanine, thiotepa, tirapazamine (Tirazone^{®}), topotecan hydrochloride for injection (Hycamptin^{®}), vinblastine (Velban^{®}), vincristine (Oncovin^{®}), and vinorelbine (Navelbine^{®}), and pemetrexed.

In some embodiments, the present disclosure provides a method of treating or preventing cancer by administering to a subject in need thereof an antibody drug conjugate of the present disclosure in combination with one or more MDM2 inhibitors, PKC inhibitors, PRC2 inhibitors, MAPK inhibitors, GPCR inhibitors, tyrosine kinase inhibitors, including but not limited to, BTK inhibitors, EGFR inhibitors, Her2 inhibitors, Her3 inhibitors, IGFR inhibitors, and Met inhibitors.

For example, MDM2 inhibitors include but are not limited to, RG7112 (RO5045337); RG7388 (RO5503781, Idasanutlin); MI-77301 (SAR405838); MK-8242 (SCH-900242); AMG232; CGM097; DS3032b; HDM201; and ALRN-6924.

For example, PKC inhibitors include but are not limited to, Balanol; Riluzole; Staurosporin; Enzastaurin; δV1-1 (KAI-9803 or Delcasertib); εV1-2 (KAI-1678); Aprinocarsen; Midostaurin (PKC412); UCN-01 (7-hydroxy-staurosporin); Rottlerin (5, 7, dihydroxy-2,2-dimethyl-6-(2,4,6-trihydroxy-3-methyl-5-acetlybenzyl)-8-cinnamoyl-1,2-chromene); and Bryostatin 1.

For example, PRC2 inhibitors include but are not limited to, EI1; EPZ011989; EPZ005687; Tetramethylpiperidinyl Benzamides; UNC1999; and GSK126.

For example, MAPK inhibitors include but are not limited to, Vemurafenib (Zelboraf);, dabrafenib (Tafinlar); encorafenib (Braftovi); trametinib (Mekinist); cobimetinib (Cotellic); binimerinib (Mektovi); and ulixertinib.

For example, tyrosine kinase inhibitors include but are not limited to, Ibrutinib (PCI-32765); Erlotinib hydrochloride (Tarceva^{®}); Linifanib (N-[4-(3-amino-1H-indazol-4-yl)phenyl]-N'-(2-fluoro-5-methylphenyl)urea, also known as ABT 869, available from Genentech); Sunitinib malate (Sutent^{®}); Bosutinib (4-[(2,4-dichloro-5-methoxyphenyl)amino]-6-methoxy-7-[3-(4-methylpiperazin-1-yl)propoxy]quinoline-3-carbonitrile, also known as SKI-606, and described in US Patent No. 6,780,996); Dasatinib (Sprycel^{®}); Pazopanib (Votrient^{®}); Sorafenib (Nexavar^{®}); Zactima (ZD6474); and Imatinib or Imatinib mesylate (Gilvec^{®} and Gleevec^{®}).

Epidermal growth factor receptor (EGFR) inhibitors include but are not limited to, Erlotinib hydrochloride (Tarceva^{®}), Gefitinib (Iressa^{®}); N-[4-[(3-Chloro-4-fluorophenyl)amino]-7-[[(3"S")-tetrahydro-3-furanyl]oxy]-6-quinazolinyl]-4(dimethylamino)-2-butenamide, Tovok^{®}); Vandetanib (Caprelsa^{®}); Lapatinib (Tykerb^{®}); (3R,4R)-4-Amino-1-((4-((3-methoxyphenyl)amino)pyrrolo[2,1-f][1,2,4]triazin-5-yl)methyl)piperidin-3-ol (BMS690514); Canertinib dihydrochloride (CI-1033); 6-[4-[(4-Ethyl-1-piperazinyl)methyl]phenyl]-N-[(1R)-1-phenylethyl]- 7H-Pyrrolo[2,3-d]pyrimidin-4-amine (AEE788, CAS 497839-62-0); Mubritinib (TAK165); Pelitinib (EKB569); Afatinib (BIBW2992); Neratinib (HKI-272); N-[4-[[1-[(3-Fluorophenyl)methyl]-1H-indazol-5-yl]amino]-5-methylpyrrolo[2,1-f][1,2,4]triazin-6-yl]-carbamic acid, (3S)-3-morpholinylmethyl ester (BMS599626); N-(3,4-Dichloro-2-fluorophenyl)-6-methoxy-7-[[(3aα,5β,6aα)-octahydro-2-methylcyclopenta[c]pyrrol-5-yl]methoxy]- 4-quinazolinamine (XL647, CAS 781613-23-8); and 4-[4-[[(1R)-1-Phenylethyl]amino]-7H-pyrrolo[2,3-d]pyrimidin-6-yl]-phenol (PKI166, CAS 187724-61-4).

EGFR antibodies include but are not limited to, Cetuximab (Erbitux^{®}); Panitumumab (Vectibix^{®}); Matuzumab (EMD-72000); ; Nimotuzumab (hR3); Zahitumumab; TheraCIM h-R3; MDX0447 (CAS 339151-96-1); and ch806 (mAb-806, CAS 946414-09-1).

Human Epidermal Growth Factor Receptor 2 (Her2 receptor) (also known as Neu, ErbB-2, CD340, or p185) inhibitors include but are not limited to, Trastuzumab (Herceptin^{®}); Pertuzumab (Omnitarg^{®}); trastuzumab emtansine (Kadcyla^{®}); Neratinib (HKI-272, (2E)-N-[4-[[3-chloro-4-[(pyridin-2-yl)methoxy]phenyl]amino]-3-cyano-7-ethoxyquinolin-6-yl]-4-(dimethylamino)but-2-enamide, and described PCT Publication No. WO 05/028443); Lapatinib or Lapatinib ditosylate (Tykerb^{®}); (3R,4R)-4-amino-1-((4-((3-methoayphenyl)amino)pyrrolo[2,1-f][1,2,4]triazin-5-yl)methyl)piperidin-3-ol (BMS690514); (2E)-N-[4-[(3-Chloro-4-fluorophenyl)amino]-7-[[(3S)-tetrahydro-3-furanyl]oxy]-6-quinazolinyl]-4-(dimethylamino)-2-butenamide (BIBW-2992, CAS 850140-72-6); N-[4-[[1-[(3-Fluorophenyl)methyl]-1H-indazol-5-yl]amino]-5-methylpyrrolo[2,1-f][1,2,4]triazin-6-yl]-carbamic acid, (3S)-3-morpholinylmethyl ester (BMS 599626, CAS 714971-09-2); Canertinib dihydrochloride (PD183805 or CI-1033); and N-(3,4-Dichloro-2-fluorophenyl)-6-methoxy-7-[[(3aα,5β,6aα)-octahydro-2-methylcyclopenta[c]pyrrol-5-yl]methoxy]- 4-quinazolinamine (XL647, CAS 781613-23-8).

Her3 inhibitors include but are not limited to, LJM716, MM-121, AMG-888, RG7116, REGN-1400, AV-203, MP-RM-1, MM-111, and MEHD-7945A.

MET inhibitors include but are not limited to, Cabozantinib (XL184, CAS 849217-68-1); Foretinib (GSK1363089, formerly XL880, CAS 849217-64-7); Tivantinib (ARQ197, CAS 1000873-98-2); 1-(2-Hydroxy-2-methylpropyl)-N-(5-(7-methoxyquinolin-4-yloxy)pyridin-2-yl)-5-methyl-3-oxo-2-phenyl-2,3-dihydro-1H-pyrazole-4-carboxamide (AMG 458); Cryzotinib (Xalkori^{®}, PF-02341066); (3Z)-5-(2,3-Dihydro-1H-indol-1-ylsulfonyl)-3-({3,5-dimethyl-4-[(4-methylpiperazin-1-yl)carbonyl]-1H-pyrrol-2-yl}methylene)-1,3-dihydro-2H-indol-2-one (SU11271); (3Z)-N-(3-Chlorophenyl)-3-({3,5-dimethyl-4-[(4-methylpiperazin-1-yl)carbonyl]-1H-pyrrol-2-yl}methylene)-N-methyl-2-oxoindoline-5-sulfonamide (SU11274); (3Z)-N-(3-Chlorophenyl)-3-{[3,5-dimethyl-4-(3-morpholin-4-ylpropyl)-1H-pyrrol-2-yl]methylene}-N-methyl-2-oxoindoline-5-sulfonamide (SU11606); 6-[Difluoro[6-(1-methyl-1H-pyrazol-4-yl)-1,2,4-triazolo[4,3-b]pyridazin-3-yl]methyl]-quinoline (JNJ38877605, CAS 943540-75-8); 2-[4-[1-(Quinolin-6-ylmethyl)-1H-[1,2,3]triazolo[4,5-b]pyrazin-6-yl]-1H-pyrazol-1-yl]ethanol (PF04217903, CAS 956905-27-4); N-((2R)-1,4-Dioxan-2-ylmethyl)-N-methyl-N'-[3-(1-methyl-1H-pyrazol-4-yl)-5-oxo-5H-benzo[4,5]cyclohepta[1,2-b]pyridin-7-yl]sulfamide (MK2461, CAS 917879-39-1); 6-[[6-(1-Methyl-1H-pyrazol-4-yl)-1,2,4-triazolo[4,3-b]pyridazin-3-yl]thio]-quinoline (SGX523, CAS 1022150-57-7); and (3Z)-5-[[(2,6-Dichlorophenyl)methyl]sulfonyl]-3-[[3,5-dimethyl-4-[[(2R)-2-(1-pyrrolidinylmethyl)-1-pyrrolidinyl]carbonyl]-1H-pyrrol-2-yl]methylene]-1,3-dihydro-2H-indol-2-one (PHA665752, CAS 477575-56-7).

IGF1R inhibitors include but are not limited to, BMS-754807, XL-228, OSI-906, GSK0904529A, A-928605, AXL1717, KW-2450, MK0646, AMG479, IMCA12, MEDI-573, and BI836845. See *e.g.,* Yee, JNCI, 104; 975 (2012) for review.

In some embodiments, the present disclosure provides a method of treating or preventing cancer by administering to a subject in need thereof an antibody drug conjugate or formulation of the present disclosure in combination with one or more GNAQ/GNA11 downstream signaling pathway inhibitors, including but not limited to, β-arrestin inhibitors, GRK inhibitors, MAPK inhibitors, PI3K inhibitors, JAK inhibitors, etc.

For example, phosphoinositide 3-kinase (PI3K) inhibitors include but are not limited to, Idelalisib (Zydelig, GS-1101, Cal-101), 4-[2-(1H-Indazol-4-yl)-6-[[4-(methylsulfonyl)piperazin-1-yl]methyl]thieno[3,2-d]pyrimidin-4-yl]morpholine (also known as GDC 0941 and described in PCT Publication Nos. WO 09/036082 and WO 09/055730); 2-Methyl-2-[4-[3-methyl-2-oxo-8-(quinolin-3-yl)-2,3-dihydroimidazo[4,5-c]quinolin-1-yl]phenyl]propionitrile (also known as BEZ 235 or NVP-BEZ 235, and described in PCT Publication No. WO 06/122806); 4-(trifluoromethyl)-5-(2,6-dimorpholinopyrimidin-4-yl)pyridin-2-amine (also known as BKM120 or NVP-BKM120, and described in PCT Publication No. WO2007/084786); Tozasertib (VX680 or MK-0457, CAS 639089-54-6); (5Z)-5-[[4-(4-Pyridinyl)-6-quinolinyl]methylene]-2,4-thiazolidinedione (GSK1059615, CAS 958852-01-2); (1E,4S,4aR,5R,6aS,9aR)-5-(Acetyloxy)-1-[(di-2-propenylamino)methylene]-4,4a,5,6,6a,8,9,9a-octahydro-11-hydroxy-4-(methoxymethyl)-4a,6a-dimethyl-cyclopenta[5,6]naphtho[1,2-c]pyran-2,7,10(1H)-trione (PX866, CAS 502632-66-8); and 8-Phenyl-2-(morpholin-4-yl)-chromen-4-one (LY294002, CAS 154447-36-6).

In some embodiments, the present disclosure provides a method of treating or preventing cancer by administering to a subject in need thereof an antibody drug conjugate or formulation of the present disclosure in combination with one or more pro-apoptosis, including but not limited to, IAP inhibitors, Bcl2 inhibitors, MCl1 inhibitors, Trail agents, Chk inhibitors.

For examples, IAP inhibitors include but are not limited to, LCL161, GDC-0917, AEG-35156, AT406, and TL32711. Other examples of IAP inhibitors include but are not limited to those disclosed in WO04/005284, WO 04/007529, WO05/097791, WO 05/069894, WO 05/069888, WO 05/094818, US2006/0014700, US2006/0025347, WO 06/069063, WO 06/010118, WO 06/017295, and WO08/134679, all of which are incorporated herein by reference.

BCL-2 inhibitors include but are not limited to, Venetoclax (also known as GDC-0199, ABT-199, RG7601); 4-[4-[[2-(4-Chlorophenyl)-5,5-dimethyl-1-cyclohexen-1-yl]methyl]-1-piperazinyl]-N-[[4-[[(1R)-3-(4-morpholinyl)-1-[(phenylthio)methyl]propyl]amino]-3-[(trifluoromethyl)sulfonyl]phenyl]sulfonyl]benzamide (also known as ABT-263 and described in PCT Publication No. WO 09/155386); Tetrocarcin A; Antimycin; Gossypol ((-)BL-193); Obatoclax; Ethyl-2-amino-6-cyclopentyl-4-(1-cyano-2-ethoxy-2-oxoethyl)-4Hchromone-3-carboxylate (HA14 - 1); Oblimersen (G3139, Genasense^{®}); Bak BH3 peptide; (-)-Gossypol acetic acid (AT-101); 4-[4-[(4'-Chloro[1,1'-biphenyl]-2-yl)methyl]-1-piperazinyl]-*N-*[[4-[[(1*R*)-3-(dimethylamino)-1-[(phenylthio)methyl]propyl]amino]-3-nitrophenyl]sulfonyl]-benzamide (ABT-737, CAS 852808-04-9); and Navitoclax (ABT-263, CAS 923564-51-6).

Proapoptotic receptor agonists (PARAs) including DR4 (TRAILR1) and DR5 (TRAILR2), including but are not limited to, Dulanermin (AMG-951, RhApo2L/TRAIL); Mapatumumab (HRS-ETR1, CAS 658052-09-6); Lexatumumab (HGS-ETR2, CAS 845816-02-6); Apomab (Apomab^{®}); Conatumumab (AMG655, CAS 896731-82-1); and Tigatuzumab (CS1008, CAS 946415-34-5, available from Daiichi Sankyo).

Checkpoint Kinase (CHK) inhibitors include but are not limited to, 7-Hydroxystaurosporine (UCN-01); 6-Bromo-3-(1-methyl-1*H*-pyrazol-4-yl)-5-(3*R*)-3-piperidinyl-pyrazolo[1,5-*a*]pyrimidin-7-amine (SCH900776, CAS 891494-63-6); 5-(3-Fluorophenyl)-3-ureidothiophene-2-carboxylic acid N-[(S)-piperidin-3-yl]amide (AZD7762, CAS 860352-01-8); 4-[((3S)-1-Azabicyclo[2.2.2]oct-3-yl)amino]-3-(1H-benzimidazol-2-yl)-6-chloroquinolin-2(1H)-one (CHIR 124, CAS 405168-58-3); 7-Aminodactinomycin (7-AAD), Isogranulatimide, debromohymenialdisine; N-[5-Bromo-4-methyl-2-[(2S)-2-morpholinylmethoxy]-phenyl]-N'-(5-methyl-2-pyrazinyl)urea (LY2603618, CAS 911222-45-2); Sulforaphane (CAS 4478-93-7, 4-Methylsulfinylbutyl isothiocyanate); 9,10,11,12-Tetrahydro-9,12-epoxy-1*H*-diindolo[1,2,3-*fg*:3',2',1'-*kl*]pyrrolo[3,4-*i*][1,6]benzodiazocine-1,3(2*H*-)dione (SB-218078, CAS 135897-06-2); and TAT-S216A (YGRKKRRQRRRLYRSPAMPENL (SEQ ID NO: 282)), and CBP501 ((d-Bpa)sws(d-Phe-F5)(d-Cha)rrrqrr).

In some embodiments, the present disclosure provides a method of treating or preventing cancer by administering to a subject in need thereof an antibody drug conjugate or formulation of the present disclosure in combination with one or more immunomodulators (*e.g*., one or more of: an activator of a costimulatory molecule or an inhibitor of an immune checkpoint molecule).

In certain embodiments, the immunomodulator is an activator of a costimulatory molecule. In one embodiment, the agonist of the costimulatory molecule is chosen from an agonist (*e.g*., an agonistic antibody or antigen-binding fragment thereof, or a soluble fusion) of OX40, CD2, CD27, CDS, ICAM-1, LFA-1 (CD11a/CD18), ICOS (CD278), 4-1BB (CD137), GITR, CD30, CD40, BAFFR, HVEM, CD7, LIGHT, NKG2C, SLAMF7, NKp80, CD160, B7-H3, STING, or CD83 ligand.

In certain embodiments, the immunomodulator is an inhibitor of an immune checkpoint molecule. In one embodiment, the immunomodulator is an inhibitor of PD-1, PD-L1, PD-L2, CTLA4, TIM3, LAG3, VISTA, BTLA, TIGIT, LAIR1, CD160, 2B4 and/or TGFR beta. In one embodiment, the inhibitor of an immune checkpoint molecule inhibits PD-1, PD-L1, LAG-3, TIM-3 or CTLA4, or any combination thereof. The term "inhibition" or "inhibitor" includes a reduction in a certain parameter, *e.g.,* an activity, of a given molecule, *e.g.,* an immune checkpoint inhibitor. For example, inhibition of an activity, *e.g*., a PD-1 or PD-L1 activity, of at least 5%, 10%, 20%, 30%, 40%, 50% or more is included by this term. Thus, inhibition need not be 100%.

Inhibition of an inhibitory molecule can be performed at the DNA, RNA or protein level. In some embodiments, an inhibitory nucleic acid (*e.g*., a dsRNA, siRNA or shRNA), can be used to inhibit expression of an inhibitory molecule. In other embodiments, the inhibitor of an inhibitory signal is a polypeptide *e.g.,* a soluble ligand (*e.g.*, PD-1-Ig or CTLA-4 Ig), or an antibody or antigen-binding fragment thereof, that binds to the inhibitory molecule; *e.g*., an antibody or fragment thereof (also referred to herein as "an antibody molecule") that binds to PD-1, PD-L1, PD-L2, CTLA4, TIM3, LAG3, VISTA, BTLA, TIGIT, LAIR1, CD160, 2B4 and/or TGFR beta, or a combination thereof.

In some embodiments, the antibody molecule is a full antibody or fragment thereof (*e.g*., a Fab, F(ab')₂, Fv, or a single chain Fv fragment (scFv)). In yet other embodiments, the antibody molecule has a heavy chain constant region (Fc) chosen from, *e.g.,* the heavy chain constant regions of IgG1, IgG2, IgG3, IgG4, IgM, IgA1, IgA2, IgD, and IgE; particularly, chosen from, *e.g.,* the heavy chain constant regions of IgG1, IgG2, IgG3, and IgG4, more particularly, the heavy chain constant region of IgG1 or IgG4 (*e.g*., human IgG1 or IgG4). In one embodiment, the heavy chain constant region is human IgG1 or human IgG4. In one embodiment, the constant region is altered, *e.g.*, mutated, to modify the properties of the antibody molecule (*e.g*., to increase or decrease one or more of: Fc receptor binding, antibody glycosylation, the number of cysteine residues, effector cell function, or complement function).

In certain embodiments, the antibody molecule is in the form of a bispecific or multispecific antibody molecule. In some embodiments, the bispecific antibody molecule has a first binding specificity to PD-1 or PD-L1 and a second binding specificity, *e.g.,* a second binding specificity to TIM-3, LAG-3, or PD-L2. In some embodiments, the bispecific antibody molecule binds to PD-1 or PD-L1 and TIM-3. In some embodiments, the bispecific antibody molecule binds to PD-1 or PD-L1 and LAG-3. In some embodiments, the bispecific antibody molecule binds to PD-1 and PD-L1. In some embodiments, the bispecific antibody molecule binds to PD-1 and PD-L2. In some embodiments, the bispecific antibody molecule binds to TIM-3 and LAG-3. Any combination of the aforesaid molecules can be made in a multi-specific antibody molecule, *e.g.,* a tri-specific antibody that includes a first binding specificity to PD-1 or PD-1, and a second and third binding specificities to two or more of: TIM-3, LAG-3, or PD-L2.

In certain embodiments, the immunomodulator is an inhibitor of PD-1, *e.g.,* human PD-1. In another embodiment, the immunomodulator is an inhibitor of PD-L1, *e.g.,* human PD-L1. In one embodiment, the inhibitor of PD-1 or PD-L1 is an antibody molecule to PD-1 or PD-L1. The PD-1 or PD-L1 inhibitor can be administered alone, or in combination with other immunomodulators, *e.g*., in combination with an inhibitor of LAG-3, TIM-3 or CTLA4. In some embodiments, the inhibitor of PD-1 or PD-L1, *e.g.,* the anti-PD-1 or PD-L1 antibody molecule, is administered in combination with a LAG-3 inhibitor, *e.g.,* an anti-LAG-3 antibody molecule. In another embodiment, the inhibitor of PD-1 or PD-L1, *e.g.,* the anti-PD-1 or PD-L1 antibody molecule, is administered in combination with a TIM-3 inhibitor, *e.g.,* an anti-TIM-3 antibody molecule. In some embodiments, the inhibitor of PD-1 or PD-L1, *e.g.,* the anti-PD-1 antibody molecule, is administered in combination with a LAG-3 inhibitor, *e.g.,* an anti-LAG-3 antibody molecule, and a TIM-3 inhibitor, *e.g.,* an anti-TIM-3 antibody molecule. Other combinations of immunomodulators with a PD-1 inhibitor (*e.g*., one or more of PD-L2, CTLA4, TIM3, LAG3, VISTA, BTLA, TIGIT, LAIR1, CD160, 2B4 and/or TGFR) are also within the present disclosure. Any of the antibody molecules known in the art or disclosed herein can be used in the aforesaid combinations of inhibitors of checkpoint molecule.

In some embodiments, the PD-1 inhibitor is an anti-PD-1 antibody chosen from Nivolumab, Pembrolizumab or Pidilizumab. In some embodiments, the anti-PD-1 antibody is Nivolumab. Alternative names for Nivolumab include MDX- 1106, MDX-1106-04, ONO-4538, or BMS-936558. In some embodiments, the anti-PD- 1 antibody is Nivolumab (CAS Registry Number: 946414-94-4). Nivolumab is a fully human IgG4 monoclonal antibody which specifically blocks PD-1. Nivolumab (clone 5C4) and other human monoclonal antibodies that specifically bind to PD-1 are disclosed in US Pat No. 8,008,449 and PCT Publication No. WO2006/121168.

In some embodiments, the anti-PD-1 antibody is Pembrolizutnab. Pembrolizumab (Trade name KEYTRUDA formerly Lambrolizumab, also known as Merck 3745, MK-3475 or SCH-900475) is a humanized IgG4 monoclonal antibody that binds to PD-1. Pembrolizumab is disclosed, *e.g*., in Hamid, O. et al. (2013) New England Journal of Medicine 369 (2): 134-44, PCT Publication No. WO2009/114335, and US Patent No. 8,354,509.

In some embodiments, the anti-PD-1 antibody is Pidilizumab. Pidilizumab (CT-011; Cure Tech) is a humanized IgGlk monoclonal antibody that binds to PD-1. Pidilizumab and other humanized anti-PD-1 monoclonal antibodies are disclosed in PCT Publication No. WO2009/101611. Other anri-PD1 antibodies are disclosed in US Patent No. 8,609,089, US Publication No. 2010028330, and/or US Publication No. 20120114649. Other anti-PD-1 antibodies include AMP 514 (Amplimmune).

In some embodiments, the PD-1 inhibitor is PDR001, also known as spartalizumab, or any other anti-PD-1 antibody disclosed in WO2015/112900.

In some embodiments, the PD-1 inhibitor is an immunoadhesin (*e.g*., an immunoadhesin comprising an extracellular or PD-1 binding portion of PD-Ll or PD-L2 fused to a constant region (*e.g.*, an Fc region of an immunoglobulin sequence). In some embodiments, the PD-1 inhibitor is AMP-224.

In some embodiments, the PD-Ll inhibitor is anti-PD-Ll antibody. In some embodiments, the anti-PD-Ll inhibitor is chosen from YW243.55.S70, MPDL3280A, MEDI-4736, or MDX-1105MSB-0010718C (also referred to as A09-246-2) disclosed in, *e.g.,* WO 2013/0179174, and having a sequence disclosed herein (or a sequence substantially identical or similar thereto, *e.g.,* a sequence at least 85%, 90%, 95% identical or higher to the sequence specified).

In some embodiments, the PD-L1 inhibitor is MDX-1105. MDX-1105, also known as BMS-936559, is an anti-PD-Ll antibody described in PCT Publication No. WO2007/005874.

In some embodiments, the PD-L1 inhibitor is YW243.55.S70. The YW243.55.S70 antibody is an anti-PD-Ll described in PCT Publication No. WO 2010/077634 (heavy and light chain variable region sequences shown in SEQ ID Nos. 20 and 21, respectively).

In some embodiments, the PD-L1 inhibitor is MDPL3280A (Genentech / Roche). MDPL3280A is a human Fc optimized IgG1 monoclonal antibody that binds to PD-L1. MDPL3280A and other human monoclonal antibodies to PD-L1 are disclosed in U.S. Patent No.: 7,943,743 and U.S Publication No.: 20120039906.

In some embodiments, the PD-L2 inhibitor is AMP-224. AMP-224 is a PD-L2 Fc fusion soluble receptor that blocks the interaction between PD1 and B7-H1 (B7-DCIg; Amplimmune; *e.g*., disclosed in PCT Publication Nos. WO2010/027827 and WO2011/066342).

In o some embodiments, the LAG-3 inhibitor is an anti-LAG-3 antibody molecule. In one embodiment, the LAG-3 inhibitor is BMS-986016. In one embodiment, the LAG-3 inhibitor is LAG525 or any anti-LAG3 antibody disclosed in WO2015/138920.

In some embodiments, the TIM-3 inhibitor is an anti-TIM3 antibody molecule. In one embodiment, the TIM-3 inhibitor is MBG453 or any anti-TIM3 antibody disclosed in WO2015/117002.

### Pharmaceutical Compositions and Formulations

To prepare pharmaceutical or sterile compositions including immunoconjugates (*e.g*., immunoconjugates made by a process described herein), the immunoconjugates of the disclosure are mixed with a pharmaceutically acceptable carrier or excipient. The compositions can additionally contain one or more other therapeutic agents that are suitable for treating or preventing a PMEL17 expressing cancer (including, but not limited to carcinoma (*e.g*., hepatocellular carcinoma), sarcoma, leukemia, lymphoma, eye cancer, eye neoplasm, melanoma (*e.g*., uveal melanoma, non-uveal melanoma, malignant melanoma, ocular melanoma, subcutaneous melanoma, cutaneous melanoma, or mucosal melanoma), or a metastatic cancer thereof.

In certain embodiments, the immunoconjugates or pharmaceutical compositions of the disclosure can be formulated into a formulation (*e.g*., a dose formulation or dosage form) suitable for administration to a subject as described herein.

In some embodiments, the formulation is a liquid formulation. In other embodiments, the formulation is a reconstituted or dried formulation, *e.g*., reconstituted or dried from a lyophilized formulation. In other embodiments, the formulation is a lyophilized or dried formulation.

In some embodiments, the formulation comprises an antibody drug conjugate described herein (e.g., an antibody drug conjugate made by a process described herein), a buffering agent, a stabilizing agent, and a surfactant, wherein the formulation has a pH of 4.5 to 6.5.

In some embodiments, the antibody drug conjugate is present at a concentration of about 5 mg/mL to about 30 mg/mL, *e.g*., about 10 mg to about 30 mg, about 15 mg/mL to about 25 mg/mL, about 18 mg/mL to about 22 mg/mL, about 10 mg/mL to 25 mg/mL, about 10 mg/mL to about 20 mg/mL, about 10 mg/mL to about 15 mg/mL, about 25 mg to about 30 mg/mL, about 20 mg/mL to about 30 mg/mL, about 5 mg/mL to about 15 mg/mL, about 15 mg/mL to about 25 mg/mL, or about 18 mg/mL to about 22 mg/mL, *e*.*g*., about 5 mg/mL, about 6 mg/mL, about 7 mg/mL, about 8 mg/mL, about 9 mg/mL, about 10 mg/mL, about 11 mg/mL, about 12 mg/mL, about 13 mg/mL, about 14 mg/mL, about 15 mg/mL, about 16 mg/mL, about 17 mg/mL, about 18 mg/mL, about 19 mg/mL, about 20 mg/mL, about 21 mg/mL, about 22 mg/mL, about 23 mg/mL, about 24 mg/mL, or about 25 mg/mL. In some embodiments, the antibody drug conjugate is present at a concentration of about 15 mg/mL to about 25 mg/mL, *e*.*g*., about 20 mg/mL.

In some embodiments, the buffering agent comprises histidine. In some embodiments, the buffering agent comprises succinate. In some embodiments, the buffering agent is present at a concentration of about 5 mM to about 50 mM, *e*.*g*., about 10 mM to about 40 nM, about 15 mM to about 35 mM, about 20 mM to about 30 mM, about 5 mM to about 40 mM, about 5 mM to about 30 mM, about 5 mM to about 20 mM, about 5 mM to about 15 mM, about 5 mM to about 10 mM, about 40 mM to about 50 mM, about 35 mM to about 50 mM, about 30 mM to about 50 mM, about 25 mM to about 50 mM, about 20 mM to about 50 mM, about 15 mM to about 50 mM, about 10 mM to about 50 mM, about 10 mM to about 20 mM, about 15 mM to about 25 mM, about 25 mM to about 35 mM, about 30 mM to about 40 mM, or about 35 mM to about 45 mM, *e.g.,* about 5 mM, about 10 mM, about 15 mM, about 20 mM, about 25 mM, about 30 mM, about 35 mM, about 40 mM, about 45 mM, or about 50 mM. In some embodiments, the buffering agent is present at a concentration of about 15 mM to about 25 mM, *e.g.,* about 20 mM.

In some embodiments, the stabilizing agent comprises sucrose. In some embodiments, the stabilizing agent comprises trehalose. In some embodiments, the stabilizing agent is present at a concentration of about 100 mM to about 500 mM, *e*.*g*., about 150 mM to about 450 mM, about 200 mM to about 400 mM, about 250 mM to about 350 mM, about 100 mM to about 450 mM, about 100 mM to about 400 mM, about 100 mM to about 350 mM, about 100 mM to about 300 mM, about 100 mM to about 250 mM, about 100 mM to about 200 mM, about 100 mM to about 150 mM, about 450 mM to about 500 mM, about 400 mM to about 500 mM, about 350 mM to about 500 mM, about 300 mM to about 500 mM, about 250 mM to about 500 mM, about 200 mM to about 500 mM, about 150 mM to about 500 mM, about 150 mM to about 250 mM, about 200 mM to about 250 mM, about 200 mM to about 300 mM, about 300 mM to about 400 mM, or about 350 mM to about 450 mM, *e.g.,* about 100 mM, about 150 mM, about 200 mM, about 240 mM, about 250 mM, about 300 mM, about 350 mM, about 400 mM, about 450 mM, or about 500 mM. In some embodiments, the stabilizing agent and is present at a concentration of about 200 mM to about 300 mM, *e*.*g*., about 240 mM.

In some embodiments, the surfactant comprises polysorbate 20. In some embodiments, the surfactant is present at a concentration of about 0.01% to about 0.06%, *e.g.,* about 0.02% to about 0.05%, about 0.03% to about 0.04%, about 0.01% to about 0.05%, about 0.01% to about 0.04%, about 0.01% to about 0.03%, about 0.01% to about 0.02%, about 0.05% to about 0.06%, about 0.04% to about 0.06%, about 0.03% to about 0.06%, about 0.02% to about 0.06%, about 0.02% to about 0.04%, about 0.03% to about 0.05%, *e.g.,* about 0.01%, about 0.02%, about 0.03%, about 0.04%, about 0.05%, or about 0.06%. In some embodiments, the surfactant is present at a concentration of about 0.01% to about 0.03%, *e.g.,* about 0.02%.

In some embodiments, the formulation has a pH of about 4.7 to about 5.3, about 5.0 to about 6.0, about 5.2 to about 5.8, about 5.4 to about 5.6, about 5.2 to about 6.0, about 5.4 to about 6.0, about 5.6 to about 6.0, about 5.8 to about 6.0, about 5 to about 5.8, about 5 to about 5.6.0, about 5 to about 5.4, about 5 to about 5.2, about 4.8 to about 5.2, about 5.1 to about 5.3, about 5.2 to about 5.4, about 5.3 to about 5.5, about 5.5 to about 5.7, about 5.6 to about 5.8, about 5.7 to about 5.9, about 4.9 to about 5.5, about 5.5 to about 6.1, or about 5.7 to about 6.3, *e.g.,* about 4.5, about 4.6, about 4.7, about 4.8, about 4.9, about 5, about 5.1, about 5.2, about 5.3, about 5.4, about 5.5, about 5.6, about 5.7, about 5.8, about 5.9, about 6.0, about 6.1, about 6.2, about 6.3, about 6.4, or about 6.5. In some embodiments, the formulation has a pH of about 5.0 to about 5.6, *e.g.,* about 5.3.

In some embodiments, the formulation comprises about 15 mg/mL to about 25 mg/mL of the antibody drug conjugate, about 15 mM to about 25 mM of a buffering agent comprising histidine, about 200 mM to about 300 mM of a stabilizing agent comprising sucrose, and about 0.01% to about 0.03% of a surfactant comprising polysorbate 20, wherein the formulation has a pH of about 5.0 to about 5.6. In some embodiments, the formulation comprises about 20 mg/mL of the antibody drug conjugate, about 20 mM of a buffering agent comprising histidine, about 240 mM of a stabilizing agent comprising sucrose, and about 0.02% of a surfactant comprising polysorbate 20, wherein the formulation has a pH of about 5.3.

In some embodiments, the formulation comprises about 15 mg/mL to about 25 mg/mL of the antibody drug conjugate, about 15 mM to about 25 mM of a buffering agent comprising histidine, about 200 mM to about 300 mM of a stabilizing agent comprising sucrose, and about 0.01% to about 0.03% of a surfactant comprising polysorbate 20, wherein the formulation has a pH of about 5.0 to about 5.6. In some embodiments, the formulation comprises about 20 mg/mL of the antibody drug conjugate, about 20 mM of a buffering agent comprising histidine, about 240 mM of a stabilizing agent comprising sucrose, and about 0.02% of a surfactant comprising polysorbate 20, wherein the formulation has a pH of about 5.3.

In some embodiments, the formulation comprises about 15 mg/mL to about 25 mg/mL of the antibody drug conjugate, about 15 mM to about 25 mM of a buffering agent comprising histidine, about 200 mM to about 300 mM of a stabilizing agent comprising sucrose, and about 0.01% to about 0.03% of a surfactant comprising polysorbate 20, wherein the formulation has a pH of about 4.7 to about 5.3. In some embodiments, the formulation comprises about 20 mg/mL of the antibody drug conjugate, about 20 mM of a buffering agent comprising histidine, about 240 mM of a stabilizing agent comprising sucrose, and about 0.02% of a surfactant comprising polysorbate 20, wherein the formulation has a pH of about 5.0.

In some embodiments, the formulation comprises about 15 mg/mL to about 25 mg/mL of the antibody drug conjugate, about 15 mM to about 25 mM of a buffering agent comprising histidine, about 200 mM to about 300 mM of a stabilizing agent comprising sucrose, and about 0.01% to about 0.03% of a surfactant comprising polysorbate 20, wherein the formulation has a pH of about 4.7 to about 5.3. In some embodiments, the formulation comprises about 20 mg/mL of the antibody drug conjugate, about 20 mM of a buffering agent comprising histidine, about 240 mM of a stabilizing agent comprising sucrose, and about 0.02% of a surfactant comprising polysorbate 20, wherein the formulation has a pH of about 5.0.

In some embodiments, the formulation comprises about 15 mg/mL to about 25 mg/mL of the antibody drug conjugate, about 15 mM to about 25 mM of a buffering agent comprising histidine, about 200 mM to about 300 mM of a stabilizing agent comprising sucrose, and about 0.01% to about 0.03% of a surfactant comprising polysorbate 20, wherein the formulation has a pH of about 5.2 to about 5.8. In some embodiments, the formulation comprises about 20 mg/mL of the antibody drug conjugate, about 20 mM of a buffering agent comprising histidine, about 240 mM of a stabilizing agent comprising sucrose, and about 0.02% of a surfactant comprising polysorbate 20, wherein the formulation has a pH of about 5.5.

In some embodiments, the formulation comprises about 15 mg/mL to about 25 mg/mL of the antibody drug conjugate, about 15 mM to about 25 mM of a buffering agent comprising histidine, about 200 mM to about 300 mM of a stabilizing agent comprising sucrose, and about 0.01% to about 0.03% of a surfactant comprising polysorbate 20, wherein the formulation has a pH of about 5.2 to about 5.8. In some embodiments, the formulation comprises about 20 mg/mL of the antibody drug conjugate, about 20 mM of a buffering agent comprising histidine, about 240 mM of a stabilizing agent comprising sucrose, and about 0.02% of a surfactant comprising polysorbate 20, wherein the formulation has a pH of about 5.5.

In some embodiments, the formulation comprises about 15 mg/mL to about 25 mg/mL of the antibody drug conjugate, about 15 mM to about 25 mM of a buffering agent comprising histidine, about 200 mM to about 300 mM of a stabilizing agent comprising sucrose, and about 0.03% to about 0.05% of a surfactant comprising polysorbate 20, wherein the formulation has a pH of about 5.2 to about 5.8. In some embodiments, the formulation comprises about 20 mg/mL of the antibody drug conjugate, about 20 mM of a buffering agent comprising histidine, about 240 mM of a stabilizing agent comprising sucrose, and about 0.04% of a surfactant comprising polysorbate 20, wherein the formulation has a pH of about 5.5.

In some embodiments, the formulation comprises about 15 mg/mL to about 25 mg/mL of the antibody drug conjugate, about 15 mM to about 25 mM of a buffering agent comprising histidine, about 200 mM to about 300 mM of a stabilizing agent comprising sucrose, and about 0.03% to about 0.05% of a surfactant comprising polysorbate 20, wherein the formulation has a pH of about 5.2 to about 5.8. In some embodiments, the formulation comprises about 20 mg/mL of the antibody drug conjugate, about 20 mM of a buffering agent comprising histidine, about 240 mM of a stabilizing agent comprising sucrose, and about 0.04% of a surfactant comprising polysorbate 20, wherein the formulation has a pH of about 5.5.

In some embodiments, the formulation comprises about 5 mg/mL to about 15 mg/mL of the antibody drug conjugate, about 15 mM to about 25 mM of a buffering agent comprising histidine, about 200 mM to about 300 mM of a stabilizing agent comprising sucrose, and about 0.03% to about 0.05% of a surfactant comprising polysorbate 20, wherein the formulation has a pH of about 5.2 to about 5.8. In some embodiments, the formulation comprises about 10 mg/mL of the antibody drug conjugate, about 20 mM of a buffering agent comprising histidine, about 240 mM of a stabilizing agent comprising sucrose, and about 0.02% of a surfactant comprising polysorbate 20, wherein the formulation has a pH of about 5.5.

In some embodiments, the formulation comprises about 5 mg/mL to about 15 mg/mL of the antibody drug conjugate, about 15 mM to about 25 mM of a buffering agent comprising histidine, about 200 mM to about 300 mM of a stabilizing agent comprising sucrose, and about 0.03% to about 0.05% of a surfactant comprising polysorbate 20, wherein the formulation has a pH of about 5.2 to about 5.8. In some embodiments, the formulation comprises about 10 mg/mL of the antibody drug conjugate, about 20 mM of a buffering agent comprising histidine, about 240 mM of a stabilizing agent comprising sucrose, and about 0.02% of a surfactant comprising polysorbate 20, wherein the formulation has a pH of about 5.5.

In some embodiments, the formulation comprises about 15 mg/mL to about 25 mg/mL of the antibody drug conjugate, about 15 mM to about 25 mM of a buffering agent comprising histidine, about 200 mM to about 300 mM of a stabilizing agent comprising sucrose, and about 0.01% to about 0.03% of a surfactant comprising polysorbate 20, wherein the formulation has a pH of about 5.7 to about 6.3. In some embodiments, the formulation comprises about 20 mg/mL of the antibody drug conjugate, about 20 mM of a buffering agent comprising histidine, about 240 mM of a stabilizing agent comprising sucrose, and about 0.02% of a surfactant comprising polysorbate 20, wherein the formulation has a pH of about 6.0.

In some embodiments, the formulation comprises about 15 mg/mL to about 25 mg/mL of the antibody drug conjugate, about 15 mM to about 25 mM of a buffering agent comprising histidine, about 200 mM to about 300 mM of a stabilizing agent comprising sucrose, and about 0.01% to about 0.03% of a surfactant comprising polysorbate 20, wherein the formulation has a pH of about 5.7 to about 6.3. In some embodiments, the formulation comprises about 20 mg/mL of the antibody drug conjugate, about 20 mM of a buffering agent comprising histidine, about 240 mM of a stabilizing agent comprising sucrose, and about 0.02% of a surfactant comprising polysorbate 20, wherein the formulation has a pH of about 6.0.

In some embodiments, the formulation comprises about 15 mg/mL to about 25 mg/mL of the antibody drug conjugate, about 15 mM to about 25 mM of a buffering agent comprising histidine, about 200 mM to about 300 mM of a stabilizing agent comprising sucrose, and about 0.01% to about 0.03% of a surfactant comprising polysorbate 20, wherein the formulation has a pH of about 4.9 to about 5.5. In some embodiments, the formulation comprises about 20 mg/mL of the antibody drug conjugate, about 20 mM of a buffering agent comprising histidine, about 240 mM of a stabilizing agent comprising sucrose, and about 0.02% of a surfactant comprising polysorbate 20, wherein the formulation has a pH of about 5.2.

In some embodiments, the formulation comprises about 15 mg/mL to about 25 mg/mL of the antibody drug conjugate, about 15 mM to about 25 mM of a buffering agent comprising histidine, about 200 mM to about 300 mM of a stabilizing agent comprising sucrose, and about 0.01% to about 0.03% of a surfactant comprising polysorbate 20, wherein the formulation has a pH of about 4.9 to about 5.5. In some embodiments, the formulation comprises about 20 mg/mL of the antibody drug conjugate, about 20 mM of a buffering agent comprising histidine, about 240 mM of a stabilizing agent comprising sucrose, and about 0.02% of a surfactant comprising polysorbate 20, wherein the formulation has a pH of about 5.2.

In some embodiments, the formulation comprises about 15 mg/mL to about 25 mg/mL of the antibody drug conjugate, about 15 mM to about 25 mM of a buffering agent comprising histidine, about 200 mM to about 300 mM of a stabilizing agent comprising sucrose, and about 0.01% to about 0.03% of a surfactant comprising polysorbate 20, wherein the formulation has a pH of about 5.5 to about 6.1. In some embodiments, the formulation comprises about 20 mg/mL of the antibody drug conjugate, about 20 mM of a buffering agent comprising histidine, about 240 mM of a stabilizing agent comprising sucrose, and about 0.02% of a surfactant comprising polysorbate 20, wherein the formulation has a pH of about 5.8.

In some embodiments, the formulation comprises about 15 mg/mL to about 25 mg/mL of the antibody drug conjugate, about 15 mM to about 25 mM of a buffering agent comprising histidine, about 200 mM to about 300 mM of a stabilizing agent comprising sucrose, and about 0.01% to about 0.03% of a surfactant comprising polysorbate 20, wherein the formulation has a pH of about 5.5 to about 6.1. In some embodiments, the formulation comprises about 20 mg/mL of the antibody drug conjugate, about 20 mM of a buffering agent comprising histidine, about 240 mM of a stabilizing agent comprising sucrose, and about 0.02% of a surfactant comprising polysorbate 20, wherein the formulation has a pH of about 5.8.

In an embodiment, the formulation is a formation described in Example 4, *e.g.,* Table 27. In an embodiment, the formuation comprises 20 mg/mL of the antibody drug conjugate, 20 mM histidine, 240 mM sucrose, 0.02% polysorbate, at pH 5.0. In an embodiment, the formuation comprises 20 mg/mL of the antibody drug conjugate, 20 mM histidine, 240 mM sucrose, 0.02% polysorbate, at pH 5.5. In an embodiment, the formuation comprises 20 mg/mL of the antibody drug conjugate, 20 mM histidine, 240 mM sucrose, 0.04% polysorbate, at pH 5.5. In an embodiment, the formuation comprises 10 mg/mL of the antibody drug conjugate, 20 mM histidine, 240 mM sucrose, 0.02% polysorbate, at pH 5.5. In an embodiment, the formuation comprises 20 mg/mL of the antibody drug conjugate, 20 mM histidine, 240 mM sucrose, 0.02% polysorbate, at pH 6.0. In an embodiment, the formuation comprises 20 mg/mL of the antibody drug conjugate, 20 mM histidine, 240 mM sucrose, 0.02% polysorbate, at pH 5.2. In an embodiment, the formuation comprises 20 mg/mL of the antibody drug conjugate, 20 mM histidine, 240 mM sucrose, 0.02% polysorbate, at pH 5.8.

In some embodiments, the D in the formulation (*e*.*g*., lyophilized formulation) is at least 1, 2, 3, 4, 5, 6, 7, 8, 9, or 10-fold more stable, compared to a reference formulation (*e*.*g*., an otherwise identical formulation that is not lyophilized), after storage for about 0, about 4, or about 8 weeks, at about 5°C, 25°C, or 40°C, *e.g.,* as determined by CZE. In some embodiments, the percentage of D that has a ring-opening conformation in the formulation (*e*.*g*., lyophilized formulation) is at least 1, 2, 3, 4, 5, 6, 7, 8, 9, or 10-fold lower, compared to a reference formulation (*e*.*g*., an otherwise identical formulation that is not lyophilized), after storage for about 0, about 4, or about 8 weeks, at about 5°C, 25°C, or 40°C, *e*.*g*., as determined by CZE.

In some embodiments, the formulation (e.g., lyophilized formulation) comprises about 80 mg to about 120 mg (*e*.*g*., about 107 mg) of the antibody drug conjugate. In some embodiments, about 5 mL to about 5.5 mL of the formulation is lyophilized.

In some embodiments, the level of monomers in the formulation (*e*.*g*., lyophilized formulation) is at least about 95%, *e.g.,* at least about 96%, 97%, 98%, or 99%, after storage for about 0, about 4, or about 8 weeks, at about 5°C, 25°C, or 40°C, *e*.*g*., as determined by SEC. In some embodiments, the level of fragments in the formulation (*e*.*g*., lyophilized formulation) is less than about 3%, *e*.*g*., less than about 2.5%, 2%, 1.5%, 1%, or 0.5%, after storage for about 0, about 4, or about 8 weeks, at about 5°C, 25°C, or 40°C, *e*.*g*., as determined by SEC. In some embodiments, the level of aggregates in the formulation (*e*.*g*., lyophilized formulation) is less than about 3%, *e*.*g*., less than about 2.5%, 2%, 1.5%, 1%, or 0.5%, after storage for about 0, about 4, or about 8 weeks, at about 5°C, 25°C, or 40°C, *e*.*g*., as determined by SEC. In some embodiments, the level of degradation in the formulation (e.g., lyophilized formulation) is less than about 2%, *e.g.,* less than about 1.5%, 1%, or 0.5%, after storage for about 4 weeks or about 8 weeks, at about 5°C, 25°C, or 40°C, *e*.*g*., as determined by SEC.

In an embodiment, the level of particles greater than or equal to 10 µm in the lyophilized formulation is less than about 300 particles/ml, *e*.*g*., less than about 280 particles/mL, less than about 260 particles/mL, less than about 240 particles/mL, less than about 220 particles/mL, less than about 200 particles/mL, less than about 180 particles/mL, less than about 160 paricles/mL, less than about 140 particles/mL, 120 particles/mL, less than about 100 particles/mL, 80 particles/mL, 60 particles/mL, 40 particles/mL, 20 particles/mL, or 10 particles/mL, after storage for about 0, about 4, or about 8 weeks, at about 5°C, 25°C, or 40°C, *e.g.,* as determined by light obscuration. In some embodiments, the level of particles greater than 10 µm in the formulation (*e*.*g*., lyophilized formulation) is increased by no more than about 3-fold, *e.g.,* no more than about 2.5, 2, 1.5, 1, or 0.5-fold, after storage for about 4 or about 8 weeks, at about 5°C, 25°C, or 40°C, *e.g.,* as determined by light obscuration. In some embodiments, the level of particles greater than 25 µm in the formulation (*e*.*g*., lyophilized formulation) is increased by no more than about 3-fold, *e.g.,* no more than about 2.5, 2, 1.5, 1, or 0.5-fold, after storage for about 4 or about 8 weeks, at about 5°C, 25°C, or 40°C, *e.g.,* as determined by light obscuration. In some embodiments, the level of particles greater than 25 µm in the formulation (*e*.*g*., lyophilized formulation) is less than about 20 particles/mL, *e.g.,* less than about 15 particles/mL, 10 particles/mL, 5 particles/mL, or 2 particles/mL, after storage for about 0, about 4, or about 8 weeks, at about 5°C, 25°C, or 40°C, *e.g.,* as determined by light obscuration.

In some embodiments, the level of impurity in the formulation (*e*.*g*., lyophilized formulation) is less than about 15%, *e.g.,* less than about 14%, 13%, 12%, 11%, 10%, 9%, 8%, 7%, 6%, 5%, 4%, 3%, 2%, or 1%, after storage for about 0, about 4, or about 8 weeks, at about 5°C, 25°C, or 40°C, *e*.*g*., as determined by capillary electrophoresis. In some embodiments, the level of impurity in the formulation (*e*.*g*., lyophilized formulation) is increased by no more than 20%, *e*.*g*., no more than about 15%, 10%, 5%, 2%, or 1%, after storage for about 4 weeks or about 8 weeks, at about 5°C, 25°C, or 40°C, *e.g.,* as determined by capillary electrophoresis.

In some embodiments, the level of neutral variants in the formulation (*e*.*g*., lyophilized formulation) is greater than about 50%, *e.g.,* greater than about 55%, 60%, 65%, 70%, 75%, 80%, 85%, or 90%, after storage for about 0, about 4, or about 8 weeks, at about 5°C, 25°C, or 40°C, *e*.*g*., as determined by capillary zone electrophoresis (CZE). In some embodiments, the level of neutral variants in the formulation (*e*.*g*., lyophilized formulation) is decreased by no more than about 20%, *e*.*g*., no more than about 15%, 10%, 5%, or 2%, after storage for about 4 weeks or about 8 weeks, at about 5°C, 25°C, or 40°C, *e.g.,* as determined by CZE.

In some embodiments, the level of acidic variants in the formulation (*e*.*g*., lyophilized formulation) is less than about 30%, *e.g.,* less than about 25%, 20%, 15%, 5%, or 2%, after storage for about 0, about 4, or about 8 weeks, at about 5°C, 25°C, or 40°C, *e*.*g*., as determined by CZE. In some embodiments, the level of acid variants in the formulation (*e*.*g*., lyophilized formulation) is increased by no more than about 50%, *e.g.,* no more than about 40%, 30%, 20%, 10%, or 5%, after storage for about 4 weeks or about 8 weeks, at about 5°C, 25°C, or 40°C, *e*.*g*., as determined by CZE.

In some embodiments, the level of basic variants in the formulation (*e*.*g*., lyophilized formulation) is less than about 30%, *e.g.,* less than about 25%, 20%, 15%, 5%, or 2%, after storage for about 0, about 4, or about 8 weeks, at about 5°C, 25°C, or 40°C, *e*.*g*., as determined by CZE. In some embodiments, the level of basic variants in the formulation (e.g., lyophilized formulation) is increased by no more than about 50%, *e.g.,* no more than about 40%, 30%, 20%, 10%, or 5%, after storage for about 4 weeks or about 8 weeks, at about 5°C, 25°C, or 40°C, *e*.*g*., as determined by CZE.

In some embodiments, the potency of the formulation (*e*.*g*., lyophilized formulation) is decreased by no more than about 25%, *e.g.,* no more than about 20%, 15%, 10%, 5%, or 2%, after storage for about 8 weeks, at about 5°C, 25°C, or 40°C, *e.g.,* as determined by a bioactivity assay. In some embodiments, the osmolality of the formulation (*e*.*g*., lyophilized formulation) is about 200 mOsm/L to 400 mOsm/L, *e*.*g*., 200 mOsm/L to 300 mOsm/L, 250 mOsm/L to 350 mOsm/L, 270 mOsm/L to 330 mOsm/L, 290 mOsm/L to 310 mOsm/L, 270 mOsm/L to 350 mOsm/L, 290 mOsm/L to 350 mOsm/L, 310 mOsm/L to 350 mOsm/L, 330 mOsm/L to 350 mOsm/L, 250 mOsm/L to 330 mOsm/L, 250 mOsm/L to 310 mOsm/L, 250 mOsm/L to 290 mOsm/L, 250 mOsm/L to 270 mOsm/L, 260 mOsm/L to 280 mOsm/L, 270 mOsm/L to 290 mOsm/L, 280 mOsm/L to 300 mOsm/L, 300 mOsm/L to 320 mOsm/L, 310 mOsm/L to 330 mOsm/L, or 320 mOsm/L to 340 mOsm/L, *e*.*g*., 200 mOsm/L, 250 mOsm/L, 260 mOsm/L, 270 mOsm/L, 280 mOsm/L, 290 mOsm/L, 300 mOsm/L, 310 mOsm/L, 320 mOsm/L, 330 mOsm/L, 340 mOsm/L, 350 mOsm/L, or 400 mOsm/L.

In some embodiments, the formulation is present in a container. In some embodiments, the container is a vial, *e.g.,* a 25 R glass vial. In some embodiment, the container further comprises a stopper and a cap (*e*.*g*., a flip-off aluminum cap). In some embodiments, the formulation is stored in a polyethylene terephthalate (PET) bottle with a high density polyethylene (HDPE) screw closure, *e*.*g*., both of which are USP class VI compliant. In some embodiments, the container (*e*.*g*., bottle) and/or the closure is sterilized by gamma radiation prior to filling. In some embodiments, the container (*e.g*., bottle) is determined to be non-pyrogenic per USP.

In certain embodiments, the formulation ensures proper distribution *in vivo.* For example, the blood-brain barrier (BBB) excludes many highly hydrophilic compounds. To ensure that the therapeutic compounds of the disclosure cross the BBB (if desired), they can be formulated, for example, in liposomes. For methods of manufacturing liposomes, *see, e.g.,* U.S. Pat. Nos. 4,522,811; 5,374,548; and 5,399,331. The liposomes may comprise one or more moieties which are selectively transported into specific cells or organs, thus enhance targeted drug delivery *(see, e.g.,* Ranade, (1989) J. Clin. Pharmacol. 29:685). Exemplary targeting moieties include folate or biotin *(see, e.g.,* U.S. Pat. No. 5,416,016 to Low et al.); mannosides (Umezawa et al., (1988) Biochem. Biophys. Res. Commun. 153:1038); antibodies (Bloeman et al., (1995) FEBS Lett. 357:140; Owais et al., (1995) Antimicrob. Agents Chemother. 39:180); surfactant protein A receptor (Briscoe et al., (1995) Am. J. Physiol. 1233:134); p 120 (Schreier et al., (1994) J. Biol. Chem. 269:9090); *see also* K. Keinanen; M. L. Laukkanen (1994) FEBS Lett. 346:123; J. J. Killion; I. J. Fidler (1994) Immunomethods 4:273.

Formulations of therapeutic and diagnostic agents can be prepared by mixing with physiologically acceptable carriers, excipients, or stabilizers in the form of, *e*.*g*., lyophilized powders, slurries, aqueous solutions, lotions, or suspensions (*see, e.g.,* Hardman et al., Goodman and Gilman's The Pharmacological Basis of Therapeutics, McGraw-Hill, New York, N.Y., 2001; Gennaro, Remington: The Science and Practice of Pharmacy, Lippincott, Williams, and Wilkins, New York, N.Y., 2000; Avis, et al. (eds.), Pharmaceutical Dosage Forms: Parenteral Medications, Marcel Dekker, NY, 1993; Lieberman, et al. (eds.), Pharmaceutical Dosage Forms: tablets, Marcel Dekker, NY, 1990; Lieberman, et al. (eds.) Pharmaceutical Dosage Forms: Disperse Systems, Marcel Dekker, NY, 1990; Weiner and Kotkoskie, Excipient Toxicity and Safety, Marcel Dekker, Inc., New York, N.Y., 2000).

Selecting an administration regimen for a therapeutic depends on several factors, including the serum or tissue turnover rate of the entity, the level of symptoms, the immunogenicity of the entity, and the accessibility of the target cells in the biological matrix. In certain embodiments, an administration regimen maximizes the amount of therapeutic delivered to the patient consistent with an acceptable level of side effects. Accordingly, the amount of biologic delivered depends in part on the particular entity and the severity of the condition being treated. Guidance in selecting appropriate doses of antibodies, cytokines, and small molecules are available (*see, e.g.,* Wawrzynczak, Antibody Therapy, Bios Scientific Pub. Ltd, Oxfordshire, UK, 1996; Kresina (ed.), Monoclonal Antibodies, Cytokines and Arthritis, Marcel Dekker, New York, N.Y., 1991; Bach (ed.), Monoclonal Antibodies and Peptide Therapy in Autoimmune Diseases, Marcel Dekker, New York, N.Y., 1993; Baert et al., New Engl. J. Med. 348:601-608, 2003; Milgrom et al., New Engl. J. Med. 341:1966-1973, 1999; Slamon et al., New Engl. J. Med. 344:783-792, 2001; Beniaminovitz et al., New Engl. J. Med. 342:613-619, 2000; Ghosh et al., New Engl. J. Med. 348:24-32, 2003; Lipsky et al., New Engl. J. Med. 343:1594-1602, 2000).

Determination of the appropriate dose is made by the clinician, *e*.*g*., using parameters or factors known or suspected in the art to affect treatment or prevention or predicted to affect treatment or prevention. Generally, the dose begins with an amount somewhat less than the optimum dose and it is increased by small increments thereafter until the desired or optimum effect is achieved relative to any negative side effects. Important diagnostic measures include those of symptoms of, *e.g.,* the inflammation or level of inflammatory cytokines produced.

Actual dosage levels of the active ingredients in the pharmaceutical compositions of the present disclosure may be varied so as to obtain an amount of the active ingredient which is effective to achieve the desired therapeutic response for a particular patient, composition, and mode of administration, without being toxic to the patient. The selected dosage level will depend upon a variety of pharmacokinetic factors including the activity of the particular compositions of the present disclosure employed, or the ester, salt or amide thereof, the route of administration, the time of administration, the rate of excretion of the particular compound being employed, the duration of the treatment, other drugs, compounds and/or materials used in combination with the particular compositions employed, the age, sex, weight, condition, general health and prior medical history of the patient being treated, and like factors known in the medical arts.

Compositions comprising immunoconjugates of the disclosure can be provided by continuous infusion, or by doses at intervals of, *e.g.,* one day, one week, or 1-7 times per week, once every other week, once every three weeks, once every four weeks, once every five weeks, once every six weeks, once every seven weeks, or once very eight weeks. Doses may be provided intravenously, subcutaneously, topically, orally, nasally, rectally, intramuscular, intracerebrally, or by inhalation. A specific dose protocol is one involving the maximal dose or dose frequency that avoids significant undesirable side effects.

For the immunoconjugates of the disclosure, the dosage administered to a patient may be about 0.0001 mg/kg to about 100 mg/kg of the patient's body weight. The dosage may be between about 0.0001 mg/kg and about 30 mg/kg, about 0.0001 mg/kg and about 20 mg/kg, about 0.0001 mg/kg and about 10 mg/kg, about 0.0001 mg/kg and about 5 mg/kg, 0.0001 and about 2 mg/kg, about 0.0001 and about 1 mg/kg, about 0.0001 mg/kg and about 0.75 mg/kg, about 0.0001 mg/kg and about 0.5 mg/kg, about 0.0001 mg/kg to about 0.25 mg/kg, about 0.0001 to about 0.15 mg/kg, about 0.0001 to about 0.10 mg/kg, about 0.001 to about 0.5 mg/kg, about 0.01 to about 0.25 mg/kg, or about 0.01 to about 0.10 mg/kg of the patient's body weight. The dosage of the immunoconjugates of the disclosure may be calculated using the patient's weight in kilograms (kg) multiplied by the dose to be administered in mg/kg.

In some embodiments, the dosage is about 1 mg/kg to about 20 mg/kg, *e*.*g*., about 2 mg/kg to about 16 mg/kg, about 2 mg/kg to about 12 mg/kg, about 4 mg/kg to about 8 mg/kg, about 1 mg/kg to about 12 mg/kg, about 1 mg/kg to about 8 mg/kg, about 1 mg/kg to about 4 mg/kg, about 1 mg/kg to about 2 mg/kg, about 2 mg/kg to about 16 mg/kg, about 4 mg/kg to about 16 mg/kg, about 8 mg/kg to about 16 mg/kg, about 12 mg/kg to about 16 mg/kg, about 1 mg/kg to 3 mg/kg, about 3 mg/kg to 5 mg/kg, about 7 mg/kg to 9 mg/kg, about 11 mg/kg to 13 mg/kg, or about 15 mg/kg to about 16 mg/kg. In some embodiments, the dosage is about 2 mg/kg to about 15 mg/kg. In some embodiments, the dosage is about 1 mg/kg, about 2 mg/kg, about 4 mg/kg, about 8 mg/kg, about 12 mg/kg, or about 16 mg/kg. In some embodiments, the dosage is about 1 mg/kg. In some embodiments, the dosage is about 2 mg/kg. In some embodiments, the dosage is about 4 mg/kg. In some embodiments, the dosage is about 8 mg/kg. In some embodiments, the dosage is about 12 mg/kg. In some embodiments, the dosage is about 16 mg/kg.

Doses of the immunoconjugates the disclosure may be repeated and the administrations may be separated by less than about 1 day, at least about 1 day, about 2 days, about 3 days, about 5 days, about 10 days, about 15 days, about 30 days, about 45 days, about 2 months, about 75 days, about 3 months, about 4 months, about 5 months, or at least about 6 months. In some embodiments, the immunoconjugates of the disclosure may be given twice weekly, once weekly, once every two weeks, once every three weeks, once every four weeks, or less frequently. In a specific embodiment, doses of the immunoconjugates of the disclosure are repeated every 2 weeks.

In some embodiments, the immunoconjugates of the disclosure are administered at a dosage of about 2 mg/kg to about 15 mg/kg intravenously once every two weeks. In some embodiments, the immunoconjugates of the disclosure are administered at a dosage of about 1 mg/kg, about 2 mg/kg, about 4 mg/kg, about 8 mg/kg, about 12 mg/kg, or about 16 mg/kg, intravenously, once every two weeks. In some embodiments, the immunoconjugates of the disclosure are administered at a dosage of about 2 mg/kg to about 15 mg/kg intravenously once a week. In some embodiments, the immunoconjugates of the disclosure are administered at a dosage of about 1 mg/kg, about 2 mg/kg, about 4 mg/kg, about 8 mg/kg, about 12 mg/kg, or about 16 mg/kg, intravenously, once a week. In some embodiments, the immunoconjugates of the disclosure are administered at a dosage of about 2 mg/kg to about 15 mg/kg intravenously once every four weeks. In some embodiments, the immunoconjugates of the disclosure are administered at a dosage of about 1 mg/kg, about 2 mg/kg, about 4 mg/kg, about 8 mg/kg, about 12 mg/kg, or about 16 mg/kg, intravenously, once every four weeks.

An effective amount for a particular patient may vary depending on factors such as the condition being treated, the overall health of the patient, the method, route and dose of administration and the severity of side effects (*see, e.g.,* Maynard et al., A Handbook of SOPs for Good Clinical Practice, Interpharm Press, Boca Raton, Fla., 1996; Dent, Good Laboratory and Good Clinical Practice, Urch Publ., London, UK, 2001).

The route of administration may be by, *e*.*g*., topical or cutaneous application, injection or infusion by subcutaneous, intravenous, intraperitoneal, intracerebral, intramuscular, intraocular, intraarterial, intracerebrospinal, intralesional administration, or by sustained release systems or an implant *(see, e.g.,* Sidman et al., Biopolymers 22:547-556, 1983; Langer et al., J. Biomed. Mater. Res. 15:167-277, 1981; Langer, Chem. Tech. 12:98-105, 1982; Epstein et al., Proc. Natl. Acad. Sci. USA 82:3688-3692, 1985; Hwang et al., Proc. Natl. Acad. Sci. USA 77:4030-4034, 1980; U.S. Pat. Nos. 6,350,466 and 6,316,024). Where necessary, the composition may also include a solubilizing agent or a local anesthetic such as lidocaine to ease pain at the site of the injection, or both. In addition, pulmonary administration can also be employed, *e*.*g*., by use of an inhaler or nebulizer, and formulation with an aerosolizing agent. *See, e.g.,* U.S. Pat. Nos. 6,019,968, 5,985,320, 5,985,309, 5,934,272, 5,874,064, 5,855,913, 5,290,540, and 4,880,078; and PCT Publication Nos. WO 92/19244, WO 97/32572, WO 97/44013, WO 98/31346, and WO 99/66903, each of which is incorporated herein by reference their entirety.

A composition of the present disclosure may also be administered via one or more routes of administration using one or more of a variety of methods known in the art. As will be appreciated by the skilled artisan, the route and/or mode of administration will vary depending upon the desired results. Selected routes of administration for the immunoconjugates of the disclosure include intravenous, intramuscular, intradermal, intraperitoneal, subcutaneous, spinal or other parenteral routes of administration, for example by injection or infusion. Parenteral administration may represent modes of administration other than enteral and topical administration, usually by injection, and includes, without limitation, intravenous, intramuscular, intraarterial, intrathecal, intracapsular, intraorbital, intracardiac, intradermal, intraperitoneal, transtracheal, subcutaneous, subcuticular, intraarticular, subcapsular, subarachnoid, intraspinal, epidural and intrasternal injection and infusion. Alternatively, a composition of the disclosure can be administered via a non-parenteral route, such as a topical, epidermal or mucosal route of administration, for example, intranasally, orally, vaginally, rectally, sublingually or topically. In one embodiment, the immunoconjugates of the disclosure is administered by infusion. In another embodiment, the immunoconjugates of the disclosure is administered subcutaneously.

If the immunoconjugates of the disclosure are administered in a controlled release or sustained release system, a pump may be used to achieve controlled or sustained release (*see* Langer, *supra*; Sefton, CRC Crit. Ref Biomed. Eng. 14:20, 1987; Buchwald et al., Surgery 88:507, 1980; Saudek et al., N. Engl. J. Med. 321:574, 1989). Polymeric materials can be used to achieve controlled or sustained release of the therapies of the disclosure (*see, e.g.,* Medical Applications of Controlled Release, Langer and Wise (eds.), CRC Pres., Boca Raton, Fla., 1974; Controlled Drug Bioavailability, Drug Product Design and Performance, Smolen and Ball (eds.), Wiley, New York, 1984; Ranger and Peppas, J. Macromol. Sci. Rev. Macromol. Chem. 23:61, 1983; see also Levy et al., Science 228:190, 1985; During et al., Ann. Neurol. 25:351, 1989; Howard et al., J. Neurosurg. 7 1:105, 1989; U.S. Pat. No. 5,679,377; U.S. Pat. No. 5,916,597; U.S. Pat. No. 5,912,015; U.S. Pat. No. 5,989,463; U.S. Pat. No. 5,128,326; PCT Publication No. WO 99/15154; and PCT Publication No. WO 99/20253. Examples of polymers used in sustained release formulations include, but are not limited to, poly(2-hydroxy ethyl methacrylate), poly(methyl methacrylate), poly(acrylic acid), poly(ethylene-co-vinyl acetate), poly(methacrylic acid), polyglycolides (PLG), polyanhydrides, poly(N-vinyl pyrrolidone), poly(vinyl alcohol), polyacrylamide, polyethylene glycol), polylactides (PLA), poly(lactide-co-glycolides) (PLGA), and polyorthoesters. In one embodiment, the polymer used in a sustained release formulation is inert, free of leachable impurities, stable on storage, sterile, and biodegradable. A controlled or sustained release system can be placed in proximity of the prophylactic or therapeutic target, thus requiring only a fraction of the systemic dose (see, *e.g.,* Goodson, in Medical Applications of Controlled Release, supra, vol. 2, pp. 115-138, 1984).

Controlled release systems are discussed in the review by Langer, Science 249:1527-1533, 1990). Any technique known to one of skill in the art can be used to produce sustained release formulations comprising one or more immunoconjugates of the disclosure. *See, e.g.,* U.S. Pat. No. 4,526,938, PCT publication WO 91/05548, PCT publication WO 96/20698, Ning et al., Radiotherapy & Oncology 39:179-189, 1996; Song et al., PDA Journal of Pharmaceutical Science & Technology 50:372-397, 1995; Cleek et al., Pro. Int'l. Symp. Control. Rel. Bioact. Mater. 24:853-854, 1997; and Lam et al., Proc. Int'l. Symp. Control Rel. Bioact. Mater. 24:759-760, 1997, each of which is incorporated herein by reference in their entirety.

If the immunoconjugates of the disclosure are administered topically, they can be formulated in the form of an ointment, cream, transdermal patch, lotion, gel, spray, aerosol, solution, emulsion, or other form well-known to one of skill in the art. *See, e.g.,* Remington's Pharmaceutical Sciences and Introduction to Pharmaceutical Dosage Forms, 19th ed., Mack Pub. Co., Easton, Pa. (1995). For non-sprayable topical dosage forms, viscous to semi-solid or solid forms comprising a carrier or one or more excipients compatible with topical application and having a dynamic viscosity, in some instances, greater than water are typically employed. Suitable formulations include, without limitation, solutions, suspensions, emulsions, creams, ointments, powders, liniments, salves, and the like, which are, if desired, sterilized or mixed with auxiliary agents (*e*.*g*., preservatives, stabilizers, wetting agents, buffers, or salts) for influencing various properties, such as, for example, osmotic pressure. Other suitable topical dosage forms include sprayable aerosol preparations wherein the active ingredient, in some instances, in combination with a solid or liquid inert carrier, is packaged in a mixture with a pressurized volatile (*e*.*g*., a gaseous propellant, such as freon) or in a squeeze bottle. Moisturizers or humectants can also be added to pharmaceutical compositions and dosage forms if desired. Examples of such additional ingredients are well-known in the art.

If the compositions comprising the immunoconjugates are administered intranasally, it can be formulated in an aerosol form, spray, mist or in the form of drops. In particular, prophylactic or therapeutic agents for use according to the present disclosure can be conveniently delivered in the form of an aerosol spray presentation from pressurized packs or a nebulizer, with the use of a suitable propellant (*e*.*g*., dichlorodifluoromethane, trichlorofluoromethane, dichlorotetrafluoroethane, carbon dioxide or other suitable gas). In the case of a pressurized aerosol the dosage unit may be determined by providing a valve to deliver a metered amount. Capsules and cartridges (composed of, *e.g.,* gelatin) for use in an inhaler or insufflator may be formulated containing a powder mix of the compound and a suitable powder base such as lactose or starch.

Methods for co-administration or treatment with a second therapeutic agent, *e.g.,* a cytokine, steroid, chemotherapeutic agent, antibiotic, or radiation, are known in the art *(see, e.g.,* Hardman et al., (eds.) (2001) Goodman and Gilman's The Pharmacological Basis of Therapeutics, 10th ed., McGraw-Hill, New York, N.Y.; Poole and Peterson (eds.) (2001) Pharmacotherapeutics for Advanced Practice:A Practical Approach, Lippincott, Williams & Wilkins, Phila., Pa.; Chabner and Longo (eds.) (2001) Cancer Chemotherapy and Biotherapy, Lippincott, Williams & Wilkins, Phila., Pa.). An effective amount of therapeutic may decrease the symptoms by at least 10%; by at least 20%; at least about 30%; at least 40%, or at least 50%.

Additional therapies (*e*.*g*., prophylactic or therapeutic agents), which can be administered in combination with the immunoconjugates of the disclosure may be administered less than 5 minutes apart, less than 30 minutes apart, 1 hour apart, at about 1 hour apart, at about 1 to about 2 hours apart, at about 2 hours to about 3 hours apart, at about 3 hours to about 4 hours apart, at about 4 hours to about 5 hours apart, at about 5 hours to about 6 hours apart, at about 6 hours to about 7 hours apart, at about 7 hours to about 8 hours apart, at about 8 hours to about 9 hours apart, at about 9 hours to about 10 hours apart, at about 10 hours to about 11 hours apart, at about 11 hours to about 12 hours apart, at about 12 hours to 18 hours apart, 18 hours to 24 hours apart, 24 hours to 36 hours apart, 36 hours to 48 hours apart, 48 hours to 52 hours apart, 52 hours to 60 hours apart, 60 hours to 72 hours apart, 72 hours to 84 hours apart, 84 hours to 96 hours apart, or 96 hours to 120 hours apart from the immunoconjugates of the disclosure. The two or more therapies may be administered within one same patient visit.

The disclosure provides protocols for the administration of pharmaceutical composition comprising immunoconjugates of the disclosure alone or in combination with other therapies to a subject in need thereof. The therapies (*e*.*g*., prophylactic or therapeutic agents) of the combination therapies of the present disclosure can be administered concomitantly or sequentially to a subject. The therapy (*e*.*g*., prophylactic or therapeutic agents) of the combination therapies of the present disclosure can also be cyclically administered. Cycling therapy involves the administration of a first therapy (*e*.*g*., a first prophylactic or therapeutic agent) for a period of time, followed by the administration of a second therapy (*e.g*., a second prophylactic or therapeutic agent) for a period of time and repeating this sequential administration, *i.e.,* the cycle, in order to reduce the development of resistance to one of the therapies (*e*.*g*., agents) to avoid or reduce the side effects of one of the therapies (*e*.*g*., agents), and/or to improve, the efficacy of the therapies.

The therapies (*e*.*g*., prophylactic or therapeutic agents) of the combination therapies of the disclosure can be administered to a subject concurrently.

The term "concurrently" is not limited to the administration of therapies (*e*.*g*., prophylactic or therapeutic agents) at exactly the same time, but rather it is meant that a pharmaceutical composition comprising antibodies or fragments thereof the disclosure are administered to a subject in a sequence and within a time interval such that the antibody drug conjugates of the disclosure can act together with the other therapy(ies) to provide an increased benefit than if they were administered otherwise. For example, each therapy may be administered to a subject at the same time or sequentially in any order at different points in time; however, if not administered at the same time, they should be administered sufficiently close in time so as to provide the desired therapeutic or prophylactic effect. Each therapy can be administered to a subject separately, in any appropriate form and by any suitable route. In various embodiments, the therapies (*e*.*g*., prophylactic or therapeutic agents) are administered to a subject less than 5 minutes apart, less than 15 minutes apart, less than 30 minutes apart, less than 1 hour apart, at about 1 hour apart, at about 1 hour to about 2 hours apart, at about 2 hours to about 3 hours apart, at about 3 hours to about 4 hours apart, at about 4 hours to about 5 hours apart, at about 5 hours to about 6 hours apart, at about 6 hours to about 7 hours apart, at about 7 hours to about 8 hours apart, at about 8 hours to about 9 hours apart, at about 9 hours to about 10 hours apart, at about 10 hours to about 11 hours apart, at about 11 hours to about 12 hours apart, 24 hours apart, 48 hours apart, 72 hours apart, or 1 week apart. In other embodiments, two or more therapies (*e*.*g*., prophylactic or therapeutic agents) are administered to a within the same patient visit.

The prophylactic or therapeutic agents of the combination therapies can be administered to a subject in the same pharmaceutical composition. Alternatively, the prophylactic or therapeutic agents of the combination therapies can be administered concurrently to a subject in separate pharmaceutical compositions. The prophylactic or therapeutic agents may be administered to a subject by the same or different routes of administration. The prophylactic or therapeutic agents of the combination therapies can be administered to a subject in the same pharmaceutical composition. Alternatively, the prophylactic or therapeutic agents of the combination therapies can be administered concurrently to a subject in separate pharmaceutical compositions. The prophylactic or therapeutic agents may be administered to a subject by the same or different routes of administration.

The disclosure also includes the following enumerated embodiments.

### Enumerated Embodiments

1. A microorganism, comprising a nucleic acid that comprises a nucleotide sequence encoding a polypeptide associated with the production of a compound having the structure of Formula (A1), wherein the nucleotide sequence is operably linked to a non-native promoter.
2. The microorganism of embodiment 1, which is a genetically engineered form of a microorganism that naturally produces the compound.
3. The microorganism of any of embodiments 1 or 2, which is a bacterium, *e.g., Chromobacterium.*
4. The microorganism of any of embodiments 1-3, which is *Chromobacterium vaccinii, e.g., Chromobacterium vaccinii* DSM 25150 (= ATCC BAA-2314, = NRRL B-50840, = MWU205).
5. The microorganism of any of embodiments 1-4, which is an isolated microorganism or a synthetic microorganism.
6. The microorganism of any of embodiments 1-5, wherein the nucleic acid is located on a chromosome of the microorganism, *e*.*g*., naturally located on the chromosome or stably integrated to the chromosome.
7. The microorganism of any of embodiments 1-6, wherein the nucleotide sequence encoding a polypeptide associated with the production of the compound is located in a biosynthetic gene cluster (BGC).
8. The microorganism of any of embodiments 1-7, wherein the BGC is a compound (A1)-BGC, *e.g.,* shown in FIG. 1.
9. The microorganism of any of embodiments 1-8, wherein the BGC comprises one or more (*e*.*g*., two, three, four, five, six, seven, or all) of *frsA, frsB, frsC, frsD, frsE, frsF, frsG,* or *frsH,* or a homolog thereof.
10. The microorganism of any of embodiments 1-9, wherein the BGC comprises frsA, *frsB, frsC, frsD, frsE, frsF, frsG,* and *frsH,* or a homolog thereof.
11. The microorganism of any of embodiments 1-10, wherein the nucleotide sequence encoding a polypeptide associated with the production of the compound is located in *frsA, frsB, frsC, frsD, frsE, frsF, frsG,* or *frsH,* or a homolog thereof.
12. The microorganism of any of embodiments 1-11, wherein the nucleotide sequence encoding a polypeptide associated with the production of the compound is located in a region comprising *frsA* or a homolog thereof.
13. The microorganism of any of embodiments 1-12, wherein the nucleotide sequence encoding a polypeptide associated with the production of the compound is located in a region comprising *frsA and frsB,* or a homolog thereof.
14. The microorganism of any of embodiments 1-13, wherein the nucleotide sequence encoding a polypeptide associated with the production of the compound is located in a region comprising *frsA*, *frsB, and frsC,* or a homolog thereof.
15. The microorganism of any of embodiments 1-14, wherein the nucleotide sequence encoding a polypeptide associated with the production of the compound is located in a region comprising *frsA*, *frsB, frsC,* and *frsD,* or a homolog thereof.
16. The microorganism of any of embodiments 1-15, wherein the nucleotide sequence encoding a polypeptide associated with the production of the compound is located in a region comprising *frsA*, *frsB, frsC, frsD,* and *frsE,* or a homolog thereof.
17. The microorganism of any of embodiments 1-16, wherein the nucleotide sequence encoding a polypeptide associated with the production of the compound is located in a region comprising *frsA*, *frsB, frsC, frsD, frsE,* and *frsF,* or a homolog thereof.
18. The microorganism of any of embodiments 1-17, wherein the nucleotide sequence encoding a polypeptide associated with the production of the compound is located in a region comprising *frsA*, *frsB, frsC, frsD, frsE, frsF,* and *frsG,* or a homolog thereof.
19. The microorganism of any of embodiments 1-18, wherein the nucleotide sequence encoding a polypeptide associated with the production of the compound is located in a region comprising *frsA*, *frsB, frsC, frsD, frsE, frsF, frsG,* and *FrsH,* or a homolog thereof.
20. The microorganism of any of embodiments 1-19, wherein the nucleotide sequence encoding a polypeptide associated with the production of the compound comprises the coding sequence *offrsA, frsB, frsC, frsD, frsE, frsF, frsG, frsH,* or a homolog thereof.
21. The microorganism of any of embodiments 1-20, wherein the nucleotide sequence encoding a polypeptide associated with the production of the compound comprises the coding sequence of *frsA* or a homolog thereof.
22. The microorganism of any of embodiments 1-21, wherein the nucleotide sequence encoding a polypeptide associated with the production of the compound comprises the coding sequence *offrsA and frsB,* or a homolog thereof.
23. The microorganism of any of embodiments 1-22, wherein the nucleotide sequence encoding a polypeptide associated with the production of the compound comprises the coding sequence *offrsA, frsB, and frsC,* or a homolog thereof.
24. The microorganism of any of embodiments 1-23, wherein the nucleotide sequence encoding a polypeptide associated with the production of the compound comprises the coding sequence of *frsA, frsB, frsC,* and *frsD,* or a homolog thereof.
25. The microorganism of any of embodiments 1-24, wherein the nucleotide sequence encoding a polypeptide associated with the production of the compound comprises the coding sequence of *frsA, frsB, frsC, frsD,* and *frsE,* or a homolog thereof.
26. The microorganism of any of embodiments 1-25, wherein the nucleotide sequence encoding a polypeptide associated with the production of the compound comprises the coding sequence of*frsA, frsB, frsC, frsD, frsE,* and *frsF,* or a homolog thereof.
27. The microorganism of any of embodiments 1-26, wherein the nucleotide sequence encoding a polypeptide associated with the production of the compound comprises the coding sequence of*frsA, frsB, frsC, frsD, frsE, frsF,* and *frsG,* or a homolog thereof.
28. The microorganism of any of embodiments 1-27, wherein the nucleotide sequence encoding a polypeptide associated with the production of the compound comprises the coding sequence of*frsA, frsB, frsC, frsD, frsE, frsF, frsG,* and *FrsH,* or a homolog thereof.
29. The microorganism of any of embodiments 1-28, wherein the nucleic acid comprises a nucleotide sequence resulted from a homologous recombination event (*e*.*g*., for promoter exchange) using the nucleotide sequence of SEQ ID NO: 310, or a functional fragment thereof, or a nucleotide sequence having at least 80%, 85%, 90%, 95%, 96%, 97%, 98%, or 99% identity thereto, or differing by no more than 100, 90, 80, 70, 60, 50, 40, 30, 20, 15, 10, 5, or 2 nucleotides therefrom.
30. The microorganism of any of embodiments 1-29, wherein the nucleic acid comprises a nucleotide sequence associated GenBank accession number: BankIt2437961 BSeq#1 MW732719, or a functional fragment thereof, or a nucleotide sequence having at least 75%, 80%, 85%, 90%, 95%, 96%, 97%, 98%, or 99% identity thereto.
31. The microorganism of any of embodiments 1-30, wherein the non-native promoter is from the same microorganism.
32. The microorganism of any of embodiments 1-31, wherein the non-native promoter is from a different microorganism.
33. The microorganism of any of embodiments 1-32, wherein the non-native promoter comprises a *vioP* promoter, an *nptII* promoter, an *rbs* promoter, a *J23119* promoter, a *pLpp* promoter, a *PS12burk* promoter, a *Pem7,* or an *ErmE** promoter, optionally, wherein the non-native promoter comprises a *vioP* promoter, an *nptII* promoter, or an *rbs* promoter.
34. The microorganism of any of embodiments 1-33, wherein the non-native promoter comprises the nucleotide sequence of any of SEQ ID NOs: 264-266, 268-271, or 316, or a functional fragment thereof, or a nucleotide sequence having at least 80%, 85%, 90%, 95%, 96%, 97%, 98%, or 99% identity thereto, or differing by no more than 60, 50, 40, 30, 20, 15, 10, 5, or 2 nucleotides therefrom.
35. The microorganism of any of embodiments 1-34, wherein the non-native promoter comprises or consists of the nucleotide sequence of any of SEQ ID NOs: 264-266, 268-271, or 316, or a functional fragment thereof.
36. The microorganism of any of embodiments 1-35, wherein the non-native promoter comprises a *vioP* promoter.
37. The microorganism of any of embodiments 1-36, wherein the non-native promoter comprises the nucleotide sequence of SEQ ID NO: 264, or a functional fragment thereof, or a nucleotide sequence having at least 80%, 85%, 90%, 95%, 96%, 97%, 98%, or 99% identity thereto, or differing by no more than 60, 50, 40, 30, 20, 15, 10, 5, or 2 nucleotides therefrom.
38. The microorganism of any of embodiments 1-37, wherein the non-native promoter comprises or consists of the nucleotide sequence of SEQ ID NO: 264, or a functional fragment thereof.
39. The microorganism of any of embodiments 1-38, wherein the non-native promoter comprises an *nptII* promoter.
40. The microorganism of any of embodiments 1-39, wherein the non-native promoter comprises the nucleotide sequence of SEQ ID NO: 265, or a functional fragment thereof, or a nucleotide sequence having at least 80%, 85%, 90%, 95%, 96%, 97%, 98%, or 99% identity thereto, or differing by no more than 60, 50, 40, 30, 20, 15, 10, 5, or 2 nucleotides therefrom.
41. The microorganism of any of embodiments 1-40, wherein the non-native promoter comprises or consists of the nucleotide sequence of SEQ ID NO: 265, or a functional fragment thereof.
42. The microorganism of any of embodiments 1-41, wherein the non-native promoter comprises an *rbs* promoter.
43. The microorganism of any of embodiments 1-42, wherein the non-native promoter comprises the nucleotide sequence of SEQ ID NO: 266, or a functional fragment thereof, or a nucleotide sequence having at least 80%, 85%, 90%, 95%, 96%, 97%, 98%, or 99% identity thereto, or differing by no more than 60, 50, 40, 30, 20, 15, 10, 5, or 2 nucleotides therefrom.
44. The microorganism of any of embodiments 1-43, wherein the non-native promoter comprises or consists of the nucleotide sequence of SEQ ID NO: 266, or a functional fragment thereof.
45. The microorganism of any of embodiments 1-44, wherein the non-native promoter comprises a *J23119* promoter.
46. The microorganism of any of embodiments 1-45, wherein the non-native promoter comprises the nucleotide sequence of SEQ ID NO: 316, or a functional fragment thereof, or a nucleotide sequence having at least 80%, 85%, 90%, 95%, 96%, 97%, 98%, or 99% identity thereto, or differing by no more than 60, 50, 40, 30, 20, 15, 10, 5, or 2 nucleotides therefrom.
47. The microorganism of any of embodiments 1-46, wherein the non-native promoter comprises or consists of the nucleotide sequence of SEQ ID NO: 316, or a functional fragment thereof.
48. The microorganism of any of embodiments 1-47, wherein the non-native promoter comprises a *pLpp* promoter.
49. The microorganism of any of embodiments 1-48, wherein the non-native promoter comprises the nucleotide sequence of SEQ ID NO: 268, or a functional fragment thereof, or a nucleotide sequence having at least 80%, 85%, 90%, 95%, 96%, 97%, 98%, or 99% identity thereto, or differing by no more than 60, 50, 40, 30, 20, 15, 10, 5, or 2 nucleotides therefrom.
50. The microorganism of any of embodiments 1-49, wherein the non-native promoter comprises or consists of the nucleotide sequence of SEQ ID NO: 268, or a functional fragment thereof.
51. The microorganism of any of embodiments 1-50, wherein the non-native promoter comprises a *Pem7* promoter.
52. The microorganism of any of embodiments 1-51, wherein the non-native promoter comprises the nucleotide sequence of SEQ ID NO: 269, or a functional fragment thereof, or a nucleotide sequence having at least 80%, 85%, 90%, 95%, 96%, 97%, 98%, or 99% identity thereto, or differing by no more than 60, 50, 40, 30, 20, 15, 10, 5, or 2 nucleotides therefrom.
53. The microorganism of any of embodiments 1-52, wherein the non-native promoter comprises or consists of the nucleotide sequence of SEQ ID NO: 269, or a functional fragment thereof.
54. The microorganism of any of embodiments 1-53, wherein the non-native promoter comprises a *PS12burk* promoter.
55. The microorganism of any of embodiments 1-54, wherein the non-native promoter comprises the nucleotide sequence of SEQ ID NO: 270, or a functional fragment thereof, or a nucleotide sequence having at least 80%, 85%, 90%, 95%, 96%, 97%, 98%, or 99% identity thereto, or differing by no more than 60, 50, 40, 30, 20, 15, 10, 5, or 2 nucleotides therefrom.
56. The microorganism of any of embodiments 1-55, wherein the non-native promoter comprises or consists of the nucleotide sequence of SEQ ID NO: 270, or a functional fragment thereof.
57. The microorganism of any of embodiments 1-56, wherein the non-native promoter comprises an *ErmE** promoter.
58. The microorganism of any of embodiments 1-57, wherein the non-native promoter comprises the nucleotide sequence of SEQ ID NO: 271, or a functional fragment thereof, or a nucleotide sequence having at least 80%, 85%, 90%, 95%, 96%, 97%, 98%, or 99% identity thereto, or differing by no more than 60, 50, 40, 30, 20, 15, 10, 5, or 2 nucleotides therefrom.
59. The microorganism of any of embodiments 1-58, wherein the non-native promoter comprises or consists of the nucleotide sequence of SEQ ID NO: 271, or a functional fragment thereof.
60. The microorganism of any of embodiments 1-59, wherein the non-native promoter controls the transcription of one or more (*e*.*g*., two, three, four, five, six, seven, or all) of *FrsA, FrsB, FrsC, FrsD, FrsE, FrsF, FrsG,* or *FrsH,* or a homolog thereof.
61. The microorganism of any of embodiments 1-60, wherein the non-native promoter controls the transcription of *FrsA, FrsB, FrsC, FrsD, FrsE, FrsF, FrsG,* and *FrsH,* or a homolog thereof.
62. The microorganism of any of embodiments 1-61, wherein the non-native promoter is inserted upstream of the coding region of *FrsA,* or a homolog thereof, *e.g.,* upstream of the coding region of all of *FrsA, FrsB, FrsC, FrsD, FrsE, FrsF, FrsG,* and *FrsH,* or a homolog thereof.
63. The microorganism of any of embodiments 1-62, wherein the polypeptide associated with the production of the compound is a non-ribosomal peptide synthetase (NRPS) or a functional fragment thereof.
64. The microorganism of any of embodiments 1-63, wherein the NRPS is FrsA, FrsD, FrsE, FrsF, or FrsG, or a homolog thereof.
65. The microorganism of any of embodiments 1-64, wherein the polypeptide associated with the production of the compound is an MbtH-like protein or a functional fragment thereof.
66. The microorganism of any of embodiments 1-65, wherein the MbtH-like protein is FrsB or a homolog thereof.
67. The microorganism of any of embodiments 1-66, wherein the polypeptide associated with the production of the compound is a malate dehydrogenase.
68. The microorganism of any of embodiments 1-67, wherein the malate dehydrogenase is FrsC or a homolog thereof.
69. The microorganism of any of embodiments 1-68, wherein the polypeptide associated with the production of the compound is a hydroxylase.
70. The microorganism of any of embodiments 1-69, wherein the hydroxylase is FrsH or a homolog thereof.
71. The microorganism of any of embodiments 1-70, wherein the nucleic acid comprises a plurality of nucleotide sequences encoding a plurality of polypeptides associated with the production of the compound.
72. The microorganism of any of embodiments 1-71, wherein the plurality of polypeptides associated with the production of the compound comprises a plurality of NRPSs, or a functional fragment thereof.
73. The microorganism of any of embodiments 1-72, wherein the plurality of NRPSs comprise two or more (*e*.*g*., three, four, or all) of FrsA, FrsD, FrsE, FrsF, or FrsG, or a homolog thereof.
74. The microorganism of any of embodiments 1-73, wherein the plurality of polypeptides associated with the production of the compound comprises an NRPS, a MbtH-like protein, a malate dehydrogenase, and a hydroxylase, or a functional fragment thereof.
75. The microorganism of any of embodiments 1-74, wherein the plurality of polypeptides associated with the production of the compound comprises FrsA, FrsB, FrsC, FrsD, FrsE, FrsF, FrsG, and FrsH, or a homolog thereof.
76. The microorganism of any of embodiments 1-75, wherein the microorganism has an increased titer of the compound.
77. The microorganism of any of embodiments 1-76, wherein the titer of the compound is increased by at least 1, 2, 3, 4, 5, 6, 7, 8, 9, or 10-fold, compared to a reference microorganism, *e*.*g*., an otherwise identical microorganism that does not comprise the non-native promoter in the compound (A1)-BGC (*e*.*g*., a wild-type), when cultured under conditions that allow production of the compound.
78. The microorganism of any of embodiments 1-77, wherein the titer of the compound is increased by at least about 100 mg/L, 200 mg/L, 300 mg/L, 400 mg/L, 500 mg/L, 600 mg/L, 700 mg/L, 800 mg/L, 900 mg/L, or 1000 mg/L, compared to a reference microorganism, *e*.*g*., an otherwise identical microorganism that does not comprise the non-native promoter in the compound (A1)-BGC (*e*.*g*., a wild-type), when cultured under conditions that allow production of the compound.
79. The microorganism of any of embodiments 1-78, wherein the BGC of a natural product that interferes with the isolation of the compound is altered, *e*.*g*., disrupted.
80. The microorganism of any of embodiments 1-79, wherein the natural product comprises one or more (*e*.*g*., two, three, or all) of: violacein, compound J, compound F5, compound F3, or compound D.
81. The microorganism of any of embodiments 1-80, wherein at least a portion of the BGC of the natural product interferes with the isolation of the compound is deleted.
82. The microorganism of any of embodiments 1-81, wherein the violacein-BGC is altered, *e.g.,* disrupted.
83. The microorganism of any of embodiments 1-82, wherein the compound J-BGC is altered, *e.g.,* disrupted.
84. The microorganism of any of embodiments 1-83, wherein the compound J-BGC is associated with the production of compound J, compound F5, compound F3, and compound D.
85. The microorganism of any of embodiments 1-84, wherein both the violacein-BGC and the compound J-BGC are altered, *e*.*g*., disrupted.
86. The microorganism of any of embodiments 1-85, wherein the production of the natural product is reduced, *e*.*g*., by at least 50%, 60%, 70%, 80%, 90%, 95%, 96%, 97%, 98%, 99%, or 100%.
87. The microorganism of any of embodiments 1-86, wherein the microorganism has an increased isolation yield of the compound.
88. The microorganism of any of embodiments 1-87, wherein the isolation yield of the compound is increased by 20%, 30%, 40%, 50%, 60%, 70%, 80%, 90%, or more, compared to a reference microorganism, *e*.*g*., an otherwise identical microorganism in which the BGC of the natural product that interferes with the isolation of the compound is not altered (*e*.*g*., a wild-type).
89. The microorganism of any of embodiments 1-88, wherein the BGC of a natural product that interferes with the isolation of the compound is not altered, *e.g.,* not disrupted.
90. A microorganism that produces a compound having the structure of Formula (A1), wherein the BGC of a natural product that interferes with the isolation of the compound is altered.
91. The microorganism of embodiment 90, wherein the natural product comprises one or more (*e*.*g*., two, three, or all) of: violacein, compound J, compound F5, compound F3, or compound D.
92. The microorganism of any of embodiments 90 or 91, wherein at least a portion of the BGC of the natural product interferes with the isolation of the compound is deleted.
93. The microorganism of any of embodiments 90-92, wherein the violacein-BGC is altered, *e.g.,* disrupted.
94. The microorganism of any of embodiments 90-93, wherein the compound J-BGC is altered, *e.g.,* disrupted.
95. The microorganism of any of embodiments 90-94, wherein the compound J-BGC is associated with the production of compound J, compound F5, compound F3, and compound D.
96. The microorganism of any of embodiments 90-95, wherein both the violacein-BGC and the compound J-BGC are altered, *e*.*g*., disrupted.
97. The microorganism of any of embodiments 90-96, wherein the production of the natural product is reduced, *e*.*g*., by at least 50%, 60%, 70%, 80%, 90%, 95%, 96%, 97%, 98%, 99%, or 100%.
98. The microorganism of any of embodiments 90-97, wherein the microorganism has an increased isolation yield of the compound.
99. The microorganism of any of embodiments 90-98, wherein the isolation yield of the compound is increased by 20%, 30%, 40%, 50%, 60%, 70%, 80%, 90%, or more, compared to a reference microorganism, *e*.*g*., an otherwise identical microorganism in which the BGC of the natural product that interferes with the isolation of the compound is not altered (*e*.*g*., a wild-type).
100. A method of producing a compound having the structure of Formula (A1), comprising: culturing (*e*.*g*., fermenting) a microorganism of any of embodiments 1-84 under conditions that allow the expression of the compound, thereby producing the compound.
101. The method of embodiment 100, wherein the microorganism is cultured (*e*.*g*., fermented) at 50 L to 500 L scale, *e*.*g*., at 50 L, 100 L, 150 L, 200 L, 250 L, 300 L, 350 L, 400 L, 450 L, or 500 L scale.
102. The method of embodiment 100, wherein the microorganism is cultured (e.g., fermented) at 1,000 L to 20,000 L scale, *e*.*g*., 1,000 L to 10,000 L or 10,000 L to 20,000 L, *e*.*g*., 1,000 L, 2,000 L, 5,000 L, 7,500 L, 10,000 L, 15,000 L, or 20,000 L scale.
103. The method of any of embodiments 100-102, wherein the culturing (*e*.*g*., fermentation) yields a titer of at least 200 mL of the compound, *e*.*g*., at least 250 mg/mL, 300 mg/L, 400 mg/L, 500 mg/L, 600 mg/L, 700 mg/L, 800 mg/L, 900 mg/L, 1000 mg/L, 1100 mg/L, 1200 mg/L, 1300 mg/L, 1400 mg/L, 1500 mg/L, 1600 mg/L, 1700 mg/L, 1800 mg/L, 1900 mg/L, or 2000 mg/L of the compound.
104. The method of any of embodiments 100-103, the method further comprising isolating the compound, *e.g.,* to a purity of at least 90%, *e.g.,* at least 95%, 96%, 97%, 98%, or 99%, *e.g.,* as determined by a method described herein.
105. A nucleic acid comprising a nucleotide sequence encoding a polypeptide associated with the production of a compound having the structure of Formula (A1), wherein the nucleotide sequence is operably linked to a non-native promoter.
106. The nucleic acid of embodiment 105, wherein the nucleic acid is located on a chromosome of the microorganism, *e*.*g*., naturally located on the chromosome or stably integrated to the chromosome.
107. The nucleic acid of any of embodiments 105 or 106, wherein the nucleotide sequence encoding a polypeptide associated with the production of the compound is located in a biosynthetic gene cluster (BGC).
108. The nucleic acid of any of embodiments 105-107, wherein the BGC is a compound (A1)-BGC.
109. The nucleic acid of any of embodiments 105-108, wherein the compound (A1)-BGC has the gene organization shown in FIG. 1.
110. The nucleic acid of any of embodiments 105-109, wherein the BGC comprises one or more (*e*.*g*., two, three, four, five, six, seven, or all) of *frsA, frsB, frsC, frsD, frsE, frsF, frsG,* or *frsH,* or a homolog thereof.
111. The nucleic acid of any of embodiments 105-110, wherein the BGC comprises *frsA*, *frsB, frsC, frsD, frsE, frsF, frsG,* and *frsH,* or a homolog thereof.
112. The nucleic acid of any of embodiments 105-111, wherein the nucleotide sequence encoding a polypeptide associated with the production of the compound is located in *frsA, frsB, frsC, frsD, frsE, frsF, frsG, frsH,* or a homolog thereof.
113. The nucleic acid of any of embodiments 105-112, wherein the nucleotide sequence encoding a polypeptide associated with the production of the compound is located in a region comprising *frsA* or a homolog thereof.
114. The nucleic acid of any of embodiments 105-113, wherein the nucleotide sequence encoding a polypeptide associated with the production of the compound is located in a region comprising *frsA and frsB,* or a homolog thereof.
115. The nucleic acid of any of embodiments 105-114, wherein the nucleotide sequence encoding a polypeptide associated with the production of the compound is located in a region comprising *frsA*, *frsB, and frsC,* or a homolog thereof.
116. The nucleic acid of any of embodiments 105-115, wherein the nucleotide sequence encoding a polypeptide associated with the production of the compound is located in a region comprising *frsA*, *frsB, frsC,* and *frsD,* or a homolog thereof.
117. The nucleic acid of any of embodiments 105-116, wherein the nucleotide sequence encoding a polypeptide associated with the production of the compound is located in a region comprising *frsA*, *frsB, frsC, frsD,* and *frsE,* or a homolog thereof.
118. The nucleic acid of any of embodiments 105-117, wherein the nucleotide sequence encoding a polypeptide associated with the production of the compound is located in a region comprising *frsA*, *frsB, frsC, frsD, frsE,* and *frsF,* or a homolog thereof.
119. The nucleic acid of any of embodiments 105-118, wherein the nucleotide sequence encoding a polypeptide associated with the production of the compound is located in a region comprising *frsA*, *frsB, frsC, frsD, frsE, frsF,* and *frsG,* or a homolog thereof.
120. The nucleic acid of any of embodiments 105-119, wherein the nucleotide sequence encoding a polypeptide associated with the production of the compound is located in a region comprising *frsA*, *frsB, frsC, frsD, frsE, frsF, frsG,* and *FrsH,* or a homolog thereof.
121. The nucleic acid of any of embodiments 105-120, wherein the nucleotide sequence encoding a polypeptide associated with the production of the compound comprises the coding sequence of*frsA, frsB, frsC, frsD, frsE, frsF, frsG, frsH,* or a homolog thereof.
122. The nucleic acid of any of embodiments 105-121, wherein the nucleotide sequence encoding a polypeptide associated with the production of the compound comprises the coding sequence of *frsA* or a homolog thereof.
123. The nucleic acid of any of embodiments 105-122, wherein the nucleotide sequence encoding a polypeptide associated with the production of the compound comprises the coding sequence of*frsA and frsB,* or a homolog thereof.
124. The nucleic acid of any of embodiments 105-123, wherein the nucleotide sequence encoding a polypeptide associated with the production of the compound comprises the coding sequence of*frsA, frsB, and frsC,* or a homolog thereof.
125. The nucleic acid of any of embodiments 105-124, wherein the nucleotide sequence encoding a polypeptide associated with the production of the compound comprises the coding sequence of*frsA, frsB, frsC,* and *frsD,* or a homolog thereof.
126. The nucleic acid of any of embodiments 105-125, wherein the nucleotide sequence encoding a polypeptide associated with the production of the compound comprises the coding sequence of *frsA, frsB, frsC, frsD,* and *frsE,* or a homolog thereof.
127. The nucleic acid of any of embodiments 105-126, wherein the nucleotide sequence encoding a polypeptide associated with the production of the compound comprises the coding sequence of *frsA, frsB, frsC, frsD, frsE,* and *frsF,* or a homolog thereof.
128. The nucleic acid of any of embodiments 105-127, wherein the nucleotide sequence encoding a polypeptide associated with the production of the compound comprises the coding sequence *offrsA, frsB, frsC, frsD, frsE, frsF,* and *frsG,* or a homolog thereof.
129. The nucleic acid of any of embodiments 105-128, wherein the nucleotide sequence encoding a polypeptide associated with the production of the compound comprises the coding sequence of*frsA, frsB, frsC, frsD, frsE, frsF, frsG,* and *FrsH,* or a homolog thereof.
130. The nucleic acid of any of embodiments 105-129, wherein the nucleic acid comprises a nucleotide sequence resulted from a homologous recombination event (*e*.*g*., for promoter exchange) using the nucleotide sequence of SEQ ID NO: 310, or a functional fragment thereof, or a nucleotide sequence having at least 80%, 85%, 90%, 95%, 96%, 97%, 98%, or 99% identity thereto, or differing by no more than 100, 90, 80, 70, 60, 50, 40, 30, 20, 15, 10, 5, or 2 nucleotides therefrom.
131. The nucleic acid of any of embodiments 105-130, wherein the nucleic acid comprises a nucleotide sequence associated GenBank accession number: BankIt2437961 BSeq#1 MW732719, or a functional fragment thereof, or a nucleotide sequence having at least 75%, 80%, 85%, 90%, 95%, 96%, 97%, 98%, or 99% identity thereto.
132. The nucleic acid of any of embodiments 105-131, wherein the non-native promoter comprises a *vioP* promoter, an *nptII* promoter, an *rbs* promoter, a *J23119* promoter, a *pLpp* promoter, a *PS12burk* promoter, a *Pem7,* or an *ErmE** promoter, optionally, wherein the non-native promoter comprises a *vioP* promoter, an *nptII* promoter, or an *rbs* promoter.
133. The nucleic acid of any of embodiments 105-132, wherein the non-native promoter comprises the nucleotide sequence of any of SEQ ID NOs: 264-266, 268-271, or 316, or a functional fragment thereof, or a nucleotide sequence having at least 80%, 85%, 90%, 95%, 96%, 97%, 98%, or 99% identity thereto, or differing by no more than 60, 50, 40, 30, 20, 15, 10, 5, or 2 nucleotides therefrom.
134. The nucleic acid of any of embodiments 105-133, wherein the non-native promoter comprises or consists of the nucleotide sequence of any of SEQ ID NOs: 264-266, 268-271, or 316, or a functional fragment thereof.
135. The nucleic acid of any of embodiments 105-134, wherein the non-native promoter comprises a *vioP* promoter.
136. The nucleic acid of any of embodiments 105-135, wherein the non-native promoter comprises the nucleotide sequence of SEQ ID NO: 264, or a functional fragment thereof, or a nucleotide sequence having at least 80%, 85%, 90%, 95%, 96%, 97%, 98%, or 99% identity thereto, or differing by no more than 60, 50, 40, 30, 20, 15, 10, 5, or 2 nucleotides therefrom.
137. The nucleic acid of any of embodiments 105-136, wherein the non-native promoter comprises or consists of the nucleotide sequence of SEQ ID NO: 264, or a functional fragment thereof.
138. The nucleic acid of any of embodiments 105-137 wherein the non-native promoter comprises an *nptII* promoter.
139. The nucleic acid of any of embodiments 105-138, wherein the non-native promoter comprises the nucleotide sequence of SEQ ID NO: 265, or a functional fragment thereof, or a nucleotide sequence having at least 80%, 85%, 90%, 95%, 96%, 97%, 98%, or 99% identity thereto, or differing by no more than 60, 50, 40, 30, 20, 15, 10, 5, or 2 nucleotides therefrom.
140. The nucleic acid of any of embodiments 105-139, wherein the non-native promoter comprises or consists of the nucleotide sequence of SEQ ID NO: 265, or a functional fragment thereof.
141. The nucleic acid of any of embodiments 105-140, wherein the non-native promoter comprises an *rbs* promoter.
142. The nucleic acid of any of embodiments 105-141, wherein the non-native promoter comprises the nucleotide sequence of SEQ ID NO: 266, or a functional fragment thereof, or a nucleotide sequence having at least 80%, 85%, 90%, 95%, 96%, 97%, 98%, or 99% identity thereto, or differing by no more than 60, 50, 40, 30, 20, 15, 10, 5, or 2 nucleotides therefrom.
143. The nucleic acid of any of embodiments 105-142, wherein the non-native promoter comprises or consists of the nucleotide sequence of SEQ ID NO: 266, or a functional fragment thereof.
144. The nucleic acid of any of embodiments 105-143, wherein the non-native promoter comprises a *J23119* promoter.
145. The nucleic acid of any of embodiments 105-144, wherein the non-native promoter comprises the nucleotide sequence of SEQ ID NO: 316, or a functional fragment thereof, or a nucleotide sequence having at least 80%, 85%, 90%, 95%, 96%, 97%, 98%, or 99% identity thereto, or differing by no more than 60, 50, 40, 30, 20, 15, 10, 5, or 2 nucleotides therefrom.
146. The nucleic acid of any of embodiments 105-145, wherein the non-native promoter comprises or consists of the nucleotide sequence of SEQ ID NO: 316, or a functional fragment thereof.
147. The nucleic acid of any of embodiments 105-146, wherein the non-native promoter comprises a *pLpp* promoter.
148. The nucleic acid of any of embodiments 105-147, wherein the non-native promoter comprises the nucleotide sequence of SEQ ID NO: 268, or a functional fragment thereof, or a nucleotide sequence having at least 80%, 85%, 90%, 95%, 96%, 97%, 98%, or 99% identity thereto, or differing by no more than 60, 50, 40, 30, 20, 15, 10, 5, or 2 nucleotides therefrom.
149. The nucleic acid of any of embodiments 105-148, wherein the non-native promoter comprises or consists of the nucleotide sequence of SEQ ID NO: 268, or a functional fragment thereof.
150. The nucleic acid of any of embodiments 105-149, wherein the non-native promoter comprises a *Pem* 7 promoter.
151. The nucleic acid of any of embodiments 105-150, wherein the non-native promoter comprises the nucleotide sequence of SEQ ID NO: 269, or a functional fragment thereof, or a nucleotide sequence having at least 80%, 85%, 90%, 95%, 96%, 97%, 98%, or 99% identity thereto, or differing by no more than 60, 50, 40, 30, 20, 15, 10, 5, or 2 nucleotides therefrom.
152. The nucleic acid of any of embodiments 105-151, wherein the non-native promoter comprises or consists of the nucleotide sequence of SEQ ID NO: 269, or a functional fragment thereof.
153. The nucleic acid of any of embodiments 105-152, wherein the non-native promoter comprises a *PS12burk* promoter.
154. The nucleic acid of any of embodiments 105-153, wherein the non-native promoter comprises the nucleotide sequence of SEQ ID NO: 270, or a functional fragment thereof, or a nucleotide sequence having at least 80%, 85%, 90%, 95%, 96%, 97%, 98%, or 99% identity thereto, or differing by no more than 60, 50, 40, 30, 20, 15, 10, 5, or 2 nucleotides therefrom.
155. The nucleic acid of any of embodiments 105-154, wherein the non-native promoter comprises or consists of the nucleotide sequence of SEQ ID NO: 270, or a functional fragment thereof.
156. The nucleic acid of any of embodiments 105-155, wherein the non-native promoter comprises an *ErmE** promoter.
157. The nucleic acid of any of embodiments 105-156, wherein the non-native promoter comprises the nucleotide sequence of SEQ ID NO: 271, or a functional fragment thereof, or a nucleotide sequence having at least 80%, 85%, 90%, 95%, 96%, 97%, 98%, or 99% identity thereto, or differing by no more than 60, 50, 40, 30, 20, 15, 10, 5, or 2 nucleotides therefrom.
158. The nucleic acid of any of embodiments 105-157, wherein the non-native promoter comprises or consists of the nucleotide sequence of SEQ ID NO: 271, or a functional fragment thereof.
159. The nucleic acid of any of embodiments 105-158, wherein the non-native promoter controls the transcription of one or more (e.g., two, three, four, five, six, seven, or all) of *FrsA, FrsB, FrsC, FrsD, FrsE, FrsF, FrsG,* or *FrsH,* or a homolog thereof.
160. The nucleic acid of any of embodiments 105-159, wherein the non-native promoter controls the transcription of *FrsA, FrsB, FrsC, FrsD, FrsE, FrsF, FrsG,* and *FrsH,* or a homolog thereof.
161. The nucleic acid of any of embodiments 105-160, wherein the non-native promoter is inserted upstream of the coding region of *FrsA,* or a homolog thereof, *e.g.,* upstream of the coding region of all of *FrsA, FrsB, FrsC, FrsD, FrsE, FrsF, FrsG,* and *FrsH,* or a homolog thereof.
162. The nucleic acid of any of embodiments 105-161, wherein the polypeptide associated with the production of the compound is a non-ribosomal peptide synthetase (NRPS) or a functional fragment thereof.
163. The nucleic acid of any of embodiments 105-162, wherein the NRPS is FrsA, FrsD, FrsE, FrsF, or FrsG, or a homolog thereof.
164. The nucleic acid of any of embodiments 105-163, wherein the polypeptide associated with the production of the compound is a MbtH-like protein or a functional fragment thereof.
165. The nucleic acid of any of embodiments 105-164, wherein the MbtH-like protein is FrsB or a homolog thereof.
166. The nucleic acid of any of embodiments 105-165, wherein the polypeptide associated with the production of the compound is a malate dehydrogenase.
167. The nucleic acid of any of embodiments 105-166, wherein the malate dehydrogenase is FrsC or a homolog thereof.
168. The nucleic acid of any of embodiments 105-167, wherein the polypeptide associated with the production of the compound is a hydroxylase.
169. The nucleic acid of any of embodiments 105-168, wherein hydroxylase is FrsH or a homolog thereof.
170. The nucleic acid of any of embodiments 105-169, wherein the nucleic acid comprises a plurality of nucleotide sequences encoding a plurality of polypeptides associated with the production of the compound.
171. The nucleic acid of any of embodiments 105-170, wherein the plurality of polypeptides associated with the production of the compound comprises a plurality of NRPSs, or a functional fragment thereof.
172. The nucleic acid of any of embodiments 105-171, wherein the plurality of NRPSs comprise two or more (e.g., three, four, or all) of FrsA, FrsD, FrsE, FrsF, or FrsG, or a homolog thereof.
173. The nucleic acid of any of embodiments 105-172, wherein the plurality of polypeptides associated with the production of the compound comprises an NRPS, a MbtH-like protein, a malate dehydrogenase, and a hydroxylase, or a functional fragment thereof.
174. The nucleic acid of any of embodiments 105-173, wherein the plurality of polypeptides associated with the production of the compound comprises FrsA, FrsB, FrsC, FrsD, FrsE, FrsF, FrsG, and FrsH, or a homolog thereof.
175. A nucleic acid comprising a nucleotide sequence encoding a polypeptide associated with the production of one or more of: compound J, compound F5, compound F3, or compound D.
176. The nucleic acid of embodiment 175, which is an isolated nucleic acid, a non-naturally occurring nucleic acid, or a synthetic nucleic acid.
177. The nucleic acid of any of embodiments 175 or 176, which comprises a mutation, *e*.*g*., a deletion.
178. The nucleic acid of any of embodiments 175-177, wherein the nucleic acid comprises a plurality of nucleotide sequences encoding a plurality of polypeptides associated with the production of one or more of: compound J, compound F5, compound F3, or compound D.
179. The nucleic acid of any of embodiments 175-178, wherein the nucleotide sequence is from a BGC.
180. The nucleic acid of any of embodiments 175-179, wherein the BGC is a compound J-BGC.
181. The nucleic acid of any of embodiments 175-180, wherein the compound J-BGC has the gene organization shown in FIG. 1.
182. The nucleic acid of any of embodiments 175-181, wherein the nucleotide sequence comprises one or more (e.g., two, three, or all) of *dis1, dis2, dis3,* or *dis4,* or a homolog thereof.
183. The nucleic acid of any of embodiments 175-182, wherein the nucleotide sequence comprises *dis1, dis2, dis3,* and *dis4,* or a homolog thereof.
184. The nucleic acid of any of embodiments 175-183, wherein the nucleic acid comprises a nucleotide sequence associated GenBank accession number: BankIt2437961 BSeq#1 MW732719, or a functional fragment thereof, or a nucleotide sequence having at least 75%, 80%, 85%, 90%, 95%, 96%, 97%, 98%, or 99% identity thereto.
185. A vector comprising a nucleic acid of any of embodiments 105-184.
186. A cell comprising a nucleic acid of any of embodiments 105-184 or a vector of embodiment 185.
187. A method of engineering a cell, comprising: altering a nucleotide sequence encoding a polypeptide associated with the production of one or more of: compound J, compound F5, compound F3, or compound D, thereby engineering the cell.
188. The method of embodiment 187, wherein the cell is a microorganism that naturally produces a compound having the structure of Formula (A1).
189. The method of any of embodiments 187 or 188, wherein the microorganism is a bacterium, *e*.*g*., *Chromobacterium.*
190. The method of any of embodiments 187-189, wherein the microorganism is *Chromobacterium vaccinii, e.g., Chromobacterium vaccinii* DSM 25150 (= ATCC BAA-2314, = NRRL B-50840, = MWU205).
191. The method of any of embodiments 187-190, wherein the nucleotide sequence is disrupted, *e*.*g*., at least a portion of the nucleotide sequence is deleted.
192. The method of any of embodiments 187-191, wherein the nucleic acid comprises a plurality of nucleotide sequences encoding a plurality of polypeptides associated with the production of one or more of: compound J, compound F5, compound F3, or compound D.
193. The method of any of embodiments 187-192, wherein the production of one or more (*e*.*g*., two, three, or all) of: compound J, compound F5, compound F3, or compound D is reduced, *e*.*g*., by at least 50%, 60%, 70%, 80%, 90%, 95%, 96%, 97%, 98%, 99%, or 100%.
194. The method of any of embodiments 187-193, wherein the nucleotide sequence is from a BGC.
195. The method of embodiment 194, wherein the BGC is a compound J-BGC.
196. The method of embodiment 195, wherein the compound J-BGC has the gene organization shown in FIG. 1.
197. The method of any of embodiments 187-196, wherein the nucleotide sequence comprises one or more *(e.g.,* two, three, or all) of *dis1, dis2, dis3,* or *dis4,* or a homolog thereof.
198. The method of any of embodiments 187-197, wherein the nucleotide sequence comprises *dis1, dis2, dis3,* and *dis4,* or a homolog thereof.
199. The method of any of embodiments 187-198, wherein the nucleic acid comprises a nucleotide sequence associated GenBank accession number: BankIt2437961 BSeq#1 MW732719, or a functional fragment thereof, or a nucleotide sequence having at least 75%, 80%, 85%, 90%, 95%, 96%, 97%, 98%, or 99% identity thereto.
200. The method of any of embodiments 187-199, wherein the alteration increases the isolation yield of the compound.
201. The method of any of embodiments 187-200, wherein the isolation yield of the compound is increased by 20%, 30%, 40%, 50%, 60%, 70%, 80%, 90%, or more, compared to a reference microorganism, *e*.*g*., an otherwise identical microorganism in which the BGC of the natural product that interferes with the isolation of the compound is not altered (*e.g.,* a wild-type).
202. A process for producing a partially reoxidized antibody or antigen binding fragment thereof, comprising: providing an antibody or antigen binding fragment thereof comprising one or more capped cysteine residues; reducing the one or more capped cysteine residues, thereby providing an antibody or antigen binding fragment thereof comprising one or more decapped cysteine residues; and storing the antibody or antigen binding fragment thereof comprising one or more decapped cysteine residues under oxidation conditions, thereby producing the partially reoxidized antibody or antigen binding fragment thereof.
203. The process of embodiment 202, wherein the one or more capped cysteine residues are located in the constant domain a heavy chain of the antibody or antigen binding fragment thereof.
204. The process of any of embodiments 202 or 203, wherein the antibody or antigen binding fragment thereof comprises four capped cysteine residues.
205. The process of any of embodiments 202-204, wherein the antibody or antigen binding fragment thereof comprises two capped cysteine residues.
206. The process of any of embodiments 202-205, wherein the antibody or antigen binding fragment thereof comprises a capped cysteine residue located in a CH1 region of the antibody or antigen binding fragment thereof.
207. The process of any of embodiments 202-206, wherein the antibody or antigen binding fragment thereof comprises a capped cysteine residue located in each CH1 region of the antibody or antigen binding fragment thereof.
208. The process of any of embodiments 202-207, wherein the capped cysteine residue is an engineered surface-exposed cysteine residue.
209. The process of any of embodiments 202-208, wherein the antibody or antigen binding fragment thereof comprises a capped cysteine residue located at residue 152 (EU numbering) in the CH1 region of the antibody or antigen binding fragment thereof.
210. The process of any of embodiments 202-209, wherein the one or more capped cysteine residues are capped with a cysteine or a glutathione.
211. The process of any of embodiments 202-210, wherein the one or more decapped cysteine residues are located in the constant domain a heavy chain of the antibody or antigen binding fragment thereof.
212. The process of any of embodiments 202-211, wherein the antibody or antigen binding fragment thereof comprises four decapped cysteine residues.
213. The process of any of embodiments 202-212, wherein the antibody or antigen binding fragment thereof comprises two decapped cysteine residues.
214. The process of any of embodiments 202-213, wherein the antibody or antigen binding fragment thereof comprises a decapped cysteine residue located in a CH1 region of the antibody or antigen binding fragment thereof.
215. The process of any of embodiments 202-214, wherein the antibody or antigen binding fragment thereof comprises a decapped cysteine residue located in each CH1 region of the antibody or antigen binding fragment thereof.
216. The process of any of embodiments 202-215, wherein the decapped cysteine residue is an engineered surface-exposed cysteine residue.
217. The process of any of embodiments 202-216, wherein the antibody or antigen binding fragment thereof comprises a decapped cysteine residue located at residue 152 (EU numbering) in the CH1 region of the antibody or antigen binding fragment thereof.
218. The process of any of embodiments 202-217, wherein the process comprises reducing the one or more capped cysteine residues by contacting the antibody or antigen binding fragment thereof comprising one or more capped cysteine residues with a reduction wash buffer, thereby providing an antibody or antigen binding fragment comprising one or more decapped cysteine residue.
219. The process of any of embodiments 202-218, wherein the reduction wash buffer comprises 5 mM to 50 mM cysteine, *e*.*g*., 10 mM to 40 mM, 20 mM to 30 mM, 5 mM to 15 mM, 5 mM to 25 mM, 5 mM to 35 mM, 5 mM to 45 mM, 40 mM to 50 mM, 30 mM to 50 mM, 20 mM to 50 mM, 10 mM to 50 mM, 10 mM to 20 mM, 15 mM to 25 mM, 25 mM to 35 mM, 30 mM to 40 mM, or 35 mM to 45 mM, *e.g.,* 10 mM, 12 mM, 14 mM, 16 mM, 20 mM, 25 mM, or 30 mM.
220. The process of any of embodiments 202-219, wherein the reduction wash buffer comprises 5 mM to 15 mM cysteine, *e*.*g*., 10 mM cysteine.
221. The process of any of embodiments 202-220, wherein the reduction wash buffer has a pH of 6.0 to 7.5, *e.g.,* 6.5 to 7.2 or 6.8 to 7.0.
222. The process of any of embodiments 202-221, wherein the reduction wash buffer has a pH of 6.8 to 7.0, *e.g.,* 6.9.
223. The process of any of embodiments 202-222, wherein contacting the antibody or antigen binding fragment thereof comprising one or more capped cysteine residues with the reduction wash buffer is performed on a column (*e*.*g*., a cation exchange chromatography column).
224. The process of any of embodiments 202-223, wherein the process further comprises collecting the antibody or antigen binding fragment thereof comprising one or more decapped cysteine residues in an eluate after contacting with the reduction wash buffer.
225. The process of any of embodiments 202-224, wherein the eluate has a pH of 5.5 to 6.0, *e.g.,* 5.8.
226. The process of any of embodiments 202-225, wherein the concentration of the antibody or antigen binding fragment thereof comprising one or more decapped cysteine residues in the eluate is 5 g/L to 25 g/L, *e.g.,* 8 g/L to 20 g/L, 10 g/L to 18 g/L, 12 g/L to 15 g/L, 5 g/L to 20 g/L, 5 g/L to 15 g/L, 5 g/L to 10 g/L, 20 g/L to 25 g/L, 15 g/L to 25 g/L, 10 g/L to 25 g/L, 10 g/L to 20 g/L, 13 g/L to 14 g/L, 12 g/L to 15 g/L, *e.g., e.g.,* 5 g/L, 6 g/L, 7 g/L, 8 g/L, 9 g/L, 10 g/L, 11 g/L, 12 g/L, 13 g/L, 13.5 g/L, 14 g/L, 15 g/L, 16 g/L, 17 g/L, 18 g/L, 19 g/L, 20 g/L, 21 g/L, 22 g/L, 23 g/L, 24 g/L, or 25 g/L.
227. The process of any of embodiments 202-226, wherein the antibody or antigen binding fragment thereof comprising one or more decapped cysteine residues is stored in the eluate with stirring.
228. The process of any of embodiments 202-227, wherein the antibody or antigen binding fragment thereof comprising one or more decapped cysteine residues is stored in the eluate without stirring.
229. The process of any of embodiments 202-228, wherein the antibody or antigen binding fragment thereof comprising one or more decapped cysteine residues is stored in a container filled to a maximum of 50% to 70% volume, *e.g.,* for 12 hours to 96 hours, *e.g.,* 12 hours to 84 hours, 24 hours to 84 hours, 36 hours to 72 hours, 48 hours to 60 hours, 24 hours to 72 hours, 24 hours to 60 hours, 24 hours to 48 hours, 24 hours to 36 hours, 72 hours to 84 hours, 60 hours to 84 hours, 48 hours to 84 hours, 36 hours to 84 hours, 12 hours to 36 hours, 24 hours to 48 hours, 36 hours to 60 hours, 48 hours to 72 hours, or 72 hours to 96 hours, *e*.*g*., 12 hours, 24 hours, 36 hours, 48 hours, 60 hours, 72 hours, 84 hours, or 96 hours.
230. The process of any of embodiments 202-229, wherein the antibody or antigen binding fragment thereof comprising one or more decapped cysteine residues is stored in the container filled to a maximum of 60%.
231. The process of any of embodiments 202-230, wherein the antibody or antigen binding fragment thereof comprising one or more decapped cysteine residues is stored for 48 hours to 72 hours, *e*.*g*., without stirring.
232. The process of any of embodiments 202-231, wherein the antibody or antigen binding fragment thereof comprising one or more decapped cysteine residues is stored for 12 hours to 36 hours, *e*.*g*., 24 hours, *e*.*g*., with stirring.
233. The process of any of embodiments 202-232, wherein the antibody or antigen binding fragment thereof comprising one or more decapped cysteine residues is stored at room temperature.
234. The process of any of embodiments 202-233, wherein the antibody or antigen binding fragment thereof comprising one or more decapped cysteine residues is stored at 2 °C to 8 °C *(e.g.,* 4 °C), *e.g.,* for at least 48 hours (*e*.*g*., 48 hours to 96 hours).
235. The process of any of embodiments 202-234, wherein the antibody or antigen binding fragment thereof comprising one or more decapped cysteine residues is stored with air overlay.
236. The process of any of embodiments 202-235, wherein the process further comprises purifying the partially reoxidized antibody or antigen binding fragment thereof.
237. The process of any of embodiments 202-236, wherein the process further comprises conjugating a linker-drug moiety (*e*.*g*., a linker-drug moiety described herein) to the partially reoxidized antibody or antigen binding fragment thereof to produce an antibody drug conjugate (*e*.*g*., an antibody drug conjugate described herein).
238. The process of any of embodiments 202-237, wherein the process further comprises pre-forming a linker-drug moiety of the following Formula (B):

   R⁸-L_{B}-(D)ₙ (B)

   wherein:
   D is a GNAQ inhibitor (*e*.*g*., a GNAQ inhibitor described herein), a GNA11 inhibitor (*e*.*g*., a GNA11 inhibitor described herein), or an inhibitor of GNAQ and GNA11 (*e*.*g*., an inhibitor of GNAQ and GNA11 as described herein);
   R⁸ is a reactive group (*e*.*g*., a reactive group described herein);
   L_{B} is a cleavable or non-cleavable linker (*e*.*g*. a cleavable linker described herein or non-cleavable linker described herein), and
   n is 1, 2, 3 or 4;
239. The process of any of embodiments 202-238, wherein the process further comprises purifying the antibody drug conjugate.
240. The process of any of embodiments 202-239, wherein the process results in at least 75%, *e.g.,* at least 80%, 85%, 90%, or 95%, on-site coupling, *e*.*g*., one linker-drug moiety per heavy chain ("HC1").
241. The process of any of embodiments 202-240, wherein the process results in less than 25%, *e.g.,* at least 20%, 15%, 10%, or 5%, off-site coupling, *e*.*g*., one linker-drug moiety per light chain ("LC1") and/or two linker-drug moieties per heavy chain ("HC2").
242. The process of any of embodiments 202-241, wherein the process results in at least 70%, *e.g.,* at least 75%, 80%, 85%, 90%, or 95%, purity, *e*.*g*., as determined by non-reduced CE-SDS.
243. A process for producing an anti-PMEL17 antibody drug conjugate, comprising:
   contacting an antibody or antigen binding fragment thereof comprising one or more capped cysteine residues with a reduction wash buffer, thereby providing an antibody or antigen binding fragment comprising one or more decapped cysteine residues;
   storing the antibody or fragment thereof comprising one or more decapped cysteine residues under oxidation conditions, thereby producing a partially reoxidized antibody or antigen binding fragment thereof; and
   conjugating a linker-drug moiety to the partially reoxidized antibody or antigen binding fragment thereof,
   thereby producing the anti-PMEL17 antibody drug conjugate,
   wherein the antibody or antigen binding fragment thereof binds to PMEL17; and
   wherein the linker-drug moiety of the following Formula (B):

      R⁸-L_{B}-(D)ₙ (B)

      wherein:
      D is a GNAQ inhibitor, a GNA11 inhibitor or an inhibitor of GNAQ and GNA11;
      R⁸ is a reactive group;
      L_{B} is a cleavable or non-cleavable linker, and
      n is 1, 2, 3 or 4.
244. A formulation comprising an antibody drug conjugate, a buffering agent, a stabilizing agent, and a surfactant,
   wherein the formulation has a pH of 4.5 to 6.5; and
   wherein the antibody drug conjugate comprises the formula (C)

      Ab-(L_{A}-(D)ₙ)_{y} (C)
   wherein:
      D is a GNAQ inhibitor, a GNA11 inhibitor or an inhibitor of GNAQ and GNA11 (*e.g.,* a GNAQ inhibitor, a GNA11 inhibitor or an inhibitor of GNAQ and GNA11, as described herein);
      Ab is an antibody or antigen binding fragment thereof that binds to human PMEL17 protein (*e*.*g*., an antibody or antigen binding fragment thereof that binds to human PMEL17 protein as described herein;
      L_{A} is a linker (*e*.*g*., a linker described herein);
      n is 1, 2, 3 or 4, and
      y is 1, 2, 3 or 4.
245. The formulation of embodiment 244, wherein antibody drug conjugate is present at a concentration of 5 mg/mL to 30 mg/mL, *e*.*g*., 10 mg to 30 mg, 15 mg/mL to 25 mg/mL, 18 mg/mL to 22 mg/mL, 10 mg/mL to 25 mg/mL, 10 mg/mL to 20 mg/mL, 10 mg/mL to 15 mg/mL, 25 mg to 30 mg/mL, 20 mg/mL to 30 mg/mL, 5 mg/mL to 15 mg/mL, 15 mg/mL to 25 mg/mL, or 18 mg/mL to 22 mg/mL, *e*.*g*., 5 mg/mL, 6 mg/mL, 7 mg/mL, 8 mg/mL, 9 mg/mL, 10 mg/mL, 11 mg/mL, 12 mg/mL, 13 mg/mL, 14 mg/mL, 15 mg/mL, 16 mg/mL, 17 mg/mL, 18 mg/mL, 19 mg/mL, 20 mg/mL, 21 mg/mL, 22 mg/mL, 23 mg/mL, 24 mg/mL, or 25 mg/mL.
246. The formulation of any of embodiments 244 or 245, wherein the antibody drug conjugate is present at a concentration of 15 mg/mL to 25 mg/mL, *e*.*g*., 20 mg/mL.
247. The formulation of any of embodiments 244-246, wherein the buffering agent comprises histidine or succinate.
248. The formulation of any of embodiments 244-247, wherein the buffering agent is present at a concentration of 5 mM to 50 mM, *e*.*g*., 10 mM to 40 nM, 15 mM to 35 mM, 20 mM to 30 mM, 5 mM to 40 mM, 5 mM to 30 mM, 5 mM to 20 mM, 5 mM to 15 mM, 5 mM to 10 mM, 40 mM to 50 mM, 35 mM to 50 mM, 30 mM to 50 mM, 25 mM to 50 mM, 20 mM to 50 mM, 15 mM to 50 mM, 10 mM to 50 mM, 10 mM to 20 mM, 15 mM to 25 mM, 25 mM to 35 mM, 30 mM to 40 mM, or 35 mM to 45 mM, *e*.*g*., 5 mM, 10 mM, 15 mM, 20 mM, 25 mM, 30 mM, 35 mM, 40 mM, 45 mM, or 50 mM.
249. The formulation of any of embodiments 244-248, wherein the buffering agent is present at a concentration of 15 mM to 25 mM, *e*.*g*., 20 mM.
250. The formulation of any of embodiments 244-249, wherein the stabilizing agent comprises sucrose or trehalose.
251. The formulation of any of embodiments 244-250, wherein the stabilizing agent is present at a concentration of 100 mM to 500 mM, *e*.*g*., 150 mM to 450 mM, 200 mM to 400 mM, 250 mM to 350 mM, 100 mM to 450 mM, 100 mM to 400 mM, 100 mM to 350 mM, 100 mM to 300 mM, 100 mM to 250 mM, 100 mM to 200 mM, 100 mM to 150 mM, 450 mM to 500 mM, 400 mM to 500 mM, 350 mM to 500 mM, 300 mM to 500 mM, 250 mM to 500 mM, 200 mM to 500 mM, 150 mM to 500 mM, 150 mM to 250 mM, 200 mM to 250 mM, 200 mM to 300 mM, 300 mM to 400 mM, or 350 mM to 450 mM, *e.g.,* 100 mM, 150 mM, 200 mM, 240 mM, 250 mM, 300 mM, 350 mM, 400 mM, 450 mM, or 500 mM.
252. The formulation of any of embodiments 244-251, wherein the stabilizing agent and is present at a concentration of 200 mM to 300 mM, *e.g.,* 240 mM.
253. The formulation of any of embodiments 244-252, wherein the surfactant comprises polysorbate 20 or polysorbate 80.
254. The formulation of any of embodiments 244-253, wherein the surfactant is present at a concentration of 0.01% to 0.06%, *e.g.,* 0.02% to 0.05%, 0.03% to 0.04%, 0.01% to 0.05%, 0.01% to 0.04%, 0.01% to 0.03%, 0.01% to 0.02%, 0.05% to 0.06%, 0.04% to 0.06%, 0.03% to 0.06%, 0.02% to 0.06%, 0.02% to 0.04%, 0.03% to 0.05%, *e.g.,* 0.01%, 0.02%, 0.03%, 0.04%, 0.05%, or 0.06%.
255. The formulation of any of embodiments 244-254, wherein the surfactant is present at a concentration of 0.01% to 0.05%, *e.g.,* 0.02%.
256. The formulation of any of embodiments 244-255, wherein the formulation has a pH of 4.7 to 5.3, 5.0 to 6.0, 5.2 to 5.8, 5.4 to 5.6, 5.2 to 6.0, 5.4 to 6.0, 5.6 to 6.0, 5.8 to 6.0, 5 to 5.8, 5 to 5.6.0, 5 to 5.4, 5 to 5.2, 4.8 to 5.2, 5.1 to 5.3, 5.2 to 5.4, 5.3 to 5.5, 5.5 to 5.7, 5.6 to 5.8, 5.7 to 5.9, 4.9 to 5.5, 5.5 to 6.1, or 5.7 to 6.3, *e.g.,* 4.5, 4.6, 4.7, 4.8, 4.9, 5, 5.1, 5.2, 5.3, 5.4, 5.5, 5.6, 5.7, 5.8, 5.9, 6.0, 6.1, 6.2, 6.3, 6.4, or 6.5.
257. The formulation of any of embodiments 244-256, wherein the formulation has a pH of 5.0 to 5.6, *e.g.,* 5.3.
258. The formulation of any of embodiments 244-257, comprising 15 mg/mL to 25 mg/mL of the antibody drug conjugate, 15 mM to 25 mM of a buffering agent comprising histidine, 200 mM to 300 mM of a stabilizing agent comprising sucrose, and 0.01% to 0.03% of a surfactant comprising polysorbate 20, wherein the formulation has a pH of 5.0 to 5.6.
259. The formulation of any of embodiments 244-258, comprising 20 mg/mL of the antibody drug conjugate, 20 mM of a buffering agent comprising histidine, 240 mM of a stabilizing agent comprising sucrose, and 0.02% of a surfactant comprising polysorbate 20, wherein the formulation has a pH of 5.3.
260. The formulation of any of embodiments 244-259, comprising 15 mg/mL to 25 mg/mL of the antibody drug conjugate, 15 mM to 25 mM of a buffering agent comprising histidine, 200 mM to 300 mM of a stabilizing agent comprising sucrose, and 0.01% to 0.03% of a surfactant comprising polysorbate 20, wherein the formulation has a pH of 4.7 to 5.3.
261. The formulation of any of embodiments 244-260, comprising 20 mg/mL of the antibody drug conjugate, 20 mM of a buffering agent comprising histidine, 240 mM of a stabilizing agent comprising sucrose, and 0.02% of a surfactant comprising polysorbate 20, wherein the formulation has a pH of 5.0.
262. The formulation of any of embodiments 244-261, comprising 15 mg/mL to 25 mg/mL of the antibody drug conjugate, 15 mM to 25 mM of a buffering agent comprising histidine, 200 mM to 300 mM of a stabilizing agent comprising sucrose, and 0.01% to 0.03% of a surfactant comprising polysorbate 20, wherein the formulation has a pH of 5.2 to 5.8.
263. The formulation of any of embodiments 244-262, comprising 20 mg/mL of the antibody drug conjugate, 20 mM of a buffering agent comprising histidine, 240 mM of a stabilizing agent comprising sucrose, and 0.02% of a surfactant comprising polysorbate 20, wherein the formulation has a pH of 5.5.
264. The formulation of any of embodiments 244-263, comprising 15 mg/mL to 25 mg/mL of the antibody drug conjugate, 15 mM to 25 mM of a buffering agent comprising histidine, 200 mM to 300 mM of a stabilizing agent comprising sucrose, and 0.03% to 0.05% of a surfactant comprising polysorbate 20, wherein the formulation has a pH of 5.2 to 5.8.
265. The formulation of any of embodiments 244-264, comprising 20 mg/mL of the antibody drug conjugate, 20 mM of a buffering agent comprising histidine, 240 mM of a stabilizing agent comprising sucrose, and 0.04% of a surfactant comprising polysorbate 20, wherein the formulation has a pH of 5.5.
266. The formulation of any of embodiments 244-265, comprising 5 mg/mL to 15 mg/mL of the antibody drug conjugate, 15 mM to 25 mM of a buffering agent comprising histidine, 200 mM to 300 mM of a stabilizing agent comprising sucrose, and 0.01% to 0.03% of a surfactant comprising polysorbate 20, wherein the formulation has a pH of 5.2 to 5.8.
267. The formulation of any of embodiments 244-266, comprising 10 mg/mL of the antibody drug conjugate, 20 mM of a buffering agent comprising histidine, 240 mM of a stabilizing agent comprising sucrose, and 0.02% of a surfactant comprising polysorbate 20, wherein the formulation has a pH of 5.5.
268. The formulation of any of embodiments 244-267, comprising 15 mg/mL to 25 mg/mL of the antibody drug conjugate, 15 mM to 25 mM of a buffering agent comprising histidine, 200 mM to 300 mM of a stabilizing agent comprising sucrose, and 0.01% to 0.03% of a surfactant comprising polysorbate 20, wherein the formulation has a pH of 5.7 to 6.3.
269. The formulation of any of embodiments 244-268, comprising 20 mg/mL of the antibody drug conjugate, 20 mM of a buffering agent comprising histidine, 240 mM of a stabilizing agent comprising sucrose, and 0.02% of a surfactant comprising polysorbate 20, wherein the formulation has a pH of 6.0.
270. The formulation of any of embodiments 244-269, comprising 15 mg/mL to 25 mg/mL of the antibody drug conjugate, 15 mM to 25 mM of a buffering agent comprising histidine, 200 mM to 300 mM of a stabilizing agent comprising sucrose, and 0.01% to 0.03% of a surfactant comprising polysorbate 20, wherein the formulation has a pH of 4.9 to 5.5.
271. The formulation of any of embodiments 244-270, comprising 20 mg/mL of the antibody drug conjugate, 20 mM of a buffering agent comprising histidine, 240 mM of a stabilizing agent comprising sucrose, and 0.02% of a surfactant comprising polysorbate 20, wherein the formulation has a pH of 5.2.
272. The formulation of any of embodiments 244-271, comprising 15 mg/mL to 25 mg/mL of the antibody drug conjugate, 15 mM to 25 mM of a buffering agent comprising histidine, 200 mM to 300 mM of a stabilizing agent comprising sucrose, and 0.01% to 0.03% of a surfactant comprising polysorbate 20, wherein the formulation has a pH of 5.5 to 6.1.
273. The formulation of any of embodiments 244-272, comprising 20 mg/mL of the antibody drug conjugate, 20 mM of a buffering agent comprising histidine, 240 mM of a stabilizing agent comprising sucrose, and 0.02% of a surfactant comprising polysorbate 20, wherein the formulation has a pH of 5.8.
274. A lyophilized formulation, which is lyophilized from the formulation of any of embodiments 244-273.
275. The lyophilized formulation of embodiment 274, wherein 5 mL to 5.5 mL of the formulation is lyophilized.
276. The lyophilized formulation of any of embodiments 274 or 275, wherein D is at least 1, 2, 3, 4, 5, 6, 7, 8, 9, or 10-fold more stable, compared to an otherwise identical formulation that is not lyophilized, after storage for 0, 4, or 8 weeks, at 5°C or 25°C, *e.g.,* as determined by CZE.
277. The lyophilized formulation of any of embodiments 274-276, wherein D is at least 1, 2, 3, 4, 5, 6, 7, 8, 9, or 10-fold more stable, compared to an otherwise identical formulation that is not lyophilized, after storage for 0, 4, or 8 weeks, at 40°C, *e.g.,* as determined by CZE.
278. The lyophilized formulation of any of embodiments 274-277, wherein the percentage of D that has a ring-opening conformation is at least 1, 2, 3, 4, 5, 6, 7, 8, 9, or 10-fold lower, compared to an otherwise identical formulation that is not lyophilized, after storage for 0, 4, or 8 weeks, at 5°C or 25°C, *e.g.,* as determined by CZE.
279. The lyophilized formulation of any of embodiments 274-278, wherein the percentage of D that has a ring-opening conformation is at least 1, 2, 3, 4, 5, 6, 7, 8, 9, or 10-fold lower, compared to an otherwise identical formulation that is not lyophilized, after storage for 0, 4, or 8 weeks, 40°C, *e.g.,* as determined by CZE.
280. The lyophilized formulation of any of embodiments 274-279, which comprises 80 mg to 120 mg (*e.g.,* 107 mg) of the antibody drug conjugate.
281. A liquid formulation, which is reconstituted from the lyophilized formulation of any one of embodiments 274-280.
282. The formulation of any of embodiments 244-281, wherein the level of monomers in the formulation is at least 95%, *e.g.,* at least 96%, 97%, 98%, or 99%, after storage for 0, 4, or 8 weeks, at 5°C, 25°C, or 40°C, *e.g.,* as determined by SEC.
283. The formulation of any of embodiments 244-282, wherein the level of fragments in the formulation is less than 3%, *e.g.,* less than 2.5%, 2%, 1.5%, 1%, or 0.5%, after storage for 0, 4, or 8 weeks, at 5°C, 25°C, or 40°C, *e.g.,* as determined by SEC.
284. The formulation of any of embodiments 244-283, wherein the level of aggregates in the formulation is less than 3%, *e.g.,* less than 2.5%, 2%, 1.5%, 1%, or 0.5%, after storage for 0, 4, or 8 weeks, at 5°C, 25°C, or 40°C, *e.g.,* as determined by SEC.
285. The formulation of any of embodiments 244-284, wherein the level of degradation is less than 2%, *e.g.,* less than 1.5%, 1%, or 0.5%, after storage for 4 weeks or 8 weeks, at 5°C, 25°C, or 40°C, *e.g.,* as determined by SEC.
286. The formulation of any of embodiments 244-285, wherein the level of particles greater than or equal to 10 µm in the formulation is less than about 300 particles/ml, *e*.*g*., less than about 280 particles/mL, less than about 260 particles/mL, less than about 240 particles/mL, less than about 220 particles/mL, less than about 200 particles/mL, less than about 180 particles/mL, less than about 160 paricles/mL, less than about 140 particles/mL, less than about 120 particles/mL, less than 100 particles/mL, 80 particles/mL, 60 particles/mL, 40 particles/mL, 20 particles/mL, or 10 particles/mL, after storage for 0, 4, or 8 weeks, at 5°C, 25°C, or 40°C, *e.g.,* as determined by light obscuration.
287. The formulation of any of embodiments 244-286, wherein the level of particles greater than or equal to 10 µm in the formulation is increased by no more than 3-fold, *e.g.,* no more than 2.5, 2, 1.5, 1, or 0.5-fold, after storage for 4 or 8 weeks, at 5°C, 25°C, or 40°C, *e.g.,* as determined by light obscuration.
288. The formulation of any of embodiments 244-287, wherein the level of particles greater than 25 µm in the formulation is increased by no more than 3-fold, *e.g.,* no more than 2.5, 2, 1.5, 1, or 0.5-fold, after storage for 4 or 8 weeks, at 5°C, 25°C, or 40°C, *e.g.,* as determined by light obscuration.
289. The formulation of any of embodiments 244-288, wherein the level of particles greater than 25 µm in the formulation is less than 20 particles/mL, *e*.*g*., less than 15 particles/mL, 10 particles/mL, 5 particles/mL, or 2 particles/mL, after storage for 0, 4, or 8 weeks, at 5°C, 25°C, or 40°C, *e.g.,* as determined by light obscuration.
290. The formulation of any of embodiments 244-289, wherein the level of impurity is less than 15%, *e.g.,* less than 14%, 13%, 12%, 11%, 10%, 9%, 8%, 7%, 6%, 5%, 4%, 3%, 2%, or 1%, after storage for 0, 4, or 8 weeks, at 5°C, 25°C, or 40°C, *e.g.,* as determined by capillary electrophoresis.
291. The formulation of any of embodiments 244-290, wherein the level of impurity is increased by no more than 20%, *e.g.,* no more than 15%, 10%, 5%, 2%, or 1%, after storage for 4 weeks or 8 weeks, at 5°C, 25°C, or 40°C, *e.g.,* as determined by capillary electrophoresis.
292. The formulation of any of embodiments 244-291, wherein the level of neutral variants is greater than 50%, *e.g.,* greater than 55%, 60%, 65%, 70%, 75%, 80%, 85%, or 90%, after storage for 0, 4, or 8 weeks, at 5°C, 25°C, or 40°C, *e.g.,* as determined by capillary zone electrophoresis (CZE).
293. The formulation of any of embodiments 244-292, wherein the level of neutral variants is decreased by no more than 20%, *e.g.,* no more than 15%, 10%, 5%, or 2%, after storage for 4 weeks or 8 weeks, at 5°C, 25°C, or 40°C, *e.g.,* as determined by CZE.
294. The formulation of any of embodiments 244-293, wherein the level of acidic variants is less than 30%, *e.g.,* less than 25%, 20%, 15%, 5%, or 2%, after storage for 0, 4, or 8 weeks, at 5°C, 25°C, or 40°C, *e.g.,* as determined by CZE.
295. The formulation of any of embodiments 244-294, wherein the level of acid variants is increased by no more than 50%, *e.g.,* no more than 40%, 30%, 20%, 10%, or 5%, after storage for 4 weeks or 8 weeks, at 5°C, 25°C, or 40°C, *e.g.,* as determined by CZE.
296. The formulation of any of embodiments 244-295, wherein the level of basic variants is less than 30%, *e.g.,* less than 25%, 20%, 15%, 5%, or 2%, after storage for 0, 4, or 8 weeks, at 5°C, 25°C, or 40°C, *e.g.,* as determined by CZE.
297. The formulation of any of embodiments 244-296, wherein the level of basic variants is increased by no more than 50%, *e.g.,* no more than 40%, 30%, 20%, 10%, or 5%, after storage for 4 weeks or 8 weeks, at 5°C, 25°C, or 40°C, *e.g.,* as determined by CZE.
298. The formulation of any of embodiments 244-297, wherein the potency is decreased by no more than 25%, *e.g.,* no more than 20%, 15%, 10%, 5%, or 2%, after storage for 8 weeks, at 5°C, 25°C, or 40°C, *e.g.,* as determined by a bioactivity assay.
299. The formulation of any of embodiments 244-298, wherein the osmolality of the formulation is 200 mOsm/L to 400 mOsm/L, *e*.*g*., 200 mOsm/L to 300 mOsm/L, 250 mOsm/L to 350 mOsm/L, 270 mOsm/L to 330 mOsm/L, 290 mOsm/L to 310 mOsm/L, 270 mOsm/L to 350 mOsm/L, 290 mOsm/L to 350 mOsm/L, 310 mOsm/L to 350 mOsm/L, 330 mOsm/L to 350 mOsm/L, 250 mOsm/L to 330 mOsm/L, 250 mOsm/L to 310 mOsm/L, 250 mOsm/L to 290 mOsm/L, 250 mOsm/L to 270 mOsm/L, 260 mOsm/L to 280 mOsm/L, 270 mOsm/L to 290 mOsm/L, 280 mOsm/L to 300 mOsm/L, 300 mOsm/L to 320 mOsm/L, 310 mOsm/L to 330 mOsm/L, or 320 mOsm/L to 340 mOsm/L, *e*.*g*., 200 mOsm/L, 250 mOsm/L, 260 mOsm/L, 270 mOsm/L, 280 mOsm/L, 290 mOsm/L, 300 mOsm/L, 310 mOsm/L, 320 mOsm/L, 330 mOsm/L, 340 mOsm/L, 350 mOsm/L, or 400 mOsm/L.
300. A container comprising the lyophilized formulation of any of embodiments 274-299.
301. The container of embodiment 300, which is a vial, *e.g.,* a 20R glass vial or a 25 R glass vial.
302. The container of any of embodiments 300 or 301, which further comprises a stopper and a cap (*e*.*g*., a flip-off aluminum cap).
303. A method of treating or preventing cancer in a subject in need thereof, comprising administering to the subject a formulation of any one of embodiments 244-299, wherein the cancer expresses PMEL17, contains a mutation of the GNAQ or GNA11 gene, or the cancer expresses PMEL17 and contains a mutation of GNAQ, GNA11, or both.
304. The method of embodiment 303, wherein the cancer is a carcinoma, sarcoma, leukemia, lymphoma, eye cancer, eye neoplasm, melanoma, or a metastatic cancer thereof.
305. The method of embodiment 304, wherein the carcinoma is a hepatocellular carcinoma, or a metastatic lesion thereof.
306. The method of embodiment 304, wherein the melanoma is uveal melanoma, non-uveal melanoma, malignant melanoma, ocular melanoma, mucosal melanoma, subcutaneous melanoma, cutaneous melanoma, or a metastatic lesion thereof.
307. The method of any of embodiments 303-306, wherein the formulation is administered to the patient in combination with one or more additional therapeutic compounds.
308. The method of embodiment 303-307, wherein the one or more additional therapeutic compounds is selected from a standard of care chemotherapeutic, an MDM2 inhibitor, an MRC2 inhibitor, a PKC inhibitor, a MAPK inhibitor, a costimulatory molecule, or a checkpoint inhibitor.
309. The method of embodiment 308, wherein the costimulatory molecule is selected from an agonist of OX40, CD2, CD27, CDS, ICAM-1, LFA-1 (CD11a/CD18), ICOS (CD278), 4-1BB (CD137), GITR, CD30, CD40, BAFFR, HVEM, CD7, LIGHT, NKG2C, SLAMF7, NKp80, CD160, B7-H3, STING, or CD83 ligand.
310. The method of embodiment 309, wherein the checkpoint inhibitor is selected from an inhibitor of PD-1, PD-L1, PD-L2, CTLA4, TIM3, LAG3, VISTA, BTLA, TIGIT, LAIR1, CD160, 2B4 and/or TGFR beta.
311. A formulation of any of embodiments 244-299 for use in a method of treating or preventing cancer in a subject in need thereof, comprising administering to the subject a formulation of any one of embodiments 244-299, wherein the cancer expresses PMEL17, contains a mutation of the GNAQ or GNA11 gene, or the cancer expresses PMEL17 and contains a mutation of GNAQ, GNA11, or both.
312. The formulation for use of embodiment 311, wherein the cancer is a carcinoma, sarcoma, leukemia, lymphoma, eye cancer, eye neoplasm, melanoma, or a metastatic lesion thereof.
313. The formulation for use of embodiment 312, wherein the carcinoma is a hepatocellular carcinoma, or a metastatic lesion thereof.
314. The formulation for use of embodiment 312, wherein the melanoma is uveal melanoma, non-uveal melanoma, malignant melanoma, ocular melanoma, mucosal melanoma, subcutaneous melanoma, cutaneous melanoma, or a metastatic lesion thereof.
315. The formulation for use of any of embodiments 311-314, wherein the formulation is administered to the patient in combination with one or more additional therapeutic compounds.
316. The formulation for use of any of embodiments 311-315, wherein the one or more additional therapeutic compounds is selected from a standard of care chemotherapeutic, an MDM2 inhibitor, an MRC2 inhibitor, a PKC inhibitor, a MAPK inhibitor, a costimulatory molecule, or a checkpoint inhibitor.
317. The formulation for use of any of embodiments 311-316, wherein the costimulatory molecule is selected from an agonist of OX40, CD2, CD27, CDS, ICAM-1, LFA-1 (CD11a/CD18), ICOS (CD278), 4-1BB (CD137), GITR, CD30, CD40, BAFFR, HVEM, CD7, LIGHT, NKG2C, SLAMF7, NKp80, CD160, B7-H3, STING, or CD83 ligand.
318. The formulation for use of any of embodiments 311-317, wherein the checkpoint inhibitor is selected from an inhibitor of PD-1, PD-L1, PD-L2, CTLA4, TIM3, LAG3, VISTA, BTLA, TIGIT, LAIR1, CD160, 2B4 and/or TGFR beta.
319. The process of any of embodiments 202-243, the formulation of any of embodiments 244-299, the container of any of embodiments 300-302, the method of any of embodiments 303-310, or the formulation for use of any of embodiments 311-318, wherein the antibody or antigen binding fragment thereof that binds PMEL17 comprises:
   (a) a heavy chain variable region that comprises a heavy chain CDR1 (Complementarity Determining Region 1) of SEQ ID NO:1, 4, 5 or 7, a heavy chain CDR2 (Complementarity Determining Region 2) of SEQ ID NO:2, 6 or 8, and a heavy chain CDR3 (Complementarity Determining Region 3) of SEQ ID NO:3 or 9; and a light chain variable region that comprises a light chain CDR1 (Complementarity Determining Region 1) of SEQ ID NO:14, 17 or 20, a light chain CDR2 (Complementarity Determining Region 2) of SEQ ID NO:15 or 18, and a light chain CDR3 (Complementarity Determining Region 3) of SEQ ID NO:16 or 19;
   (b) a heavy chain variable region that comprises a heavy chain CDR1 of SEQ ID NO:33, 36, 37 or 39, a heavy chain CDR2 of SEQ ID NO:34, 38 or 40; a heavy chain CDR3 of SEQ ID NO:35 or 41; a light chain CDR1 of SEQ ID NO:46, 49 or 52; a light chain CDR2 of SEQ ID NO:47 or 50; and a light chain CDR3 of SEQ ID NO:48 or 51;
   (c) a heavy chain variable region that comprises a heavy chain CDR1 of SEQ ID NO:5, 7, 57 or 60, a heavy chain CDR2 of SEQ ID NO:58, 61 or 62; a heavy chain CDR3 of SEQ ID NO:59 or 63; a light chain CDR1 of SEQ ID NO:68, 71 or 74; a light chain CDR2 of SEQ ID NO:69 or 72; and a light chain CDR3 of SEQ ID NO: 70 or 73;
   (d) a heavy chain variable region that comprises a heavy chain CDR1 of SEQ ID NO:79, 82, 83 or 85, a heavy chain CDR2 of SEQ ID NO:80, 84 or 86; a heavy chain CDR3 of SEQ ID NO:81 or 87; a light chain CDR1 of SEQ ID NO:92, 95 or 98; a light chain CDR2 of SEQ ID NO:93 or 96; and a light chain CDR3 of SEQ ID NO:94 or 97;
   (e) a heavy chain variable region that comprises a heavy chain CDR1 of SEQ ID NO:103, 106, 107 or 109, a heavy chain CDR2 of SEQ ID NO:104, 108 or 110; a heavy chain CDR3 of SEQ ID NO:105 or 111; a light chain CDR1 of SEQ ID NO:49, 52 or 116; a light chain CDR2 of SEQ ID NO:47 or 50; and a light chain CDR3 of SEQ ID NO:117 or 118;
   (f) a heavy chain variable region that comprises a heavy chain CDR1 of SEQ ID NO:123, 126, 127 or 129, a heavy chain CDR2 of SEQ ID NO:124, 128 or 130; a heavy chain CDR3 of SEQ ID NO:125 or 131; a light chain CDR1 of SEQ ID NO:136, 139 or 142; a light chain CDR2 of SEQ ID NO:137 or 140; and a light chain CDR3 of SEQ ID NO:138 or 141;
   (g) a heavy chain variable region that comprises a heavy chain CDR1 of SEQ ID NO:123, 126, 127 or 129, a heavy chain CDR2 of SEQ ID NO:124, 128 or 130; a heavy chain CDR3 of SEQ ID NO:147 or 148; a light chain CDR1 of SEQ ID NO:153, 156 or 158; a light chain CDR2 of SEQ ID NO:50 or 154; and a light chain CDR3 of SEQ ID NO:155 or 157;
   (h) a heavy chain variable region that comprises a heavy chain CDR1 of SEQ ID NO:103, 106, 107 or 109, a heavy chain CDR2 of SEQ ID NO:104, 108 or 110; a heavy chain CDR3 of SEQ ID NO:163 or 164; a light chain CDR1 of SEQ ID NO:49, 52 or 116; a light chain CDR2 of SEQ ID NO:47 or 50; and a light chain CDR3 of SEQ ID NO:169 or 170;
   (i) a heavy chain variable region that comprises a heavy chain CDR1 of SEQ ID NO:175, 178, 179 or 181, a heavy chain CDR2 of SEQ ID NO:176, 180 or 182; a heavy chain CDR3 of SEQ ID NO:177 or 183; a light chain CDR1 of SEQ ID NO:49, 52 or 116; a light chain CDR2 of SEQ ID NO:47 or 50; and a light chain CDR3 of SEQ ID NO:188 or 189;
   (j) a heavy chain variable region that comprises a heavy chain CDR1 of SEQ ID NO: 103, 106, 107 or 109, a heavy chain CDR2 of SEQ ID NO: 104, 108 or 110; a heavy chain CDR3 of SEQ ID NO:194 or 195; a light chain CDR1 of SEQ ID NO: 49, 52 or 116; a light chain CDR2 of SEQ ID NO: 47 or 50; and a light chain CDR3 of SEQ ID NO:200 or 201;
   (k) a heavy chain variable region that comprises a heavy chain CDR1 of SEQ ID NO:206, 209, 210 or 212, a heavy chain CDR2 of SEQ ID NO:207, 211 or 213; a heavy chain CDR3 of SEQ ID NO:208 or 214; a light chain CDR1 of SEQ ID NO:153, 156 or 158; a light chain CDR2 of SEQ ID NO:50 or 154; and a light chain CDR3 of SEQ ID NO:219 or 220;
   (l) a heavy chain variable region that comprises a heavy chain CDR1 of SEQ ID NO: 206, 209, 210 or 212, a heavy chain CDR2 of SEQ ID NO: 207, 211 or 213; a heavy chain CDR3 of SEQ ID NO:225 or 226; a light chain CDR1 of SEQ ID NO:136, 139 or 142; a light chain CDR2 of SEQ ID NO:137 or 140; and a light chain CDR3 of SEQ ID NO:231 or 232;
   (m) a heavy chain variable region that comprises a heavy chain variable region that comprises an HCDR1 of SEQ ID NO: 206, 209, 210 or 212, an HCDR2 of SEQ ID NO: 207, 211 or 213, and an HCDR3 of SEQ ID NO:237 or 238; and a light chain variable region that comprises an LCDR1 of SEQ ID NO:243, 245 or 247, an LCDR2 of SEQ ID NO:47 or 50, and an LCDR3 of SEQ ID NO:244 or 246;
   (n) a heavy chain variable region that comprises a heavy chain variable region that comprises an HCDR1 of SEQ ID NO: 206, 209, 210 or 212, an HCDR2 of SEQ ID NO: 207, 211 or 213, and an HCDR3 of SEQ ID NO:252 or 253; and a light chain variable region that comprises an LCDR1 of SEQ ID NO:153, 156 or 158, an LCDR2 of SEQ ID NO:50 or 154, and an LCDR3 of SEQ ID NO:258 or 259;
   (o) a heavy chain CDR1 of SEQ ID NO:1, a heavy chain CDR2 of SEQ ID NO:2, a heavy chain CDR3 of SEQ ID NO:3, a light chain CDR1 of SEQ ID NO:14, a light chain CDR2 of SEQ ID NO:15, and a light chain CDR3 of SEQ ID NO:16;
   (p) a heavy chain CDR1 of SEQ ID NO: 4, a heavy chain CDR2 of SEQ ID NO:2, a heavy chain CDR3 of SEQ ID NO:3, a light chain CDR1 of SEQ ID NO:14, a light chain CDR2 of SEQ ID NO:15, and a light chain CDR3 of SEQ ID NO:16;
   (q) a heavy chain CDR1 of SEQ ID NO:5, a heavy chain CDR2 of SEQ ID NO:6, a heavy chain CDR3 of SEQ ID NO:3, a light chain CDR1 of SEQ ID NO:17, a light chain CDR2 of SEQ ID NO: 18, and a light chain CDR3 of SEQ ID NO: 19;
   (r) a heavy chain CDR1 of SEQ ID NO:7, a heavy chain CDR2 of SEQ ID NO:8, a heavy chain CDR3 of SEQ ID NO:9, a light chain CDR1 of SEQ ID NO:20, a light chain CDR2 of SEQ ID NO:18, and a light chain CDR3 of SEQ ID NO:16;
   (s) a heavy chain CDR1 of SEQ ID NO:33, a heavy chain CDR2 of SEQ ID NO:34, a heavy chain CDR3 of SEQ ID NO:35, a light chain CDR1 of SEQ ID NO:46, a light chain CDR2 of SEQ ID NO:47, and a light chain CDR3 of SEQ ID NO:48;
   (t) a heavy chain CDR1 of SEQ ID NO:36, a heavy chain CDR2 of SEQ ID NO:34, a heavy chain CDR3 of SEQ ID NO:35, a light chain CDR1 of SEQ ID NO:46, a light chain CDR2 of SEQ ID NO:47, and a light chain CDR3 of SEQ ID NO:48;
   (u) a heavy chain CDR1 of SEQ ID NO:37, a heavy chain CDR2 of SEQ ID NO:38, a heavy chain CDR3 of SEQ ID NO:35, a light chain CDR1 of SEQ ID NO:49, a light chain CDR2 of SEQ ID NO:50, and a light chain CDR3 of SEQ ID NO:51;
   (v) a heavy chain CDR1 of SEQ ID NO: 39, a heavy chain CDR2 of SEQ ID NO:40, a heavy chain CDR3 of SEQ ID NO:41, a light chain CDR1 of SEQ ID NO:52, a light chain CDR2 of SEQ ID NO:50, and a light chain CDR3 of SEQ ID NO:48;
   (w) a heavy chain CDR1 of SEQ ID NO:57, a heavy chain CDR2 of SEQ ID NO:58, a heavy chain CDR3 of SEQ ID NO:59, a light chain CDR1 of SEQ ID NO:68, a light chain CDR2 of SEQ ID NO:69, and a light chain CDR3 of SEQ ID NO:70;
   (x) a heavy chain CDR1 of SEQ ID NO:60, a heavy chain CDR2 of SEQ ID NO:58, a heavy chain CDR3 of SEQ ID NO:59, a light chain CDR1 of SEQ ID NO:68, a light chain CDR2 of SEQ ID NO:69, and a light chain CDR3 of SEQ ID NO:70;
   (y) a heavy chain CDR1 of SEQ ID NO:5, a heavy chain CDR2 of SEQ ID NO:61, a heavy chain CDR3 of SEQ ID NO:59, a light chain CDR1 of SEQ ID NO:71, a light chain CDR2 of SEQ ID NO:72, and a light chain CDR3 of SEQ ID NO:73;
   (z) a heavy chain CDR1 of SEQ ID NO:7, a heavy chain CDR2 of SEQ ID NO:62, a heavy chain CDR3 of SEQ ID NO:63, a light chain CDR1 of SEQ ID NO:74, a light chain CDR2 of SEQ ID NO:72, and a light chain CDR3 of SEQ ID NO:70;
   (aa) a heavy chain CDR1 of SEQ ID NO:79, , a heavy chain CDR2 of SEQ ID NO:80, a heavy chain CDR3 of SEQ ID NO:81, a light chain CDR1 of SEQ ID NO:92, a light chain CDR2 of SEQ ID NO:93, and a light chain CDR3 of SEQ ID NO:94;
   (bb) a heavy chain CDR1 of SEQ ID NO:82, a heavy chain CDR2 of SEQ ID NO:80, a heavy chain CDR3 of SEQ ID NO:81, a light chain CDR1 of SEQ ID NO:92, a light chain CDR2 of SEQ ID NO:93, and a light chain CDR3 of SEQ ID NO:94;
   (cc) a heavy chain CDR1 of SEQ ID NO:83, a heavy chain CDR2 of SEQ ID NO:84, a heavy chain CDR3 of SEQ ID NO:81, a light chain CDR1 of SEQ ID NO:95, a light chain CDR2 of SEQ ID NO:96, and a light chain CDR3 of SEQ ID NO: 97;
   (dd) a heavy chain CDR1 of SEQ ID NO: 85, a heavy chain CDR2 of SEQ ID NO:86, a heavy chain CDR3 of SEQ ID NO:87, a light chain CDR1 of SEQ ID NO:98, a light chain CDR2 of SEQ ID NO:96, and a light chain CDR3 of SEQ ID NO:94;
   (ee) a heavy chain CDR1 of SEQ ID NO:103, a heavy chain CDR2 of SEQ ID NO:104, a heavy chain CDR3 of SEQ ID NO:105, a light chain CDR1 of SEQ ID NO: 116; a light chain CDR2 of SEQ ID NO:47; and a light chain CDR3 of SEQ ID NO:117;
   (ff) a heavy chain CDR1 of SEQ ID NO:106, a heavy chain CDR2 of SEQ ID NO:104, a heavy chain CDR3 of SEQ ID NO:105, a light chain CDR1 of SEQ ID NO: 116, a light chain CDR2 of SEQ ID NO:47, and a light chain CDR3 of SEQ ID NO:117;
   (gg) a heavy chain CDR1 of SEQ ID NO:107, a heavy chain CDR2 of SEQ ID NO:108, a heavy chain CDR3 of SEQ ID NO:105, a light chain CDR1 of SEQ ID NO:49, a light chain CDR2 of SEQ ID NO:50, and a light chain CDR3 of SEQ ID NO:118;
   (hh) a heavy chain CDR1 of SEQ ID NO:109, a heavy chain CDR2 of SEQ ID NO:110, a heavy chain CDR3 of SEQ ID NO:111, a light chain CDR1 of SEQ ID NO:52 a light chain CDR2 of SEQ ID NO:50, and a light chain CDR3 of SEQ ID NO:117;
   (ii) a heavy chain CDR1 of SEQ ID NO:123, a heavy chain CDR2 of SEQ ID NO:124, a heavy chain CDR3 of SEQ ID NO:125, a light chain CDR1 of SEQ ID NO:136, a light chain CDR2 of SEQ ID NO:137,and a light chain CDR3 of SEQ ID NO:138;
   (jj) a heavy chain CDR1 of SEQ ID NO:126, a heavy chain CDR2 of SEQ ID NO:124, a heavy chain CDR3 of SEQ ID NO:125, a light chain CDR1 of SEQ ID NO:136, a light chain CDR2 of SEQ ID NO:137, and a light chain CDR3 of SEQ ID NO:138;
   (kk) a heavy chain CDR1 of SEQ ID NO:127, a heavy chain CDR2 of SEQ ID NO:128, a heavy chain CDR3 of SEQ ID NO:125, a light chain CDR1 of SEQ ID NO:139, a light chain CDR2 of SEQ ID NO:140, and a light chain CDR3 of SEQ ID NO: 141;
   (ll) a heavy chain CDR1 of SEQ ID NO: 129, a heavy chain CDR2 of SEQ ID NO:130, a heavy chain CDR3 of SEQ ID NO:131, a light chain CDR1 of SEQ ID NO:142, a light chain CDR2 of SEQ ID NO:140, and a light chain CDR3 of SEQ ID NO:138;
   (mm) a heavy chain CDR1 of SEQ ID NO:123, a heavy chain CDR2 of SEQ ID NO:124, a heavy chain CDR3 of SEQ ID NO:147, a light chain CDR1 of SEQ ID NO:153, a light chain CDR2 of SEQ ID NO:154, and a light chain CDR3 of SEQ ID NO:155;
   (nn) a heavy chain CDR1 of SEQ ID NO:126, a heavy chain CDR2 of SEQ ID NO:124, a heavy chain CDR3 of SEQ ID NO:147, a light chain CDR1 of SEQ ID NO:153, a light chain CDR2 of SEQ ID NO: 154, and a light chain CDR3 of SEQ ID NO:155;
   (oo) a heavy chain CDR1 of SEQ ID NO:127, a heavy chain CDR2 of SEQ ID NO:128, a heavy chain CDR3 of SEQ ID NO:147, a light chain CDR1 of SEQ ID NO:156, a light chain CDR2 of SEQ ID NO:50, and a light chain CDR3 of SEQ ID NO:157;
   (pp) a heavy chain CDR1 of SEQ ID NO: 129, a heavy chain CDR2 of SEQ ID NO:130, a heavy chain CDR3 of SEQ ID NO:148, a light chain CDR1 of SEQ ID NO:158, a light chain CDR2 of SEQ ID NO:50, and a light chain CDR3 of SEQ ID NO:155;
   (qq) a heavy chain CDR1 of SEQ ID NO:103, a heavy chain CDR2 of SEQ ID NO:104, a heavy chain CDR3 of SEQ ID NO:163, a light chain CDR1 of SEQ ID NO: 116, a light chain CDR2 of SEQ ID NO:47, and a light chain CDR3 of SEQ ID NO:169;
   (rr) a heavy chain CDR1 of SEQ ID NO:106, a heavy chain CDR2 of SEQ ID NO:104, a heavy chain CDR3 of SEQ ID NO:163, a light chain CDR1 of SEQ ID NO:116, a light chain CDR2 of SEQ ID NO:47, and a light chain CDR3 of SEQ ID NO:169;
   (ss) a heavy chain CDR1 of SEQ ID NO:107, a heavy chain CDR2 of SEQ ID NO:108, a heavy chain CDR3 of SEQ ID NO:163, a light chain CDR1 of SEQ ID NO:49, a light chain CDR2 of SEQ ID NO:50, and a light chain CDR3 of SEQ ID NO:170;
   (tt) a heavy chain CDR1 of SEQ ID NO: 109, a heavy chain CDR2 of SEQ ID NO:110, a heavy chain CDR3 of SEQ ID NO:164, a light chain CDR1 of SEQ ID NO:52, a light chain CDR2 of SEQ ID NO:50, and a light chain CDR3 of SEQ ID NO:169;
   (uu) a heavy chain CDR1 of SEQ ID NO:175, a heavy chain CDR2 of SEQ ID NO:176, a heavy chain CDR3 of SEQ ID NO:177, a light chain CDR1 of SEQ ID NO:116, a light chain CDR2 of SEQ ID NO:47, and a light chain CDR3 of SEQ ID NO:188;
   (vv) a heavy chain CDR1 of SEQ ID NO:178, a heavy chain CDR2 of SEQ ID NO:176, a heavy chain CDR3 of SEQ ID NO:177, a light chain CDR1 of SEQ ID NO:116, a light chain CDR2 of SEQ ID NO:47, and a light chain CDR3 of SEQ ID NO:188;
   (ww) a heavy chain CDR1 of SEQ ID NO:179, a heavy chain CDR2 of SEQ ID NO:180, a heavy chain CDR3 of SEQ ID NO:177, a light chain CDR1 of SEQ ID NO:49, a light chain CDR2 of SEQ ID NO:50, and a light chain CDR3 of SEQ ID NO:189;
   (xx) a heavy chain CDR1 of SEQ ID NO: 181, a heavy chain CDR2 of SEQ ID NO:182; a heavy chain CDR3 of SEQ ID NO:183, a light chain CDR1 of SEQ ID NO:52, a light chain CDR2 of SEQ ID NO:50, and a light chain CDR3 of SEQ ID NO:188;
   (yy) a heavy chain CDR1 of SEQ ID NO: 103, a heavy chain CDR2 of SEQ ID NO: 104, a heavy chain CDR3 of SEQ ID NO:194, a light chain CDR1 of SEQ ID NO: 116, a light chain CDR2 of SEQ ID NO: 47, and a light chain CDR3 of SEQ ID NO:200;
   (zz) a heavy chain CDR1 of SEQ ID NO: 106, a heavy chain CDR2 of SEQ ID NO: 104, a heavy chain CDR3 of SEQ ID NO:194, a light chain CDR1 of SEQ ID NO: 116, a light chain CDR2 of SEQ ID NO: 47, and a light chain CDR3 of SEQ ID NO:200;
   (aaa) a heavy chain CDR1 of SEQ ID NO: 107, a heavy chain CDR2 of SEQ ID NO: 108, a heavy chain CDR3 of SEQ ID NO: 194, a light chain CDR1 of SEQ ID NO: 49, a light chain CDR2 of SEQ ID NO: 50, and a light chain CDR3 of SEQ ID NO: 201;
   (bbb) a heavy chain CDR1 of SEQ ID NO: 109, a heavy chain CDR2 of SEQ ID NO: 110, a heavy chain CDR3 of SEQ ID NO: 195, a light chain CDR1 of SEQ ID NO: 52, a light chain CDR2 of SEQ ID NO: 50, and a light chain CDR3 of SEQ ID NO:200;
   (ccc) a heavy chain CDR1 of SEQ ID NO:206, a heavy chain CDR2 of SEQ ID NO:207, a heavy chain CDR3 of SEQ ID NO:208, a light chain CDR1 of SEQ ID NO:153, a light chain CDR2 of SEQ ID NO:154, and a light chain CDR3 of SEQ ID NO:219;
   (ddd) a heavy chain CDR1 of SEQ ID NO:209, a heavy chain CDR2 of SEQ ID NO:207, a heavy chain CDR3 of SEQ ID NO:208, a light chain CDR1 of SEQ ID NO:153, a light chain CDR2 of SEQ ID NO: 154, and a light chain CDR3 of SEQ ID NO:219;
   (eee) a heavy chain CDR1 of SEQ ID NO:210, a heavy chain CDR2 of SEQ ID NO:211, a heavy chain CDR3 of SEQ ID NO:208, a light chain CDR1 of SEQ ID NO:156, a light chain CDR2 of SEQ ID NO:50, and a light chain CDR3 of SEQ ID NO:220;
   (fff) a heavy chain CDR1 of SEQ ID NO: 212, a heavy chain CDR2 of SEQ ID NO:213, a heavy chain CDR3 of SEQ ID NO:214, a light chain CDR1 of SEQ ID NO:158, a light chain CDR2 of SEQ ID NO:50, and a light chain CDR3 of SEQ ID NO:219;
   (ggg) a heavy chain CDR1 of SEQ ID NO: 206, a heavy chain CDR2 of SEQ ID NO: 207, a heavy chain CDR3 of SEQ ID NO:225, a light chain CDR1 of SEQ ID NO:136, a light chain CDR2 of SEQ ID NO:137,and a light chain CDR3 of SEQ ID NO:231;
   (hhh) a heavy chain CDR1 of SEQ ID NO: 209, a heavy chain CDR2 of SEQ ID NO: 207, a heavy chain CDR3 of SEQ ID NO:225, a light chain CDR1 of SEQ ID NO:136, a light chain CDR2 of SEQ ID NO:137, and a light chain CDR3 of SEQ ID NO:231;
   (iii) a heavy chain CDR1 of SEQ ID NO: 210, a heavy chain CDR2 of SEQ ID NO: 211, a heavy chain CDR3 of SEQ ID NO:225, a light chain CDR1 of SEQ ID NO:139, a light chain CDR2 of SEQ ID NO:140, and a light chain CDR3 of SEQ ID NO: 232;
   (jjj) a heavy chain CDR1 of SEQ ID NO: 212, a heavy chain CDR2 of SEQ ID NO: 213, a heavy chain CDR3 of SEQ ID NO: 226, a light chain CDR1 of SEQ ID NO:142; a light chain CDR2 of SEQ ID NO: 140; and a light chain CDR3 of SEQ ID NO:231;
   (kkk) a heavy chain variable region that comprises an HCDR1 of SEQ ID NO: 206, an HCDR2 of SEQ ID NO: 207, and an HCDR3 of SEQ ID NO:237,and a light chain variable region that comprises an LCDR1 of SEQ ID NO:243, an LCDR2 of SEQ ID NO:47, and an LCDR3 of SEQ ID NO:244;
   (lll) a heavy chain variable region that comprises an HCDR1 of SEQ ID NO: 209, an HCDR2 of SEQ ID NO: 207, and an HCDR3 of SEQ ID NO:237, and a light chain variable region that comprises an LCDR1 of SEQ ID NO:243, an LCDR2 of SEQ ID NO:47, and an LCDR3 of SEQ ID NO:244;
   (mmm) a heavy chain variable region that comprises an HCDR1 of SEQ ID NO: 210, an HCDR2 of SEQ ID NO: 211, and an HCDR3 of SEQ ID NO:237, and a light chain variable region that comprises an LCDR1 of SEQ ID NO:245, an LCDR2 of SEQ ID NO:50, and an LCDR3 of SEQ ID NO:246;
   (nnn) a heavy chain variable region that comprises an HCDR1 of SEQ ID NO: 212, an HCDR2 of SEQ ID NO: 213, and an HCDR3 of SEQ ID NO:238; and a light chain variable region that comprises an LCDR1 of SEQ ID NO:247, an LCDR2 of SEQ ID NO: 50, and an LCDR3 of SEQ ID NO:244;
   (ooo) a heavy chain variable region that comprises an HCDR1 of SEQ ID NO: 206, an HCDR2 of SEQ ID NO: 207, and an HCDR3 of SEQ ID NO:252,and a light chain variable region that comprises an LCDR1 of SEQ ID NO:153, an LCDR2 of SEQ ID NO: 154, and an LCDR3 of SEQ ID NO:258;
   (ppp) a heavy chain variable region that comprises an HCDR1 of SEQ ID NO: 209, an HCDR2 of SEQ ID NO: 207, and an HCDR3 of SEQ ID NO:252, and a light chain variable region that comprises an LCDR1 of SEQ ID NO:153, an LCDR2 of SEQ ID NO:154, and an LCDR3 of SEQ ID NO:258;
   (qqq) a heavy chain variable region that comprises an HCDR1 of SEQ ID NO: 210, an HCDR2 of SEQ ID NO: 211, and an HCDR3 of SEQ ID NO:252, and a light chain variable region that comprises an LCDR1 of SEQ ID NO:156, an LCDR2 of SEQ ID NO:50, and an LCDR3 of SEQ ID NO:259; or
   (rrr) a heavy chain variable region that comprises an HCDR1 of SEQ ID NO: 212, an HCDR2 of SEQ ID NO: 213, and an HCDR3 of SEQ ID NO: 253; and a light chain variable region that comprises an LCDR1 of SEQ ID NO:158, an LCDR2 of SEQ ID NO:50, and an LCDR3 of SEQ ID NO:258.
320. The process of any of embodiments 202-243 or 319, the formulation of any of embodiments 244-299 or 319, the container of any of embodiments 300-302 or 319, the method of any of embodiments 303-310 or 319, or the formulation for use of any of embodiments 311-319, wherein the antibody or antigen binding fragment thereof that binds PMEL17 comprises:
   (a) a heavy chain variable region (VH) comprising the amino acid sequence of SEQ ID NO:10, and a light chain variable region (VL) comprising the amino acid sequence of SEQ ID NO:21;
   (b) a heavy chain variable region (VH) comprising the amino acid sequence of SEQ ID NO:10, and a light chain variable region (VL) comprising the amino acid sequence of SEQ ID NO:25;
   (c) a heavy chain variable region (VH) comprising the amino acid sequence of SEQ ID NO:10, and a light chain variable region (VL) comprising the amino acid sequence of SEQ ID NO:29;
   (d) a heavy chain variable region (VH) comprising the amino acid sequence of SEQ ID NO:42, and a light chain variable region (VL) comprising the amino acid sequence of SEQ ID NO:53;
   (e) a heavy chain variable region (VH) comprising the amino acid sequence of SEQ ID NO:64, and a light chain variable region (VL) comprising the amino acid sequence of SEQ ID NO:75;
   (f) a heavy chain variable region (VH) comprising the amino acid sequence of SEQ ID NO:88, and a light chain variable region (VL) comprising the amino acid sequence of SEQ ID NO:99;
   (g) heavy chain variable region (VH) comprising the amino acid sequence of SEQ ID NO:112, and a light chain variable region (VL) comprising the amino acid sequence of SEQ ID NO:119;
   (h) heavy chain variable region (VH) comprising the amino acid sequence of SEQ ID NO:132, and a light chain variable region (VL) comprising the amino acid sequence of SEQ ID NO:143;
   (i) heavy chain variable region (VH) comprising the amino acid sequence of SEQ ID NO:149, and a light chain variable region (VL) comprising the amino acid sequence of SEQ ID NO:159;
   (j) heavy chain variable region (VH) comprising the amino acid sequence of SEQ ID NO:165, and a light chain variable region (VL) comprising the amino acid sequence of SEQ ID NO:171;
   (k) heavy chain variable region (VH) comprising the amino acid sequence of SEQ ID NO:184, and a light chain variable region (VL) comprising the amino acid sequence of SEQ ID NO:190;
   (l) heavy chain variable region (VH) comprising the amino acid sequence of SEQ ID NO:196, and a light chain variable region (VL) comprising the amino acid sequence of SEQ ID NO:202;
   (m) heavy chain variable region (VH) comprising the amino acid sequence of SEQ ID NO:215, and a light chain variable region (VL) comprising the amino acid sequence of SEQ ID NO:221;
   (n) heavy chain variable region (VH) comprising the amino acid sequence of SEQ ID NO:227, and a light chain variable region (VL) comprising the amino acid sequence of SEQ ID NO:233;
   (o) heavy chain variable region (VH) comprising the amino acid sequence of SEQ ID NO:239, and a light chain variable region (VL) comprising the amino acid sequence of SEQ ID NO:248; or
   (p) heavy chain variable region (VH) comprising the amino acid sequence of SEQ ID NO:254, and a light chain variable region (VL) comprising the amino acid sequence of SEQ ID NO:260.
321. The process of any of embodiments 202-243, 319, or 320, the formulation of any of embodiments 244-299, 319, or 320, the container of any of embodiments 300-302, 319, or 320, the method of any of embodiments 303-310, 319, or 320, or the formulation for use of any of embodiments 311-320, wherein the antibody or antigen binding fragment thereof that binds PMEL17 comprises:
   (a) a heavy chain comprising the amino acid sequence of SEQ ID NO:12, and a light chain comprising the amino acid sequence of SEQ ID NO:23;
   (b) a heavy chain comprising the amino acid sequence of SEQ ID NO:12, and a light chain comprising the amino acid sequence of SEQ ID NO:27;
   (c) a heavy chain comprising the amino acid sequence of SEQ ID NO:12, and a light chain comprising the amino acid sequence of SEQ ID NO:31;
   (d) a heavy chain comprising the amino acid sequence of SEQ ID NO:44, and a light chain comprising the amino acid sequence of SEQ ID NO:55;
   (e) a heavy chain comprising the amino acid sequence of SEQ ID NO:66, and a light chain comprising the amino acid sequence of SEQ ID NO:77;
   (f) a heavy chain comprising the amino acid sequence of SEQ ID NO:90, and a light chain comprising the amino acid sequence of SEQ ID NO:101;
   (g) a heavy chain comprising the amino acid sequence of SEQ ID NO:114, and a light chain comprising the amino acid sequence of SEQ ID NO:121;
   (h) a heavy chain comprising the amino acid sequence of SEQ ID NO:134, and a light chain comprising the amino acid sequence of SEQ ID NO:145;
   (i) a heavy chain comprising the amino acid sequence of SEQ ID NO:151, and a light chain comprising the amino acid sequence of SEQ ID NO:161;
   (j) a heavy chain comprising the amino acid sequence of SEQ ID NO:167, and a light chain comprising the amino acid sequence of SEQ ID NO:173;
   (k) a heavy chain comprising the amino acid sequence of SEQ ID NO:186, and a light chain comprising the amino acid sequence of SEQ ID NO:192;
   (l) a heavy chain comprising the amino acid sequence of SEQ ID NO:198, and a light chain comprising the amino acid sequence of SEQ ID NO:204;
   (m) a heavy chain comprising the amino acid sequence of SEQ ID NO:217, and a light chain comprising the amino acid sequence of SEQ ID NO:223;
   (n) a heavy chain comprising the amino acid sequence of SEQ ID NO:229, and a light chain comprising the amino acid sequence of SEQ ID NO:235;
   (o) a heavy chain comprising the amino acid sequence of SEQ ID NO:241, and a light chain comprising the amino acid sequence of SEQ ID NO:250;
   (p) a heavy chain comprising the amino acid sequence of SEQ ID NO:256, and a light chain comprising the amino acid sequence of SEQ ID NO:262;
   (q) a heavy chain comprising the amino acid sequence of SEQ ID NO:283, and a light chain comprising the amino acid sequence of SEQ ID NO:27;
   (r) a heavy chain comprising the amino acid sequence of SEQ ID NO:283, and a light chain comprising the amino acid sequence of SEQ ID NO:31; or
   (s) a heavy chain comprising the amino acid sequence of SEQ ID NO:315, and a light chain comprising the amino acid sequence of SEQ ID NO:101.
322. The process of any of embodiments 202-243 or 319-321, the formulation of any of embodiments 244-299 or 319-321, the container of any of embodiments 300-302 or 319-321, the method of any of embodiments 303-310 or 319-321, or the formulation for use of any of embodiments 311-321, wherein the antibody or antigen binding fragment thereof comprises one or more cysteine substitutions.
323. The process of any of embodiments 202-243 or 319-322, the formulation of any of embodiments 244-299 or 319-322, the container of any of embodiments 300-302 or 319-322, the method of any of embodiments 303-310 or 319-322, or the formulation for use of any of embodiments 311-322, wherein the antibody or antigen binding fragment thereof comprises one or more cysteine substitutions selected from E152C, S375C, or both E152C and S375C of the heavy chain of the antibody or antigen binding fragment thereof, wherein the position is numbered according to the EU system.
324. The process of any of embodiments 202-243 or 319-323, the formulation of any of embodiments 244-299 or 319-323, the container of any of embodiments 300-302 or 319-323, the method of any of embodiments 303-310 or 319-323, or the formulation for use of any of embodiments 311-323, wherein the antibody is a monoclonal antibody, an isolated antibody, a synthetic antibody, or an engineered antibody.
325. The process of any of embodiments 237-243 or 319-324, the formulation of any of embodiments 244-299 or 319-324, the container of any of embodiments 300-302 or 319-324, the method of any of embodiments 303-310 or 319-324, or the formulation for use of any of embodiments 311-324, wherein the antibody drug conjugate comprises the formula (C)

   Ab-(L_{A}-(D)ₙ)_{y} (C)

   wherein:
   D is a GNAQ inhibitor, a GNA11 inhibitor or an inhibitor of GNAQ and GNA11 (*e.g.,* a GNAQ inhibitor, a GNA11 inhibitor or an inhibitor of GNAQ and GNA11, as described herein);
   Ab is an antibody or antigen binding fragment thereof that binds to human PMEL17 protein (*e.g.,* an antibody or antigen binding fragment thereof that binds to human PMEL17 protein as described herein);
   L_{A} is a linker (*e.g.,* a linker described herein);
   n is 1, 2, 3 or 4, and
   y is 1, 2, 3 or 4.
326. The process or the formulation of embodiment 325, wherein said n is 1.
327. The process or the formulation of any of embodiments 325 or 326, wherein said y is 2.
328. The process or the formulation of any of embodiments 325-327, wherein said linker is a cleavable linker or a non-cleavable linker.
329. The process or the formulation of any of embodiments 325-328, wherein said linker comprises a ValCit peptide linker.
330. The process or the formulation of any of embodiments 325-329, wherein said drug moiety is an inhibitor of GNAQ and GNA11.
331. The process or the formulation of any of embodiments 325-330, wherein D is 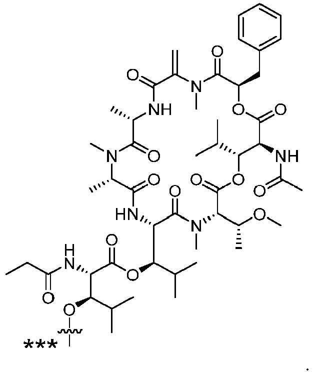
332. The process or the formulation of any of embodiments 325-331, wherein D is any of compound (A1) or compounds 2-15.
333. The process or the formulation of any of embodiments 325-331, wherein the antibody drug conjugate has the following structure, 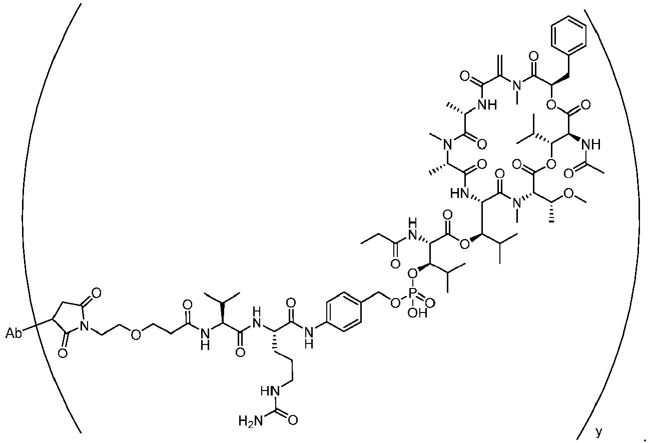
334. The process or the formulation of any of embodiments 325-330, wherein the antibody drug conjugate has the following Formula (C-2): 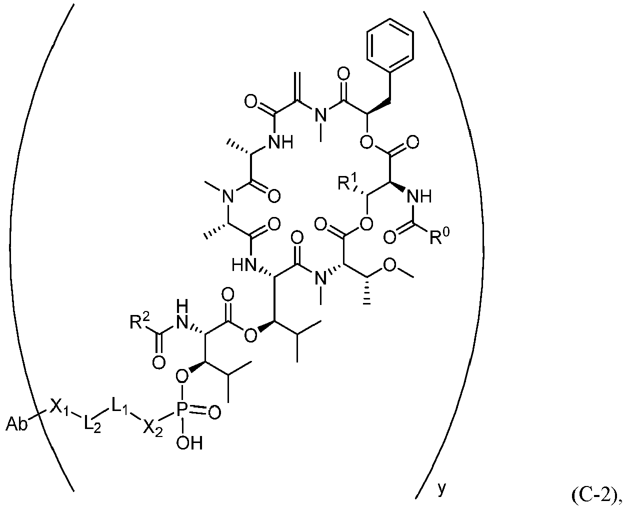 wherein:
   R⁰ is methyl or ethyl;
   R¹ is methyl or isopropyl;
   R² is methyl or ethyl;
   Ab is an antibody or antigen binding fragment thereof that binds to human PMEL17 protein;
   X₁ is a bivalent coupling group;
   X₂ is a self-immolative spacer;
   L₁ is a bivalent peptide linker;
   L₂ is a bond or a linker, and
   y is 1, 2, 3 or 4.
335. The process or the formulation of any of embodiments 325-330, wherein the antibody drug conjugate has the following Formula (Cb-2): 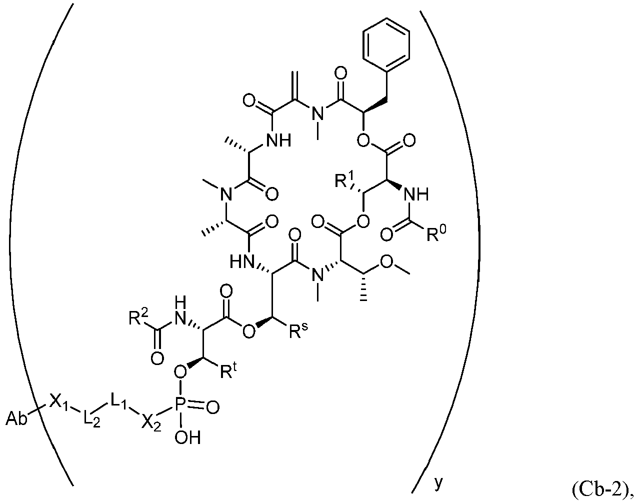 wherein:
   R⁰ is methyl or ethyl;
   R¹, R^{s}, and R^{t} are each independently methyl, methylthiomethyl, or isopropyl;
   R² is methyl or ethyl;
   Ab is an antibody or antigen binding fragment thereof that binds to human PMEL17 protein;
   X₁ is a bivalent coupling group;
   X₂ is a self-immolative spacer;
   L₁ is a bivalent peptide linker;
   L₂ is a bond or a linker, and
   y is 1, 2, 3 or 4.
336. The process or the formulation of any of embodiments 325-330, wherein the antibody drug conjugate has the following Formula (Cc-2): 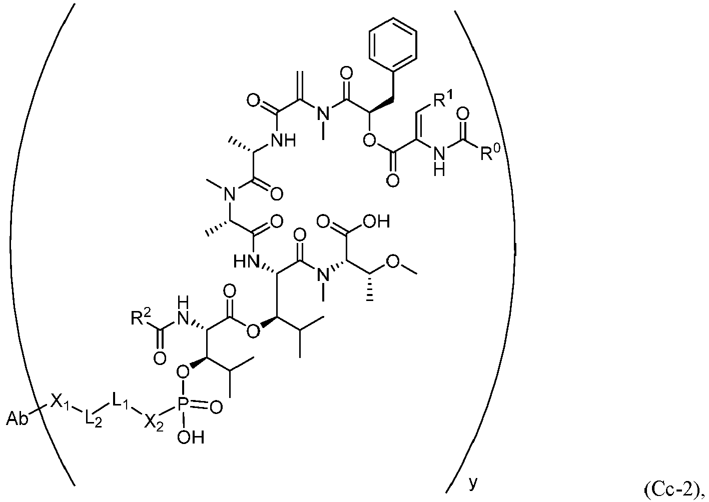 wherein:
   R⁰ is methyl or ethyl;
   R¹ is methyl or isopropyl;
   R² is methyl or ethyl;
   Ab is an antibody or antigen binding fragment thereof that binds to human PMEL17 protein;
   X₁ is a bivalent coupling group;
   X₂ is a self-immolative spacer;
   L₁ is a bivalent peptide linker;
   L₂ is a bond or a linker, and
   y is 1, 2, 3 or 4.
337. A method of treating a cancer, comprising administering to a subject in need thereof an antibody drug conjugate (ADC) described herein, *e.g.,* at a dose of 1 mg/kg to 16 mg/kg, *e.g.,* once every two weeks intravenously, optionally wherein the ADC has following structure: 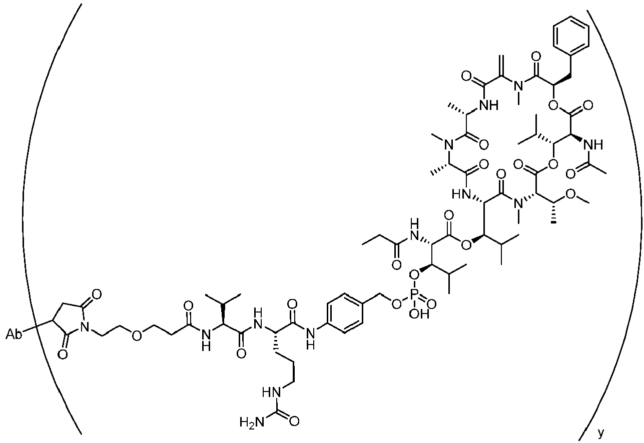 wherein:
   Ab is an antibody or antigen binding fragment thereof that binds to human PMEL17 protein and comprises:
      (a) a heavy chain CDR1 of SEQ ID NO: 1, a heavy chain CDR2 of SEQ ID NO: 2, a heavy chain CDR3 of SEQ ID NO: 3, a light chain CDR1 of SEQ ID NO: 14, a light chain CDR2 of SEQ ID NO: 15, and a light chain CDR3 of SEQ ID NO: 16;
      (b) a heavy chain CDR1 of SEQ ID NO: 4, a heavy chain CDR2 of SEQ ID NO: 2, a heavy chain CDR3 of SEQ ID NO: 3, a light chain CDR1 of SEQ ID NO: 14, a light chain CDR2 of SEQ ID NO: 15, and a light chain CDR3 of SEQ ID NO: 16;
      (c) a heavy chain CDR1 of SEQ ID NO: 5, a heavy chain CDR2 of SEQ ID NO: 6, a heavy chain CDR3 of SEQ ID NO: 3, a light chain CDR1 of SEQ ID NO: 17, a light chain CDR2 of SEQ ID NO: 18, and a light chain CDR3 of SEQ ID NO: 19; or
      (d) a heavy chain CDR1 of SEQ ID NO: 7, a heavy chain CDR2 of SEQ ID NO: 8, a heavy chain CDR3 of SEQ ID NO: 9, a light chain CDR1 of SEQ ID NO: 20, a light chain CDR2 of SEQ ID NO: 18, and a light chain CDR3 of SEQ ID NO: 16;
   y is 2; and
   wherein the cancer expresses PMEL17, contains a mutation of the GNAQ or GNA11 gene, or expresses PMEL17 and contains a mutation of GNAQ, GNA11, or both,
   thereby treating the cancer.
338. The method of embodiment 337, wherein the ADC is administered at a dose of 2 mg/kg to 15 mg/kg once every two weeks intravenously.
339. The method of embodiment 337, wherein the ADC is administered at a dose of 1 mg/kg, 2 mg/kg, 4 mg/kg, 8 mg/kg, 12 mg/kg, or 16 mg/kg, once every two weeks intravenously.
340. The method of any of embodiments 337-339, wherein the cancer is a carcinoma, sarcoma, leukemia, lymphoma, eye cancer, eye neoplasm, melanoma, or a metastatic cancer thereof.
341. The method of embodiment 340, wherein the carcinoma is a hepatocellular carcinoma, or a metastatic lesion thereof.
342. The method of embodiment 340, wherein the melanoma is a uveal melanoma, non-uveal melanoma, malignant melanoma, ocular melanoma, mucosal melanoma, subcutaneous melanoma, cutaneous melanoma, or a metastatic lesion thereof.
343. The method of any of embodiments 337-342, wherein the subject has been treated with tebentafusp prior to the administration of the ADC.
344. The method of any of embodiments 337-342, wherein the subject has not been treated with tebentafusp prior to the administration of the ADC.
345. The method of any of embodiments 337-344, wherein the Ab comprises a heavy chain variable region (VH) comprising the amino acid sequence of SEQ ID NO: 10 and a light chain variable region (VL) comprising the amino acid sequence of SEQ ID NO: 25.
346. The method of any of embodiments 337-345, wherein the Ab comprises a heavy chain comprising the amino acid sequence of SEQ ID NO: 283 and a light chain comprising the amino acid sequence of SEQ ID NO: 27.
347. The method of embodiment 346, wherein the heavy chain comprises an N-glycosylation site located at Asn306.
348. The method of any of embodiments 337-347, wherein the method further comprises determining the expression of one or more (*e.g.,* 2, 3, 4, 5, or 6) biomarkers selected from PMEL17, pERK, CD8, PD-L1, DUSP6, RASGRP3, or any combination thereof in a sample from the subject.
349. The method of embodiment 348, wherein the sample is obtained from the subject either before, during and/or after administration of the ADC.
350. The method of embodiment 348 or 349, wherein the sample is a tumor sample, tumor-adjacent tissue sample, or a bodily fluid sample (*e.g.,* blood, serum, spinal fluid, or urine).
351. Use of an antibody drug conjugate (ADC) described herein in the manufacture of a mediacament for treating a cancer in a subject, optionally wherein the ADC has following structure: 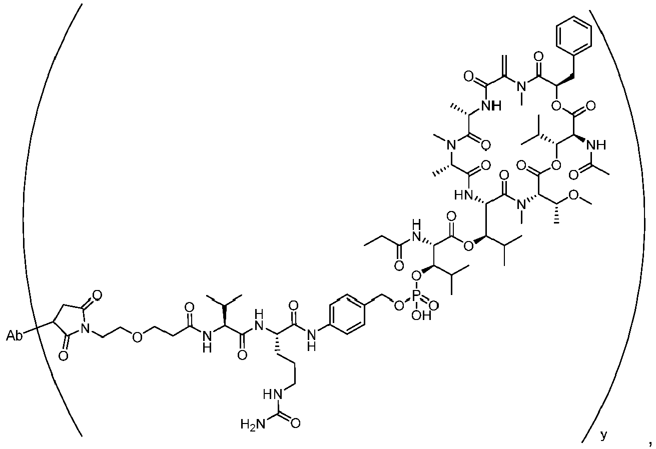 wherein:
   Ab is an antibody or antigen binding fragment thereof that binds to human PMEL17 protein and comprises:
      (a) a heavy chain CDR1 of SEQ ID NO: 1, a heavy chain CDR2 of SEQ ID NO: 2, a heavy chain CDR3 of SEQ ID NO: 3, a light chain CDR1 of SEQ ID NO: 14, a light chain CDR2 of SEQ ID NO: 15, and a light chain CDR3 of SEQ ID NO: 16;
      (b) a heavy chain CDR1 of SEQ ID NO: 4, a heavy chain CDR2 of SEQ ID NO: 2, a heavy chain CDR3 of SEQ ID NO: 3, a light chain CDR1 of SEQ ID NO: 14, a light chain CDR2 of SEQ ID NO: 15, and a light chain CDR3 of SEQ ID NO: 16;
      (c) a heavy chain CDR1 of SEQ ID NO: 5, a heavy chain CDR2 of SEQ ID NO: 6, a heavy chain CDR3 of SEQ ID NO: 3, a light chain CDR1 of SEQ ID NO: 17, a light chain CDR2 of SEQ ID NO: 18, and a light chain CDR3 of SEQ ID NO: 19; or
      (d) a heavy chain CDR1 of SEQ ID NO: 7, a heavy chain CDR2 of SEQ ID NO: 8, a heavy chain CDR3 of SEQ ID NO: 9, a light chain CDR1 of SEQ ID NO: 20, a light chain CDR2 of SEQ ID NO: 18, and a light chain CDR3 of SEQ ID NO: 16;
   y is 2; and
   wherein the cancer expresses PMEL17, contains a mutation of the GNAQ or GNA11 gene, or expresses PMEL17 and contains a mutation of GNAQ, GNA11, or both, optionally wherein the ADC is administered at a dose of 1 mg/kg to 16 mg/kg, *e.g.*, once every two weeks intravenously.
352. The use of embodiment 351, wherein the ADC is administered at a dose of 2 mg/kg to 15 mg/kg once every two weeks intravenously.
353. The use of embodiment 351, wherein the ADC is administered at a dose of 1 mg/kg, 2 mg/kg, 4 mg/kg, 8 mg/kg, 12 mg/kg, or 16 mg/kg, once every two weeks intravenously.
354. The use of any of embodiments 351-353, wherein the cancer is a carcinoma, sarcoma, leukemia, lymphoma, eye cancer, eye neoplasm, melanoma, or a metastatic cancer thereof.
355. The use of embodiment 354, wherein the carcinoma is a hepatocellular carcinoma, or a metastatic lesion thereof.
356. The use of any embodiment 354, wherein the melanoma is a malignant melanoma, uveal melanoma, non-uveal melanoma, ocular melanoma, subcutaneous melanoma, cutaneous melanoma, mucosal melanoma, or a metastatic lesion thereof.
357. The use of any of embodiments 351-356, wherein the subject has been treated with tebentafusp prior to the administration of the ADC.
358. The use of any of embodiments 351-356, wherein the subject has not been treated with tebentafusp prior to the administration of the ADC.
359. The use of any of embodiments 351-358, wherein the Ab comprises a heavy chain variable region (VH) comprising the amino acid sequence of SEQ ID NO: 10 and a light chain variable region (VL) comprising the amino acid sequence of SEQ ID NO: 25.
360. The use of any of embodiments 351-359, wherein the Ab comprises a heavy chain comprising the amino acid sequence of SEQ ID NO: 283 and a light chain comprising the amino acid sequence of SEQ ID NO: 27.
361. The use of embodiment 360, wherein the heavy chain comprises an N-glycosylation site located at Asn306.
362. The use of any of embodiments 351-361, wherein the use further comprises determining the expression of one or more (*e.g.,* 2, 3, 4, 5, or 6) biomarkers selected from PMEL17, pERK, CD8, PD-L1, DUSP6, RASGRP3, or any combination thereof in a sample from the subject.
363. The use of embodiment 362, wherein the sample is obtained from the subject either before, during and/or after administration of the ADC.
364. The use of embodiment 362 or 363, wherein the sample is a tumor sample, tumor-adjacent tissue sample, or a bodily fluid sample (*e.g.,* blood, serum, spinal fluid, or urine).
365. An antibody drug conjugate (ADC) described herein for use in a method of treating a cancer in a subject, optionally wherein the ADC has following structure: 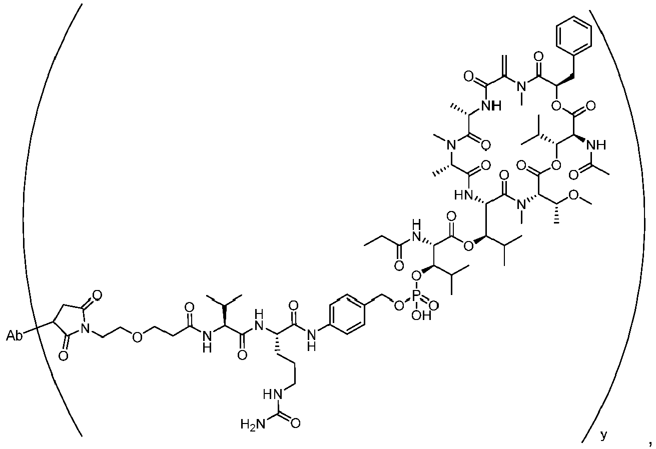 wherein:
   Ab is an antibody or antigen binding fragment thereof that binds to human PMEL17 protein and comprises:
      (a) a heavy chain CDR1 of SEQ ID NO: 1, a heavy chain CDR2 of SEQ ID NO: 2, a heavy chain CDR3 of SEQ ID NO: 3, a light chain CDR1 of SEQ ID NO: 14, a light chain CDR2 of SEQ ID NO: 15, and a light chain CDR3 of SEQ ID NO: 16;
      (b) a heavy chain CDR1 of SEQ ID NO: 4, a heavy chain CDR2 of SEQ ID NO: 2, a heavy chain CDR3 of SEQ ID NO: 3, a light chain CDR1 of SEQ ID NO: 14, a light chain CDR2 of SEQ ID NO: 15, and a light chain CDR3 of SEQ ID NO: 16;
      (c) a heavy chain CDR1 of SEQ ID NO: 5, a heavy chain CDR2 of SEQ ID NO: 6, a heavy chain CDR3 of SEQ ID NO: 3, a light chain CDR1 of SEQ ID NO: 17, a light chain CDR2 of SEQ ID NO: 18, and a light chain CDR3 of SEQ ID NO: 19; or
      (d) a heavy chain CDR1 of SEQ ID NO: 7, a heavy chain CDR2 of SEQ ID NO: 8, a heavy chain CDR3 of SEQ ID NO: 9, a light chain CDR1 of SEQ ID NO: 20, a light chain CDR2 of SEQ ID NO: 18, and a light chain CDR3 of SEQ ID NO: 16;
   y is 2; and
   wherein the cancer expresses PMEL17, contains a mutation of the GNAQ or GNA11 gene, or expresses PMEL17 and contains a mutation of GNAQ, GNA11, or both, optionally wherein the ADC is administered at a dose of 1 mg/kg to 16 mg/kg, *e.g.*, once every two weeks intravenously.
366. The ADC for use of embodiment 365, wherein the ADC is administered at a dose of 2 mg/kg to 15 mg/kg once every two weeks intravenously.
367. The ADC for use of embodiment 365, wherein the ADC is administered at a dose of 1 mg/kg, 2 mg/kg, 4 mg/kg, 8 mg/kg, 12 mg/kg, or 16 mg/kg, once every two weeks intravenously.
368. The ADC for use of any of embodiments 355-367, wherein the cancer is a carcinoma, sarcoma, leukemia, lymphoma, eye cancer, eye neoplasm, melanoma, or a metastatic cancer thereof.
369. The ADC for use of embodiment 368, wherein the carcinoma is a hepatocellular carcinoma, or a metastatic lesion thereof.
370. The ADC for use of any embodiment 368, wherein the melanoma is a malignant melanoma, uveal melanoma, non-uveal melanoma, ocular melanoma, subcutaneous melanoma, cutaneous melanoma, mucosal melanoma, or a metastatic lesion thereof.
371. The ADC for use of any of embodiments 365-370, wherein the subject has been treated with tebentafusp prior to the administration of the ADC.
372. The ADC for use of any of embodiments 365-370, wherein the subject has not been treated with tebentafusp prior to the administration of the ADC.
373. The ADC for use of any of embodiments 365-372, wherein the Ab comprises a heavy chain variable region (VH) comprising the amino acid sequence of SEQ ID NO: 10 and a light chain variable region (VL) comprising the amino acid sequence of SEQ ID NO: 25.
374. The ADC for use of any of embodiments 365-373, wherein the Ab comprises a heavy chain comprising the amino acid sequence of SEQ ID NO: 283 and a light chain comprising the amino acid sequence of SEQ ID NO: 27.
375. The ADC for use of embodiment 374, wherein the heavy chain comprises an N-glycosylation site located at Asn306.
376. The ADC for use of any of embodiments 365-375, wherein the use further comprises determining the expression of one or more (*e.g.,* 2, 3, 4, 5, or 6) biomarkers selected from PMEL17, pERK, CD8, PD-L1, DUSP6, RASGRP3, or any combination thereof in a sample from the subject.
377. The ADC for use of embodiment 376, wherein the sample is obtained from the subject either before, during and/or after administration of the ADC.
378. The ADC for use of embodiment 376 or 377, wherein the sample is a tumor sample, tumor-adjacent tissue sample, or a bodily fluid sample (*e.g.,* blood, serum, spinal fluid, or urine).
379. A method of evaluating a treatment for a cancer in a subject, comprising determining the expression of one or more (*e.g.,* 2, 3, 4, 5, or 6) biomarkers selected from PMEL17, pERK, CD8, PD-L1, DUSP6, RASGRP3, or any combination thereof in a sample from the subject, wherein the treatment comprises administering to the subject an antibody drug conjugate (ADC) described herein, *e.g.,* at a dose of 1 mg/kg to 16 mg/kg, *e.g.*, once every two weeks intravenously, optionally wherein the ADC has following structure: 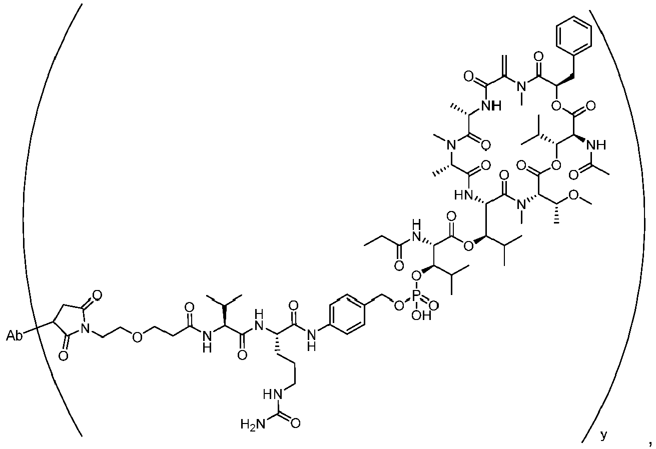 wherein:
   Ab is an antibody or antigen binding fragment thereof that binds to human PMEL17 protein and comprises:
      (a) a heavy chain CDR1 of SEQ ID NO: 1, a heavy chain CDR2 of SEQ ID NO: 2, a heavy chain CDR3 of SEQ ID NO: 3, a light chain CDR1 of SEQ ID NO: 14, a light chain CDR2 of SEQ ID NO: 15, and a light chain CDR3 of SEQ ID NO: 16;
      (b) a heavy chain CDR1 of SEQ ID NO: 4, a heavy chain CDR2 of SEQ ID NO: 2, a heavy chain CDR3 of SEQ ID NO: 3, a light chain CDR1 of SEQ ID NO: 14, a light chain CDR2 of SEQ ID NO: 15, and a light chain CDR3 of SEQ ID NO: 16;
      (c) a heavy chain CDR1 of SEQ ID NO: 5, a heavy chain CDR2 of SEQ ID NO: 6, a heavy chain CDR3 of SEQ ID NO: 3, a light chain CDR1 of SEQ ID NO: 17, a light chain CDR2 of SEQ ID NO: 18, and a light chain CDR3 of SEQ ID NO: 19; or
      (d) a heavy chain CDR1 of SEQ ID NO: 7, a heavy chain CDR2 of SEQ ID NO: 8, a heavy chain CDR3 of SEQ ID NO: 9, a light chain CDR1 of SEQ ID NO: 20, a light chain CDR2 of SEQ ID NO: 18, and a light chain CDR3 of SEQ ID NO: 16;
   y is 2; and
   wherein the cancer expresses PMEL17, contains a mutation of the GNAQ or GNA11 gene, or expresses PMEL17 and contains a mutation of GNAQ, GNA11, or both,
   thereby evaluating the treatment for the cancer in the subject.
380. A method of evaluating the progression of a cancer in a subject, comprising determining the expression of one or more (*e.g.,* 2, 3, 4, 5, or 6) biomarkers selected from PMEL17, pERK, CD8, PD-L1, DUSP6, RASGRP3, or any combination thereof in a sample from the subject, wherein the subject has received, is receiving, or will receive an antibody drug conjugate (ADC) described herein, *e.g.,* at a dose of 1 mg/kg to 16 mg/kg, *e.g.*, once every two weeks intravenously, optionally wherein the ADC has following structure: 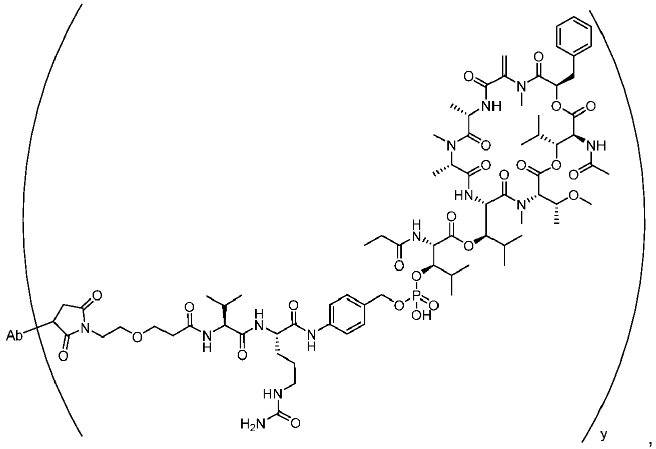 wherein:
   Ab is an antibody or antigen binding fragment thereof that binds to human PMEL17 protein and comprises:
      (a) a heavy chain CDR1 of SEQ ID NO: 1, a heavy chain CDR2 of SEQ ID NO: 2, a heavy chain CDR3 of SEQ ID NO: 3, a light chain CDR1 of SEQ ID NO: 14, a light chain CDR2 of SEQ ID NO: 15, and a light chain CDR3 of SEQ ID NO: 16;
      (b) a heavy chain CDR1 of SEQ ID NO: 4, a heavy chain CDR2 of SEQ ID NO: 2, a heavy chain CDR3 of SEQ ID NO: 3, a light chain CDR1 of SEQ ID NO: 14, a light chain CDR2 of SEQ ID NO:15, and a light chain CDR3 of SEQ ID NO: 16;
      (c) a heavy chain CDR1 of SEQ ID NO: 5, a heavy chain CDR2 of SEQ ID NO: 6, a heavy chain CDR3 of SEQ ID NO: 3, a light chain CDR1 of SEQ ID NO: 17, a light chain CDR2 of SEQ ID NO: 18, and a light chain CDR3 of SEQ ID NO: 19; or
      (d) a heavy chain CDR1 of SEQ ID NO: 7, a heavy chain CDR2 of SEQ ID NO: 8, a heavy chain CDR3 of SEQ ID NO: 9, a light chain CDR1 of SEQ ID NO: 20, a light chain CDR2 of SEQ ID NO: 18, and a light chain CDR3 of SEQ ID NO: 16;
   y is 2; and
   wherein the cancer expresses PMEL17, contains a mutation of the GNAQ or GNA11 gene, or expresses PMEL17 and contains a mutation of GNAQ, GNA11, or both,
   thereby evaluating the progression of the cancer in the subject.
381. A method of selecting a treatment for a subject having a cancer, comprising determining the expression of one or more (*e.g.,* 2, 3, 4, 5, or 6) biomarkers selected from PMEL17, pERK, CD8, PD-L1, DUSP6, RASGRP3, or any combination thereof in a sample from the subject, wherein the treatment comprises administering to the subject an antibody drug conjugate (ADC) described herein, *e.g.,* at a dose described herein (*e.g.,* at a dose of 1 mg/kg to 16 mg/kg), *e.g.,* once every two weeks intravenously, optionally wherein the ADC has following structure: 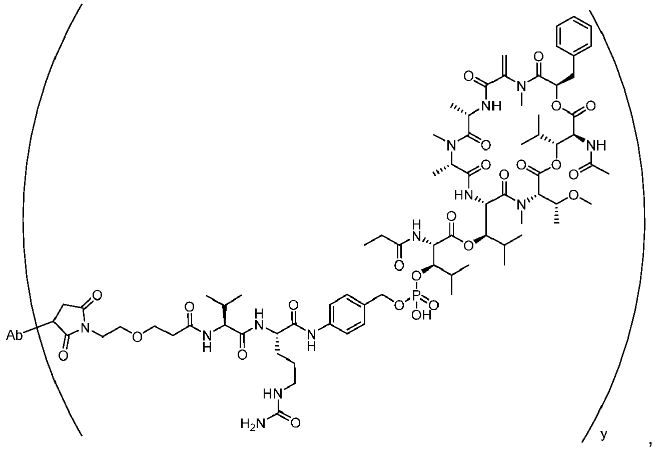 wherein:
   Ab is an antibody or antigen binding fragment thereof that binds to human PMEL17 protein and comprises:
      (a) a heavy chain CDR1 of SEQ ID NO: 1, a heavy chain CDR2 of SEQ ID NO: 2, a heavy chain CDR3 of SEQ ID NO: 3, a light chain CDR1 of SEQ ID NO: 14, a light chain CDR2 of SEQ ID NO: 15, and a light chain CDR3 of SEQ ID NO: 16;
      (b) a heavy chain CDR1 of SEQ ID NO: 4, a heavy chain CDR2 of SEQ ID NO: 2, a heavy chain CDR3 of SEQ ID NO: 3, a light chain CDR1 of SEQ ID NO: 14, a light chain CDR2 of SEQ ID NO: 15, and a light chain CDR3 of SEQ ID NO: 16;
      (c) a heavy chain CDR1 of SEQ ID NO: 5, a heavy chain CDR2 of SEQ ID NO: 6, a heavy chain CDR3 of SEQ ID NO: 3, a light chain CDR1 of SEQ ID NO: 17, a light chain CDR2 of SEQ ID NO: 18, and a light chain CDR3 of SEQ ID NO: 19; or
      (d) a heavy chain CDR1 of SEQ ID NO: 7, a heavy chain CDR2 of SEQ ID NO: 8, a heavy chain CDR3 of SEQ ID NO: 9, a light chain CDR1 of SEQ ID NO: 20, a light chain CDR2 of SEQ ID NO: 18, and a light chain CDR3 of SEQ ID NO: 16;
   y is 2; and
   wherein the cancer expresses PMEL17, contains a mutation of the GNAQ or GNA11 gene, or expresses PMEL17 and contains a mutation of GNAQ, GNA11, or both,
   thereby selecting the treatment for the subject having the cancer.
382. A method of selecting a subject for a treatment for a cancer, comprising determining the expression of one or more (*e.g.,* 2, 3, 4, 5, or 6) biomarkers selected from PMEL17, pERK, CD8, PD-L1, DUSP6, RASGRP3, or any combination thereof in a sample from the subject, wherein the treatment comprises administering to the subject an antibody drug conjugate (ADC) described herein, *e.g.,* at a dose described herein (*e.g.,* at a dose of 1 mg/kg to 16 mg/kg), *e.g.,* once every two weeks intravenously, optionally wherein the ADC has following structure: 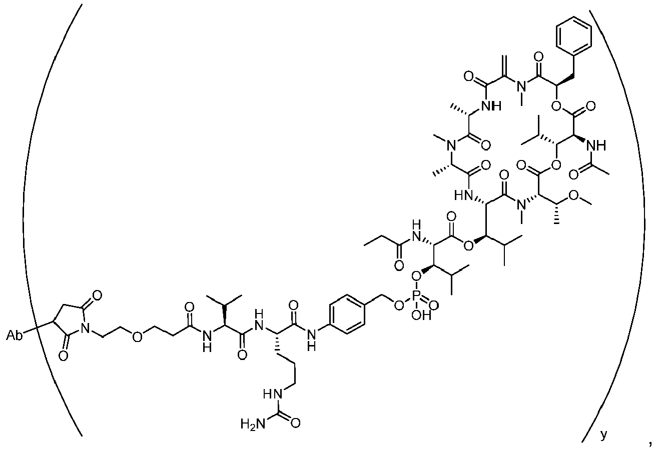 wherein:
   Ab is an antibody or antigen binding fragment thereof that binds to human PMEL17 protein and comprises:
      (a) a heavy chain CDR1 of SEQ ID NO: 1, a heavy chain CDR2 of SEQ ID NO: 2, a heavy chain CDR3 of SEQ ID NO: 3, a light chain CDR1 of SEQ ID NO: 14, a light chain CDR2 of SEQ ID NO: 15, and a light chain CDR3 of SEQ ID NO: 16;
      (b) a heavy chain CDR1 of SEQ ID NO: 4, a heavy chain CDR2 of SEQ ID NO: 2, a heavy chain CDR3 of SEQ ID NO: 3, a light chain CDR1 of SEQ ID NO: 14, a light chain CDR2 of SEQ ID NO: 15, and a light chain CDR3 of SEQ ID NO: 16;
      (c) a heavy chain CDR1 of SEQ ID NO: 5, a heavy chain CDR2 of SEQ ID NO: 6, a heavy chain CDR3 of SEQ ID NO: 3, a light chain CDR1 of SEQ ID NO: 17, a light chain CDR2 of SEQ ID NO: 18, and a light chain CDR3 of SEQ ID NO: 19; or
      (d) a heavy chain CDR1 of SEQ ID NO: 7, a heavy chain CDR2 of SEQ ID NO: 8, a heavy chain CDR3 of SEQ ID NO: 9, a light chain CDR1 of SEQ ID NO: 20, a light chain CDR2 of SEQ ID NO: 18, and a light chain CDR3 of SEQ ID NO: 16;
   y is 2; and
   wherein the cancer expresses PMEL17, contains a mutation of the GNAQ or GNA11 gene, or expresses PMEL17 and contains a mutation of GNAQ, GNA11, or both,
   thereby selecting the subject for the treatment of the cancer.

The following examples are provided to further illustrate some embodiments of the present disclosure, but are not intended to limit the scope of the disclosure; it will be understood by their exemplary nature that other procedures, methodologies, or techniques known to those skilled in the art may alternatively be used.

### EXAMPLES

### Example 1: Genetic Engineering of Chromobacterium vaccinii and Fermentative Production and Isolation of Compound (A1)

In order to identify alternative and scalable methods for production of compound (A1), a genome mining effort on bacterial genomes from genome sequence databases and genome sequences generated from sequencing efforts was initiated. Translated amino acid sequences of the compound (A1)-BGC from *Candidatus B. crenata* were used as query sequence. A cluster with very high homology in translated amino acid sequence and identical prediction of protein functions was discovered in the genome of *Chromobacterium vaccinii* DSM 25150.

### Method of production of compound (A1)

### Chromobacterium vaccinii DSM 25150 - a bacterial producer of compound (A1)

### Origin of the strain

The bacterial strain *Chromobacterium vaccinii* DSM 25150 (= ATCC BAA-2314, = NRRL B-50840, = MWU205), sometimes referred to as *C. vaccinii,* was obtained from the German collection of microorganisms and cell cultures DSMZ.

### Morphological characterization

*Chromobacterium* is a genus of Gram-negative rod-shaped bacteria. *C. vaccinii* grows readily on LB agar, producing distinctive smooth low convex colonies with a dark violet metallic sheen, due to production of violacein, upon incubation at 28 °C for 2 days.

### Genome sequencing of C. vaccinii DSM 25150

Cells were grown overnight at 30 °C in 12 mL LB at 200 rpm. After centrifugation the supernatant was removed and the cell pellet was resuspended in 4.5 mL SET buffer (2.42 g/L Tris-(hydroxymethyl)-aminomethan, 9.31 g/L NaEDTA, 4.38 g/L NaCl). The sample was homogenized using a glass homogenizer and 5 mL phenol were added. Subsequently, the sample was shaken rigorously for 2 min and centrifuged for 30 min at 12000 x g at 4 °C. The supernatant was transferred to a new tube and the phenol extraction was repeated for a second time. Afterwards 5 mL chloroform were added and the sample was shaken rigorously for 1 min followed by centrifugation for 30 min at 12000-x g at 4 °C. The upper phase was transferred to a new tube. Following a second chloroform extraction step 5 µL/mL RNAase A were added and the sample was incubated at 37 °C for 90 min. Subsequently isopropanol corresponding to two times of the sample volume were added and smoothly mixed. With an inoculation loop the genomic DNA was carefully fished out of the isopropanol mixture and transferred to an Eppendorf tube containing 500 µL 70% ethanol. After centrifugation at 12000-x g for 10 min the supernatant was discarded and replaced by 500 µL 70% ethanol. Following a second wash step the supernatant was again discarded and the pellet was air-dried before it was dissolved in 50 µL of 10 mM Tris-HCl (pH 7.5).

Genome sequencing was performed on a Sequel system from PacBio using SMRT (Single Molecule, Real-time) Link Version: 4.0.0.190159. As binding kit Sequel^{™} Binding Kit 2.0 was applied and the: Sequel^{™} Sequencing Plate 2.0 was utilized as sequencing kit plate.

*De novo* assembly of the *C. vaccinii* genome was done using the hierarchical genome assembly process HGAP version 4 (Chin et al. (2013) Nat Methods;10(6):563-9). The assembly resulted in one closed contig with a genome size of 5091614 bp and a mean coverage of 434.0.

For the identification of open reading frames in the C. *vaccinii* genome the 3 gene prediction programs Critica (Badger et al. (1999) Mol Biol Evol 16(4):512-24), Glimmer (Kelley et al. (2012) Nucleic Acids Res 40(1)) and Prodigal (Hyatt et al. (2010) BMC Bioinformatics 11:119) were applied. Afterwards the genome was analyzed for secondary metabolite biosynthetic gene clusters (BGCs) using antiSMASH 5.0 (Blin et al. (2019) Nucleic acids Res. 2;47(W1):W81-W87).

### Identification of the compound (A1)-BGC in C. vaccinii DSM 25150 by genome mining

Translated amino acid sequences of the compound (A1)-BGC from *B. crenata* were used as query sequence in a genome mining effort on bacterial genomes from sequence databases and bacterial genomes from sequencing efforts performed with PacBio sequencing technology. A cluster with very high homology in translated amino acid sequence and identical prediction of protein functions was discovered in the sequenced genome of *C. vaccinii.* The sequence generated in this example demonstrates the advantage of PacBio sequencing concerning highly identical repetitive sequence stretches. The complete compound (A1)-BGC could be identified including the up- and downstream regions encoding an additional large NRPS and several transposable elements (GenBank accession number: BankIt2437961 BSeq#1 MW732719). The gene organization on transposable element containing BGCs of compound (A1) (*frsA-H*) and compound J (*dis1-4*) in *C. vaccinii* (76974 bp) is shown in **FIG. 1****.** The organization of genome region containing compound (A1)-BGC and NRPS A is further shown in **Table 3.**

**Table 3. Organization of genome region containing compound (A1)-BGC and compound J-BGC**

| **Gene** | **Size [aa]** | **Predicted function** | **Best match: protein/organism** | **Acc. No. (identity/similarity % [aa])** |
|---|---|---|---|---|
| *orf1* | 143 | transposase | IS3 family transposase (*Chromobacterium vaccinii*) | WP_082113854.1 (99/100) |
| *orf2* | 386 | transposase | transposase (*Chromobacterium violaceum*) | OQS41431.1 (97/97) |
| *orf3* | 99 | transposase | multispecies transposase (*Chromobacterium*) | WP_046158739.1 (97/100) |
| *frsA* | 1273 | NRPS (C-A_{Hle}-T-TE) | | |
| *frsB* | 73 | MbtH | MbtH-like protein (*Chromobacterium vaccinii*) | QPI18724.1 (100/100) |
| *frsC* | 329 | malate dehydrogenase | malate dehydrogenase (*Chromobacterium vaccinii*) | WP_082113907.1 (100/100) |
| *frsD* | 1039 | NRPS (C-A_{Hle}-T) | | |
| *frsE* | 3017 | NRPS (C-A_{Pla}-T-E-C-A_{Dha}-MT-T) | | |
| *frsf* | 2519 | NRPS (C-A_{Ala}-T-C-A_{Ala}-MT-T) | | |
| *frsG* | 3136 | NRPS (C-A_{Hle}-T-C-A_{Thr}-MT-MT-T-TE) | | |
| *frsH* | 521 | Hydrolase | MBL fold metallo-hydrolase (*Chromobacterium vaccinii*) | WP_046159154.1 (100/100) |
| *dis1* | 3593 | NRPS (C-A_{Ser}-T-C-A_{Val}-T-C-A_{His}-T-C) | | |
| *dis2* | 2125 | NRPS (A_{Val}-T-C-A_{His}-T-C) | | |
| *dis3* | 2125 | NRPS (A_{Val}-T-C-A_{His}-T-C) | | |
| *dis4* | 3305 | NRPS (AVal-T-C-ALeu-T-C-AVal-T-Te-Te) | | |
| *orfA* | 318 | transposase | IS5 family transposase (*Chromobacterium vaccinii*) | WP_115617200.1 (98/99) |
| *orfB* | 121 | transposase | transposase partial (*Chromobacterium haemolyticum*) | OQS38342.1 (86/92) |
| *orfC* | 47 | transposase | transposase partial (*Chromobacterium haemolyticum*) | WP_1411111082.1 (87/91) |
| *orfD* | 178 | transposase | IS3 family transposase (*Chromobacterium subtsugae*) | WP_081050422.1 (99/99) |
| *orfE* | 99 | transposase | multispecies transposase (*Chromobacterium*) | WP_046158739.1 (97/97) |

### Culture conditions

The ratio of total volume of the shake flasks to volume of culture medium used was between 4 to 5. A -80 °C ampoule of *C. vaccinii* was thawed and used to inoculate a preculture in 25 mL LB (**Table 4**) medium. Preculture was cultivated for 12-16 h at 28 °C on an orbital shaker with 50 mm amplitude at 200 rpm. The main culture in 25 mL LB medium was inoculated with 1% of the preculture and incubated for 72 h at 24 °C on an orbital shaker with 50 mm amplitude at 200 rpm. All cultures were performed in triplicates. Upon harvest the cultures broths were frozen and kept at -20 °C for at least 24 h.

**Table 4. LB-Medium**

| **Substance** | **Provider** | **Order #** | **Concentration [g/L]** |
|---|---|---|---|
| Peptone | Merck | 71280 | 10 |
| Yeast extract | Merck | 71279 | 5 |
| Sodium chloride | Merck | 106400 | 5 |
| MOPS | Sigma | M-1254 | 10.5 |
| pH adjusted to 7.0 (HCl/NaOH) | | | |
| Sterilization 20 min at 121 °C | | | |

**Table 5. LB-Agar**

| **Substance** | **Provider** | **Order #** | **Concentration [g/L]** |
|---|---|---|---|
| Peptone | Merck | 71280 | 10 |
| Yeast extract | Merck | 71279 | 5 |
| Sodium chloride | Merck | 106400 | 5 |
| Agar | Merck | 101615 | 15 |
| pH adjusted to 7.3 (HCl/NaOH) | | | |
| Sterilization 20 min at 121 °C | | | |

### Analytical methods

### Sample preparation for HPLC-UV-MS analysis

5 mL freeze-thawed culture broth was transferred to a 15 mL conical centrifuge tube and mixed thoroughly with 5 mL acetonitrile. 2 g of magnesium sulfate and 0.5 g of sodium acetate were added to the mixture and shaken vigorously. The mixture was centrifuged at 4.000 rpm for 10 min to obtain phase separation. 4 mL of the organic layer were transferred to a test tube and the solvent was removed under vacuum. The resulting residue was dissolved in 400 µL of DMSO and shaken at room temperature for 10 minutes. After centrifugation at 4.000 rpm for 10 min an aliquot of the solution was transferred to a HPLC vial. Thereby the sample is ten-fold concentrated.

### Sample analysis

Samples were analyzed using a BEH C18 column (1.7 µM, 2.1x100 mm column + 2.1x10 mm pre-column; Waters AG). The flow rate was 0.9 mL/min. The column oven was set to 60 °C. The solvent composition and gradient are provided in **Table 6.** Absorption at wavelength of 220 nm was used as main readout. MS- and CAD-signals were recorded simultaneously.

Quantitation of A1 was performed by UV, using a calibration curve (based on UV-peak area at 220 nm) generated with external standards (10, 50, 100, 200, 400 µg/mL compound (A1)). The injection volume for both samples and standards was 1 µL.

**Table 6. Gradient for HPLC-UV-MS analysis**

| | | |
|---|---|---|
| Solvent A: | water + 0.02% formic acid | |
| Solvent B: | acetonitrile + 0.014% formic acid | |
| Flow rate: | 0.9 mL/min | |

| Time [min]: | % A | % B |
|---|---|---|
| 0 | 95 | 5 |
| 0.15 | 95 | 5 |
| 6.23 | 0 | 100 |
| 8.40 | 0 | 100 |
| 8.50 | 95 | 5 |
| 10.0 | 95 | 5 |

### Generation of C. vaccinii mutants by genomic integrations

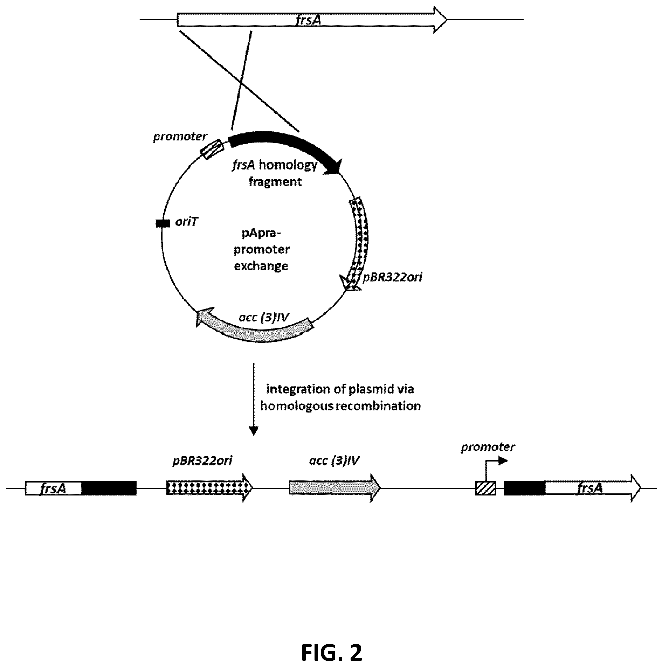

The following section describes the generation of mutants of *C. vaccinii* generated by stable genomic integrations. Exemplary *C. vaccinii* mutants are shown in **Table 7.** Exemplary promoter sequences are shown in **Table 8.** The scheme of the insertion strategy used for promoter exchanges is shown in **FIG. 2****.**

**Table 7. C. vaccinii mutants**

| **Mutant name** | **Construct name** | **Comment** |
|---|---|---|
| *C. vaccinii vioP* | pApra*-vioP-frsA* | exchange of native A1 promoter against *vioP* promoter |

| | | |
|---|---|---|
| *C. vaccinii rbs* | pApra*-rbs-frsA* | exchange of native A1 promoter against *rbs* promoter |
| *C.vaccinii nptII* | pApra- *nptII -frsA* | exchange of native A1 promoter against *nptII* promoter |
| *C. vaccinii vioB* KO | pApra_vioB_KO | disruption of the violacein cluster |
| *C.vaccinii dis* KO | pApra_KO_dis | disruption of the compound J cluster |
| *C.vaccinii vioB* KO + *dis* KO | pPhoto_vioB_KO and pApra_KOdis | disruption of the violacein and compound J cluster |

**Table 8. Sequences of exemplary promoters**

| **Promoter name** | **Promoter sequence (5'- 3')** |
|---|---|
| *vioP* (from violacein BGC of C. *vaccinii)* | CGTCGTTGATCCCAGGCAGCCCTTTGTCTCCGTTTCTCCACGTCATGCCCTGACC CTTGGAACAGGATGGGCCGTCCTGTCAGACAATATGCTTGATGAATTAGTTCGGT TGAACTAACGTGGCTGAAAATTACAAAGGCATTTAATTTTAAAAATATAAATGCC TTATAAATTTCATGCCGGGAAACCGGTCATGACGAGGATACAAGAGGCTGCATCC CGATTTCGAGGCGAGAGTGCCAAGCATTTACGTCGTCCATGCCCGTTCGTTGTTG CCGCGCGGCGGGCGTGAATTGAACAGTCAAAGGGACATTCGCG (SEQ ID NO: 264) |
| *nptII* (MT521917) | TGGACAGCAAGCGAACCGGAATTGCCAGCTGGGGCGCCCTCTGGTAAGGTTGGGA AGCCCTGCAAAGTAAACTGGATGGCTTTCTTGCCGCCAAGGATCTGATGGCGCAG GGGATCAAGATCTGATCAAGAGACAGGATAAGGAGGTACAGATTCAT (SEQ ID NO: 265) |
| *rbs* (ribosomal promoter of *C. vaccinii)* | CACCGGCCTGACGGCCAAGTGTTGAAAAAACGCTGCTCCATCAACGGTTAACGTT GGCGGGGCGGCGTTTTTATTTGACGCTGGGCCGTTGACTAAAATATAATCCTCC GTCCTTTCTGGAGGCGTGTTGTCTTCGGGAAGAATCAACTAGGAACTGATAGTA (SEQ ID NO: 266) |
| *J23119* (synthetic promoter) | TTGACAGCTAGCTCAGTCCTAGGTATAATGCTAGC (SEQ ID NO: 316) |
| *pLpp* (synthetic promoter) | ATCAAAAAAATATTCTCAACATAAAAAACTTTGTGTAATACTTGTAACG (SEQ ID NO: 268) |
| *PS12burk* (ribosomal promoter from *Burkholderia thailandensis*) | GAGCTGTTGACTCGCTTGGGATTTTCGGAATATCATGCCGGGTGGCCCGGGAAAGCCAC GTTGTGTCTCAAAATCTCTGATGTTACATTGCACAAGATAAAAATATATCATCATGAACA ATAAAACTGTCTGCTTACATAAACAGTAATACAAGGGGTGTT (SEQ ID NO: 270) |

### Generation of genetic constructs

Synthetic DNA, containing either the violacein, *rbs* or *nptII* promoter and the first 1200 bp of the A1 biosynthetic gene *frsA,* was ordered at Genewiz and delivered cloned in the pUC57 backbone. All plasmid pUC57 promoter plasmids were transformed into *E*. *coli* XL1 Blue and plated onto LB agar (**Table 5**) containing 50 µg/mL kanamycin. The plates were incubated overnight at 37 °C and on the next day, a loop was inoculated in 50 mL LB medium (**Table 4**) containing 50 µg/mL kanamycin and incubated overnight at 37 °C and 200 rpm. Midi-prep of all three pUC57 promoter plasmids were performed using the Qiagen Plasmid Plus midi kit. pUC57 promoter plasmids were digested with *NotI* and *Xba*I to cut out the respective promoter-*frsA* fragment. Subsequently, the purified respective promoter-*frsA* fragment (NucleoSpin gel and PCR clean up kit from Macherey Nagel) was cloned into the *NotI* and *XbaI* predigested pApra plasmid, an internal conjugation vector, using the Rapid DNA Ligation kit from Roche. The ligation mix was transformed into *E. coli* XL1 Blue cells and plated on LB agar containing 100 µg/mL apramycin. Plates were incubated overnight at 37 °C until single clones appeared. Single clones were cultivated overnight at 37 °C in LB medium containing 100 µg/mL apramycin. On the next day plasmids were isolated using the NucleoSpin plasmid Easy Pure kit (Macherey Nagel) and pApra-promoter-*frsA* constructs were verified by sequencing or restriction digest. For promoter constructs containing *J23119, Plpp, PS12burk, or ErmE** promoters, the promoter sequence as well as the first 1200 bp of *frsA* were generated synthetically either by Genewiz or Genscript and directly inserted into the pApra backbone by the respective synthesis company. For inactivation of the *vioB* gene in the violacein-BGC a 1004 bp long synthetic insert containing an artificial stop-codon was ordered and cloned by Genewiz into plasmid pApra. A midi-prep of papra_*vioB*_KO was generated using the Qiagen Plasmid Plus midi kit. pPhoto_*vioB*_KO was generated by digestion of pApra_*vioB*_KO with *Xba*I and *Nde*I followed by a ligation of the purified insert into the *Xba*I and *Nde*I precut pPhoto backbone (internal vector backbone). The ligation mix was transformed into *E. coli* XL1 blue cells and plated on LB agar containing 50 µg/mL chloramphenicol. Plates were incubated overnight at 37 °C until single clones appeared. Single clones were cultivated overnight at 37 °C in LB medium containing 50 µg/mL chloramphenicol. The next day plasmids were isolated using the NucleoSpin plasmid Easy Pure kit (Macherey Nagel) and verified by control digestion with *Xba*I and *Nde*I*.* For disruption of the compound J-BGC a 1053 bp long synthetic insert with an artificial stop codon was ordered, and cloned by Genewiz into plasmid pUC57. The plasmid pUC57-KO_dis was transformed into *E. coli* XL1 Blue and plated onto LB agar containing 50 µg/mL kanamycin. The plates were incubated overnight at 37 °C and on the next day, a loop was inoculated in 50 mL LB medium containing 50 µg/mL kanamycin and incubated overnight at 37 °C and 200 rpm. A midi-prep of pUC57-KO_dis was generated using the Qiagen Plasmid Plus midi kit. pUC57-KO_dis was digested with *Xba*I and *EcoR*V to obtain the KO_dis fragment. Subsequently, the purified KO_dis fragment (NucleoSpin gel and PCR clean up kit from Macherey Nagel) was cloned into the *EcoR*V and *Xba*I predigested pApra plasmid using the Rapid DNA Ligation kit from Roche. The ligation mix was transformed into *E. coli* XL1 blue cells and plated on LB agar containing 100 µg/mL apramycin. Plates were incubated overnight at 37 °C until single clones appeared. Single clones were cultivated overnight at 37 °C in LB medium containing 100 µg/mL apramycin. Verification of the correct clones was done by restriction digest using the *Xba*I and *EcoR*V*.*

### Preparation of electro competent E. coli ST18

A loop of *E. coli* ST18 was used to inoculate 10 mL of LB medium supplemented with 50 µg/mL 5-aminolevulinic acid (supplement absolutely required for growth of auxotroph mutant) and incubation of the culture was done overnight at 37 °C 200 rpm. The next day 2 mL of the overnight culture were used to inoculate 250 mL of LB medium supplemented with 50 µg/mL 5-aminolevulinic acid and incubated at 37 °C 200 rpm to an OD₆₀₀ of 0.4-0.6. The culture was rapidly transferred to an ice-water bath for 15-30 min and swirled occasionally to ensure steady cooling. The culture was then transferred to ice-cold 50 mL tubes and cells were centrifuged for 15 min at 2500 g and 4 °C and resuspended in 50 mL of ice-cold sterile bidest water. Wash step was repeated for a second time before each pellet was resuspended in 25 mL of ice-cold sterile bidest water. Wash step was repeated for a third time before each pellet was resuspended in 10 mL of ice-cold sterile 10% glycerol and the tubes were pooled. Cells were centrifuged for 15 min at 2500 g and 4 °C and resuspended in 500 µL of ice-cold sterile 10% glycerol by gently swirling and aliquots of 50 µL were prepared and stored at -80 °C.

### Conjugation of C. vaccinii with E. coli ST18

Electro competent *E*. *coli* ST18 were transformed with pApra*-vioP-frsA*, pApra-*nptII-frsA,* pApra*-rbs-frsA,* pApra-*vioB*-KO and pApra-*dis*-KO. Electroporation conditions: 1.5 kV, 200 Ω and 25 µF in 0.1 cm electroporation cuvettes. Recovery was done in LB medium containing 50 µg/mL 5-aminolevulinic acid for 1 h at 37 °C. Afterwards cells were plated on LB agar containing 50 µg/mL 5-aminolevulinic acid and 100 µg/mL apramycin and incubated at 37 °C overnight. A loop of E. coli ST18 containing the corresponding construct was used to inoculate 2 mL LB medium supplemented with 50 µg/mL 5-aminolevulinic acid and 100 µg/mL apramycin. Incubation was done at 37 °C at 200 rpm overnight. In parallel 2 mL LB medium were inoculated with *C. vaccinii* and incubated overnight at 28 °C and 200 rpm. The next day 25 mL of LB medium with 5-aminolevulinic acid and 100 µg/mL apramycin were inoculated with 500 µL of the *E*. *coli* overnight culture and cultivated at 37 °C and 200 rpm to an OD₆₀₀ of 0.6-0.8. At the same time, 25 mL of LB medium were inoculated with 500 µL of the *C. vaccinii* overnight culture and cultivated at 28 °C and 200 rpm to an OD₆₀₀ of 0.6-0.8. Cells were centrifuged for 10 min at 1000 g and resuspended in 10 mL LB medium. Wash step was repeated for a second time before each pellet was resuspended in 1 mL LB medium. *E. coli* and *C. vaccinii* were mixed in a ratio of 1:3 and spotted on a LB agar plate containing 50 µg/mL 5-aminolevulinic acid. After an initial incubation at 37 °C degrees for 2 h, the plate was transferred to 28 °C and incubated overnight. On the next day, the spot was resuspended in 1 mL LB medium. A dilution of 1:100 was made and 200 µL of diluted as well as undiluted sample were spread on LB agar plates containing 100 µg/mL apramycin. Plates were incubated at 28 °C until single clones appeared (1-2 days).

### Genotypic and phenotypic verification of exconjugants

Single exconjugants were restreaked on LB agar plates supplemented with 100 µg/mL apramycin and incubated overnight at 28 °C. Correct integration of the respective promoter constructs was verified by PCR. For this purpose, a little amount of cell mass was transferred in 10 µl of water. The sample was boiled for 10 min at 95 °C and 2 µL were applied in a PCR Hot Start Master Mix reaction with the corresponding verification primers (**Table 9**). Correct exconjugants showed a band in the appropriate size, while wild type and false-positive clones showed no PCR product. For example, Correct exconjugants showed a band at 1638 bp for *vioP* integration and 1482 bp for *nptII* integration with verification primers vioPcontfwd or *nptII*KOntfwd and PromoKOntrev. Integration of the *rbs* promoter was verified by amplification of the apramycin resistance cassette using the primers Aprafwd and Aprarev. Knock out of *vioB* as well as disruption of compound J-BGC was also verified by PCR. For correct *vioB* KO mutants PCR bands with 1287 bp for the primers vioBKOfwd and pApraKOntrev and 1126 bp for the primers pApraKOntfwd and vioBKOrev were obtained. For correct *dis* KO mutants primer Aprafwd and Aprarev resulted in PCR products of 560 bp. Exconjugants showing the correct genotype were inoculated in 3 mL LB medium containing 100 µg/mL apramycin and cultivation was done overnight at 28 °C and 200 rpm. For preparation of cyrovials 1 mL of overnight culture was mixed with 0.5 mL 60% glycerol and stored at -80 °C. For phenotypic control 25 mL of LB were inoculated with 1% of the overnight culture and cultivated for 48h at 200 rpm and 24 °C. Upon harvest, the culture broth was extracted in a 1:1 ratio with methanol. After shaking for 30 min at 1000 rpm, the sample was centrifuged for 2 min at 11000g and 150 µL of the supernatant were analysis by HPLC-MS to analyze the titers of compound (A1).

### Generation of double mutant vio B KO + dis KO

In a first step pPhoto_vioB_KO was conjugated into *C. vaccinii* as described above and selected on LB agar containing 50 µg/mL chloramphenicol. Correct exconjugants were verified by PCR using the primer vioBKOfwd and pPhoto_KOntrev resulting in a 1351 bp PCR product. *C. vaccinii vioB* KO was used to inoculate 2 mL LB medium with 50 µg/mL chloramphenicol and incubated overnight at 28 °C and 200 rpm. In parallel a loop of *E. coli* ST18 containing pApra_KO_dis was used to inoculate 2 mL LB medium supplemented with 50 µg/mL 5-aminolevulinic acid and 100 µg/mL apramycin. The next day 25 mL of LB medium with 5-aminolevulinic acid and 100 µg/mL apramycin were inoculated with 500 µL of the *E*. *coli* overnight culture and cultivated at 37 °C and 200 rpm to an OD₆₀₀ of 0.6-0.8. At the same time 25 mL of LB medium with 50 µg/mL chloramphenicol were inoculated with 500 µL of the *C. vaccinii* overnight culture and cultivated at 28 °C and 200 rpm to an OD₆₀₀ of 0.6-0.8. Cells were centrifuged for 10 min at 1000 g and resuspended in 10 mL LB medium. Wash step was repeated for a second time before each pellet was resuspended in 1 mL LB medium. *E. coli* and *C. vaccinii* were mixed in a ratio of 1:3 and spotted on a LB agar plate containing 50 µg/mL 5-aminolevulinic acid. After an initial incubation at 37 °C degrees for 2h, the plate was transferred to 28 °C and incubated overnight. On the next day, the spot was resuspended in 1 mL LB medium. A dilution of 1:100 was made and 200 µL of diluted as well as undiluted sample were spread on LB agar plates containing 100 µg/mL apramycin and 50 µg/mL chloramphenicol. Plates were incubated at 28 °C until single clones appeared (1-2 days). Double mutants were verified by PCR using the primers pAprafwd and pAprarev as described above.

The primers used for mutant verification are shown in **Table 9.** The sequence of homologous frsA fragment for promoter exchange is shown in **Table 10.**

**Table 9. Primers used for mutant verification**

| **Primer name** | **Primer sequence** | **Comment** |
|---|---|---|
| vioPcontfwd | 5'-CTCCGTTTCTCCACGTCATGCC-3' (SEQ ID NO: 300) | Verification primer *vioP* insertion (forward) |
| PromoKOntrev | 5'-GTTCCAGTCGACCAGCAGTTGC-3' (SEQ ID NO: 301) | Verification primer for all insertions (reverse) |
| pApraKOntrev | 5'-GTATGTTGTGTGGAATTGTGAGC-3' (SEQ ID NO: 302) | Verification primer *vioB* knock out (reverse) |
| vioBKOfwd | 5'-CCACTACAACGACTACTTGC-3' (SEQ ID NO: 303) | Verification primer *vioB* knock out (forward) |
| nptIIKOnt fwd | 5'-GACAGCAAGCGAACCGGAATTGC-3' (SEQ ID NO: 304) | Verification primer *nptII* promoter insertion (forward) |
| pApraKOntfwd | 5'-GATCCGTCGACCTGCAGG-3' (SEQ ID NO: 305) | Verification primer *vioB* knock out (forward) |
| vioBKOrev | 5'-CTGAACACCTTGTCCGACATGAACG-3' (SEQ ID NO: 306) | Verification primer *vioB* knock out (reverse) |
| Aprafwd | 5'-CATGACCGACTGGACCTTCC-3' (SEQ ID NO: 307) | Verification primer compound J knock out (forward) |
| Aprarev | 5'-CCACAGCTCCTTCCGTAGC-3' (SEQ ID NO: 308) | Verification primer compound J knock out (reverse) |
| pPhotoKOntrev | 5'-CAGCAACTTAAATAGCCTCTAAGG-3' (SEQ ID NO: 309) | Verification primer *vioB* knock out (reverse) |
| rbs_fwd | 5'-GCTGCTCCATCAACGGTTAACG-3' (SEQ ID NO: 310) | Verification primer *rbs* insertion (forward) |
| J23119fwd | 5'-CTTGACAGCTAGCTCAGTCCTAGG-3' (SEQ ID NO: 311) | Verification primer *J23119* insertion (forward) |
| S12burkfwd | 5' -TCGGAATATCATGCCGGGTGG-3' (SEQ ID NO: 312) | Verification primer *PS12burk* insertion (forward) |
| Plpp_fwd | 5'-CTGGAGGCGTGTTGTCTTCGG-3' (SEQ ID NO: 313) | Verification primer *plpp* insertion (forward) |
| ErmE_fwd | 5'-CGATGCTAGTCGCGGTTGATCG-3' (SEQ ID NO: 314) | Verification primer *ErmE** insertion (forward) |

**Table 10. Sequence of homologous frsA fragment for promoter exchange**

| |
|---|
| ATGAAAAACAGTGAATCGCCAATCCATCATTTTCAGGCATCTTCAGCACAGCTGGATGTATGGATTTC TCAGGAAGTTTCACCGAATCTGCCCAACAATATTGCCGAGTATCTGAATCTCGCCGGCTCGTTGGATG CTGGATTGTTTCTGCAGGCTTTAAGCCAGGTCGCCAGTGAGAGCGCGGAGCTGCAATACAACTTCCGT CACGATGGTCTCCAGTTGACCAAGTTTCGTCGAGATGATGAAGGCTGGGAGCCGGACTTCATCGATGT ATCGACGCACGGCGAGCCGGAACACGCAGCCCTGCGCGCCATGCGGGAGCGGGTGGAGAAACCCTTCG ATCTGGCGCGGGACGCGTTGTTTCGCTGGACCTTGATCCGCCTGGCCGACGAGCGCCACATCTTCTGC CATGTGTATCACCACATCGCGATGGATGGGGCCGGCTATGTGATGCTGCTGCAGCGCATAGCCGAGGT TTACGGCGCGCTGCGGAAGGCCAGCCGGCACCGGCCTGCGGTTTCGCCGATGCGGATGCCATCGTCC GCGAGGAAGAGCGCTACCGCCAGTCGGAGCAGTTCGCGGTCGACCGGGCATTCTGGCAAGCGCGCTCG GCCGAGCTGGCGACGGCGGAGCCGCCGCTGCCGGCGGCCGATGGCCCGTTCCTGGCGTTCGCCCAGAC GGCGGTGATTCCGGAAGACGCCTGCGGCGGGCTGCGGATGACGGCCGAGCGGCTGGGCGTCTCCCAGT CCCGTTTGCTGACAGCAGCCATCGTCGCTTATTTCCATCGCTGGGGCGGCCAGCAAGAGATCTTGTTC CGGCTGGCGGTATCGGCGCGCAGCGATGCGACGCGACACGCGCCCGGCCACCTGGCGCATGCGTTGCC GCTGCTGGCCAGCCTGCCGCCGCGCGCCAGTCTGGCCGACATCGCGCGACAGCTGGACGGCGAGGTGG AGCGGATGCGTCCGCATACCCGCTATCGGGCTGAGGACATCGTGCGCGACCAGGCCGGTGCCGGTTTG GGGCGCGGGGCGCAGGGGCCTGTGATCAACCTCATGCCTTTTGCTTACCGCTTCGAGTTTGGCGCCTG TCGCGTGGAGTCCGCCCATCAGCTGACCGTCGGCGTGCTGGACACGCTGGAAGTGGCGGTGCACGACC GCAAGAACGGTGACGGCCTCCACCTCGATTTGTACGCATCCGAG (SEQ ID NO: 317) |

### Results

### Verification of production of A1 by C. vaccinii

Cultivation of *C. vaccinii,* followed by extraction and HPLC-HRMS analysis confirmed the production of compound (A1). Compound (A1) was isolated from the culture broth and characterized thoroughly by 1D- and 2D-NMR experiments. All signals and observed correlations are in very good accordance with those reported in the literature (Reher et al. (2018) J Nat Prod.; 81(7):1628-1635) as well as to reference material that was previously isolated from the leaves of *Ardisia crenata.*

### Promoter insertion mutants

In order to increase the titer of compound (A1), the native promoter upstream of the compound (A1)-BGC was replaced by different promoters. This strategy was promising as the cluster consists of a single operon and its transcription is likely driven by a single promoter. Several different promoters *(ErmE*, J23119, nptII, plpp, P_{S12}burk, rbs,* and *vioP*) were investigated in this context. These are all constitutive promoters, with the exception of *vioP.* The ribosomal promoter rbs (sequence extracted from the genome sequence of *C. vaccinii*), and the promoter *nptII* (from the neomycin resistance cassette) are constitutive promoters. Promoter *vioP* was extracted from the violacein-BGC of *C. vaccinii.* This promoter is regulated via N-acyl homoserine lactones (N-AHLs) involved in quorum sensing as described by Morohoshi et al. (2010) Bioscie. Biotechnol. Biochem.;74 (10),2116-2119). The influence of the promoter insertions on the titer of compound (A1) was analyzed by cultivation of genetically verified mutants in triplicates.

While the A1 titers of the *rbs, nptII,* and *P_{S12}burk* mutants were below those of the wild type, the insertion of the *vioP, ErmE*, plpp,* and *J23119* promoters respectively, led to a more than fourfold increase in titer of compound (A1) (**Table 11**).

**Table 11. Average titer and fold-change of A1**

| **Promoter** | **Average Titer A1 [mg/L]** | **A1 fold change** |
|---|---|---|
| Wild type (control) | 8.1 | - |
| *vioP* | 35.4 | 4.4 |
| *nptII* | 6.1 | 0.8 |
| *rbs* | 3.3 | 0.4 |
| *ErmE** | 68.2 | 8.4 |
| *plpp* | 66.8 | 8.2 |
| *J23119* | 42.7 | 5.3 |
| *P_{S12}burk* | <1 mg/L | Not applicable |

### Violacein disruption mutant

In addition to compound (A1) and its analogs, *C. vaccinii* produces violacein and desoxyviolacein, purple pigments of bis-indole structure, that are common to most *Chromobacteria* and even accountable for the naming of this genus. Its biosynthesis has been elucidated and the respective BGC has been published by August et al. (2000) J Mol Microbiol Biotechnol.; 2(4):513-519). The high amounts of violacein produced by *C. vaccinii* were troublesome during the extraction and purification process due to the formation of poorly soluble residues at every evaporation. In order to ease the purification of compound (A1), a knockout of the gene *vioB* was performed.

The resulting mutant was devoid of violacein production, evident by the lack of the purple color. The titer of compound (A1) and its isolated yield were comparable to that of the wild type strain.

### Compound J disruption mutant

In addition the violacein, a class of novel cyclic lipodepsipeptides was discovered in the culture extracts of *C. vaccinii.* Isolation and structure elucidation (see below) revealed two cyclic lipodepsipeptides as well as two linear lipodepsipeptides, all composed of six amino acids of the sequence *N*-Acyl-¹Ser-²Val-³His-⁴Val-⁵Leu-⁶Val (SEQ ID NO: 318). The cyclic compounds feature a hexapeptide lactone ring formed between the ¹Ser hydroxyl group and the C-terminal carboxyl group of ⁶Val.

These compounds were causing problems during the purification process of compound (A1). Upon preparation of concentrated fractions for purification of compound (A1) by preparative HPLC, solutions of very high viscosity (almost gel-like) were obtained due to the presence of these lipopeptides. Thereby sample injection was hampered and further dilution was required. This limited the amounts of compound (A1) to be processed per run.

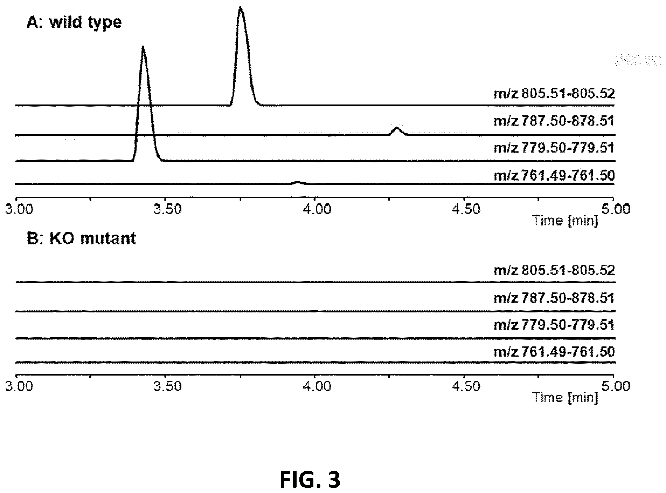

An inactivation mutant of the *dis*-BGC responsible for formation of compound J was generated. This mutant was shown to be devoid of the production of all members of this class (**FIG. 3**).

This mutant yielded a compound (A1) titer comparable to *C. vaccinii* wild type. Purification of compound (A1) from cultivation extracts of this mutant exhibited an improved isolated yield compared to extracts of the wild type strain (68% vs 40%).

### Double mutant

A double mutant of both the compound J and the violacein-BGC was generated to even further reduce the complexity of the chemical background. The production of both secondary metabolite classes was abolished in the double-mutant. The titer of compound (A1) was comparable to *C. vaccinii* wild type. The isolated yield of compound (A1) was comparable to that obtained for the compound J disruption mutant.

### Structure elucidation of compound J

Compound J is a cyclic lipodepsipeptide of the sequence *N*-(Z)-dec-3-enoyl -¹Ser-²Val-³His-⁴Val-⁵Leu-⁶Val (SEQ ID NO: 321). The hexapeptide lactone ring is formed between the ¹Ser hydroxyl group and the C-terminal carboxyl group of ⁶Val.

### HR-MS and HR-MS/MS

MS and MS/MS spectra have been recorded on a maXis 4G QTOF mass spectrometer from Bruker Daltonics in positive ionization mode. Fragmentation energies are provided at the top right of the spectrum. Compound J was purified as amorphous white powder. The HR-ESIMS data indicated a molecular formula of C₄₀H₆₆N₈O₈ with RDBE = 12.

### NMR

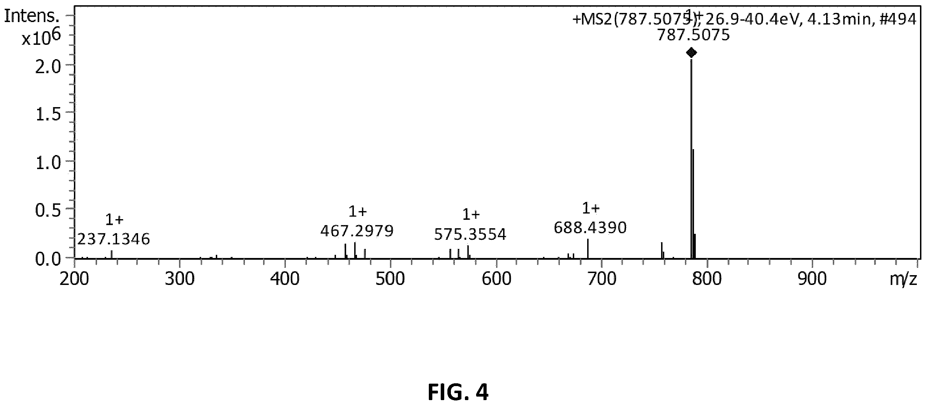

The ¹H- and ¹³C-NMR spectra of compound J have been recorded at 600 MHz in DMSO-d6. Analysis of the ¹H NMR spectrum, as well as the HSQC NMR spectrum of compound J indicated the existence of six amide hydrogens (δ_{H} 7.19, 7.82, 7.88, 7.96, 8.08, 8.43), two olefinic methine hydrogens (δ_{H} 5.47, 5.48), and two aromatic hydrogens (δ_{H} 6.85, 7.54). Furthermore, six α-amino methine hydrogen signals were detected at δ_{H} 3.9 - 4.5 (3.95, twice 4.17, 4.24, 4.33, 4.48), suggesting that compound J contained six amino acid units in its structure. Signals corresponding to seven carbonyl carbon atoms (δ_{C} 168.7, 170.6, 170.9, 171.0, 171.4, 171.6, 172.5) were observed in the ¹³C NMR spectrum, as well as five sp² carbon atoms (δ_{C} 118.0, 122.7, 129.2, 132.0, 134.9) which account combined for 10 out of the 12 double bond equivalents of compound J. This indicates that compound J is a bicyclic compound. Further, carbon signals corresponding to one oxygen-bound carbon atom (δ_{C} 62.6) and six carbon atoms at the α-position of the amino acids (δ_{C} 51.3, 52.3, 54.3, 57.2, 57.9, 58.3) were detected. All one-bond ¹H-¹³C correlations were assigned by interpretation of the HSQC NMR spectrum with the ¹H and ¹³C NMR spectroscopic data (**Table 12,** **FIG. 4**).

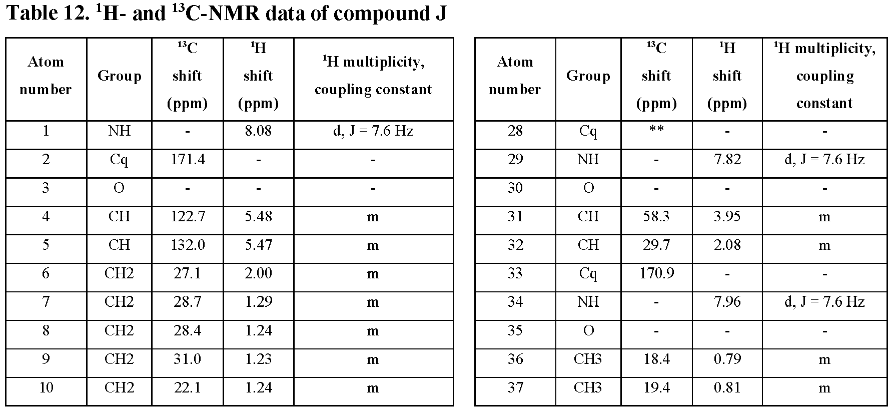

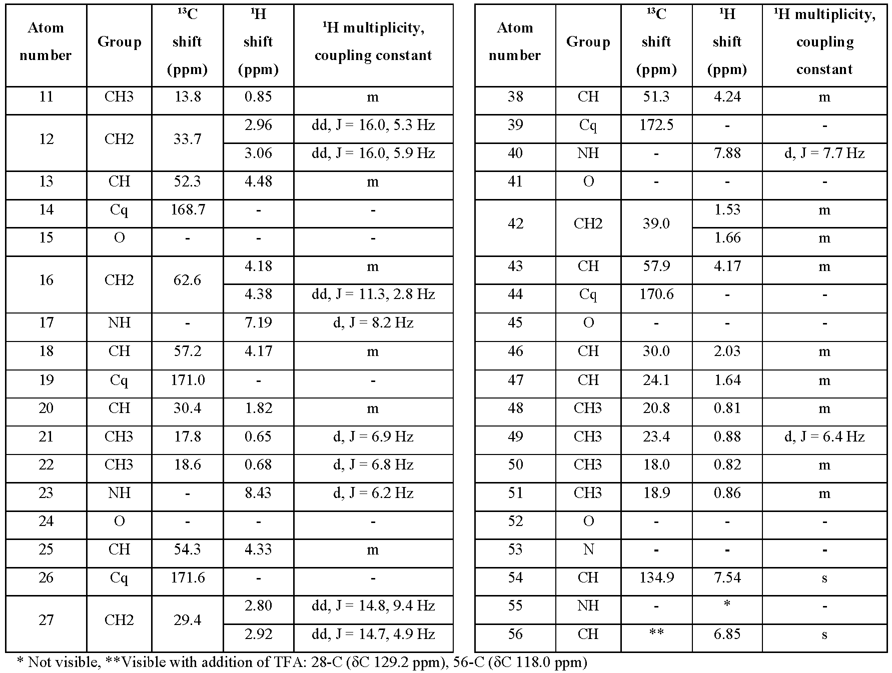

The chemical structure and atomic number of compound J is shown below.

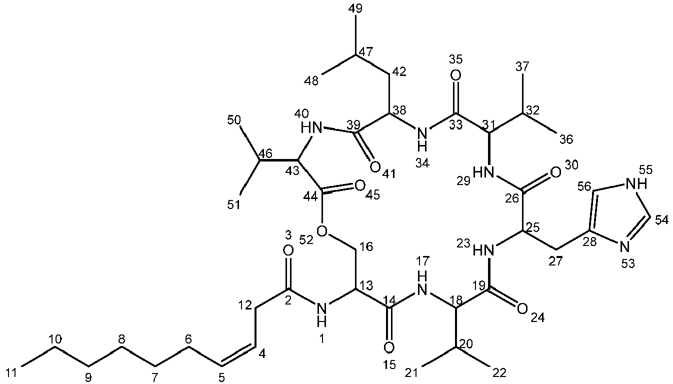

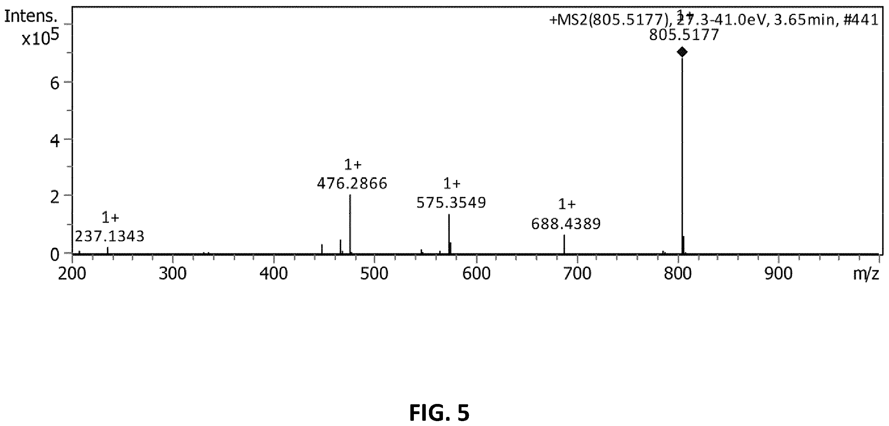

Combinational analysis of the COSY and HMBC NMR data (**FIG. 5**) revealed that compound J contained four common amino acids (histidine, leucine, serine, valine). Further, this identified the presence of a dec-3-enoyl unit. The carbon shift of 6-C (δ_{C} 27.1) in combination with an observed ROESY correlation between 12-CH₂ (δ_{H} 2.96, 3.06) and 6-CH₂ (δ_{H} 2.00) identified the double bond geometry as Z.

The connections between the amino acid units were subsequently elucidated through three-bond ¹H-¹³C couplings (**FIG. 5**). 18-CH (δ_{H} 4.17)/C-14 (δ_{C} 168.7), 25-CH (δ_{H} 4.33)/C-19 (δ_{C} 171.0), 31-CH (δ_{H} 3.95)/C-26 (δ_{C} 171.6), 38-CH (δ_{H} 4.24)/C-33 (δ_{C} 170.9), 43-CH (δ_{H} 4.17)/C-39 (δ_{C} 172.5). The macrocyclic structure was established by coupling of 16-CH₂ (δ_{H} 4.18, 4.38)/C-44 (δ_{C} 170.6). This sequence was further crosschecked and confirmed by ROESY correlations between the amide hydrogens and the α-hydrogens of the amino acid units.

The (Z)-dec-3-enoic acid unit was found to be connected to 1-NH by a HMBC correlation of 13-CH (δ_{H} 4.48) to C-2 (δ_{C} 171.4) and a ROESY correlation between 1-NH (δ_{H} 8.08) and 12-CH₂ (δ_{H} 2.96 and 3.06).

The key correlations of compound J is shown below.

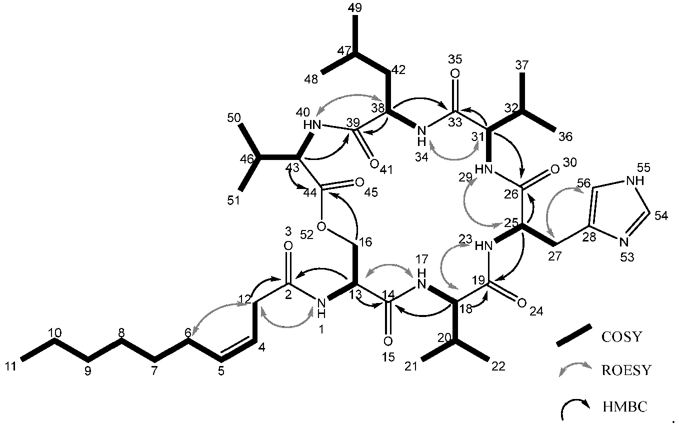

### Structure elucidation of compound F5

Compound F5 is a linear lipopeptide of the sequence *N*-(Z)-dec-3-enoyl-¹Ser-²Val-³His-⁴Val-⁵Leu-⁶Val (SEQ ID NO: 321). In comparison to compound J, the lactone between the ¹Ser hydroxyl group and the C-terminal carboxyl group of ⁶Val has been hydrolyzed.

### HR-MS and HR-MS/MS

MS and MS/MS spectra have been recorded on a maXis 4G QTOF mass spectrometer from Bruker Daltonics in positive ionization mode. Fragmentation energies are provided at the top right of the spectrum. Compound F5 was purified as amorphous white powder. The HR-ESIMS data indicated a molecular formula of C₄₀H₆₈N₈O₉ with RDBE = 11.

### NMR

The ¹H- and ¹³C-NMR spectra of compound F5 have been recorded at 600 MHz in DMSO-d6. The NMR data for compound F5 and compound J were similar (**Table 13**). The most significant differences were the missing correlation of 13-CH₂ to 41-C in HMBC (**FIG. 5**) and the difference in shift of 13-CH2 (δ_{H} 3.49 and 3.55) compared to 16-CH2 (δ_{H} 4.18 and 4.38) for compound J. These observations in combination with the observed unsaturation number of 11 indicated that compound F5 was a linear lipopeptide.

**Table 13. ¹H- and ¹³C-NMR data of compound F5**

| **Atom number** | **Group** | **¹³C shift (ppm)** | **¹H shift (ppm)** | **¹H multiplicity, coupling constant** |
|---|---|---|---|---|
| 1 | NH | | 7.90 | d, J = 7.6 Hz |
| 2 | Cq | 170.4 | - | - |
| 3 | O | - | - | - |
| 4 | CH | 123.4 | 5.45 | m |
| 5 | CH | 131.7 | 5.44 | m |
| 6 | CH2 | 26.9 | 1.99 | m |
| 7 | CH2 | 28.9 | 1.28 | m |
| 8 | CH2 | 28.4 | 1.24 | m |
| 9 | CH2 | 34.1 | 2.95 | m |
| 10 | CH | 55.1 | 4.36 | dd, J = 6.0, 5.9 Hz |
| 11 | Cq | 170.2 | - | - |
| 12 | O | - | - | - |
| 13 | CH2 | 61.9 | 3.49 | dd, J = 10.6, 5.7 Hz |
| | | | 3.55 | dd, J = 10.6, 5.6 Hz |
| 14 | NH | - | 7.83 | d, J = 8.7 Hz |
| 15 | CH | 57.7 | 4.13 | m |
| 16 | Cq | 170.8 | - | - |
| 17 | CH | 30.4 | 1.87 | m |
| 18 | CH3 | 19.0 | 0.67 | d, J = 6.9 Hz |
| 19 | CH3 | 17.7 | 0.69 | d, J = 6.9 Hz |
| 20 | NH | - | 8.23 | d, J = 8.0 Hz |
| 21 | O | - | - | - |
| 22 | CH | 52.7 | 453 | m |
| 23 | Cq | 171.0 | - | - |
| 24 | CH2 | 28.9 | 2.75 | dd, J = 14.7, 9.2 Hz |
| | | | 2.89 | dd, J = 14.8, 4.8 Hz |
| 25 | Cq | ** | - | - |
| 26 | NH | - | 7.75 | d, J = 8.4 Hz |
| 27 | O | - | - | - |
| 28 | CH | 58.1 | 4.14 | m |
| 29 | CH | 30.5 | 1.95 | m |
| 30 | Cq | 170.8 | - | - |
| 31 | NH | - | 8.41 | d, J = 8.4 Hz |
| 32 | O | - | - | - |
| 33 | CH3 | 18.0 | 0.79 | m |
| 34 | CH3 | 19.1 | 0.79 | m |
| 35 | CH | 50.8 | 4.45 | m |
| 36 | Cq | 172.0 | - | - |
| 37 | NH | - | 7.93 | d, J = 8.8 Hz |
| 38 | O | - | - | - |
| 39 | CH2 | 40.8 | 1.41 | m |
| | | | 1.48 | m |
| 40 | CH | 57.3 | 4.11 | m |
| 41 | Cq | 173.2 | - | - |
| 42 | O | - | - | - |
| 43 | CH | 30.1 | 2.03 | m |
| 44 | CH | 24.2 | 1.57 | m |
| 45 | CH3 | 21.3 | 0.81 | m |
| 46 | CH3 | 231 | 0.84 | m |
| 47 | CH3 | 18.0 | 0.83 | m |
| 48 | CH3 | 19.2 | 0.84 | m |
| 49 | N | - | - | - |
| 50 | CH | 134.5 | 7.60 | s |
| 51 | NH | - | * | - |
| 52 | CH | ** | 6.78 | s |
| 53 | OH | - | * | - |
| 54 | OH | - | * | - |
| 55 | CH2 | 31.2 | 1.23 | m |
| 56 | CH2 | 22.1 | 1.24 | m |
| 57 | CH3 | 140 | 0.85 | m |

| | | | | |
|---|---|---|---|---|
| * Not visible, **Visible with addition of TFA: 25-C (δC 129.8 ppm), 52-C (δC 117.6 ppm) | | | | |

The chemical structure and atomic numbering of compound F5 are shown below.

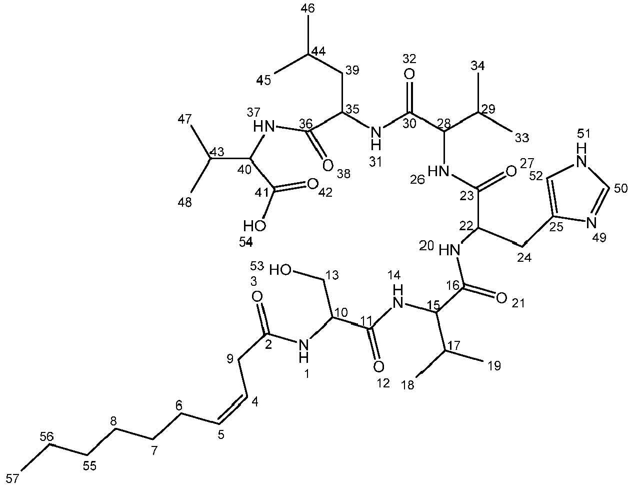

The key correlation of compound F5 is shown below.

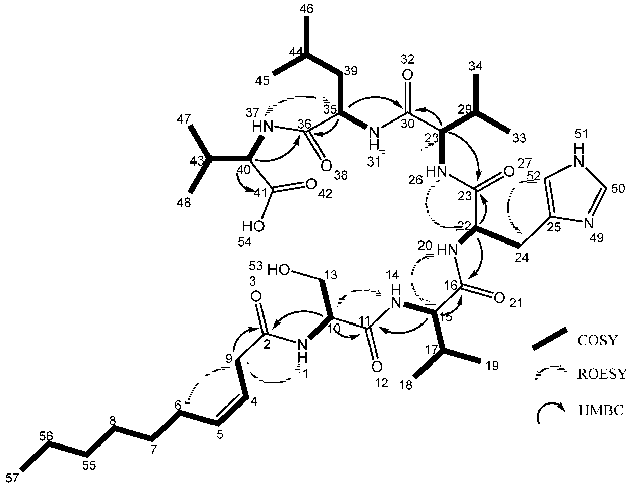

### Structure elucidation of compound F3

Compound F3 is a cyclic lipodepsipeptide of the sequence *N*-octanoyl-¹Ser-²Val-³His-⁴Val-⁵Leu-⁶Val (SEQ ID NO: 322). The hexapeptide lactone ring is formed between the ¹Ser hydroxyl group and the C-terminal carboxyl group of ⁶Val.

### HR-MS and HR-MS/MS

MS and MS/MS spectra have been recorded on a maXis 4G QTOF mass spectrometer from Bruker Daltonics in positive ionization mode. Fragmentation energies are provided at the top right of the spectrum. Compound F3 was purified as amorphous white powder. The HR-ESIMS data indicated a molecular formula of C38H64N8O8 with RDBE = 11.

### NMR

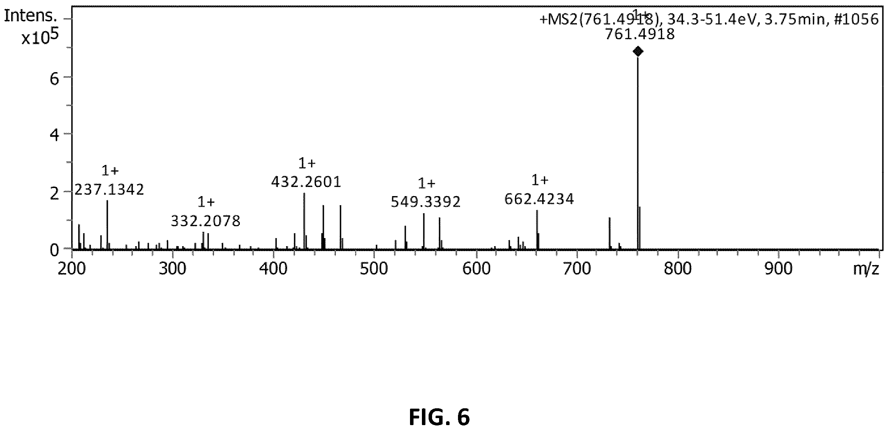

The ¹H- and ¹³C-NMR spectra of compound F3 have been recorded at 600 MHz in DMSO-d6. The NMR data for compound F3 and compound J were similar (**Table 14**; **FIG. 6**). The unsaturation number of 11 for compound F3 and the missing two olefinic methine hydrogens (δ_{H} 5.47, 5.48), compared to compound J, revealed an octanyl instead of a decenoyl side chain in compound F3. Observed HMBC correlations from 13-CH₂ to 41-C in HMBC, in combination with the observed unsaturation number of 11, indicated that compound F3 was a cyclic lipodepsipeptide.

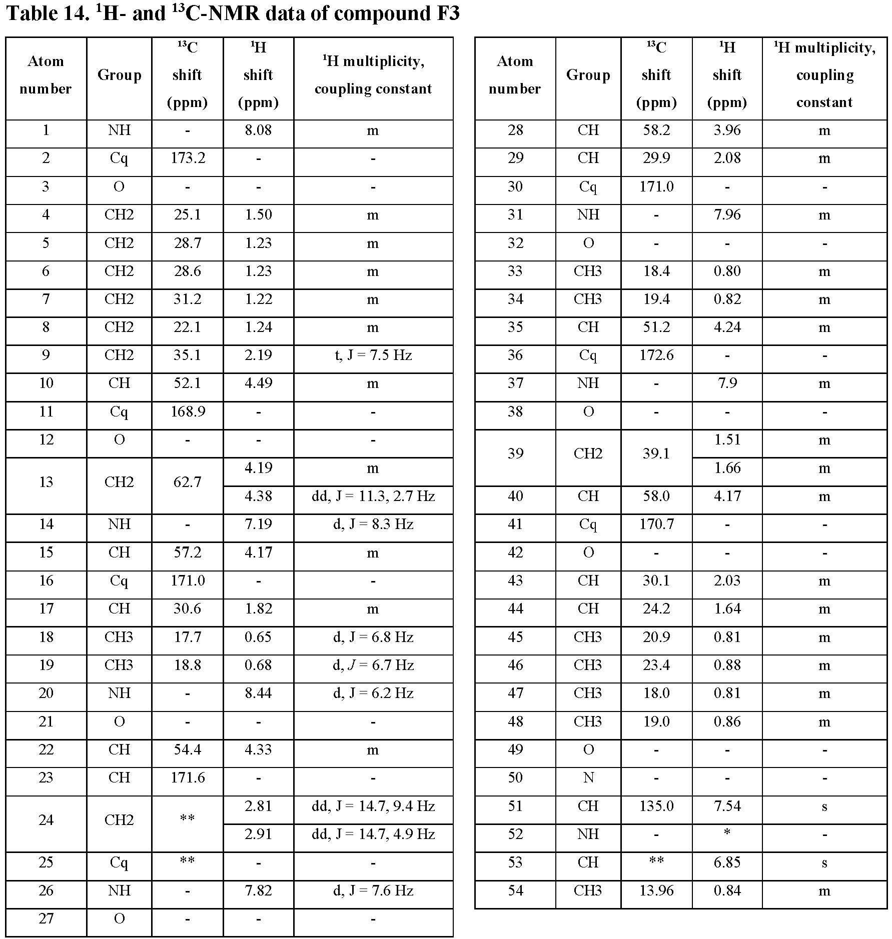

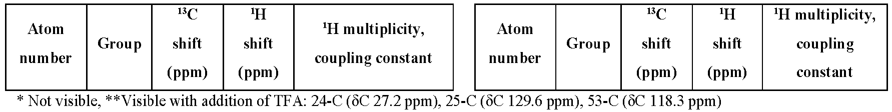

The chemical structure and atomic numbering of compound F3 are shown below.

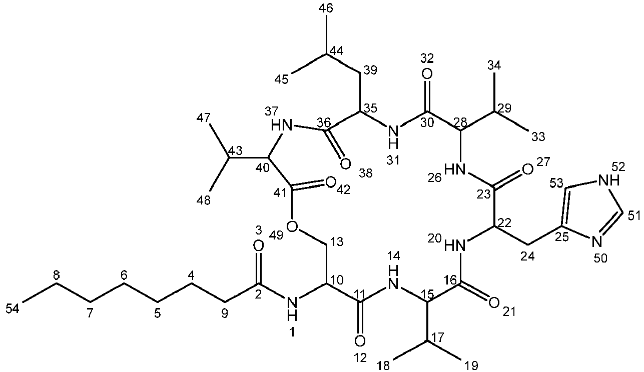

### Structure elucidation of compound D

Compound D is a linear lipopeptide of the sequence *N*-octanyl-¹Ser-²Val-³His-⁴Val-⁵Leu-⁶Val (SEQ ID NO: 323). In comparison to compound F3 the lactone between the ¹Ser hydroxyl group and the C-terminal carboxyl group of ⁶Val has been hydrolyzed.

### HR-MS and HR-MS/MS

MS and MS/MS spectra have been recorded on a maXis 4G QTOF mass spectrometer from Bruker Daltonics in positive ionization mode. Fragmentation energies are provided at the top right of the spectrum. Compound D was purified as amorphous white powder. The HR-ESIMS data indicated a molecular formula of C₃₈H₆₆N₈O₉ with RDBE = 10.

### NMR

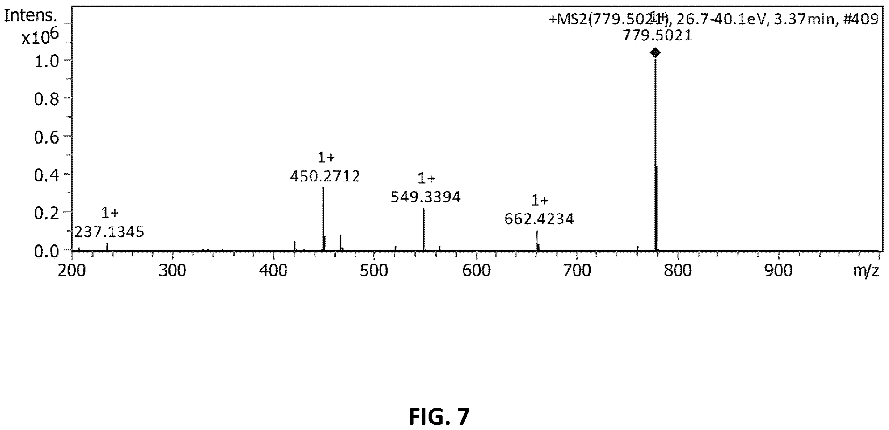

The ¹H- and ¹³C-NMR spectra of compound D have been recorded at 600 MHz in DMSO-d6. The NMR data for compound D and compound F3 were similar (**Table 15**; **FIG. 7**). The most significant differences were the missing correlation of 13-CH₂ to 41-C in HMBC and difference in shift of 13-CH2 (δ_{H} 3.50 and 3.56) compared to 13-CH2 (δ_{H} 4.19 and 4.38) for compound F3. These observations in combination with the observed unsaturation number of 10 indicated that compound D was a linear lipopeptide.

**Table 15 ¹H- and ¹³C-NMR data of compound D**

| **Atom number** | **Group** | **¹³C shift (ppm)** | **¹H shift (ppm)** | **¹H multiplicity, coupling constant** |
|---|---|---|---|---|
| 1 | NH | - | 7.96 | d, J = 7.6 Hz |
| 2 | Cq | 172.4 | - | - |
| 3 | O | - | - | - |
| 4 | CH2 | 25.3 | 1.46 | m |
| 5 | CH2 | 28.7 | 1.22 | m |
| 6 | CH2 | 28.5 | 1.22 | m |
| 7 | CH2 | 31.2 | 1.22 | m |
| 8 | CH2 | 22.1 | 1.24 | m |
| 9 | CH2 | 35.2 | 213 | t, J = 7.4 Hz |
| 10 | CH | 55.3 | 4.35 | m |
| 11 | Cq | 170.4 | - | - |
| 12 | O | - | - | - |
| 13 | CH2 | 61.8 | 3.50 | dd, J = 10.5, 5.7 Hz |
| | | | 3.56 | dd, J = 108, 5.9 Hz |
| 14 | NH | - | 7.82 | d, J = 8.8 Hz |
| 15 | CH | 57.6 | 4.13 | m |
| 16 | Cq | 170.8 | - | - |
| 17 | CH | 30.4 | 1.89 | m |
| 18 | CH3 | 19.0 | 0.68 | m |
| 19 | CH3 | 17.6 | 0.69 | m |
| 20 | NH | - | 8.23 | d, J = 7.7 Hz |
| 21 | O | - | - | - |
| 22 | CH | 52.9 | 4.53 | m |
| 23 | Cq | 171.0 | - | - |
| 24 | CH2 | 29.1 | 2.75 | dd, J = 14.8, 9.2 Hz |
| | | | 2.90 | dd, J = 14.9, 4.8 Hz |
| 25 | Cq | ** | - | - |
| 26 | NH | - | 779 | m |
| 27 | O | - | - | - |
| 28 | CH | 57.6 | 4.13 | m |
| 29 | CH | 30.4 | 1.97 | m |
| 30 | Cq | 170.9 | - | - |
| 31 | NH | - | 8.42 | m |
| 32 | O | - | - | - |
| 33 | CH3 | 18.1 | 0.80 | m |
| 34 | CH3 | 19.1 | 0.81 | m |
| 35 | CH | 50.9 | 4.43 | m |
| 36 | Cq | 171.9 | - | - |
| 37 | NH | - | 7.85 | d, J = 9.4 Hz |
| 38 | O | - | - | - |
| 39 | CH2 | 40.8 | 1.41 | m |
| | | | 1.49 | m |
| 40 | CH | 57.6 | 4.08 | dd, J = 8.6, 5.7 Hz |
| 41 | Cq | 173.4 | - | - |
| 42 | O | - | - | - |
| 43 | CH | 30.2 | 2.04 | m |
| 44 | CH | 24.2 | 1.57 | m |
| 45 | CH3 | 21.3 | 0.81 | m |
| 46 | CH3 | 231 | 0.84 | m |
| 47 | CH3 | 18.1 | 0.82 | m |
| 48 | CH3 | 19.3 | 0.84 | m |
| 49 | N | - | - | - |
| 50 | CH | 134.6 | 758 | s |
| 51 | NH | - | * | - |
| 52 | CH | ** | 6.77 | s |
| 53 | OH | - | * | - |
| 54 | OH | - | * | - |
| 55 | CH3 | 140 | 0.84 | m |

| | | | | |
|---|---|---|---|---|
| * Not visible, **Visible with addition of TFA: 25-C (δ_{C} 129.8 ppm), 52-C (δ_{C} 117.5 ppm) | | | | |

The chemical structure and atomic numbering of compound D are shown below.

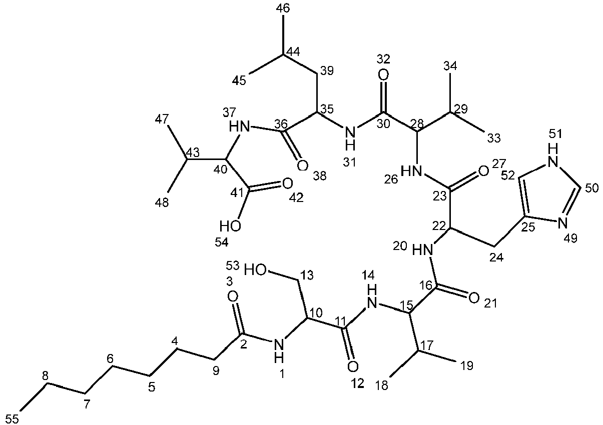

### Biosynthesis of compound J and related analogs

A retrobiosynthetic analysis of compound J (cyclic hexadepsipeptide of biosynthetic sequence: *N*-Acyl-Ser-Val-His-Val-Leu-Val (SEQ ID NO: 318)) allowed for straightforward identification of its BGC from the internally sequenced genome of *C. vaccinii.* The BGC is located directly downstream of the A1-BGC. The compound J-BGC consists of four genes, *dis1, dis2, dis3, dis4.* Their deduced protein products are typical nonribosomal peptide synthetase (NRPS) enzymes with a modular organization. The genes *dis2* and *dis3* represent gene duplications of the partial sequence of *dis1.* For the biosynthesis of compound J and related analogs the enzymes encoded by *dis2* and *dis3* have no function. The assembly line of compound J contains 6 modules and 20 domains in total. The substrate specificity of each adenylation (A) domain was predicted according to the putative binding-pocket constituents, the so-called Stachelhaus-code (Stachelhaus et al. (1999) Chem Biol.;6(8):493-505). The predicted and observed amino acids match almost perfectly (**Table 16**). Only for the third A domain of Dis1 incorporation of His instead of the predicted Tyr is observed.

**Table 16. Summary table A domain analysis**

| **A domain** | **Stachelhaus code** | **predicted class** | **nearest Stachelhaus code** | **observed in structure** |
|---|---|---|---|---|
| 1 | DVWHISLVDK | hydrophobic-aliphatic | Ser | Ser |
| 2 | DALWIGGTFK | hydrophobic-aliphatic | Val | Val |
| 3 | DtSTIAAVCK | hydrophobic-aromatic | Tyr | His |
| 4 | DALWIGGTFK | hydrophobic-aliphatic | Val | Val |
| 5 | DAWFLGNVVK | hydrophobic-aliphatic | Leu | Leu |
| 6 | DALWIGGTFK | hydrophobic-aliphatic | Val | Val |

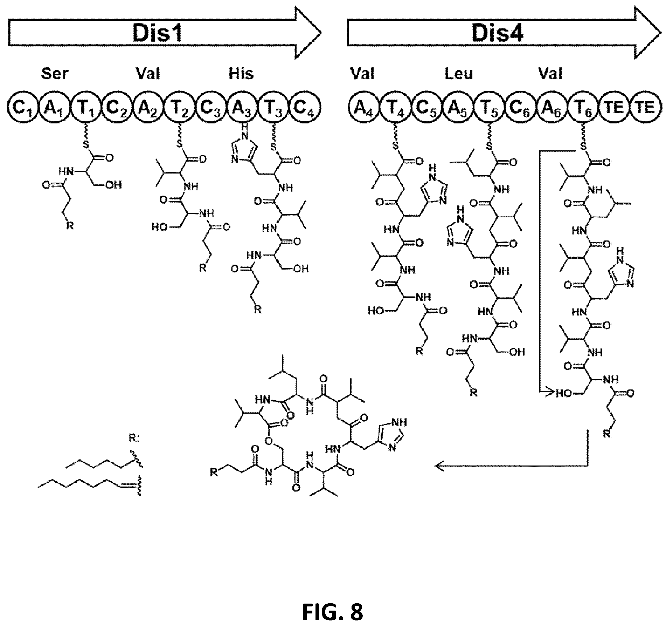

The C domain located at the N-terminus of Dis1 is expected to N-acylate Ser with activated fatty acid moieties. The thioesterase (TE) domains at the C-terminus of Dis4 catalyze off-loading and intramolecular cyclization of the final product (**FIG. 8**).

### Example 2: Process for Partial Reoxidation and Producing Antibody Drug Conjugates

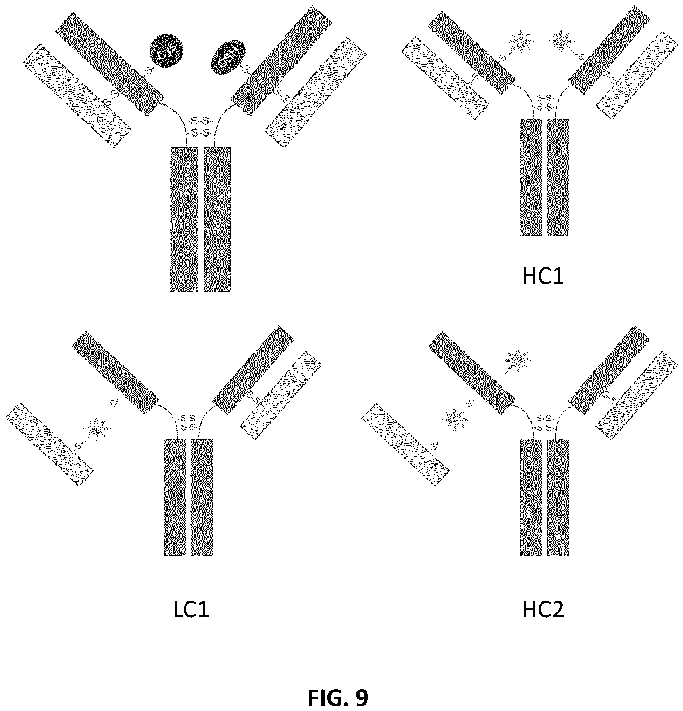

Conditions for the production of anti-PMEL17 antibody drug conjugates were screened to optimize the drug to antibody ratio and % on-site coupling. Production of anti-PMEL17 antibody drug conjugates on scale was afford by a reduction wash step, followed by a partial reoxidation step. The reduction wash afforded de-capping of engineered cysteine residues, which provides open sites for coupling of the drug to the antibody. Over-reduction in the reduction wash step (e.g., reduction of both targeted cysteine residues as well as intrachain disulfide bridges) was corrected through a partial reoxidation step, which results in re-capping of off-target cysteine residues (*e.g.,* to reform intrachain disulfide bridges). In the over-reduction - reoxidation process, there is potential for conjugation of the drug to the antibody on site (HC1) or off-site (LC1 or HC2), as depicted in **FIG. 9****.**

Antibody was incubated with RMP Protein A resin (GE) at a ratio of 10 mg Ab to 1 ml resin in PBS for 15 minutes with mixing in an appropriately sized disposable column. Cysteine HCl was added and incubated with agitation for 30 min at room temperature to allow the reactive cysteines to be deblocked. Cysteine HCl concentration and pH were modulated to optimize drug to antibody ratio and % on-site coupling. Concentration of the cysteine HCl wash at pH 6 was varied from 10 to 30 mM *(i.e.,* 10, 12, 14, 16, 20, 25, and 30 mM). Buffer pH was varied over the range of 6.95 to 7.25. Conditions were assessed by coupling a surrogate linker to the antibody and measuring the Surrogate to Antibody Ratio (SAR). The resin was quickly washed with 4 column volumes PBS on a vacuum manifold. The resin was then resuspended in an equal volume PBS containing 250 nM CuCl₂. Reformation of antibody interchain disulfides was monitored by taking time points. At each time point, 25 µL of resin slurry was removed, 1 µL of 20 mM of Compound (B1) or Compound (B2) described herein was added, and the tube flicked several times. The resin was spun down, supernatant removed, and then eluted with 50 µL Antibody elution buffer (Thermo). The resin was pelleted and the supernatant analyzed by reverse phase chromatography using an Agilent PLRP-S 4000A 5um, 4.6x50mm column (Buffer A is water, 0.1% TFA, Buffer B Acetonitrile, 0.1% TFA, column held at 80 C, Flowrate 1.5 ml/min). For each set of conditions tested, the sample was allowed to reoxidize over the course of 3 days at room temperature before measuring the SAR. A selection of the results from this screen are summarized in **Table 17.**

**Table 17. Results of Reduction Wash Screening**

| | | | | **Surrogate to Antibody Ratio (SAR)** | | | | |
|---|---|---|---|---|---|---|---|---|
| | | | | ***After 3 days of reoxidation at RT*** | | | | |
| ***LD (g*/*L)*** | ***Cystein (mM)*** | ***pH*** | ***Yield (%)*** | ***LC1 (%)*** | ***HC0 (%)*** | ***HC1 (%)*** | ***HC2 (%)**** | ***SAR*** |
| 15 | 10 | 6.90 | 80.6 | **4.9** | 8.3 | **89.3** | n.a. | 1.9 |
| 30 | 10 | 6.90 | 82.1 | **5.1** | 9 | **88.4** | n.a. | 1.9 |
| 30 | 10 | 6.75 | 78.7 | **2.5** | 10.4 | **87.3** | n.a. | 1.8 |
| 30 | 10 | 7.10 | 84.7 | **4.9** | 8.8 | **88.9** | n.a. | 1.9 |

Conditions that were modulated to optimize the reoxidation step were vessel geometry, amount of headspace, amount of mixing, temperature, pH and antibody concentration. It was observed that increased pH and decreased antibody concentration result in accelerated reoxidation. For further development, reoxidation was performed on CEC eluate at a pH of 5.8 and a protein concentration of 13.5 g/L. Reoxidation was performed in both a beaker *(i.e.,* cylindrical vessel) as well as an Erlenmeyer flask *(i.e.,* tapered vessel) to determine the impact of vessel shape on the reoxidation process. To evaluate the impact of headspace, the reoxidation step was performed in vessels with 60% headspace as well as with no headspace. To evaluate the impact of mixing, the reoxidation step was performed both without stirring and at a stir rate of 100 rpm. No impact on reoxidation was observed across changes in vessel geometry, head space, or mixing. Reoxidation was performed at both room temperature *(i.e.,* 25 °C) and in a cold room; reoxidation was slowed at reduced temperature. For production of the drug-antibody conjugate, CEC eluate was held for 48 to 72 h in a tank with up to 60% air overlay to afford reoxidation. An improvement in on-site to off-site coupling and purity was observed after the reoxidation step.

**Table 18. Purity of Antibody Conjugate Following Reoxidation.**

| | | **RPC-Surogate to Antibody Ratio** | | | | **NR CE-SDS** | | **SEC** | |
|---|---|---|---|---|---|---|---|---|---|
| | | LC1 | HC1 | HC2 | SAR | Purity | Impurity | HMW | LMW |
| | | (%) | | | | (%) | | (%) | |
| **Process development** | **CEC eluate** | 16.8 | 69.0 | 19.8 | 2.6 | 62.4 | 36.3 | 1.4 | 0.67 |
| | **After reoxidation step (48h)** | 1.8 | 82.2 | 5.7 | 1.9 | 91.5 | 7.1 | 1.7 | 0.71 |

In summary, reoxidation was assessed for 5 days. Reoxidation occurred after 48 hours. No apparent impact of head space, vessel geometry or mixing could be detected in this small-scale experiment, but mixing could lead to an increase of turbidity. In cold room, a much slower reoxidation was observed. As scaling effects and effect of buffer preparation on dissolved oxygen could not completely be excluded, the following reoxidation step was suggested: 48 hours - 3 days of hold of CEC eluate in tank filled to max. 60% with air overlay. Stirring could be beneficial for reoxidation at larger scale.

Once it was determined that the antibody has reformed its interchain disulfide bonds, the resin was washed with 10 column volumes PBS and the resin was resuspended in an equal volume PBS and 8 equivalents of linker-payload (20 mM) in DMSO was added and then incubated at room temperature for 2 hours. The resin was then washed with 50 column volumes PBS. The ADC was eluted from the protein A resin with Antibody elution buffer and neutralized with 1/10 volume 1 M Tris pH 9.0. The ADC was then buffer exchanged into PBS or other suitable buffer and preparative size exclusion chromatography to remove aggregates was performed (S200 Increase; GE), if needed. The following analyses were performed - analytical SEC to determine percent monomer, mass spectroscopy to determine DAR, LAL test to determine endotoxin load and protein concentration was determined by A280 utilizing extinction coefficient and molecular weight of antibody.

By targeting an over-reduction step followed by a dedicated, robust reoxidation step, engineered cysteines were efficiently de-capped to afford drug-antibody conjugation at the targeted site with high purity.

### Example 3: Isolation and Analysis of Compound (A1) and Analogs

Different promoters in exchange of the native promoter of the compound (A1) assembly line were characterized, resulting in an overexpression mutant with significantly increased production of compound (A1). Thereby, the isolation and structure elucidation of novel compound (A1) analogs of low abundance was allowed. Further, the antiproliferative activities of fifteen chromodepsins against uveal melanoma cell lines harboring Gaq/11 mutations were explored and the major metabolite of compound (A1) formed in plasma was characterized.

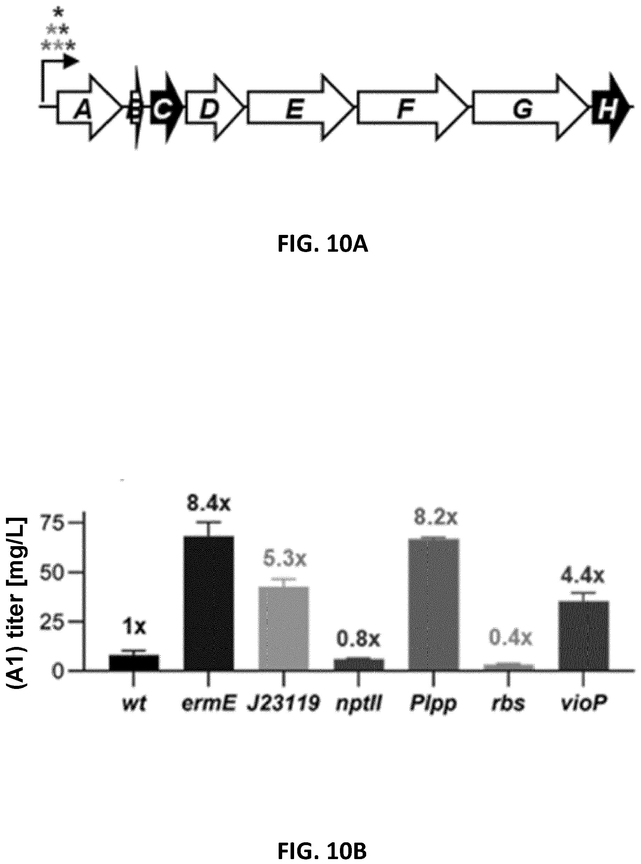

As described in Example 1, *Chromobacterium vaccinii* MWU205 (sometimes referred to herein as *C. vaccinii)* was identified as producer of compound (A1) by a genome mining approach. The titer of compound (A1) in *C. vaccinii* culture broth upon cultivation in LB medium was in the range of 5-10 mg/L, which did not fulfill the prerequisites of an efficient biotechnological production. Thus, approaches to improve the production of *C. vaccinii* by genetic engineering were investigated. The compound (A1)-BGC includes eight genes, *frsA-H,* organized in a single operon. Since the BGC lacks additional regulatory genes, the exchange of the native promoter upstream of *frsA* by alternative promoters was considered. A conjugation protocol for *C. vaccinii* with *E*. *coli* as donor was established and genetic constructs were designed to generate the desired promoter exchange mutants by homologous recombination driven genomic integrations. Six different promoters (*ErmE*, J23119, nptII, Plpp, rbs,* and *vioP*) were investigated in this context (**FIG. 10A**). These are all constitutive promoters, with the exception of *vioP.* The sequence of the ribosomal *rbs* promoter was extracted from the genome sequence of *C. vaccinii.* The *vioP* promoter, which is regulated via *N*-acyl homoserine lactones involved in quorum sensing, was extracted from the violacein BGC of *C. vaccinii.* The impact of promoter insertions on the compound (A1) titer was analyzed by cultivation of genetically verified mutants in triplicates. While the compound (A1) titers of the *rbs* as well as *nptII* mutants were below those of the wild type, the insertion of the *vioP, J23119, Plpp* and *ErmE** promoter resulted in FR titer increases ranging from 4.4 up to 8.4-fold (**FIG. 10B**). This outcome allows for further strain optimization to establish a scalable, sustainable and cost-effective biotechnological process for production of compound (A1).

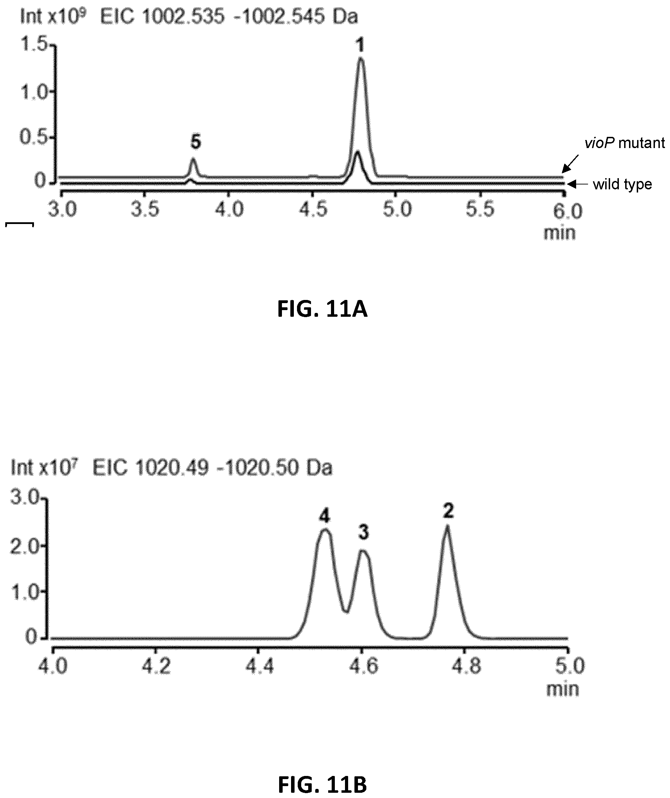

Upon isolation of compound (A1) from culture extracts of the *C. vaccinii vioP* mutant, three compounds with a mass difference of +18 Da, were detected at very low abundance in the enriched fractions. The initial assumption that these compounds might correspond to hydrolyzed compound (A1) was disproven by the HR-ESIMS data (formula: C₄₉H₇₈N₇O₁₆+ ; calcd [M+H]+ = 1020.5500; obsd [M+H]+ = 1020.4948 Δ = 54.09 ppm). The molecular formulae of these three compounds were established by HR ESIMS as C₄₈H₇₄N₇O₁₅S+ (calcd [M+H]+ = 1020.4958; Δ = 0.98 ppm). However, the abundance of these compounds was too low for isolation. The presence of sulfur in the molecules was suspected to originate from incorporation of either cysteine (Cys) or methionine (Met). Indeed, feeding of L-Met, but not L Cys, to the culture broth increased the abundance of these compounds (**FIG. 11B**). A subsequent cultivation of *C. vaccinii vioP* mutant at 50 L scale in a bioreactor in combination with feeding of L-Met allowed the isolation of sufficient amounts of all three compounds for structural assignment (shown below) by extensive 1D and 2D NMR experiments (¹H, ¹³C, ¹H ¹H COSY, ¹H-¹H ROESY, ¹H-¹³C HSQC, ¹H-¹³C HMBC). The comparison of the ¹H and ¹³C chemical shifts of 2-4 and (A1) showed very good agreement for five of the eight residues (Ala, N methyldehydroalanine (N Me Dha), 3-phenyllactic acid (Pla), N,O-Me2-Thr, N-Me-Ala; see Supporting Information). The main differences of compounds 2-4 were respectively spotted in one of the three hydroxyleucine (Hle) residues, which are further modified by N acetylation or N propionylation depending on their position in the structure of compound (A1). For compound 2 the shifts of the N acetylhydroxyleucine (N Ac-Hle) residue differed significantly from those observed for compound (A1), whereas the shifts for the N propionylhydroxyleucine (N Pr Hle) and Hie residues were in good agreement with those of compound (A1). Evaluation of the 1D and 2D NMR spectra of 2 revealed a replacement of the N Ac Hle residue by a N acetylhydroxymethionine (N-Ac-Hme) residue.

The structures of compound (A1) and analogs compounds 2-7 are shown below.

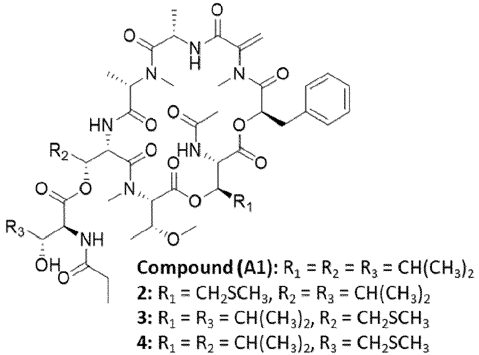

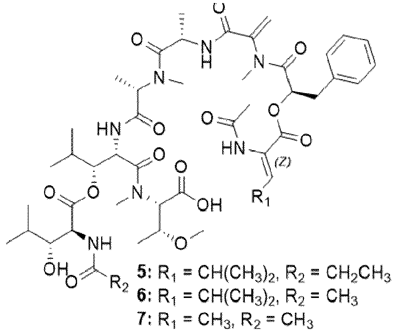

The expected COSY and HMBC correlations within the N-Ac-Hme residue were observed and the connectivity to the other residues was established by three-bond ¹H-¹³C couplings observed in HMBC from the α-position of the Pla residue to the N-Ac-Hme carbonyl and from the N-Ac-Hme β-position to the carbonyl of the *N*,*O*-Me₂-Thr residue. For compound 3 the hydroxymethionine (Hme) residue replaces the Hle residue in (A1), as confirmed by HMBC correlations from the α-position of the *N*,*O*-Me₂-Thr residue to the Hme carbonyl, from the Hme α-position to the carbonyl of the *N*-Me-Ala residue, and from the Hme β-position to the carbonyl of the N-Pr-Hle side chain (shown above). In Compound 4, the *N*-Pr-Hle side chain is replaced by *N*-propionylhydroxylmethionine (*N*-Pr-Hme), as established by HMBC correlations from the β-position of the Hle residue to the *N*-Pr-Hme carbonyl and from the *N*-Pr-Hme α-position to the carbonyl of the propionyl. The configurations of Compounds 2-4 are deemed to be identical to that of compound (A1) due to their emergence from the same biosynthetic assembly line. The existence of all three possible compound (A1) analogs with single replacements of the Hle residue by Hme, including modifications by *N*-acetylation or *N*-propionylation, manifests a remarkable flexibility in the biosynthetic assembly line of (A1). The initial biosynthetic divergence is the selection and activation of L-Met instead of L-Leu by the adenylation (A) domains of FrsA, FrsD or FrsG respectively, followed by priming of the respective downstream thiolation (T) domains. The non-heme diiron monooxygenase FrsH has been demonstrated to catalyze β-hydroxylation of T domain tethered L-Leu and apparently it can also catalyze β-hydroxylation of L-Met. The condensation (C) domains of FrsA and FrsD can apparently accept the T domain tethered Hme for catalyzing *N*-propionylation and *N*-acetylation respectively. Further, the concerned C domains in the assembly seem to be able to catalyze the condensation of the Hme residues with various (depsi)peptide intermediates. L-Met appears to be a "common denominator" for the downstream biosynthetic cascades at different entry points, as it offers the potential for β-hydroxylation in combination with adequate steric similarity to L-Leu. This flexibility, especially of the C domains, is encouraging for the generation of further analogs of compound (A1) by genetic engineering of the assembly line.

Analysis of the culture extracts of the *C. vaccinii vioP* mutant additionally revealed a second peak with exact mass identical to (A1), eluting at shorter retention time in HPLC (**FIG. 11A**). As a consequence of higher titers of (A1) produced by the *C. vaccinii vioP* mutant, the production of compound 5 is increased as well, thereby allowing its isolation and subsequent structure elucidation, along with its analog compound 6. Furthermore, an isomer of compound 10 (compound 7) was isolated from the culture extracts of *Chromobacterium* sp. QS3666. Evaluation of the data from extensive 1D and 2D NMR experiments revealed the structures of these isomers, compounds 5-7, as linear depsipeptides arising from compounds (A1), 8, and Compound 10 respectively by formal hydrolysis of the macrolacton between the *N*,*O*-Me₂-Thr and *N*-Ae-Hle (*N*-Ac-Thr in YM) residues in combination with dehydration of the *N*-Ac-Hle (*N*-Ac-Thr in YM) residues. This was deduced by the loss of ¹H and ¹³C signals for the respective α- and β-methine groups in the *N*-Ac-Hle and *N-*Ac-Thr residues. The concomitant shift of the respective ¹³C signals to the olefinic range (δ_{C} 124.8 and 144.0 for compound 5) indicated the presence of a double bond and accordingly a *N*-acetyldehydroleucine (*N*-Ac-Dhl) residue in compounds 5 and 6, and a *N*-acetyldehydrobutyrine (*N*-Ac-Dhb) residue in Compound 7. The double bond configuration of compounds 5 and 6 was established as Z due to strong NOE between the olefinic proton and the proton at the tertiary carbon of the isopropyl group in combination with a strong NOE between the tertiary carbon of the isopropyl group and the NH of*N-*Ac-Dhl. Compound 7 showed analogous NOE. The linear constitution of compound 5 was in addition supported by derivatization with 4-bromophenacyl bromide and interpretation of the HR-MS/MS spectra of the resulting product. As no hydrated analogs of compound 5 were detected in the culture extracts, it was postulated that formation of compound 5 from Compound A1 likely occurs via E1cB elimination mechanism. Isotope labelling experiments further support this hypothesis. When compound (A1) is treated with alkaline ¹⁸O-water, compound 5 forms without incorporating ¹⁸O. In a hydrolysis-dehydration sequence however, the expected product would have incorporated ¹⁸O into the carboxylic acid functional group of compound 5 during hydrolysis and would have eliminated the ¹⁶O-hydroxy group (not observed). Alternatively to the isolation from the culture extracts, compounds 5-7 can be accessed in good yields by treatment of compounds (A1), 8, or 10 with triethylamine in DMSO. Two potential structures were proposed arising from transesterification of the hydroxyl of the *N*-Pr-Hle side chain with either of the macrolactones.

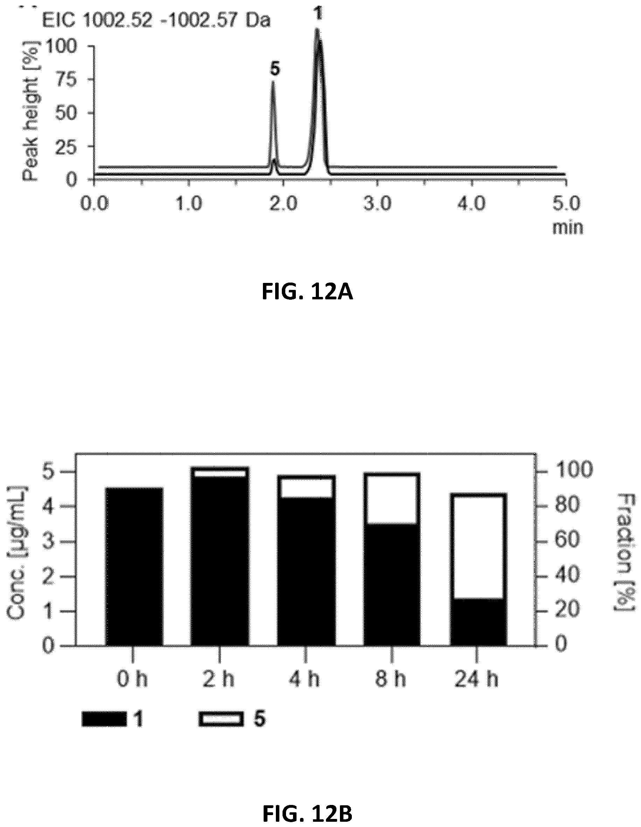

The stability of compound (A1) was also investigated in mouse, monkey and human plasma. By spiking additional reference material of compound 5 in the human plasma sample following 8 h incubation of compound (A1), it could be unambiguously demonstrated that the formed compound (A1) isomer instead corresponds to compound 5 (**FIG. 12A**). Further, absolute quantification of compounds (A1) and 5 was performed following incubation of compound 1 in human plasma for up to 24 h. The combined fractions of compound (A1) and 5 after 24 h incubation accounted for ~90% of the deployed concentration of compound (A1) (**FIG. 12B**), thereby indicating that compound 5 is the major metabolite formed in human plasma. Very similar results were obtained using mouse and monkey plasma.

Fifteen chromodepsins shown herein, eleven from the compound (A1)-class and four from the compound 10-class, were characterized in cell proliferation assays against four cancer cell lines. Compounds (A1), 2-6, 8, 9, and 12 were isolated from culture broths of *C. vaccinii.* Compounds 10 and 11 were prepared from compound (A1) as previously described for compound 10. Compounds 7, and 13-15 were isolated from culture broths of *Chromobacterium* sp. QS3666. Uveal melanoma, the most common eye cancer in adults, is genetically defined by oncogenic gain-of-function mutations in Gaq and Gα11 at the recurrent hotspots Q209 and R183. Both mutations blunt the GTPase activity leading to the GTP-bound, active conformation of the protein. Compound (A1) inhibits these oncoproteins by trapping constitutively active Gaq/11 in inactive, GDP-bound Gαβγ heterotrimers. In response to compound (A1), Gaq/11-mutant uveal melanoma cells arrest the cell cycle and undergo melanocytic differentiation and apoptosis. Systemically administered compound (A1) selectively inhibited the growth of Gaq-mutant uveal melanoma xenografts at doses that were well tolerated, but had no effect on BRAF(V600E)-driven tumors. Two of the cancer cell lines in the test panel, 92.1 and MP41, were uveal melanoma cell lines harboring heterozygous GNAQ Q209L or GNA11 Q209L mutations, respectively. The other two cell lines, A375 and SK MEL 30, are skin cutaneous melanoma cells with no oncogenic mutations in GNAQ or GNA11, but harboring homozygous BRAF V600E or heterozygous NRAS Q61K mutations respectively. This dataset represents a comprehensive compilation of compound (A1) and analogs in context of antiproliferative activities against uveal melanoma cell lines.

The structures of compounds 8-15 are shown below.

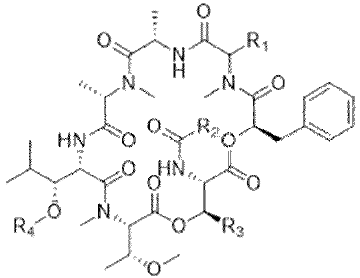

| **Cmpd** | **R₁** | **R₂** | **R₃** | **R₄** |
|---|---|---|---|---|
| **8** | =CH₂ | CH₃ | CH(CH₃) | *N*-Ac-Hle |
| **9** | =CH₂ | CH₂CH₃ | CH(CH₃)₂ | *N*-Pr-Hle |
| **10** | CH₃ (S) | CH₃ | CH(CH₃)₂ | *N-*Pr-Hle |
| **11** | CH₃ (*R*) | CH₃ | CH(CH₃)₂ | *N*-Pr-Hle |
| **12** | =CH₂ | CH₃ | CH(CH₃)₂ | H |
| **13** | =CH₂ | CH₃ | CH₃ | *N*-Ac-Hle |
| **14** | =CH₂ | CH₃ | CH₃ | *N*-Pr-Hle |
| **15** | =CH₂ | CH₃ | CH₃ | H |

The growth inhibition (GI)₅₀ values of the tested compounds for inhibition of cell proliferation in the 92.1 (GNAQ Q209L), MP41 (GNA11 Q209L), A375 and SK-MEL-30 (both GNAQ/11 wild type) cell lines, ranked by potency, are shown in **Table 19.**

**Table 19. Growth inhibition (GI₅₀) values for inhibition of cell proliferation in different cell lines.**

| **Compound** | **GI₅₀ by cell line [nM]** | | | |
|---|---|---|---|---|
| | **92.1** | **MP41** | **A375** | **SK-MEL-30** |
| **1** | 0.6 | 0.2 | n.a. | n.a. |
| **8** | 1.0 | 0.4 | n.a. | n.a. |
| **4** | 1.2 | 0.5 | n.a. | n.a. |
| **9** | 1.4 | 0.7 | n.a. | n.a. |
| **11** | 1.8 | 0.7 | n.a. | n.a. |
| **14** | 5.2 | 2.2 | n.a. | n.a. |
| **2** | 9.8 | 3.7 | n.a. | n.a. |
| **3** | 10.4 | 4.0 | n.a. | n.a. |
| **13** | 15.7 | 6.0 | n.a. | n.a. |
| **10** | 607 | 396 | n.a.* | n.a.* |
| **12** | 1742 | 1180 | n.a.* | n.a.* |
| **15** | n.a.* | n.a.* | n.a.* | n.a.* |
| **5** | n.a. | n.a. | n.a. | n.a. |
| **6** | n.a.* | n.a.* | n.a.* | n.a.* |
| **7** | n.a. | n.a. | n.a. | n.a. |

| | | | | |
|---|---|---|---|---|
| n.a. = not active, GI₅₀ > 260 nM; n.a.* = not active, GI₅₀ > 10 µM | | | | |

The selective antiproliferative profile of the chromodepsins against GNAQ/GNA11 mutant cell lines is confirmed by the lack of activity against the A375 and SK MEL 30 cell lines that are independent from GNAQ/GNA11 signaling. The potency ranking using the 92.1 and MP41 cell lines is in good agreement with the previously SAR studies. Compound (A1) is the most potent compound with an exquisite potency in the sub-nM range. Compounds 8, 9 and 11 lose potency by a factor of two to three. Interestingly, the newly identified analog compound 4 is only two-fold less active, in contrast to factors of ~15 for compounds 2 and 3. These findings are in line with a recent report from Voss *et al.*, highlighting the importance of the "lipophilic anchors" of compound (A1) at the residues varied in compounds 2 and 3, in context of binding kinetics and prolonged residence time on the target proteins. The retained potency of compound 4 indicates a promising exit vector for potential synthetic or biosynthetic modifications of the scaffold. Compound 10 is by a factor of -25-30 less potent compared to (A1), whereas compound 14 drops only by -10-fold. Matched molecular pair analysis of members of the compound (A1) class (compounds (A1) and 8) and the compound 10 class (compounds 10 and 14) highlights the crucial potency contributions by the isopropyl group at R3 in comparison to the reduced contribution of the N-propionyl group at R4. The compound (A1) core structure of compound 12 exhibits a > 3.000-fold change compared to compound (A1) concerning growth inhibition. Notably, this fold change is significantly higher compared to what has been reported (16-fold change) concerning inhibition of Gaq proteins using real-time live-cell phenotypic biosensing based on dynamic mass redistribution. The compound 10 core structure compound 15 has an GI50 value > 10 µM, with some antiproliferative activity observed at the highest doses tested. The linearized depsipeptides compounds 5-7 lack any antiproliferative activity. In this experimental setup, the cellular permeability of the compounds was a crucial factor contributing to the observed antiproliferative potency. Therefore, all compounds were characterized in a low efflux MCDK assay and determined their exposed polar surface areas (EPSA) in order to assess their passive cellular permeabilities. All macrocyclic chromodepsins that feature the N acyl Hle side chain show similar results concerning their % fraction absorbed (% FA; average -60% FA ± 20%) and EPSA values. Compounds 12 and 15, the core structures lacking the N-acyl-Hle side chain, exhibit a ~5 fold reduction in % FA and slight changes in EPSA, whereas the linearized compounds 5-7 show a ≥ 10 fold reduction in % FA and > 2-fold change in EPSA, thereby indicating a very likely drastically decreased passive cellular permeability of these compounds. Unbiased assessment of the binding affinities and kinetics of compounds 12 and 15 to Gaq/11 proteins will require an experimental set-up like *e.g.* surface plasmon resonance with purified proteins.

In summary, the successful genetic engineering of *C. vaccinii* resulting in a mutant with increased production of compound (A1), which allows for further strain and process development to establish a sustainable and high-yielding biotechnological process for scalable production of compound (A1). The overproducing mutant allowed the isolation and structural characterization of five novel chromodepsins which permit encouraging insights in the flexibility of the biosynthetic assembly line and allowed for the structural assignment of the major metabolite of compound (A1) formed in human plasma. Further, the comprehensive SAR of fifteen chromodepsins in a cell proliferation assay underscores their exquisite potency and selectivity against cancer cell lines harboring Gα_{q/11} mutations.

### Supporting Information

### Experiment Procedures

Bacterial strains and culture conditions, cloning and conjugation, sequences of investigated promoters are described in the specification herein, *e.g.*, Example 1.

### Compound isolation and analytic procedures

*Compound isolation*: 50 L culture broth was extracted with 50 L ethyl acetate. The ethyl acetate layer was separated and evaporated to dryness. The crude extract was dissolved in 30 L methanol/water (9/1) and extracted three times with each 30 L cyclohexane. The methanol/water layer was evaporated until only water was left. This was then extracted with 25 L ethyl acetate. The ethyl acetate layer was evaporated to dryness. The degreased extract was dissolved in 400 mL methanol and fractionated by SEC on a column (length 25 cm, diameter 12.5 cm) packed with Sephadex LH20 and methanol as eluent. Fractions of 200 mL each were collected and the collected fractions were analyzed by UPLC-UV-MS for presence of chromodepsins or valhidepsins. Rich fractions were pooled and evaporated to dryness. The enriched fractions were dissolved in methanol and further purified by preparative HPLC on a Sunfire C18 column, 30 x 150mm, 5µm particle size; eluent A: deionized water with 0.1 % formic acid, eluent B: methanol with 0.1 % formic acid; flow rate 60 mL/min; gradient: 65% B to 85% B in 15 min. Fractionation was triggered by mass spectrometry. Rich fractions were pooled and evaporated to dryness. If necessary a second preparative HPLC purification was performed to obtain compounds with a purity > 95 %.

*LC-UV-MS measurements*: Measurements were performed on a Vanquish UHPLC (Thermo Scientific) equipped with a photodiode array and a charged aerosol detector. The UHPLC was coupled to an Orbitrap ID-X (Thermo Scientific) mass spectrometer. MS-spectra were recorded at a mass resolution of 60.000 and MS/MS spectra at a mass resolution of 15.000. The spectra were processed with FreeStyle^{™} 1.5 software (Thermo Scientific). Quantification of compound (A1) was evaluated with Chromeleon^{™} 7.3.

*Sample preparation for LC-UV-MS analysis*: 5 mL freeze-thawed culture broth was transferred to a 15 mL conical centrifuge tube and mixed thoroughly with 5 mL CH3CN. 2 g of magnesium sulfate and 0.5 g of sodium acetate were added to the mixture and shaken vigorously. The mixture was centrifuged at 4.000 rpm for 10 min to obtain phase separation. 4 mL of the organic layer were transferred to a test tube and the solvent was removed under vacuum. The resulting residue was dissolved in 400 µL of DMSO and shaken at room temperature for 10 minutes. After centrifugation at 4.000 rpm for 10 min an aliquot of the solution was transferred to a HPLC vial. Thereby the sample is ten-fold concentrated.*LC Method*: Samples were analyzed using a BEH C18 column (1.7 µM, 2.1x100 mm column + 2.1x10 mm pre-column; Waters AG) at a flow rate of 0.9 mL/min and column temperature of 60 °C. The starting conditions were 95% H2O containing 0.02% formic acid and 5% CH3CN containing 0.014% formic acid. The gradient started after 0.2 min and was increased linearly to 100% CH3CN in 6 min. PDA-, MS-, and CAD-signals were recorded simultaneously. Quantification of compound (A1) was performed by UV at 220 nm with a calibration curve generated with external standards of 10, 50, 100, 200, and 400 µg/mL compound (A1). The injection volume for both samples and standards was 1 µL.

*LC method:* Samples were analyzed using a BEH C18 column (1.7 µM, 2.1x100 mm column + 2.1x10 mm pre-column; Waters AG) at a flow rate of 0.9 mL/min and column temperature of 60 °C. The starting conditions were 95% H₂O containing 0.02% formic acid and 5% CH₃CN containing 0.014% formic acid. The gradient started after 0.2 min and was increased linearly to 100% CH₃CN in 6 min. PDA-, MS-, and CAD-signals were recorded simultaneously. Quantification of FR was performed by UV at 220 nm with a calibration curve generated with external standards of 10, 50, 100, 200, and 400 µg/mL FR. The injection volume for both samples and standards was 1 µL.

*NMR measurements*: NMR experiments were performed on a Bruker Ultrashield 600 Plus NMR spectrometer operating at 600 MHz (¹H) and 150 MHz (¹³C). NMR spectra were processed using Bruker Topspin (version 3.5) and MestReNova (version 14.2). Spectra were referenced to residual solvent signals with resonances at δH/C 2.50/39.52 for DMSO-d6 and δH/C 1.94/1.32 for CD3CN.

### Synthetic procedures

*Preparation of compounds 10 and 11*: compound (A1) (20 mg, 0.20 mmol) and ammonium formate (12.6 mg, 0.2 mmol) were dissolved in 1 mL methanol. Palladium on carbon (8.5 mg, 0.0080 mmol) was added and the reaction mixture was stirred for 1 hour at 50 °C. The reaction was stopped by filtration through celite and evaporated to dryness. The resulting residue was purified by preparative HPLC to give 7 and 8 with purities >95% and isolated yields of ~20% and 15% respectively.

*Preparation of 5-7*: compounds (A1), 8, or 10 (50 mg, -0.05 mmol) were dissolved in 2 mL DMSO. Triethylamine (70 µL, 0.5 mmol) was added and the reaction mixture was stirred for 4 hours at 100 °C. The reaction was quenched by pouring into 100 mL water and then extracted twice with ethyl acetate. The organic phase was washed with brine, dried with Na₂SO₄ and evaporated to dryness. The resulting residue was purified by preparative HPLC to give 5-7 with purities >95% and isolated yields of ~40%.

*Derivatization of compound 5 with 4-bromophenacyl bromide:* compound 5 (2 mg, 2 µmol) was dissolved in 1 mL acetonitrile. 4 bromophenacyl bromide (0.66 mg, 2.4 µmol) and N-ethyl-N-isopropylpropan-2-amine (0.31 mg, 2.4 µmol) were added and the reaction mixture was stirred for 30 min at 20 °C. The reaction was quenched by addition of 10 mL water, leading to precipitation of the product, which was recovered by centrifugation and dissolution in acetonitrile. The crude reaction product was used for LC-MS/MS experiments to provide proof of the linear sequence of 5 by a complementary analytical method.

### Cell proliferation assays

*Cell Proliferation Assays with Chromodepsins (compounds (A1) and 2-15):* Cell line descriptions: 92.1 - uveal melanoma cell line, derived from primary tumor, harboring heterozygous GNAQ Q209L mutation. Purchased from Sigma Aldrich. MP41 (CRL-3297) - uveal melanoma cell line, derived from primary tumor, harboring heterozygous GNA11 Q209L mutation. Purchased from ATCC. A375 - skin melanoma cell line, harboring homozygous BRAF V600E mutation. Origin: Cancer Cell Line Encyclopedia (CCLE). SK-MEL-30 - skin melanoma cell line, harboring heterozygous NRAS Q61K mutation. Origin: CCLE.

Cells were cultured in 96-well plates in a total volume of 100 µL at 37°C, 5% CO2 and 95% relative humidity. All cell lines were cultured in RPMI Medium (RPMI 1640 w/o L-glutamine, Bioconcept ref. 1-41F01-1) except A375, DMEM medium (DMEM high glucose (4.5g/L), Bioconcept, ref. 1-26F01-1), and supplemented with 10% FCS (Bioconcept, ref. 2-01F30-I). Two to three thousand cells were seeded in 96-well plates per well the night before they were treated with compounds (concentration range: from 260nM to 0.036nM with 1/3 dilution steps; for 7 the concentration range was increased from 10000nM to 1.52nM with 1/3 dilution steps). Cell viability was measured by addition of 10ul of resazurin per well after 96 hours of treatment. Results were analyzed and plotted using GraphPad Prism 9.0.1.

### Assessment of cellular permeability

*Cell-based transwell assays with MDCK-low efflux monolayers:* Compounds (A1) and 2-15 were tested in MDCK cell sub-clones with low effluxt ransporter activity (MDCK-LE) in order to determine their passive permeability. The incubations were performed at a contract research organization as described previously.

*ESAPSA determination by supercritical fluid chromatography:* Compounds (A1) and 2-15 were tested by supercritical fluid chromatography (SFC) to determine their ESAPSA values. Samples were analyzed using a Chirex 3014 column (5 µM, 3x100 mm column, 100 Å pore size; Phenomenex. The starting conditions were 5% methanol containing 20 mM ammonium formate and 95% scCO₂. The gradient was increased linearly to 60% methanol containing 20 mM ammonium formate in 4.5 min.

### Plasma incubations and quantifications

*Plasma incubations and sample preparation:* Compound (A1) was spiked at 5.00 µg/mL in PBS and pooled mixed gender mouse (CD-1), cynomolgus monkey and human plasma. Samples were incubated on a Thermomixer (Eppendorf) at 37 °C while shaking at 650 rpm. Aliquots of 50 µL (n=3) were drawn from each incubation tube following 0, 0.5, 1, 2, 4, 8 and 24 h of incubation.50 µL of sample was quenched with 200 µL of acetonitrile, containing both generic internal standards, glybenclamide and warfarin, at 62.5 ng/mL. Samples were briefly vortexed and centrifuged for 20 min at 4000 rpm and 4 °C. 200 µL of the supernatant was transferred into a clean 800 µL 96-well sample collection plate.

*Quantification by LC-MS*/*MS:* LC-MS/MS analysis was conducted with a Shimadzu Nexera UPLC coupled to AB Sciex QTRAP 6500 using the SRM acquisition mode (3 µL injected). Samples were analyzed using a ACE 3 C18-PFP (3 µM, 2.1x50 mm column) at a column temperature of 65 °C. The starting conditions were 97% H2O containing 0.1% formic acid and 3% CH3CN containing 0.1% formic acid. Quantification of compound (A1) and 5 was performed by MS-MRM (electrospray in positive mode, ion spray voltage: 5000 V, source temperature: 500 °C, curtain gas: 25 psig, nebulizer gas: 60 psig, turbo ion spray gas: 40 psig, mass range: low, resolution Q1 / Q3: unit, collision gas: medium, dwell time: 12 msec). Separate calibration curves were established with calibration standards of compounds (A1) and 5 at concentrations of 10.0, 5.00, 2.50, 1.00, 0.500, 0.100, 0.050 and 0.010 µg/mL respectively. Peak integration and quantification was conducted with the AB Sciex Analyst software (v.1.7) using the Analyst Classic algorithm.

*Spiking of 5 to plasma incubation of FR:* The sample prepared following incubation in human plasma at 37 °C for 8 h was spiked with 100 ng of 5 (final conc. 6 µg/mL). Samples prior to and after spiking were analyzed using a BEH C18 column (1.7 µM, 2.1x100 mm column; Waters AG) at a flow rate of 0.5 mL/min and column temperature of 40 °C. The starting conditions were 70% water containing 0.1% formic acid and 30% CH3CN containing 0.1% formic acid. The gradient started after 0.5 min and was increased linearly to 99% CH3CN in 1.5 min. LC-MS analysis was conducted with a Waters Acquity I-Class UPLC coupled to a Waters Synapt G2-Si QTOF using the MSe acquisition mode (5 µL injected).

### Results

### Characterization of promoter insertion mutants of C. vaccinii

The strongest positive effects on production of compound (A1) were observed for the promoters *ermE** and *plpp,* followed by *J23119* and *vioP.* Promoters *nptll* and *rbs,* yielded lower titers of compound (A1) compared to the wild type control.

**Table 20. Individual and mean concentrations of Compound (A1) obtained from cultivations of promoter exchange mutants of C. vaccinii**

| Promoter | wild type (control) | *ermE** | *J23119* | *nptII* | *Plpp* | *rbs* | *vioP* |
|---|---|---|---|---|---|---|---|
| | **Concentration of (A1) (mg/L)** | | | | | | |
| Replicate 1 | 5.7 | 63.3 | 47.0 | 6.4 | 67.2 | 3.2 | 32.0 |
| Replicate 2 | 8.9 | 76.2 | 41.7 | 6.0 | 65.9 | 3.8 | 34.2 |
| Replicate 3 | 9.7 | 65.2 | 39.3 | 6.0 | 67.3 | 2.9 | 40.1 |
| **Mean concentration (mg/L)** | **8.1** | **68.2** | **42.7** | **6.1** | **66.8** | **3.3** | **35.4** |
| Standard deviation (µg/mL) | 1.7 | 5.7 | 3.2 | 0.2 | 0.6 | 0.4 | 3.4 |
| Coefficient of variation (%) | 21.3 | 8.3 | 7.5 | 3.1 | 1.0 | 11.3 | 9.7 |

### Structure Elucidation of Compounds 2-4

The chemical structure and atomic numbering of compounds (A1) and 2 with assignments of carbon atom numbers and residues are shown below.

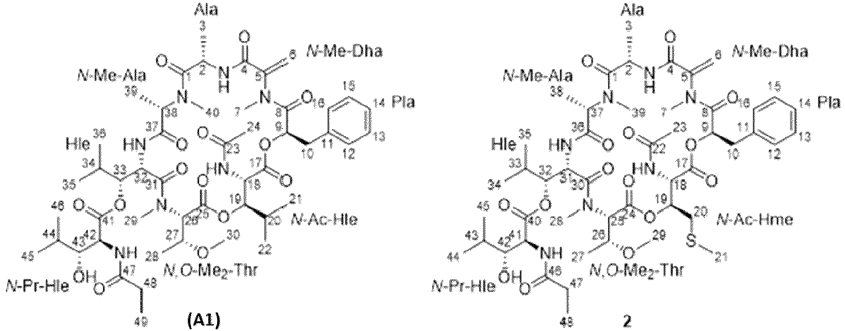

**Table 21. ¹H and ¹³C NMR spectroscopic data of Compounds (A1) and 2 in CD₃CN (¹H: 600 MHz; ¹³C 150 MHz).**

| **Compound (A1)** | | | | **2** | | | |
|---|---|---|---|---|---|---|---|
| **Residue** | **No C/H** | **δ_{C}, mult** | **δ_{H}, (mult, *J* in Hz)** | **Residue** | **No C/H** | **δ_{C}, mult** | **δ_{H}, (mult, *J* in Hz)** |
| Ala | 1 | 173.4, C | - | Ala | 1 | 173.5, C | - |
| | 2 | 46.5, CH | 4.85 (m) | | 2 | 46.6, CH | 4.85 (m) |
| | 2-NH | - | 8.36 (d, 9.4) | | 2-NH | . | 8.34 (d, 9.4) |
| | 3 | 18.4, CH₃ | 1.30 (d, 6.6) | | 3 | 18.5, CH₃ | 1.30 (d, 6.5) |
| W-Me-Dha | 4 | 164.6, C | - | *N*-Me-Dha | 4 | 164.6, C | - |
| | 5 | 146.6, C | - | | 5 | 146.7, C | - |
| | 6a | 107.4, CH₂ | 5.27 (d, 2.2) | | 6a | 107.6, CH₂ | 5.28 (d, 2.2) |
| | 6b | 107.4, CH₂ | 5.18 (d, 2.2) | | 6b | 107.6, CH₂ | 5.19 (d, 2.1) |
| | 7 | 37.0, CH₃ | 3.21 (s) | | 7 | 37.1, CH₃ | 3.21 (s) |
| Pla | 8 | 168.9, C | - | Pla | 8 | 168.9, C | - |
| | 9 | 72.9, CH | 5.32 (dd, 8.8, 3.7) 3.11 (dd, 14.9, | | 9 | 73.1, CH | 5.33 (dd, 8.8, 3.8) |
| | 10a | 37.2, CH₂ | 3.6) 2.85 (dd, 14.8, | | 10a | 37.1, CH₂ | 3.13 (m) |
| | 10b | 37.2, CH₂ | 8.8) | | 10b | 37.1, CH₂ | 2.88 m) |
| | 11 | 137.3, C | - | | 11 | 137.3, C | - |
| | 12/16 | 130.8, CH | 7.29 (m) | | 12/16 | 130.8, CH | 7.29 (m) |
| | 13/15 | 129.1, CH | 7.27 (m) | | 13/15 | 129.1, CH | 7.26 (m) |
| | 14 | 127.6, CH | 7.22 (m) | | 14 | 127.7, CH | 7.22 (m) |
| *N*-Ac-Hle | 17 | 169.9, C | - | W-Ac-Hme | 17 | 169.6, C | - |
| | 18 | 51.2, CH | 5.15 (dd, 10.1, 2.0) | | 18 | 52.1, CH | 5.11 (dd, 10.0, 1.9) |
| | 18-NH | - | 7.49 (d, 10.0) | | 18- NH | - | 7.58 (d, 9.9) |
| | 19 | 78.5, CH | 5.09 (dd, 9.9, 1.9) | | 19 | 70.8, CH | 5.48 (ddd, 9.0, 5.0, 1.8) |
| | 20 | 29.8, CH | 1.82 (m) | | 20a | 35.5, CH₂ | 2.58 (m) |
| | 21 | 19.0, CH₃ | 0.94 (d, 6.7) | | 20b | 35.5, CH₂ | 2.52 (m) |
| | 22 | 19.3, CH₃ | 0.83 (d, 6.7) | | 21 | 15.7, CH₃ | 2.05 (s) |
| | 23 | 171.7, C | - | | 22 | 171.8, C | - |
| | 24 | 22.8, CH₃ | 2.14 (s) | | 23 | 22.6, CH₃ | 2.13 (s) |
| *N,O*-Me₂-Thr | 25 | 167.4, C | - | *N,O-Me*₂-Thr | 24 | 167.2, C | - |
| | 26 | 65.4, CH | 4.08 (d, 9.9) 3.76 (dq, 10.0, | | 25 | 65.2, CH | 4.09 (d, 9.8) |
| | 27 | 73.1, CH | 5.9) | | 26 | 73.0, CH | 3.78 (dq, 9.6, 5.9) |
| | 28 | 16.6, CH₃ | 1.15 (d, 6.1) | | 27 | 16.8, CH₃ | 1.21 (d, 5.9) |
| | 29 | 29.4, CH₃ | 2.64 (s) | | 28 | 29.4, CH₃ | 2.65 (s) |
| | 30 | 57.4, CH₃ | 3.36 (s) | | 29 | 57.4, CH₃ | 3.36 (s) |
| Hle | 31 | 172.5, C | - | Hle | 30 | 172.5, C | - |
| | 32 | 47.5, CH | 5.36 (d, 9.9) | | 31 | 47.5, CH | 5.38 (d, 10.0) |
| | 32-NH | - | 6.75 (d, 9.9) | | 31- NH | - | 6.78 (d, 9.8,) |
| | 33 | 77.8, CH | 5.25 (d, 9.8) | | 32 | 78.0, CH | 5.22 (d, 9.9) |
| | 34 | 31.3, CH | 1.76 (m) | | 33 | 31.2, CH | 1.75 (m) |
| | 35 | 19.6, CH₃ | 1.04 (d, 6.6) | | 34 | 19.7, CH₃ | 1.04 (d, 6.8) |
| | 36 | 18.3, CH₃ | 0.75 (d, 6.6) | | 35 | 18.4, CH₃ | 0.75 (d, 6.6) |
| *N*-Me-Ala | 37 | 171.1, C | - | *N*-Me-Ala | 36 | 171.3, C | - |
| | 38 | 57.3, CH | 4.67 (q, 6.9) | | 37 | 57.3, CH | 4.69 (q, 6.8) |
| | 39 | 14.2, CH₃ | 1.35 (d, 6.9) | | 38 | 14.2, CH₃ | 1.35 (d, 6.9) |
| | 40 | 32.2, CH₃ | 2.82 (s) | | 39 | 32.2, CH₃ | 2.81 (s) |
| *N*-Pr-Hle | 41 | 171.3, C | - | *N*-Pr-Hle | 40 | 171.3, C | - |
| | 42 | 57.8, CH | 4.36 (dd, 7.7, 2.0) | | 41 | 57.8, CH | 4.36 (dd, 8.0, 2.1) |
| | 42-NH | - | 7.27 (m) | | 41- NH | - | 7.27 (m) |
| | 43 | 79.0, CH | 3.58 (m) | | 42 | 79.0, CH | 3.58 (m) |
| | 43-OH | - | 6.94 (d, 4.4) | | 42- OH | - | 6.97 (d, 4.5) |
| | 44 | 30.9, CH | 1.94 (m) | | 43 | 31.0, CH | 1.92 (m) |
| | 45 | 20.8, CH₃ | 1.17 (d, 6.6) | | 44 | 20.8, CH₃ | 1. 17 (d, 6.6) |
| | 46 | 18.6, CH₃ | 0.85 (d, 6.7) | | 45 | 18.6, CH₃ | 0.85 (d, 6.7) |
| | 47 | 175.4, C | - | | 46 | 175.4, C | - |
| | 48 | 29.1, CH₂ | 2.48 (m) | | 47 | 29.1, CH₂ | 2.48 (m) |
| | 49 | 10.2, CH₃ | 1.11 (t, 7.6) | | 48 | 10.2, CH₃ | 1.11 (t, 7.5) |

| | | | | | | | |
|---|---|---|---|---|---|---|---|
| Assignments are based on extensive 1D (¹H, ¹³C) and 2D (COSY, HMBC, HSQC, ROESY) NMR measurements. Ala = alanine, *N*-Me-Dha = *N*-methyldehydroalanine, Pla = 3-phenyllactic acid, *N*-Ac-Hle = *N*-acetylhydroxyleucine, *N*-Ac-Hme = *N*-acetylhydroxymethionine, *N,O*Me2-Thr = *N,O*-dimethylthreonine, Hle = hydroxyleucine, *N*-Me-Ala = *N*-methylalanine, *N*-Pr Hle = *N* propionylhydroxyleucine. | | | | | | | |

The chemical structure and atomic numbering of compounds 3 and 4 with assignments of carbon atom numbers and residues are shown below.

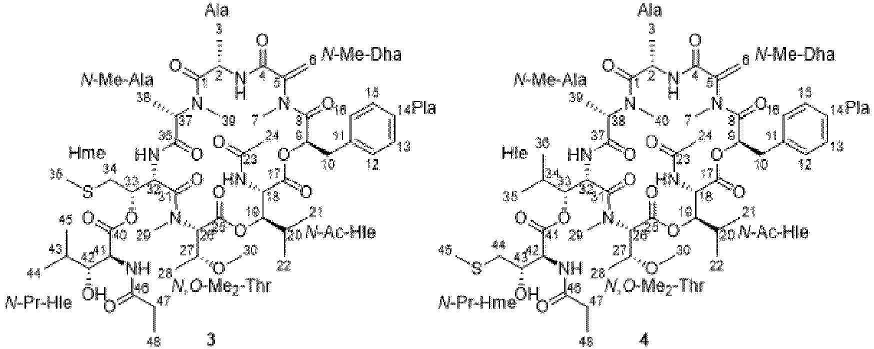

**Table 22. ¹H and ¹³C NMR spectroscopic data of 3 and 4 in CD₃CN (¹H: 600 MHz; ¹³C 150 MHz).**

| 3 | | | | 4 | | | |
|---|---|---|---|---|---|---|---|
| **Residue^{[a]}** | **No C/H** | **δ_{C}, mult** | **δ_{H}, (mult, *J* in Hz)** | **Residue** | **No C/H** | **δ_{C}, mult** | **δ_{H}, (mult, *J* in Hz**) |
| Ala | 1 | 173.4, C | - | Ala | 1 | 173.8, C | - |
| | 2 | 46.4, CH | 4.86 (dq, 9.4, 6.6) | | 2 | 46.5, CH | 4.80 (dq, 9.2, 6.5) |
| | 2-NH | - | 8.32 (d, 9.4) | | 2-NH | - | 8.54 (d, 9.2) |
| | 3 | 18.4, CH₃ | 1.29 (d, 6.4) | | 3 | 18.6, CH₃ | 1.28 (d, 6.6) |
| *N*-Me-Dha | 4 | 164.6, C | - | *N*-Me-Dha | 4 | 164.4, C | - |
| | 5 | 146.6, C | - | | 5 | 146.6, C | - |
| | 6a | 107.3, CH₂ | 5.27 (d, 2.2) | | 6a | 108.1, CH₂ | 5.62 (d, 2.2) |
| | 6b | 107.3, CH₂ | 5.18 (d, 2.1) | | 6b | 108.1, CH₂ | 5.20 (d, 2.2) |
| | 7 | 37.0, CH₃ | 3.21 (s) | | 7 | 37.2, CH₃ | 3.21 (s) |
| Pla | 8 | 168.9, C | - | Pla | 8 | 168.8, C | - |
| | 9 | 73.0, CH | 5.33 (dd, 8.8, 3.7) 3.11 (dd, 14.8, | | 9 | 73.0, CH | 5.31 (dd, 8.9, 3.7) |
| | 10a | 37.2, CH₂ | 3.8) | | 10a | 37.1, CH₂ | 3.12 (m) |
| | 10b | 37.2, CH₂ | 2.85 m) | | 10b | 37.1, CH₂ | 2.86 m) |
| | 11 | 137.3, C | - | | 11 | 137.4, C | - |
| | 12/16 | 130.8, CH | 7.29 (m) | | 12/16 | 130.8, CH | 7.29 (m) |
| | 13/15 | 129.2, CH | 7.26 (m) | | 13/15 | 129.1, CH | 7.26 (m) |
| | 14 | 127.6, CH | 7.22 (m) | | 14 | 127.6, CH | 7.22 (m) |
| *N*-Ac-Hle | 17 | 170.0, C | - | *N*-Ac-Hle | 17 | 169.9, C | - |
| | 18 | 51.2, CH | 5.15 (dd, 10.1, 1.9) | | 18 | 51.2, CH | 5.15 (dd, 10.1, 1.9) |
| | 18-NH | - | 7.49 (d, 10.1) | | 18-NH | - | 7.50 (d, 10.1) |
| | 19 | 78.4, CH | 5.09 (dd, 10.2, 1.9) | | 19 | 78.5, CH | 5.08 (dd, 10.0, 1.9) |
| | 20 | 29.9, CH | 1.81 (m) | | 20 | 29.8, CH | 1.82 (m) |
| | 21 | 19.1, CHa | 0.94 (d, 6.7) | | 21 | 19.1, CH₃ | 0.94 (d, 6.7) |
| | 22 | 19.3, CH₃ | 0.84 (m) | | 22 | 19.3, CH₃ | 0.83 (d, 6.8) |
| | 23 | 171.7, C | - | | 23 | 171.7, C | - |
| | 24 | 22.8, CH₃ | 2.13 (s) | | 24 | 22.8, CH₃ | 2.14 (s) |
| *N,O*-Me₂-Thr | 25 | 167.8, C | - | *N,O*-Me₂-Thr | 25 | 167.4, C | - |
| | 26 | 64.9, CH | 4.67 (m) | | 26 | 65.5, CH | 4.09 (m) |
| | 27 | 73.3, CH | 3.77 (dq, 9.6, 5.9) | | 27 | 73.1, CH | 3.76 (m) |
| | 28 | 16.0, CH₃ | 1.17 (m) | | 28 | 16.5, CH₃ | 1.15 (d, 6.0) |
| | 29 | 29.4, CH₃ | 2.64 (s) | | 29 | 29.5, CH₃ | 2.65 (s) |
| | 30 | 57.4, CH₃ | 3.36 (s) | | 30 | 57.4, CH₃ | 3.36 (s) |
| Hme | 31 | 172.0, C | - | Hle | 31 | 172.7, C | - |
| | 32 | 48.1, CH | 5.47 (d, 10.0) | | 32 | 47.7, CH | 5.37 (d, 9.8) |
| | 32-NH | - | 6.70 (d, 9.9) | | 32-NH | - | 6.87 (d, 9.8) |
| | 33 | 71.3, CH | 5.63 (dd, 10.2, 4.5) | | 33 | 77.8, CH | 5.26 (d, 9.4) |
| | 34a | 36.5, CH₂ | 2.59 (dd, 14.2, 10.3) | | 34 | 31.4, CH | 1.78 (m) |
| | 34b | 36.5, CH₂ | 2.44 (m) | | 35 | 19.7, CH₃ | 1.03 (d, 6.6) |
| | 35 | 16.5, CH₃ | 2.16 (s) | | 36 | 18.3, CH₃ | 0.75 (d, 6.6) |
| N-Me-Ala | 36 | 171.5, C | - | N-Me-Ala | 37 | 171.1, C | - |
| | 37 | 57.4, CH | 4.66 (m) | | 38 | 57.4, CH | 4.69 (q, 6.8) |
| | 38 | 14.2, CH₃ | 1.36 (d, 6.9) | | 39 | 14.3, CH₃ | 1.36 (d, 6.8) |
| | 39 | 32.1, CH₃ | 2.83 (s) | | 40 | 32.2, CH₃ | 2.82 (s) |
| *N*-Pr-Hle | 40 | 170.8, C | - | *N*-Pr-Hme | 41 | 170.7, C | - |
| | 41 | 57.5, CH | 4.44 (dd, 8.1, 2.1) | | 42 | 59.2, CH | 4.36 (dd, 7.8, 2.0) |
| | 41-NH | - | 7.19 (d, 8.3) | | 42-NH | - | 7.25 (m) |
| | 42 | 79.2, CH | 3.56 (m) | | 43 | 71.3, CH | 4.12 (m) |
| | 42-OH | - | 6.87 (d, 4.5) | | 43-OH | - | 7.64 (d, 4.7) |
| | 43 | 31.0, CH | 1.90 (m) | | 44a | 37.0, CH₂ | 2.88 (m) |
| | 44 | 20.7, CH₃ | 1.16 (m) | | 44b | 37.0, CH₂ | 2.65 (m) |
| | 45 | 18.6, CH₃ | 0.85 (m) | | 45 | 15.6, CH₃ | 2.14 (s) |
| | 46 | 175.3, C | - | | 46 | 175.6, C | - |
| | 47 | 29.2, CH₂ | 2.47 (m) | | 47 | 29.1, CH₂ | 2.47 (m) |
| | 48 | 10.3, CH₃ | 1.11 (t, 7.6) | | 48 | 10.2, CH₃ | 1.10 (t, 7.6) |

| | | | | | | | |
|---|---|---|---|---|---|---|---|
| Assignments are based on extensive 1D (¹H, ¹³C) and 2D (COSY, HMBC, HSQC, ROESY) NMR measurements. Ala = alanine, *N*-Me-Dha = *N*-methyldehydroalanine, Pla = 3-phenyllactic acid, *N*-Ac-Hle = *N*-acetylhydroxyleucine, *N,O*Me2-Thr = *N,O*-dimethylthreonine, Hme = hydroxymethione, Hle = hydroxyleucine, *N*-Me-Ala = *N*-methylalanine, *N*-Pr-Hle = *N*-propionylhydroxyleucine, *N*-Pr-Hme = *N-*propionylhydroxymethionine. | | | | | | | |

The chemical structure and atomic numbering of compounds 5 and 7 with assignments of carbon atom numbers and residues are shown below.

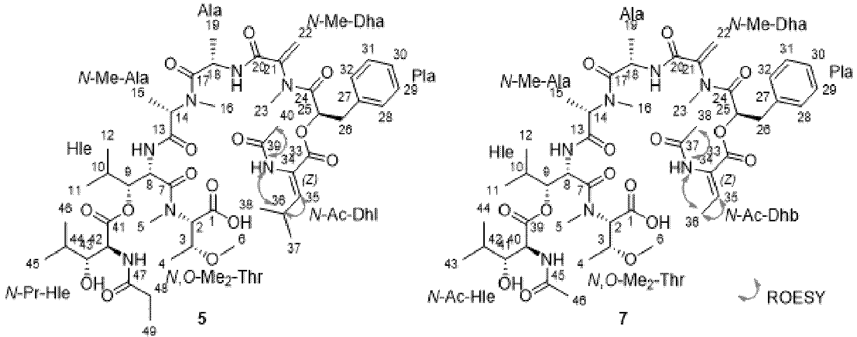

**Table 23. ¹H and ¹³C NMR spectroscopic data of Compounds 5 and 7 in DMSO-d6 (¹H: 600 MHz; ¹³C 150 MHz).**

| 5 | | | | 7 | | | |
|---|---|---|---|---|---|---|---|
| **Residue^{[a]}** | **No C/H^{[b]}** | **δ_{C}, mult^{[c]}** | **δ_{H}, (mult, *J* in Hz)^{[c]}** | **Residue^{[a]}** | **No C/H^{[b]}** | **δ_{C}, mult^{[c]}** | **δ_{H}, (mult, *J* in Hz)^{[c]}** |
| *N,O*-Me₂-Thr | 1 | 172.1, C | - | *N,O*-Me₂-Thr | 1 | 172.4, C | - |
| | 2 | 60.2, CH | 5.00 (m) | | 2 | 61.1, CH | 4.88 (m) |
| | 3 | 76.3, CH | 3.97 (m) | | 3 | 76.6, CH | 3.98 (m) |
| | 4 | 15.2, CH₃ | 0.96 (m) | | 4 | 15.6, CH₃ | 0.95 (m) |
| | 5 | 33.6, CH₃ | 3.10 (m) | | 5 | 33.7, CH₃ | 3.10 (m) |
| | 6 | 56.8, CH₃ | 3.20 (s) | | 6 | 56.7, CH₃ | 3.19 (m) |
| Hle | 7 | 170.4, C | - | Hle | 7 | 170.1, C | - |
| | 8 | 48.9, CH | 5.06 (m) | | 8 | 48.7, CH | 5.07 (m) |
| | 8-NH | - | 7.73 (m) | | 8-NH | - | 7.65 (m) |
| | 9 | 76.8, CH | 5.11 (m) | | 9 | 76.9, CH | 5.09 (m) |
| | 10 | 29.0, CH | 1.83 (m) | | 10 | 29.0, CH | 1.83 (m) |
| | 11 | 19.7, CH₃ | 0.86 (m) | | 11 | 19.8, CH₃ | 0.85 (m) |
| | 12 | 16.3, CH₃ | 0.85 (m) | | 12 | 16.5, CH₃ | 0.85 (m) |
| *N-*Me-Ala | 13 | 170.8, C | | *N*-Me-Ala | 13 | 170.8, C | |
| | 14 | 52.0, CH | 4.99 (m) | | 14 | 52.1, CH | 4.98 (m) |
| | 15 | 14.3, CH₃ | 1.20 (m) | | 15 | 14.3, CH₃ | 1.20 (m) |
| | 16 | 30.5, CH₃ | 2.81 (m) | | 16 | 30.6, CH₃ | 2.82 (m) |
| Ala | 17 | 172.1, C | - | Ala | 17 | 172.2, C | - |
| | 18 | 45.9, CH | 4.82 (m) | | 18 | 45.9, CH | 4.82 (m) |
| | 18-NH | - | 8.50 (m) | | 18- NH | - | 8.52 (m) |
| | 19 | 16.7, CH₃ | 1.24 (m) | | 19 | 16.8, CH₃ | 1.24 (m) |
| *N*-Me-Dha | 20 | 162.3, C | - | *N*-Me-Dha | 20 | 162.4, C | - |
| | 21 | 141.9, C | - | | 21 | 141.8, C | - |
| | 22a | 122.6, CH₂ | 6.25 (m) | | 22a | 122.6, CH₂ | 6.27 (m) |
| | 22b | 122.6, CH₂ | 5.74 (m) | | 22b | 122.6, CH₂ | 5.77 (m) |
| | 23 | 35.5, CH₃ | 2.93 (m) | | 23 | 35.6, CH₃ | 2.93 (m) |
| Pla | 24 | 168.3, C | - | Pla | 24 | 168.4, C | - |
| | 25 | 71.9, CH | 5.18 (m) | | 25 | 72.0, CH | 5.20 (m) |
| | 26a | 36.7, CH₂ | 3.09 (m) | | 26a | 36.6, CH₂ | 3.11 (m) |
| | 26b | 36.7, CH₂ | 2.89 (m) | | 26b | 36.6, CH₂ | 2.89 (m) |
| | 27 | 136.8 | - | | 27 | 137.0 | - |
| | 28/32 | 129.2, CH | 7.15 (m) | | 28/32 | 129.1, CH | 7.16 (m) |
| | 29/31 | 128.3, CH | 7.27 (m) | | 29/31 | 128.5, CH | 7.27 (m) |
| | 30 | 126.7, CH | 7.21 (m) | | 30 | 126.8, CH | 7.21 (m) |
| *N*-Ac-Dhl | 33 | 164.0, C | - | *N*-Ac-Dhb | 33 | 163.7, C | - |
| | 34 | 124.8, C | - | | 34 | 127.6, C | - |
| | 34-NH | - | 9.16 (m) | | 34- NH | - | 9.20 (m) |
| | 35 | 144.0, CH | 6.18 (d, 10.0) | | 35 | 133.2, CH | 6.52 (q, 7.2) |
| | 36 | 26.7, CH | 2.52 (m) | | 36 | 13.7, CH₃ | 1.66 (d, 7.1) |
| | 37 | 21.5, CH₃ | 0.95 (m) | | 37 | 168.4, C | - |
| | 38 | 21.6, CH₃ | 0.93 (m) | | 38 | 22.3, CH₃ | 1.88 (m) |
| | 39 | 168.7, C | - | | | | |
| | 40 | 22.2, CH₃ | 1.85 (s) | | | | |
| *N-*Pr-Hle | 41 | 171.1, C | - | *N*-Ac-Hle | 39 | 171.2, C | - |
| | 42 | 54.7, CH | 4.59 (m) | | 40 | 54.9, CH | 4.57 (m) |
| | 42-NH | - | 7.71 (m) | | 40- NH | - | 7.92 (m) |
| | 43 | 76.6, CH | 3.54 (m) | | 41 | 76.7, CH | 3.55 (m) |
| | 43-OH | - | n.d. ^{[e]} | | 41- OH | - | n.d. ^{[e]} |
| | 44 | 30.8 | 1.54 (m) | | 42 | 30.9 | 1.56 (m) |
| | 45 | 19.3 | 0.92 (m) | | 43 | 19.4 | 0.93 (m) |
| | 46 | 18.9 | 0.80 (m) | | 44 | 19.0 | 0.79 (m) |
| | 47 | 173.4, C | - | | 45 | 169.9, C | - |
| | 48 | 28.4, CH₂ | 2.18 (m) | | 46 | 22.3, CH₃ | 1.88 (m) |
| | 49 | 10.0, CH₃ | 0.97 (m) | | | | |

| | | | | | | | |
|---|---|---|---|---|---|---|---|
| Assignments are based on extensive 1D (¹H, ¹³C) and 2D (COSY, HMBC, HSQC, ROESY) NMR measurements. *N,O*Me2-Thr = *N*,*O*-dimethylthreonine, Hle = hydroxyleucine, *N*-Me-Ala = *N*-methylalanine, Ala = alanine, *N-*Me-Dha = *N*-methyldehydroalanine, Pla = 3-phenyllactic acid, *N*-Ac-Dhl = *N-*acetyldehydroleucine, *N*-Ac-Dhb = *N*-acetyldehydrobutyrine *N*-Pr-Hle = *N-*propionylhydroxyleucine, *N*-Ac-Hle = *N*-acetylhydroxyleucine. [d] n.d. = not detectable. | | | | | | | |

### Biosynthetic proposals for 2-4

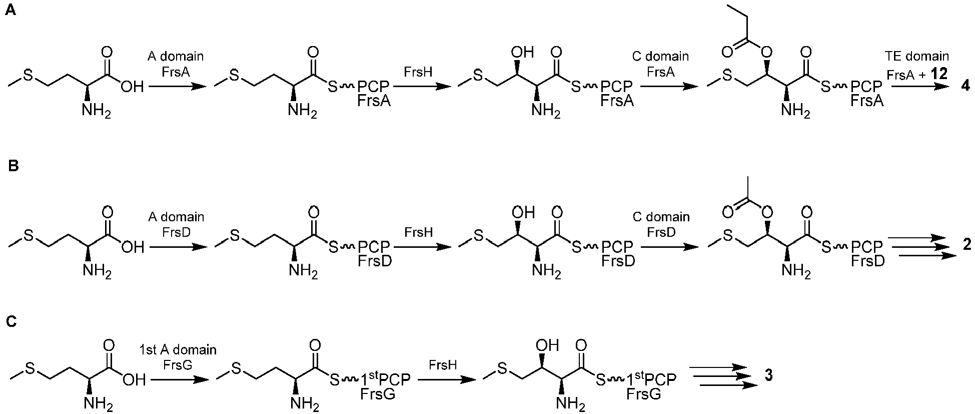

### Effects of chromodepsins on cell proliferation

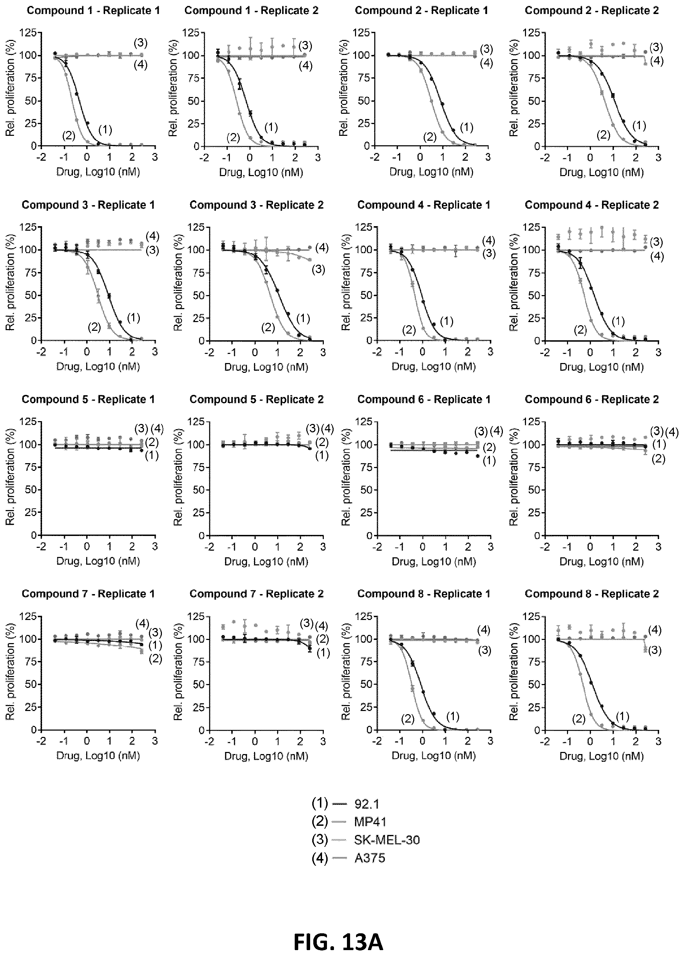

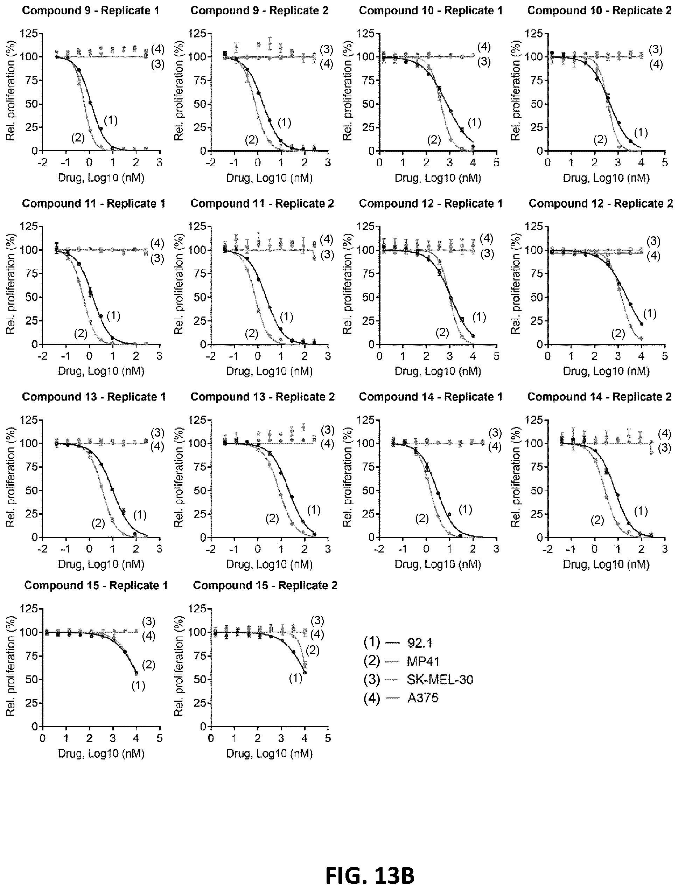

The effects of tested compounds on proliferation of Gq or G11 mutant UM cell lines and Gq or G11 wild type skin melanoma cell lines are shown in **FIGs. 13A-13B****.**

**Table 24. GI₅₀s of compounds (A1) and 2-15 on Gq or G11 mutant UM cell lines and Gq or G11 wild type skin melanoma cell lines**

| **Compound** | **GI₅₀ 92.1 [nM]** | | | **GI₅₀ MP41 [nM]** | | | **GI₅₀ SK-MEL-30 [nM]** | | | **GI₅₀ A375 [nM]** | | |
|---|---|---|---|---|---|---|---|---|---|---|---|---|
| | Rep. 1 | Rep. 2 | Average | Rep. 1 | Rep. 2 | Average | Rep. 1 | Rep. 2 | Average | Rep. 1 | Rep. 2 | Average |
| **A1** | 0.46 | 0.69 | 0.6 | 0.22 | 0.27 | 0.2 | >260 | >260 | >260 | >260 | >260 | >260 |
| **2** | 8.00 | 11.61 | 9.8 | 3.17 | 4.30 | 3.7 | >260 | >260 | >260 | >260 | >260 | >260 |
| **3** | 9.05 | 11.7 | 10.4 | 3.12 | 4.89 | 4.0 | >260 | >260 | >260 | >260 | >260 | >260 |
| **4** | 0.91 | 1.5 | 1.2 | 0.45 | 0.53 | 0.5 | >260 | >260 | >260 | >260 | >260 | >260 |
| **5** | >260 | >260 | >260 | >260 | >260 | >260 | >260 | >260 | >260 | >260 | >260 | >260 |
| **6** | >10000 | >10000 | >10000 | >10000 | >10000 | >10000 | >10000 | >10000 | >10000 | >10000 | >10000 | >10000 |
| **7** | >260 | >260 | >260 | >260 | >260 | >260 | >260 | >260 | >260 | >260 | >260 | >260 |
| **8** | 0.82 | 1.24 | 1.0 | 0.33 | 0.46 | 0.4 | >260 | >260 | >260 | >260 | >260 | >260 |
| **9** | 1.20 | 1.69 | 1.4 | 0.60 | 0.78 | 0.7 | >260 | >260 | >260 | >260 | >260 | >260 |
| **10** | 760.5 | 453.1 | 607 | 419.2 | 371.8 | 396+ | >10000 | >10000 | >10000 | >10000 | >10000 | >10000 |
| **11** | 1.40 | 2.24 | 1.8 | 0.54 | 0.78 | 0.7 | >260 | >260 | >260 | >260 | >260 | >260 |
| **12** | 1133 | 2350 | 1742 | 993.8 | 1350 | 1180 | >10000 | >10000 | >10000 | >10000 | >10000 | >10000 |
| **13** | 10.10 | 21.35 | 15.7 | 3.60 | 8.31 | 6.0 | >260 | >260 | >260 | >260 | >260 | >260 |
| **14** | 2.95 | 7.53 | 5.2 | 1.50 | 2.81 | 2.2 | >260 | >260 | >260 | >260 | >260 | >260 |
| **15** | >10000 | >10000 | >10000 | >10000 | >10000 | >10000 | >10000 | >10000 | >10000 | >10000 | >10000 | >10000 |

### Assessment of passive cellular permeability

**Table 25. Cellular permeabilities of compounds (A1)-15 in MDCK-LE monolayers and values from modified EPSA method**

| **Compound** | Pₐₚₚ A-B (x10 -6) [cm s-1] | % Fraction absorbed | % Recovery | EPSA [A^2] | calculated tPSA [A^2] |
|---|---|---|---|---|---|
| **A1** | 4.06 | 59.7 | 65 | 94 | 286 |
| **2** | 6.35 | 72.6 | 72 | 104 | 286 |
| **3** | 12.2 | 86.1 | 90 | 98 | 286 |
| **4** | 1.71 | 32.5 | 75 | 104 | 286 |
| **5** | 0.27 | 4.2 | 86 | 229 | 297 |
| **6** | 0.17 | 2.4 | 90 | 230 | 297 |
| **7** | 0.37 | 6.1 | 90 | not tested | 297 |
| **8** | 5.84 | 70.4 | 78 | 94 | 286 |
| **9** | not tested | not tested | not tested | not tested | 286 |
| **10** | 2.03 | 37.6 | 77 | not tested | 286 |
| **11** | 8.5 | 79.5 | 76 | 95 | 286 |
| **12** | 0.55 | 9.9 | 83 | 106 | 230 |
| **13** | 2.69 | 46.5 | 85 | 95 | 286 |
| **14** | 3.32 | 53.3 | 85 | 95 | 286 |
| **15** | 0.79 | 15 | 90 | 109 | 230 |

### Quantification of compounds (A1) and 5 in human plasma incubations

**Table 26. Individual and mean concentrations of Compound (A1) and Compound 5 following incubations in human plasma for up to 24 h at 37°C.**

| Incubation time | 0 h | 0.5 h | 1 h | 2 h | 4 h | 8 h | 24 h |
|---|---|---|---|---|---|---|---|
| | **Concentration of (A1) (µg/mL)** | | | | | | |
| Aliquot 1 | 4.62 | 5.15 | 4.98 | 4.79 | 4.14 | 3.26 | 1.35 |
| Aliquot 2 | 4.35 | 5.10 | 4.58 | 5.13 | 4.35 | 3.66 | 1.43 |
| Aliquot 3 | 4.75 | 5.10 | 5.02 | 4.68 | 4.35 | 3.60 | 1.29 |
| **Mean concentration (µg/mL)** | **4.57** | **5.12** | **4.86** | **4.87** | **4.28** | **3.51** | **1.36** |
| Standard deviation (µg/mL) | 0.20 | 0.03 | 0.24 | 0.23 | 0.12 | 0.22 | 0.07 |
| Coefficient of variation (%) | 4.5 | 0.6 | 5.0 | 4.8 | 2.8 | 6.2 | 5.2 |
| Percentage of theoretical initial concentration | 91.4 | 102.4 | 97.2 | 97.4 | 85.6 | 70.2 | 27.2 |

| | **Concentration of 5 (µg/mL)** | | | | | | |
|---|---|---|---|---|---|---|---|
| Aliquot 1 | BLQ | 0.0613 | 0.117 | 0.255 | 0.615 | 1.37 | 3.14 |
| Aliquot 2 | BLQ | 0.0673 | 0.123 | 0.289 | 0.647 | 1.48 | 2.82 |
| Aliquot 3 | BLQ | 0.0671 | 0.142 | 0.317 | 0.677 | 1.57 | 3.14 |
| **Mean concentration (µg/mL)** | **-** | **0.0652** | **0.127** | **0.287** | **0.646** | **1.47** | **3.03** |
| Standard deviation (µg/mL) | - | 0.0034 | 0.013 | 0.031 | 0.031 | 0.10 | 0.18 |
| CV (%) | - | 5.2 | 10.2 | 10.8 | 4.8 | 6.8 | 6.1 |
| Sum compounds **(A1)** and **5** (µg/mL) | 4.57 | 5.18 | 4.99 | 5.15 | 4.93 | 4.98 | 4.39 |
| Percentage of theoretical initial compound **(A1)** concentration | 91.4 | 103.6 | 99.8 | 103 | 98.6 | 99.6 | 87.8 |

### Example 4: Development of Formulations for Antibody Drug Conjugate

Seven different formulations for an exemplary PMEL17 antibody-drug (GNAQ and/or GNA11 inhibitor) conjugate were tested **(Table 27).**

**Table 27. Exemplary Antibody Drug Conjugate Formulations**

| **Components** | **F1** | **F2** | **F3** | **F4** | **F5** | **F6** | **F7** |
|---|---|---|---|---|---|---|---|
| ADC (mg/mL) | 20 | 20 | 20 | 10 | 20 | 20 | 20 |
| Histidine (mM) | 20 | | | | | | |
| Sucrose (mM) | 240 | | | | | | |
| Polysorbate 20 (%) | 0.02 | 0.02 | 0.04 | 0.02 | 0.02 | 0.02 | 0.02 |
| pH | 5.0 | 5.5 | 5.5 | 5.5 | 6.0 | 5.2 | 5.8 |

Once formulated, the formulations were subjected to different storage conditions to determine stability. The lyophilisate in vial (LYVI) study tested the formulated ADC at 5±3°C, 25±2°C, or 40±2°C over 0, 1, 2, or 4 months **(Table 28).** The liquid in vial (LIVI) study tested the formations at 5±3°C, 25±2°C/60±5% relative humidity (RH), or 40±2°C/75±5% RH over 0 or 1 month **(Table 29).**

**Table 28. LYVI Stability Study**

| **Storage** | **Months** | | | |
|---|---|---|---|---|
| | 0 | 1 | 2 | 4 |
| 5±3°C | | x | x | x |
| 25±2°C | x | x | x | x |
| 40±2°C | | x | | |

**Table 29. LIVI Stability Study**

| **Storage** | **Months** | |
|---|---|---|
| | 0 | 1 |
| 5±3°C | | x |
| 25±2°C/60±5% RH | x | x |
| 40±2°C/75±5% RH | | x |

### Analysis

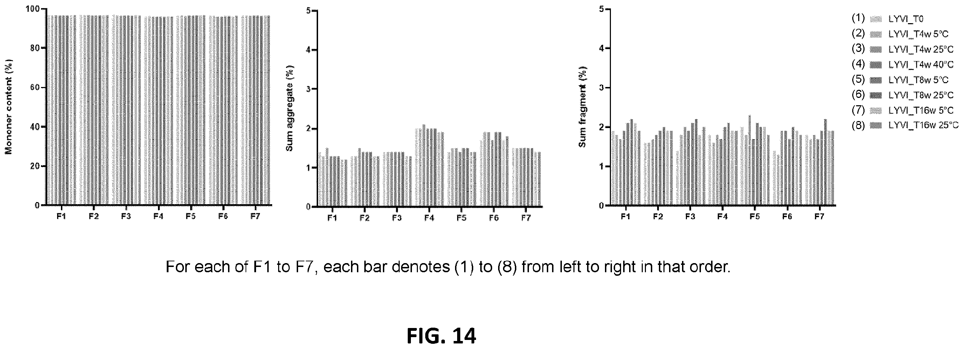

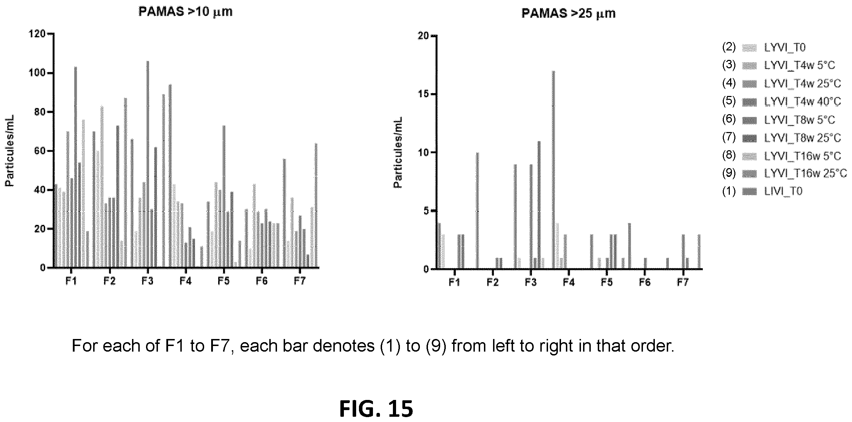

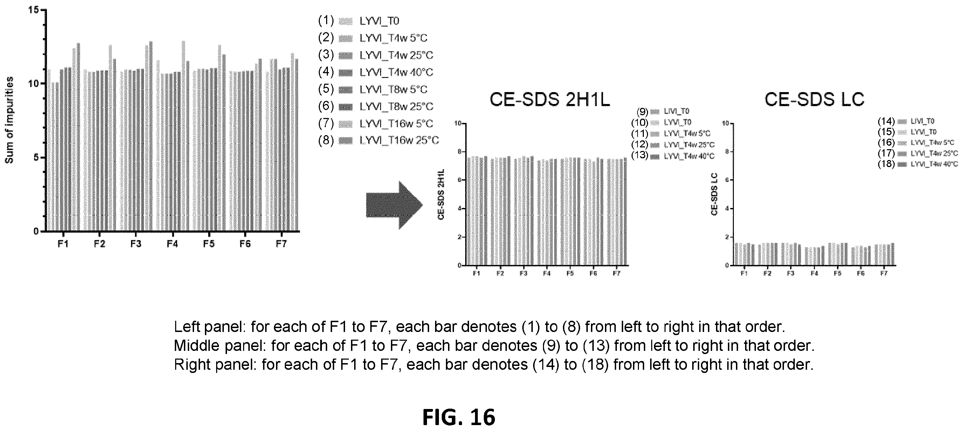

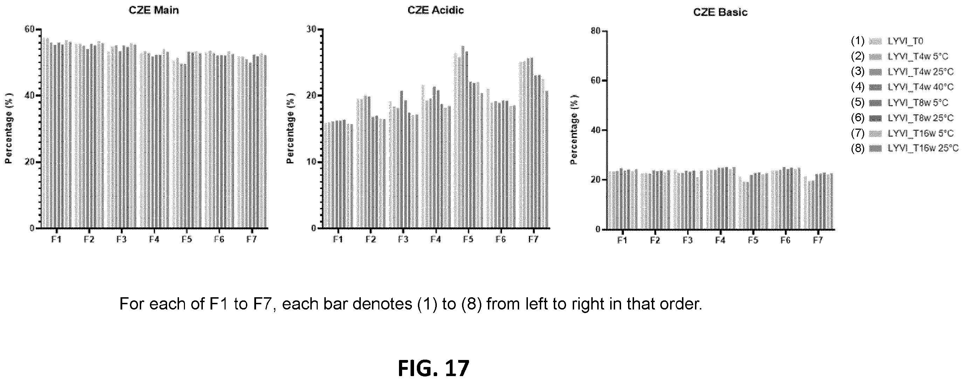

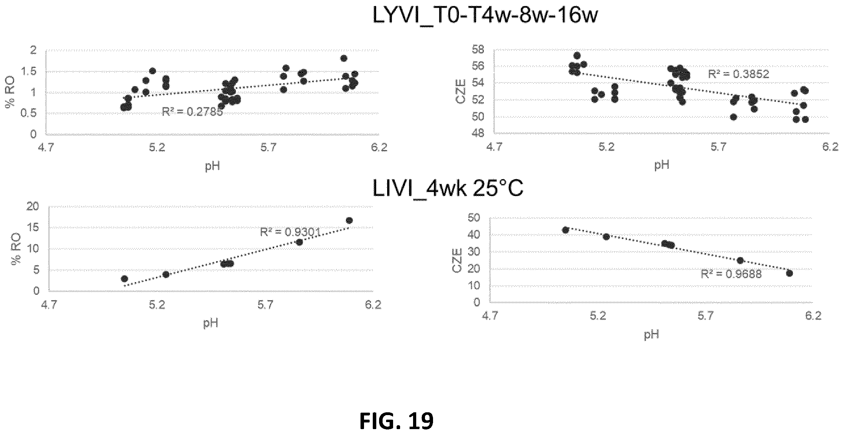

The formulations were then analyzed using size exclusion chromatography (SEC) after 0, 1 month, 2 months, or 4 months at various storage conditions. Between 0 months and 4 months, degradation of less than 2% of the monomer content was observed, and it was determined that aggregation of the ADC was one of the main degradation mechanisms **(****FIG. 14****).** The samples were also analyzed using light obscuration to detect an increase in sub-visible particles. This analysis found that there was no major increase in sub-visible particles over time **(****FIG. 15****).** Capillary electrophoresis showed impurities originating from the PMEL17 antibody **(****FIG. 16****).** Using capillary zone electrophoresis, it was found that there was a high level of charge variants in the different formulations **(****FIG. 17****),** and for the LIVI the capillary zone electrophoresis is correlated with a lower pH **(****FIG. 19****).**

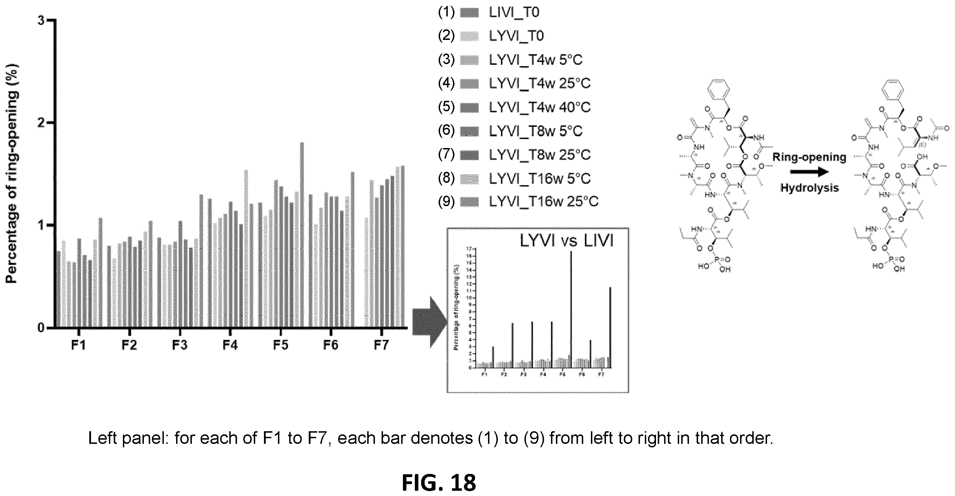

It was found that the LYVI allows for stabilization of the payload by preventing degradation through ring opening through hydrolysis of the payload **(****FIG. 18****),** and for LIVI the ring opening is correlated with a high pH **(****FIG. 19****).**

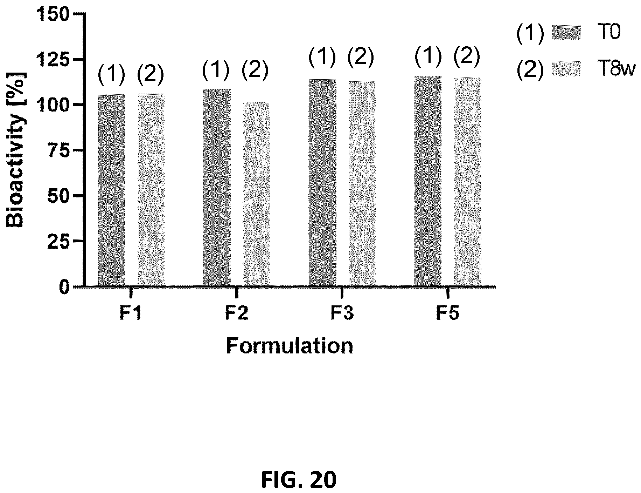

The formulations were assayed to test potency after a two-month storage period. The assay indicated that there was no potency decay after two months for formulations F1, F2, F3, and F5 **(****FIG. 20****).**

ADC lyophilization cycle feasibility testing was performed using 20R topline vials.

### Example 5: Evaluation of Compound (E) in Cynomolgus Monkeys

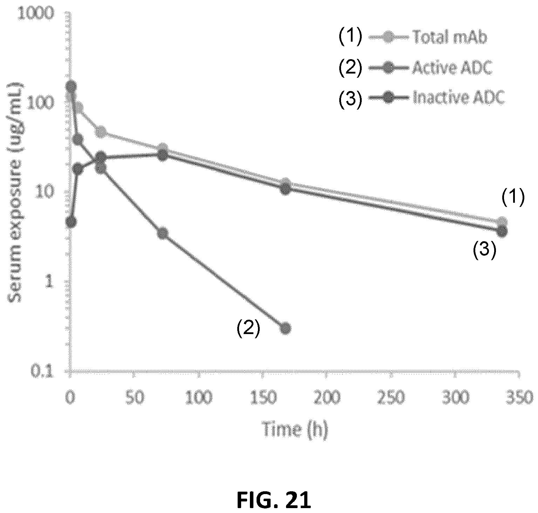

An exemplary ADC, compound (E), was administered intravenously to cynomolgus monkeys, either as a single dose or as repeated doses in the dose range of 3 to 75 mg/kg. Total antibody (tmAb) levels were measured; tmAb exposure decreased over time with an elimination half-life of roughly 100 hours. The exposure to the total active ADC (tACDa; ADC carrying at least one active payload) decreased faster, with a half-life of approximately 40 hours, whereas total inactive ADC (tACDi; ADC carrying at least one inactive payload) increased to a maximum around 72 hours after dosing, after which it decreased roughly in parallel to the tmAb **(****FIG. 21****).** The exposure profiles of tACDa and tACDi aligned with the concentration-time profiles of conjugated active and inactive payload measured by a papain release assay (PRA).

Compound (E) showed roughly dose proportional exposure between 3 and 75 mg/kg. Only minor accumulations for tmAb and tADCi were observed was after three Q2W doses (1.0-1.4), whereas tADCa did not accumulate. Plasma exposure of free active payload was only detected at very low levels (<1 ng/mL) in all samples, even at the highest tested compound (E) dose of 75 mg/kg. Findings were consistent with *in vitro* assessment of plasma stability. No target-mediated drug disposition was observed.

The nonclinical safety profile of compound (E) was evaluated in cynomolgus monkeys both in a non-GLP dose-range-finding (DRF) study of 6 weeks (4 doses) and the pivotal GLP-compliant 13-week toxicity study (7 doses). All in-life observations as well as most clinical and anatomic pathology observations can be related to the pharmacology of Compound (A1) (Gq/11 protein inhibition). All findings were minimal to mild in severity and showed full or partial reversibility within the 3-week recovery period at all doses levels tested; therefore, the highest non-severely toxic dose (HNSTD) was set to the highest tested dose of 75 mg/kg Q2W.

Two retrospective analyses of multiple ADCs found that selecting a starting dose using 1/6th of the BSA-based converted HNSTD in cynomolgus monkeys generally resulted in an acceptable balance of evaluation of safety with an effective dose-escalation in Phase 1 trials. This approach also avoids treating patients at sub-therapeutic dose levels and allows for an efficient determination of the MTD. Based on the above, the starting dose for compound (E) was calculated by BSA conversion of 75 mg/kg (HNSTD) and applying a safety factor of 6, yielding a starting dose of 4 mg/kg.

Based on the predicted human PK at 4 mg/kg, the exposure margins achieved at HNSTD in cynomolgus monkey relative to the proposed starting dose (4 mg/kg Q2W) and the predicted efficacious dose range (2-15 mg/kg Q2W) are shown in **Table 30.**

**Table 30. Compound (E) tmAb and active conjugated payload exposure in cynomolgus monkey relative to the predicted human exposure at the starting dose (4 mg/kg) and predicted efficacious dose range (2-15 mg/kg).**

| | **HNSTD in cynomolgus monkey GLP study** | | **Starting dose in patients** | | **Predicted efficacious dose range in patients*** | |
|---|---|---|---|---|---|---|
| **Dose (mg/kg)** | **75** | | **4** | | **2-15** | |
| **Analyte** | **tmAb** | **Conjugated payload**** | **tmAb** | **Conjugated payload**** | **tmAb** | **Conjugated payload**** |
| **Cmax (µg/mL)** | **3790** | **25.1** | **159** | **2.1** | **80-591** | **1.1-7.8** |
| **AUC336h (µg*h/mL)** | **328000** | **831** | **8900** | **54** | **4480-32900** | **27-198** |
| **Cmax exposure multiple in monkey compared to patients** | | | **24** | **12** | **47-6.4** | **24-3.2** |
| **AUC336h exposure multiple in monkey compared to patients** | | | **37** | **15** | **73-10** | **31-4.2** |

| | | | | | | |
|---|---|---|---|---|---|---|
| *This dose range represents the range of minimum efficacious doses (MED), whereas the lowest dose (2 mg/kg) reflects the predicted MED when applying the PK-efficacy model to the most sensitive xenograft tumor model 92.1, and the highest dose (15 mg/kg) the predicted MED when using the least sensitive preclinical tumor model 30201-HX. ** payload = initially released phosphorylated form of active payload compound (A1) | | | | | | |

Exposures represent steady state exposures in cynomolgus monkey (mean on Day 71) and predicted steady state exposures in human. The predicted half-life in human is 160 h for compound (E) total mAb, and approximately 44 h for the conjugated active payload.

Based on the predicted human PK and computational models of tumor growth inhibition, exposures achieved between 2 and 15 mg/kg, administered Q2W, are predicted to achieve anti-tumor activity in patients. These dosing regimens were chosen for the study described in Example 5. More or less frequent dosing regimens (*e*.*g*., QW or Q4W) may be used, *e.g.,* based on emerging clinical data.

### Example 6: A Study of Compound (E) in Patients with Metastatic Uveal Melanoma (MUM) and Other GNAQ/11 Mutant Melanomas

This Example describes a phase I/II study of an exemplary ADC, compound (E), in patients with metastatic uveal melanoma (MUM) and other GNAQ/11 mutant melanomas. The purpose of this study is to characterize the safety, tolerability, and anti-tumor activity of compound (E) as a single agent in patients with MUM and other melanomas harboring GNAQ/11 mutations.

### Objectives and Endpoints

The primary objectives of phase I are to characterize the safety and tolerability and to identify the MTD and/or RD and regimen for additional studies of compound (E) as a single agent. The endpoints for the primary objectives are determining the incidence and severity of dose limiting toxicities (DLTs) during the first 28 days of treatment and incidence and severity of adverse events (AEs) and serious adverse events (SAEs), including changes in laboratory values, electrocardiograms (ECGs), and vital signs. Tolerability as an endpoint is determined by looking at the frequency of dose interruptions, dose reductions, and discontinuations.

The secondary objectives of Phase I are to: 1) characterize the pharmacokinetics of compound (E) as a single agent; 2) assess the immunogenicity of compound (E) as a single agent; and 3) evaluate the preliminary anti-tumor activity of compound (E) as a single agent. The endpoints for the secondary objectives are determine the PK parameters for total mAb, conjugated active and inactive payload, and unconjugated active payloads (*e*.*g*., AUC, Cₘₐₓ, CL, and half life). The prevalence and incidence of antidrug antibodies (ADA), as well as the best overall response (BOR) and overall response rate (ORR) per RECIST v 1.1 are determined as endpoints for the secondary objections of Phase I. The ORR is defined as the proportion of patients with BOR of either confirmed complete response (CR) or confirmed partial response (PR), as per central assessment according to RECIST 1.1, recorded from starting study treatment until PD, death, start of new therapy, withdrawal of consent, or the end of the study, whichever comes first.

The primary objective of Phase II of the study is to evaluate the anti-tumor activity of compound (E) as a single agent, with the ORR per RECIST 1.1 as the endpoint.

The secondary objectives of Phase II are to: 1) further evaluate the anti-tumor activity of compound (E) as a single agent; 2) evaluate overall survival of compound (E) as a single agent; 3) further characterize the safety and tolerability of compound (E) as a single agent; and 4) further characterize the pharmacokinetics of compound (E) as a single agent. The objectives are evaluated by determining the duration of response (DoR), progression free survival (PFS) and DCR per RECIST v1.1. Overall survival is also determined. Safety endpoints include incidence and severity of AEs and SAEs, including changes in laboratory values, ECGs, and vital signs. Tolerability endpoints is determined by looking at the frequency of dose interruptions, dose reductions, and discontinuations. PK parameters include total mAb, conjugated active and inactive payload, and unconjugated active payloads (*e*.*g*., AUC, Cₘₐₓ, CL, and half life).

The exploratory objective of Phase I is to evaluate the preliminary anti-tumor activity of compound (E) as a single agent. The exploratory objectives of Phase I/II are to: 1) assess the presence of ADC target in the tumor; 2) assess the pharmacodynamics and PK/PD relationship in the tumor and to quantify the extent of target inhibition in the tumor; 3) assess the pharmacodynamics (PD) effect of compound (E); 4) characterize the immunomodulatory effects of compound (E) as a single agent; 5) assess genetic characteristics and prognostic markers of cell free DNA (cfDNA) relative to potential predictive markers of efficacy and/or resistance in tumor and cfDNA; and 6) assess changes in transcriptomic profiles following treatment in tumor (*e*.*g*., define GNAQ/11 gene expression signature for disease response, monitoring, and resistance in the tumor. The endpoints for the exploratory objections include assessment of PFS per RESCIST v1.1 and OS; PMEL protein expression in the tumor and changes at baseline, during treatment, and at the end of treatment; determining the correlation between exposure of compound (E) related compounds and/or the derived PK parameters; and biomarkers (*e*.*g*., changes in DUSP6 and RASGRP3 mRNA and pERK protein expression in the tumor at baseline, during treatment, and at the end of treatment); changes from baseline of PD markers in tumor tissue (*e*.*g*., DUSP6, RASGRP3, and pERK); and changes from baseline in immune cell markers in tumor samples (*e*.*g*., CD8, PD-L1). Baseline and post-baseline genetic alterations in cancer related genes and/or changes from baseline of cfDNA levels, and anti-tumor endpoints per RECIST v 1.1 and changes from baseline in gene expression profiles in tumor samples are also observed.

### Study Design

There are two parts to this study: a phase I, dose escalation part followed by a phase II part. Dose escalation is conducted in adult patients with MUM and other melanomas harboring GNAQ/11 mutations. Once the maximum tolerated dose (MTD) and/or recommended doses (RD(s)) is determined in the dose escalation part, the study may continue with a phase II part. The phase II part is conducted in two groups of patients with MUM, a prior tebentafusp-treated group and a tebentafusp-naive group. In addition to MUM, a third group of patients with non-uveal GNAQ/11 mutant melanomas may also be explored.

The escalation part of the study is conducted in patients with MUM and other melanomas harboring GNAQ/11 mutations. All dose escalation decisions are guided by an adaptive Bayesian logistic regression model (BLRM) following the Escalation with Overdose Control (EWOC) principle. Approximately A minimum of 14 patients may be required during dose escalation to define the MTD. The RD(s) refers to the recommended regimen, dose(s) and schedule(s) for further exploration. The safety (including the dose-DLT relationship) and tolerability of study treatment are assessed, and regimen(s) including dose(s) and schedule(s) is identified for use in the phase II part based on the review of all available data including PK, PD, and preliminary anti-tumor activity.

To begin, compound (E) single agent is dosed every two weeks (Q2W) during a 28-day cycle. Based on emerging data, alternative dosing regimens (*e*.*g*., less or more frequent dosing) may be implemented during the study if supported by available non-clinical and clinical data including preliminary PK, PD, safety and efficacy findings. Once an MTD/RD(s) has been identified the study continues in the phase II part in order to assess the anti-tumor activity of compound (E) as single agent in defined patient populations and further characterize the safety, PK, and PD of the study treatment. The enrollment includes both the prior tebentafusp treated and tebentafusp naive groups. The enrollment may also include the non-uveal GNAQ/11 mutant melanoma group, if explored.

### Study Population

To be included in the study, patients must meet all of the following criteria:
1. Be ≥18 years of age at the time of informed consent for the dose escalation study. For Phase II, patients ≥12 years of age at the time of informed consent. Patients must have a minimum weight of 40 kg.
2. ECOG performance status ≤ 1 for patients ≥ 18 years of age; Karnofsky performance status ≥ 70 for patients ≥ 16 and < 18 years of age; Lansky performance status ≥ 70 for patients ≥ 12 and < 16 years of age
3. Measurable disease at the time of enrollment, one lesion that can be accurately measured in at least one dimension (longest diameter to be recorded) as > 20 mm with conventional techniques or as >10 mm with CT scan.
4. Patients must be suitable and willing to undergo study required biopsies according to the treating institution's own guidelines and requirements.

For all patients in the dose escalation study (Phase I):
1. MUM patients: uveal melanoma with histologically or cytologically confirmed metastatic disease. Patient must be either treatment naive or have received any number of prior lines and progressed on most recent therapy.
2. Non-MUM patients: advanced cutaneous or mucosal melanoma with histologically or cytologically confirmed metastatic disease that has progressed following standard therapies or that has no satisfactory alternative therapies and has evidence of GNAQ/11 mutation based on local data.

For patients participating in Phase II:
1. Tebentafusp naive group: Diagnosis of uveal melanoma with histologically or cytologically confirmed metastatic disease that has progressed following standard therapies or that has no satisfactory alternative therapies.
2. Tebentafusp pre-treated group: Diagnosis of uveal melanoma with histologically or cytologically confirmed metastatic disease. Patients must be previously treated with tebentafusp and have progressed.
3. Non-MUM patients: patients with diagnosis of cutaneous or mucosal melanomas harboring GNAQ/11 mutations based on local data, with histologically or cytologically confirmed metastatic disease that has progressed following standard therapies or that has no satisfactory alternative therapies.

Patients meeting any of the following criteria are not eligible for inclusion in the study:
1. Malignant disease, other than that being treated in this study.
2. Active brain metastases, *i.e.,* symptomatic brain metastases or known leptomeningeal disease.
3. Evidence of active bleeding or bleeding diathesis or significant coagulopathy (including familial) or a medical condition requiring long term systemic anticoagulation that would interfere with biopsies.
4. History of anaphylactic or other severe hypersensitivity / infusion reactions to ADCs or monoclonal antibodies, which in the opinion of the investigator may pose an increased risk of serious infusion reaction.
5. Treatment with any of the following anti-cancer therapies prior to the first dose of study treatment within the stated timeframes:
   a. ≤ 2 weeks for fluoropyrimidine therapy
   b. ≤ 4 weeks for radiation therapy or limited field radiation for palliation within ≤ 2 weeks prior to the first dose of study treatment.
   c. ≤ 4 weeks or ≤ 5 half-lives (whichever is shorter) for chemotherapy or biological therapy (including monoclonal antibodies) or continuous or intermittent small molecule therapeutics or any other investigational agent.
   d. ≤ 6 weeks for cytotoxic agents with major delayed toxicities, such as nitrosoureas and mitomycin C.
   e. ≤ 4 weeks for immuno-oncologic therapy, such as CTLA-4, PD-1, or PD-L1 antagonists.
   f. ≤ 4 weeks for tebentafusp treatment
6. Clinically significant and / or uncontrolled heart disease such as congestive heart failure requiring treatment (NYHA grade ≥ 2) or clinically significant arrhythmia despite medical treatment.

Other inclusion/exclusion criteria may apply.

### Study Treatment

Compound (E) is provided as 100 mg lyophilisate in vial (powder for solution for infusion). The starting dose of is 4 mg/kg administered intravenously once every 2 weeks (Q2W). **Table 31** describes the starting dose and the dose levels evaluated in this study.

**Table 31. Dose levels of single agent**

| **Dose level** | **Daily Dose*** | **Increment from previous dose** |
|---|---|---|
| -2** | 1 mg/kg | -50% |
| -1** | 2 mg/kg | -50% |
| 1 | 4 mg/kg | (starting dose) |
| 2 | 8 mg/kg | 100% |
| 3 | 12 mg/kg | 50% |
| 4 | 16 mg/kg | 33% |
| *It is possible for additional and/or intermediate dose levels to be added during the course of the study. Cohorts may be added at any dose level below the MTD in order to better understand safety, PK, or PD. Dose levels represent total daily doses administered Q2W regimen. | | |
| **If dose de-escalation from starting dose level is required due to DLTs, dose level -1 and -2 represent treatment dose level at which a new cohort may be enrolled. Dose reduction below dose level -2 is permitted for this study. | | |

### Pharmacokinetics

Serial blood samples are collected from all patients in dose escalation and the Phase II part of the study in order to characterize the single dose and steady-state PK of compound (E) in serum, plasma, and blood. In addition, remaining plasma/serum/blood samples might be used for exploratory PK and/or biomarker analysis. This could include using remaining serum, plasma, and blood samples for protein binding analysis, metabolite profiling, exploratory biomarker analyses, protein binding, or other assays if there is sufficient sample remaining.

Concentrations of compound (E) (as total antibody) in human serum are determined with a validated ligand binding assay. Concentrations of conjugated active and inactive phosphorylated payload are analyzed in plasma with a validated assay using an incubation with papain followed by liquid chromatography-tandem mass spectrometry (LC-MS/MS) analysis. Concentrations of the unconjugated in blood are determined with a validated LC-MS/MS assay. Anti-drug antibodies are analyzed with a validated ligand binding assay.

### Biomarkers

Biomarker analyses are used to investigate the effect of study therapies at the molecular and cellular level as well as to determine how changes in the markers may relate to exposure and clinical outcomes. In addition, potential predictive markers of efficacy, as well as mechanisms of resistance may be explored. Exemplary biomarkers are described in **Table 32.**

**Table 32. Biomarker sample collection**

| **Sample Type** | **Volume** | **Visit** | **Marker** | **Purpose** |
|---|---|---|---|---|
| **Tumor Samples** | | | | |
| Archival tumor Newly obtained tumor sample | **Archival tumor:** Block (preferred) or a minimum of 20 newly cut sections (4um thickness) | Screening | Expression of markers such as: | Presence of ADC target confirmation |
| | | Anytime between C1D16-C1D18 (Q2W and QW dosing regimen) | PMEL17 PERK CD8 PD-L1 | Spatial determination & extent of target inhibition |
| | Newly obtained tumor: 3-6 passes of core needle biopsy | Anytime between C2D2-C2D4 (Q4W dosing regimen) Disease Progression +14 days and prior to starting new treatment (optional) | Gene expression of DUSP6 and/or RASGRP3 | Assess expression status of potential predictors of efficacy. Pharmacodynamic markers |
| | | | Cancer-related genes by next generation DNA sequencing | |
| | | | Whole transcriptome analysis; expression of immune and cancer related genes | Assess potential predictive markers of response & mechanisms of resistance |

| **Blood samples** | | | | |
|---|---|---|---|---|
| Blood sample for cfDNA | 10 mL per visit | C1D1 pre-infusion | DNA sequencing in cell free DNA | Assessing cfDNA as surrogate for determination of driver mutation status, predictive pharmacodynamics, for mutational burden and resistance markers in tumor biopsy DNA |
| | | C3D1 pre-infusion | | |
| | | C5D1 pre-infusion | | |
| | | End of Treatment | | |

Newly obtained pre- and on-treatment paired tumor samples are required and are collected at screening, on-treatment and disease progression (optional). The status of several immune checkpoint targets, target modulation and cell populations may be analyzed in tumor tissue. Expression and localization of biomarkers including, but not limited to, PMEL17 and pERK may be measured by IHC or using additional techniques deemed suitable. Tumor specimens are examined for expression of DUSP6 and RASGRP3 in mRNA. Sequencing of cancer related genes may be performed in tumor. Tumor may be sequenced at screening, on-treatment, and end of treatment. Blood is collected on treatment and at end of treatment to allow for sequence analysis of cfDNA. This analysis explores the presence of emerging, existing, and resistance mutations and potentially the tumor mutation burden in tumor and cfDNA at different time points and investigate their relationship with clinical response and the development of tumor resistance. During the study, in addition to the biomarkers specified above, exploratory biomarker research may be conducted on any remaining biomarker and/or PK samples.

Other examples are described in International Application Publication No. WO 2020/128612 and U.S. Application Publication No. US 2020/0197528, which are incorporated by reference in their entirety.

### INCORPORATION BY REFERENCE

All publications, patents, patent applications, and Accession numbers mentioned herein are hereby incorporated by reference in their entirety as if each individual publication or patent was specifically and individually indicated to be incorporated by reference.

### EQUIVALENTS

While this invention has been disclosed with reference to specific aspects, it is apparent that other aspects and variations of this invention may be devised by others skilled in the art without departing from the true spirit and scope of the invention. The appended claims are intended to be construed to include all such aspects and equivalent variations.

The following numbered clauses, describing aspects of the invention, are part of the description.
1. A microorganism, comprising a nucleic acid that comprises a nucleotide sequence encoding a polypeptide associated with the production of a compound having the structure of Formula (A1), wherein the nucleotide sequence is operably linked to a non-native promoter.
2. The microorganism of clause 1, which is a genetically engineered form of a microorganism that naturally produces the compound.
3. The microorganism of any of clauses 1 or 2, which is a bacterium, *e.g., C'hromobαcterium.*
4. The microorganism of any of clauses 1-3, which is *Chromobacterium vaccinii, e.g., Chromobacterium vaccinii* DSM 25150 (= ATCC BAA-2314, = NRRL B-50840, = MWU205).
5. The microorganism of any of clauses 1-4, which is an isolated microorganism or a synthetic microorganism.
6. The microorganism of any of clauses 1-5, wherein the nucleic acid is located on a chromosome of the microorganism, *e*.*g*., naturally located on the chromosome or stably integrated to the chromosome.
7. The microorganism of any of clauses 1-6, wherein the nucleotide sequence encoding a polypeptide associated with the production of the compound is located in a biosynthetic gene cluster (BGC).
8. The microorganism of any of clauses 1-7, wherein the BGC is a compound (A1)-BGC, *e*.*g*., shown in FIG. 1.
9. The microorganism of any of clauses 1-8, wherein the BGC comprises one or more (e.g., two, three, four, five, six, seven, or all) of *frsA, frsB, frsC, frsD, frsE, frsF, frsG,* or *frsH,* or a homolog thereof.
10. The microorganism of any of clauses 1-9, wherein the BGC comprises *frsA, frsB, frsC, frsD, frsE, frsF, frsG,* and *frsH,* or a homolog thereof.
11. The microorganism of any of clauses 1-10, wherein the nucleotide sequence encoding a polypeptide associated with the production of the compound is located in *frsA, frsB, frsC, frsD, frsE, frsF, frsG,* or *frsH,* or a homolog thereof.
12. The microorganism of any of clauses 1-11, wherein the nucleotide sequence encoding a polypeptide associated with the production of the compound is located in a region comprising *frsA* or a homolog thereof.
13. The microorganism of any of clauses 1-12, wherein the nucleotide sequence encoding a polypeptide associated with the production of the compound is located in a region comprising *frsA and frsB,* or a homolog thereof.
14. The microorganism of any of clauses 1-13, wherein the nucleotide sequence encoding a polypeptide associated with the production of the compound is located in a region comprising *frsA*, *frsB, and frsC,* or a homolog thereof.
15. The microorganism of any of clauses 1-14, wherein the nucleotide sequence encoding a polypeptide associated with the production of the compound is located in a region comprising *frsA*, *frsB, frsC,* and *frsD,* or a homolog thereof.
16. The microorganism of any of clauses 1-15, wherein the nucleotide sequence encoding a polypeptide associated with the production of the compound is located in a region comprising *frsA*, *frsb, frsc, frsd, and frsE,* or a homolog thereof.
17. The microorganism of any of clauses 1-16, wherein the nucleotide sequence encoding a polypeptide associated with the production of the compound is located in a region comprising *frsA*, *frsB, frsC, frsD, frsE,* and *frsF,* or a homolog thereof.
18. The microorganism of any of clauses 1-17, wherein the nucleotide sequence encoding a polypeptide associated with the production of the compound is located in a region comprising *frsA*, *frsB, frsC, frsD, frsE, frsF,* and *frsG,* or a homolog thereof.
19. The microorganism of any of clauses 1-18, wherein the nucleotide sequence encoding a polypeptide associated with the production of the compound is located in a region comprising *frsA*, *frsB, frsC, frsD, frsE, frsF, frsG,* and *FrsH,* or a homolog thereof.
20. The microorganism of any of clauses 1-19, wherein the nucleotide sequence encoding a polypeptide associated with the production of the compound comprises the coding sequence of*frsA, frsB, frsC, frsD, frsE, frsF, frsG, frsH,* or a homolog thereof.
21. The microorganism of any of clauses 1-20, wherein the nucleotide sequence encoding a polypeptide associated with the production of the compound comprises the coding sequence of *frsA* or a homolog thereof.
22. The microorganism of any of clauses 1-21, wherein the nucleotide sequence encoding a polypeptide associated with the production of the compound comprises the coding sequence of*frsA and frsB,* or a homolog thereof.
23. The microorganism of any of clauses 1-22, wherein the nucleotide sequence encoding a polypeptide associated with the production of the compound comprises the coding sequence of*frsA, frsB, and frsC,* or a homolog thereof.
24. The microorganism of any of clauses 1-23, wherein the nucleotide sequence encoding a polypeptide associated with the production of the compound comprises the coding sequence of*frsA, frsB, frsC,* and *frsD,* or a homolog thereof.
25. The microorganism of any of clauses 1-24, wherein the nucleotide sequence encoding a polypeptide associated with the production of the compound comprises the coding sequence of*frsA, frsb, frsc, frsd, and frsE,* or a homolog thereof.
26. The microorganism of any of clauses 1-25, wherein the nucleotide sequence encoding a polypeptide associated with the production of the compound comprises the coding sequence of*frsA, frsB, frsC, frsD, frsE,* and *frsF,* or a homolog thereof.
27. The microorganism of any of clauses 1-26, wherein the nucleotide sequence encoding a polypeptide associated with the production of the compound comprises the coding sequence of*frsA, frsB, frsC, frsD, frsE, frsF,* and *frsG,* or a homolog thereof.
28. The microorganism of any of clauses 1-27, wherein the nucleotide sequence encoding a polypeptide associated with the production of the compound comprises the coding sequence *offrsA, frsB, frsC, frsD, frsE, frsF, frsG,* and *FrsH,* or a homolog thereof.
29. The microorganism of any of clauses 1-28, wherein the nucleic acid comprises a nucleotide sequence resulted from a homologous recombination event (*e*.*g*., for promoter exchange) using the nucleotide sequence of SEQ ID NO: 317, or a functional fragment thereof, or a nucleotide sequence having at least 80%, 85%, 90%, 95%, 96%, 97%, 98%, or 99% identity thereto, or differing by no more than 100, 90, 80, 70, 60, 50, 40, 30, 20, 15, 10, 5, or 2 nucleotides therefrom.
30. The microorganism of any of clauses 1-29, wherein the nucleic acid comprises a nucleotide sequence associated GenBank accession number: BankIt2437961 BSeq#1 MW732719, or a functional fragment thereof, or a nucleotide sequence having at least 75%, 80%, 85%, 90%, 95%, 96%, 97%, 98%, or 99% identity thereto.
31. The microorganism of any of clauses 1-30, wherein the non-native promoter is from the same microorganism.
32. The microorganism of any of clauses 1-30, wherein the non-native promoter is from a different microorganism.
33. The microorganism of any of clauses 1-32, wherein the non-native promoter comprises a *vioP* promoter, an *nptII* promoter, an *rbs* promoter, a *J23119* promoter, a *pLpp* promoter, a *PS12burk* promoter, a *Pem7,* or an *ErmE** promoter, optionally, wherein the non-native promoter comprises a *vioP* promoter, an *nptII* promoter, or an *rbs* promoter.
34. The microorganism of any of clauses 1-33, wherein the non-native promoter comprises the nucleotide sequence of any of SEQ ID NOs: 264-266, 268-271, or 316, or a functional fragment thereof, or a nucleotide sequence having at least 80%, 85%, 90%, 95%, 96%, 97%, 98%, or 99% identity thereto, or differing by no more than 60, 50, 40, 30, 20, 15, 10, 5, or 2 nucleotides therefrom.
35. The microorganism of any of clauses 1-34, wherein the non-native promoter comprises or consists of the nucleotide sequence of any of SEQ ID NOs: 264-266, 268-271, or 316, or a functional fragment thereof.
36. The microorganism of any of clauses 1-35, wherein the non-native promoter comprises a *vioP* promoter.
37. The microorganism of any of clauses 1-36, wherein the non-native promoter comprises the nucleotide sequence of SEQ ID NO: 264, or a functional fragment thereof, or a nucleotide sequence having at least 80%, 85%, 90%, 95%, 96%, 97%, 98%, or 99% identity thereto, or differing by no more than 60, 50, 40, 30, 20, 15, 10, 5, or 2 nucleotides therefrom.
38. The microorganism of any of clauses 1-37, wherein the non-native promoter comprises or consists of the nucleotide sequence of SEQ ID NO: 264, or a functional fragment thereof.
39. The microorganism of any of clauses 1-35, wherein the non-native promoter comprises an *nptII* promoter.
40. The microorganism of any of clauses 1-35 or 39, wherein the non-native promoter comprises the nucleotide sequence of SEQ ID NO: 265, or a functional fragment thereof, or a nucleotide sequence having at least 80%, 85%, 90%, 95%, 96%, 97%, 98%, or 99% identity thereto, or differing by no more than 60, 50, 40, 30, 20, 15, 10, 5, or 2 nucleotides therefrom.
41. The microorganism of any of clauses 1-35, 39, or 40, wherein the non-native promoter comprises or consists of the nucleotide sequence of SEQ ID NO: 265, or a functional fragment thereof.
42. The microorganism of any of clauses 1-35, wherein the non-native promoter comprises an *rbs* promoter.
43. The microorganism of any of clauses 1-35 or 42, wherein the non-native promoter comprises the nucleotide sequence of SEQ ID NO: 266, or a functional fragment thereof, or a nucleotide sequence having at least 80%, 85%, 90%, 95%, 96%, 97%, 98%, or 99% identity thereto, or differing by no more than 60, 50, 40, 30, 20, 15, 10, 5, or 2 nucleotides therefrom.
44. The microorganism of any of clauses 1-35, 42, or 43, wherein the non-native promoter comprises or consists of the nucleotide sequence of SEQ ID NO: 266, or a functional fragment thereof.
45. The microorganism of any of clauses 1-35, wherein the non-native promoter comprises a *J23119* promoter.
46. The microorganism of any of clauses 1-35 or 45, wherein the non-native promoter comprises the nucleotide sequence of SEQ ID NO: 316, or a functional fragment thereof, or a nucleotide sequence having at least 80%, 85%, 90%, 95%, 96%, 97%, 98%, or 99% identity thereto, or differing by no more than 60, 50, 40, 30, 20, 15, 10, 5, or 2 nucleotides therefrom.
47. The microorganism of any of clauses 1-35, 45, or 46, wherein the non-native promoter comprises or consists of the nucleotide sequence of SEQ ID NO: 316, or a functional fragment thereof.
48. The microorganism of any of clauses 1-35, wherein the non-native promoter comprises a *pLpp* promoter.
49. The microorganism of any of clauses 1-35 or 48, wherein the non-native promoter comprises the nucleotide sequence of SEQ ID NO: 268, or a functional fragment thereof, or a nucleotide sequence having at least 80%, 85%, 90%, 95%, 96%, 97%, 98%, or 99% identity thereto, or differing by no more than 60, 50, 40, 30, 20, 15, 10, 5, or 2 nucleotides therefrom.
50. The microorganism of any of clauses 1-35, 48 or 49, wherein the non-native promoter comprises or consists of the nucleotide sequence of SEQ ID NO: 268, or a functional fragment thereof.
51. The microorganism of any of clauses 1-35, wherein the non-native promoter comprises a *Pem7* promoter.
52. The microorganism of any of clauses 1-35 or 51, wherein the non-native promoter comprises the nucleotide sequence of SEQ ID NO: 269, or a functional fragment thereof, or a nucleotide sequence having at least 80%, 85%, 90%, 95%, 96%, 97%, 98%, or 99% identity thereto, or differing by no more than 60, 50, 40, 30, 20, 15, 10, 5, or 2 nucleotides therefrom.
53. The microorganism of any of clauses 1-35, 51, or 52, wherein the non-native promoter comprises or consists of the nucleotide sequence of SEQ ID NO: 269, or a functional fragment thereof.
54. The microorganism of any of clauses 1-35, wherein the non-native promoter comprises a *PS12burk* promoter.
55. The microorganism of any of clauses 1-35 or 54, wherein the non-native promoter comprises the nucleotide sequence of SEQ ID NO: 270, or a functional fragment thereof, or a nucleotide sequence having at least 80%, 85%, 90%, 95%, 96%, 97%, 98%, or 99% identity thereto, or differing by no more than 60, 50, 40, 30, 20, 15, 10, 5, or 2 nucleotides therefrom.
56. The microorganism of any of clauses 1-35, 54, or 55, wherein the non-native promoter comprises or consists of the nucleotide sequence of SEQ ID NO: 270, or a functional fragment thereof.
57. The microorganism of any of clauses 1-35, wherein the non-native promoter comprises an *ErmE** promoter.
58. The microorganism of any of clauses 1-35 or 57, wherein the non-native promoter comprises the nucleotide sequence of SEQ ID NO: 271, or a functional fragment thereof, or a nucleotide sequence having at least 80%, 85%, 90%, 95%, 96%, 97%, 98%, or 99% identity thereto, or differing by no more than 60, 50, 40, 30, 20, 15, 10, 5, or 2 nucleotides therefrom.
59. The microorganism of any of clauses 1-35, 57, or 58, wherein the non-native promoter comprises or consists of the nucleotide sequence of SEQ ID NO: 271, or a functional fragment thereof.
60. The microorganism of any of clauses 1-59, wherein the non-native promoter controls the transcription of one or more (e.g., two, three, four, five, six, seven, or all) of *FrsA, FrsB, FrsC, FrsD, FrsE, FrsF, FrsG,* or *FrsH,* or a homolog thereof.
61. The microorganism of any of clauses 1-60, wherein the non-native promoter controls the transcription of *FrsA, FrsB, FrsC, FrsD, FrsE, FrsF, FrsG,* and *FrsH,* or a homolog thereof.
62. The microorganism of any of clauses 1-61, wherein the non-native promoter is inserted upstream of the coding region of *FrsA,* or a homolog thereof, *e.g.,* upstream of the coding region of all of *FrsA, FrsB, FrsC, FrsD, FrsE, FrsF, FrsG,* and *FrsH,* or a homolog thereof.
63. The microorganism of any of clauses 1-62, wherein the polypeptide associated with the production of the compound is a non-ribosomal peptide synthetase (NRPS) or a functional fragment thereof.
64. The microorganism of any of clauses 1-63, wherein the NRPS is FrsA, FrsD, FrsE, FrsF, or FrsG, or a homolog thereof.
65. The microorganism of any of clauses 1-64, wherein the polypeptide associated with the production of the compound is an MbtH-like protein or a functional fragment thereof.
66. The microorganism of any of clauses 1-65, wherein the MbtH-like protein is FrsB or a homolog thereof.
67. The microorganism of any of clauses 1-66, wherein the polypeptide associated with the production of the compound is a malate dehydrogenase.
68. The microorganism of any of clauses 1-67, wherein the malate dehydrogenase is FrsC or a homolog thereof.
69. The microorganism of any of clauses 1-68, wherein the polypeptide associated with the production of the compound is a hydroxylase.
70. The microorganism of any of clauses 1-69, wherein the hydroxylase is FrsH or a homolog thereof.
71. The microorganism of any of clauses 1-70, wherein the nucleic acid comprises a plurality of nucleotide sequences encoding a plurality of polypeptides associated with the production of the compound.
72. The microorganism of any of clauses 1-71, wherein the plurality of polypeptides associated with the production of the compound comprises a plurality of NRPSs, or a functional fragment thereof.
73. The microorganism of any of clauses 1-72, wherein the plurality of NRPSs comprise two or more (e.g., three, four, or all) of FrsA, FrsD, FrsE, FrsF, or FrsG, or a homolog thereof.
74. The microorganism of any of clauses 1-73, wherein the plurality of polypeptides associated with the production of the compound comprises an NRPS, a MbtH-like protein, a malate dehydrogenase, and a hydroxylase, or a functional fragment thereof.
75. The microorganism of any of clauses 1-74, wherein the plurality of polypeptides associated with the production of the compound comprises FrsA, FrsB, FrsC, FrsD, FrsE, FrsF, FrsG, and FrsH, or a homolog thereof.
76. The microorganism of any of clauses 1-75, wherein the microorganism has an increased titer of the compound.
77. The microorganism of any of clauses 1-76, wherein the titer of the compound is increased by at least 1, 2, 3, 4, 5, 6, 7, 8, 9, or 10-fold, compared to a reference microorganism, *e*.*g*., an otherwise identical microorganism that does not comprise the non-native promoter in the compound (A1)-BGC (e.g., a wild-type), when cultured under conditions that allow production of the compound.
78. The microorganism of any of clauses 1-77, wherein the titer of the compound is increased by at least about 100 mg/L, 200 mg/L, 300 mg/L, 400 mg/L, 500 mg/L, 600 mg/L, 700 mg/L, 800 mg/L, 900 mg/L, or 1000 mg/L, compared to a reference microorganism, *e*.*g*., an otherwise identical microorganism that does not comprise the non-native promoter in the compound (A1)-BGC (*e*.*g*., a wild-type), when cultured under conditions that allow production of the compound.
79. The microorganism of any of clauses 1-78, wherein the BGC of a natural product that interferes with the isolation of the compound is altered, *e*.*g*., disrupted.
80. The microorganism of any of clauses 1-79, wherein the natural product comprises one or more (e.g., two, three, or all) of: violacein, compound J, compound F5, compound F3, or compound D.
81. The microorganism of any of clauses 1-80, wherein at least a portion of the BGC of the natural product interferes with the isolation of the compound is deleted.
82. The microorganism of any of clauses 1-81, wherein the violacein-BGC is altered, *e*.*g*., disrupted.
83. The microorganism of any of clauses 1-82, wherein the compound J-BGC is altered, *e.g.,* disrupted.
84. The microorganism of any of clauses 1-83, wherein the compound J-BGC is associated with the production of compound J, compound F5, compound F3, and compound D.
85. The microorganism of any of clauses 1-84, wherein both the violacein-BGC and the compound J-BGC are altered, *e*.*g*., disrupted.
86. The microorganism of any of clauses 1-85, wherein the production of the natural product is reduced, *e*.*g*., by at least 50%, 60%, 70%, 80%, 90%, 95%, 96%, 97%, 98%, 99%, or 100%.
87. The microorganism of any of clauses 1-86, wherein the microorganism has an increased isolation yield of the compound.
88. The microorganism of any of clauses 1-87, wherein the isolation yield of the compound is increased by 20%, 30%, 40%, 50%, 60%, 70%, 80%, 90%, or more, compared to a reference microorganism, *e.g.,* an otherwise identical microorganism in which the BGC of the natural product that interferes with the isolation of the compound is not altered (*e*.*g*., a wild-type).
89. The microorganism of any of clauses 1-88, wherein the BGC of a natural product that interferes with the isolation of the compound is not altered, *e.g.,* not disrupted.
90. A microorganism that produces a compound having the structure of Formula (A1), wherein the BGC of a natural product that interferes with the isolation of the compound is altered.
91. The microorganism of clause 90, wherein the natural product comprises one or more (*e*.*g*., two, three, or all) of: violacein, compound J, compound F5, compound F3, or compound D.
92. The microorganism of any of clauses 90 or 91, wherein at least a portion of the BGC of the natural product interferes with the isolation of the compound is deleted.
93. The microorganism of any of clauses 90-92, wherein the violacein-BGC is altered, *e*.*g*., disrupted.
94. The microorganism of any of clauses 90-93, wherein the compound J-BGC is altered, *e.g.,* disrupted.
95. The microorganism of any of clauses 90-94, wherein the compound J-BGC is associated with the production of compound J, compound F5, compound F3, and compound D.
96. The microorganism of any of clauses 90-95, wherein both the violacein-BGC and the compound J-BGC are altered, *e*.*g*., disrupted.
97. The microorganism of any of clauses 90-96, wherein the production of the natural product is reduced, *e*.*g*., by at least 50%, 60%, 70%, 80%, 90%, 95%, 96%, 97%, 98%, 99%, or 100%.
98. The microorganism of any of clauses 90-97, wherein the microorganism has an increased isolation yield of the compound.
99. The microorganism of any of clauses 90-98, wherein the isolation yield of the compound is increased by 20%, 30%, 40%, 50%, 60%, 70%, 80%, 90%, or more, compared to a reference microorganism, *e.g*., an otherwise identical microorganism in which the BGC of the natural product that interferes with the isolation of the compound is not altered (e.g., a wild-type).
100. A method of producing a compound having the structure of Formula (A1), comprising:
   culturing (*e*.*g*., fermenting) a microorganism of any of clauses 1-99 under conditions that allow the expression of the compound,
   thereby producing the compound.
101. The method of clause 100, wherein the microorganism is cultured (*e*.*g*., fermented) at 50 L to 500 L scale, *e*.*g*., at 50 L, 100 L, 150 L, 200 L, 250 L, 300 L, 350 L, 400 L, 450 L, or 500 L scale.
102. The method of clause 100, wherein the microorganism is cultured (*e*.*g*., fermented) at 1,000 L to 20,000 L scale, *e*.*g*., 1,000 L to 10,000 L or 10,000 L to 20,000 L, *e*.*g*., 1,000 L, 2,000 L, 5,000 L, 7,500 L, 10,000 L, 15,000 L, or 20,000 L scale.
103. The method of any of clauses 100-102, wherein the culturing (*e*.*g*., fermentation) yields a titer of at least 200 mL of the compound, *e*.*g*., at least 250 mg/mL, 300 mg/L, 400 mg/L, 500 mg/L, 600 mg/L, 700 mg/L, 800 mg/L, 900 mg/L, 1000 mg/L, 1100 mg/L, 1200 mg/L, 1300 mg/L, 1400 mg/L, 1500 mg/L, 1600 mg/L, 1700 mg/L, 1800 mg/L, 1900 mg/L, or 2000 mg/L of the compound.
104. The method of any of clauses 100-103, the method further comprising isolating the compound, *e.g.,* to a purity of at least 90%, *e.g.,* at least 95%, 96%, 97%, 98%, or 99%, *e.g.,* as determined by a method described herein.
105. A nucleic acid comprising a nucleotide sequence encoding a polypeptide associated with the production of a compound having the structure of Formula (A1), wherein the nucleotide sequence is operably linked to a non-native promoter.
106. The nucleic acid of clause 105, wherein the nucleic acid is located on a chromosome of the microorganism, *e*.*g*., naturally located on the chromosome or stably integrated to the chromosome.
107. The nucleic acid of any of clauses 105 or 106, wherein the nucleotide sequence encoding a polypeptide associated with the production of the compound is located in a biosynthetic gene cluster (BGC).
108. The nucleic acid of any of clauses 105-107, wherein the BGC is a compound (A1)-BGC.
109. The nucleic acid of any of clauses 105-108, wherein the compound (A1)-BGC has the gene organization shown in FIG. 1.
110. The nucleic acid of any of clauses 105-109, wherein the BGC comprises one or more (*e*.*g*., two, three, four, five, six, seven, or all) of *frsA, frsB, frsC, frsD, frsE, frsF, frsG,* or *frsH,* or a homolog thereof.
111. The nucleic acid of any of clauses 105-110, wherein the BGC comprises *frsA, frsB, frsC, frsD, frsE, frsF, frsG,* and *frsH,* or a homolog thereof.
112. The nucleic acid of any of clauses 105-111, wherein the nucleotide sequence encoding a polypeptide associated with the production of the compound is located in *frsA, frsB, frsC, frsD, frsE, frsF, frsG, frsH,* or a homolog thereof.
113. The nucleic acid of any of clauses 105-112, wherein the nucleotide sequence encoding a polypeptide associated with the production of the compound is located in a region comprising *frsA* or a homolog thereof.
114. The nucleic acid of any of clauses 105-113, wherein the nucleotide sequence encoding a polypeptide associated with the production of the compound is located in a region comprising *frsA and frsB,* or a homolog thereof.
115. The nucleic acid of any of clauses 105-114, wherein the nucleotide sequence encoding a polypeptide associated with the production of the compound is located in a region comprising *frsA*, *frsB, and frsC,* or a homolog thereof.
116. The nucleic acid of any of clauses 105-115, wherein the nucleotide sequence encoding a polypeptide associated with the production of the compound is located in a region comprising *frsA*, *frsB, frsC,* and *frsD,* or a homolog thereof.
117. The nucleic acid of any of clauses 105-116, wherein the nucleotide sequence encoding a polypeptide associated with the production of the compound is located in a region comprising *frsA*, *frsb, frsc, frsd, and frsE,* or a homolog thereof.
118. The nucleic acid of any of clauses 105-117, wherein the nucleotide sequence encoding a polypeptide associated with the production of the compound is located in a region comprising*frsA*, *frsB, frsC, frsD, frsE,* and *frsF,* or a homolog thereof.
119. The nucleic acid of any of clauses 105-118, wherein the nucleotide sequence encoding a polypeptide associated with the production of the compound is located in a region comprising *frsA*, *frsB, frsC, frsD, frsE, frsF,* and *frsG,* or a homolog thereof.
120. The nucleic acid of any of clauses 105-119, wherein the nucleotide sequence encoding a polypeptide associated with the production of the compound is located in a region comprising *frsA*, *frsB, frsC, frsD, frsE, frsF, frsG,* and *FrsH,* or a homolog thereof.
121. The nucleic acid of any of clauses 105-120, wherein the nucleotide sequence encoding a polypeptide associated with the production of the compound comprises the coding sequence of *frsA, frsB, frsC, frsD, frsE, frsF, frsG, frsH,* or a homolog thereof.
122. The nucleic acid of any of clauses 105-121, wherein the nucleotide sequence encoding a polypeptide associated with the production of the compound comprises the coding sequence of *frsA* or a homolog thereof.
123. The nucleic acid of any of clauses 105-122, wherein the nucleotide sequence encoding a polypeptide associated with the production of the compound comprises the coding sequence *offrsA and frsB,* or a homolog thereof.
124. The nucleic acid of any of clauses 105-123, wherein the nucleotide sequence encoding a polypeptide associated with the production of the compound comprises the coding sequence *offrsA, frsB, and frsC,* or a homolog thereof.
125. The nucleic acid of any of clauses 105-124, wherein the nucleotide sequence encoding a polypeptide associated with the production of the compound comprises the coding sequence of*frsA, frsB, frsC,* and *frsD,* or a homolog thereof.
126. The nucleic acid of any of clauses 105-125, wherein the nucleotide sequence encoding a polypeptide associated with the production of the compound comprises the coding sequence of*frsA, frsb, frsc, frsd, and frsE,* or a homolog thereof.
127. The nucleic acid of any of clauses 105-126, wherein the nucleotide sequence encoding a polypeptide associated with the production of the compound comprises the coding sequence of*frsA, frsB, frsC, frsD, frsE,* and *frsF,* or a homolog thereof.
128. The nucleic acid of any of clauses 105-127, wherein the nucleotide sequence encoding a polypeptide associated with the production of the compound comprises the coding sequence of*frsA, frsB, frsC, frsD, frsE, frsF,* and *frsG,* or a homolog thereof.
129. The nucleic acid of any of clauses 105-128, wherein the nucleotide sequence encoding a polypeptide associated with the production of the compound comprises the coding sequence of*frsA, frsB, frsC, frsD, frsE, frsF, frsG,* and *FrsH,* or a homolog thereof.
130. The nucleic acid of any of clauses 105-129, wherein the nucleic acid comprises a nucleotide sequence resulted from a homologous recombination event (*e*.*g*., for promoter exchange) using the nucleotide sequence of SEQ ID NO: 317, or a functional fragment thereof, or a nucleotide sequence having at least 80%, 85%, 90%, 95%, 96%, 97%, 98%, or 99% identity thereto, or differing by no more than 100, 90, 80, 70, 60, 50, 40, 30, 20, 15, 10, 5, or 2 nucleotides therefrom.
131. The nucleic acid of any of clauses 105-130, wherein the nucleic acid comprises a nucleotide sequence associated GenBank accession number: BankIt2437961 BSeq#1 MW732719, or a functional fragment thereof, or a nucleotide sequence having at least 75%, 80%, 85%, 90%, 95%, 96%, 97%, 98%, or 99% identity thereto.
132. The nucleic acid of any of clauses 105-131, wherein the non-native promoter comprises a *vioP* promoter, an *nptII* promoter, an *rbs* promoter, a *J23119* promoter, a *pLpp* promoter, a *PS12burk* promoter, a *Pem7,* or an *ErmE** promoter, optionally, wherein the non-native promoter comprises a *vioP* promoter, an *nptII* promoter, or an *rbs* promoter.
133. The nucleic acid of any of clauses 105-132, wherein the non-native promoter comprises the nucleotide sequence of any of SEQ ID NOs: 264-266, 268-271, or 316, or a functional fragment thereof, or a nucleotide sequence having at least 80%, 85%, 90%, 95%, 96%, 97%, 98%, or 99% identity thereto, or differing by no more than 60, 50, 40, 30, 20, 15, 10, 5, or 2 nucleotides therefrom.
134. The nucleic acid of any of clauses 105-133, wherein the non-native promoter comprises or consists of the nucleotide sequence of any of SEQ ID NOs: 264-266, 268-271, or 316, or a functional fragment thereof.
135. The nucleic acid of any of clauses 105-134, wherein the non-native promoter comprises a *vioP* promoter.
136. The nucleic acid of any of clauses 1 05-135, wherein the non-native promoter comprises the nucleotide sequence of SEQ ID NO: 264, or a functional fragment thereof, or a nucleotide sequence having at least 80%, 85%, 90%, 95%, 96%, 97%, 98%, or 99% identity thereto, or differing by no more than 60, 50, 40, 30, 20, 15, 10, 5, or 2 nucleotides therefrom.
137. The nucleic acid of any of clauses 105-136, wherein the non-native promoter comprises or consists of the nucleotide sequence of SEQ ID NO: 264, or a functional fragment thereof.
138. The nucleic acid of any of clauses 105-134, wherein the non-native promoter comprises an *nptII* promoter.
139. The nucleic acid of any of clauses 105-134 or 138, wherein the non-native promoter comprises the nucleotide sequence of SEQ ID NO: 265, or a functional fragment thereof, or a nucleotide sequence having at least 80%, 85%, 90%, 95%, 96%, 97%, 98%, or 99% identity thereto, or differing by no more than 60, 50, 40, 30, 20, 15, 10, 5, or 2 nucleotides therefrom.
140. The nucleic acid of any of clauses 105-134, 138, or 139, wherein the non-native promoter comprises or consists of the nucleotide sequence of SEQ ID NO: 265, or a functional fragment thereof.
141. The nucleic acid of any of clauses 105-134, wherein the non-native promoter comprises an *rbs* promoter.
142. The nucleic acid of any of clauses 105-134 or 141, wherein the non-native promoter comprises the nucleotide sequence of SEQ ID NO: 266, or a functional fragment thereof, or a nucleotide sequence having at least 80%, 85%, 90%, 95%, 96%, 97%, 98%, or 99% identity thereto, or differing by no more than 60, 50, 40, 30, 20, 15, 10, 5, or 2 nucleotides therefrom.
143. The nucleic acid of any of clauses 105-134, 141 or 142, wherein the non-native promoter comprises or consists of the nucleotide sequence of SEQ ID NO: 266, or a functional fragment thereof.
144. The nucleic acid of any of clauses 105-134, wherein the non-native promoter comprises a *J23119* promoter.
145. The nucleic acid of any of clauses 105-134 or 144, wherein the non-native promoter comprises the nucleotide sequence of SEQ ID NO: 316, or a functional fragment thereof, or a nucleotide sequence having at least 80%, 85%, 90%, 95%, 96%, 97%, 98%, or 99% identity thereto, or differing by no more than 60, 50, 40, 30, 20, 15, 10, 5, or 2 nucleotides therefrom.
146. The nucleic acid of any of clauses 105-134, 144, or 145, wherein the non-native promoter comprises or consists of the nucleotide sequence of SEQ ID NO: 316, or a functional fragment thereof.
147. The nucleic acid of any of clauses 105-134, wherein the non-native promoter comprises a *pLpp* promoter.
148. The nucleic acid of any of clauses 105-134 or 147, wherein the non-native promoter comprises the nucleotide sequence of SEQ ID NO: 268, or a functional fragment thereof, or a nucleotide sequence having at least 80%, 85%, 90%, 95%, 96%, 97%, 98%, or 99% identity thereto, or differing by no more than 60, 50, 40, 30, 20, 15, 10, 5, or 2 nucleotides therefrom.
149. The nucleic acid of any of clauses 105-134, 147, or 148, wherein the non-native promoter comprises or consists of the nucleotide sequence of SEQ ID NO: 268, or a functional fragment thereof.
150. The nucleic acid of any of clauses 105-134, wherein the non-native promoter comprises a *Pem7* promoter.
151. The nucleic acid of any of clauses 105-134 or 150, wherein the non-native promoter comprises the nucleotide sequence of SEQ ID NO: 269, or a functional fragment thereof, or a nucleotide sequence having at least 80%, 85%, 90%, 95%, 96%, 97%, 98%, or 99% identity thereto, or differing by no more than 60, 50, 40, 30, 20, 15, 10, 5, or 2 nucleotides therefrom.
152. The nucleic acid of any of clauses 105-134, 150, or 151, wherein the non-native promoter comprises or consists of the nucleotide sequence of SEQ ID NO: 269, or a functional fragment thereof.
153. The nucleic acid of any of clauses 105-134, wherein the non-native promoter comprises a *PS12burk* promoter.
154. The nucleic acid of any of clauses 105-134 or 153, wherein the non-native promoter comprises the nucleotide sequence of SEQ ID NO: 270, or a functional fragment thereof, or a nucleotide sequence having at least 80%, 85%, 90%, 95%, 96%, 97%, 98%, or 99% identity thereto, or differing by no more than 60, 50, 40, 30, 20, 15, 10, 5, or 2 nucleotides therefrom.
155. The nucleic acid of any of clauses 105-134, 153, or 154, wherein the non-native promoter comprises or consists of the nucleotide sequence of SEQ ID NO: 270, or a functional fragment thereof.
156. The nucleic acid of any of clauses 105-134, wherein the non-native promoter comprises an *ErmE** promoter.
157. The nucleic acid of any of clauses 105-134 or 156, wherein the non-native promoter comprises the nucleotide sequence of SEQ ID NO: 271, or a functional fragment thereof, or a nucleotide sequence having at least 80%, 85%, 90%, 95%, 96%, 97%, 98%, or 99% identity thereto, or differing by no more than 60, 50, 40, 30, 20, 15, 10, 5, or 2 nucleotides therefrom.
158. The nucleic acid of any of clauses 105-134, 156, or 157, wherein the non-native promoter comprises or consists of the nucleotide sequence of SEQ ID NO: 271, or a functional fragment thereof.
159. The nucleic acid of any of clauses 105-158, wherein the non-native promoter controls the transcription of one or more (*e*.*g*., two, three, four, five, six, seven, or all) of *FrsA, FrsB, FrsC, FrsD, FrsE, FrsF, FrsG,* or *FrsH,* or a homolog thereof.
160. The nucleic acid of any of clauses 105-159, wherein the non-native promoter controls the transcription of *FrsA, FrsB, FrsC, FrsD, FrsE, FrsF, FrsG,* and *FrsH,* or a homolog thereof.
161. The nucleic acid of any of clauses 105-160, wherein the non-native promoter is inserted upstream of the coding region of *FrsA,* or a homolog thereof, *e.g.,* upstream of the coding region of all of *FrsA, FrsB, FrsC, FrsD, FrsE, FrsF, FrsG,* and *FrsH,* or a homolog thereof.
162. The nucleic acid of any of clauses 105-161, wherein the polypeptide associated with the production of the compound is a non-ribosomal peptide synthetase (NRPS) or a functional fragment thereof.
163. The nucleic acid of any of clauses 105-162, wherein the NRPS is FrsA, FrsD, FrsE, FrsF, or FrsG, or a homolog thereof.
164. The nucleic acid of any of clauses 105-163, wherein the polypeptide associated with the production of the compound is a MbtH-like protein or a functional fragment thereof.
165. The nucleic acid of any of clauses 105-164, wherein the MbtH-like protein is FrsB or a homolog thereof.
166. The nucleic acid of any of clauses 105-165, wherein the polypeptide associated with the production of the compound is a malate dehydrogenase.
167. The nucleic acid of any of clauses 105-166, wherein the malate dehydrogenase is FrsC or a homolog thereof.
168. The nucleic acid of any of clauses 105-167, wherein the polypeptide associated with the production of the compound is a hydroxylase.
169. The nucleic acid of any of clauses 105-168, wherein hydroxylase is FrsH or a homolog thereof.
170. The nucleic acid of any of clauses 105-169, wherein the nucleic acid comprises a plurality of nucleotide sequences encoding a plurality of polypeptides associated with the production of the compound.
171. The nucleic acid of any of clauses 105-170, wherein the plurality of polypeptides associated with the production of the compound comprises a plurality of NRPSs, or a functional fragment thereof.
172. The nucleic acid of any of clauses 105-171, wherein the plurality of NRPSs comprise two or more (e.g., three, four, or all) of FrsA, FrsD, FrsE, FrsF, or FrsG, or a homolog thereof.
173. The nucleic acid of any of clauses 105-172, wherein the plurality of polypeptides associated with the production of the compound comprises an NRPS, a MbtH-like protein, a malate dehydrogenase, and a hydroxylase, or a functional fragment thereof.
174. The nucleic acid of any of clauses 105-173, wherein the plurality of polypeptides associated with the production of the compound comprises FrsA, FrsB, FrsC, FrsD, FrsE, FrsF, FrsG, and FrsH, or a homolog thereof.
175. A nucleic acid comprising a nucleotide sequence encoding a polypeptide associated with the production of one or more of: compound J, compound F5, compound F3, or compound D.
176. The nucleic acid of clause 175, which is an isolated nucleic acid, a non-naturally occurring nucleic acid, or a synthetic nucleic acid.
177. The nucleic acid of any of clauses 175 or 176, which comprises a mutation, *e*.*g*., a deletion.
178. The nucleic acid of any of clauses 175-177, wherein the nucleic acid comprises a plurality of nucleotide sequences encoding a plurality of polypeptides associated with the production of one or more of: compound J, compound F5, compound F3, or compound D.
179. The nucleic acid of any of clauses 175-178, wherein the nucleotide sequence is from a BGC.
180. The nucleic acid of clause 179, wherein the BGC is a compound J-BGC.
181. The nucleic acid of clause 180, wherein the compound J-BGC has the gene organization shown in FIG. 1.
182. The nucleic acid of any of clauses 175-181, wherein the nucleotide sequence comprises one or more (*e*.*g*., two, three, or all) of *dis1, dis2, dis3,* or *dis4,* or a homolog thereof.
183. The nucleic acid of any of clauses 175-182, wherein the nucleotide sequence comprises *dis1, dis2, dis3,* and *dis4,* or a homolog thereof.
184. The nucleic acid of any of clauses 175-183, wherein the nucleic acid comprises a nucleotide sequence associated GenBank accession number: BankIt2437961 BSeq#1 MW732719, or a functional fragment thereof, or a nucleotide sequence having at least 75%, 80%, 85%, 90%, 95%, 96%, 97%, 98%, or 99% identity thereto.
185. A vector comprising a nucleic acid of any of clauses 105-184.
186. A cell comprising a nucleic acid of any of clauses 105-184 or a vector of clause 185.
187. A method of engineering a cell, comprising:
   altering a nucleotide sequence encoding a polypeptide associated with the production of one or more of: compound J, compound F5, compound F3, or compound D,
   thereby engineering the cell.
188. The method of clause 187, wherein the cell is a microorganism that naturally produces a compound having the structure of Formula (A1).
189. The method of any of clauses 187 or 188, wherein the microorganism is a bacterium, *e*.*g*., *C'hromobαcterium.*
190. The method of any of clauses 187-189, wherein the microorganism is *Chromobacterium vaccinii, e.g., Chromobacterium vaccinii* DSM 25150 (= ATCC BAA-2314, = NRRL B-50840, = MWU205).
191. The method of any of clauses 187-190, wherein the nucleotide sequence is disrupted, *e*.*g*., at least a portion of the nucleotide sequence is deleted.
192. The method of any of clauses 187-191, wherein the nucleic acid comprises a plurality of nucleotide sequences encoding a plurality of polypeptides associated with the production of one or more of: compound J, compound F5, compound F3, or compound D.
193. The method of any of clauses 187-192, wherein the production of one or more (e.g., two, three, or all) of: compound J, compound F5, compound F3, or compound D is reduced, *e*.*g*., by at least 50%, 60%, 70%, 80%, 90%, 95%, 96%, 97%, 98%, 99%, or 100%.
194. The method of any of clauses 187-193, wherein the nucleotide sequence is from a BGC.
195. The method of clause 194, wherein the BGC is a compound J-BGC.
196. The method of clause 195, wherein the compound J-BGC has the gene organization shown in FIG. 1.
197. The method of any of clauses 187-196, wherein the nucleotide sequence comprises one or more (e.g., two, three, or all) of *dis1, dis2, dis3,* or *dis4,* or a homolog thereof.
198. The method of any of clauses 187-197, wherein the nucleotide sequence comprises *dis1, dis2, dis3,* and *dis4,* or a homolog thereof.
199. The method of any of clauses 187-198, wherein the nucleic acid comprises a nucleotide sequence associated GenBank accession number: BankIt2437961 BSeq#1 MW732719, or a functional fragment thereof, or a nucleotide sequence having at least 75%, 80%, 85%, 90%, 95%, 96%, 97%, 98%, or 99% identity thereto.
200. The method of any of clauses 187-199, wherein the alteration increases the isolation yield of the compound.
201. The method of any of clauses 187-200, wherein the isolation yield of the compound is increased by 20%, 30%, 40%, 50%, 60%, 70%, 80%, 90%, or more, compared to a reference microorganism, *e.g.,* an otherwise identical microorganism in which the BGC of the natural product that interferes with the isolation of the compound is not altered (*e*.*g*., a wild-type).
202. A process for producing a partially reoxidized antibody or antigen binding fragment thereof, comprising:
   providing an antibody or antigen binding fragment thereof comprising one or more capped cysteine residues;
   reducing the one or more capped cysteine residues, thereby providing an antibody or antigen binding fragment thereof comprising one or more decapped cysteine residues; and
   storing the antibody or antigen binding fragment thereof comprising one or more decapped cysteine residues under oxidation conditions,
   thereby producing the partially reoxidized antibody or antigen binding fragment thereof.
203. The process of clause 202, wherein the one or more capped cysteine residues are located in the constant domain a heavy chain of the antibody or antigen binding fragment thereof.
204. The process of any of clauses 202 or 203, wherein the antibody or antigen binding fragment thereof comprises four capped cysteine residues.
205. The process of any of clauses 202-204, wherein the antibody or antigen binding fragment thereof comprises two capped cysteine residues.
206. The process of any of clauses 202-205, wherein the antibody or antigen binding fragment thereof comprises a capped cysteine residue located in a CH1 region of the antibody or antigen binding fragment thereof.
207. The process of any of clauses 202-206, wherein the antibody or antigen binding fragment thereof comprises a capped cysteine residue located in each CH1 region of the antibody or antigen binding fragment thereof.
208. The process of any of clauses 202-207, wherein the capped cysteine residue is an engineered surface-exposed cysteine residue.
209. The process of any of clauses 202-208, wherein the antibody or antigen binding fragment thereof comprises a capped cysteine residue located at residue 152 (EU numbering) in the CH1 region of the antibody or antigen binding fragment thereof.
210. The process of any of clauses 202-209, wherein the one or more capped cysteine residues are capped with a cysteine or a glutathione.
211. The process of any of clauses 202-210, wherein the one or more decapped cysteine residues are located in the constant domain a heavy chain of the antibody or antigen binding fragment thereof.
212. The process of any of clauses 202-211, wherein the antibody or antigen binding fragment thereof comprises four decapped cysteine residues.
213. The process of any of clauses 202-212, wherein the antibody or antigen binding fragment thereof comprises two decapped cysteine residues.
214. The process of any of clauses 202-213, wherein the antibody or antigen binding fragment thereof comprises a decapped cysteine residue located in a CH1 region of the antibody or antigen binding fragment thereof.
215. The process of any of clauses 202-214, wherein the antibody or antigen binding fragment thereof comprises a decapped cysteine residue located in each CH1 region of the antibody or antigen binding fragment thereof.
216. The process of any of clauses202-215, wherein the decapped cysteine residue is an engineered surface-exposed cysteine residue.
217. The process of any of clauses 202-216, wherein the antibody or antigen binding fragment thereof comprises a decapped cysteine residue located at residue 152 (EU numbering) in the CH1 region of the antibody or antigen binding fragment thereof.
218. The process of any of clauses 202-217, wherein the process comprises reducing the one or more capped cysteine residues by contacting the antibody or antigen binding fragment thereof comprising one or more capped cysteine residues with a reduction wash buffer, thereby providing an antibody or antigen binding fragment comprising one or more decapped cysteine residue.
219. The process of any of clauses 202-218, wherein the reduction wash buffer comprises 5 mM to 50 mM cysteine, *e.g*., 10 mM to 40 mM, 20 mM to 30 mM, 5 mM to 15 mM, 5 mM to 25 mM, 5 mM to 35 mM, 5 mM to 45 mM, 40 mM to 50 mM, 30 mM to 50 mM, 20 mM to 50 mM, 10 mM to 50 mM, 10 mM to 20 mM, 15 mM to 25 mM, 25 mM to 35 mM, 30 mM to 40 mM, or 35 mM to 45 mM, *e.g.,* 10 mM, 12 mM, 14 mM, 16 mM, 20 mM, 25 mM, or 30 mM.
220. The process of any of clauses 202-219, wherein the reduction wash buffer comprises 5 mM to 15 mM cysteine, *e.g.,* 10 mM cysteine.
221. The process of any of clauses 202-220, wherein the reduction wash buffer has a pH of 6.0 to 7.5, *e.g.,* 6.5 to 7.2 or 6.8 to 7.0.
222. The process of any of clauses 202-221, wherein the reduction wash buffer has a pH of 6.8 to 7.0, *e.g.,* 6*.*9*.*
223. The process of any of clauses 202-222, wherein contacting the antibody or antigen binding fragment thereof comprising one or more capped cysteine residues with the reduction wash buffer is performed on a column (*e*.*g*., a cation exchange chromatography column).
224. The process of any of clauses 202-223, wherein the process further comprises collecting the antibody or antigen binding fragment thereof comprising one or more decapped cysteine residues in an eluate after contacting with the reduction wash buffer.
225. The process of any of clauses 202-224, wherein the eluate has a pH of 5.5 to 6.0, *e.g.,* 5.8.
226. The process of any of clauses 202-225, wherein the concentration of the antibody or antigen binding fragment thereof comprising one or more decapped cysteine residues in the eluate is 5 g/L to 25 g/L, *e.g.,* 8 g/L to 20 g/L, 10 g/L to 18 g/L, 12 g/L to 15 g/L, 5 g/L to 20 g/L, 5 g/L to 15 g/L, 5 g/L to 10 g/L, 20 g/L to 25 g/L, 15 g/L to 25 g/L, 10 g/L to 25 g/L, 10 g/L to 20 g/L, 13 g/L to 14 g/L, 12 g/L to 15 g/L, *e.g., e.g.,* 5 g/L, 6 g/L, 7 g/L, 8 g/L, 9 g/L, 10 g/L, 11 g/L, 12 g/L, 13 g/L, 13.5 g/L, 14 g/L, 15 g/L, 16 g/L, 17 g/L, 18 g/L, 19 g/L, 20 g/L, 21 g/L, 22 g/L, 23 g/L, 24 g/L, or 25 g/L.
227. The process of any of clauses 202-226, wherein the antibody or antigen binding fragment thereof comprising one or more decapped cysteine residues is stored in the eluate with stirring.
228. The process of any of clauses 202-227, wherein the antibody or antigen binding fragment thereof comprising one or more decapped cysteine residues is stored in the eluate without stirring.
229. The process of any of clauses 202-228, wherein the antibody or antigen binding fragment thereof comprising one or more decapped cysteine residues is stored in a container filled to a maximum of 50% to 70% volume, *e.g.,* for 12 hours to 96 hours, *e.g.,* 12 hours to 84 hours, 24 hours to 84 hours, 36 hours to 72 hours, 48 hours to 60 hours, 24 hours to 72 hours, 24 hours to 60 hours, 24 hours to 48 hours, 24 hours to 36 hours, 72 hours to 84 hours, 60 hours to 84 hours, 48 hours to 84 hours, 36 hours to 84 hours, 12 hours to 36 hours, 24 hours to 48 hours, 36 hours to 60 hours, 48 hours to 72 hours, or 72 hours to 96 hours, *e.g.,* 12 hours, 24 hours, 36 hours, 48 hours, 60 hours, 72 hours, 84 hours, or 96 hours.
230. The process of any of clauses 202-229, wherein the antibody or antigen binding fragment thereof comprising one or more decapped cysteine residues is stored in the container filled to a maximum of 60%.
231. The process of any of clauses 202-230, wherein the antibody or antigen binding fragment thereof comprising one or more decapped cysteine residues is stored for 48 hours to 72 hours, *e*.*g*., without stirring.
232. The process of any of clauses 202-231, wherein the antibody or antigen binding fragment thereof comprising one or more decapped cysteine residues is stored for 12 hours to 36 hours, *e*.*g*., 24 hours, *e*.*g*., with stirring.
233. The process of any of clauses 202-232, wherein the antibody or antigen binding fragment thereof comprising one or more decapped cysteine residues is stored at room temperature.
234. The process of any of clauses 202-233, wherein the antibody or antigen binding fragment thereof comprising one or more decapped cysteine residues is stored at 2 °C to 8 °C (*e.g.,* 4 °C), *e.g.,* for at least 48 hours (*e*.*g*., 48 hours to 96 hours).
235. The process of any of clauses 202-234, wherein the antibody or antigen binding fragment thereof comprising one or more decapped cysteine residues is stored with air overlay.
236. The process of any of clauses 202-235, wherein the process further comprises purifying the partially reoxidized antibody or antigen binding fragment thereof.
237. The process of any of clauses 202-236, wherein the process further comprises conjugating a linker-drug moiety (*e.g*., a linker-drug moiety described herein) to the partially reoxidized antibody or antigen binding fragment thereof to produce an antibody drug conjugate (e.g., an antibody drug conjugate described herein).
238. The process of any of clauses 202-237, wherein the process further comprises pre-forming a linker-drug moiety of the following Formula (B):

   R⁸-L_{B}-(D)ₙ (B)

   wherein:
   D is a GNAQ inhibitor, a GNA11 inhibitor or an inhibitor of GNAQ and GNA11;
   R⁸ is a reactive group;
   L_{B} is a cleavable or non-cleavable linker, and
   n is 1, 2, 3 or 4;
239. The process of any of clauses 202-238, wherein the process further comprises purifying the antibody drug conjugate.
240. The process of any of clauses 202-239, wherein the process results in at least 75%, *e.g.,* at least 80%, 85%, 90%, or 95%, on-site coupling, *e*.*g*., one linker-drug moiety per heavy chain ("HC1").
241. The process of any of clauses 202-240, wherein the process results in less than 25%, *e.g.,* at least 20%, 15%, 10%, or 5%, off-site coupling, *e*.*g*., one linker-drug moiety per light chain ("LC1") and/or two linker-drug moieties per heavy chain ("HC2").
242. The process of any of clauses 202-241, wherein the process results in at least 70%, *e.g.,* at least 75%, 80%, 85%, 90%, or 95%, purity, *e*.*g*., as determined by non-reduced CE-SDS.
243. A process for producing an anti-PMEL17 antibody drug conjugate, comprising:
   contacting an antibody or antigen binding fragment thereof comprising one or more capped cysteine residues with a reduction wash buffer, thereby providing an antibody or antigen binding fragment comprising one or more decapped cysteine residues;
   storing the antibody or fragment thereof comprising one or more decapped cysteine residues under oxidation conditions, thereby producing a partially reoxidized antibody or antigen binding fragment thereof; and
   conjugating a linker-drug moiety to the partially reoxidized antibody or antigen binding fragment thereof,
   thereby producing the anti-PMEL17 antibody drug conjugate,
   wherein the antibody or antigen binding fragment thereof binds to PMEL17; and
   wherein the linker-drug moiety of the following Formula (B):

      R⁸-L_{B}-(D)ₙ (B)
   wherein:
      D is a GNAQ inhibitor, a GNA11 inhibitor or an inhibitor of GNAQ and GNA11;
      R⁸ is a reactive group;
      L_{B} is a cleavable or non-cleavable linker, and
      n is 1, 2, 3 or 4.
244. A formulation comprising an antibody drug conjugate, a buffering agent, a stabilizing agent, and a surfactant,
   wherein the formulation has a pH of 4.5 to 6.5; and
   wherein the antibody drug conjugate comprises the formula (C)

      Ab-(L_{A}-(D)ₙ)_{y} (C)
   wherein:
      D is a GNAQ inhibitor, a GNA11 inhibitor or an inhibitor of GNAQ and GNA11;
      Ab is an antibody or antigen binding fragment thereof that binds to human PMEL17 protein;
      L_{A} is a linker;
      n is 1, 2, 3 or 4, and
      y is 1, 2, 3 or 4.
245. The formulation of clause 244, wherein antibody drug conjugate is present at a concentration of 5 mg/mL to 30 mg/mL, *e.g.,* 10 mg to 30 mg, 15 mg/mL to 25 mg/mL, 18 mg/mL to 22 mg/mL, 10 mg/mL to 25 mg/mL, 10 mg/mL to 20 mg/mL, 10 mg/mL to 15 mg/mL, 25 mg to 30 mg/mL, 20 mg/mL to 30 mg/mL, 5 mg/mL to 15 mg/mL, 15 mg/mL to 25 mg/mL, or 18 mg/mL to 22 mg/mL, *e.g.,* 5 mg/mL, 6 mg/mL, 7 mg/mL, 8 mg/mL, 9 mg/mL, 10 mg/mL, 11 mg/mL, 12 mg/mL, 13 mg/mL, 14 mg/mL, 15 mg/mL, 16 mg/mL, 17 mg/mL, 18 mg/mL, 19 mg/mL, 20 mg/mL, 21 mg/mL, 22 mg/mL, 23 mg/mL, 24 mg/mL, or 25 mg/mL.
246. The formulation of any of clauses 244 or 245, wherein the antibody drug conjugate is present at a concentration of 15 mg/mL to 25 mg/mL, *e*.*g*., 20 mg/mL.
247. The formulation of any of clauses 244-246, wherein the buffering agent comprises histidine or succinate.
248. The formulation of any of clauses 244-247, wherein the buffering agent is present at a concentration of 5 mM to 50 mM, *e.g.,* 10 mM to 40 nM, 15 mM to 35 mM, 20 mM to 30 mM, 5 mM to 40 mM, 5 mM to 30 mM, 5 mM to 20 mM, 5 mM to 15 mM, 5 mM to 10 mM, 40 mM to 50 mM, 35 mM to 50 mM, 30 mM to 50 mM, 25 mM to 50 mM, 20 mM to 50 mM, 15 mM to 50 mM, 10 mM to 50 mM, 10 mM to 20 mM, 15 mM to 25 mM, 25 mM to 35 mM, 30 mM to 40 mM, or 35 mM to 45 mM, *e.g.,* 5 mM, 10 mM, 15 mM, 20 mM, 25 mM, 30 mM, 35 mM, 40 mM, 45 mM, or 50 mM.
249. The formulation of any of clauses 244-248, wherein the buffering agent is present at a concentration of 15 mM to 25 mM, *e.g.,* 20 mM.
250. The formulation of any of clauses 244-249, wherein the stabilizing agent comprises sucrose or trehalose.
251. The formulation of any of clauses244-250, wherein the stabilizing agent is present at a concentration of 100 mM to 500 mM, *e.g.,* 150 mM to 450 mM, 200 mM to 400 mM, 250 mM to 350 mM, 100 mM to 450 mM, 100 mM to 400 mM, 100 mM to 350 mM, 100 mM to 300 mM, 100 mM to 250 mM, 100 mM to 200 mM, 100 mM to 150 mM, 450 mM to 500 mM, 400 mM to 500 mM, 350 mM to 500 mM, 300 mM to 500 mM, 250 mM to 500 mM, 200 mM to 500 mM, 150 mM to 500 mM, 150 mM to 250 mM, 200 mM to 250 mM, 200 mM to 300 mM, 300 mM to 400 mM, or 350 mM to 450 mM, *e.g.,* 100 mM, 150 mM, 200 mM, 240 mM, 250 mM, 300 mM, 350 mM, 400 mM, 450 mM, or 500 mM.
252. The formulation of any of clauses 244-251, wherein the stabilizing agent and is present at a concentration of 200 mM to 300 mM, *e.g.,* 240 mM.
253. The formulation of any of clauses 244-252, wherein the surfactant comprises polysorbate 20 or polysorbate 80.
254. The formulation of any of clauses 244-253, wherein the surfactant is present at a concentration of 0.01% to 0.06%, *e.g.,* 0.02% to 0.05%, 0.03% to 0.04%, 0.01% to 0.05%, 0.01% to 0.04%, 0.01% to 0.03%, 0.01% to 0.02%, 0.05% to 0.06%, 0.04% to 0.06%, 0.03% to 0.06%, 0.02% to 0.06%, 0.02% to 0.04%, 0.03% to 0.05%, *e.g.,* 0.01%, 0.02%, 0.03%, 0.04%, 0.05%, or 0.06%.
255. The formulation of any of clauses 244-254, wherein the surfactant is present at a concentration of 0.01% to 0.05%, *e.g.,* 0.02%.
256. The formulation of any of clauses 244-255, wherein the formulation has a pH of 4.7 to 5.3, 5.0 to 6.0, 5.2 to 5.8, 5.4 to 5.6, 5.2 to 6.0, 5.4 to 6.0, 5.6 to 6.0, 5.8 to 6.0, 5 to 5.8, 5 to 5.6.0, 5 to 5.4, 5 to 5.2, 4.8 to 5.2, 5.1 to 5.3, 5.2 to 5.4, 5.3 to 5.5, 5.5 to 5.7, 5.6 to 5.8, 5.7 to 5.9, 4.9 to 5.5, 5.5 to 6.1, or 5.7 to 6.3, *e.g.,* 4.5, 4.6, 4.7, 4.8, 4.9, 5, 5.1, 5.2, 5.3, 5.4, 5.5, 5.6, 5.7, 5.8, 5.9, 6.0, 6.1, 6.2, 6.3, 6.4, or 6.5.
257. The formulation of any of clauses 244-256, wherein the formulation has a pH of 5.0 to 5.6, *e.g.,* 5.3.
258. The formulation of any of clauses 244-257, comprising 15 mg/mL to 25 mg/mL of the antibody drug conjugate, 15 mM to 25 mM of a buffering agent comprising histidine, 200 mM to 300 mM of a stabilizing agent comprising sucrose, and 0.01% to 0.03% of a surfactant comprising polysorbate 20, wherein the formulation has a pH of 5.0 to 5.6.
259. The formulation of clause 258, comprising 20 mg/mL of the antibody drug conjugate, 20 mM of a buffering agent comprising histidine, 240 mM of a stabilizing agent comprising sucrose, and 0.02% of a surfactant comprising polysorbate 20, wherein the formulation has a pH of 5.3.
260. The formulation of any of clauses 244-257, comprising 15 mg/mL to 25 mg/mL of the antibody drug conjugate, 15 mM to 25 mM of a buffering agent comprising histidine, 200 mM to 300 mM of a stabilizing agent comprising sucrose, and 0.01% to 0.03% of a surfactant comprising polysorbate 20, wherein the formulation has a pH of 4.7 to 5.3.
261. The formulation of clause 260, comprising 20 mg/mL of the antibody drug conjugate, 20 mM of a buffering agent comprising histidine, 240 mM of a stabilizing agent comprising sucrose, and 0.02% of a surfactant comprising polysorbate 20, wherein the formulation has a pH of 5.0.
262. The formulation of any of clauses 244-257, comprising 15 mg/mL to 25 mg/mL of the antibody drug conjugate, 15 mM to 25 mM of a buffering agent comprising histidine, 200 mM to 300 mM of a stabilizing agent comprising sucrose, and 0.01% to 0.03% of a surfactant comprising polysorbate 20, wherein the formulation has a pH of 5.2 to 5.8.
263. The formulation of clause 262, comprising 20 mg/mL of the antibody drug conjugate, 20 mM of a buffering agent comprising histidine, 240 mM of a stabilizing agent comprising sucrose, and 0.02% of a surfactant comprising polysorbate 20, wherein the formulation has a pH of 5.5.
264. The formulation of any of clauses 244-257, comprising 15 mg/mL to 25 mg/mL of the antibody drug conjugate, 15 mM to 25 mM of a buffering agent comprising histidine, 200 mM to 300 mM of a stabilizing agent comprising sucrose, and 0.03% to 0.05% of a surfactant comprising polysorbate 20, wherein the formulation has a pH of 5.2 to 5.8.
265. The formulation of clause 264, comprising 20 mg/mL of the antibody drug conjugate, 20 mM of a buffering agent comprising histidine, 240 mM of a stabilizing agent comprising sucrose, and 0.04% of a surfactant comprising polysorbate 20, wherein the formulation has a pH of 5.5.
266. The formulation of any of clauses 244-257, comprising 5 mg/mL to 15 mg/mL of the antibody drug conjugate, 15 mM to 25 mM of a buffering agent comprising histidine, 200 mM to 300 mM of a stabilizing agent comprising sucrose, and 0.01% to 0.03% of a surfactant comprising polysorbate 20, wherein the formulation has a pH of 5.2 to 5.8.
267. The formulation of clause 266, comprising 10 mg/mL of the antibody drug conjugate, 20 mM of a buffering agent comprising histidine, 240 mM of a stabilizing agent comprising sucrose, and 0.02% of a surfactant comprising polysorbate 20, wherein the formulation has a pH of 5.5.
268. The formulation of any of clauses 244-257, comprising 15 mg/mL to 25 mg/mL of the antibody drug conjugate, 15 mM to 25 mM of a buffering agent comprising histidine, 200 mM to 300 mM of a stabilizing agent comprising sucrose, and 0.01% to 0.03% of a surfactant comprising polysorbate 20, wherein the formulation has a pH of 5.7 to 6.3.
269. The formulation of clause 268, comprising 20 mg/mL of the antibody drug conjugate, 20 mM of a buffering agent comprising histidine, 240 mM of a stabilizing agent comprising sucrose, and 0.02% of a surfactant comprising polysorbate 20, wherein the formulation has a pH of 6.0.
270. The formulation of any of clauses 244-257, comprising 15 mg/mL to 25 mg/mL of the antibody drug conjugate, 15 mM to 25 mM of a buffering agent comprising histidine, 200 mM to 300 mM of a stabilizing agent comprising sucrose, and 0.01% to 0.03% of a surfactant comprising polysorbate 20, wherein the formulation has a pH of 4.9 to 5.5.
271. The formulation of clause 270, comprising 20 mg/mL of the antibody drug conjugate, 20 mM of a buffering agent comprising histidine, 240 mM of a stabilizing agent comprising sucrose, and 0.02% of a surfactant comprising polysorbate 20, wherein the formulation has a pH of 5.2.
272. The formulation of any of clauses 244-257, comprising 15 mg/mL to 25 mg/mL of the antibody drug conjugate, 15 mM to 25 mM of a buffering agent comprising histidine, 200 mM to 300 mM of a stabilizing agent comprising sucrose, and 0.01% to 0.03% of a surfactant comprising polysorbate 20, wherein the formulation has a pH of 5.5 to 6.1.
273. The formulation of clause 272, comprising 20 mg/mL of the antibody drug conjugate, 20 mM of a buffering agent comprising histidine, 240 mM of a stabilizing agent comprising sucrose, and 0.02% of a surfactant comprising polysorbate 20, wherein the formulation has a pH of 5.8.
274. A lyophilized formulation, which is lyophilized from the formulation of any of clauses 244-273.
275. The lyophilized formulation of clause 274, wherein 5 mL to 5.5 mL of the formulation is lyophilized.
276. The lyophilized formulation of any of clauses 274 or 275, wherein D is at least 1, 2, 3, 4, 5, 6, 7, 8, 9, or 10-fold more stable, compared to an otherwise identical formulation that is not lyophilized, after storage for 0, 4, or 8 weeks, at 5°C or 25°C, *e.g.,* as determined by CZE.
277. The lyophilized formulation of any of clauses 274-276, wherein D is at least 1, 2, 3, 4, 5, 6, 7, 8, 9, or 10-fold more stable, compared to an otherwise identical formulation that is not lyophilized, after storage for 0, 4, or 8 weeks, at 40°C, *e.g.,* as determined by CZE.
278. The lyophilized formulation of any of clauses 274-277, wherein the percentage of D that has a ring-opening conformation is at least 1, 2, 3, 4, 5, 6, 7, 8, 9, or 10-fold lower, compared to an otherwise identical formulation that is not lyophilized, after storage for 0, 4, or 8 weeks, at 5°C or 25°C, *e.g.,* as determined by CZE.
279. The lyophilized formulation of any of clauses 274-278, wherein the percentage of D that has a ring-opening conformation is at least 1, 2, 3, 4, 5, 6, 7, 8, 9, or 10-fold lower, compared to an otherwise identical formulation that is not lyophilized, after storage for 0, 4, or 8 weeks, 40°C, *e.g.,* as determined by CZE.
280. The lyophilized formulation of clause 274-279, which comprises 80 mg to 120 mg (e.g., 107 mg) of the antibody drug conjugate.
281. A liquid formulation, which is reconstituted from the lyophilized formulation of any one of clauses 274-280.
282. The formulation of any of clauses 244-281, wherein the level of monomers in the formulation is at least 95%, *e.g.,* at least 96%, 97%, 98%, or 99%, after storage for 0, 4, or 8 weeks, at 5°C, 25°C, or 40°C, *e.g.,* as determined by SEC.
283. The formulation of any of clauses 244-282, wherein the level of fragments in the formulation is less than 3%, *e.g.,* less than 2.5%, 2%, 1.5%, 1%, or 0.5%, after storage for 0, 4, or 8 weeks, at 5°C, 25°C, or 40°C, *e.g.,* as determined by SEC.
284. The formulation of any of clauses 244-283, wherein the level of aggregates in the formulation is less than 3%, *e.g.,* less than 2.5%, 2%, 1.5%, 1%, or 0.5%, after storage for 0, 4, or 8 weeks, at 5°C, 25°C, or 40°C, *e.g.,* as determined by SEC.
285. The formulation of any of clauses 244-284, wherein the level of degradation is less than 2%, *e.g.,* less than 1.5%, 1%, or 0.5%, after storage for 4 weeks or 8 weeks, at 5°C, 25°C, or 40°C, *e.g.,* as determined by SEC.
286. The formulation of any of clauses 244-285, wherein the level of particles greater than or equal to 10 µm in the formulation is less than about 300 particles/ml, *e.g.*, less than about 280 particles/mL, less than about 260 particles/mL, less than about 240 particles/mL, less than about 220 particles/mL, less than about 200 particles/mL, less than about 180 particles/mL, less than about 160 paricles/mL, less than about 140 particles/mL, less than about 120 particles/mL, less than 100 particles/mL, 80 particles/mL, 60 particles/mL, 40 particles/mL, 20 particles/mL, or 10 particles/mL, after storage for 0, 4, or 8 weeks, at 5°C, 25°C, or 40°C, *e.g.,* as determined by light obscuration.
287. The formulation of any of clauses 244-286, wherein the level of particles greater than or equal to 10 µm in the formulation is increased by no more than 3-fold, *e.g.,* no more than 2.5, 2, 1.5, 1, or 0.5-fold, after storage for 4 or 8 weeks, at 5°C, 25°C, or 40°C, *e.g.,* as determined by light obscuration.
288. The formulation of any of clauses 244-287, wherein the level of particles greater than 25 µm in the formulation is increased by no more than 3-fold, *e.g.,* no more than 2.5, 2, 1.5, 1, or 0.5-fold, after storage for 4 or 8 weeks, at 5°C, 25°C, or 40°C, *e.g.,* as determined by light obscuration.
289. The formulation of any of clauses 244-288, wherein the level of particles greater than 25 µm in the formulation is less than 20 particles/mL, *e.g.*, less than 15 particles/mL, 10 particles/mL, 5 particles/mL, or 2 particles/mL, after storage for 0, 4, or 8 weeks, at 5°C, 25°C, or 40°C, *e.g.,* as determined by light obscuration.
290. The formulation of any of clauses 244-289, wherein the level of impurity is less than 15%, *e.g.,* less than 14%, 13%, 12%, 11%, 10%, 9%, 8%, 7%, 6%, 5%, 4%, 3%, 2%, or 1%, after storage for 0, 4, or 8 weeks, at 5°C, 25°C, or 40°C, *e.g.,* as determined by capillary electrophoresis.
291. The formulation of any of clauses 244-290, wherein the level of impurity is increased by no more than 20%, *e.g.,* no more than 15%, 10%, 5%, 2%, or 1%, after storage for 4 weeks or 8 weeks, at 5°C, 25°C, or 40°C, *e.g.,* as determined by capillary electrophoresis.
292. The formulation of any of clauses 244-291, wherein the level of neutral variants is greater than *50%, e.g.,* greater than 55%, 60%, 65%, 70%, 75%, 80%, 85%, or 90%, after storage for 0, 4, or 8 weeks, at 5°C, 25°C, or 40°C, *e.g.,* as determined by capillary zone electrophoresis (CZE).
293. The formulation of any of clauses 244-292, wherein the level of neutral variants is decreased by no more than 20%, *e.g.,* no more than 15%, 10%, 5%, or 2%, after storage for 4 weeks or 8 weeks, at 5°C, 25°C, or 40°C, *e.g.,* as determined by CZE.
294. The formulation of any of clauses 244-293, wherein the level of acidic variants is less than 30%, *e.g.,* less than 25%, 20%, 15%, 5%, or 2%, after storage for 0, 4, or 8 weeks, at 5°C, 25°C, or 40°C, *e.g.,* as determined by CZE.
295. The formulation of any of clauses 244-294, wherein the level of acid variants is increased by no more than 50%, *e.g.,* no more than 40%, 30%, 20%, 10%, or 5%, after storage for 4 weeks or 8 weeks, at 5°C, 25°C, or 40°C, *e.g.,* as determined by CZE.
296. The formulation of any of clauses 244-295, wherein the level of basic variants is less than 30%, *e.g.,* less than 25%, 20%, 15%, 5%, or 2%, after storage for 0, 4, or 8 weeks, at 5°C, 25°C, or 40°C, *e.g.,* as determined by CZE.
297. The formulation of any of clauses 244-296, wherein the level of basic variants is increased by no more than 50%, *e.g.,* no more than 40%, 30%, 20%, 10%, or 5%, after storage for 4 weeks or 8 weeks, at 5°C, 25°C, or 40°C, *e.g.,* as determined by CZE.
298. The formulation of any of clauses 244-297, wherein the potency is decreased by no more than *25%, e.g.,* no more than 20%, 15%, 10%, 5%, or 2%, after storage for 8 weeks, at 5°C, 25°C, or 40°C, *e.g.,* as determined by a bioactivity assay.
299. The formulation of any of clauses 244-298, wherein the osmolality of the formulation is 200 mOsm/L to 400 mOsm/L, *e.g.*, 200 mOsm/L to 300 mOsm/L, 250 mOsm/L to 350 mOsm/L, 270 mOsm/L to 330 mOsm/L, 290 mOsm/L to 310 mOsm/L, 270 mOsm/L to 350 mOsm/L, 290 mOsm/L to 350 mOsm/L, 310 mOsm/L to 350 mOsm/L, 330 mOsm/L to 350 mOsm/L, 250 mOsm/L to 330 mOsm/L, 250 mOsm/L to 310 mOsm/L, 250 mOsm/L to 290 mOsm/L, 250 mOsm/L to 270 mOsm/L, 260 mOsm/L to 280 mOsm/L, 270 mOsm/L to 290 mOsm/L, 280 mOsm/L to 300 mOsm/L, 300 mOsm/L to 320 mOsm/L, 310 mOsm/L to 330 mOsm/L, or 320 mOsm/L to 340 mOsm/L, *e.g.*, 200 mOsm/L, 250 mOsm/L, 260 mOsm/L, 270 mOsm/L, 280 mOsm/L, 290 mOsm/L, 300 mOsm/L, 310 mOsm/L, 320 mOsm/L, 330 mOsm/L, 340 mOsm/L, 350 mOsm/L, or 400 mOsm/L.
300. A container comprising the lyophilized formulation of any of clauses 274-299.
301. The container of clause 300, which is a vial, *e.g.,* a 20R glass vial or a 25 R glass vial.
302. The container of any of clauses 300 or 301, which further comprises a stopper and a cap (e.g., a flip-off aluminum cap).
303. A method of treating or preventing cancer in a subject in need thereof, comprising administering to the subject a formulation of any one of clauses 244-299, wherein the cancer expresses PMEL17, contains a mutation of the GNAQ or GNA11 gene, or the cancer expresses PMEL17 and contains a mutation of GNAQ, GNA11, or both.
304. The method of clause 303, wherein the cancer is a carcinoma, sarcoma, leukemia, lymphoma, eye cancer, eye neoplasm, melanoma, or a metastatic cancer thereof.
305. The method of clause 304, wherein the carcinoma is a hepatocellular carcinoma, or a metastatic lesion thereof.
306. The method of clause 304, wherein the melanoma is uveal melanoma, non-uveal melanoma, malignant melanoma, ocular melanoma, mucosal melanoma, subcutaneous melanoma, cutaneous melanoma, or a metastatic lesion thereof.
307. The method of any of clauses 303-306, wherein the formulation is administered to the patient in combination with one or more additional therapeutic compounds.
308. The method of clauses 303-307, wherein the one or more additional therapeutic compounds is selected from a standard of care chemotherapeutic, an MDM2 inhibitor, an MRC2 inhibitor, a PKC inhibitor, a MAPK inhibitor, a costimulatory molecule, or a checkpoint inhibitor.
309. The method of clause 308, wherein the costimulatory molecule is selected from an agonist of OX40, CD2, CD27, CDS, ICAM-1, LFA-1 (CD11a/CD18), ICOS (CD278), 4-1BB (CD137), GITR, CD30, CD40, BAFFR, HVEM, CD7, LIGHT, NKG2C, SLAMF7, NKp80, CD160, B7-H3, STING, or CD83 ligand.
310. The method of clause 309, wherein the checkpoint inhibitor is selected from an inhibitor of PD-1, PD-L1, PD-L2, CTLA4, TIM3, LAG3, VISTA, BTLA, TIGIT, LAIR1, CD160, 2B4 and/or TGFR beta.
311. A formulation of any of clauses 244-299 for use in a method of treating or preventing cancer in a subject in need thereof, comprising administering to the subject a formulation of any one of clauses 244-299, wherein the cancer expresses PMEL17, contains a mutation of the GNAQ or GNA11 gene, or the cancer expresses PMEL17 and contains a mutation of GNAQ, GNA11, or both.
312. The formulation for use of clause 311, wherein the cancer is a carcinoma, sarcoma, leukemia, lymphoma, eye cancer, eye neoplasm, melanoma, or a metastatic lesion thereof.
313. The formulation for use of clause 312, wherein the carcinoma is a hepatocellular carcinoma, or a metastatic lesion thereof.
314. The formulation for use of clause 312, wherein the melanoma is uveal melanoma, non-uveal melanoma, malignant melanoma, ocular melanoma, mucosal melanoma, subcutaneous melanoma, cutaneous melanoma, or a metastatic lesion thereof.
315. The formulation for use of any of clauses 311-314, wherein the formulation is administered to the patient in combination with one or more additional therapeutic compounds.
316. The formulation for use of any of clauses 311-315, wherein the one or more additional therapeutic compounds is selected from a standard of care chemotherapeutic, an MDM2 inhibitor, an MRC2 inhibitor, a PKC inhibitor, a MAPK inhibitor, a costimulatory molecule, or a checkpoint inhibitor.
317. The formulation for use of any of clauses 311-316, wherein the costimulatory molecule is selected from an agonist of OX40, CD2, CD27, CDS, ICAM-1, LFA-1 (CD11a/CD18), ICOS (CD278), 4-1BB (CD137), GITR, CD30, CD40, BAFFR, HVEM, CD7, LIGHT, NKG2C, SLAMF7, NKp80, CD160, B7-H3, STING, or CD83 ligand.
318. The formulation for use of any of clauses 311-317, wherein the checkpoint inhibitor is selected from an inhibitor of PD-1, PD-L1, PD-L2, CTLA4, TIM3, LAG3, VISTA, BTLA, TIGIT, LAIR1, CD160, 2B4 and/or TGFR beta.
319. The process of any of clauses 202-243, the formulation of any of clauses 244-299, the container of any of clauses 300-302, the method of any of clauses 303-310, or the formulation for use of any of clauses 311-318, wherein the antibody or antigen binding fragment thereof that binds PMEL17 comprises:
   (a) a heavy chain variable region that comprises a heavy chain CDR1 (Complementarity Determining Region 1) of SEQ ID NO:1, 4, 5 or 7, a heavy chain CDR2 (Complementarity Determining Region 2) of SEQ ID NO:2, 6 or 8, and a heavy chain CDR3 (Complementarity Determining Region 3) of SEQ ID NO:3 or 9; and a light chain variable region that comprises a light chain CDR1 (Complementarity Determining Region 1) of SEQ ID NO:14, 17 or 20, a light chain CDR2 (Complementarity Determining Region 2) of SEQ ID NO: 15 or 18, and a light chain CDR3 (Complementarity Determining Region 3) of SEQ ID NO:16 or 19;
   (b) a heavy chain variable region that comprises a heavy chain CDR1 of SEQ ID NO:33, 36, 37 or 39, a heavy chain CDR2 of SEQ ID NO:34, 38 or 40; a heavy chain CDR3 of SEQ ID NO:35 or 41; a light chain CDR1 of SEQ ID NO:46, 49 or 52; a light chain CDR2 of SEQ ID NO:47 or 50; and a light chain CDR3 of SEQ ID NO:48 or 51;
   (c) a heavy chain variable region that comprises a heavy chain CDR1 of SEQ ID NO:5, 7, 57 or 60, a heavy chain CDR2 of SEQ ID NO:58, 61 or 62; a heavy chain CDR3 of SEQ ID NO:59 or 63; a light chain CDR1 of SEQ ID NO:68, 71 or 74; a light chain CDR2 of SEQ ID NO:69 or 72; and a light chain CDR3 of SEQ ID NO:70 or 73;
   (d) a heavy chain variable region that comprises a heavy chain CDR1 of SEQ ID NO:79, 82, 83 or 85, a heavy chain CDR2 of SEQ ID NO:80, 84 or 86; a heavy chain CDR3 of SEQ ID NO:81 or 87; a light chain CDR1 of SEQ ID NO:92, 95 or 98; a light chain CDR2 of SEQ ID NO:93 or 96; and a light chain CDR3 of SEQ ID NO:94 or 97;
   (e) a heavy chain variable region that comprises a heavy chain CDR1 of SEQ ID NO:103, 106, 107 or 109, a heavy chain CDR2 of SEQ ID NO:104, 108 or 110; a heavy chain CDR3 of SEQ ID NO:105 or 111; a light chain CDR1 of SEQ ID NO:49, 52 or 116; a light chain CDR2 of SEQ ID NO:47 or 50; and a light chain CDR3 of SEQ ID NO:117 or 118;
   (f) a heavy chain variable region that comprises a heavy chain CDR1 of SEQ ID NO:123, 126, 127 or 129, a heavy chain CDR2 of SEQ ID NO:124, 128 or 130; a heavy chain CDR3 of SEQ ID NO:125 or 131; a light chain CDR1 of SEQ ID NO:136, 139 or 142; a light chain CDR2 of SEQ ID NO:137 or 140; and a light chain CDR3 of SEQ ID NO:138 or 141;
   (g) a heavy chain variable region that comprises a heavy chain CDR1 of SEQ ID NO:123, 126, 127 or 129, a heavy chain CDR2 of SEQ ID NO:124, 128 or 130; a heavy chain CDR3 of SEQ ID NO:147 or 148; a light chain CDR1 of SEQ ID NO:153, 156 or 158; a light chain CDR2 of SEQ ID NO:50 or 154; and a light chain CDR3 of SEQ ID NO:155 or 157;
   (h) a heavy chain variable region that comprises a heavy chain CDR1 of SEQ ID NO:103, 106, 107 or 109, a heavy chain CDR2 of SEQ ID NO:104, 108 or 110; a heavy chain CDR3 of SEQ ID NO:163 or 164; a light chain CDR1 of SEQ ID NO:49, 52 or 116; a light chain CDR2 of SEQ ID NO:47 or 50; and a light chain CDR3 of SEQ ID NO:169 or 170;
   (i) a heavy chain variable region that comprises a heavy chain CDR1 of SEQ ID NO:175, 178, 179 or 181, a heavy chain CDR2 of SEQ ID NO:176, 180 or 182; a heavy chain CDR3 of SEQ ID NO:177 or 183; a light chain CDR1 of SEQ ID NO:49, 52 or 116; a light chain CDR2 of SEQ ID NO:47 or 50; and a light chain CDR3 of SEQ ID NO:188 or 189;
   (j) a heavy chain variable region that comprises a heavy chain CDR1 of SEQ ID NO: 103, 106, 107 or 109, a heavy chain CDR2 of SEQ ID NO: 104, 108 or 110; a heavy chain CDR3 of SEQ ID NO:194 or 195; a light chain CDR1 of SEQ ID NO: 49, 52 or 116; a light chain CDR2 of SEQ ID NO: 47 or 50; and a light chain CDR3 of SEQ ID NO:200 or 201;
   (k) a heavy chain variable region that comprises a heavy chain CDR1 of SEQ ID NO:206, 209, 210 or 212, a heavy chain CDR2 of SEQ ID NO:207, 211 or 213; a heavy chain CDR3 of SEQ ID NO:208 or 214; a light chain CDR1 of SEQ ID NO:153, 156 or 158; a light chain CDR2 of SEQ ID NO:50 or 154; and a light chain CDR3 of SEQ ID NO:219 or 220;
   (l) a heavy chain variable region that comprises a heavy chain CDR1 of SEQ ID NO: 206, 209, 210 or 212, a heavy chain CDR2 of SEQ ID NO: 207, 211 or 213; a heavy chain CDR3 of SEQ ID NO:225 or 226; a light chain CDR1 of SEQ ID NO:136, 139 or 142; a light chain CDR2 of SEQ ID NO:137 or 140; and a light chain CDR3 of SEQ ID NO:231 or 232;
   (m) a heavy chain variable region that comprises a heavy chain variable region that comprises an HCDR1 of SEQ ID NO: 206, 209, 210 or 212, an HCDR2 of SEQ ID NO: 207, 211 or 213, and an HCDR3 of SEQ ID NO:237 or 238; and a light chain variable region that comprises an LCDR1 of SEQ ID NO:243, 245 or 247, an LCDR2 of SEQ ID NO:47 or 50, and an LCDR3 of SEQ ID NO:244 or 246;
   (n) a heavy chain variable region that comprises a heavy chain variable region that comprises an HCDR1 of SEQ ID NO: 206, 209, 210 or 212, an HCDR2 of SEQ ID NO: 207, 211 or 213, and an HCDR3 of SEQ ID NO:252 or 253; and a light chain variable region that comprises an LCDR1 of SEQ ID NO:153, 156 or 158, an LCDR2 of SEQ ID NO:50 or 154, and an LCDR3 of SEQ ID NO:258 or 259;
   (o) a heavy chain CDR1 of SEQ ID NO:1, a heavy chain CDR2 of SEQ ID NO:2, a heavy chain CDR3 of SEQ ID NO:3, a light chain CDR1 of SEQ ID NO:14, a light chain CDR2 of SEQ ID NO:15, and a light chain CDR3 of SEQ ID NO:16;
   (p) a heavy chain CDR1 of SEQ ID NO: 4, a heavy chain CDR2 of SEQ ID NO:2, a heavy chain CDR3 of SEQ ID NO:3, a light chain CDR1 of SEQ ID NO:14, a light chain CDR2 of SEQ ID NO:15, and a light chain CDR3 of SEQ ID NO:16;
   (q) a heavy chain CDR1 of SEQ ID NO:5, a heavy chain CDR2 of SEQ ID NO:6, a heavy chain CDR3 of SEQ ID NO:3, a light chain CDR1 of SEQ ID NO:17, a light chain CDR2 of SEQ ID NO: 18, and a light chain CDR3 of SEQ ID NO: 19;
   (r) a heavy chain CDR1 of SEQ ID NO:7, a heavy chain CDR2 of SEQ ID NO:8, a heavy chain CDR3 of SEQ ID NO:9, a light chain CDR1 of SEQ ID NO:20, a light chain CDR2 of SEQ ID NO:18, and a light chain CDR3 of SEQ ID NO:16;
   (s) a heavy chain CDR1 of SEQ ID NO:33, a heavy chain CDR2 of SEQ ID NO:34, a heavy chain CDR3 of SEQ ID NO:35, a light chain CDR1 of SEQ ID NO:46, a light chain CDR2 of SEQ ID NO:47, and a light chain CDR3 of SEQ ID NO:48;
   (t) a heavy chain CDR1 of SEQ ID NO:36, a heavy chain CDR2 of SEQ ID NO:34, a heavy chain CDR3 of SEQ ID NO:35, a light chain CDR1 of SEQ ID NO:46, a light chain CDR2 of SEQ ID NO:47, and a light chain CDR3 of SEQ ID NO:48;
   (u) a heavy chain CDR1 of SEQ ID NO:37, a heavy chain CDR2 of SEQ ID NO:38, a heavy chain CDR3 of SEQ ID NO:35, a light chain CDR1 of SEQ ID NO:49, a light chain CDR2 of SEQ ID NO:50, and a light chain CDR3 of SEQ ID NO:51;
   (v) a heavy chain CDR1 of SEQ ID NO: 39, a heavy chain CDR2 of SEQ ID NO:40, a heavy chain CDR3 of SEQ ID NO:41, a light chain CDR1 of SEQ ID NO:52, a light chain CDR2 of SEQ ID NO:50, and a light chain CDR3 of SEQ ID NO:48;
   (w) a heavy chain CDR1 of SEQ ID NO:57, a heavy chain CDR2 of SEQ ID NO:58, a heavy chain CDR3 of SEQ ID NO:59, a light chain CDR1 of SEQ ID NO:68, a light chain CDR2 of SEQ ID NO:69, and a light chain CDR3 of SEQ ID NO:70;
   (x) a heavy chain CDR1 of SEQ ID NO:60, a heavy chain CDR2 of SEQ ID NO:58, a heavy chain CDR3 of SEQ ID NO:59, a light chain CDR1 of SEQ ID NO:68, a light chain CDR2 of SEQ ID NO:69, and a light chain CDR3 of SEQ ID NO:70;
   (y) a heavy chain CDR1 of SEQ ID NO:5, a heavy chain CDR2 of SEQ ID NO:61, a heavy chain CDR3 of SEQ ID NO:59, a light chain CDR1 of SEQ ID NO:71, a light chain CDR2 of SEQ ID NO:72, and a light chain CDR3 of SEQ ID NO:73;
   (z) a heavy chain CDR1 of SEQ ID NO:7, a heavy chain CDR2 of SEQ ID NO:62, a heavy chain CDR3 of SEQ ID NO:63, a light chain CDR1 of SEQ ID NO:74, a light chain CDR2 of SEQ ID NO:72, and a light chain CDR3 of SEQ ID NO:70;
   (aa) a heavy chain CDR1 of SEQ ID NO:79, , a heavy chain CDR2 of SEQ ID NO:80, a heavy chain CDR3 of SEQ ID NO:81, a light chain CDR1 of SEQ ID NO:92, a light chain CDR2 of SEQ ID NO:93, and a light chain CDR3 of SEQ ID NO:94;
   (bb) a heavy chain CDR1 of SEQ ID NO:82, a heavy chain CDR2 of SEQ ID NO:80, a heavy chain CDR3 of SEQ ID NO:81, a light chain CDR1 of SEQ ID NO:92, a light chain CDR2 of SEQ ID NO:93, and a light chain CDR3 of SEQ ID NO:94;
   (cc) a heavy chain CDR1 of SEQ ID NO:83, a heavy chain CDR2 of SEQ ID NO:84, a heavy chain CDR3 of SEQ ID NO:81, a light chain CDR1 of SEQ ID NO:95, a light chain CDR2 of SEQ ID NO:96, and a light chain CDR3 of SEQ ID NO: 97;
   (dd) a heavy chain CDR1 of SEQ ID NO: 85, a heavy chain CDR2 of SEQ ID NO:86, a heavy chain CDR3 of SEQ ID NO:87, a light chain CDR1 of SEQ ID NO:98, a light chain CDR2 of SEQ ID NO:96, and a light chain CDR3 of SEQ ID NO:94;
   (ee) a heavy chain CDR1 of SEQ ID NO:103, a heavy chain CDR2 of SEQ ID NO:104, a heavy chain CDR3 of SEQ ID NO:105, a light chain CDR1 of SEQ ID NO: 116; a light chain CDR2 of SEQ ID NO:47; and a light chain CDR3 of SEQ ID NO:117;
   (ff) a heavy chain CDR1 of SEQ ID NO:106, a heavy chain CDR2 of SEQ ID NO:104, a heavy chain CDR3 of SEQ ID NO:105, a light chain CDR1 of SEQ ID NO: 116, a light chain CDR2 of SEQ ID NO:47, and a light chain CDR3 of SEQ ID NO:117;
   (gg) a heavy chain CDR1 of SEQ ID NO:107, a heavy chain CDR2 of SEQ ID NO:108, a heavy chain CDR3 of SEQ ID NO:105, a light chain CDR1 of SEQ ID NO:49, a light chain CDR2 of SEQ ID NO:50, and a light chain CDR3 of SEQ ID NO:118;
   (hh) a heavy chain CDR1 of SEQ ID NO:109, a heavy chain CDR2 of SEQ ID NO:110, a heavy chain CDR3 of SEQ ID NO:111, a light chain CDR1 of SEQ ID NO:52 a light chain CDR2 of SEQ ID NO:50, and a light chain CDR3 of SEQ ID NO:117;
   (ii) a heavy chain CDR1 of SEQ ID NO:123, a heavy chain CDR2 of SEQ ID NO:124, a heavy chain CDR3 of SEQ ID NO:125, a light chain CDR1 of SEQ ID NO:136, a light chain CDR2 of SEQ ID NO:137,and a light chain CDR3 of SEQ ID NO:138;
   (jj) a heavy chain CDR1 of SEQ ID NO:126, a heavy chain CDR2 of SEQ ID NO:124, a heavy chain CDR3 of SEQ ID NO:125, a light chain CDR1 of SEQ ID NO:136, a light chain CDR2 of SEQ ID NO:137, and a light chain CDR3 of SEQ ID NO:138;
   (kk) a heavy chain CDR1 of SEQ ID NO:127, a heavy chain CDR2 of SEQ ID NO:128, a heavy chain CDR3 of SEQ ID NO:125, a light chain CDR1 of SEQ ID NO:139, a light chain CDR2 of SEQ ID NO:140, and a light chain CDR3 of SEQ ID NO: 141;
   (ll) a heavy chain CDR1 of SEQ ID NO: 129, a heavy chain CDR2 of SEQ ID NO:130, a heavy chain CDR3 of SEQ ID NO:131, a light chain CDR1 of SEQ ID NO:142, a light chain CDR2 of SEQ ID NO:140, and a light chain CDR3 of SEQ ID NO:138;
   (mm) a heavy chain CDR1 of SEQ ID NO:123, a heavy chain CDR2 of SEQ ID NO:124, a heavy chain CDR3 of SEQ ID NO:147, a light chain CDR1 of SEQ ID NO:153, a light chain CDR2 of SEQ ID NO:154, and a light chain CDR3 of SEQ ID NO:155;
   (nn) a heavy chain CDR1 of SEQ ID NO:126, a heavy chain CDR2 of SEQ ID NO:124, a heavy chain CDR3 of SEQ ID NO:147, a light chain CDR1 of SEQ ID NO:153, a light chain CDR2 of SEQ ID NO: 154, and a light chain CDR3 of SEQ ID NO:155;
   (oo) a heavy chain CDR1 of SEQ ID NO:127, a heavy chain CDR2 of SEQ ID NO:128, a heavy chain CDR3 of SEQ ID NO:147, a light chain CDR1 of SEQ ID NO:156, a light chain CDR2 of SEQ ID NO:50, and a light chain CDR3 of SEQ ID NO:157;
   (pp) a heavy chain CDR1 of SEQ ID NO: 129, a heavy chain CDR2 of SEQ ID NO:130, a heavy chain CDR3 of SEQ ID NO:148, a light chain CDR1 of SEQ ID NO:158, a light chain CDR2 of SEQ ID NO:50, and a light chain CDR3 of SEQ ID NO:155;
   (qq) a heavy chain CDR1 of SEQ ID NO:103, a heavy chain CDR2 of SEQ ID NO:104, a heavy chain CDR3 of SEQ ID NO:163, a light chain CDR1 of SEQ ID NO: 116, a light chain CDR2 of SEQ ID NO:47, and a light chain CDR3 of SEQ ID NO:169;
   (rr) a heavy chain CDR1 of SEQ ID NO:106, a heavy chain CDR2 of SEQ ID NO:104, a heavy chain CDR3 of SEQ ID NO:163, a light chain CDR1 of SEQ ID NO:116, a light chain CDR2 of SEQ ID NO:47, and a light chain CDR3 of SEQ ID NO:169;
   (ss) a heavy chain CDR1 of SEQ ID NO:107, a heavy chain CDR2 of SEQ ID NO:108, a heavy chain CDR3 of SEQ ID NO:163, a light chain CDR1 of SEQ ID NO:49, a light chain CDR2 of SEQ ID NO:50, and a light chain CDR3 of SEQ ID NO:170;
   (tt) a heavy chain CDR1 of SEQ ID NO: 109, a heavy chain CDR2 of SEQ ID NO:110, a heavy chain CDR3 of SEQ ID NO:164, a light chain CDR1 of SEQ ID NO:52, a light chain CDR2 of SEQ ID NO:50, and a light chain CDR3 of SEQ ID NO:169;
   (uu) a heavy chain CDR1 of SEQ ID NO:175, a heavy chain CDR2 of SEQ ID NO:176, a heavy chain CDR3 of SEQ ID NO:177, a light chain CDR1 of SEQ ID NO:116, a light chain CDR2 of SEQ ID NO:47, and a light chain CDR3 of SEQ ID NO:188;
   (vv) a heavy chain CDR1 of SEQ ID NO:178, a heavy chain CDR2 of SEQ ID NO:176, a heavy chain CDR3 of SEQ ID NO:177, a light chain CDR1 of SEQ ID NO:116, a light chain CDR2 of SEQ ID NO:47, and a light chain CDR3 of SEQ ID NO:188;
   (ww) a heavy chain CDR1 of SEQ ID NO:179, a heavy chain CDR2 of SEQ ID NO:180, a heavy chain CDR3 of SEQ ID NO:177, a light chain CDR1 of SEQ ID NO:49, a light chain CDR2 of SEQ ID NO:50, and a light chain CDR3 of SEQ ID NO:189;
   (xx) a heavy chain CDR1 of SEQ ID NO: 181, a heavy chain CDR2 of SEQ ID NO:182; a heavy chain CDR3 of SEQ ID NO:183, a light chain CDR1 of SEQ ID NO:52, a light chain CDR2 of SEQ ID NO:50, and a light chain CDR3 of SEQ ID NO:188;
   (yy) a heavy chain CDR1 of SEQ ID NO: 103, a heavy chain CDR2 of SEQ ID NO: 104, a heavy chain CDR3 of SEQ ID NO:194, a light chain CDR1 of SEQ ID NO: 116, a light chain CDR2 of SEQ ID NO: 47, and a light chain CDR3 of SEQ ID NO:200;
   (zz) a heavy chain CDR1 of SEQ ID NO: 106, a heavy chain CDR2 of SEQ ID NO: 104, a heavy chain CDR3 of SEQ ID NO:194, a light chain CDR1 of SEQ ID NO: 116, a light chain CDR2 of SEQ ID NO: 47, and a light chain CDR3 of SEQ ID NO:200;
   (aaa) a heavy chain CDR1 of SEQ ID NO: 107, a heavy chain CDR2 of SEQ ID NO: 108, a heavy chain CDR3 of SEQ ID NO: 194, a light chain CDR1 of SEQ ID NO: 49, a light chain CDR2 of SEQ ID NO: 50, and a light chain CDR3 of SEQ ID NO: 201;
   (bbb) a heavy chain CDR1 of SEQ ID NO: 109, a heavy chain CDR2 of SEQ ID NO: 110, a heavy chain CDR3 of SEQ ID NO: 195, a light chain CDR1 of SEQ ID NO: 52, a light chain CDR2 of SEQ ID NO: 50, and a light chain CDR3 of SEQ ID NO:200;
   (ccc) a heavy chain CDR1 of SEQ ID NO:206, a heavy chain CDR2 of SEQ ID NO:207, a heavy chain CDR3 of SEQ ID NO:208, a light chain CDR1 of SEQ ID NO:153, a light chain CDR2 of SEQ ID NO:154, and a light chain CDR3 of SEQ ID NO:219;
   (ddd) a heavy chain CDR1 of SEQ ID NO:209, a heavy chain CDR2 of SEQ ID NO:207, a heavy chain CDR3 of SEQ ID NO:208, a light chain CDR1 of SEQ ID NO:153, a light chain CDR2 of SEQ ID NO: 154, and a light chain CDR3 of SEQ ID NO:219;
   (eee) a heavy chain CDR1 of SEQ ID NO:210, a heavy chain CDR2 of SEQ ID NO:211, a heavy chain CDR3 of SEQ ID NO:208, a light chain CDR1 of SEQ ID NO:156, a light chain CDR2 of SEQ ID NO:50, and a light chain CDR3 of SEQ ID NO:220;
   (fff) a heavy chain CDR1 of SEQ ID NO: 212, a heavy chain CDR2 of SEQ ID NO:213, a heavy chain CDR3 of SEQ ID NO:214, a light chain CDR1 of SEQ ID NO:158, a light chain CDR2 of SEQ ID NO:50, and a light chain CDR3 of SEQ ID NO:219;
   (ggg) a heavy chain CDR1 of SEQ ID NO: 206, a heavy chain CDR2 of SEQ ID NO: 207, a heavy chain CDR3 of SEQ ID NO:225, a light chain CDR1 of SEQ ID NO:136, a light chain CDR2 of SEQ ID NO:137,and a light chain CDR3 of SEQ ID NO:231;
   (hhh) a heavy chain CDR1 of SEQ ID NO: 209, a heavy chain CDR2 of SEQ ID NO: 207, a heavy chain CDR3 of SEQ ID NO:225, a light chain CDR1 of SEQ ID NO:136, a light chain CDR2 of SEQ ID NO:137, and a light chain CDR3 of SEQ ID NO:231;
   (iii) a heavy chain CDR1 of SEQ ID NO: 210, a heavy chain CDR2 of SEQ ID NO: 211, a heavy chain CDR3 of SEQ ID NO:225, a light chain CDR1 of SEQ ID NO:139, a light chain CDR2 of SEQ ID NO:140, and a light chain CDR3 of SEQ ID NO: 232;
   (jjj) a heavy chain CDR1 of SEQ ID NO: 212, a heavy chain CDR2 of SEQ ID NO: 213, a heavy chain CDR3 of SEQ ID NO: 226, a light chain CDR1 of SEQ ID NO:142; a light chain CDR2 of SEQ ID NO: 140; and a light chain CDR3 of SEQ ID NO:231;
   (kkk) a heavy chain variable region that comprises an HCDR1 of SEQ ID NO: 206, an HCDR2 of SEQ ID NO: 207, and an HCDR3 of SEQ ID NO:237,and a light chain variable region that comprises an LCDR1 of SEQ ID NO:243, an LCDR2 of SEQ ID NO:47, and an LCDR3 of SEQ ID NO:244;
   (lll) a heavy chain variable region that comprises an HCDR1 of SEQ ID NO: 209, an HCDR2 of SEQ ID NO: 207, and an HCDR3 of SEQ ID NO:237, and a light chain variable region that comprises an LCDR1 of SEQ ID NO:243, an LCDR2 of SEQ ID NO:47, and an LCDR3 of SEQ ID NO:244;
   (mmm) a heavy chain variable region that comprises an HCDR1 of SEQ ID NO: 210, an HCDR2 of SEQ ID NO: 211, and an HCDR3 of SEQ ID NO:237, and a light chain variable region that comprises an LCDR1 of SEQ ID NO:245, an LCDR2 of SEQ ID NO:50, and an LCDR3 of SEQ ID NO:246;
   (nnn) a heavy chain variable region that comprises an HCDR1 of SEQ ID NO: 212, an HCDR2 of SEQ ID NO: 213, and an HCDR3 of SEQ ID NO:238; and a light chain variable region that comprises an LCDR1 of SEQ ID NO:247, an LCDR2 of SEQ ID NO: 50, and an LCDR3 of SEQ ID NO:244;
   (ooo) a heavy chain variable region that comprises an HCDR1 of SEQ ID NO: 206, an HCDR2 of SEQ ID NO: 207, and an HCDR3 of SEQ ID NO:252,and a light chain variable region that comprises an LCDR1 of SEQ ID NO:153, an LCDR2 of SEQ ID NO: 154, and an LCDR3 of SEQ ID NO:258;
   (ppp) a heavy chain variable region that comprises an HCDR1 of SEQ ID NO: 209, an HCDR2 of SEQ ID NO: 207, and an HCDR3 of SEQ ID NO:252, and a light chain variable region that comprises an LCDR1 of SEQ ID NO:153, an LCDR2 of SEQ ID NO:154, and an LCDR3 of SEQ ID NO:258;
   (qqq) a heavy chain variable region that comprises an HCDR1 of SEQ ID NO: 210, an HCDR2 of SEQ ID NO: 211, and an HCDR3 of SEQ ID NO:252, and a light chain variable region that comprises an LCDR1 of SEQ ID NO:156, an LCDR2 of SEQ ID NO:50, and an LCDR3 of SEQ ID NO:259; or
   (rrr) a heavy chain variable region that comprises an HCDR1 of SEQ ID NO: 212, an HCDR2 of SEQ ID NO: 213, and an HCDR3 of SEQ ID NO: 253; and a light chain variable region that comprises an LCDR1 of SEQ ID NO:158, an LCDR2 of SEQ ID NO:50, and an LCDR3 of SEQ ID NO:258.
320. The process of any of clauses 202-243 or 319, the formulation of any of clauses 244-299 or 319, the container of any of clauses 300-302 or 319, the method of any of clauses 303-310 or 319, or the formulation for use of any of clauses 311-319, wherein the antibody or antigen binding fragment thereof that binds PMEL17 comprises:
   (a) a heavy chain variable region (VH) comprising the amino acid sequence of SEQ ID NO: 10, and a light chain variable region (VL) comprising the amino acid sequence of SEQ ID NO:21;
   (b) a heavy chain variable region (VH) comprising the amino acid sequence of SEQ ID NO:10, and a light chain variable region (VL) comprising the amino acid sequence of SEQ ID NO:25;
   (c) a heavy chain variable region (VH) comprising the amino acid sequence of SEQ ID NO: 10, and a light chain variable region (VL) comprising the amino acid sequence of SEQ ID NO:29;
   (d) a heavy chain variable region (VH) comprising the amino acid sequence of SEQ ID NO:42, and a light chain variable region (VL) comprising the amino acid sequence of SEQ ID NO:53;
   (e) a heavy chain variable region (VH) comprising the amino acid sequence of SEQ ID NO:64, and a light chain variable region (VL) comprising the amino acid sequence of SEQ ID NO:75;
   (f) a heavy chain variable region (VH) comprising the amino acid sequence of SEQ ID NO:88, and a light chain variable region (VL) comprising the amino acid sequence of SEQ ID NO:99;
   (g) heavy chain variable region (VH) comprising the amino acid sequence of SEQ ID NO:112, and a light chain variable region (VL) comprising the amino acid sequence of SEQ ID NO: 119;
   (h) heavy chain variable region (VH) comprising the amino acid sequence of SEQ ID NO:132, and a light chain variable region (VL) comprising the amino acid sequence of SEQ ID NO:143;
   (i) heavy chain variable region (VH) comprising the amino acid sequence of SEQ ID NO:149, and a light chain variable region (VL) comprising the amino acid sequence of SEQ ID NO:159;
   (j) heavy chain variable region (VH) comprising the amino acid sequence of SEQ ID NO:165, and a light chain variable region (VL) comprising the amino acid sequence of SEQ ID NO:171;
   (k) heavy chain variable region (VH) comprising the amino acid sequence of SEQ ID NO:184, and a light chain variable region (VL) comprising the amino acid sequence of SEQ ID NO:190;
   (l) heavy chain variable region (VH) comprising the amino acid sequence of SEQ ID NO:196, and a light chain variable region (VL) comprising the amino acid sequence of SEQ ID NO:202;
   (m) heavy chain variable region (VH) comprising the amino acid sequence of SEQ ID NO:215, and a light chain variable region (VL) comprising the amino acid sequence of SEQ ID NO:221;
   (n) heavy chain variable region (VH) comprising the amino acid sequence of SEQ ID NO:227, and a light chain variable region (VL) comprising the amino acid sequence of SEQ ID NO:233;
   (o) heavy chain variable region (VH) comprising the amino acid sequence of SEQ ID NO:239, and a light chain variable region (VL) comprising the amino acid sequence of SEQ ID NO:248; or
   (p) heavy chain variable region (VH) comprising the amino acid sequence of SEQ ID NO:254, and a light chain variable region (VL) comprising the amino acid sequence of SEQ ID NO:260.
321. The process of any of clauses 202-243, 319, or 320, the formulation of any of clauses 244-299, 319, or 320, the container of any of clauses 300-302, 319, or 320, the method of any of clauses 303-310, 319, or 320, or the formulation for use of any of clauses 311-320, wherein the antibody or antigen binding fragment thereof that binds PMEL17 comprises:
   (a) a heavy chain comprising the amino acid sequence of SEQ ID NO:12, and a light chain comprising the amino acid sequence of SEQ ID NO:23;
   (b) a heavy chain comprising the amino acid sequence of SEQ ID NO:12, and a light chain comprising the amino acid sequence of SEQ ID NO:27;
   (c) a heavy chain comprising the amino acid sequence of SEQ ID NO:12, and a light chain comprising the amino acid sequence of SEQ ID NO:31;
   (d) a heavy chain comprising the amino acid sequence of SEQ ID NO:44, and a light chain comprising the amino acid sequence of SEQ ID NO:55;
   (e) a heavy chain comprising the amino acid sequence of SEQ ID NO:66, and a light chain comprising the amino acid sequence of SEQ ID NO:77;
   (f) a heavy chain comprising the amino acid sequence of SEQ ID NO:90, and a light chain comprising the amino acid sequence of SEQ ID NO:101;
   (g) a heavy chain comprising the amino acid sequence of SEQ ID NO:114, and a light chain comprising the amino acid sequence of SEQ ID NO:121;
   (h) a heavy chain comprising the amino acid sequence of SEQ ID NO:134, and a light chain comprising the amino acid sequence of SEQ ID NO:145;
   (i) a heavy chain comprising the amino acid sequence of SEQ ID NO:151, and a light chain comprising the amino acid sequence of SEQ ID NO:161;
   (j) a heavy chain comprising the amino acid sequence of SEQ ID NO:167, and a light chain comprising the amino acid sequence of SEQ ID NO:173;
   (k) a heavy chain comprising the amino acid sequence of SEQ ID NO:186, and a light chain comprising the amino acid sequence of SEQ ID NO:192;
   (l) a heavy chain comprising the amino acid sequence of SEQ ID NO:198, and a light chain comprising the amino acid sequence of SEQ ID NO:204;
   (m) a heavy chain comprising the amino acid sequence of SEQ ID NO:217, and a light chain comprising the amino acid sequence of SEQ ID NO:223;
   (n) a heavy chain comprising the amino acid sequence of SEQ ID NO:229, and a light chain comprising the amino acid sequence of SEQ ID NO:235;
   (o) a heavy chain comprising the amino acid sequence of SEQ ID NO:241, and a light chain comprising the amino acid sequence of SEQ ID NO:250;
   (p) a heavy chain comprising the amino acid sequence of SEQ ID NO:256, and a light chain comprising the amino acid sequence of SEQ ID NO:262;
   (q) a heavy chain comprising the amino acid sequence of SEQ ID NO:283, and a light chain comprising the amino acid sequence of SEQ ID NO:27;
   (r) a heavy chain comprising the amino acid sequence of SEQ ID NO:283, and a light chain comprising the amino acid sequence of SEQ ID NO:31; or
   (s) a heavy chain comprising the amino acid sequence of SQ ID NO:315, and a light chain comprising the amino acid sequence of SEQ ID NO:101.
322. The process of any of clauses 202-243 or 319-321, the formulation of any of clauses 244-299 or 319-321, the container of any of clauses 300-302 or 319-321, the method of any of clauses 303-310 or 319-321, or the formulation for use of any of clauses 311-321, wherein the antibody or antigen binding fragment thereof comprises one or more cysteine substitutions.
323. The process of any of clauses 202-243 or 319-322, the formulation of any of clauses 244-299 or 319-322, the container of any of clauses 300-302 or 319-322, the method of any of clauses 303-310 or 319-322, or the formulation for use of any of clauses 311-322, wherein the antibody or antigen binding fragment thereof comprises one or more cysteine substitutions selected from E152C, S375C, or both E152C and S375C of the heavy chain of the antibody or antigen binding fragment thereof, wherein the position is numbered according to the EU system.
324. The process of any of clauses 202-243 or 319-323, the formulation of any of clauses 244-299 or 319-323, the container of any of clauses 300-302 or 319-323, the method of any of clauses 303-310 or 319-323, or the formulation for use of any of clauses 311-323, wherein the antibody is a monoclonal antibody, an isolated antibody, a synthetic antibody, or an engineered antibody.
325. The process of any of clauses 237-243 or 319-324, the formulation of any of clauses 244-299 or 319-324, the container of any of clauses 300-302 or 319-324, the method of any of clauses 303-310 or 319-324, or the formulation for use of any of clauses 311-324, wherein the antibody drug conjugate comprises the formula (C)

   Ab-(L_{A}-(D)ₙ)_{y} (C)

   wherein:
   D is a GNAQ inhibitor, a GNA11 inhibitor or an inhibitor of GNAQ and GNA11;
   Ab is an antibody or antigen binding fragment thereof that binds to human PMEL17 protein;
   L_{A} is a linker;
   n is 1, 2, 3 or 4, and
   y is 1, 2, 3 or 4.
326. The process or the formulation of clause 325, wherein said n is 1.
327. The process or the formulation of any of clauses 325 or 326, wherein said y is 2.
328. The process or the formulation of any of clauses 325-327, wherein said linker is a cleavable linker or a non-cleavable linker.
329. The process or the formulation of any of clauses 325-328, wherein said linker comprises a ValCit peptide linker.
330. The process or the formulation of any of clauses325-329, wherein said drug moiety is an inhibitor of GNAQ and GNA11.
331. The process or the formulation of any of clauses 325-330, wherein D is 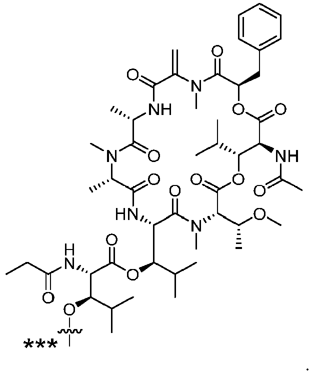
332. The process or the formulation of any of clauses 325-330, wherein D is any of compound (A1) or compounds 2-15.
333. The process or the formulation of any of clauses 325-330, wherein the antibody drug conjugate has the following structure, 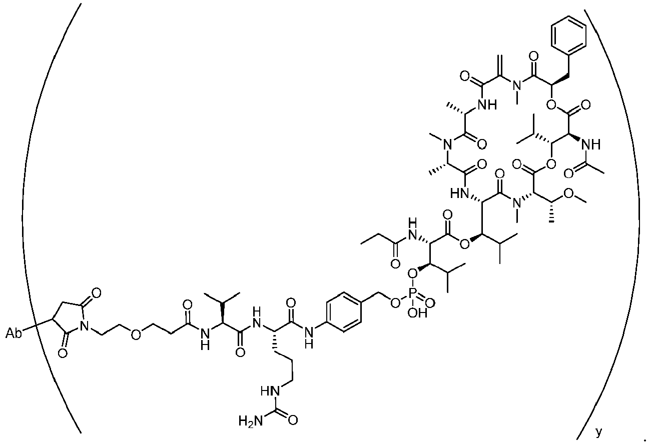
334. The process or the formulation of any of clauses 325-330, wherein the antibody drug conjugate has the following Formula (C-2): 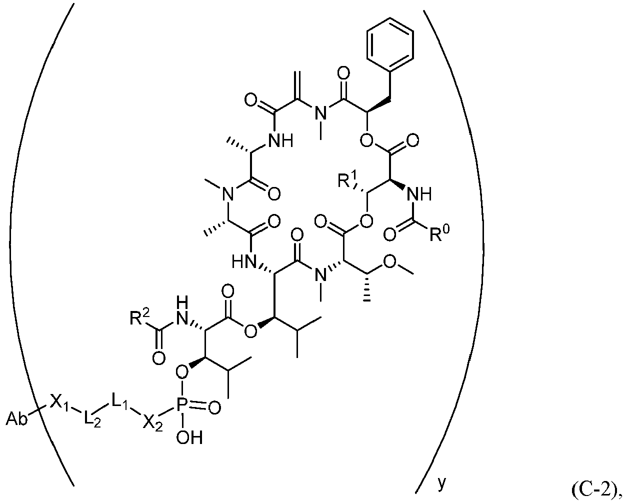 wherein:
   R⁰ is methyl or ethyl;
   R¹ is methyl or isopropyl;
   R² is methyl or ethyl;
   Ab is an antibody or antigen binding fragment thereof that binds to human PMEL17 protein;
   X₁ is a bivalent coupling group;
   X₂ is a self-immolative spacer;
   L₁ is a bivalent peptide linker;
   L₂ is a bond or a linker, and
   y is 1, 2, 3 or 4.
335. The process or the formulation of any of clauses 325-330, wherein the antibody drug conjugate has the following Formula (Cb-2): 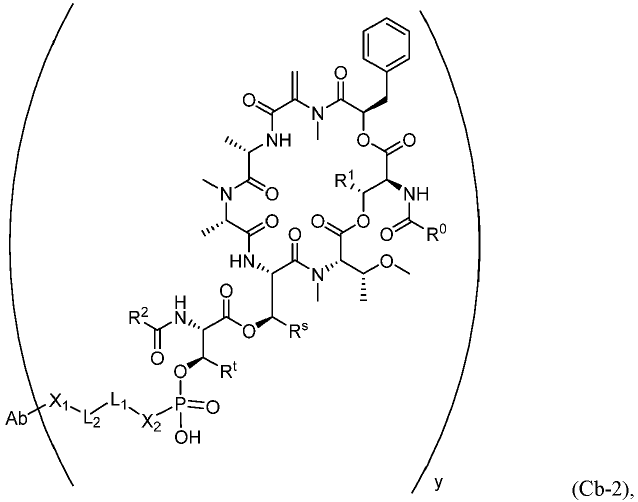 wherein:
   R⁰ is methyl or ethyl;
   R¹, R⁵, and R¹ are each independently methyl, methylthiomethyl, or isopropyl;
   R² is methyl or ethyl;
   Ab is an antibody or antigen binding fragment thereof that binds to human PMEL17 protein;
   X₁ is a bivalent coupling group;
   X₂ is a self-immolative spacer;
   L₁ is a bivalent peptide linker;
   L₂ is a bond or a linker, and
   y is 1, 2, 3 or 4.
336. The process or the formulation of any of clauses 325-330, wherein the antibody drug conjugate has the following Formula (Cc-2): 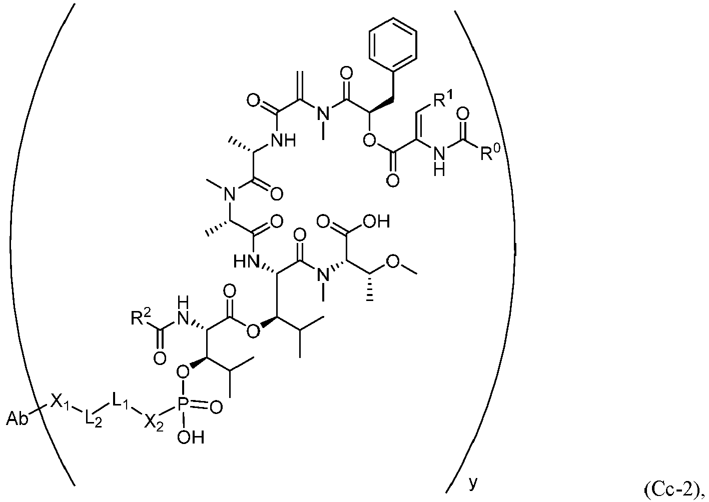 wherein:
   R⁰ is methyl or ethyl;
   R¹ is methyl or isopropyl;
   R² is methyl or ethyl;
   Ab is an antibody or antigen binding fragment thereof that binds to human PMEL17 protein;
   X₁ is a bivalent coupling group;
   X₂ is a self-immolative spacer;
   L₁ is a bivalent peptide linker;
   L₂ is a bond or a linker, and
   y is 1, 2, 3 or 4.
337. A method of treating a cancer, comprising administering to a subject in need thereof an antibody drug conjugate (ADC) at a dose of 1 mg/kg to 16 mg/kg, wherein the ADC has following structure: 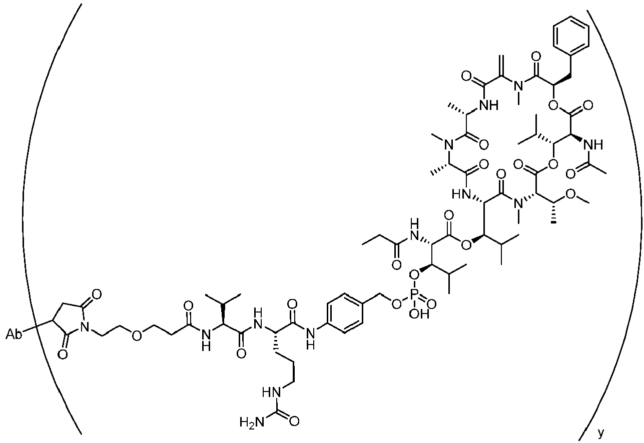 wherein:
   Ab is an antibody or antigen binding fragment thereof that binds to human PMEL17 protein and comprises:
      (a) a heavy chain CDR1 of SEQ ID NO: 1, a heavy chain CDR2 of SEQ ID NO: 2, a heavy chain CDR3 of SEQ ID NO: 3, a light chain CDR1 of SEQ ID NO: 14, a light chain CDR2 of SEQ ID NO: 15, and a light chain CDR3 of SEQ ID NO: 16;
      (b) a heavy chain CDR1 of SEQ ID NO: 4, a heavy chain CDR2 of SEQ ID NO: 2, a heavy chain CDR3 of SEQ ID NO: 3, a light chain CDR1 of SEQ ID NO: 14, a light chain CDR2 of SEQ ID NO: 15, and a light chain CDR3 of SEQ ID NO: 16;
      (c) a heavy chain CDR1 of SEQ ID NO: 5, a heavy chain CDR2 of SEQ ID NO: 6, a heavy chain CDR3 of SEQ ID NO: 3, a light chain CDR1 of SEQ ID NO: 17, a light chain CDR2 of SEQ ID NO: 18, and a light chain CDR3 of SEQ ID NO: 19; or
      (d) a heavy chain CDR1 of SEQ ID NO: 7, a heavy chain CDR2 of SEQ ID NO: 8, a heavy chain CDR3 of SEQ ID NO: 9, a light chain CDR1 of SEQ ID NO: 20, a light chain CDR2 of SEQ ID NO: 18, and a light chain CDR3 of SEQ ID NO: 16;
   y is 2; and
   wherein the cancer expresses PMEL17, contains a mutation of the GNAQ or GNA11 gene, or expresses PMEL17 and contains a mutation of GNAQ, GNA11, or both
   thereby treating the cancer.
338. The method of clause 337, wherein the ADC is administered at a dose of 2 mg/kg to 15 mg/kg once every two weeks intravenously.
339. The method of clause 337, wherein the ADC is administered at a dose of 1 mg/kg, 2 mg/kg, 4 mg/kg, 8 mg/kg, 12 mg/kg, or 16 mg/kg, once every two weeks intravenously.
340. The method of any of clauses 337-339, wherein the cancer is a carcinoma, sarcoma, leukemia, lymphoma, eye cancer, eye neoplasm, melanoma, or a metastatic cancer thereof.
341. The method of clause 340, wherein the carcinoma is a hepatocellular carcinoma, or a metastatic lesion thereof.
342. The method of clause 340, wherein the melanoma is a uveal melanoma, non-uveal melanoma, malignant melanoma, ocular melanoma, mucosal melanoma, subcutaneous melanoma, cutaneous melanoma, or a metastatic lesion thereof.
343. The method of any of clauses 337-342, wherein the subject has been treated with tebentafusp prior to the administration of the ADC.
344. The method of any of clauses 337-342, wherein the subject has not been treated with tebentafusp prior to the administration of the ADC.
345. The method of any of clauses 337-344, wherein the Ab comprises a heavy chain variable region (VH) comprising the amino acid sequence of SEQ ID NO: 10 and a light chain variable region (VL) comprising the amino acid sequence of SEQ ID NO: 25.
346. The method of any of clauses 337-345, wherein the Ab comprises a heavy chain comprising the amino acid sequence of SEQ ID NO: 283 and a light chain comprising the amino acid sequence of SEQ ID NO: 27.
347. The method of clause 346, wherein the heavy chain comprises an N-glycosylation site located at Asn306.
348. The method of any of clauses 337-347, wherein the method further comprises determining the expression of one or more biomarkers selected from PMEL17, pERK, CD8, PD-L1, DUSP6, RASGRP3, or any combination thereof in a sample from the subject.
349. The method of clause 348, wherein the sample is obtained from the subject either before, during and/or after administration of the ADC.
350. The method of clause 348 or 349, wherein the sample is a tumor sample, tumor-adjacent tissue sample, or a bodily fluid sample (e.g., blood, serum, spinal fluid, or urine).
351. Use of an antibody drug conjugate (ADC) in the manufacture of a mediacament for treating a cancer in a subject, wherein the ADC has following structure: 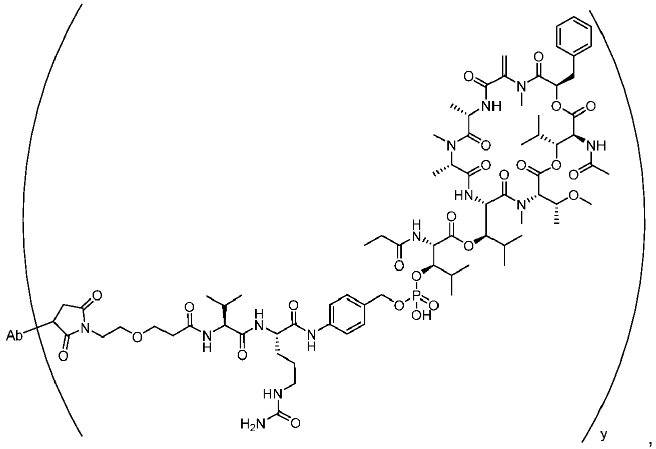 wherein:
   Ab is an antibody or antigen binding fragment thereof that binds to human PMEL17 protein and comprises:
      (a) a heavy chain CDR1 of SEQ ID NO: 1, a heavy chain CDR2 of SEQ ID NO: 2, a heavy chain CDR3 of SEQ ID NO: 3, a light chain CDR1 of SEQ ID NO: 14, a light chain CDR2 of SEQ ID NO: 15, and a light chain CDR3 of SEQ ID NO: 16;
      (b) a heavy chain CDR1 of SEQ ID NO: 4, a heavy chain CDR2 of SEQ ID NO: 2, a heavy chain CDR3 of SEQ ID NO: 3, a light chain CDR1 of SEQ ID NO: 14, a light chain CDR2 of SEQ ID NO: 15, and a light chain CDR3 of SEQ ID NO: 16;
      (c) a heavy chain CDR1 of SEQ ID NO: 5, a heavy chain CDR2 of SEQ ID NO: 6, a heavy chain CDR3 of SEQ ID NO: 3, a light chain CDR1 of SEQ ID NO: 17, a light chain CDR2 of SEQ ID NO: 18, and a light chain CDR3 of SEQ ID NO: 19; or
      (d) a heavy chain CDR1 of SEQ ID NO: 7, a heavy chain CDR2 of SEQ ID NO: 8, a heavy chain CDR3 of SEQ ID NO: 9, a light chain CDR1 of SEQ ID NO: 20, a light chain CDR2 of SEQ ID NO: 18, and a light chain CDR3 of SEQ ID NO: 16;
   y is 2;
   wherein the ADC is administered at a dose of 1 mg/kg to 16 mg/kg, and
   wherein the cancer expresses PMEL17, contains a mutation of the GNAQ or GNA11 gene, or expresses PMEL17 and contains a mutation of GNAQ, GNA11, or both.
352. The use of clause 351, wherein the ADC is administered at a dose of 2 mg/kg to 15 mg/kg once every two weeks intravenously.
353. The use of clause 351, wherein the ADC is administered at a dose of 1 mg/kg, 2 mg/kg, 4 mg/kg, 8 mg/kg, 12 mg/kg, or 16 mg/kg, once every two weeks intravenously.
354. The use of any of clauses 351-353, wherein the cancer is a carcinoma, sarcoma, leukemia, lymphoma, eye cancer, eye neoplasm, melanoma, or a metastatic cancer thereof.
355. The use of clause 354, wherein the carcinoma is a hepatocellular carcinoma, or a metastatic lesion thereof.
356. The use of any clause 354, wherein the melanoma is a malignant melanoma, uveal melanoma, non-uveal melanoma, ocular melanoma, subcutaneous melanoma, cutaneous melanoma, mucosal melanoma, or a metastatic lesion thereof.
357. The use of any of clauses 351-356, wherein the subject has been treated with tebentafusp prior to the administration of the ADC.
358. The use of any of clauses 351-356, wherein the subject has not been treated with tebentafusp prior to the administration of the ADC.
359. The use of any of clauses 351-358, wherein the Ab comprises a heavy chain variable region (VH) comprising the amino acid sequence of SEQ ID NO: 10 and a light chain variable region (VL) comprising the amino acid sequence of SEQ ID NO: 25.
360. The use of any of clauses 351-359, wherein the Ab comprises a heavy chain comprising the amino acid sequence of SEQ ID NO: 283 and a light chain comprising the amino acid sequence of SEQ ID NO: 27.
361. The use of clause 360, wherein the heavy chain comprises an N-glycosylation site located at Asn306.
362. The use of any of clauses 351-361, wherein the use further comprises determining the expression of one or more biomarkers selected from PMEL17, pERK, CD8, PD-L1, DUSP6, RASGRP3, or any combination thereof in a sample from the subject.
363. The use of clause 362, wherein the sample is obtained from the subject either before, during and/or after administration of the ADC.
364. The use of clause 362 or 363, wherein the sample is a tumor sample, tumor-adjacent tissue sample, or a bodily fluid sample (e.g., blood, serum, spinal fluid, or urine).
365. An antibody drug conjugate (ADC) for use in a method of treating a cancer in a subject, wherein the ADC has following structure: 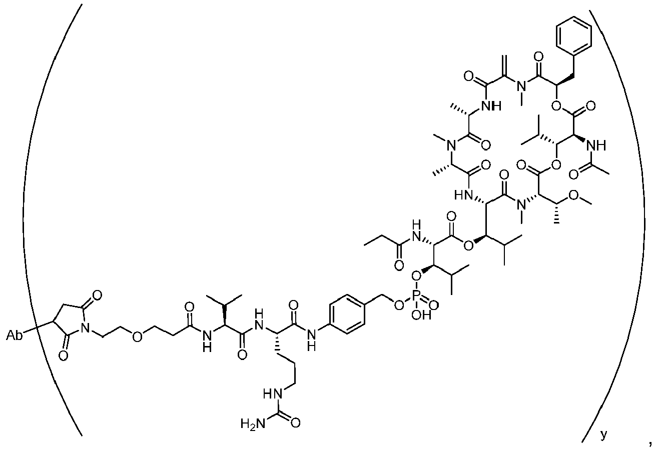 wherein:
   Ab is an antibody or antigen binding fragment thereof that binds to human PMEL17 protein and comprises:
      (a) a heavy chain CDR1 of SEQ ID NO: 1, a heavy chain CDR2 of SEQ ID NO: 2, a heavy chain CDR3 of SEQ ID NO: 3, a light chain CDR1 of SEQ ID NO: 14, a light chain CDR2 of SEQ ID NO: 15, and a light chain CDR3 of SEQ ID NO: 16;
      (b) a heavy chain CDR1 of SEQ ID NO: 4, a heavy chain CDR2 of SEQ ID NO: 2, a heavy chain CDR3 of SEQ ID NO: 3, a light chain CDR1 of SEQ ID NO: 14, a light chain CDR2 of SEQ ID NO: 15, and a light chain CDR3 of SEQ ID NO: 16;
      (c) a heavy chain CDR1 of SEQ ID NO: 5, a heavy chain CDR2 of SEQ ID NO: 6, a heavy chain CDR3 of SEQ ID NO: 3, a light chain CDR1 of SEQ ID NO: 17, a light chain CDR2 of SEQ ID NO: 18, and a light chain CDR3 of SEQ ID NO: 19; or
      (d) a heavy chain CDR1 of SEQ ID NO: 7, a heavy chain CDR2 of SEQ ID NO: 8, a heavy chain CDR3 of SEQ ID NO: 9, a light chain CDR1 of SEQ ID NO: 20, a light chain CDR2 of SEQ ID NO: 18, and a light chain CDR3 of SEQ ID NO: 16;
   y is 2;
   wherein the ADC is administered at a dose of 1 mg/kg to 16 mg/kg, and
   wherein the cancer expresses PMEL17, contains a mutation of the GNAQ or GNA11 gene, or expresses PMEL17 and contains a mutation of GNAQ, GNA11, or both.
366. The ADC for use of clause 365, wherein the ADC is administered at a dose of 2 mg/kg to 15 mg/kg once every two weeks intravenously.
367. The ADC for use of clause 365, wherein the ADC is administered at a dose of 1 mg/kg, 2 mg/kg, 4 mg/kg, 8 mg/kg, 12 mg/kg, or 16 mg/kg, once every two weeks intravenously.
368. The ADC for use of any of clauses 355-367, wherein the cancer is a carcinoma, sarcoma, leukemia, lymphoma, eye cancer, eye neoplasm, melanoma, or a metastatic cancer thereof.
369. The ADC for use of clause 368, wherein the carcinoma is a hepatocellular carcinoma, or a metastatic lesion thereof.
370. The ADC for use of any clause 368, wherein the melanoma is a malignant melanoma, uveal melanoma, non-uveal melanoma, ocular melanoma, subcutaneous melanoma, cutaneous melanoma, mucosal melanoma, or a metastatic lesion thereof.
371. The ADC for use of any of clauses 365-370, wherein the subject has been treated with tebentafusp prior to the administration of the ADC.
372. The ADC for use of any of clauses 365-370, wherein the subject has not been treated with tebentafusp prior to the administration of the ADC.
373. The ADC for use of any of clauses 365-372, wherein the Ab comprises a heavy chain variable region (VH) comprising the amino acid sequence of SEQ ID NO: 10 and a light chain variable region (VL) comprising the amino acid sequence of SEQ ID NO: 25.
374. The ADC for use of any of clauses 365-373, wherein the Ab comprises a heavy chain comprising the amino acid sequence of SEQ ID NO: 283 and a light chain comprising the amino acid sequence of SEQ ID NO: 27.
375. The ADC for use of clause 374, wherein the heavy chain comprises an N-glycosylation site located at Asn306.
376. The ADC for use of any of clauses 365-375, wherein the use further comprises determining the expression of one or more biomarkers selected from PMEL17, pERK, CD8, PD-L1, DUSP6, RASGRP3, or any combination thereof in a sample from the subject.
377. The ADC for use of clause 376, wherein the sample is obtained from the subject either before, during and/or after administration of the ADC.
378. The ADC for use of clause 376 or 377, wherein the sample is a tumor sample, tumor-adjacent tissue sample, or a bodily fluid sample (e.g., blood, serum, spinal fluid, or urine).
379. A method of evaluating a treatment for a cancer in a subject, comprising determining the expression of one or more biomarkers selected from PMEL17, pERK, CD8, PD-L1, DUSP6, RASGRP3, or any combination thereof in a sample from the subject, wherein the treatment comprises administering to the subject an antibody drug conjugate (ADC) at a dose of 1 mg/kg to 16 mg/kg, wherein the ADC has following structure: 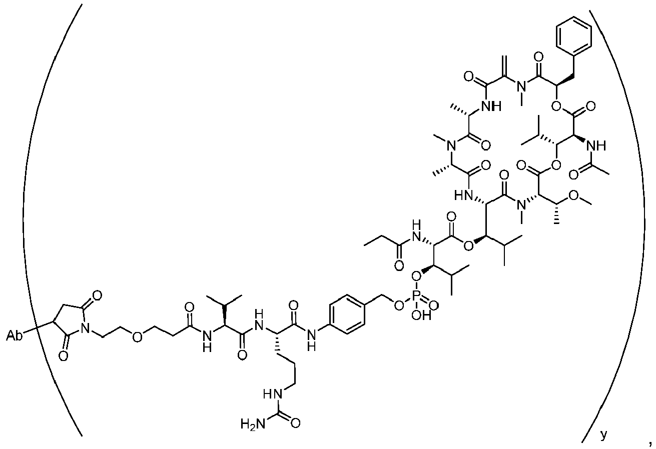 wherein:
   Ab is an antibody or antigen binding fragment thereof that binds to human PMEL17 protein and comprises:
      (a) a heavy chain CDR1 of SEQ ID NO: 1, a heavy chain CDR2 of SEQ ID NO: 2, a heavy chain CDR3 of SEQ ID NO: 3, a light chain CDR1 of SEQ ID NO: 14, a light chain CDR2 of SEQ ID NO: 15, and a light chain CDR3 of SEQ ID NO: 16;
      (b) a heavy chain CDR1 of SEQ ID NO: 4, a heavy chain CDR2 of SEQ ID NO: 2, a heavy chain CDR3 of SEQ ID NO: 3, a light chain CDR1 of SEQ ID NO: 14, a light chain CDR2 of SEQ ID NO:15, and a light chain CDR3 of SEQ ID NO: 16;
      (c) a heavy chain CDR1 of SEQ ID NO: 5, a heavy chain CDR2 of SEQ ID NO: 6, a heavy chain CDR3 of SEQ ID NO: 3, a light chain CDR1 of SEQ ID NO: 17, a light chain CDR2 of SEQ ID NO: 18, and a light chain CDR3 of SEQ ID NO: 19; or
      (d) a heavy chain CDR1 of SEQ ID NO: 7, a heavy chain CDR2 of SEQ ID NO: 8, a heavy chain CDR3 of SEQ ID NO: 9, a light chain CDR1 of SEQ ID NO: 20, a light chain CDR2 of SEQ ID NO: 18, and a light chain CDR3 of SEQ ID NO: 16;
   y is 2; and
   wherein the cancer expresses PMEL17, contains a mutation of the GNAQ or GNA11 gene, or expresses PMEL17 and contains a mutation of GNAQ, GNA11, or both,
   thereby evaluating the treatment for the cancer in the subject.
380. A method of evaluating the progression of a cancer in a subject, comprising determining the expression of one or more biomarkers selected from PMEL17, pERK, CD8, PD-L1, DUSP6, RASGRP3, or any combination thereof in a sample from the subject, wherein the subject has received, is receiving, or will receive an antibody drug conjugate (ADC) at a dose of 1 mg/kg to 16 mg/kg, wherein the ADC has following structure: 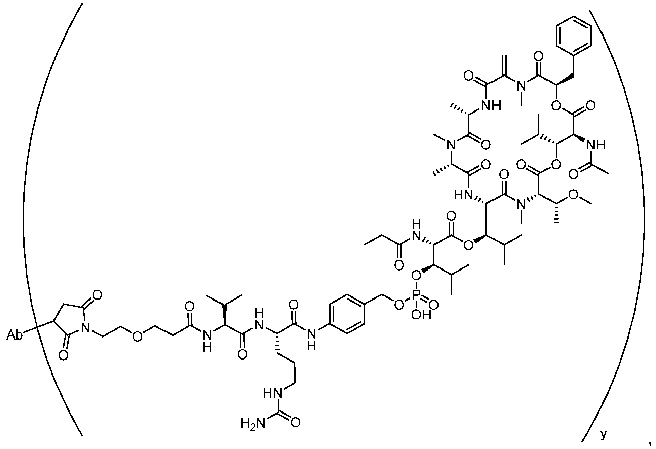 wherein:
   Ab is an antibody or antigen binding fragment thereof that binds to human PMEL17 protein and comprises:
      (a) a heavy chain CDR1 of SEQ ID NO: 1, a heavy chain CDR2 of SEQ ID NO: 2, a heavy chain CDR3 of SEQ ID NO: 3, a light chain CDR1 of SEQ ID NO: 14, a light chain CDR2 of SEQ ID NO: 15, and a light chain CDR3 of SEQ ID NO: 16;
      (b) a heavy chain CDR1 of SEQ ID NO: 4, a heavy chain CDR2 of SEQ ID NO: 2, a heavy chain CDR3 of SEQ ID NO: 3, a light chain CDR1 of SEQ ID NO: 14, a light chain CDR2 of SEQ ID NO:15, and a light chain CDR3 of SEQ ID NO: 16;
      (c) a heavy chain CDR1 of SEQ ID NO: 5, a heavy chain CDR2 of SEQ ID NO: 6, a heavy chain CDR3 of SEQ ID NO: 3, a light chain CDR1 of SEQ ID NO: 17, a light chain CDR2 of SEQ ID NO: 18, and a light chain CDR3 of SEQ ID NO: 19; or
      (d) a heavy chain CDR1 of SEQ ID NO: 7, a heavy chain CDR2 of SEQ ID NO: 8, a heavy chain CDR3 of SEQ ID NO: 9, a light chain CDR1 of SEQ ID NO: 20, a light chain CDR2 of SEQ ID NO: 18, and a light chain CDR3 of SEQ ID NO: 16;
   y is 2; and
   wherein the cancer expresses PMEL17, contains a mutation of the GNAQ or GNA11 gene, or expresses PMEL17 and contains a mutation of GNAQ, GNA11, or both,
   thereby evaluating the progression of the cancer in the subject.
381. A method of selecting a treatment for a subject having a cancer, comprising determining the expression of one or more biomarkers selected from PMEL17, pERK, CD8, PD-L1, DUSP6, RASGRP3, or any combination thereof in a sample from the subject, wherein the treatment comprises administering to the subject an antibody drug conjugate (ADC) at a dose of 1 mg/kg to 16 mg/kg, wherein the ADC has following structure: 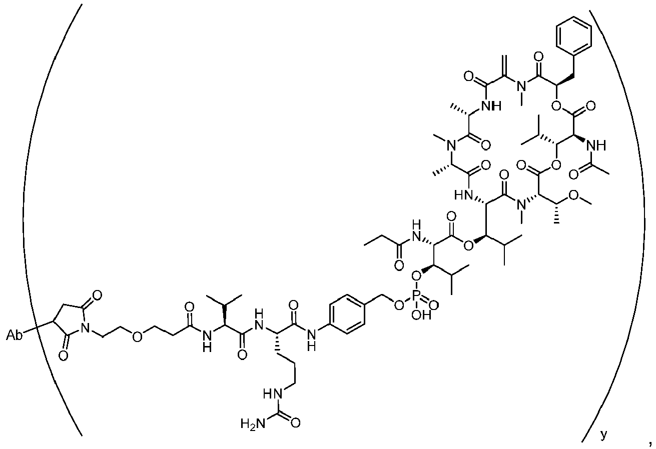 wherein:
   Ab is an antibody or antigen binding fragment thereof that binds to human PMEL17 protein and comprises:
      (a) a heavy chain CDR1 of SEQ ID NO: 1, a heavy chain CDR2 of SEQ ID NO: 2, a heavy chain CDR3 of SEQ ID NO: 3, a light chain CDR1 of SEQ ID NO: 14, a light chain CDR2 of SEQ ID NO: 15, and a light chain CDR3 of SEQ ID NO: 16;
      (b) a heavy chain CDR1 of SEQ ID NO: 4, a heavy chain CDR2 of SEQ ID NO: 2, a heavy chain CDR3 of SEQ ID NO: 3, a light chain CDR1 of SEQ ID NO: 14, a light chain CDR2 of SEQ ID NO: 15, and a light chain CDR3 of SEQ ID NO: 16;
      (c) a heavy chain CDR1 of SEQ ID NO: 5, a heavy chain CDR2 of SEQ ID NO: 6, a heavy chain CDR3 of SEQ ID NO: 3, a light chain CDR1 of SEQ ID NO: 17, a light chain CDR2 of SEQ ID NO: 18, and a light chain CDR3 of SEQ ID NO: 19; or
      (d) a heavy chain CDR1 of SEQ ID NO: 7, a heavy chain CDR2 of SEQ ID NO: 8, a heavy chain CDR3 of SEQ ID NO: 9, a light chain CDR1 of SEQ ID NO: 20, a light chain CDR2 of SEQ ID NO: 18, and a light chain CDR3 of SEQ ID NO: 16;
   y is 2; and
   wherein the cancer expresses PMEL17, contains a mutation of the GNAQ or GNA11 gene, or expresses PMEL17 and contains a mutation of GNAQ, GNA11, or both,
   thereby selecting the treatment for the subject having the cancer.
382. A method of selecting a subject for a treatment for a cancer, comprising determining the expression of one or more biomarkers selected from PMEL17, pERK, CD8, PD-L1, DUSP6, RASGRP3, or any combination thereof in a sample from the subject, wherein the treatment comprises administering to the subject an antibody drug conjugate (ADC) at a dose of 1 mg/kg to 16 mg/kg, wherein the ADC has following structure: 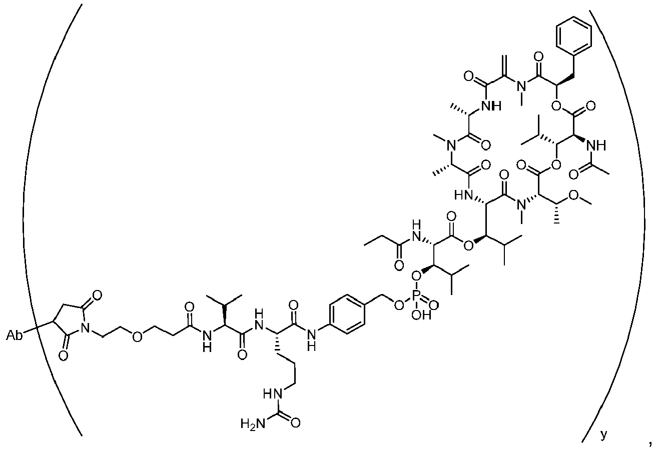 wherein:
   Ab is an antibody or antigen binding fragment thereof that binds to human PMEL17 protein and comprises:
      (a) a heavy chain CDR1 of SEQ ID NO: 1, a heavy chain CDR2 of SEQ ID NO: 2, a heavy chain CDR3 of SEQ ID NO: 3, a light chain CDR1 of SEQ ID NO: 14, a light chain CDR2 of SEQ ID NO: 15, and a light chain CDR3 of SEQ ID NO: 16;
      (b) a heavy chain CDR1 of SEQ ID NO: 4, a heavy chain CDR2 of SEQ ID NO: 2, a heavy chain CDR3 of SEQ ID NO: 3, a light chain CDR1 of SEQ ID NO: 14, a light chain CDR2 of SEQ ID NO: 15, and a light chain CDR3 of SEQ ID NO: 16;
      (c) a heavy chain CDR1 of SEQ ID NO: 5, a heavy chain CDR2 of SEQ ID NO: 6, a heavy chain CDR3 of SEQ ID NO: 3, a light chain CDR1 of SEQ ID NO: 17, a light chain CDR2 of SEQ ID NO: 18, and a light chain CDR3 of SEQ ID NO: 19; or
      (d) a heavy chain CDR1 of SEQ ID NO: 7, a heavy chain CDR2 of SEQ ID NO: 8, a heavy chain CDR3 of SEQ ID NO: 9, a light chain CDR1 of SEQ ID NO: 20, a light chain CDR2 of SEQ ID NO: 18, and a light chain CDR3 of SEQ ID NO: 16;
   y is 2; and
   wherein the cancer expresses PMEL17, contains a mutation of the GNAQ or GNA11 gene, or expresses PMEL17 and contains a mutation of GNAQ, GNA11, or both,
   thereby selecting the subject for the treatment of the cancer.

## Claims

1. A process for producing a partially reoxidized antibody or antigen binding fragment thereof, comprising:
providing an antibody or antigen binding fragment thereof comprising one or more capped cysteine residues;
reducing the one or more capped cysteine residues, thereby providing an antibody or antigen binding fragment thereof comprising one or more decapped cysteine residues; and
storing the antibody or antigen binding fragment thereof comprising the one or more decapped cysteine residues under oxidation conditions that allow intrachain disulfide bridges opened during the reducing step to reform,
thereby producing the partially reoxidized antibody or antigen binding fragment thereof.

2. The process of claim 1, wherein:
(a) the one or more capped cysteine residues are located in the constant domain of a heavy chain of the antibody or antigen binding fragment thereof,
(b) the antibody or antigen binding fragment thereof comprises two or four capped cysteine residues;
(c) the antibody or antigen binding fragment thereof comprises a capped cysteine residue located in a CH1 region, or each CH1 region, of the antibody or antigen binding fragment thereof,
(d) each capped cysteine residue is an engineered surface-exposed cysteine residue;
(e) the antibody or antigen binding fragment thereof comprises a capped cysteine residue located at residue 152 (EU numbering) in the CH1 region of the antibody or antigen binding fragment thereof, and/or
(f) the one or more capped cysteine residues are capped with a cysteine or a glutathione.

3. The process of claim 1 or 2, wherein:
(a) the one or more decapped cysteine residues are located in the constant domain of a heavy chain of the antibody or antigen binding fragment thereof,
(b) the antibody or antigen binding fragment thereof comprises two or four decapped cysteine residues;
(c) the antibody or antigen binding fragment thereof comprises a decapped cysteine residue located in a CH1 region, or each CH1 region, of the antibody or antigen binding fragment thereof,
(d) each decapped cysteine residue is an engineered surface-exposed cysteine residue; and/or
(e) the antibody or antigen binding fragment thereof comprises a decapped cysteine residue located at residue 152 (EU numbering) in the CH1 region of the antibody or antigen binding fragment thereof.

4. The process of any one of claims 1-3, wherein the process comprises reducing the one or more capped cysteine residues by contacting the antibody or antigen binding fragment thereof comprising one or more capped cysteine residues with a reduction wash buffer, thereby providing an antibody or antigen binding fragment comprising one or more decapped cysteine residues.

5. The process of claim 4, wherein:
(a) the reduction wash buffer comprises 5 mM to 50 mM, 10 mM to 40 mM, 20 mM to 30 mM, 5 mM to 15 mM, 5 mM to 25 mM, 5 mM to 35 mM, 5 mM to 45 mM, 40 mM to 50 mM, 30 mM to 50 mM, 20 mM to 50 mM, 10 mM to 50 mM, 10 mM to 20 mM, 15 mM to 25 mM, 25 mM to 35 mM, 30 mM to 40 mM, 35 mM to 45 mM, 10 mM, 12 mM, 14 mM, 16 mM, 20 mM, 25 mM, or 30 mM cysteine;
(b) the reduction wash buffer has a pH of 6.0 to 7.5, 6.5 to 7.2, 6.8 to 7.0, or 6.9; and/or
(c) the step of contacting the antibody or antigen binding fragment thereof comprising one or more capped cysteine residues with the reduction wash buffer is performed on a column, optionally wherein the column is a cation exchange chromatography column.

6. The process of claim 4 or 5, wherein the process further comprises collecting the antibody or antigen binding fragment thereof comprising one or more decapped cysteine residues in an eluate after contacting with the reduction wash buffer.

7. The process of claim 6, wherein:
(a) the eluate has a pH of 5.5 to 6.0, optionally wherein the pH is 5.8; and/or
(b) the concentration of the antibody or antigen binding fragment thereof comprising one or more decapped cysteine residues in the eluate is 5 g/L to 25 g/L, 8 g/L to 20 g/L, 10 g/L to 18 g/L, 12 g/L to 15 g/L, 5 g/L to 20 g/L, 5 g/L to 15 g/L, 5 g/L to 10 g/L, 20 g/L to 25 g/L, 15 g/L to 25 g/L, 10 g/L to 25 g/L, 10 g/L to 20 g/L, 13 g/L to 14 g/L, 12 g/L to 15 g/L, 5 g/L, 6 g/L, 7 g/L, 8 g/L, 9 g/L, 10 g/L, 11 g/L, 12 g/L, 13 g/L, 13.5 g/L, 14 g/L, 15 g/L, 16 g/L, 17 g/L, 18 g/L, 19 g/L, 20 g/L, 21 g/L, 22 g/L, 23 g/L, 24 g/L, or 25 g/L.

8. The process of claim 6 or 7, wherein the antibody or antigen binding fragment thereof comprising one or more decapped cysteine residues is stored in the eluate with or without stirring.

9. The process of any one of claims 1-8, wherein the antibody or antigen binding fragment thereof comprising one or more decapped cysteine residues is stored:
(a) in a container filled to a maximum of 50% to 70% volume, or at 60%;
(b) for 12 hours to 96 hours, 12 hours to 84 hours, 24 hours to 84 hours, 36 hours to 72 hours, 48 hours to 60 hours, 24 hours to 72 hours, 24 hours to 60 hours, 24 hours to 48 hours, 24 hours to 36 hours, 72 hours to 84 hours, 60 hours to 84 hours, 48 hours to 84 hours, 36 hours to 84 hours, 12 hours to 36 hours, 24 hours to 48 hours, 36 hours to 60 hours, 48 hours to 72 hours, 72 hours to 96 hours, 12 hours, 24 hours, 36 hours, 48 hours, 60 hours, 72 hours, 84 hours, or 96 hours;
(c) for 48 hours to 72 hours without stirring or for 12 to 36 hours, or 24 hours, with stirring;
(d) at room temperature, or at 2 °C to 8 °C, or at 4 °C, optionally for at least 48 hours or from 48 hours to 96 hours; and/or
(e) with air overlay.

10. The process of any one of claims 1-9, wherein the process further comprises purifying the partially reoxidized antibody or antigen binding fragment thereof.

11. The process of any one of claims 1-10, wherein the antibody or antigen binding fragment thereof comprises one or more cysteine substitutions, optionally comprising a substitution of:
(a) one or more amino acids with cysteine on a constant region at positions selected from the group consisting of positions 117, 119, 121, 124, 139, 152, 153, 155, 157, 164, 169, 171, 174, 189, 191, 195, 197, 205, 207, 246, 258, 269, 274, 286, 288, 290, 292, 293, 320, 322, 326, 333, 334, 335, 337, 344, 355, 360, 375, 382, 390, 392, 398, 400, and 422 of a heavy chain of the antibody or antigen binding fragment thereof, and wherein the positions are numbered according to the EU system;
(b) one or more amino acids with cysteine on a constant region at positions selected from the group consisting of positions 107, 108, 109, 114, 129, 142, 143, 145, 152, 154, 156, 159, 161, 165, 168, 169, 170, 182, 183, 197, 199, and 203 of a light chain of the antibody or antigen binding fragment thereof, wherein the positions are numbered according to the EU system, and wherein the light chain is a human kappa light chain; and/or
(c) an amino acid with cysteine on a constant region at position 152, position 375, or both positions 152 and 375, of a heavy chain of the antibody or antigen binding fragment thereof, wherein the positions are numbered according to the EU system.

12. The process of any one of claims 1-11, wherein the antibody or antigen binding fragment thereof is a monoclonal antibody, an isolated antibody, a synthetic antibody, or an engineered antibody.

13. A process of producing an antibody drug conjugate, the process comprising conjugating a linker-drug moiety to the partially reoxidized antibody or antigen binding fragment thereof produced by a process of any one of claims 1-12, thereby producing the antibody drug conjugate.

14. The process of claim 13, wherein the process:
(a) further comprises purifying the antibody drug conjugate;
(b) results in at least 75%, 80%, 85%, 90%, or 95% on-site coupling, or one linker-drug moiety per heavy chain ("HC1"); and/or
(c) results in less than 25%, 20%, 15%, 10%, or 5% off-site coupling, or one linker-drug moiety per light chain ("LC1") and/or two linker-drug moieties per heavy chain ("HC2").

15. The process of any one of claims 1-14, wherein the process results in at least 70%, 75%, 80%, 85%, 90%, or 95% purity, optionally wherein the purity is determined by non-reduced CE-SDS.
